# EUROPEAN PATENT SPECIFICATION

(11) **EP 2 954 323 B1**
(45) Date of publication and mention of the grant of the patent: **15.04.2020**
(21) Application number: 14706740.9
(22) Date of filing: 07.02.2014
(51) Int. Cl.: G01N 33/50, C12Q 1/68

(54) **USE OF TRANSLATIONAL PROFILING TO IDENTIFY TARGET MOLECULES FOR THERAPEUTIC TREATMENT**
VERWENDUNG VON TRANSLATORISCHER PROFILIERUNG ZUR IDENTIFIZIERUNG VON ZIELMOLEKÜLEN ZUR THERAPEUTISCHEN BEHANDLUNG
UTILISATION DU PROFILAGE TRADUCTIONNEL POUR IDENTIFIER DES MOLÉCULES CIBLES POUR UN TRAITEMENT THÉRAPEUTIQUE

(30) Priority: 07.02.2013 US 201361762115 P
(43) Date of publication of application: 16.12.2015
(73) Proprietor: The Regents of the University of California, Oakland, CA 94607 (US)
(72) Inventor: RUGGERO, Davide, San Francisco California 94103 (US); HSIEH, Andrew, San Francisco California 94118 (US); EDLIND, Merritt, Berkeley California 94705 (US); SHOKAT, Kevan M., San Francisco California 94121 (US); APPLEMAN, James, San Diego California 92117 (US); WORLAND, Steve, Del Mar California 92014 (US)
(74) Representative: J A Kemp LLP
(86) International application number: PCT/US2014/015428
(87) International publication number: WO 2014/124339

(56) References cited:
- D. RUGGERO: "Translational Control in Cancer Etiology", COLD SPRING HARBOR PERSPECTIVES IN BIOLOGY, vol. 5, no. 2, 5 July 2012 (2012-07-05), pages a012336-a012336, XP055381595, DOI: 10.1101/cshperspect.a012336
- KEYUR PATEL ET AL: "High predictive accuracy of an unbiased proteomic profile for sustained virologic response in chronic hepatitis C patients", HEPATOLOGY, vol. 53, no. 6, 1 June 2011 (2011-06-01), pages 1809-1818, XP055381745, ISSN: 0270-9139, DOI: 10.1002/hep.24284
- ANDREW C. HSIEH ET AL: "The translational landscape of mTOR signalling steers cancer initiation and metastasis", NATURE, vol. 485, no. 7396, 22 February 2012 (2012-02-22), pages 55-61, XP055120586, ISSN: 0028-0836, DOI: 10.1038/nature10912
- ANDREW C. HSIEH ET AL: "Genetic Dissection of the Oncogenic mTOR Pathway Reveals Druggable Addiction to Translational Control via 4EBP-eIF4E", CANCER CELL, vol. 17, no. 3, 1 March 2010 (2010-03-01), pages 249-261, XP055120584, ISSN: 1535-6108, DOI: 10.1016/j.ccr.2010.01.021
- D. RUGGERO: "Translational Control in Cancer Etiology", COLD SPRING HARBOR PERSPECTIVES IN BIOLOGY, vol. 5, no. 2, 5 July 2012 (2012-07-05), pages a012336-a012336, XP055381595, DOI: 10.1101/cshperspect.a012336
- KEYUR PATEL ET AL: "High predictive accuracy of an unbiased proteomic profile for sustained virologic response in chronic hepatitis C patients", HEPATOLOGY, vol. 53, no. 6, 1 June 2011 (2011-06-01), pages 1809-1818, XP055381745, ISSN: 0270-9139, DOI: 10.1002/hep.24284
- ROBERT B WEISS ET AL: "Translation Goes Global", SCIENCE, vol. 334, no. 6062, 16 December 2011 (2011-12-16), pages 1509-15010, XP055534523, US ISSN: 0036-8075, DOI: 10.1126/science.1215655

### SEQUENCE LISTING

<110> Ruggero, Davide
   Hsieh, Andrew
   Edlind, Merritt
   Shokat, Kevan M.
   Appleman, James
   Worland, Steve
   The Regents of the University of California
<120> Use of Translational Profiling to
   Identify Target Molecules for Therapeutic Treatment
<130> 84850-898998
<140> WO Not yet assigned
   <141> Not yet assigned
<150> US 61/762,115
   <151> 2013-02-07
<160> 287
<170> FastSEQ for Windows Version 4.0
<210> 1
   <211> 3163
   <212> DNA
   <213> Homo sapiens
<220>
   <223> eukaryotic translation elongation factor 2 (EEF2, EF-2, EF2), polypeptidyl-tRNA translocase, SCA26
<220>
   <221> CDS
   <222> (84) ... (2660)
   <223> EEF2
<400> 1
   ctcttccgcc gtcgtcgccg ccatcctcgg cgcgactcgc ttctttcggt tctacctggg 60
   agaatccacc gccatccgcc accatggtga acttcacggt agaccagatc cgcgccatca 120
   tggacaagaa ggccaacatc cgcaacatgt ctgtcatcgc ccacgtggac catggcaagt 180
   ccacgctgac agactccctg gtgtgcaagg cgggcatcat cgcctcggcc cgggccgggg 240
   agacacgctt cactgatacc cggaaggacg agcaggagcg ttgcatcacc atcaagtcaa 300
   ctgccatctc cctcttctac gagctctcgg agaatgactt gaacttcatc aagcagagca 360
   aggacggtgc cggcttcctc atcaacctca ttgactcccc cgggcatgtc gacttctcct 420
   cggaggtgac tgctgccctc cgagtcaccg atggcgcatt ggtggtggtg gactgcgtgt 480
   caggcgtgtg cgtgcagacg gagacagtgc tgcggcaggc cattgccgag cgcatcaagc 540
   ctgtgctgat gatgaacaag atggaccgcg ccctgctgga gctgcagctg gagcccgagg 600
   agctctacca gactttccag cgcatcgtgg agaacgtgaa cgtcatcatc tccacctacg 660
   gcgagggcga gagcggcccc atgggcaaca tcatgatcga tcctgtcctc ggtaccgtgg 720
   gctttgggtc tggcctccac gggtgggcct tcaccctgaa gcagtttgcc gagatgtatg 780
   tggccaagtt cgccgccaag ggggagggcc agttggggcc tgccgagcgg gccaagaaag 840
   tagaggacat gatgaagaag ctgtggggtg acaggtactt tgacccagcc aacggcaagt 900
   tcagcaagtc agccaccagc cccgaaggga agaagctgcc acgcaccttc tgccagctga 960
   tcctggaccc catcttcaag gtgtttgatg cgatcatgaa tttcaagaaa gaggagacag 1020
   caaaactgat agagaaactg gacatcaaac tggacagcga ggacaaggac aaagaaggca 1080
   aacccctgct gaaggctgtg atgcgccgct ggctgcctgc cggagacgcc ttgttgcaga 1140
   tgatcaccat ccacctgccc tcccctgtga cggcccagaa gtaccgctgc gagctcctgt 1200
   acgaggggcc cccggacgac gaggctgcca tgggcattaa aagctgtgac cccaaaggcc 1260
   ctcttatgat gtatatttcc aaaatggtgc caacctccga caaaggtcgg ttctacgcct 1320
   ttggacgagt cttctcgggg ctggtctcca ctggcctgaa ggtcaggatc atggggccca 1380
   actatacccc tgggaagaag gaggacctct acctgaagcc aatccagaga acaatcttga 1440
   tgatgggccg ctacgtggag cccatcgagg atgtgccttg tgggaacatt gtgggcctcg 1500
   tgggcgtgga ccagttcctg gtgaagacgg gcaccatcac caccttcgag cacgcgcaca 1560
   acatgcgggt gatgaagttc agcgtcagcc ctgttgtcag agtggccgtg gaggccaaga 1620
   acccggctga cctgcccaag ctggtggagg ggctgaagcg gctggccaag tccgacccca 1680
   tggtgcagtg catcatcgag gagtcgggag agcatatcat cgcgggcgcc ggcgagctgc 1740
   acctggagat ctgcctgaag gacctggagg aggaccacgc ctgcatcccc atcaagaaat 1800
   ctgacccggt cgtctcgtac cgcgagacgg tcagtgaaga gtcgaacgtg ctctgcctct 1860
   ccaagtcccc caacaagcac aaccggctgt acatgaaggc gcggcccttc cccgacggcc 1920
   tggccgagga catcgataaa ggcgaggtgt ccgcccgtca ggagctcaag cagcgggcgc 1980
   gctacctggc cgagaagtac gagtgggacg tggctgaggc ccgcaagatc tggtgctttg 2040
   ggcccgacgg caccggcccc aacatcctca ccgacatcac caagggtgtg cagtacctca 2100
   acgagatcaa ggacagtgtg gtggccggct tccagtgggc caccaaggag ggcgcactgt 2160
   gtgaggagaa catgcggggt gtgcgcttcg acgtccacga cgtcaccctg cacgccgacg 2220
   ccatccaccg cggagggggc cagatcatcc ccacagcacg gcgctgcctc tatgccagtg 2280
   tgctgaccgc ccagccacgc ctcatggagc ccatctacct tgtggagatc cagtgtccag 2340
   agcaggtggt cggtggcatc tacggggttt tgaacaggaa gcggggccac gtgttcgagg 2400
   agtcccaggt ggccggcacc cccatgtttg tggtcaaggc ctatctgccc gtcaacgagt 2460
   cctttggctt caccgctgac ctgaggtcca acacgggcgg ccaggcgttc ccccagtgtg 2520
   tgtttgacca ctggcagatc ctgcccggag accccttcga caacagcagc cgccccagcc 2580
   aggtggtggc ggagacccgc aagcgcaagg gcctgaaaga aggcatccct gccctggaca 2640
   acttcctgga caaattgtag gcggcccttc ctgcagcgcc tgccgccccg gggactcgca 2700
   gcacccacag caccacgtcc tcgaattctc agacgacacc tggagactgt cccgacacag 2760
   cgacgctccc ctgagaggtt tctggggccc gctgcgtgcc atcactcaac cataacactt 2820
   gatgccgttt ctttcaatat ttatttccag agtccggagg cagcagacac gccctcttag 2880
   tagggactta atgggccggt cggggagggg gaggcgggat gggacaccca acactttttc 2940
   catttcttca gagggaaact cagatgtcca aactaatttt aacaaacgca ttaagaggtt 3000
   tatttgggta catggcccgc agtggctttt gccccagaaa ggggaaagga acacgcgggt 3060
   agatgatttc tagcaggcag gaagtcctgt gcggtgtcac catgagcacc tccagctgta 3120
   ctagtgccat tggaataata aatttgataa ggtggtgaaa aaa                   3163
<210> 2
   <211> 532
   <212> DNA
   <213> Homo sapiens
<220>
   <223> 40S ribosomal protein S12, S12
<220>
   <221> CDS
   <222> (83) ... (481)
   <223> RPS12
<400> 2
   ctctttccct gccgccgccg agtcgcgcgg aggcggaggc ttgggtgcgt tcaagattca 60
   acttcacccg taacccaccg ccatggccga ggaaggcatt gctgctggag gtgtaatgga 120
   cgttaatact gctttacaag aggttctgaa gactgccctc atccacgatg gcctagcacg 180
   tggaattcgc gaagctgcca aagccttaga caagcgccaa gcccatcttt gtgtgcttgc 240
   atccaactgt gatgagccta tgtatgtcaa gttggtggag gccctttgtg ctgaacacca 300
   aatcaaccta attaaggttg atgacaacaa gaaactagga gaatgggtag gcctttgtaa 360
   aattgacaga gaggggaaac cccgtaaagt ggttggttgc agttgtgtag tagttaagga 420
   ctatggcaag gagtctcagg ccaaggatgt cattgaagag tatttcaaat gcaagaaatg 480
   aagaaataaa tctttggctc acaaaaaaaa aaaaaaaaaa aaaaaaaaaa aa         532
<210> 3
   <211> 674
   <212> DNA
   <213> Homo sapiens
<220>
   <223> 60S ribosomal protein L12
<220>
   <221> CDS
   <222> (116) ... (613)
   <223> RPL12
<400> 3
   atctggcttg tccgcgcgat ttccggcctc tcggctttcg gctcggagga ggccaaggtg 60
   caacttcctt cggtcgtccc gaatccgggt tcatccgaca ccagccgcct ccaccatgcc 120
   gccgaagttc gaccccaacg agatcaaagt cgtatacctg aggtgcaccg gaggtgaagt 180
   cggtgccact tctgccctgg cccccaagat cggccccctg ggtctgtctc caaaaaaagt 240
   tggtgatgac attgccaagg caacgggtga ctggaagggc ctgaggatta cagtgaaact 300
   gaccattcag aacagacagg cccagattga ggtggtgcct tctgcctctg ccctgatcat 360
   caaagccctc aaggaaccac caagagacag aaagaaacag aaaaacatta aacacagtgg 420
   gaatatcact tttgatgaga ttgtcaacat tgctcgacag atgcggcacc gatccttagc 480
   cagagaactc tctggaacca ttaaagagat cctggggact gcccagtcag tgggctgtaa 540
   tgttgatggc cgccatcctc atgacatcat cgatgacatc aacagtggtg ctgtggaatg 600
   cccagccagt taagcacaaa ggaaaacatt tcaataaagg atcatttgac aactggtgga 660
   aaaaaaaaaa aaaa                                                   674
<210> 4
   <211> 962
   <212> DNA
   <213> Homo sapiens
<220>
   <223> 40S ribosomal protein S2, S2, LLREP3, protein LLRep3, OK/KNS-cl.6
<220>
   <221> CDS
   <222> (24) ... (905)
   <223> RPS2
<400> 4
   cttcttttcc gacaaaacac caaatggcgg atgacgccgg tgcagcgggg gggcccgggg 60
   gccctggtgg ccctgggatg gggaaccgcg gtggcttccg cggaggtttc ggcagtggca 120
   tccggggccg gggtcgcggc cgtggacggg gccggggccg aggccgcgga gctcgcggag 180
   gcaaggccga ggataaggag tggatgcccg tcaccaagtt gggccgcttg gtcaaggaca 240
   tgaagatcaa gtccctggag gagatctatc tcttctccct gcctattaag gaatcagaga 300
   tcattgattt cttcctgggg gcctctctca aggatgaggt tttgaagatt atgccagtgc 360
   agaagcagac ccgtgccggc cagcgcacca ggttcaaggc atttgttgct atcggggact 420
   acaatggcca cgtcggtctg ggtgttaagt gctccaagga ggtggccacc gccatccgtg 480
   gggccatcat cctggccaag ctctccatcg tccccgtgcg cagaggctac tgggggaaca 540
   agatcggcaa gccccacact gtcccttgca aggtgacagg ccgctgcggc tctgtgctgg 600
   tacgcctcat ccctgcaccc aggggcactg gcatcgtctc cgcacctgtg cctaagaagc 660
   tgctcatgat ggctggtatc gatgactgct acacctcagc ccggggctgc actgccaccc 720
   tgggcaactt cgccaaggcc acctttgatg ccatttctaa gacctacagc tacctgaccc 780
   ccgacctctg gaaggagact gtattcacca agtctcccta tcaggagttc actgaccacc 840
   tcgtcaagac ccacaccaga gtctccgtgc agcggactca ggctccagct gtggctacaa 900
   catagggttt ttatacaaga aaaataaagt gaattaagcg tgaaaaaaaa aaaaaaaaaa 960
   aa                                                                962
<210> 5
   <211> 1196
   <212> DNA
   <213> Homo sapiens
<220>
   <223> 60S ribosomal protein L13a, L13A, transcript variant 1, tissue specific transplantation antigen 1 (TSTA1), 23 kDa highly basic protein
<220>
   <221> CDS
   <222> (77) ... (688)
   <223> RPL13A
<400> 5
   cacttctgcc gcccctgttt caagggataa gaaaccctgc gacaaaacct cctccttttc 60
   caagcggctg ccgaagatgg cggaggtgca ggtcctggtg cttgatggtc gaggccatct 120
   cctgggccgc ctggcggcca tcgtggctaa acaggtactg ctgggccgga aggtggtggt 180
   cgtacgctgt gaaggcatca acatttctgg caatttctac agaaacaagt tgaagtacct 240
   ggctttcctc cgcaagcgga tgaacaccaa cccttcccga ggcccctacc acttccgggc 300
   ccccagccgc atcttctggc ggaccgtgcg aggtatgctg ccccacaaaa ccaagcgagg 360
   ccaggccgct ctggaccgtc tcaaggtgtt tgacggcatc ccaccgccct acgacaagaa 420
   aaagcggatg gtggttcctg ctgccctcaa ggtcgtgcgt ctgaagccta caagaaagtt 480
   tgcctatctg gggcgcctgg ctcacgaggt tggctggaag taccaggcag tgacagccac 540
   cctggaggag aagaggaaag agaaagccaa gatccactac cggaagaaga aacagctcat 600
   gaggctacgg aaacaggccg agaagaacgt ggagaagaaa attgacaaat acacagaggt 660
   cctcaagacc cacggactcc tggtctgagc ccaataaaga ctgttaattc ctcatgcgtt 720
   gcctgccctt cctccattgt tgccctggaa tgtacgggac ccaggggcag cagcagtcca 780
   ggtgccacag gcagccctgg gacataggaa gctgggagca aggaaagggt cttagtcact 840
   gcctcccgaa gttgcttgaa agcactcgga gaattgtgca ggtgtcattt atctatgacc 900
   aataggaaga gcaaccagtt actatgagtg aaagggagcc agaagactga ttggagggcc 960
   ctatcttgtg agtggggcat ctgttggact ttccacctgg tcatatactc tgcagctgtt 1020
   agaatgtgca agcacttggg gacagcatga gcttgctgtt gtacacaggg tatttctaga 1080
   agcagaaata gactgggaag atgcacaacc aaggggttac aggcatcgcc catgctcctc 1140
   acctgtattt tgtaatcaga aataaattgc ttttaaagaa aaaaaaaaaa aaaaaa     1196
<210> 6
   <211> 671
   <212> DNA
   <213> Homo sapiens
<220>
   <223> 60S ribosomal protein L18a, L18A, ribosomal protein L18a-like protein
<220>
   <221> CDS
   <222> (80) ... (610)
   <223> RPL18A
<400> 6
   ggtagtgaag gcctggtgaa cggctgcgcg acagaggaca cttccttttg cgggtggcgg 60
   cgaacgcgga gagcacgcca tgaaggcctc gggcacgcta cgagagtaca aggtagtggg 120
   tcgctgcctg cccaccccca aatgccacac gccgcccctc taccgcatgc gaatctttgc 180
   gcctaatcat gtcgtcgcca agtcccgctt ctggtacttt gtatctcagt taaagaagat 240
   gaagaagtct tcaggggaga ttgtctactg tgggcaggtg tttgagaagt cccccctgcg 300
   ggtgaagaac ttcgggatct ggctgcgcta tgactcccgg agcggcaccc acaacatgta 360
   ccgggaatac cgggacctga ccaccgcagg cgctgtcacc cagtgctacc gagacatggg 420
   tgcccggcac cgcgcccgag cccactccat tcagatcatg aaggtggagg agatcgcggc 480
   cagcaagtgc cgccggccgg ctgtcaagca gttccacgac tccaagatca agttcccgct 540
   gccccaccgg gtcctgcgcc gtcagcacaa gccacgcttc accaccaaga ggcccaacac 600
   cttcttctag gtgcagggcc ctcgtccggg tgtgccccaa ataaactcag gaacgccccg 660
   gtgctcgccg c                                                      671
<210> 7
   <211> 3528
   <212> DNA
   <213> Homo sapiens
<220>
   <223> eukaryotic translation elongation factor 1 alpha 1 (EEF1A1, EE1A1, EEF-1, EEF1A), cervical cancer suppressor 3 (CCS3), leukocyte receptor cluster member 7, prostate tumor-inducing protein 1 (PTI1), glucocorticoid receptor AF-1 specific elongation factor (GRAF-1EF)
<220>
   <221> CDS
   <222> (64) ... (1452)
   <223> EEF1A1
<400> 7
   ctttttcgca acgggtttgc cgccagaaca caggtgtcgt gaaaactacc cctaaaagcc 60
   aaaatgggaa aggaaaagac tcatatcaac attgtcgtca ttggacacgt agattcgggc 120
   aagtccacca ctactggcca tctgatctat aaatgcggtg gcatcgacaa aagaaccatt 180
   gaaaaatttg agaaggaggc tgctgagatg ggaaagggct ccttcaagta tgcctgggtc 240
   ttggataaac tgaaagctga gcgtgaacgt ggtatcacca ttgatatctc cttgtggaaa 300
   tttgagacca gcaagtacta tgtgactatc attgatgccc caggacacag agactttatc 360
   aaaaacatga ttacagggac atctcaggct gactgtgctg tcctgattgt tgctgctggt 420
   gttggtgaat ttgaagctgg tatctccaag aatgggcaga cccgagagca tgcccttctg 480
   gcttacacac tgggtgtgaa acaactaatt gtcggtgtta acaaaatgga ttccactgag 540
   ccaccctaca gccagaagag atatgaggaa attgttaagg aagtcagcac ttacattaag 600
   aaaattggct acaaccccga cacagtagca tttgtgccaa tttctggttg gaatggtgac 660
   aacatgctgg agccaagtgc taacatgcct tggttcaagg gatggaaagt cacccgtaag 720
   gatggcaatg ccagtggaac cacgctgctt gaggctctgg actgcatcct accaccaact 780
   cgtccaactg acaagccctt gcgcctgcct ctccaggatg tctacaaaat tggtggtatt 840
   ggtactgttc ctgttggccg agtggagact ggtgttctca aacccggtat ggtggtcacc 900
   tttgctccag tcaacgttac aacggaagta aaatctgtcg aaatgcacca tgaagctttg 960
   agtgaagctc ttcctgggga caatgtgggc ttcaatgtca agaatgtgtc tgtcaaggat 1020
   gttcgtcgtg gcaacgttgc tggtgacagc aaaaatgacc caccaatgga agcagctggc 1080
   ttcactgctc aggtgattat cctgaaccat ccaggccaaa taagcgccgg ctatgcccct 1140
   gtattggatt gccacacggc tcacattgca tgcaagtttg ctgagctgaa ggaaaagatt 1200
   gatcgccgtt ctggtaaaaa gctggaagat ggccctaaat tcttgaagtc tggtgatgct 1260
   gccattgttg atatggttcc tggcaagccc atgtgtgttg agagcttctc agactatcca 1320
   cctttgggtc gctttgctgt tcgtgatatg agacagacag ttgcggtggg tgtcatcaaa 1380
   gcagtggaca agaaggctgc tggagctggc aaggtcacca agtctgccca gaaagctcag 1440
   aaggctaaat gaatattatc cctaatacct gccaccccac tcttaatcag tggtggaaga 1500
   acggtctcag aactgtttgt ttcaattggc catttaagtt tagtagtaaa agactggtta 1560
   atgataacaa tgcatcgtaa aaccttcaga aggaaaggag aatgttttgt ggaccacttt 1620
   ggttttcttt tttgcgtgtg gcagttttaa gttattagtt tttaaaatca gtacttttta 1680
   atggaaacaa cttgaccaaa aatttgtcac agaattttga gacccattaa aaaagttaaa 1740
   tgagaaacct gtgtgttcct ttggtcaaca ccgagacatt taggtgaaag acatctaatt 1800
   ctggttttac gaatctggaa acttcttgaa aatgtaattc ttgagttaac acttctgggt 1860
   ggagaatagg gttgttttcc ccccacataa ttggaagggg aaggaatatc atttaaagct 1920
   atgggagggt tgctttgatt acaacactgg agagaaatgc agcatgttgc tgattgcctg 1980
   tcactaaaac aggccaaaaa ctgagtcctt gtgttgcata gaaagcttca tgttgctaaa 2040
   ccaatgttaa gtgaatcttt ggaaacaaaa tgtttccaaa ttactgggat gtgcatgttg 2100
   aaacgtgggt taaaatgact gggcagtgaa agttgactat ttgccatgac ataagaaata 2160
   agtgtagtgg ctagtgtaca ccctatgagt ggaagggtcc attttgaagt cagtggagta 2220
   agctttatgc cagtttgatg gtttcacaag ttctattgag tgctattcag aataggaaca 2280
   aggttctaat agaaaaagat ggcaatttga agtagctata aaattagact aatctacatt 2340
   gcttttctcc tgcagagtct aatacctttt atgctttgat aattagcagt ttgtctactt 2400
   ggtcactagg aatgaaacta catggtaata ggcttaacag gtgtaatagc ccacttactc 2460
   ctgaatcttt aagcatttgt gcatttgaaa aatgcttttc gcgatcttcc tgctgggatt 2520
   acaggcatga gccactgtgc ctgacctccc atatgtaaaa gtgtctaaag gttttttttt 2580
   ggttataaaa ggaaaatttt tgcttaagtt tgaaggatag gtaaaattaa aggacatgct 2640
   ttctgtttgt gtgatggttt ttaaaaattt tttttaagat ggagttcttg ttgcccaggc 2700
   tagaatgcaa tggcaaaatc tcactgcaat ctcctcctcc tgggttcaag caattctcct 2760
   acttcagcct cccaagtagc tgggattaca ggcatgtgct aatttggtgt ttttaataga 2820
   gatgaggttt ttccatgttg gtcaggctgg tctcaaactc ctgaccttag gtgatcgcct 2880
   cggcctccta aagtgctgga attacaggca tgagccacca tgcctggcca ggacatgtgt 2940
   tcttaaggac atgctaagca ggagttaaag cagcccaaga gataaggcct cttaaagtga 3000
   ctggcaatgt gtattgctca agattcaaag gtacttgaat tggccataga caagtctgta 3060
   atgaagtgtt atcgttttcc ctcatctgag tctgaattag ataaaatgcc ttcccatcag 3120
   ccagtgctct gaggtatcaa gtctaaattg aactagagat ttttgtcctt agtttctttg 3180
   ctatctaatg tttacacaag taaatagtct aagatttgct ggatgacaga aaaaacaggt 3240
   aaggccttta atagatggcc aatagatgcc ctgataatga aagttgacac ctgtaagatt 3300
   taccagtaga gaattcttga catgcaagga agcaagattt aactgaaaaa ttgttcccac 3360
   tggaagcagg aatgagtcag tttacttgca tatactgaga ttgagattaa cttcctgtga 3420
   aacccagtgt cttagacaac tgtggcttga gcaccacctg ctggtattca ttacaaactt 3480
   gctcactaca ataaatgaat tttaagcttt aaaaaaaaaa aaaaaaaa          3528
<210> 8
   <211> 4439
   <212> DNA
   <213> Homo sapiens
<220>
   <223> 60S ribosomal protein L28 transcript variant 1, L28
<220>
   <221> CDS
   <222> (43) ... (534)
   <223> RPL28
<400> 8
   ctctttccgt ctcaggtcgc cgctgcgaag ggagccgccg ccatgtctgc gcatctgcaa 60
   tggatggtcg tgcggaactg ctccagtttc ctgatcaaga ggaataagca gacctacagc 120
   actgagccca ataacttgaa ggcccgcaat tccttccgct acaacggact gattcaccgc 180
   aagactgtgg gcgtggagcc ggcagccgac ggcaaaggtg tcgtggtggt cattaagcgg 240
   agatccggcc agcggaagcc tgccacctcc tatgtgcgga ccaccatcaa caagaatgct 300
   cgcgccacgc tcagcagcat cagacacatg atccgcaaga acaagtaccg ccccgacctg 360
   cgcatggtga gctggggttt ggggatcagg cttggggaga ctggccagtg ctgtggggaa 420
   gggcctccca ctactggttg caatatgggc tggagaggga tggattcttg ctttcagcct 480
   actccccaca cccagcattg gcctaggggg cggcttgtgg agtgtatggg ctgagccttg 540
   ctctgctccc ccgcccccag gcagccatcc gcagggccag cgccatcctg cgcagccaga 600
   agcctgtgat ggtgaagagg aagcggaccc gccccaccaa gagctcctga gccccctgcc 660
   cccagagcaa taaagtcagc tggctttctc acctgcctcg actgggcctc cctttttgaa 720
   acgctctggg gagctctggc cctgtgtgtt gtcattcagg ccatgtcatc aaaactctgc 780
   atgtcacctt gtccatctgg aggtgatgtc aatggctggc catgcaggag gggtggggta 840
   gctgccttgt ccctggtgag ggcaagggtc actgtcttca cagaaaaagt ttgctgactt 900
   gtgattgaga cctactgtcc cattgtgagg tggcctgaag aatcccagct ggggcagtgg 960
   cttccattca gaagaagaaa ggccttttct agcccagaag ggtgcaggct gagggctggg 1020
   ccctgggccc tggtgctgta gcacggtttg gggacttggg gtgttcccaa gacctggggg 1080
   acgacagaca tcacgggagg aagatgagat gacttttgca tccagggagt gggtgcagcc 1140
   acatttggag gggatgggct ttacttgatg caacctcatc tctgagatgg gcaacttggt 1200
   gggtggtggc ttataactgt aagggagatg gcagccccag ggtacagcca gcaggcattg 1260
   agcagcctta gcattgtccc cctactcccg tcctccaggt gtccccatcc ctcccctgtc 1320
   tctttgagct ggctcttgtc acttaggtct catctcagtg gccgctcctg ggccaccctg 1380
   tcacccaagc tttcctgatt gcccagccct cttgtttcct ttggcctgtt tgctccctag 1440
   tgtttattac agcttgtgag gccaggagtt tgagaccatc ctaggcaaca taatgagaca 1500
   ccgtctctaa aataaaatta gctgggtgtg gtggtgcacc gcctgtggtc ccagctcctc 1560
   agaggttgag tagaggctga ggtgagcgga gcacttgagc caagagtatg aggctgcagt 1620
   gagcccatga gccccaccac tacactccag cctggaagac accatgacac acagtgaggc 1680
   ctggatgggg aaagagtcct gctgttgatc ctcacatgtt tcctgggcac ctaactctgt 1740
   cagccactgc cagggaccaa ggatccagca tccatggcac ccctggttcc tgccatcctg 1800
   gggtacccga ttcaaagaag gactctgctc cctgtctgag accacccccg gctctgactg 1860
   agagtaaggg gactgtcagg gcctcgactt gccattggtt ggggtcgtac ggggctggga 1920
   gccctgcgtt ttgaggcaga ccactgccct tccgacctca gtcctgtctg ctccagtctt 1980
   gcccagctcg aaggagagca gatctgacca cttgccagcc cctgtctgct gtgaattacc 2040
   atttcctttg tccttccctt agttgggtct attagctcag attgagaggt gttgccttaa 2100
   aactgagttg ggtgacttgg tacctgctca ggaccccccg cactgtccca atcccactca 2160
   ggcccacctc cagctggcct cactccgctg gtgacttcgt acctgctcag gagcccccac 2220
   tgtcccagtc ccactcaggc ccatctctgg ctggcctcac tgcgctggga ctccgccttc 2280
   ataaggagag ctcactgctc acgttagtag atggcccctt ctcgtgaggc ctctcccctg 2340
   gcacctgctt cagttgtcct ccacagcact gatttgcagc ccacaagctg gcaggtttat 2400
   ctgtctcatg tttgtcttgt gctggtgggc aaggggtttg tctagcacac cagcatataa 2460
   tgagatgctt gatgaatggt gcatattgaa tgtataaagc ccaccggtcc tgagagtttg 2520
   ctcactggag actttctgga gatggagtct cgctctgttg cccaggctgg cgagtgcaat 2580
   ggcgcgatct tggctcactg cagcctccac ctcctgggtt caagcgattc tcctgcctca 2640
   gcctcccgag tagctgggat tacaggtggg tgtcaccaca cccagctcag tattgtattt 2700
   ttagcagaga tggggtttca ccattttgcc caggctggtt tggaactcct gacttcaaat 2760
   tacccacctg cctcagcctc ccaaagtgct ggcattacag gcgctcgagg ctttctgatg 2820
   tggctgctgc tgctcagaag gccttgtcct taaccacctc cttgcctgcc ctggaggctt 2880
   gtgcctctag gccccacccc ctgtggagtc ctgctggctt tctccatccc tatctgaatc 2940
   ctccctgctg tgtggcctcc cctggtctca tccgtaacac agcccagctt agtgggcctc 3000
   tgttcctgcg ggtggccagc ctgtctgtgt ggctgggctg gggaggccac gtctggtatc 3060
   tgaatgctat cggtgggttg gggtggagga accaggagag ggctggaggg agggagatgg 3120
   tctcagcccc acagagtttg gagtcctcag tgtgctgagc aaacgtggag acaccatttc 3180
   cctcctctag acctcatctt ggagagagag atgttggatg gggccatcta ttccagcttt 3240
   attcacacaa atcatgtctg ttggcctgga aattggaaaa ccagttaaac caaaaacatg 3300
   atattaagaa aacaggcagg ctcaccatag taaaaatgct gaaagccaaa gacaaaattg 3360
   ggagaacaaa agaaaagcgt cttgtcacat acagaaggtc cctgataaag ttagtagctg 3420
   ccctcatcag aaaccaggcc caggcagtgg ggacacatcc agagtgctga aagaacctcc 3480
   cccaggtcat cctatcccca agagtgatgc ccggcagcat tcccagctca gggctaatgg 3540
   ttcacggaag ccaggaatca aactgcctgg gttccagtcc cagctctgcc agttatgccc 3600
   agctgtgggg acttgggcag ctcgtttagt agcaccgtgc ctcagtttcc catatgtaaa 3660
   aggccatttt gagtgccttt cacagccctg cataaggcag gtgtctcagt gttcactgct 3720
   gtctctccag ctcttagtcc agtagctgca tggtgagtga gcgtagggcg caccctggaa 3780
   ggctgccaag cccaaagttg tgcagagcgc tggggactcc agactcccca cagcagcaga 3840
   gactcgggac tgaggcatcc tctgttcaca ggacatgctg gcatctactg ggtcagggct 3900
   ctgctgctcg gtggctgtgc aaccttgggc aagttcctca acctctctgt gtcttcgtac 3960
   cctcatctgt aacatgcgtg tcgatagacc ctactactca gggttgatga gaagattaaa 4020
   tgtgcaaaac ctgcttgact gtgcccacaa atcctgattg taggaataaa ttaatgactt 4080
   tttataaata ttttgatcag atggactcat gatcacagat gtcttcacat gcctatgact 4140
   aatttgtaca caaactaatg ctcgtgtttc ccaagcacct ggaagacatg ccagatccat 4200
   gtgcagtaat gcctggtggc tccaggtctg ccccgccgtc ctgtggggct gtgagctttc 4260
   ccagcctcct gcccgtgttt gtgaatatca ttctgtcctc agctgcattt ccagcccagg 4320
   ctgtttggcg ctgcccagga atggtatcaa ttcccctgtt tctcttgtag ccagttacta 4380
   gaataaaatc atctacttta aaaaaaaaaa aaaaaaaaaa aaaaaaaaaa aaaaaaaaa  4439
<210> 9
   <211> 401
   <212> DNA
   <213> Homo sapiens
<220>
   <223> 40S ribosomal protein S28, S28
<220>
   <221> CDS
   <222> (32) ... (241)
   <223> RPS28
<400> 9
   ctctccgcca gaccgccgcc gcgccgccat catggacacc agccgtgtgc agcctatcaa 60
   gctggccagg gtcaccaagg tcctgggcag gaccggttct cagggacagt gcacgcaggt 120
   gcgcgtggaa ttcatggacg acacgagccg atccatcatc cgcaatgtaa aaggccccgt 180
   gcgcgagggc gacgtgctca cccttttgga gtcagagcga gaagcccgga ggttgcgctg 240
   agcttggctg ctcgctgggt cttggatgtc gggttcgacc acttggccga tgggaatggt 300
   ctgtcacagt ctgctccttt tttttgtccg ccacacgtaa ctgagatgct cctttaaata 360
   aagcgtttgt gtttcaagtt aaaaaaaaaa aaaaaaaaaa a                     401
<210> 10
   <211> 361
   <212> DNA
   <213> Homo sapiens
<220>
   <223> 40S ribosomal protein S27, S27, metallopan-stimulin 1, metallopanstimulin 1 (MPS-1, MPS1)
<220>
   <221> CDS
   <222> (35) ... (289)
   <223> RPS27
<400> 10
   ctttccggcg gtgacgacct acgcacacga gaacatgcct ctcgcaaagg atctccttca 60
   tccctctcca gaagaggaga agaggaaaca caagaagaaa cgcctggtgc agagccccaa 120
   ttcctacttc atggatgtga aatgcccagg atgctataaa atcaccacgg tctttagcca 180
   tgcacaaacg gtagttttgt gtgttggctg ctccactgtc ctctgccagc ctacaggagg 240
   aaaagcaagg cttacagaag gatgttcctt caggaggaag cagcactaaa agcactctga 300
   gtcaagatga gtgggaaacc atctcaataa acacattttg gataaatcct gaaaaaaaaa 360
   a                                                                 361
<210> 11
   <211> 918
   <212> DNA
   <213> Homo sapiens
<220>
   <223> 60S ribosomal protein L34 transcript variant 1, L34, leukemia-associated protein
<220>
   <221> CDS
   <222> (45) ... (398)
   <223> RPL34
<400> 11
   cttttttctt cctcttccgg ggacgttgtc tgcaggcact cagaatggtc cagcgtttga 60
   cataccgacg taggctttcc tacaatacag cctctaacaa aactaggctg tcccgaaccc 120
   ctggtaatag aattgtttac ctttatacca agaaggttgg gaaagcacca aaatctgcat 180
   gtggtgtgtg cccaggcaga cttcgagggg ttcgtgctgt aagacctaaa gttcttatga 240
   gattgtccaa aacaaagaaa catgtcagca gggcctatgg tggttccatg tgtgctaaat 300
   gtgttcgtga caggatcaag cgtgctttcc ttatcgagga gcagaaaatc gttgtgaaag 360
   tgttgaaggc acaagcacag agtcagaaag ctaaataaaa aaatgaaact tttttgagta 420
   ataaaaatga aaagacgctg tccaatagaa aaagttggtg tgctggagct acctcacctc 480
   agcttgagag agccagttgt gtgcatctct ttccagtttt gcatccagtg acgtctgctt 540
   ggcatcttga gattgttatg gtgagagtat ttacacctca gcaaatgctg caaaatcctg 600
   ttttccccca gagagctgga ggttaaatac taccagcaca tccctagata ctactcaagt 660
   tacagtatat gatcactaat atagtatgct cttggtacca ggagctctga tatatatctg 720
   gtacatgttt gataatgact tgattgttat tataagtact tattaatact tcgattctgt 780
   aaagagttta gggtttgatt ttataaaatc caaaatgagc cttttattga atccagttct 840
   ctatgtgacc agttctctgt atgaatggaa gggaaaagaa ttaaaaatct tgcaaagggg 900
   aaaaaaaaaa aaaaaaaa                                               918
<210> 12
   <211> 4906
   <212> DNA
   <213> Homo sapiens
<220>
   <223> 60S ribosomal protein L27a, L27A
<220>
   <221> CDS
   <222> (367) ... (813)
   <223> RPL27A
<400> 12
   aacgaggccc ttagggtcgg cttaggcggt tccctgacca aggcgccaga aaagggcctg 60
   gctcaagcaa gcacgggcgg cgtgcagtac agcacaccta gccccgattc ttcaacagtt 120
   ctcgccctcc gagcctagca caacgagcct caccgaaacc gtacaccgcc accaggacac 180
   tccgtgatgg gggatcacca ccctcagaaa gaggaagcga ctagcaggcg cgcaatcccg 240
   cgagaccagg aggccccgcc cgaagcccgg cctctgtgac cggaagtgag gcgttttgcc 300
   ccgcccccgt ggccgatacc tcgcgagact tggcgaaggc cttccttttt cgtctgggct 360
   gccaacatgc catccagact gaggaagacc cggaaactta ggggccacgt gagccacggc 420
   cacggccgca taggcaagca ccggaagcac cccggcggcc gcggtaatgc tggtggtctg 480
   catcaccacc ggatcaactt cgacaaatac cacccaggct actttgggaa agttggtatg 540
   aagcattacc acttaaagag gaaccagagc ttctgcccaa ctgtcaacct tgacaaattg 600
   tggactttgg tcagtgaaca gacacgggtg aatgctgcta aaaacaagac tggggctgct 660
   cccatcattg atgtggtgcg atcgggctac tacaaagttc tgggaaaggg aaagctccca 720
   aagcagcctg tcatcgtgaa ggccaaattc ttcagcagaa gagctgagga gaagattaag 780
   agtgttgggg gggcctgtgt cctggtggct tgaagccaca tggagggagt ttcattaaat 840
   gctaactact ttttccttgt ggtgtgagtg taggttcttc agtggcacct ctacatcctg 900
   tgtgcattgg gagcccaggt tctagtactt agggtatgaa gacatggggt cctctcctga 960
   cttccctcaa atatatggta aacgtaagac caacacagac gttggccagt taaacatttc 1020
   tgtttataaa gtcagaataa tacctgttga tcactgaaag gcctgcatgt attgtactct 1080
   gaattttaca gtgaatgaga gaatgtaccc taattgttca acagggctca aaaggaaaga 1140
   ttccattttg atgggtcaca ttctaaagag gggcagtgtg ataggaatga gatggtcctt 1200
   taggacttaa gttctcagcc caaggttttt ccacgtggcc ccctcatctt tttttttttt 1260
   ttaaacggag tctctcttgc caggctggag tgcagtggca cgatctcggc tcactgcagc 1320
   ctccgcctcc caggttaagc gattctcctg cctcagcttc ctgactaact gggattacag 1380
   gcgcccacca ccatgcccag ctaatttttg tattttcagt agagatgggg tttcaccatg 1440
   ttggccatgc tggtctctaa ctcctaacct caagtgatct gcccacatcg gcctccaaaa 1500
   gttctgggat tatagtgtga gccactgcgc ccggccatgg ctccttaatc ttgatccaaa 1560
   ttattgttac atccagaatg tgatgaatca aaatctcgag atgggggtcc agcaatctga 1620
   aatttcagta tgccagggct tttctgtatg tcaaagtggg tttgaaatag ttaatttttc 1680
   ttctagtctg aaatgtatcg ggaaaatttg gaaatcctga aggctggaaa ttgaaataag 1740
   tttttctagg atttgtgtct cttgctattg gaaaactgat ggtgaccaat tcatgtttac 1800
   aaataagatc ctcatagatc tcggtaaatt ataatttgct acagttttat ggttcttcct 1860
   gtgattttga gctttttttg acccaaaata atacagtcta aaactataga caaataagat 1920
   ggcacttaga ctcctgggtt ttagttagtg gaggtttcct tagtgcactg tggggtcata 1980
   ataagccgag aaccatggct gtctatggga cacatctgtc aggacaacct ttagaggatg 2040
   ttggggatca aatagaaggc acagagaagc actgaattgg cttacataag aataggctag 2100
   aattacaagt agtgaaacct cgattcagct ggacaatttt aaacaaatgt atcatttggc 2160
   ttgtatcttc tgttgtgctg gagaagttag aaataagggc tctccagacc agcctgacca 2220
   acctggagaa accttgtctc tactaaatac acaaaattag ccaggcgtgg tggcacatgc 2280
   ctgtaatccc agctactttg gaggctgagc caggagaatc tccaggaggc ggaggttgct 2340
   gtgagccgag atcgtgccat tgcactccag cttgggcaac aagagtgaaa ctctgtccac 2400
   cccccccaaa aaaagtaagg gctctccatt agggcccata gaggacttgt aatatggaac 2460
   ctgaatccaa ggatcccaca ataagtggtc agtagttcat gatgaattaa aagactcaat 2520
   atttggtctt cacccaatac ctgtgtgact tttagtccta atttcctcat ctttaaaatt 2580
   tcagtgaaag tgcctacctg aggattgtgt agattaaaat ggaaaccgtg cacttaattt 2640
   tttgttttgt tttgagacgg agtctcgctc tgtcgcccag gctggagtgc agtggtgcga 2700
   tctcagatca ctgcaagctc cgcctcctag gttcagacca ttctcctgcc tcagcttccc 2760
   aagtagctgg gactacaggc gcccgccact gcgcccggct aattttttgc atttttagta 2820
   gagacagggt ttcaccgtgt tagccaggat ggtctcgatc tcctgatctg cccgcctcag 2880
   cctcccaaag tgctgggatt acaggcatga gccaccgcgc ccggcccagg cacttaattt 2940
   ttgtgtttga cttagtaact taagtgcaaa ctattacggg agcagatgga gtcaattggc 3000
   cttcatgtga ttgtcagtgg gaaattggtc caagcagagg gaatactggt tcaggaaact 3060
   ggtttgggaa ggttaggcaa acgggaagtg ctatggtgga gagaaagatt actctggccg 3120
   ggctgtaaag gacggctaca atgggaggct gaaggcagaa ccaagaaaat gggagtgagt 3180
   atggaaaagg tacgattcag acggcataat ggacgggact tggagactga attgtagtgg 3240
   gccgaccaca aaatgataag gcatggaagg aagtagagtt tggggggaag gatccctagt 3300
   cccttaatgg ctaccttctt ccccaggagt tgttaggcca tccgatcccc tggcctggga 3360
   aagaaacact gatttcgttg ctggcttgtt cactcaccag aagctacagc tactaacagt 3420
   tctaaaaact gtttcatgtg atgaggaaca gacgaaaata gttttgagcc ctaagtccgc 3480
   cgattccagt gctttcttga acccgcattt actaaaatat tttcatgact gccaagcttt 3540
   gaatagcctg ctgtgttcat ggaggctcat actggcgatc tctagtggct ggctaaagct 3600
   tgaattgcaa aagatctaat ttctggtcta atgtatatat gccttaaata tagttgcgtt 3660
   caaacgtggg agctgcaggt gcaacttgat tttatgacaa atggctgcca cataatttgc 3720
   acaagcagtg ctcgtcaagg gcagctaaat caggcgagct ttcaatcaaa ataaatgtac 3780
   tactaaaccc tacttagcgg ctaactagcc caagagcaga cagcccacgg acggactgca 3840
   agtcggaagc gcgggcggaa gctgtgcagc gcccacctgg tggctccatc ggccgcgttc 3900
   atcagtcagc acgacccgac ctcagtggcg tcctcacaac acagaccgga ccttgggtct 3960
   taccccggca cctgagaacc acttccggtg agtagcttct acttccggag acgatgactc 4020
   ccccgcgtcc cagaccggaa gaagcccggc ggagaccggc ctcgctcggc cacttccggc 4080
   aagggcggag ccggccagtg gtgcgcgagc gcagataact cccctggaga ggcgggatgt 4140
   tcaactccac ccctggtcct tgggcggccg tgggtcccct tcgaagcgga ggaatggcca 4200
   acctcgccgc acttcgagcc cctttagggt gcgtttaaga acagtgggcg tggcctttac 4260
   gtaaatcttc gagatgggaa cctccagaat ttgtctcaat tgtctaaaag gtaatgagcg 4320
   tcagcgacat tcaagggcac tttgggctaa aaaagaaagt gcttgtacac ggatggaaat 4380
   attctagaag aacataaaag gaatttcctc ttaggaggtt agggaaatga gcacgaagta 4440
   tgttttggtg cagttttttg ttcaacccaa tgcgtatttt catattgaga ggcaatataa 4500
   atggagcgaa agtatcttga gaaaaaaaaa aaaactacca gaacttgccg ttgctgaaaa 4560
   gtaatatttt ctctttcgag agttttcatg gccttttaaa ttacaccccc acctccacag 4620
   gcaaataaat ttgttttgga atgcatacca catcatctgg ctctagaaac gtattttgtg 4680
   tagctcccct agcaagaata taggttaaag cgtaaattta attcctggct ctattttaca 4740
   tcccaatttt tattttcctc tcattcccac tttacgttgt ttcaaataac ctagtttgtg 4800
   tatccctgta agtcattttg gtataaagta ggttataagt gtacatgcga aaagatgttt 4860
   ttaacaaaaa tgtaactgaa aaaaaaaaaa aaaaaaaaaa aaaaaa                4906
<210> 13
   <211> 2335
   <212> DNA
   <213> Homo sapiens
<220>
   <223> 60S ribosomal protein L10 transcript variant 1, L10, laminin receptor homolog, tumor suppressor QM (QM), Wilms tumor-related protein, AUTSX5, DXS648, DXS648E, NOV
<220>
   <221> CDS
   <222> (189) ... (833)
   <223> RPL10
<400> 13
   gggctacgcc cgggcgcaag cgccaagagc ggctgcgtct atggtcatga cgtctgacag 60
   agcgtccacc cgtcttcgac aggactctat ggttcttacg cgcgcagaca gaccgcctat 120
   ataagccatg cgcaggcgga ggagcgcctc tttcccttcg gtgtgccact gaagatcctg 180
   gtgtcgccat gggccgccgc cccgcccgtt gttaccggta ttgtaagaac aagccgtacc 240
   caaagtctcg cttctgccga ggtgtccctg atgccaagat tcgcattttt gacctggggc 300
   ggaaaaaggc aaaagtggat gagtttccgc tttgtggcca catggtgtca gatgaatatg 360
   agcagctgtc ctctgaagcc ctggaggctg cccgaatttg tgccaataag tacatggtaa 420
   aaagttgtgg caaagatggc ttccatatcc gggtgcggct ccaccccttc cacgtcatcc 480
   gcatcaacaa gatgttgtcc tgtgctgggg ctgacaggct ccaaacaggc atgcgaggtg 540
   cctttggaaa gccccagggc actgtggcca gggttcacat tggccaagtt atcatgtcca 600
   tccgcaccaa gctgcagaac aaggagcatg tgattgaggc cctgcgcagg gccaagttca 660
   agtttcctgg ccgccagaag atccacatct caaagaagtg gggcttcacc aagttcaatg 720
   ctgatgaatt tgaagacatg gtggctgaaa agcggctcat cccagatggc tgtggggtca 780
   agtacatccc caatcgtggc cctctggaca agtggcgggc cctgcactca tgagggcttc 840
   caatgtgctg cccccctctt aatactcacc aataaattct acttcctgtc cacctatgtc 900
   tttgtatcta cattcttgac ggggaaggaa cttcctctgg gaacctttgg gtcattgccc 960
   tttcacttca gaaacaggtt gacaactcag ccctgctcat gaggcagcaa accctgcaaa 1020
   gggctgggac tggtggcctt atgtcagttg tctactctgg agcttgactt ggacctcccc 1080
   aggtcctagg cagtaggttg aaaaacactg aagtgctttt catgaagcac agctgcagca 1140
   aagccttgca atcccaggct ggggtcagcc tacagttgtg ttgcttatta caacacatgc 1200
   ggaccaagag gggcttgtgg gctagaggct gaccagcagc gtttatttag caagggtagg 1260
   tgtgcatcac attgggcttg ttctcaccca tctggtttgg ccattcctcc ttggtgggaa 1320
   tcatccaggt actgctgagg tcacctgcga tttgccccat ttcctatctc tagcaacctc 1380
   ctgggcccca tgcccccacc ccttctagaa cctgcattcc cagggccttc accacctgac 1440
   caaaggtcta ggctaacctt tggtcatttg taacaagacc tcggaacaga cacgtgtgtg 1500
   gcatggtttg gcctggggat cttagatgtc tgacctgaac tattgtagaa cagcgctggc 1560
   ttttggggga gcagcaaaaa tgagaggagt gctaggtggg tggcctgagc atctgtatcc 1620
   agggacagga ctccaaaggc ttttggtccc agagctgggg tatgttggcc ccagccccca 1680
   gcctgtggct cccaaaaggc ctctggtttt ttgtaatctc agtttacagc catttcttag 1740
   gtttttaatt acctttattt tattttgcca aacatacctg ggaatacctt ttattttttt 1800
   tttaccttgg ggtgatggtt ccaaaccata aatgtgatta tagttaacac atgacccttc 1860
   tagcgtccca gccagtgttt ttcctgacct ctgttctttg gagaggagga tggaagggag 1920
   gggtccggca cgctgctggc attttgctgt gtcctgcagc ccctttccgg gacacctggg 1980
   ttcacacagc tttttagctt acataactgg tgcagatttt ctgtgtggag atgttgcctt 2040
   gaccagcctt ggctggactt taccaggcat gcagaagcct gtaccaacac agactacagc 2100
   acccaggagg tgcgagtgtg gctgctcagc ggttataaca ggcctgactg cattgttcac 2160
   cggattataa tgagccaaaa tgtttcccgg tgtttgctgg tttcagggaa ggagtttgat 2220
   atagcagatt aaccaccctc cttgtagcta ttggggctta atggtttcct ggtgattctt 2280
   accaatccac aataaacatg gcccattggc atatctgcaa aaaaaaaaaa aaaaa      2335
<210> 14
   <211> 2381
   <212> DNA
   <213> Homo sapiens
<220>
   <223> eukaryotic translation elongation factor 1 delta (guanine nucleotide exchange protein) (EEF1D, EF-1D, EF1D, EF-1-delta) transcript variant 3, antigen NY-CO-4, FP1047
<220>
   <221> CDS
   <222> (343) ... (2286)
   <223> EEF1D
<400> 14
<210> 15
   <211> 1523
   <212> DNA
   <213> Homo sapiens
<220>
   <223> glioma tumor suppressor candidate region gene 2 (GLTSCR2), protein interacting with carboxyl terminus 1 (PICT-1, PICT1), p60
<220>
   <221> CDS
   <222> (25) ... (1461)
   <223> GLTSCR2
<400> 15
   tggctgagtt cttcctttga caagatggcg gcaggaggca gtggcgttgg tgggaagcgc 60
   agctcgaaaa gcgatgccga ttctggtttc ctggggctgc ggcccacttc ggtggaccca 120
   gcgctgaggc ggcggcggcg aggcccaaga aataagaagc ggggctggcg gcggcttgct 180
   caggagccgc tggggctgga ggttgaccag ttcctggaag acgtgcggct acaggagcgc 240
   acgagcggtg gcttgttgtc agaggcccca aatgaaaaac tcttcttcgt ggacactggc 300
   tccaaggaaa aagggctgac aaagaagaga accaaagtcc agaagaagtc actgcttctc 360
   aagaaacccc ttcgggttga cctcatcctc gagaacacat ccaaagtccc tgcccccaaa 420
   gacgtcctcg cccaccaggt ccccaacgcc aagaagctca ggcggaagga gcagctatgg 480
   gagaagctgg ccaagcaggg cgagctgccc cgggaggtgc gcagggccca ggcccggctc 540
   ctcaaccctt ctgcaacaag ggccaagccc gggccccagg acaccgtaga gcggcccttc 600
   tacgacctct gggcctcaga caaccccctg gacaggccgt tggttggcca ggatgagttt 660
   ttcctggagc agaccaagaa gaaaggagtg aagcggccag cacgcctgca caccaagccg 720
   tcccaggcac ccgccgtgga ggtggcgcct gccggagctt cctacaatcc atcctttgaa 780
   gaccaccaga ccctgctctc agcggcccac gaggtggagt tgcagcggca gaaggaggcg 840
   gagaagctgg agcggcagct ggccctgccc gccacggagc aggccgccac ccaggagtcc 900
   acattccagg agctgtgcga ggggctgctg gaggagtcgg atggtgaggg ggagccaggc 960
   cagggcgagg ggccggaggc tggggatgcc gaggtctgtc ccacgcccgc ccgcctggcc 1020
   accacagaga agaagacgga gcagcagcgg cggcgggaga aggctgtgca caggctgcgg 1080
   gtacagcagg ccgcgttgcg ggccgcccgg ctccggcacc aggagctgtt ccggctgcgc 1140
   gggatcaagg cccaggtggc cctgaggctg gcggagctgg cgcggcggca gaggcggcgg 1200
   caggcgcggc gggaggctga ggctgacaag ccccgaaggc tggggcggct caagtaccag 1260
   gcacctgaca tcgacgtgca gctgagctcg gagctgacag actcgctcag gaccctgaag 1320
   cccgagggca acatccttcg agaccggttc aagagcttcc agaggaggaa tatgatcgag 1380
   cctcgagaga gagccaagtt caaacgcaag tacaaggtga agctggtgga gaagcgggcg 1440
   ttccgtgaga tccagttgta gctgccatca gatgccggag actcgccctt caataaaaaa 1500
   tctcttctag ctgatcagtg gga                                         1523
<210> 16
   <211> 1348
   <212> DNA
   <213> Homo sapiens
<220>
   <223> 60S ribosomal protein L3 transcript variant 1, L3, HIV-1 TAR RNA-binding protein B (TARBP-B), ASC-1
<220>
   <221> CDS
   <222> (69) ... (1280)
   <223> RPL3
<400> 16
   ccagatttgg ctttatatag cggacccgta aggccgaccg gcctctaccg gcgggatttg 60
   atggcgtgat gtctcacaga aagttctccg ctcccagaca tgggtccctc ggcttcctgc 120
   ctcggaagcg cagcagcagg catcgtggga aggtgaagag cttccctaag gatgacccgt 180
   ccaagccggt ccacctcaca gccttcctgg gatacaaggc tggcatgact cacatcgtgc 240
   gggaagtcga caggccggga tccaaggtga acaagaagga ggtggtggag gctgtgacca 300
   ttgtagagac accacccatg gtggttgtgg gcattgtggg ctacgtggaa acccctcgag 360
   gcctccggac cttcaagact gtctttgctg agcacatcag tgatgaatgc aagaggcgtt 420
   tctataagaa ttggcataaa tctaagaaga aggcctttac caagtactgc aagaaatggc 480
   aggatgagga tggcaagaag cagctggaga aggacttcag cagcatgaag aagtactgcc 540
   aagtcatccg tgtcattgcc cacacccaga tgcgcctgct tcctctgcgc cagaagaagg 600
   cccacctgat ggagatccag gtgaacggag gcactgtggc cgagaagctg gactgggccc 660
   gcgagaggct tgagcagcag gtacctgtga accaagtgtt tgggcaggat gagatgatcg 720
   acgtcatcgg ggtgaccaag ggcaaaggct acaaaggggt caccagtcgt tggcacacca 780
   agaagctgcc ccgcaagacc caccgaggcc tgcgcaaggt ggcctgtatt ggggcatggc 840
   atcctgctcg tgtagccttc tctgtggcac gcgctgggca gaaaggctac catcaccgca 900
   ctgagatcaa caagaagatt tataagattg gccagggcta ccttatcaag gacggcaagc 960
   tgatcaagaa caatgcctcc actgactatg acctatctga caagagcatc aaccctctgg 1020
   gtggctttgt ccactatggt gaagtgacca atgactttgt catgctgaaa ggctgtgtgg 1080
   tgggaaccaa gaagcgggtg ctcaccctcc gcaagtcctt gctggtgcag acgaagcggc 1140
   gggctctgga gaagattgac cttaagttca ttgacaccac ctccaagttt ggccatggcc 1200
   gcttccagac catggaggag aagaaagcat tcatgggacc actgaagaaa gaccgaattg 1260
   caaaggaaga aggagcttaa tgccaggaac agattttgca gttggtgggg tctcaataaa 1320
   agttattttc cactgacaaa aaaaaaaa                                    1348
<210> 17
   <211> 2869
   <212> DNA
   <213> Homo sapiens
<220>
   <223> poly(A) binding protein, cytoplasmic 1 (PABPC1, PAB1, PABP1, PABPL1), poly(A) binding protein, cytoplasmic 2 (PABPC2), polyadenylate-binding protein 1 (PABP)
<220>
   <221> CDS
   <222> (505) ... (2415)
   <223> PABPC1
<400> 17
   cccttctccc cggcggttag tgctgagagt gcggagtgtg tgctccgggc tcggaacaca 60
   catttattat taaaaaatcc aaaaaaaatc taaaaaaatc ttttaaaaaa ccccaaaaaa 120
   atttacaaaa aatccgcgtc tcccccgccg gagactttta ttttttttct tcctctttta 180
   taaaataacc cggtgaagca gccgagaccg acccgcccgc ccgcggcccc gcagcagctc 240
   caagaaggaa ccaagagacc gaggccttcc cgctgcccgg acccgacacc gccaccctcg 300
   ctccccgccg gcagccggca gccagcggca gtggatcgac cccgttctgc ggccgttgag 360
   tagttttcaa ttccggttga tttttgtccc tctgcgcttg ctccccgctc ccctcccccc 420
   ggctccggcc cccagccccg gcactcgctc tcctcctctc acggaaaggt cgcggcctgt 480
   ggccctgcgg gcagccgtgc cgagatgaac cccagtgccc ccagctaccc catggcctcg 540
   ctctacgtgg gggacctcca ccccgacgtg accgaggcga tgctctacga gaagttcagc 600
   ccggccgggc ccatcctctc catccgggtc tgcagggaca tgatcacccg ccgctccttg 660
   ggctacgcgt atgtgaactt ccagcagccg gcggacgcgg agcgtgcttt ggacaccatg 720
   aattttgatg ttataaaggg caagccagta cgcatcatgt ggtctcagcg tgatccatca 780
   cttcgcaaaa gtggagtagg caacatattc attaaaaatc tggacaaatc cattgataat 840
   aaagcactgt atgatacatt ttctgctttt ggtaacatcc tttcatgtaa ggtggtttgt 900
   gatgaaaatg gttccaaggg ctatggattt gtacactttg agacgcagga agcagctgaa 960
   agagctattg aaaaaatgaa tggaatgctc ctaaatgatc gcaaagtatt tgttggacga 1020
   tttaagtctc gtaaagaacg agaagctgaa cttggagcta gggcaaaaga attcaccaat 1080
   gtttacatca agaattttgg agaagacatg gatgatgagc gccttaagga tctctttggc 1140
   aagtttgggc ctgccttaag tgtgaaagta atgactgatg aaagtggaaa atccaaagga 1200
   tttggatttg taagctttga aaggcatgaa gatgcacaga aagctgtgga tgagatgaac 1260
   ggaaaggagc tcaatggaaa acaaatttat gttggtcgag ctcagaaaaa ggtggaacgg 1320
   cagacggaac ttaagcgcaa atttgaacag atgaaacaag ataggatcac cagataccag 1380
   ggtgttaatc tttatgtgaa aaatcttgat gatggtattg atgatgaacg tctccggaaa 1440
   gagttttctc catttggtac aatcactagt gcaaaggtta tgatggaggg tggtcgcagc 1500
   aaagggtttg gttttgtatg tttctcctcc ccagaagaag ccactaaagc agttacagaa 1560
   atgaacggta gaattgtggc cacaaagcca ttgtatgtag ctttagctca gcgcaaagaa 1620
   gagcgccagg ctcacctcac taaccagtat atgcagagaa tggcaagtgt acgagctgtt 1680
   cccaaccctg taatcaaccc ctaccagcca gcacctcctt caggttactt catggcagct 1740
   atcccacaga ctcagaaccg tgctgcatac tatcctccta gccaaattgc tcaactaaga 1800
   ccaagtcctc gctggactgc tcagggtgcc agacctcatc cattccaaaa tatgcccggt 1860
   gctatccgcc cagctgctcc tagaccacca tttagtacta tgagaccagc ttcttcacag 1920
   gttccacgag tcatgtcaac acagcgtgtt gctaacacat caacacagac aatgggtcca 1980
   cgtcctgcag ctgcagccgc tgcagctact cctgctgtcc gcaccgttcc acagtataaa 2040
   tatgctgcag gagttcgcaa tcctcagcaa catcttaatg cacagccaca agttacaatg 2100
   caacagcctg ctgttcatgt acaaggtcag gaacctttga ctgcttccat gttggcatct 2160
   gcccctcctc aagagcaaaa gcaaatgttg ggtgaacggc tgtttcctct tattcaagcc 2220
   atgcacccta ctcttgctgg taaaatcact ggcatgttgt tggagattga taattcagaa 2280
   cttcttcata tgctcgagtc tccagagtca ctccgttcta aggttgatga agctgtagct 2340
   gtactacaag cccaccaagc taaagaggct gcccagaaag cagttaacag tgccaccggt 2400
   gttccaactg tttaaaattg atcagggacc atgaaaagaa acttgtgctt caccgaagaa 2460
   aaatatctaa acatcgaaaa acttaaatat tatggaaaaa aaacattgca aaatataaaa 2520
   taaataaaaa aaggaaagga aactttgaac cttatgtacc gagcaaatgc caggtctagc 2580
   aaacataatg ctagtcctag attacttatt gatttaaaaa caaaaaaaca caaaaaaata 2640
   gtaaaatata aaaacaaatt aatgttttat agaccctggg aaaaagaatt ttcagcaaag 2700
   tacaaaaatt taaagcattc ctttctttaa ttttgtaatt ctttactgtg gaatagctca 2760
   gaatgtcagt tctgttttaa gtaacagaat tgataactga gcaaggaaac gtaatttgga 2820
   ttataaaatt cttgctttaa taaaaattcc ttaaacagtg aaaaaaaaa             2869
<210> 18
   <211> 434
   <212> DNA
   <213> Homo sapiens
<220>
   <223> 60S ribosomal protein L37a, L37A
<220>
   <221> CDS
   <222> (79) ... (357)
   <223> RPL37A
<400> 18
   acttccgctc gtccgcctaa taccgcgcct gcgcaccgcg tctcttcctt tctgggctcg 60
   gacctaggtc gcggcgacat ggccaaacgt accaagaaag tcgggatcgt cggtaaatac 120
   gggacccgct atggggcctc cctccggaaa atggtgaaga aaattgaaat cagccagcac 180
   gccaagtaca cttgctcttt ctgtggcaaa accaagatga agagacgagc tgtggggatc 240
   tggcactgtg gttcctgcat gaagacagtg gctggcggtg cctggacgta caataccact 300
   tccgctgtca cggtaaagtc cgccatcaga agactgaagg agttgaaaga ccagtagacg 360
   ctcctctact ctttgagaca tcactggcct ataataaatg ggttaattta tgtaacaaaa 420
   aaaaaaaaaa aaaa                                                   434
<210> 19
   <211> 755
   <212> DNA
   <213> Homo sapiens
<220>
   <223> 40S ribosomal protein S5, S5
<220>
   <221> CDS
   <222> (73) ... (687)
   <223> RPS5
<400> 19
   ctcttcctgt ctgtaccagg gcggcgcgtg gtctacgccg agtgacagag acgctcaggc 60
   tgtgttctca ggatgaccga gtgggagaca gcagcaccag cggtggcaga gaccccagac 120
   atcaagctct ttgggaagtg gagcaccgat gatgtgcaga tcaatgacat ttccctgcag 180
   gattacattg cagtgaagga gaagtatgcc aagtacctgc ctcacagtgc agggcggtat 240
   gccgccaaac gcttccgcaa agctcagtgt cccattgtgg agcgcctcac taactccatg 300
   atgatgcacg gccgcaacaa cggcaagaag ctcatgactg tgcgcatcgt caagcatgcc 360
   ttcgagatca tacacctgct cacaggcgag aaccctctgc aggtcctggt gaacgccatc 420
   atcaacagtg gtccccggga ggactccaca cgcattgggc gcgccgggac tgtgagacga 480
   caggctgtgg atgtgtcccc cctgcgccgt gtgaaccagg ccatctggct gctgtgcaca 540
   ggcgctcgtg aggctgcctt ccggaacatt aagaccattg ctgagtgcct ggcagatgag 600
   ctcatcaatg ctgccaaggg ctcctcgaac tcctatgcca ttaagaagaa ggacgagctg 660
   gagcgtgtgg ccaagtccaa ccgctgattt tcccagctgc tgcccaataa acctgtctgc 720
   cctttggggc agtcccagcc aaaaaaaaaa aaaaa                            755
<210> 20
   <211> 582
   <212> DNA
   <213> Homo sapiens
<220>
   <223> 60S ribosomal protein L21, L21
<220>
   <221> CDS
   <222> (44) ... (526)
   <223> RPL21
<400> 20
   tttcctttcg gccggaaccg ccatcttcca gtaattcgcc aaaatgacga acacaaaggg 60
   aaagaggaga ggcacccgat atatgttctc taggcctttt agaaaacatg gagttgttcc 120
   tttggccaca tatatgcgaa tctataagaa aggtgatatt gtagacatca agggaatggg 180
   tactgttcaa aaaggaatgc cccacaagtg ttaccatggc aaaactggaa gagtctacaa 240
   tgttacccag catgctgttg gcattgttgt aaacaaacaa gttaagggca agattcttgc 300
   caagagaatt aatgtgcgta ttgagcacat taagcactct aagagccgag atagcttcct 360
   gaaacgtgtg aaggaaaatg atcagaaaaa gaaagaagcc aaagagaaag gtacctgggt 420
   tcaactaaag cgccagcctg ctccacccag agaagcacac tttgtgagaa ccaatgggaa 480
   ggagcctgag ctgctggaac ctattcccta tgaattcatg gcataatagg tgttaaaaaa 540
   aaaaataaag gacctctggg ctacaaaaaa aaaaaaaaaa aa                    582
<210> 21
   <211> 488
   <212> DNA
   <213> Homo sapiens
<220>
   <223> 40S ribosomal protein S15a transcript variant 1, S15a
<220>
   <221> CDS
   <222> (31) ... (423)
   <223> RPS15A
<400> 21
   ctctttccgc catctttccg cgccgccaca atggtgcgca tgaatgtcct ggcagatgct 60
   ctcaagagta tcaacaatgc cgaaaagaga ggcaaacgcc aggtgcttat taggccgtgc 120
   tccaaagtca tcgtccggtt tctcactgtg atgatgaagc atggttacat tggcgaattt 180
   gaaatcattg atgaccacag agctgggaaa attgttgtga acctcacagg caggctaaac 240
   aagtgtgggg tgatcagccc cagatttgac gtgcaactca aagacctgga aaaatggcag 300
   aataatctgc ttccatcccg ccagtttggt ttcattgtac tgacaacctc agctggcatc 360
   atggaccatg aagaagcaag acgaaaacac acaggaggga aaatcctggg attctttttc 420
   tagggatgta atacatatat ttacaaataa aatgcctcat ggactctggt gcttccaaaa 480
   aaaaaaaa                                                          488
<210> 22
   <211> 1125
   <212> DNA
   <213> Homo sapiens
<220>
   <223> guanine nucleotide binding protein (G protein), beta polypeptide 2-like 1 (GNB2L1), cell proliferation-inducing gene 21 protein (PIG21), receptor of activated protein kinase C 1 (RACK1), lung cancer oncogene 7 (HLC-7), H12.3
<220>
   <221> CDS
   <222> (107) ... (1060)
   <223> GNB2L1
<400> 22
   ctctctttca ctgcaaggcg gcggcaggag aggttgtggt gctagtttct ctaagccatc 60
   cagtgccatc ctcgtcgctg cagcgacaca cgctctcgcc gccgccatga ctgagcagat 120
   gacccttcgt ggcaccctca agggccacaa cggctgggta acccagatcg ctactacccc 180
   gcagttcccg gacatgatcc tctccgcctc tcgagataag accatcatca tgtggaaact 240
   gaccagggat gagaccaact atggaattcc acagcgtgct ctgcggggtc actcccactt 300
   tgttagtgat gtggttatct cctcagatgg ccagtttgcc ctctcaggct cctgggatgg 360
   aaccctgcgc ctctgggatc tcacaacggg caccaccacg aggcgatttg tgggccatac 420
   caaggatgtg ctgagtgtgg ccttctcctc tgacaaccgg cagattgtct ctggatctcg 480
   agataaaacc atcaagctat ggaataccct gggtgtgtgc aaatacactg tccaggatga 540
   gagccactca gagtgggtgt cttgtgtccg cttctcgccc aacagcagca accctatcat 600
   cgtctcctgt ggctgggaca agctggtcaa ggtatggaac ctggctaact gcaagctgaa 660
   gaccaaccac attggccaca caggctatct gaacacggtg actgtctctc cagatggatc 720
   cctctgtgct tctggaggca aggatggcca ggccatgtta tgggatctca acgaaggcaa 780
   acacctttac acgctagatg gtggggacat catcaacgcc ctgtgcttca gccctaaccg 840
   ctactggctg tgtgctgcca caggccccag catcaagatc tgggatttag agggaaagat 900
   cattgtagat gaactgaagc aagaagttat cagtaccagc agcaaggcag aaccacccca 960
   gtgcacctcc ctggcctggt ctgctgatgg ccagactctg tttgctggct acacggacaa 1020
   cctggtgcga gtgtggcagg tgaccattgg cacacgctag aagtttatgg cagagcttta 1080
   caaataaaaa aaaaactggc ttttctgaca aaaaaaaaaa aaaaa                 1125
<210> 23
   <211> 644
   <212> DNA
   <213> Homo sapiens
<220>
   <223> 60S ribosomal protein L11 transcript variant 1, L11, cell growth-inhibiting protein 34 (GIG34), CLL-associated antigen KW-12, DBA7
<220>
   <221> CDS
   <222> (46) ... (582)
   <223> RPL11
<400> 23
   aaggccctcg gccggaagct ccgctttctc ttcctgctct ccatcatggc gcaggatcaa 60
   ggtgaaaagg agaaccccat gcgggaactt cgcatccgca aactctgtct caacatctgt 120
   gttggggaga gtggagacag actgacgcga gcagccaagg tgttggagca gctcacaggg 180
   cagacccctg tgttttccaa agctagatac actgtcagat cctttggcat ccggagaaat 240
   gaaaagattg ctgtccactg cacagttcga ggggccaagg cagaagaaat cttggagaag 300
   ggtctaaagg tgcgggagta tgagttaaga aaaaacaact tctcagatac tggaaacttt 360
   ggttttggga tccaggaaca catcgatctg ggtatcaaat atgacccaag cattggtatc 420
   tacggcctgg acttctatgt ggtgctgggt aggccaggtt tcagcatcgc agacaagaag 480
   cgcaggacag gctgcattgg ggccaaacac agaatcagca aagaggaggc catgcgctgg 540
   ttccagcaga agtatgatgg gatcatcctt cctggcaaat aaattcccgt ttctatccaa 600
   aagagcaata aaaagttttc agtgaaatgt gcaaaaaaaa aaaa                  644
<210> 24
   <211> 1013
   <212> DNA
   <213> Homo sapiens
<220>
   <223> 60S ribosomal protein L7a, L7A, surfeit locus protein 3, surfeit 3 (SURF3), thyroid hormone receptor uncoupling protein (TRUP), PLA-X polypeptide
<220>
   <221> CDS
   <222> (31) ... (831)
   <223> RPL7A
<400> 24
   atatttcctg ttccggggcg tgtgggaccc ggatgcaagc gtgctatata agcgttgctc 60
   aagtcccacc cctttctttt tgaggaagac gcggtcgtaa gggctgagga tttttggtcc 120
   gcacgctcct gctcctgact caccgctgtt cgctctcgcc gaggaacaag tcggtcagga 180
   agcccgcgcg caacagccat ggcttttaag gataccggaa aaacacccgt ggagccggag 240
   gtggcaattc accgaattcg aatcacccta acaagccgca acgtaaaatc cttggaaaag 300
   gtgtgtgctg acttgataag aggcgcaaaa gaaaagaatc tcaaagtgaa aggaccagtt 360
   cgaatgccta ccaagacttt gagaatcact acaagaaaaa ctccttgtgg tgaaggttct 420
   aagacgtggg atcgtttcca gatgagaatt cacaagcgac tcattgactt gcacagtcct 480
   tctgagattg ttaagcagat tacttccatc agtattgagc caggagttga gttgatcgaa 540
   tctacagatg cagaacccat ggatacagag ggccagcagt acactttgag aagtgtattc 600
   gaatccccgg ggacatgtcc tttttaactg cattctcctc cgccaaaaaa gtgaccaagc 660
   agagtctttc tctgtcaccc aggctggagt gcaatggcgt gatctcagct cactgcaacc 720
   tctgcctcct gggttcaagt gattctcgtg tctcagcctc ctgagtagct gagactacag 780
   gtgtgcacca gtgttcccag ctgatttttg tattttatgt agagatgggg ttatgccatt 840
   ttggccaggc tagtctcgaa ctcctgagct caggtgatac acacacctca gcaaatcttt 900
   taaattatac attctgtgat atttccttga ctttcttatc cagcacttgt attgattatt 960
   tttcattttg ataatgttgg gtttttaaaa actcctttat gatggaaaat ttc        1013
<210> 25
   <211> 890
   <212> DNA
   <213> Homo sapiens
<220>
   <223> 40S ribosomal protein S20 transcript variant 1, S20
<220>
   <221> CDS
   <222> (199) ... (627)
   <223> RPS20
<400> 25
   tttcctttct ctctcctccc gccgcccaag atgccgaaag gaaagaaggc caagggaaag 60
   aaggtggctc cggccccagc tgtcgtgaag aagcaggagg ctaagaaagt ggtgaatccc 120
   ctgtttgaga aaaggcctaa gaattttggc attggacagg acatccagcc caaaagagac 180
   ctcacccgct ttgtgaaatg gccccgctat atcaggttgc agcggcagag agccatcctc 240
   tataagcggc tgaaagtgcc tcctgcgatt aaccagttca cccaggccct ggaccgccaa 300
   acagctactc agctgcttaa gctggcccac aagtacagac cagagacaaa gcaagagaag 360
   aagcagagac tgttggcccg ggccgagaag aaggctgctg gcaaagggga cgtcccaacg 420
   aagagaccac ctgtccttcg agcaggagtt aacaccgtca ccaccttggt ggagaacaag 480
   aaagctcagc tggtggtgat tgcacacgac gtggatccca tcgagctggt tgtcttcttg 540
   cctgccctgt gtcgtaaaat gggggtccct tactgcatta tcaagggaaa ggcaagactg 600
   ggacgtctag tccacaggaa gacctgcacc actgtcgcct tcacacaggt gaactcggaa 660
   gacaaaggcg ctttggctaa gctggtggaa gctatcagga ccaattacaa tgacagatac 720
   gatgagatcc gccgtcactg gggtggcaat gtcctgggtc ctaagtctgt ggctcgtatc 780
   gccaagctcg aaaaggcaaa ggctaaagaa cttgccacta aactgggtta aatgtacact 840
   gttgagtttt ctgtacataa aaataattga aataatacaa attttccttc           890
<210> 26
   <211> 872
   <212> DNA
   <213> Homo sapiens
<220>
   <223> 40S ribosomal protein S19, S19, Diamond-Blackfan anemia (DBA, DBA1)
<220>
   <221> CDS
   <222> (373) ... (810)
   <223> RPS19
<400> 26
   gtactttcgc catcatagta ttctccacca ctgttccttc cagccacgaa cgacgcaaac 60
   gaagccaagt tcccccagct ccgaacagga gctctctatc ctctctctat tacactccgg 120
   gagaaggaaa cgcgggagga aacccaggcc tccacgcgcg accccttggc cctccccttt 180
   acctctccac ccctcactag acaccctccc ctctaggcgg ggacgaactt tcgccctgag 240
   agaggcggag cctcagcgtc taccctcgct ctcgcgagct ttcggaactc tcgcgagacc 300
   ctacgcccga cttgtgcgcc cgggaaaccc cgtcgttccc tttcccctgg ctggcagcgc 360
   ggaggccgca cgatgcctgg agttactgta aaagacgtga accagcagga gttcgtcaga 420
   gctctggcag ccttcctcaa aaagtccggg aagctgaaag tccccgaatg ggtggatacc 480
   gtcaagctgg ccaagcacaa agagcttgct ccctacgatg agaactggtt ctacacgcga 540
   gctgcttcca cagcgcggca cctgtacctc cggggtggcg ctggggttgg ctccatgacc 600
   aagatctatg ggggacgtca gagaaacggc gtcatgccca gccacttcag ccgaggctcc 660
   aagagtgtgg cccgccgggt cctccaagcc ctggaggggc tgaaaatggt ggaaaaggac 720
   caagatggcg gccgcaaact gacacctcag ggacaaagag atctggacag aatcgccgga 780
   caggtggcag ctgccaacaa gaagcattag aacaaaccat gctgggttaa taaattgcct 840
   cattcgtaaa aaaaaaaaaa aaaaaaaaaa aa                               872
<210> 27
   <211> 418
   <212> DNA
   <213> Homo sapiens
<220>
   <223> 40S ribosomal protein S21, S21, 8.2 kDa differentiation factor
<220>
   <221> CDS
   <222> (91) ... (342)
   <223> RPS21
<400> 27
   cttgcccgcc gatatctctg ccgggtgact agctgcttcc tttctctctc gcgcgcggtg 60
   tggtggcagc aggcgcagcc cagcctcgaa atgcagaacg acgccggcga gttcgtggac 120
   ctgtacgtgc cgcggaaatg ctccgctagc aatcgcatca tcggtgccaa ggaccacgca 180
   tccatccaga tgaacgtggc cgaggttgac aaggtcacag gcaggtttaa tggccagttt 240
   aaaacttatg ctatctgcgg ggccattcgt aggatgggtg agtcagatga ttccattctc 300
   cgattggcca aggccgatgg catcgtctca aagaactttt gactggagag aatcacagat 360
   gtggaatatt tgtcataaat aaataatgaa aacctaaaaa aaaaaaaaaa aaaaaaaa   418
<210> 28
   <211> 1289
   <212> DNA
   <213> Homo sapiens
<220>
   <223> 60S acidic ribosomal protein P0, LP0, P0, ribosomal protein L10E, L10E, acidic ribosomal phosphoprotein P0, PRLP0, RPP0
<220>
   <221> CDS
   <222> (238) ... (1191)
   <223> RPLP0
<400> 28
   gtctgacggg cgatggcgca gccaatagac aggagcgcta tccgcggttt ctgattggct 60
   actttgttcg cattataaaa ggcacgcgcg ggcgcgaggc ccttctctcg ccaggcgtcc 120
   tcgtggaagg cccgggaccg cgggatgggt gtcggcgtga ccaggcctga gctccctgtc 180
   tctcctcagt gacatcgtct ttaaaccctg cgtggcaatc cctgacgcac cgccgtgatg 240
   cccagggaag acagggcgac ctggaagtcc aactacttcc ttaagatcat ccaactattg 300
   gatgattatc cgaaatgttt cattgtggga gcagacaatg tgggctccaa gcagatgcag 360
   cagatccgca tgtcccttcg cgggaaggct gtggtgctga tgggcaagaa caccatgatg 420
   cgcaaggcca tccgagggca cctggaaaac aacccagctc tggagaaact gctgcctcat 480
   atccggggga atgtgggctt tgtgttcacc aaggaggacc tcactgagat cagggacatg 540
   ttgctggcca ataaggtgcc agctgctgcc cgtgctggtg ccattgcccc atgtgaagtc 600
   actgtgccag cccagaacac tggtctcggg cccgagaaga cctccttttt ccaggcttta 660
   ggtatcacca ctaaaatctc caggggcacc attgaaatcc tgagtgatgt gcagctgatc 720
   aagactggag acaaagtggg agccagcgaa gccacgctgc tgaacatgct caacatctcc 780
   cccttctcct ttgggctggt catccagcag gtgttcgaca atggcagcat ctacaaccct 840
   gaagtgcttg atatcacaga ggaaactctg cattctcgct tcctggaggg tgtccgcaat 900
   gttgccagtg tctgtctgca gattggctac ccaactgttg catcagtacc ccattctatc 960
   atcaacgggt acaaacgagt cctggccttg tctgtggaga cggattacac cttcccactt 1020
   gctgaaaagg tcaaggcctt cttggctgat ccatctgcct ttgtggctgc tgcccctgtg 1080
   gctgctgcca ccacagctgc tcctgctgct gctgcagccc cagctaaggt tgaagccaag 1140
   gaagagtcgg aggagtcgga cgaggatatg ggatttggtc tctttgacta atcaccaaaa 1200
   agcaaccaac ttagccagtt ttatttgcaa aacaaggaaa taaaggctta cttctttaaa 1260
   aagtaaaaaa aaaaaaaaaa aaaaaaaaa                                  1289
<210> 29
   <211> 753
   <212> DNA
   <213> Homo sapiens
<220>
   <223> 40S ribosomal protein S9, S9
<220>
   <221> CDS
   <222> (57) ... (641)
   <223> RPS9
<400> 29
   ctctttctca gtgaccgggt ggtttgctta ggcgcagacg gggaagcgga gccaacatgc 60
   cagtggcccg gagctgggtt tgtcgcaaaa cttatgtgac cccgcggaga cccttcgaga 120
   aatctcgtct cgaccaagag ctgaagctga tcggcgagta tgggctccgg aacaaacgtg 180
   aggtctggag ggtcaaattt accctggcca agatccgcaa ggccgcccgg gaactgctga 240
   cgcttgatga gaaggaccca cggcgtctgt tcgaaggcaa cgccctgctg cggcggctgg 300
   tccgcattgg ggtgctggat gagggcaaga tgaagctgga ttacatcctg ggcctgaaga 360
   tagaggattt cttagagaga cgcctgcaga cccaggtctt caagctgggc ttggccaagt 420
   ccatccacca cgctcgcgtg ctgatccgcc agcgccatat cagggtccgc aagcaggtgg 480
   tgaacatccc gtccttcatt gtccgcctgg attcccagaa gcacatcgac ttctctctgc 540
   gctctcccta cgggggtggc cgcccgggcc gcgtgaagag gaagaatgcc aagaagggcc 600
   agggtggggc tggggctgga gacgacgagg aggaggatta agtccacctg tccctcctgg 660
   gctgctggat tgtctcgttt tcctgccaaa taaacaggat cagcgcttta caaaaaaaaa 720
   aaaaaaaaaa aaaaaaaaaa aaaaaaaaaa aaa                               753
<210> 30
   <211> 2108
   <212> DNA
   <213> Homo sapiens
<220>
   <223> 40S ribosomal protein S3 transcript variant 1, S3, IMR-90 ribosomal protein S3
<220>
   <221> CDS
   <222> (58) ... (789)
   <223> RPS3
<400> 30
   atactcactt ccgcccgcga gccacttcct ttcctttcag cggagcgcgg cggcaagatg 60
   gcagtgcaaa tatccaagaa gaggaagttt gtcgctgatg gcatcttcaa agctgaactg 120
   aatgagtttc ttactcggga gctggctgaa gatggctact ctggagttga ggtgcgagtt 180
   acaccaacca ggacagaaat cattatctta gccaccagaa cacagaatgt tcttggtgag 240
   aagggccggc ggattcggga actgactgct gtagttcaga agaggtttgg ctttccagag 300
   ggcagtgtag agctttatgc tgaaaaggtg gccactagag gtctgtgtgc cattgcccag 360
   gcagagtctc tgcgttacaa actcctagga gggcttgctg tgcggagggc ctgctatggt 420
   gtgctgcggt tcatcatgga gagtggggcc aaaggctgcg aggttgtggt gtctgggaaa 480
   ctccgaggac agagggctaa atccatgaag tttgtggatg gcctgatgat ccacagcgga 540
   gaccctgtta actactacgt tgacactgct gtgcgccacg tgttgctcag acagggtgtg 600
   ctgggcatca aggtgaagat catgctgccc tgggacccaa ctggtaagat tggccctaag 660
   aagcccctgc ctgaccacgt gagcattgtg gaacccaaag atgagatact gcccaccacc 720
   cccatctcag aacagaaggg tgggaagcca gagccgcctg ccatgcccca gccagtcccc 780
   acagcataac agggtctcct tggcagctgt attctggagt ctggatgttg ctctctaaag 840
   acctttaata aaattttgta caaagacaca aggtctgact agactgttca gtattcagac 900
   tgaggggcat gttggcctct ggagcattac atatcttctt ggttttaacc atacttgtgg 960
   tatttgcaag ggccagaaca gtaagaccca agcagagcca accagagaaa taatatttgt 1020
   gtgatagaga aggctgatag caagcaaggc agcaccttga ttcgttgtcc tgtagttcag 1080
   gattgtaggt ttagaagagg gatatgtttg agtttttcct atgcataagg cgatccacgt 1140
   tgcacataga aagtgaatat aaatggccat tatattttgt gtcatgctgt gctctaagtg 1200
   ttctttacat atgtactcgt taatcaacct ctctaaagtg taaaggaaat ttgcttgcac 1260
   cactgaaggc acataaggct cagaagtaaa tttgcctaag cagtataaag ctatcattag 1320
   aatccacatt cctaagttgt gttctcttag gggatcatgg aaccagtcat tggtactaca 1380
   ggctattatg ttctggagaa ctgtgaagaa catttaaatt gtctctgatt ttatctatca 1440
   atgttttgaa gtattttcta ccagtgtctg tacttcacaa gaaattcggc actatttttt 1500
   caggcaaaac tagtgaggga caggttggct tgaaaatcat gagactgttg ttaaatcaga 1560
   tgctggttga tcacagaggg gacttccagg gaaagctgtt atcaggtggc tgcttcctgg 1620
   tgatgcagcc tggctgatga gataaccctg gctccacaga tggcttagca ggtgctgtga 1680
   tgatttggtt ttcttctcaa ttagactgag ctgcacatgg tgtttatatt gcttggcaca 1740
   tggtaagggc ttaatatttg aggtaattat gtagggcgta cactgacaag tatctgaccc 1800
   ccccttcctt tttgactcat aaattggtca tcttaaccat ttaagtgtac acttctatag 1860
   tgacagagtt agccctctgt ccaagggatt tgcatctgtg gattcaacca actttgggtc 1920
   aaaaataatc aaaaaggatg gttgtgtgtg tattgaacat gtagacttat ttttcttatt 1980
   ttcaaaatac tatattttct tgtcacttat tttcttgtac actgcagttg taacagctat 2040
   gtagcatgta cattaggtat taaaagtaat ccagtgaaga ttgaaagtct aaaaaaaaaa 2100
   aaaaaaaa                                                          2108
<210> 31
   <211> 6668
   <212> DNA
   <213> Homo sapiens
<220>
   <223> cartilage associated protein precursor (CRTAP, CASP), leprecan-like 3 (LEPREL3), OI7
<220>
   <221> CDS
   <222> (121) ... (1326)
   <223> CRTAP
<400> 31
   aggctggcgt ccccgccccg aaagcactgg gcccgccgcg tcgcaccgtc ctctttcctt 60
   tccttctccc tccccttttc ccttccttcg tcccttcctt ccttcctttc gccgggcgcg 120
   atggagccgg ggcgccgggg ggccgcggcg ctgctagcgc tgctgtgcgt ggcctgcgcg 180
   ctgcgcgccg ggcgcgccca atacgaacgc tacagcttcc gcagcttccc acgggacgag 240
   ctgatgccgc tcgagtcggc ctaccggcac gcgctggaca agtacagcgg cgagcactgg 300
   gccgagagcg tgggctacct ggagatcagc ctgcggctgc accgcttgct gcgcgacagc 360
   gaggccttct gccaccgcaa ctgcagcgcc gcgccgcagc ccgagcccgc cgccggcctc 420
   gccagctatc ccgagctgcg cctcttcggg ggcctgctgc gccgcgcgca ctgcctcaag 480
   cgctgcaagc agggcctgcc agccttccgc cagtcccagc ccagccgcga ggtgctggcg 540
   gacttccagc gccgcgagcc ctacaagttc ctgcagttcg cttacttcaa ggcaaataat 600
   ctccccaaag ccatcgccgc tgctcacacc tttctactga agcatcctga tgacgaaatg 660
   atgaagagga acatggcata ttataagagc ctgcctggtg ccgaggacta cattaaagac 720
   ctggaaacca agtcatatga aagcctgttc atccgagcag tgcgggcata caacggtgag 780
   aactggagaa catccatcac agacatggag ctggcccttc ccgacttctt caaagccttt 840
   tacgagtgtc tcgcagcctg cgagggttcc agggagatca aggacttcaa ggatttctac 900
   ctttccatag cagatcatta tgtagaagtt ctggaatgca aaatacagtg tgaagagaac 960
   ctcaccccag ttataggagg ctatccggtt gagaaatttg tggctaccat gtatcattac 1020
   ttgcagtttg cctattataa gttgaacgac ctgaagaatg cagccccctg tgcagtcagc 1080
   tatctgctct ttgatcagaa tgacaaggtc atgcagcaga acctggtgta ttaccagtac 1140
   cacagggaca cttggggcct ctcggatgag cacttccagc ccagacctga agcagttcag 1200
   ttctttaatg tgaccacact ccagaaggag ctgtatgact ttgctaagga aaatataatg 1260
   gatgatgatg agggagaagt tgtggaatat gtggatgacc tcttggaact ggaggagacc 1320
   agctagccca cagcaaccaa agagacttcc tcttggcgtt caggaaacac agattctttg 1380
   tccttttccc aacagcccag gctgttgata cctcagagcc ttctctttac tctccaaagt 1440
   gaaagggaag cccccgtctc tctaactgca tgtcatcagg ggtgagcctg cctttcctat 1500
   cttcacacct gccacctcat gttcacacct atctttctca cctttttttt gagatggagt 1560
   ctcgctctct tgcccaggct ggagtgcaat ggcacgttct cagctcactg caacctccgc 1620
   ctcttgggtt caagcaattc tgctgcatca gcctcccgag tacctgggat tacaggcatg 1680
   tgccaccacg cccggctaat tttgtatttt tagtagagac ggggttttgc catgttggcc 1740
   aggctggtct cgaactcttg acttcagatg atccatctgc cttggcctcc cacagtgctg 1800
   ggattacagg cgtgagccac catgcccggc ctctttctca cctttacacc tgtcttctta 1860
   tcctcacatc tgttttcaca ccttcatccc tgtcttcctc atgttcacac ttgtcttccc 1920
   catgttcata gctgcctttc ttaccatttt ggtttgaagg gcagtcttct ctggcttgtt 1980
   tttttgtttt tcccagaaaa tcagtattat tttttaaata agaaaaacat tcctagaaga 2040
   tgataattgt gaaaacctcc tttggcttat ttgcttttcc agattttagt ctcctttctc 2100
   cccatccggg aaagatggtg gaagacatag gctaaatttc tccagcctca caatggtctt 2160
   cacttggtct gacttgtacc aattctagca cccactgaaa aacaagttga gtagagagtg 2220
   tagagtgcag aaatgtggct tttgccccac tttgcatctc caaaattaca acggttggcc 2280
   gatcccattt gaggacaatg cttagttata agtctccgag ttggaaaagg aagaaagcca 2340
   gagctgtcta gtttcattca ttctttcagt aaatatttat tgagtaccta ctgtgtgcta 2400
   ggcattgacc tgggaactag agatacttca cagaataaca gggaaagttc cctgtgctca 2460
   tggagcttac attctacagg gagaaagaga tagccaatac ataggaataa atatatacaa 2520
   ggtatcatgt agtgataatt gctgtggaga aaaataaagc aggggaggga gtaagaaatc 2580
   ctggagatga ggctgcagtt ttaaatgggg cctcactggg aatgtgacgt tgagcagaga 2640
   cgttagggaa gtggatcctg gacaaggcat tccaggcaga ggaacaggat gtgcactgcc 2700
   ccaaagtgag aacttgctct acgtggtcag gaaagagcag ggagaccaag cagagtcgtg 2760
   ggcaggggta gaatggaagg agaggcggct ggggaggaca ggtggtggag ggccttggct 2820
   tctgctaagt gagatgggaa ccactggagg gtttgaacag aggagtgcct tgattgattt 2880
   atattttgca agggtcattc tagctgccat attgtgaaaa actttagtgg acaagggcag 2940
   aaggaagagg gaagacctgt taggaagcta ctgcaaggtt ccaggcttgg gcctgggcca 3000
   cagcaacagc agtggtcaaa tatctagatt tattttgaaa agagccaata ggatttgctg 3060
   agagtttgaa tgtggagtgt aagagaagga agagttaatg atgacattaa ggtttttggc 3120
   ctgaatagca ggaaagatgg agttaccagt tactgaaata gggaaggatg ggctgggtaa 3180
   gtatggaatt tggtgcaaag caggctgtct gtggttggaa tgggaggttc tggctgcaaa 3240
   tcaaagtgga gagttctctc aggtcaggtc tgcagcagag ctcgagacag ggatctgaat 3300
   gcacttggtt tattgttggg ggtgctctca gaaggaacct gtgaaagcct ttatcagtca 3360
   tttattggct gtgagaagtt ctctgggagt gtgggtacat ttgaaggcaa gtgacttcag 3420
   ttgagggcaa gtctctggaa aagaggctgt aggcatctgg cagctaccat gcatggtagt 3480
   gtgttggggg tgggggtcct gggcactggc tgtgtgaagg gatctggcag ggcaccacag 3540
   cgccccctac tgaaccatca gcatgtcagt ggcatttaaa gccatgcagc tggaggggcc 3600
   actgagattg tctctgagta ttactgagaa gcaacagaaa agagccatgg atggagccct 3660
   tgggctctct gggaaatggg aaatcagcca aaggactgag aaggagttac cttaaggtca 3720
   gagaaaacca agagagtgtg gtgttctgga agctgagctt tctttattca acctcattcc 3780
   cttctccaaa taagccactt gtgtagttgg gcccctccag ggttgaaggc aagaggagaa 3840
   aggcacagcg tttgggaaac aagacttttc ctgcaatagc ctgggaagga ataaaaggat 3900
   agagtgtttg ggtttttgtg taatggtggt taattggggt ggaacactca cacgttgtgc 3960
   tttttctggg cttcccttat cccccagaac actctaccaa cctcggggaa ctcgggcaca 4020
   tccttctgtt tctccttcag ctctatcctg ctttcctcat cccttctgac accacgtcct 4080
   cactcacctg cacaagaatc cctgcatcag gttctccttt gagggtaccc acccaggaca 4140
   gtcccctacc acttctgtct tgggctgaag ttgcccacgt ccacaaaatc tgtactccca 4200
   gcgggggtgt ttggcccgag gagtcagtgt tattactggt ggatgcaccg tgtccacagc 4260
   agcccccaat cccagcgatg cgtcagatct tacgtggctt cctgctgggg gagatggcct 4320
   tcacccacgg gatgccgggt tctcctttct ttcctcaccc caacctttac tccaccagag 4380
   aaacttcctt ttgaactcag tggggaagag ggtgatgaga caggactaga aagtagtggg 4440
   ggacccagcg agtggacgcc ctgctccggg attcctgagt ctgtaaatag tgtgcccagc 4500
   agctgtgaac tccccttata gcctcaggct gcagtgtcct tcccagctgt gtgagaaaat 4560
   gaaagccgac gtccacaggg acccaggcag ggttgggtgt tgtgactcac tccacctctg 4620
   tgccctgcag aggtactgtt gggtccttgt cttgtgagcc tggggtgagc tctctgtaca 4680
   tgttgttgtt ccacgtatgg gttgacttgg catgctgggg ggtcctcgtt cactctctga 4740
   agttggcctc ctttcactgg ggattgaaaa gcacctccac ccctacccta gtgatgtccc 4800
   ctgaggaccc gggtgatagt acagtcaata ttgtcagtac tttgctttga ttgaaggctg 4860
   tagagctgag ttaccaaaat ttctatttca aaggaaacca aaccttaaaa aaaaaaaaca 4920
   aaaactgggc tgggtcttcc aaacctacca tgaaaccctg gtgtgcaggc tgcactcaat 4980
   gacctcaacc caacacctcc ctgagtgtgc ttcttggaag agcctagaag attcctggat 5040
   ggagacccca ttggttcagc ctcaagtctg gcccgtcttc gaaaaaacaa acacatttgt 5100
   aagctttgtg ggagcttcca ggcctgctct aagatgcctt gcttgtcctt tgacccatca 5160
   gcatggagct cagtggttgc tgtttggttc tgcaggctgg tggggaggcc gcccatcgtg 5220
   gtggggcatc tgtccagccc cattgccact cagggcatcc aaacaggagg cacccgctgg 5280
   gaagggtcta aagatactcc ttgtggccac tgctactgtt cacacttgac ttgtggagaa 5340
   gcgaagggct gaggggaggt ttgtgtacac ccatgtattt aaaagtgact gactgactga 5400
   aatgagcaca taccgacata tgcaacatac taataccttc ctgattttcg agactttcta 5460
   attactacaa ctaacctgtt gtgctcacct ctggaattca gaaagagagc cactgcgagc 5520
   actgaccaca agggctgcct taggaaggaa atgtgtgctt tcaggagttc ttattagggg 5580
   aattttaaga gcacaaaaat tttattgaac ccaccttagt gacaaacaga aaatgatttg 5640
   ttagattgtc agttggaagg ggtttattat gactgctggt gaaataaaca gtgaccagtt 5700
   tctgccaaca tttatggaaa taacgtttct aggttttaaa tgtgagccgt aaactgaagc 5760
   tacttcagtt aaaaaaaaaa aaatcaatac ttaaactgta gggaaaaggt ggattggtgc 5820
   cagagaaaac attatttaat agtgtcagaa catggaactg caacacagtt tgtagcaagg 5880
   cactgagaaa aacccacaac taatccttca tcgcagctgt ctgaactctt gatcatctgc 5940
   catcccccaa acagtgactt ctttttttct ggtgactcca ggcctgaatg acctagtgtg 6000
   gagagtttaa actatgtaca agggaggaaa gaaaaaaagg aaaggaacct taagtaagcc 6060
   tcagccaaag gttttatgca ttggacttcc tgtgcctgcc cccaggggca agactgatcc 6120
   ccatgcctgt gcccatgaca tctccctgaa agaggacacc atgacagccc ggctttgcct 6180
   tgactgaccc actgctaccc cagaaataag aatcaagagc agctattgtt atccttagag 6240
   tgttttccgt ctagggccgg ctcgtgaaca gccacatatc cttgcacctg acactgtccc 6300
   cacacaaata gctggctttc gttgcttgtt gaatgaatga gtgagttggc tctatatccc 6360
   cttggagctg gccggtaaga tattagtgcc tcattttaca agaagagaat aggaaggaat 6420
   taagcaattt ggccaacaga tacaagatag attccagagt tttatctccc actttagggt 6480
   ggcagccagt aggccaaact ccaaagaccg ttgctgatgt ctttttctgc ctcccctctt 6540
   tgggttagtg tggtatgtac aagctcactc ttgttgaaaa ttagaaaata gttgaaaaca 6600
   aaaggttttt gtttttcttt ttaaatcaca ttaaatgttt tacattgctt aaaaaaaaaa 6660
   aaaaaaaa                                                          6668
<210> 32
   <211> 967
   <212> DNA
   <213> Homo sapiens
<220>
   <223> hypothetical protein LOC80097, family with sequence similarity 128, member B (FAM128B), mitotic-spindle organizing protein 2B (MZT2B), mitotic-spindle organizing protein associated with a ring of gamma-tubulin 2B (MOZART2B)
<220>
   <221> CDS
   <222> (356) ... (832)
   <223> FAM128B
<400> 32
   tttcttgaaa gaggcattta ccgagcgccc aatgtatgcc tggcactggg ctgggtgctg 60
   ccacctaagc gagcacgacc aatgcagtct atcagggagg cccagatcgc caagcagcgg 120
   acccctgcgg tccgccatgc cactcccggc tcctagagcg ccgctcagca caccgtgagc 180
   gcccaataac tgttgggctt caatgacgcc gcggaggcgg ccccgtcccc gcgctcccgc 240
   ccctcccgcc agggcagccc gggaggccag acgttgacgc tgcagggaga gggtggtggg 300
   cgcagccgct agggggcgcg gcggggcgga gcgcaccttt ccgcgggccg cggggatggc 360
   ggcgcagggc gtagggcctg ggccggggtc ggcggcgccc ccggggctgg aggcggcccg 420
   gcagaagctg gcgctgcggc ggaagaaggt gctgagcacc gaggagatgg agctgtacga 480
   gctggcgcag gcggcgggcg gcgctatcga ccccgacgtg ttcaagatcc tggtggacct 540
   gctgaagctg aacgtggccc ccctcgccgt cttccagatg ctcaagtcca tgtgtgccgg 600
   gcagaggcta gcgagcgagc cccaggaccc tgcggccgtg tctctgccca cgtcgagcgt 660
   gcccgagacc cgagggagaa acaaaggcag cgctgccctc gggggagcat tggccctggc 720
   ggaacgcagc agccgcgaag gatccagcca gaggatgcca cgccagccca gcgctaccag 780
   gctgcccaag gggggcgggc ctgggaagag ccctacacgg ggcagcacct aggatggggc 840
   agagacttgt tgcatctttg tccccagcaa aggctacatg ttacctcctt caattgataa 900
   taaacctttc tgagatgcag agggtccagg tcaaaaaaaa aaaaaaaaaa aaaaaaaaaa 960
   aaaaaaa                                                           967
<210> 33
   <211> 1787
   <212> DNA
   <213> Homo sapiens
<220>
   <223> 60S ribosomal protein L32 transcript variant 3, L32, PP9932
<220>
   <221> CDS
   <222> (214) ... (621)
   <223> RPL32
<400> 33
   gacctcctgg gatcgcatct ggagagtgcc tagtattctg ccagcttcgg aaagggaggg 60
   aaagcaagcc tggcagaggc acccattcca ttcccagctt gctccgtagc tggcgattgg 120
   aagacactct gcgacagtgt tcagtccctg ggcaggaaag cctccttcca ggattcttcc 180
   tcacctgggg ccgcttcttc cccaaaaggc atcatggccg ccctcagacc ccttgtgaag 240
   cccaagatcg tcaaaaagag aaccaagaag ttcatccggc accagtcaga ccgatatgtc 300
   aaaattaagc gtaactggcg gaaacccaga ggcattgaca acagggttcg tagaagattc 360
   aagggccaga tcttgatgcc caacattggt tatggaagca acaaaaaaac aaagcacatg 420
   ctgcccagtg gcttccggaa gttcctggtc cacaacgtca aggagctgga agtgctgctg 480
   atgtgcaaca aatcttactg tgccgagatc gctcacaatg tttcctccaa gaaccgcaaa 540
   gccatcgtgg aaagagctgc ccaactggcc atcagagtca ccaaccccaa tgccaggctg 600
   cgcagtgaag aaaatgagta ggcagctcat gtgcacgttt tctgtttaaa taaatgtaaa 660
   aactgccatc tggcatcttc cttccttgat tttaagtctt cagcttcttg gccaacttag 720
   tttgccacag agattgttct tttgcttaag cccctttgga atctcccatt tggaggggat 780
   ttgtaaagga cactcagtcc ttgaacaggg gaatgtggcc tcaagtgcac agactagcct 840
   tagtcatctc cagttgaggc tgggtatgag gggtacagac ttggccctca caccaggtag 900
   gttctgagac acttgaagaa gcttgtggct cccaagccac aagtagtcat tcttagcctt 960
   gcttttgtaa agttaggtga caagttattc catgtgatgc ttgtgagaat tgagaaaata 1020
   tgcatggaaa tatccagatg aatttcttac acagattctt acgggatgcc taaattgcat 1080
   cctgtaactt ctgtccaaaa agaacaggat gatgtacaaa ttgctcttcc aggtaatcca 1140
   ccacggttaa ctggaaaagc actttcagtc tcctataacc ctcccaccag ctgctgcttc 1200
   aggtataatg ttacagcagt ttgccaaggc ggggacctaa ctggtgacaa ttgagcctct 1260
   tgactggtac tcagaattta gtgacacgtg gtcctgattt tttttggaga cggggtcttg 1320
   ctctcaccca ggctgggagt gcagtggcac actgactaca gccttgacct ccccaggctc 1380
   aggtgatctt cccacctcag ccttccaagt agctgggact acagatgcac acctccaaac 1440
   ctgggtagtt tttgaagttt ttttgtagag gtggtctagc catgttgcct aggctcccga 1500
   actcctgagc tcaagcaatc ctgcttcagc ctcccaaagt actgggatta caggcatctt 1560
   ctgtagtata taggtcatga gggatatggg atgtggtact tatgagacag aaatgcttac 1620
   aggatgtttt tctgtaacca tcctggtcaa cttagcagaa atgctgcgct gggtataata 1680
   aagcttttct acttctagtc tagacaggaa tcttacagat tgtctcctgt tcaaaaccta 1740
   gtcataaata tttataatgc aaactggtca aaaaaaaaaa aaaaaaa              1787
<210> 34
   <211> 1552
   <212> DNA
   <213> Homo sapiens
<220>
   <223> eukaryotic translation elongation factor 1 gamma (EEF1G, EF1G, EF-1-gamma; eEF-1B gamma), translation elongation factor eEF-1 gamma chain, pancreatic tumor-related protein, GIG35, PRO1608
<220>
   <221> CDS
   <222> (147) ... (1460)
   <223> EEF1G
<400> 34
   aactgtcgta cgtatgtctt ctgtttcgtc ctcgctttcc ggctgctgtt tctccacggc 60
   tctcctcttt ccccctccct tctctcccgg gcggcttact ttgcggcagc gccgagaacc 120
   ccaccccctt tctttgcgga atcaccatgg cggctgggac cctgtacacg tatcctgaaa 180
   actggagggc cttcaaggct ctcatcgctg ctcagtacag cggggctcag gtccgcgtgc 240
   tctccgcacc accccacttc cattttggcc aaaccaaccg cacccctgaa tttctccgca 300
   aatttcctgc cggcaaggtc ccagcatttg agggtgatga tggattctgt gtgtttgaga 360
   gcaacgccat tgcctactat gtgagcaatg aggagctgcg gggaagtact ccagaggcag 420
   cagcccaggt ggtgcagtgg gtgagctttg ctgattccga tatagtgccc ccagccagta 480
   cctgggtgtt ccccaccttg ggcatcatgc accacaacaa acaggccact gagaatgcaa 540
   aggaggaagt gaggcgaatt ctggggctgc tggatgctta cttgaagacg aggacttttc 600
   tggtgggcga acgagtgaca ttggctgaca tcacagttgt ctgcaccctg ttgtggctct 660
   ataagcaggt tctagagcct tctttccgcc aggcctttcc caataccaac cgctggttcc 720
   tcacctgcat taaccagccc cagttccggg ctgtcttggg cgaagtgaaa ctgtgtgaga 780
   agatggccca gtttgatgct aaaaagtttg cagagaccca acctaaaaag gacacaccac 840
   ggaaagagaa gggttcacgg gaagagaagc agaagcccca ggctgagcgg aaggaggaga 900
   aaaaggcggc tgcccctgct cctgaggagg agatggatga atgtgagcag gcgctggctg 960
   ctgagcccaa ggccaaggac cccttcgctc acctgcccaa gagtaccttt gtgttggatg 1020
   aatttaagcg caagtactcc aatgaggaca cactctctgt ggcactgcca tatttctggg 1080
   agcactttga taaggacggc tggtccctgt ggtactcaga gtatcgcttc cctgaagaac 1140
   tcactcagac cttcatgagc tgcaatctca tcactggaat gttccagcga ctggacaagc 1200
   tgaggaagaa tgccttcgcc agtgtcatcc tttttggaac caacaatagc agctccattt 1260
   ctggagtctg ggtcttccga ggccaggagc ttgcctttcc gctgagtcca gattggcagg 1320
   tggactacga gtcatacaca tggcggaaac tggatcctgg cagcgaggag acccagacgc 1380
   tggttcgaga gtacttttcc tgggaggggg ccttccagca tgtgggcaaa gccttcaatc 1440
   agggcaagat cttcaagtga acatctcttg ccatcaccta gctgcctgca cctgcccttc 1500
   agggagatgg gggtcattaa aggaaactga acattgaaaa aaaaaaaaaa aa 1552
<210> 35
   <211> 1978
   <212> DNA
   <213> Homo sapiens
<220>
   <223> 60S ribosomal protein L22-like 1
<220>
   <221> CDS
   <222> (90) ... (458)
   <223> RPL22L1
<400> 35
   cgctctcagc gcgtgacgca gcacgctttg atataaatgc agaccgcgcg gccgtagctt 60
   cctctctgct ctcgcggccg actcgcaaga tggcgccgca gaaagacagg aagcccaaga 120
   ggtcaacctg gaggtttaat ttggacctta ctcatccagt agaagatgga atttttgatt 180
   ctggaaattt tgagcaattt ctacgggaga aggttaaagt caatggcaaa actggaaatc 240
   tcgggaatgt tgttcacatt gaacgcttca agaataaaat cacagttgtt tctgagaaac 300
   agttctctaa aaggtatttg aaatacctta ccaagaaata ccttaagaag aacaatcttc 360
   gtgattggct tcgagtggtt gcatctgaca aggagaccta cgaacttcgt tacttccaga 420
   ttagtcaaga tgaagatgaa tcagagtcgg aggactaggc aaaggctccc cttacagggc 480
   tttgcttatt aataaaataa atgaagtata catgagaaat accaagaaat tggcttttag 540
   tttatcagtg aataaaaaat attatactct tgaacttttg tctcattttt ttgagtatgc 600
   tgtttatatg attttgattt ccctctgata actatcaaca gtatttaaat agcttatagc 660
   tggtataatt ttttcccacg atttccaaaa tcttttatgt actcaggtaa aagtagcgtt 720
   atataggaaa tctttttttt agacactctc gttctgtcac ccaggctgga gtgcagtgac 780
   tcagcttcct aaatagctgg aattacaggt gtgagccacc atgcccggct aattttttgt 840
   acttttagta gagtagggtt tggccatgtt ggccaggctg gtttcaaact cctgacctca 900
   agtgatctac ccacctcggc ttcccaaagt gctgattata gctgtgaacc accatgcccg 960
   gccaggaaat cttactgtag aacaattttt tatatagctg tataaaatgt atatgattgt 1020
   cttgacagtc tcaaatactg tttttaatag cttgtaaatg taatctcaag tgcttagaac 1080
   agttcttaca tataagttgc tctgtagttt gctcttatag ttagcccaaa gactctgggt 1140
   gtgaggcctg ctgtaaacca atgttaaact gcttattaga aagccctaac cacctgcttt 1200
   gtaggcacca gaaactcaaa accaaatctc aactcagcta cagaatctac tgtggtcctt 1260
   gtctgaaaaa attagttcac tcggttggaa tcttgtctca gagcatcctc atctctttct 1320
   caaaagcccc taccccaaca ccggcgtgtt ggttgtctat tgaaacttac aagtggatgg 1380
   accctttctc ccgaataaac tggcctttga aagctctaat cgaaatggtt tggcaaaatc 1440
   catactgcag gagattaggg aggacaagaa tgatgtgcct ttttgtactg ctgagcctga 1500
   tggtggtgcc actacttcag gtacttagat gagtcttgat gctaatagaa ttgtgtcgcc 1560
   aaacatatct ggacagttac aacctaatct atgcattaat tggtttggga attgcttgaa 1620
   attattgttt aattcaatgt tttaattcgt tttcctaaaa atttaagtgc ccccatcatc 1680
   gtgcaatacc tcagtgcagc aactccttga ttcttggatg actgaacttc ctaacttggc 1740
   tctgccccat tgttcccatt tttcatgttt ttcacaaata gttaaccagg tacctactac 1800
   tgtgcaccgc tgcagagcat tgaggatgta tgtgatgagt aaaaacaccc agcctgctct 1860
   gctgtgttag tattatgacg gaaactgatc aaatcacatg tgaacaaatt tactgctaca 1920
   aaagggaggg cttaataaaa ggaatttcat ctgggaaggc aaaaaaaaaa aaaaaaaa   1978
<210> 36
   <211> 1561
   <212> DNA
   <213> Homo sapiens
<220>
   <223> Y box binding protein 1 (YB1, YB-1, YBX1), nuclease sensitive element binding protein 1 (NSEP1, NSEP-1), DNA-binding protein B (DBPB), CCAAT-binding transcription factor I subunit A (CBF-A), enhancer factor I subunit A (EFI-A), BP-8, CSDA2, CSDB, MDR-NF1
<220>
   <221> CDS
   <222> (172) ... (1146)
   <223> YB1
<400> 36
   gggcttatcc cgcctgtccc gccattctcg ctagttcgat cggtagcggg agcggagagc 60
   ggaccccaga gagccctgag cagccccacc gccgccgccg gcctagttac catcacaccc 120
   cgggaggagc cgcagctgcc gcagccggcc ccagtcacca tcaccgcaac catgagcagc 180
   gaggccgaga cccagcagcc gcccgccgcc ccccccgccg cccccgccct cagcgccgcc 240
   gacaccaagc ccggcactac gggcagcggc gcagggagcg gtggcccggg cggcctcaca 300
   tcggcggcgc ctgccggcgg ggacaagaag gtcatcgcaa cgaaggtttt gggaacagta 360
   aaatggttca atgtaaggaa cggatatggt ttcatcaaca ggaatgacac caaggaagat 420
   gtatttgtac accagactgc cataaagaag aataacccca ggaagtacct tcgcagtgta 480
   ggagatggag agactgtgga gtttgatgtt gttgaaggag aaaagggtgc ggaggcagca 540
   aatgttacag gtcctggtgg tgttccagtt caaggcagta aatatgcagc agaccgtaac 600
   cattatagac gctatccacg tcgtaggggt cctccacgca attaccagca aaattaccag 660
   aatagtgaga gtggggaaaa gaacgaggga tcggagagtg ctcccgaagg ccaggcccaa 720
   caacgccggc cctaccgcag gcgaaggttc ccaccttact acatgcggag accctatggg 780
   cgtcgaccac agtattccaa ccctcctgtg cagggagaag tgatggaggg tgctgacaac 840
   cagggtgcag gagaacaagg tagaccagtg aggcagaata tgtatcgggg atatagacca 900
   cgattccgca ggggccctcc tcgccaaaga cagcctagag aggacggcaa tgaagaagat 960
   aaagaaaatc aaggagatga gacccaaggt cagcagccac ctcaacgtcg gtaccgccgc 1020
   aacttcaatt accgacgcag acgcccagaa aaccctaaac cacaagatgg caaagagaca 1080
   aaagcagccg atccaccagc tgagaattcg tccgctcccg aggctgagca gggcggggct 1140
   gagtaaatgc cggcttacca tctctaccat catccggttt agtcatccaa caagaagaaa 1200
   tatgaaattc cagcaataag aaatgaacaa aagattggag ctgaagacct aaagtgcttg 1260
   ctttttgccc gttgaccaga taaatagaac tatctgcatt atctatgcag catggggttt 1320
   ttattatttt tacctaaaga cgtctctttt tggtaataac aaacgtgttt tttaaaaaag 1380
   cctggttttt ctcaatacgc ctttaaaggt ttttaaattg tttcatatct ggtcaagttg 1440
   agatttttaa gaacttcatt tttaatttgt aataaaagtt tacaacttga ttttttcaaa 1500
   aaagtcaaca aactgcaagc acctgttaat aaaggtctta aataataaaa aaaaaaaaaa 1560
   a                                                                 1561
<210> 37
   <211> 4041
   <212> DNA
   <213> Homo sapiens
<220>
   <223> eukaryotic translation initiation factor 4B (EIF4B, EIF-4B), PRO1843
<220>
   <221> CDS
   <222> (207) ... (2042)
   <223> EIF4B
<400> 37
   ggccacatgt cgcgcatgtc ttcccgtcgg acggcgtgcc acctcgccgc gcagctttac 60
   gaacctagag cagcgccgcc ccgcctcctg tctccgtcct cacctccccg ccccctccca 120
   gcttcgcgtc tcctagctcg acgcgcccgc tataatcacg tgattgcctc atccgggtct 180
   tttgcgttct ctttccctct cccaacatgg cggcctcagc aaaaaagaag aataagaagg 240
   ggaagactat ctccctaaca gactttctgg ctgaggatgg gggtactggt ggaggaagca 300
   cctatgtttc caaaccagtc agctgggctg atgaaacgga tgacctggaa ggagatgttt 360
   cgaccacttg gcacagtaac gatgacgatg tgtatagggc gcctccaatt gaccgttcca 420
   tccttcccac tgctccacgg gctgctcggg aacccaatat cgaccggagc cgtcttccca 480
   aatcgccacc ctacactgct tttctaggaa acctacccta tgatgttaca gaagagtcaa 540
   ttaaggaatt ctttcgagga ttaaatatca gtgcagtgcg tttaccacgt gaacccagca 600
   atccagagag gttgaaaggt tttggttatg ctgaatttga ggacctggat tccctgctca 660
   gtgccctgag tctcaatgaa gagtctctag gtaacaggag aattcgagtg gacgttgctg 720
   atcaagcaca ggataaagac agggatgatc gttcttttgg ccgtgataga aatcgggatt 780
   ctgacaaaac agatacagac tggagggctc gtcctgctac agacagcttt gatgactacc 840
   cacctagaag aggtgatgat agctttggag acaagtatcg agatcgttat gattcagacc 900
   ggtatcggga tgggtatcgg gatgggtatc gggatggccc acgccgggat atggatcgat 960
   atggtggccg ggatcgctat gatgaccgag gcagcagaga ctatgataga ggctatgatt 1020
   cccggatagg cagtggcaga agagcatttg gcagtgggta tcgcagggat gatgactaca 1080
   gaggaggcgg ggaccgctat gaagaccgat atgacagacg ggatgatcgg tcgtggagct 1140
   ccagagatga ttactctcgg gatgattata ggcgtgatga tagaggtccc ccccaaagac 1200
   ccaaactgaa tctaaagcct cggagtactc ctaaggaaga tgattcctct gctagtacct 1260
   cccagtccac tcgagctgct tctatctttg gaggggcaaa gcctgttgac acagctgcta 1320
   gagaaagaga agtagaagaa cggctacaga aggaacaaga gaagttgcag cgtcagctgg 1380
   atgagccaaa actagaacga cggcctcggg agagacaccc aagctggcga agtgaagaaa 1440
   ctcaggaacg ggaacggtcg aggacaggaa gtgagtcatc acaaactggg acctccacca 1500
   catctagcag aaatgcacga aggagagaga gtgagaagtc tctagaaaat gaaacactca 1560
   ataaggagga agattgccac tctccaactt ctaaacctcc caaacctgat cagcccctaa 1620
   aggtaatgcc agcccctcca ccaaaggaga atgcttgggt gaagcgaagt tctaaccctc 1680
   ctgctcgatc tcagagctca gacacagagc agcagtcccc tacaagtggt gggggaaaag 1740
   tagctccagc tcaaccatct gaggaaggac caggaaggaa agatgaaaat aaagtagatg 1800
   ggatgaatgc cccaaaaggc caaactggga actctagccg tggtccagga gacggaggga 1860
   acagagacca ctggaaggag tcagatagga aagatggcaa aaaggatcaa gactccagat 1920
   ctgcacctga gccaaagaaa cctgaggaaa atccagcttc caagttcagt tctgcaagca 1980
   agtatgctgc tctctctgtt gatggtgaag atgaaaatga gggagaagat tatgccgaat 2040
   agacctctac atcctgtgct tttctcctag tttctctcca ccctggaaca ttcgagagca 2100
   aatcaaaacc tctatccaga caagacaaaa taaaactcac catctcctga agacctttct 2160
   tacctttttt taaaaacaaa aaatgaaatt attttgcatg ctgctgcagc ctttaaagta 2220
   ttgaagtaac tggagaattg ccaatacagc cagagagaaa gggactacag ctttttagag 2280
   gaaaagttgt ggtgcgttat gtcaccatgc agttgccagt gtgattagtg cctaggggtc 2340
   tccatttagc agaaatggta atgacagtga tataatgcct ggaacctggt tgggcagtag 2400
   gggagggagg tagaaggaaa agtgtgagat ttctaccttt tagtttttat cctattgtgg 2460
   catatatgaa ttctcaaaca ttatctgaat aaattttcca ctcttggaaa ggtagattta 2520
   gcctcaagtt gttctagtct ccaggaggct gccagcccct cctcttattt aattctgagt 2580
   tttgggggcc agcctagagg gaattccttt ttttttttta accccccagg ggggtagttg 2640
   ggagtgagac tataggccat aaagaatggg actgcattgg accaaaataa atgggaaaat 2700
   cgtggtttga aaagaagctt ttgggaagtg atgagtcatt ttgcaccagg taatagggga 2760
   aaattgtgtg acctccagca aacacatgaa tggttatttc ctggagccgg aagcacttgg 2820
   gggtcgtggt aattcccagt gttttctgtg tcctagtttt accctttcta aacactgtcc 2880
   tttttgaaag ttttgaatat atccacattc tattgaaacc ttgaaactaa aaatttagac 2940
   tcttatcatc atcttaagtt cttcatgcta ctcttaacct cccaaaaagc agtatctaag 3000
   tcacatacat gatgtcttgg gcattttctc agccatggag aactctgaaa ggaagaatcg 3060
   ctgcttttct caagcaaatc ggtttcttga tgtcttttgg ttctccttgc ctgctcctga 3120
   tgcttggacc ccttttattg atcagagtgc tctagaataa tggatggtct tggatgatgg 3180
   ataaataggg acagggacag ttaaattggg agcctttctt acaaccttga tgggattttt 3240
   ccccccaagt ttccttctcc actgaaatgc cacactaatg cttgttggat tcatgaggtg 3300
   gccagaccaa tgtgttgttt tgttgttgtt tttttaagct tcccttgaga gaataaatgg 3360
   taatggagag aactatttaa caaggtcctg gtttctcttg caacacagta gctaaacttg 3420
   cctgctttta tatgcatttt tgtagggatc agcttggtag acagtattag cggagaaaca 3480
   ccttgatctt ggtttgcaag cccttctccc atcagtccta gattaggccc tgttcagcca 3540
   tgcaggggtg ttggtttatg cgtgctgcag cagtgggcat aatgaatata atttacccag 3600
   tggacaaagg tgtgtaccaa gtgaatttaa ataattggtg tggattggcc agtagctaag 3660
   aagtgggctt ttaaagagta ttgaagattg aaagggtttt tctttctttt ttaaaaaaga 3720
   aaaacaaact attgattgta gataatgaaa agctagggtt tgccctcttc atgtctactc 3780
   tccttccaaa tagttatatc caaaactgtt tttccctctc ccctaccttg tcccccctat 3840
   taaaatagaa acagggattg attaatgtcc cgctcctgaa tacatgtaaa atttgtacaa 3900
   aaatatcttc tatgaaaatg atttgtaatc tgtagactta ttacctggga gatgtcttga 3960
   tgtaaaatcc catcctttgg gttgtgggtt ttttgttttc tccaaataaa tctgatcttt 4020
   aaagttcaaa aaaaaaaaaa a                                           4041
<210> 38
   <211> 511
   <212> DNA
   <213> Homo sapiens
<220>
   <223> 60S acidic ribosomal protein P2, LP2, P2, RPP2, D11S2243E, ribosomal protein, large, P2, acidic ribosomal phosphoprotein P2, renal carcinoma antigen NY-REN-44
<220>
   <221> CDS
   <222> (106) ... (453)
   <223> RPLP2
<400> 38
   ggtttaaccc cgcctcttgc gtcggcgcct tccttttcct ccctgtcgcc accgaggtcg 60
   cacgcgtgag acttctccgc cgcctccgcc gcagacgccg ccgcgatgcg ctacgtcgcc 120
   tcctacctgc tggctgccct agggggcaac tcctccccca gcgccaagga catcaagaag 180
   atcttggaca gcgtgggtat cgaggcggac gacgaccggc tcaacaaggt tatcagtgag 240
   ctgaatggaa aaaacattga agacgtcatt gcccagggta ttggcaagct tgccagtgta 300
   cctgctggtg gggctgtagc cgtctctgct gccccaggct ctgcagcccc tgctgctggt 360
   tctgcccctg ctgcagcaga ggagaagaaa gatgagaaga aggaggagtc tgaagagtca 420
   gatgatgaca tgggatttgg cctttttgat taaattcctg ctcccctgca aataaagcct 480
   ttttacacat ctcaaaaaaa aaaaaaaaaa a                                511
<210> 39
   <211> 603
   <212> DNA
   <213> Homo sapiens
<220>
   <223> 40S ribosomal protein S16, S16
<220>
   <221> CDS
   <222> (84) ... (524)
   <223> RPS16
<400> 39
   gaaaagcggc cagggtggcc cctagctttc cttttccggt tgcggcgccg cgcggtgagg 60
   ttgtctagtc cacgctcgga gccatgccgt ccaagggccc gctgcagtct gtgcaggtct 120
   tcggacgcaa gaagacagcg acagctgtgg cgcactgcaa acgcggcaat ggtctcatca 180
   aggtgaacgg gcggcccctg gagatgattg agccgcgcac gctacagtac aagctgctgg 240
   agccagttct gcttctcggc aaggagcgat ttgctggtgt agacatccgt gtccgtgtaa 300
   agggtggtgg tcacgtggcc cagatttatg ctatccgtca gtccatctcc aaagccctgg 360
   tggcctatta ccagaaatat gtggatgagg cttccaagaa ggagatcaaa gacatcctca 420
   tccagtatga ccggaccctg ctggtagctg accctcgtcg ctgcgagtcc aaaaagtttg 480
   gaggccctgg tgcccgcgct cgctaccaga aatcctaccg ataagcccat cgtgactcaa 540
   aactcacttg tataataaac agtttttgag ggattttaaa gtttcaagaa aaaaaaaaaa 600
   aaa                                                               603
<210> 40
   <211> 2151
   <212> DNA
   <213> Homo sapiens
<220>
   <223> vimentin (VIM), epididymis luminal protein 113 (HEL113), CTRCT30
<220>
   <221> CDS
   <222> (414) ... (1814)
   <223> VIM
<400> 40
   gcctctccaa aggctgcaga agtttcttgc taacaaaaag tccgcacatt cgagcaaaga 60
   caggctttag cgagttatta aaaacttagg ggcgctcttg tcccccacag ggcccgaccg 120
   cacacagcaa ggcgatggcc cagctgtaag ttggtagcac tgagaactag cagcgcgcgc 180
   ggagcccgct gagacttgaa tcaatctggt ctaacggttt cccctaaacc gctaggagcc 240
   ctcaatcggc gggacagcag ggcgcgtcct ctgccactct cgctccgagg tccccgcgcc 300
   agagacgcag ccgcgctccc accacccaca cccaccgcgc cctcgttcgc ctcttctccg 360
   ggagccagtc cgcgccaccg ccgccgccca ggccatcgcc accctccgca gccatgtcca 420
   ccaggtccgt gtcctcgtcc tcctaccgca ggatgttcgg cggcccgggc accgcgagcc 480
   ggccgagctc cagccggagc tacgtgacta cgtccacccg cacctacagc ctgggcagcg 540
   cgctgcgccc cagcaccagc cgcagcctct acgcctcgtc cccgggcggc gtgtatgcca 600
   cgcgctcctc tgccgtgcgc ctgcggagca gcgtgcccgg ggtgcggctc ctgcaggact 660
   cggtggactt ctcgctggcc gacgccatca acaccgagtt caagaacacc cgcaccaacg 720
   agaaggtgga gctgcaggag ctgaatgacc gcttcgccaa ctacatcgac aaggtgcgct 780
   tcctggagca gcagaataag atcctgctgg ccgagctcga gcagctcaag ggccaaggca 840
   agtcgcgcct gggggacctc tacgaggagg agatgcggga gctgcgccgg caggtggacc 900
   agctaaccaa cgacaaagcc cgcgtcgagg tggagcgcga caacctggcc gaggacatca 960
   tgcgcctccg ggagaaattg caggaggaga tgcttcagag agaggaagcc gaaaacaccc 1020
   tgcaatcttt cagacaggat gttgacaatg cgtctctggc acgtcttgac cttgaacgca 1080
   aagtggaatc tttgcaagaa gagattgcct ttttgaagaa actccacgaa gaggaaatcc 1140
   aggagctgca ggctcagatt caggaacagc atgtccaaat cgatgtggat gtttccaagc 1200
   ctgacctcac ggctgccctg cgtgacgtac gtcagcaata tgaaagtgtg gctgccaaga 1260
   acctgcagga ggcagaagaa tggtacaaat ccaagtttgc tgacctctct gaggctgcca 1320
   accggaacaa tgacgccctg cgccaggcaa agcaggagtc cactgagtac cggagacagg 1380
   tgcagtccct cacctgtgaa gtggatgccc ttaaaggaac caatgagtcc ctggaacgcc 1440
   agatgcgtga aatggaagag aactttgccg ttgaagctgc taactaccaa gacactattg 1500
   gccgcctgca ggatgagatt cagaatatga aggaggaaat ggctcgtcac cttcgtgaat 1560
   accaagacct gctcaatgtt aagatggccc ttgacattga gattgccacc tacaggaagc 1620
   tgctggaagg cgaggagagc aggatttctc tgcctcttcc aaacttttcc tccctgaacc 1680
   tgagggaaac taatctggat tcactccctc tggttgatac ccactcaaaa aggacacttc 1740
   tgattaagac ggttgaaact agagatggac aggttatcaa cgaaacttct cagcatcacg 1800
   atgaccttga ataaaaattg cacacactca gtgcagcaat atattaccag caagaataaa 1860
   aaagaaatcc atatcttaaa gaaacagctt tcaagtgcct ttctgcagtt tttcaggagc 1920
   gcaagataga tttggaatag gaataagctc tagttcttaa caaccgacac tcctacaaga 1980
   tttagaaaaa agtttacaac ataatctagt ttacagaaaa atcttgtgct agaatacttt 2040
   ttaaaaggta ttttgaatac cattaaaact gctttttttt ttccagcaag tatccaacca 2100
   acttggttct gcttcaataa atctttggaa aaactcaaaa aaaaaaaaaa a          2151
<210> 41
   <211> 1787
   <212> DNA
   <213> Homo sapiens
<220>
   <223> guanidinoacetate N-methyltransferase transcript variant 2 (GAMT), epididymis secretory protein Li 20 (HEL-S-20), CCDS2, PIG2, TP53I2
<220>
   <221> CDS
   <222> (95) ... (904)
   <223> GAMT
<400> 41
   gggcctgttg gacccgcccc cggcccacaa gcccctgcag ggagcgggcc cgggcggcgc 60
   gcgatcgagg tcgggtcgcc gtccagcctg cagcatgagc gcccccagcg cgacccccat 120
   cttcgcgccc ggcgagaact gcagccccgc gtggggggcg gcgcccgcgg cctacgacgc 180
   agcggacacg cacctgcgca tcctgggcaa gccggtgatg gagcgctggg agacccccta 240
   tatgcacgcg ctggccgccg ccgcctcctc caaagggggc cgggtcctgg aggtgggctt 300
   tggcatggcc atcgcagcgt caaaggtgca ggaggcgccc attgatgagc attggatcat 360
   cgagtgcaat gacggcgtct tccagcggct ccgggactgg gccccacggc agacacacaa 420
   ggtcatcccc ttgaaaggcc tgtgggagga tgtggcaccc accctgcctg acggtcactt 480
   tgatgggatc ctgtacgaca cgtacccact ctcggaggag acctggcaca cacaccagtt 540
   caacttcatc aagaaccacg cctttcgcct gctgaagccg gggggcgtcc tcacctactg 600
   caacctcacc tcctgggggg agctgatgaa gtccaagtac tcagacatca ccatcatgtt 660
   tgaggtgcgc ccacctgaag ttccccatgg gtctccagga agtgaccttg gatgggggtg 720
   ggaaggggct gctggagcca ccttgctacc tggggagggt cccttcctga ccccctgggt 780
   gggctggact gtgctggttc atttagaaat caaagtcctt tgcctggcgc agtggctgcc 840
   aggagcagtg gctcaggtct ataatcccag cactgtggaa ggccgaggtg ggcagattgc 900
   ttgagcctag gagttcaaga ccagcctggg ctacagagca agacctcatc tttcctaaaa 960
   aaaaaaaata caaaaaacta gctgggcgtg gtggcgtgtg cctgtactcc cagctacttg 1020
   ggaggctgag gtaggaggat tgcttgggcc ccaggggcag aggttgcagt gagcccagat 1080
   cgaaccactg cactccagcc tgggtaacag agccagaccc tgtctcaaga aaaaagaaag 1140
   aaagaaagaa agaaagaaaa tttaaaaaaa aaatggaaat caggctgggc gtggtggctc 1200
   acacctgtga tcccggcact tcggaaggcc aaggcgggtg gatcacttga gcccaggagt 1260
   ttgagcctag cctggccaac atggcagaat cccatctcta ctaaaaatac aaaagttagc 1320
   caggcgtggt ggcgggcgcc tgtaattcca gctactcggg aggctgaggc atgagaatca 1380
   cttgaacatg ggaggcggag gttgctgtga gccgagattg caccgttgta ctccagcctg 1440
   ggcaacagac cgagactcca tctcaacaaa acaaaaacaa aaaaaatgga aatcaaagtc 1500
   cctcaccagc tcaacaacct cccatggctc cccagtgccc tgtggttcag cctaagccct 1560
   gcgtgttccc ctccctccag ctgcctccac ctggctgtct ttgctggttc agcagtgctt 1620
   gtctcgctgt tccctctgcc tggtggaggc ggcctatctg agaagggctc tatgtggttg 1680
   ccctgggctg ctgttgtgag agccggctgg gtgcctgtgg cccctggggc agcttttctt 1740
   ccaaaatggg aactagtggc ctgtgttcta aaaaaaaaaa aaaaaaa              1787
<210> 42
   <211> 2331
   <212> DNA
   <213> Homo sapiens
<220>
   <223> heat shock 70kDa protein 8 transcript variant 1 (HSPA8), lipopolysaccharide-associated protein 1 (LAP1), epididymis secretory sperm binding protein Li 72p (HEL-S-72p), N-myristoyltransferase inhibitor protein 71 (NIP71)
<220>
   <221> CDS
   <222> (136) ... (2076)
   <223> HSPA8
<400> 42
   ccttctggaa ggttctaaga tagggtataa gaggcagggt ggcgggcgga aaccggtctc 60
   attgaactcg cctgcagctc ttgggttttt tgtggcttcc ttcgttattg gagccaggcc 120
   tacaccccag caaccatgtc caagggacct gcagttggta ttgatcttgg caccacctac 180
   tcttgtgtgg gtgttttcca gcacggaaaa gtcgagataa ttgccaatga tcagggaaac 240
   cgaaccactc caagctatgt cgcctttacg gacactgaac ggttgatcgg tgatgccgca 300
   aagaatcaag ttgcaatgaa ccccaccaac acagtttttg atgccaaacg tctgattgga 360
   cgcagatttg atgatgctgt tgtccagtct gatatgaaac attggccctt tatggtggtg 420
   aatgatgctg gcaggcccaa ggtccaagta gaatacaagg gagagaccaa aagcttctat 480
   ccagaggagg tgtcttctat ggttctgaca aagatgaagg aaattgcaga agcctacctt 540
   gggaagactg ttaccaatgc tgtggtcaca gtgccagctt actttaatga ctctcagcgt 600
   caggctacca aagatgctgg aactattgct ggtctcaatg tacttagaat tattaatgag 660
   ccaactgctg ctgctattgc ttacggctta gacaaaaagg ttggagcaga aagaaacgtg 720
   ctcatctttg acctgggagg tggcactttt gatgtgtcaa tcctcactat tgaggatgga 780
   atctttgagg tcaagtctac agctggagac acccacttgg gtggagaaga ttttgacaac 840
   cgaatggtca accattttat tgctgagttt aagcgcaagc ataagaagga catcagtgag 900
   aacaagagag ctgtaagacg cctccgtact gcttgtgaac gtgctaagcg taccctctct 960
   tccagcaccc aggccagtat tgagatcgat tctctctatg aaggaatcga cttctatacc 1020
   tccattaccc gtgcccgatt tgaagaactg aatgctgacc tgttccgtgg caccctggac 1080
   ccagtagaga aagcccttcg agatgccaaa ctagacaagt cacagattca tgatattgtc 1140
   ctggttggtg gttctactcg tatccccaag attcagaagc ttctccaaga cttcttcaat 1200
   ggaaaagaac tgaataagag catcaaccct gatgaagctg ttgcttatgg tgcagctgtc 1260
   caggcagcca tcttgtctgg agacaagtct gagaatgttc aagatttgct gctcttggat 1320
   gtcactcctc tttcccttgg tattgaaact gctggtggag tcatgactgt cctcatcaag 1380
   cgtaatacca ccattcctac caagcagaca cagaccttca ctacctattc tgacaaccag 1440
   cctggtgtgc ttattcaggt ttatgaaggc gagcgtgcca tgacaaagga taacaacctg 1500
   cttggcaagt ttgaactcac aggcatacct cctgcacccc gaggtgttcc tcagattgaa 1560
   gtcacttttg acattgatgc caatggtata ctcaatgtct ctgctgtgga caagagtacg 1620
   ggaaaagaga acaagattac tatcactaat gacaagggcc gtttgagcaa ggaagacatt 1680
   gaacgtatgg tccaggaagc tgagaagtac aaagctgaag atgagaagca gagggacaag 1740
   gtgtcatcca agaattcact tgagtcctat gccttcaaca tgaaagcaac tgttgaagat 1800
   gagaaacttc aaggcaagat taacgatgag gacaaacaga agattctgga caagtgtaat 1860
   gaaattatca actggcttga taagaatcag actgctgaga aggaagaatt tgaacatcaa 1920
   cagaaagagc tggagaaagt ttgcaacccc atcatcacca agctgtacca gagtgcagga 1980
   ggcatgccag gaggaatgcc tgggggattt cctggtggtg gagctcctcc ctctggtggt 2040
   gcttcctcag ggcccaccat tgaagaggtt gattaagcca accaagtgta gatgtagcat 2100
   tgttccacac atttaaaaca tttgaaggac ctaaattcgt agcaaattct gtggcagttt 2160
   taaaaagtta agctgctata gtaagttact gggcattctc aatacttgaa tatggaacat 2220
   atgcacaggg gaaggaaata acattgcact ttataaacac tgtattgtaa gtggaaaatg 2280
   caatgtctta aataaaacta tttaaaattg gcaccataaa aaaaaaaaaa a          2331
<210> 43
   <211> 401
   <212> DNA
   <213> Homo sapiens
<220>
   <223> 60S ribosomal protein L39, L39, RPL39P42, RPL39_23_1806
<220>
   <221> CDS
   <222> (68) ... (223)
   <223> RPL39
<400> 43
   cttccgccag cttccctcct cttcctttct ccgccatcgt ggtgtgttct tgactccgct 60
   gctcgccatg tcttctcaca agactttcag gattaagcga ttcctggcca agaaacaaaa 120
   gcaaaatcgt cccattcccc agtggattcg gatgaaaact ggaaataaaa tcaggtacaa 180
   ctccaaaagg agacattgga gaagaaccaa gctgggtcta taaggaattg cacatgagat 240
   ggcacacata tttatgctgt ctgaaggtca cgatcatgtt accatatcaa gctgaaaatg 300
   tcaccactat ctggagattt cgacgtgttt tcctctctga atctgttatg aacacgttgg 360
   ttggctggat tcagtaataa atatgtaagg cctttctttt t                     401
<210> 44
   <211> 2211
   <212> DNA
   <213> Homo sapiens
<220>
   <223> adenosylhomocysteinase transcript variant 1 (AHCY, adoHcyase), S-adenosylhomocysteine hydrolase, S-adenosyl-L-homocysteine hydrolase (SAHH)
<220>
   <221> CDS
   <222> (140) ... (1438)
   <223> AHCY
<400> 44
   atgaatatgc aagtgcgagg aagatattta aaggcgtcgg cgccacgcgc atatccctgc 60
   tcggcgctgc ccgcccagtt cctgttccca gactgaggcc cagccccctt cgcccgtttc 120
   catcacgagt gccgccagca tgtctgacaa actgccctac aaagtcgccg acatcggcct 180
   ggctgcctgg ggacgcaagg ccctggacat tgctgagaac gagatgccgg gcctgatgcg 240
   tatgcgggag cggtactcgg cctccaagcc actgaagggc gcccgcatcg ctggctgcct 300
   gcacatgacc gtggagacgg ccgtcctcat tgagaccctc gtcaccctgg gtgctgaggt 360
   gcagtggtcc agctgcaaca tcttctccac ccaggaccat gcggcggctg ccattgccaa 420
   ggctggcatt ccggtgtatg cctggaaggg cgaaacggac gaggagtacc tgtggtgcat 480
   tgagcagacc ctgtacttca aggacgggcc cctcaacatg attctggacg acgggggcga 540
   cctcaccaac ctcatccaca ccaagtaccc gcagcttctg ccaggcatcc gaggcatctc 600
   tgaggagacc acgactgggg tccacaacct ctacaagatg atggccaatg ggatcctcaa 660
   ggtgcctgcc atcaatgtca atgactccgt caccaagagc aagtttgaca acctctatgg 720
   ctgccgggag tccctcatag atggcatcaa gcgggccaca gatgtgatga ttgccggcaa 780
   ggtagcggtg gtagcaggct atggtgatgt gggcaagggc tgtgcccagg ccctgcgggg 840
   tttcggagcc cgcgtcatca tcaccgagat tgaccccatc aacgcactgc aggctgccat 900
   ggagggctat gaggtgacca ccatggatga ggcctgtcag gagggcaaca tctttgtcac 960
   caccacaggc tgtattgaca tcatccttgg ccggcacttt gagcagatga aggatgatgc 1020
   cattgtgtgt aacattggac actttgacgt ggagatcgat gtcaagtggc tcaacgagaa 1080
   cgccgtggag aaggtgaaca tcaagccgca ggtggaccgg tatcggttga agaatgggcg 1140
   ccgcatcatc ctgctggccg agggtcggct ggtcaacctg ggttgtgcca tgggccaccc 1200
   cagcttcgtg atgagtaact ccttcaccaa ccaggtgatg gcgcagatcg agctgtggac 1260
   ccatccagac aagtaccccg ttggggttca tttcctgccc aagaagctgg atgaggcagt 1320
   ggctgaagcc cacctgggca agctgaatgt gaagttgacc aagctaactg agaagcaagc 1380
   ccagtacctg ggcatgtcct gtgatggccc cttcaagccg gatcactacc gctactgaga 1440
   gccaggtctg cgtttcaccc tccagctgct gtccttgccc aggccccacc tctcctccct 1500
   aagagctaat ggcaccaact ttgtgattgg tttgtcagtg tcccccatcg actctctggg 1560
   gctgatcact tagtttttgg cctctgctgc agccgtcata ctgttccaaa tgtggcagcg 1620
   ggaacagagt accctcttca agccccggtc atgatggagg tcccagccac agggaaccat 1680
   gagctcagtg gtcttggaac agctcactaa gtcagtcctt ccttagcctg gaagtcagta 1740
   gtggagtcac aaagcccatg tgttttgcca tctaggcctt cacctggtct gtggacttat 1800
   acctgtgtgc ttggtttaca ggtccagtgg ttcttcagcc catgacagat gagaaggggc 1860
   tatattgaag ggcaaagagg aactgttgtt tgaattttcc tgagagcctg gcttagtgct 1920
   gggccttctc ttaaacctca ttacaatgag gttagtactt ttagtccctg ttttacaggg 1980
   gttagaatag actgttaagg ggcaactgag aaagaacaga gaagtgacag ctaggggttg 2040
   agagggccca gaaaaacatg aatgcaggca gatttcgtga aatctgccac cactttataa 2100
   ccagatggtt cctttcacaa ccctgggtca aaaagagaat aatttggcct ataatgttaa 2160
   aagaaagcag gaaggtgggt aaataaaaat cttggtgcct ggaagaaaaa a 2211
<210> 45
   <211> 2022
   <212> DNA
   <213> Homo sapiens
<220>
   <223> eukaryotic translation elongation factor 1 alpha 2, eukaryotic elongation factor 1 A-2 (EEF1A2, EF-1-alpha-2, eEF1A-2, EEF1AL), elongation factor-1 alpha (EF1A), statin S1 (HS1, STN, STNL)
<220>
   <221> CDS
   <222> (330) ... (1721)
   <223> EEF1A2
<400> 45
   gccccgcccc cgcccgcggc gcgtttctcc cccgcctccc gcgtccgtct ttgcagcccg 60
   cgcctcccgc atcgcctcgc gtccccgtgg cgcccgcccg cgcgcgtccg cgccccgccc 120
   cctcccgcgc ggttccgcat tggcgtgctg cagggcgcgg tgcactgcgc cgccaccgtc 180
   aataggtgga ccccctcccg gagataaaac cgccggcgcc ggcgccgcca gtccctctgg 240
   ctgagacctc ggctccggaa tcactgcagc ccccctcgcc ctgagccaga gcaccccggg 300
   tcccgccagc ccctcacact cccagcaaaa tgggcaagga gaagacccac atcaacatcg 360
   tggtcatcgg ccacgtggac tccggaaagt ccaccaccac gggccacctc atctacaaat 420
   gcggaggtat tgacaaaagg accattgaga agttcgagaa ggaggcggct gagatgggga 480
   agggatcctt caagtatgcc tgggtgctgg acaagctgaa ggcggagcgt gagcgcggca 540
   tcaccatcga catctccctc tggaagttcg agaccaccaa gtactacatc accatcatcg 600
   atgcccccgg ccaccgcgac ttcatcaaga acatgatcac gggtacatcc caggcggact 660
   gcgcagtgct gatcgtggcg gcgggcgtgg gcgagttcga ggcgggcatc tccaagaatg 720
   ggcagacgcg ggagcatgcc ctgctggcct acacgctggg tgtgaagcag ctcatcgtgg 780
   gcgtgaacaa aatggactcc acagagccgg cctacagcga gaagcgctac gacgagatcg 840
   tcaaggaagt cagcgcctac atcaagaaga tcggctacaa cccggccacc gtgccctttg 900
   tgcccatctc cggctggcac ggtgacaaca tgctggagcc ctcccccaac atgccgtggt 960
   tcaagggctg gaaggtggag cgtaaggagg gcaacgcaag cggcgtgtcc ctgctggagg 1020
   ccctggacac catcctgccc cccacgcgcc ccacggacaa gcccctgcgc ctgccgctgc 1080
   aggacgtgta caagattggc ggcattggca cggtgcccgt gggccgggtg gagaccggca 1140
   tcctgcggcc gggcatggtg gtgacctttg cgccagtgaa catcaccact gaggtgaagt 1200
   cagtggagat gcaccacgag gctctgagcg aagctctgcc cggcgacaac gtcggcttca 1260
   atgtgaagaa cgtgtcggtg aaggacatcc ggcggggcaa cgtgtgtggg gacagcaagt 1320
   ctgacccgcc gcaggaggct gctcagttca cctcccaggt catcatcctg aaccacccgg 1380
   ggcagattag cgccggctac tccccggtca tcgactgcca cacagcccac atcgcctgca 1440
   agtttgcgga gctgaaggag aagattgacc ggcgctctgg caagaagctg gaggacaacc 1500
   ccaagtccct gaagtctgga gacgcggcca tcgtggagat ggtgccggga aagcccatgt 1560
   gtgtggagag cttctcccag tacccgcctc tcggccgctt cgccgtgcgc gacatgaggc 1620
   agacggtggc cgtaggcgtc atcaagaacg tggagaagaa gagcggcggc gccggcaagg 1680
   tcaccaagtc ggcgcagaag gcgcagaagg cgggcaagtg aagcgcgggc gcccgcggcg 1740
   cgaccctccc cggcggcgcc gcgctccgaa ccccggcccg gcccccgccc cgcccccgcc 1800
   ccgcgcgccg ctccggcgcc ccgcaccccc gccaggcgca tgtctgcacc tccgcttgcc 1860
   agaggccctc ggtcagcgac tggatgctcg ccatcaaggt ccagtggaag ttcttcaaga 1920
   ggaaaggcgc ccccgcccca ggcttccgcg cccagcgctc gccacgctca gtgcccgttt 1980
   taccaataaa ctgagcgacc ccagaaaaaa aaaaaaaaaa aa 2022
<210> 46
   <211> 719
   <212> DNA
   <213> Homo sapiens
<220>
   <223> 60S ribosomal protein L10a, L10A, neural precursor cell expressed developmentally down-regulated protein 6 (NEDD6), Csa-19
<220>
   <221> CDS
   <222> (35) ... (688)
   <223> RPL10A
<400> 46
   agtctctttt ccggttagcg cggcgtgaga agccatgagc agcaaagtct ctcgcgacac 60
   cctgtacgag gcggtgcggg aagtcctgca cgggaaccag cgcaagcgcc gcaagttcct 120
   ggagacggtg gagttgcaga tcagcttgaa gaactatgat ccccagaagg acaagcgctt 180
   ctcgggcacc gtcaggctta agtccactcc ccgccctaag ttctctgtgt gtgtcctggg 240
   ggaccagcag cactgtgacg aggctaaggc cgtggatatc ccccacatgg acatcgaggc 300
   gctgaaaaaa ctcaacaaga ataaaaaact ggtcaagaag ctggccaaga agtatgatgc 360
   gtttttggcc tcagagtctc tgatcaagca gattccacga atcctcggcc caggtttaaa 420
   taaggcagga aagttccctt ccctgctcac acacaacgaa aacatggtgg ccaaagtgga 480
   tgaggtgaag tccacaatca agttccaaat gaagaaggtg ttatgtctgg ctgtagctgt 540
   tggtcacgtg aagatgacag acgatgagct tgtgtataac attcacctgg ctgtcaactt 600
   cttggtgtca ttgctcaaga aaaactggca gaatgtccgg gccttatata tcaagagcac 660
   catgggcaag ccccagcgcc tatattaagg cacatttgaa taaattctat taccagttc  719
<210> 47
   <211> 3230
   <212> DNA
   <213> Homo sapiens
<220>
   <223> poly(A) binding protein, cytoplasmic 4 (inducible form) transcript variant 1, inducible poly(A)-binding protein, poly(A)-binding protein 4, polyadenylate-binding protein 4 (PABPC4, PABP-4, iPABP), activated-platelet protein 1 (APP-1, APP1)
<220>
   <221> CDS
   <222> (899) ... (2881)
   <223> PABPC4
<400> 47
   ttcgaccagc agcgcccggg gcgggcgggt ataaatggag cggtggctcc ctcggccgcc 60
   tctctccgcc ccgggtcgct gccgcctccg ccgctttcgg gcttcgcagc ctgaggaaaa 120
   aaagagaaaa agataaaaaa aatctgaaaa cgcttcaaaa tcctgaaaaa aaaaaaggaa 180
   aagaaaaaac gaatcctcgg agaacccgcg gggaagtcac tttcgtacgc ttccggcctg 240
   ccccgcgccc gccgccgcag cgcttggcgt ccgtcggtct ccgtccgtcg gtccgggggt 300
   gagccgcccg cccggcccgc cgtgccctcc ccccgctcgg gccccgagcc ccgcgccccg 360
   cgcctgcccc ggcgcaccac gtgtccgtgc tgcccttcgc cgcccgcccg gggctcgccg 420
   agtcggcgcc cacaaagatt tggtttccct ctgccccggc ggttgtaatc ttaaaccgcc 480
   ggagcccgag gcctatattt atagagaaac gcgtgtcccc gaggccgccg tgggcagcgt 540
   ccggtcgcct cttaaaggat ttttaccctt cggaagggga ttccccgttt aatttttttc 600
   ctactttgat tttttgaaat ttggagcttc gcaccaggac cgcggagaag tgcaaagtcg 660
   cggggagggc cgtattgtgc ggagagcctt ttgtctgcgg tgctgcggcc gtgggagccg 720
   gcccccgcct cccgtttccg tcccgtctcc aagcccgccg actccagctc gtcctcgccg 780
   cgccggtgcc acctgtgagc cgcggcgcgg gcccgggctc cgaaggcgcc cctttgtcct 840
   gcggcgggcc cgataagaag tcctcctggc ggggctcggg gtggtggggg gcggggagat 900
   gaacgctgcg gccagcagct accccatggc ctccctgtac gtgggcgacc tgcattcgga 960
   cgtcaccgag gccatgctgt acgaaaagtt cagccccgcg gggcctgtgc tgtccatccg 1020
   ggtctgccgc gatatgatca cccgccgctc cctgggctat gcctacgtca acttccagca 1080
   gccggccgac gctgagcggg ctttggacac catgaacttt gatgtgatta agggaaagcc 1140
   aatccgcatc atgtggtctc agagggatcc ctctttgaga aaatctggtg tgggaaacgt 1200
   cttcatcaag aacctggaca aatctataga taacaaggca ctttatgata ctttttctgc 1260
   ttttggaaac atactgtcct gcaaggtggt gtgtgatgag aacggctcta agggttatgc 1320
   ctttgtccac ttcgagaccc aagaggctgc cgacaaggcc atcgagaaga tgaatggcat 1380
   gctcctcaat gaccgcaaag tatttgtggg cagattcaag tctcgcaaag agcgggaagc 1440
   tgagcttgga gccaaagcca aggaattcac caatgtttat atcaaaaact ttggggaaga 1500
   ggtggatgat gagagtctga aagagctatt cagtcagttt ggtaagaccc taagtgtcaa 1560
   ggtgatgaga gatcccaatg ggaaatccaa aggctttggc tttgtgagtt acgaaaaaca 1620
   cgaggatgcc aataaggctg tggaagagat gaatggaaaa gaaataagtg gtaaaatcat 1680
   atttgtaggc cgtgcacaaa agaaagtaga acggcaggca gagttaaaac ggaaatttga 1740
   acagttgaaa caggagagaa ttagtcgata tcagggggtg aatctctaca ttaagaactt 1800
   ggatgacact attgatgatg agaaattaag gaaagaattt tctccttttg gatcaattac 1860
   cagtgctaag gtaatgctgg aggatggaag aagcaaaggg tttggcttcg tctgcttctc 1920
   atctcctgaa gaagcaacca aagcagtcac tgagatgaat ggacgcattg tgggctccaa 1980
   gccactatat gttgccctgg cccagaggaa ggaagagaga aaggctcacc tgaccaacca 2040
   gtatatgcaa cgagtggctg gaatgagagc acttcctgcc aatgccatct taaatcagtt 2100
   ccagcctgca gcgggtggct actttgtgcc agcagtccca caggctcagg gaaggcctcc 2160
   atattataca cctaaccagt tagcacagat gaggcctaat ccacgctggc agcaaggtgg 2220
   gagacctcaa ggcttccaag gaatgccaag tgctatacgc cagtctgggc ctcgtccaac 2280
   tcttcgccat ctggctccaa ctggtaatgc tccggcctct cgtggcctcc ctactaccac 2340
   tcagagagtc gggtctgagt gcccggaccg cttggctatg gactttggtg gggctggtgc 2400
   cgcccagcaa gggctgactg acagctgcca gtctggaggc gttcccacag ctgtgcagaa 2460
   cttagcgcca cgcgctgctg ttgctgctgc tgctccccgg gctgttgccc cctacaaata 2520
   cgcctccagt gtccgcagcc ctcatcctgc catacagcct ctgcaggcac cccagcctgc 2580
   ggtccatgtg caggggcagg agccactgac tgcctccatg ctggctgcag caccccccca 2640
   ggaacagaag cagatgctgg gagaacgctt gttcccactc atccaaacaa tgcattcaaa 2700
   tctggctggg aagatcacgg gaatgctgct ggagatagac aactctgagc tgctgcacat 2760
   gttagagtcc cccgagtctc tccgctccaa ggtggatgaa gctgtagcag ttctacaggc 2820
   tcatcatgcc aagaaagaag ctgcccagaa ggtgggcgct gttgctgctg ctacctctta 2880
   gacaaggaaa aaccgattca aaagccaaat aaccccttat ggaattcaac tcaaggtttg 2940
   aagacttcct agcttgtcct atggacctca acaccaagga ttacaaattg caaatttaat 3000
   aggtcatttt gtatcaaaag gtcaattatg aagcacctag aatttttcaa ttatacgaat 3060
   atgttctttg ggttctgctg tggcccagac agtgttaact ttttttttat tgtgggtttt 3120
   gattttttcc cccagaaatt ggttttattt gatgtaccca agtcttacgt ttcccaataa 3180
   agaaaaaaaa tctccataaa actgaaaaaa aaaaaaaaaa aaaaaaaaaa           3230
<210> 48
   <211> 956
   <212> DNA
   <213> Homo sapiens
<220>
   <223> 40S ribosomal protein S4, X-linked, ribosomal protein S4X, S4X, single copy abundant mRNA protein (SCAR), cell cycle gene 2 (CCG2), DXS306, SCR10
<220>
   <221> CDS
   <222> (97) ... (888)
   <223> RPS4X
<400> 48
   gggcggagca gctgaaaatc cggcgcgcgc agtctccagc cccaatttct acgcgcaccg 60
   gaagacggag gtcctctttc cttgcctaac gcagccatgg ctcgtggtcc caagaagcat 120
   ctgaagcggg tggcagctcc aaagcattgg atgctggata aattgaccgg tgtgtttgct 180
   cctcgtccat ccaccggtcc ccacaagttg agagagtgtc tccccctcat cattttcctg 240
   aggaacagac ttaagtatgc cctgacagga gatgaagtaa agaagatttg catgcagcgg 300
   ttcattaaaa tcgatggcaa ggtccgaact gatataacct accctgctgg attcatggat 360
   gtcatcagca ttgacaagac gggagagaat ttccgtctga tctatgacac caagggtcgc 420
   tttgctgtac atcgtattac acctgaggag gccaagtaca agttgtgcaa agtgagaaag 480
   atctttgtgg gcacaaaagg aatccctcat ctggtgactc atgatgcccg caccatccgc 540
   taccccgatc ccctcatcaa ggtgaatgat accattcaga ttgatttgga gactggcaag 600
   attactgatt tcatcaagtt cgacactggt aacctgtgta tggtgactgg aggtgctaac 660
   ctaggaagaa ttggtgtgat caccaacaga gagaggcacc ctggatcttt tgacgtggtt 720
   cacgtgaaag atgccaatgg caacagcttt gccactcgac tttccaacat ttttgttatt 780
   ggcaagggca acaaaccatg gatttctctt ccccgaggaa agggtatccg cctcaccatt 840
   gctgaagaga gagacaaaag actggcggcc aaacagagca gtgggtgaaa tgggtccctg 900
   ggtgacatgt cagatctttg tacgtaatta aaaatattgt ggcaggatta atagca     956
<210> 49
   <211> 1129
   <212> DNA
   <213> Homo sapiens
<220>
   <223> 60S ribosomal protein L31 transcript variant 2, L31
<220>
   <221> CDS
   <222> (88) ... (474)
   <223> RPL31
<400> 49
   tggcgacccg gaagttgtac ttgcaactgc ggctttcctt ctcccacaat ccttcgcgct 60
   cttcctttcc aacttggacg ctgcagaatg gctcccgcaa agaagggtgg cgagaagaaa 120
   aagggccgtt ctgccatcaa cgaagtggta acccgagaat acaccatcaa cattcacaag 180
   cgcatccatg gagtgggctt caagaagcgt gcacctcggg cactcaaaga gattcggaaa 240
   tttgccatga aggagatggg aactccagat gtgcgcattg acaccaggct caacaaagct 300
   gtctgggcca aaggaataag gaatgtgcca taccgaatcc gtgtgcggct gtccagaaaa 360
   cgtaatgagg atgaagattc accaaataag ctatatactt tggttaccta tgtacctgtt 420
   accactttca aaatttctgt gctaaacagt gttacagtcg ccaagagccc ataaagggag 480
   ccctcctgga agtggatgag gccttgggtc tcggctcttc attgcttcct gagctgcagc 540
   agatgccttt acaaccaagc tcaccgagga cgtctgtctc ccatattacc ctggcagagg 600
   gccaggcctg ttctacacgg ccggggtttc aacaaggtac tgatgtcttc tgcccttgcc 660
   tcttcgacag gcaagtaata agacttaagt gaagagaatt ctttaggcac acaaattcac 720
   atttgatgta atctcattat acttcctgat ctgtgattga aaactttcat ttcgtaacta 780
   gtatgtctgt cccaccttta aaaagttttt cattatgaaa gtaagtattt gttagaatta 840
   agtctattta aatgaaaaaa acttagatat gagtctgcat ggcctcagga aaatgatgtt 900
   ttaaaataga gattttaggt tgtctgcact ctagcttttt tgtcgttttc ttaaggcttt 960
   tttaactgca tcaaaaattc agatacgaaa catacactaa aaaataatac atcatatctt 1020
   aatttccact gaacttgatt taaattcaga gttacacagt atgaatatca caatcagata 1080
   tgttcaaaaa ggtctgaaca attgattttc tgaaaccatg aaggactac 1129
<210> 50
   <211> 512
   <212> DNA
   <213> Homo sapiens
<220>
   <223> 60S acidic ribosomal protein P1 transcript variant 1, LP1, P1, RPP1, ribosomal protein, large, P1, acidic ribosomal phosphoprotein P1
<220>
   <221> CDS
   <222> (130) ... (474)
   <223> RPLP1
<400> 50
   cctttcctca gctgccgcca aggtgctcgg tccttccgag gaagctaagg ctgcgttggg 60
   gtgaggccct cacttcatcc ggcgactagc accgcgtccg gcagcgccag ccctacactc 120
   gcccgcgcca tggcctctgt ctccgagctc gcctgcatct actcggccct cattctgcac 180
   gacgatgagg tgacagtcac ggaggataag atcaatgccc tcattaaagc agccggtgta 240
   aatgttgagc ctttttggcc tggcttgttt gcaaaggccc tggccaacgt caacattggg 300
   agcctcatct gcaatgtagg ggccggtgga cctgctccag cagctggtgc tgcaccagca 360
   ggaggtcctg ccccctccac tgctgctgct ccagctgagg agaagaaagt ggaagcaaag 420
   aaagaagaat ccgaggagtc tgatgatgac atgggctttg gtctttttga ctaaacctct 480
   tttataacat gttcaataaa aagctgaact tt 512
<210> 51
   <211> 646
   <212> DNA
   <213> Homo sapiens
<220>
   <223> 40S ribosomal protein S11, S11
<220>
   <221> CDS
   <222> (93)...(569)
   <223> RPS11
<400> 51
   tctccttacg tcacttcctc tccagcccct gcgtaatcga taaggaaacc cggacgctgc 60
   tgcccctttc tttttttcag gcggccggga agatggcgga cattcagact gagcgtgcct 120
   accaaaagca gccgaccatc tttcaaaaca agaagagggt cctgctggga gaaactggca 180
   aggagaagct cccgcggtac tacaagaaca tcggtctggg cttcaagaca cccaaggagg 240
   ctattgaggg cacctacatt gacaagaaat gccccttcac tggtaatgtg tccattcgag 300
   ggcggatcct ctctggcgtg gtgaccaaga tgaagatgca gaggaccatt gtcatccgcc 360
   gagactatct gcactacatc cgcaagtaca accgcttcga gaagcgccac aagaacatgt 420
   ctgtacacct gtccccctgc ttcagggacg tccagatcgg tgacatcgtc acagtgggcg 480
   agtgccggcc tctgagcaag acagtgcgct tcaacgtgct caaggtcacc aaggctgccg 540
   gcaccaagaa gcagttccag aagttctgag gctggacatc ggcccgctcc ccacaatgaa 600
   ataaagttat tttctcattc ccaggccaga cttgggatct tccgcg 646
<210> 52
   <211> 602
   <212> DNA
   <213> Homo sapiens
<220>
   <223> 60S ribosomal protein L26, L26, DBA11
<220>
   <221> CDS
   <222> (97)...(534)
   <223> RPL26
<400> 52
   ataggtctcg cgagatcttt ggtaaactta cagaaccgga agcagcgtgt agttctcttc 60
   ccttttgcgg ccatcaccga agcgggagcg gccaaaatga agtttaatcc ctttgtgact 120
   tccgaccgaa gcaagaatcg caaaaggcat ttcaatgcac cttcccacat tcgaaggaag 180
   attatgtctt cccctctttc caaagagctg agacagaagt acaacgtgcg atccatgccc 240
   atccgaaagg atgatgaagt tcaggttgta cgtggacact ataaaggtca gcaaattggc 300
   aaagtagtcc aggtttacag gaagaaatat gttatctaca ttgaacgggt gcagcgggaa 360
   aaggctaatg gcacaactgt ccacgtaggc attcacccca gcaaggtggt tatcactagg 420
   ctaaaactgg acaaagaccg caaaaagatc ctcgaacgga aagccaaatc tcgccaagta 480
   ggaaaggaaa agggcaaata caaggaagaa accattgaga agatgcagga ataaagtaat 540
   cttatataca agctttgatt aaaacttgaa acaaagagcc tgaaaaaaaa aaaaaaaaaa 600
   aa 602
<210> 53
   <211> 939
   <212> DNA
   <213> Homo sapiens
<220>
   <223> 60S ribosomal protein L14 transcript variant 1, L14
<220>
   <221> CDS
   <222> (134)...(781)
   <223> RPL14
<400> 53
   cttctcgcct aacgccgcca acatggtgag tcttactgtt gcgggctccg gggccgtcga 60
   ccatgccgct cgacctccac ctccgctggg aagctgaggc gccaaacggc tcccagaggg 120
   tcccgggaag cgcatggtgt tcaggcgctt cgtggaggtt ggccgggtgg cctatgtctc 180
   ctttggacct catgccggaa aattggtcgc gattgtagat gttattgatc agaacagggc 240
   tttggtcgat ggaccttgca ctcaagtgag gagacaggcc atgcctttca agtgcatgca 300
   gctcactgat ttcatcctca agtttccgca cagtgcccac cagaagtatg tccgacaagc 360
   ctggcagaag gcagacatca atacaaaatg ggcagccaca cgatgggcca agaagattga 420
   agccagagaa aggaaagcca agatgacaga ttttgatcgt tttaaagtta tgaaggcaaa 480
   gaaaatgagg aacagaataa tcaagaatga agttaagaag cttcaaaagg cagctctcct 540
   gaaagcttct cccaaaaaag cacctggtac taagggtact gctgctgctg ctgctgctgc 600
   tgctgctgct aaagttccag caaaaaagat caccgccgcg agtaaaaagg ctccagccca 660
   gaaggttcct gcccagaaag ccacaggcca gaaagcagcg cctgctccaa aagctcagaa 720
   gggtcaaaaa gctccagccc agaaagcacc tgctccaaag gcatctggca agaaagcata 780
   agtggcaatc ataaaaagta ataaaggttc tttttgacct gttgacaaat gtatttaagc 840
   ctttggattt aaagcctgtt gaggctggag ttaggaggca gattgatagt aggattataa 900
   taaacattaa ataatcagtt caaaaaaaaa aaaaaaaaa                        939
<210> 54
   <211> 1586
   <212> DNA
   <213> Homo sapiens
<220>
   <223> 60S ribosomal protein L37, L37, L37a, G1.16
<220>
   <221> CDS
   <222> (101)...(394)
   <223> RPL37
<400> 54
   gcgccccgca ggaagtgctt ccctgggcgg aagcttctga gcgtgatata gcggaagtgc 60
   cttctcttcc ggtctttctg gtctcggccg cagaagcgag atgacgaagg gaacgtcatc 120
   gtttggaaag cgtcgcaata agacgcacac gttgtgccgc cgctgtggct ctaaggccta 180
   ccaccttcag aagtcgacct gtggcaaatg tggctaccct gccaagcgca agagaaagta 240
   taactggagt gccaaggcta aaagacgaaa taccaccgga actggtcgaa tgaggcacct 300
   aaaaattgta taccgcagat tcaggcatgg attccgtgaa ggaacaacac ctaaacccaa 360
   gagggcagct gttgcagcat ccagttcatc ttaagaatgt caacgattag tcatgcaata 420
   aatgttctgg ttttaaaaaa tacatatctg gttttggtaa ggtattttta atcaattagg 480
   cttgtagtat cagtgaaata ctgtaggttt agggactggg ctagcttcat atcagattta 540
   cttgttaagt gactgttttg gaatgtttac ttttggactg ggtttgtaac acggttaaag 600
   gcaatgagaa acaagcagaa ttccaggagt ccttgaagca gagggcactg gaagacaata 660
   tagcagatta aaatagcaca gctcatgtgg cataggtggg tattttagat gtttgagtaa 720
   atttgaaaga gtatgatgtt taaattacct ttagcaacat gttcatctgc tatgctgtca 780
   tgactagggg gatgattatt agtcacatag agcttgggag taccactgga aacgtatggg 840
   taggagttta ggtggcttct gtttttcaaa agatgatctt atcctagtat ctgtaatgct 900
   cacttggcac acctgacttg tgggctgtgt gtaaggtggc tagctaagtg aaaaaagcct 960
   gctaggtgtg agtcaactta agaatatgta aataggtttg agaaaaagta gggcttgggt 1020
   gcaagtaaag attgagcagg aaataaagga aaatcaagta taatccctga gatttgtaga 1080
   ctaaaggcaa tgatgtggga ctacttggtc gaattttttt agccctcaac ttggtaattg 1140
   ggtgtttctg tgttaaagca ctgaaacttg ctgtcgtgcc ttcctagttt tcgtggttta 1200
   ttgacagggt tgggggtttt ttttgttttt ttaaaatgaa gggacaaagt caactggact 1260
   gctgagtgag agggcagggg cagttgaagg gaacatgaat tgctggaaca gctacataaa 1320
   atagtgatgt agccaagtca tgctatttaa attataattc tccactgtgt ttagaataac 1380
   atctgaggtt cttaacctgg ccttggaagg gtatcacttt tacttgtaac ctggaatggc 1440
   tttataatgt gctagctaat tgctactctc atcttgtatt ttaactccta atttaccctt 1500
   caggtctcag cttcagaaca ttcacttata aagaaaccct gctgattaaa tctctcttgg 1560
   gcttcctccc aaaaaaaaaa aaaaaa                                      1586
<210> 55
   <211> 866
   <212> DNA
   <213> Homo sapiens
<220>
   <223> 60S ribosomal protein L7, L7, humL7-1
<220>
   <221> CDS
   <222> (23)...(769)
   <223> RPL7
<400> 55
   tcctcttttt ccggctggaa ccatggaggg tgtagaagag aagaagaagg aggttcctgc 60
   tgtgccagaa acccttaaga aaaagcgaag gaatttcgca gagctgaaga tcaagcgcct 120
   gagaaagaag tttgcccaaa agatgcttcg aaaggcaagg aggaagctta tctatgaaaa 180
   agcaaagcac tatcacaagg aatataggca gatgtacaga actgaaattc gaatggcgag 240
   gatggcaaga aaagctggca acttctatgt acctgcagaa cccaaattgg cgtttgtcat 300
   cagaatcaga ggtatcaatg gagtgagccc aaaggttcga aaggtgttgc agcttcttcg 360
   ccttcgtcaa atcttcaatg gaacctttgt gaagctcaac aaggcttcga ttaacatgct 420
   gaggattgta gagccatata ttgcatgggg gtaccccaat ctgaagtcag taaatgaact 480
   aatctacaag cgtggttatg gcaaaatcaa taagaagcga attgctttga cagataacgc 540
   tttgattgct cgatctcttg gtaaatacgg catcatctgc atggaggatt tgattcatga 600
   gatctatact gttggaaaac gcttcaaaga ggcaaataac ttcctgtggc ccttcaaatt 660
   gtcttctcca cgaggtggaa tgaagaaaaa gaccacccat tttgtagaag gtggagatgc 720
   tggcaacagg gaggaccaga tcaacaggct tattagaaga atgaactaag gtgtctacca 780
   tgattatttt tctaagctgg ttggttaata aacagtacct gctctcaaat tgaaataaaa 840
   aaaaaaaaaa aaaaaaaaaa aaaaaa                                      866
<210> 56
   <211> 1941
   <212> DNA
   <213> Homo sapiens
<220>
   <223> heterogeneous nuclear ribonucleoprotein A1 transcript variant 2 (HNRNPA1, hnRNP A1, hnRNP-A1, HNRPA1), heterogeneous nuclear ribonucleoprotein B2 protein, hnRNP A1-like 3 (HNRPA1L3), IBMPFD3, single-strand DNA-binding protein UP1, helix-destabilizing protein
<220>
   <221> CDS
   <222> (105)...(1223)
   <223> HNRNPA1
<400> 56
   gagagggcga aggtaggctg gcagatacgt tcgtcagctt gctcctttct gcccgtggac 60
   gccgccgaag aagcatcgtt aaagtctctc ttcaccctgc cgtcatgtct aagtcagagt 120
   ctcctaaaga gcccgaacag ctgaggaagc tcttcattgg agggttgagc tttgaaacaa 180
   ctgatgagag cctgaggagc cattttgagc aatggggaac gctcacggac tgtgtggtaa 240
   tgagagatcc aaacaccaag cgctccaggg gctttgggtt tgtcacatat gccactgtgg 300
   aggaggtgga tgcagctatg aatgcaaggc cacacaaggt ggatggaaga gttgtggaac 360
   caaagagagc tgtctccaga gaagattctc aaagaccagg tgcccactta actgtgaaaa 420
   agatatttgt tggtggcatt aaagaagaca ctgaagaaca tcacctaaga gattattttg 480
   aacagtatgg aaaaattgaa gtgattgaaa tcatgactga ccgaggcagt ggcaagaaaa 540
   ggggctttgc ctttgtaacc tttgacgacc atgactccgt ggataagatt gtcattcaga 600
   aataccatac tgtgaatggc cacaactgtg aagttagaaa agccctgtca aagcaagaga 660
   tggctagtgc ttcatccagc caaagaggtc gaagtggttc tggaaacttt ggtggtggtc 720
   gtggaggtgg tttcggtggg aatgacaact tcggtcgtgg aggaaacttc agtggtcgtg 780
   gtggctttgg tggcagccgt ggtggtggtg gatatggtgg cagtggggat ggctataatg 840
   gatttggtaa tgatggtggt tatggaggag gcggccctgg ttactctgga ggaagcagag 900
   gctatggaag tggtggacag ggttatggaa accagggcag tggctatggc gggagtggca 960
   gctatgacag ctataacaac ggaggcggag gcggctttgg cggtggtagt ggaagcaatt 1020
   ttggaggtgg tggaagctac aatgattttg ggaattacaa caatcagtct tcaaattttg 1080
   gacccatgaa gggaggaaat tttggaggca gaagctctgg cccctatggc ggtggaggcc 1140
   aatactttgc aaaaccacga aaccaaggtg gctatggcgg ttccagcagc agcagtagct 1200
   atggcagtgg cagaagattt taattaggaa acaaagctta gcaggagagg agagccagag 1260
   aagtgacagg gaagctacag gttacaacag atttgtgaac tcagccaagc acagtggtgg 1320
   cagggcctag ctgctacaaa gaagacatgt tttagacaaa tactcatgtg tatgggcaaa 1380
   aaactcgagg actgtatttg tgactaattg tataacaggt tattttagtt tctgttctgt 1440
   ggaaagtgta aagcattcca acaaagggtt ttaatgtaga tttttttttt tgcaccccat 1500
   gctgttgatt gctaaatgta acagtctgat cgtgacgctg aataaatgtc ttttttttaa 1560
   tgtgctgtgt aaagttagtc tactcttaag ccatcttggt aaatttcccc aacagtgtga 1620
   agttagaatt ccttcagggt gatgccaggt tctatttgga atttatatac aacctgcttg 1680
   ggtggagaag ccattgtctt cggaaacctt ggtgtagttg aactgatagt tactgttgtg 1740
   acctgaagtt caccattaaa agggattacc caagcaaaat catggaatgg ttataaaagt 1800
   gattgttggc acatcctatg caatatatct aaattgaata atggtaccag ataaaattat 1860
   agatgggaat gaagcttgtg tatccattat catgtgtaat caataaacga tttaattctc 1920
   ttgaaaaaaa aaaaaaaaaa a                                           1941
<210> 57
   <211> 705
   <212> DNA
   <213> Homo sapiens
<220>
   <223> 40S ribosomal protein S8, S8, OK/SW-cl.83
<220>
   <221> CDS
   <222> (24)...(650)
   <223> RPS8
<400> 57
   ctctttccag ccagcgccga gcgatgggca tctctcggga caactggcac aagcgccgca 60
   aaaccggggg caagagaaag ccctaccaca agaagcggaa gtatgagttg gggcgcccag 120
   ctgccaacac caagattggc ccccgccgca tccacacagt ccgtgtgcgg ggaggtaaca 180
   agaaataccg tgccctgagg ttggacgtgg ggaatttctc ctggggctca gagtgttgta 240
   ctcgtaaaac aaggatcatc gatgttgtct acaatgcatc taataacgag ctggttcgta 300
   ccaagaccct ggtgaagaat tgcatcgtgc tcatcgacag cacaccgtac cgacagtggt 360
   acgagtccca ctatgcgctg cccctgggcc gcaagaaggg agccaagctg actcctgagg 420
   aagaagagat tttaaacaaa aaacgatcta aaaaaattca gaagaaatat gatgaaagga 480
   aaaagaatgc caaaatcagc agtctcctgg aggagcagtt ccagcagggc aagcttcttg 540
   cgtgcatcgc ttcaaggccg ggacagtgtg gccgagcaga tggctatgtg ctagagggca 600
   aagagttgga gttctatctt aggaaaatca aggcccgcaa aggcaaataa atccttgttt 660
   tgtcttcacc catgtaataa aggtgtttat tgttttgttc ccaca                 705
<210> 58
   <211> 1401
   <212> DNA
   <213> Homo sapiens
<220>
   <223> glyceraldehyde-3-phosphate dehydrogenase transcript variant 1 (GAPDH, G3PD; GAPD), peptidyl-cysteine S-nitrosylase GAPDH, aging-associated gene 9 protein, epididymis secretory sperm binding protein Li 162eP (HEL-S-162eP)
<220>
   <221> CDS
   <222> (175) ... (1182)
   <223> GAPDH
<400> 58
   ggctgggact ggctgagcct ggcgggaggc ggggtccgag tcaccgcctg ccgccgcgcc 60
   cccggtttct ataaattgag cccgcagcct cccgcttcgc tctctgctcc tcctgttcga 120
   cagtcagccg catcttcttt tgcgtcgcca gccgagccac atcgctcaga caccatgggg 180
   aaggtgaagg tcggagtcaa cggatttggt cgtattgggc gcctggtcac cagggctgct 240
   tttaactctg gtaaagtgga tattgttgcc atcaatgacc ccttcattga cctcaactac 300
   atggtttaca tgttccaata tgattccacc catggcaaat tccatggcac cgtcaaggct 360
   gagaacggga agcttgtcat caatggaaat cccatcacca tcttccagga gcgagatccc 420
   tccaaaatca agtggggcga tgctggcgct gagtacgtcg tggagtccac tggcgtcttc 480
   accaccatgg agaaggctgg ggctcatttg caggggggag ccaaaagggt catcatctct 540
   gccccctctg ctgatgcccc catgttcgtc atgggtgtga accatgagaa gtatgacaac 600
   agcctcaaga tcatcagcaa tgcctcctgc accaccaact gcttagcacc cctggccaag 660
   gtcatccatg acaactttgg tatcgtggaa ggactcatga ccacagtcca tgccatcact 720
   gccacccaga agactgtgga tggcccctcc gggaaactgt ggcgtgatgg ccgcggggct 780
   ctccagaaca tcatccctgc ctctactggc gctgccaagg ctgtgggcaa ggtcatccct 840
   gagctgaacg ggaagctcac tggcatggcc ttccgtgtcc ccactgccaa cgtgtcagtg 900
   gtggacctga cctgccgtct agaaaaacct gccaaatatg atgacatcaa gaaggtggtg 960
   aagcaggcgt cggagggccc cctcaagggc atcctgggct acactgagca ccaggtggtc 1020
   tcctctgact tcaacagcga cacccactcc tccacctttg acgctggggc tggcattgcc 1080
   ctcaacgacc actttgtcaa gctcatttcc tggtatgaca acgaatttgg ctacagcaac 1140
   agggtggtgg acctcatggc ccacatggcc tccaaggagt aagacccctg gaccaccagc 1200
   cccagcaaga gcacaagagg aagagagaga ccctcactgc tggggagtcc ctgccacact 1260
   cagtccccca ccacactgaa tctcccctcc tcacagttgc catgtagacc ccttgaagag 1320
   gggagggggc tagggagccg caccttgtca tgtaccatca ataaagtacc ctgtgctcaa 1380
   ccaaaaaaaa aaaaaaaaaa a                                           1401
<210> 59
   <211> 903
   <212> DNA
   <213> Homo sapiens
<220>
   <223> 60S ribosomal protein L8 transcript variant 1, L8
<220>
   <221> CDS
   <222> (67)...(840)
   <223> RPL8
<400> 59
   agataaggcc gctcgctgac gccgtgtttc ctctttcggc cgcgctggtg aacaggaccc 60
   gtcgccatgg gccgtgtgat ccgtggacag aggaagggcg ccgggtctgt gttccgcgcg 120
   cacgtgaagc accgtaaagg cgctgcgcgc ctgcgcgccg tggatttcgc tgagcggcac 180
   ggctacatca agggcatcgt caaggacatc atccacgacc cgggccgcgg cgcgcccctc 240
   gccaaggtgg tcttccggga tccgtatcgg tttaagaagc ggacggagct gttcattgcc 300
   gccgagggca ttcacacggg ccagtttgtg tattgcggca agaaggccca gctcaacatt 360
   ggcaatgtgc tccctgtggg caccatgcct gagggtacaa tcgtgtgctg cctggaggag 420
   aagcctggag accgtggcaa gctggcccgg gcatcaggga actatgccac cgttatctcc 480
   cacaaccctg agaccaagaa gacccgtgtg aagctgccct ccggctccaa gaaggttatc 540
   tcctcagcca acagagctgt ggttggtgtg gtggctggag gtggccgaat tgacaaaccc 600
   atcttgaagg ctggccgggc gtaccacaaa tataaggcaa agaggaactg ctggccacga 660
   gtacggggtg tggccatgaa tcctgtggag catccttttg gaggtggcaa ccaccagcac 720
   atcggcaagc cctccaccat ccgcagagat gcccctgctg gccgcaaagt gggtctcatt 780
   gctgcccgcc ggactggacg tctccgggga accaagactg tgcaggagaa agagaactag 840
   tgctgagggc ctcaataaag tttgtgttta tgccaaaaaa aaaaaaaaaa aaaaaaaaaa 900
   aaa                                                               903
<210> 60
   <211> 737
   <212> DNA
   <213> Homo sapiens
<220>
   <223> 60S ribosomal protein L29, L29, ribosomal protein YL43 homologue, heparin/heparan sulfate-interacting protein, heparin/heparan sulfate-binding protein, HP/HS-interacting protein, cell surface heparin-binding protein (HIP), HUMRPL29, RPL29P10; RPL29_3_370
<220>
   <221> CDS
   <222> (95)...(574)
   <223> RPL29
<400> 60
   ttccggcgtt gttgacccta tttcccgtgc tgcaccgcag cccctttctc ttccggttct 60
   aggcgcttcg ggagccgcgg cttatggtgc agacatggcc aagtccaaga accacaccac 120
   acacaaccag tcccgaaaat ggcacagaaa tggtatcaag aaaccccgat cacaaagata 180
   cgaatctctt aagggggtgg accccaagtt cctgaggaac atgcgctttg ccaagaagca 240
   caacaaaaag ggcctaaaga agatgcaggc caacaatgcc aaggccatga gtgcacgtgc 300
   cgaggctatc aaggccctcg taaagcccaa ggaggttaag cccaagatcc caaagggtgt 360
   cagccgcaag ctcgatcgac ttgcctacat tgcccacccc aagcttggga agcgtgctcg 420
   tgcccgtatt gccaaggggc tcaggctgtg ccggccaaag gccaaggcca aggccaaggc 480
   caaggatcaa accaaggccc aggctgcagc cccagcttca gttccagctc aggctcccaa 540
   acgtacccag gcccctacaa aggcttcaga gtagatatct ctgccaacat gaggacagaa 600
   ggactggtgc gaccccccac ccccgcccct gggctaccat ctgcatgggg ctggggtcct 660
   cctgtgctat ttgtacaaat aaacctgagg caggaaaaaa aaaaaaaaaa aaaaaaaaaa 720
   aaaaaaaaaa aaaaaaa                                                737
<210> 61
   <211> 950
   <212> DNA
   <213> Homo sapiens
<220>
   <223> 40S ribosomal protein S3a transcript variant 1, S3A, v-fos transformation effector protein 1, fte-1, FTE1, MFTL
<220>
   <221> CDS
   <222> (81)...(875)
   <223> RPS3A
<400> 61
   tagacggcgc gccccgcccc cgtacgccta agttctcgcg cgactcccac ttccgccctt 60
   ttggctctct gaccagcacc atggcggttg gcaagaacaa gcgccttacg aaaggcggca 120
   aaaagggagc caagaagaaa gtggttgatc cattttctaa gaaagattgg tatgatgtga 180
   aagcacctgc tatgttcaat ataagaaata ttggaaagac gctcgtcacc aggacccaag 240
   gaaccaaaat tgcatctgat ggtctcaagg gtcgtgtgtt tgaagtgagt cttgctgatt 300
   tgcagaatga tgaagttgca tttagaaaat tcaagctgat tactgaagat gttcagggta 360
   aaaactgcct gactaacttc catggcatgg atcttacccg tgacaaaatg tgttccatgg 420
   tcaaaaaatg gcagacaatg attgaagctc acgttgatgt caagactacc gatggttact 480
   tgcttcgtct gttctgtgtt ggttttacta aaaaacgcaa caatcagata cggaagacct 540
   cttatgctca gcaccaacag gtccgccaaa tccggaagaa gatgatggaa atcatgaccc 600
   gagaggtgca gacaaatgac ttgaaagaag tggtcaataa attgattcca gacagcattg 660
   gaaaagacat agaaaaggct tgccaatcta tttatcctct ccatgatgtc ttcgttagaa 720
   aagtaaaaat gctgaagaag cccaagtttg aattgggaaa gctcatggag cttcatggtg 780
   aaggcagtag ttctggaaaa gccactgggg acgagacagg tgctaaagtt gaacgagctg 840
   atggatatga accaccagtc caagaatctg tttaaagttc agacttcaaa tagtggcaaa 900
   taaaaagtgc tatttgtgat ggtttgcttc tgaaaaaaaa aaaaaaaaaa            950
<210> 62
   <211> 893
   <212> DNA
   <213> Homo sapiens
<220>
   <223> 60S ribosomal protein L18 transcript variant 1, L18
<220>
   <221> CDS
   <222> (276)...(842)
   <223> RPL18
<400> 62
   gaaagctatc gagaacacgg cctgggtagg gccagagagg cccccgacgt gctggccctt 60
   ccctcttgga cgttgcgctt gttcctgcgc tctatgctct ctgccgttat cgcccggcta 120
   gtcagtcgtc caactcacca cagagaagtc cggatcgtgg tagagcgccg cgtcgcaccc 180
   atgtgacgtc acggcggcgc cactcgcttg aggctttccc cgcccacccc agcccgttct 240
   ctctttccgg acctggccga gcaggaggcg ccatcatggg agtggacatc cgccataaca 300
   aggaccgaaa ggttcggcgc aaggagccca agagccagga tatctacctg aggctgttgg 360
   tcaagttata caggtttctg gccagaagaa ccaactccac attcaaccag gttgtgttga 420
   agaggttgtt tatgagtcgc accaaccggc cgcctctgtc cctttcccgg atgatccgga 480
   agatgaagct tcctggccgg gaaaacaaga cggccgtggt tgtggggacc ataactgatg 540
   atgtgcgggt tcaggaggta cccaaactga aggtatgtgc actgcgcgtg accagccggg 600
   cccgcagccg catcctcagg gcagggggca agatcctcac tttcgaccag ctggccctgg 660
   actcccctaa gggctgtggc actgtcctgc tctccggtcc tcgcaagggc cgagaggtgt 720
   accggcattt cggcaaggcc ccaggaaccc cgcacagcca caccaaaccc tacgtccgct 780
   ccaagggccg gaagttcgag cgtgccagag gccgacgggc cagccgaggc tacaaaaact 840
   aaccctggat cctactctct tattaaaaag atttttgctg acagtgcaaa aaa        893
<210> 63
   <211> 557
   <212> DNA
   <213> Homo sapiens
<220>
   <223> 60S ribosomal protein L36 transcript variant 2, L36
<220>
   <221> CDS
   <222> (174)...(491)
   <223> RPL36
<400> 63
   gtttcccgca ctgccggtat ccgccgccat ccggactccc gggtcctctg tgcaggttgg 60
   aggatggttg gttgtggcga gcgaggctga aggagccggg acgcggggct ctgggcctcg 120
   ggaactgagc cggtactcac ctccgcccct tctccccgtc gctgtccgca gccatggccc 180
   tacgctaccc tatggccgtg ggcctcaaca agggccacaa agtgaccaag aacgtgagca 240
   agcccaggca cagccgacgc cgcgggcgtc tgaccaaaca caccaagttc gtgcgggaca 300
   tgattcggga ggtgtgtggc tttgccccgt acgagcggcg cgccatggag ttactgaagg 360
   tctccaagga caaacgggcc ctcaaattta tcaagaaaag ggtggggacg cacatccgcg 420
   ccaagaggaa gcgggaggag ctgagcaacg tactggccgc catgaggaaa gctgctgcca 480
   agaaagactg agcccctccc ctgccctctc cctgaaataa agaacagctt gacagaaaaa 540
   aaaaaaaaaa aaaaaaa                                                557
<210> 64
   <211> 7343
   <212> DNA
   <213> Homo sapiens
<220>
   <223> agrin precursor, agrin proteoglycan (AGRN)
<220>
   <221> CDS
   <222> (51)...(6188)
   <223> AGRN
<400> 64
   cccgtccccg gcgcggcccg cgcgctcctc cgccgcctct cgcctgcgcc atggccggcc 60
   ggtcccaccc gggcccgctg cggccgctgc tgccgctcct tgtggtggcc gcgtgcgtcc 120
   tgcccggagc cggcgggaca tgcccggagc gcgcgctgga gcggcgcgag gaggaggcga 180
   acgtggtgct caccgggacg gtggaggaga tcctcaacgt ggacccggtg cagcacacgt 240
   actcctgcaa ggttcgggtc tggcggtact tgaagggcaa agacctggtg gcccgggaga 300
   gcctgctgga cggcggcaac aaggtggtga tcagcggctt tggagacccc ctcatctgtg 360
   acaaccaggt gtccactggg gacaccagga tcttctttgt gaaccctgca cccccatacc 420
   tgtggccagc ccacaagaac gagctgatgc tcaactccag cctcatgcgg atcaccctgc 480
   ggaacctgga ggaggtggag ttctgtgtgg aagataaacc cgggacccac ttcactccag 540
   tgcctccgac gcctcctgat gcgtgccggg gaatgctgtg cggcttcggc gccgtgtgcg 600
   agcccaacgc ggaggggccg ggccgggcgt cctgcgtctg caagaagagc ccgtgcccca 660
   gcgtggtggc gcctgtgtgt gggtcggacg cctccaccta cagcaacgaa tgcgagctgc 720
   agcgggcgca gtgcagccag cagcgccgca tccgcctgct cagccgcggg ccgtgcggct 780
   cgcgggaccc ctgctccaac gtgacctgca gcttcggcag cacctgtgcg cgctcggccg 840
   acgggctgac ggcctcgtgc ctgtgccccg cgacctgccg tggcgccccc gaggggaccg 900
   tctgcggcag cgacggcgcc gactaccccg gcgagtgcca gctcctgcgc cgcgcctgcg 960
   cccgccagga gaatgtcttc aagaagttcg acggcccttg tgacccctgt cagggcgccc 1020
   tccctgaccc gagccgcagc tgccgtgtga acccgcgcac gcggcgccct gagatgctcc 1080
   tacggcccga gagctgccct gcccggcagg cgccagtgtg tggggacgac ggagtcacct 1140
   acgaaaacga ctgtgtcatg ggccgatcgg gggccgcccg gggtctcctc ctgcagaaag 1200
   tgcgctccgg ccagtgccag ggtcgagacc agtgcccgga gccctgccgg ttcaatgccg 1260
   tgtgcctgtc ccgccgtggc cgtccccgct gctcctgcga ccgcgtcacc tgtgacgggg 1320
   cctacaggcc cgtgtgtgcc caggacgggc gcacgtatga cagtgattgc tggcggcagc 1380
   aggctgagtg ccggcagcag cgtgccatcc ccagcaagca ccagggcccg tgtgaccagg 1440
   ccccgtcccc atgcctcggg gtgcagtgtg catttggggc gacgtgtgct gtgaagaacg 1500
   ggcaggcagc gtgtgaatgc ctgcaggcgt gctcgagcct ctacgatcct gtgtgcggca 1560
   gcgacggcgt cacatacggc agcgcgtgcg agctggaggc cacggcctgt accctcgggc 1620
   gggagatcca ggtggcgcgc aaaggaccct gtgaccgctg cgggcagtgc cgctttggag 1680
   ccctgtgcga ggccgagacc gggcgctgcg tgtgcccctc tgaatgcgtg gctttggccc 1740
   agcccgtgtg tggctccgac gggcacacgt accccagcga gtgcatgctg cacgtgcacg 1800
   cctgcacaca ccagatcagc ctgcacgtgg cctcagctgg accctgtgag acctgtggag 1860
   atgccgtgtg tgcttttggg gctgtgtgct ccgcagggca gtgtgtgtgt ccccggtgtg 1920
   agcacccccc gcccggcccc gtgtgtggca gcgacggtgt cacctacggc agtgcctgcg 1980
   agctacggga agccgcctgc ctccagcaga cacagatcga ggaggcccgg gcagggccgt 2040
   gcgagcaggc cgagtgcggt tccggaggct ctggctctgg ggaggacggt gactgtgagc 2100
   aggagctgtg ccggcagcgc ggtggcatct gggacgagga ctcggaggac gggccgtgtg 2160
   tctgtgactt cagctgccag agtgtcccag gcagcccggt gtgcggctca gatggggtca 2220
   cctacagcac cgagtgtgag ctgaagaagg ccaggtgtga gtcacagcga gggctctacg 2280
   tagcggccca gggagcctgc cgaggcccca ccttcgcccc gctgccgcct gtggccccct 2340
   tacactgtgc ccagacgccc tacggctgct gccaggacaa tatcaccgca gcccggggcg 2400
   tgggcctggc tggctgcccc agtgcctgcc agtgcaaccc ccatggctct tacggcggca 2460
   cctgtgaccc agccacaggc cagtgctcct gccgcccagg tgtggggggc ctcaggtgtg 2520
   accgctgtga gcctggcttc tggaactttc gaggcatcgt caccgatggc cggagtggct 2580
   gtacaccctg cagctgtgat ccccaaggcg ccgtgcggga tgactgtgag cagatgacgg 2640
   ggctgtgctc gtgtaagccc ggggtggctg gacccaagtg tgggcagtgt ccagacggcc 2700
   gtgccctggg ccccgcgggc tgtgaagctg acgcttctgc gcctgcgacc tgtgcggaga 2760
   tgcgctgtga gttcggtgcg cggtgcgtgg aggagtctgg ctcagcccac tgtgtctgcc 2820
   cgatgctcac ctgtccagag gccaacgcta ccaaggtctg tgggtcagat ggagtcacat 2880
   acggcaacga gtgtcagctg aagaccatcg cctgccgcca gggcctgcaa atctctatcc 2940
   agagcctggg cccgtgccag gaggctgttg ctcccagcac tcacccgaca tctgcctccg 3000
   tgactgtgac caccccaggg ctcctcctga gccaggcact gccggccccc cccggcgccc 3060
   tccccctggc tcccagcagt accgcacaca gccagaccac ccctccgccc tcatcacgac 3120
   ctcggaccac tgccagcgtc cccaggacca ccgtgtggcc cgtgctgacg gtgcccccca 3180
   cggcaccctc ccctgcaccc agcctggtgg cgtccgcctt tggtgaatct ggcagcactg 3240
   atggaagcag cgatgaggaa ctgagcgggg accaggaggc cagtgggggt ggctctgggg 3300
   ggctcgagcc cttggagggc agcagcgtgg ccacccctgg gccacctgtc gagagggctt 3360
   cctgctacaa ctccgcgttg ggctgctgct ctgatgggaa gacgccctcg ctggacgcag 3420
   agggctccaa ctgccccgcc accaaggtgt tccagggcgt cctggagctg gagggcgtcg 3480
   agggccagga gctgttctac acgcccgaga tggctgaccc caagtcagaa ctgttcgggg 3540
   agacagccag gagcattgag agcaccctgg acgacctctt ccggaattca gacgtcaaga 3600
   aggattttcg gagtgtccgc ttgcgggacc tggggcccgg caaatccgtc cgcgccattg 3660
   tggatgtgca ctttgacccc accacagcct tcagggcacc cgacgtggcc cgggccctgc 3720
   tccggcagat ccaggtgtcc aggcgccggt ccttgggggt gaggcggccg ctgcaggagc 3780
   acgtgcgatt tatggacttt gactggtttc ctgcgtttat cacgggggcc acgtcaggag 3840
   ccattgctgc gggagccacg gccagagcca ccactgcatc gcgcctgccg tcctctgctg 3900
   tgacccctcg ggccccgcac cccagtcaca caagccagcc cgttgccaag accacggcag 3960
   cccccaccac acgtcggccc cccaccactg cccccagccg tgtgcccgga cgtcggcccc 4020
   cggcccccca gcagcctcca aagccctgtg actcacagcc ctgcttccac ggggggacct 4080
   gccaggactg ggcattgggc gggggcttca cctgcagctg cccggcaggc aggggaggcg 4140
   ccgtctgtga gaaggtgctt ggcgcccctg tgccggcctt cgagggccgc tccttcctgg 4200
   ccttccccac tctccgcgcc taccacacgc tgcgcctggc actggaattc cgggcgctgg 4260
   agcctcaggg gctgctgctg tacaatggca acgcccgggg caaggacttc ctggcattgg 4320
   cgctgctaga tggccgcgtg cagctcaggt ttgacacagg ttcggggccg gcggtgctga 4380
   ccagtgccgt gccggtagag ccgggccagt ggcaccgcct ggagctgtcc cggcactggc 4440
   gccggggcac cctctcggtg gatggtgaga cccctgttct gggcgagagt cccagtggca 4500
   ccgacggcct caacctggac acagacctct ttgtgggcgg cgtacccgag gaccaggctg 4560
   ccgtggcgct ggagcggacc ttcgtgggcg ccggcctgag ggggtgcatc cgtttgctgg 4620
   acgtcaacaa ccagcgcctg gagcttggca ttgggccggg ggctgccacc cgaggctctg 4680
   gcgtgggcga gtgcggggac cacccctgcc tgcccaaccc ctgccatggc ggggccccat 4740
   gccagaacct ggaggctgga aggttccatt gccagtgccc gcccggccgc gtcggaccaa 4800
   cctgtgccga tgagaagagc ccctgccagc ccaacccctg ccatggggcg gcgccctgcc 4860
   gtgtgctgcc cgagggtggt gctcagtgcg agtgccccct ggggcgtgag ggcaccttct 4920
   gccagacagc ctcggggcag gacggctctg ggcccttcct ggctgacttc aacggcttct 4980
   cccacctgga gctgagaggc ctgcacacct ttgcacggga cctgggggag aagatggcgc 5040
   tggaggtcgt gttcctggca cgaggcccca gcggcctcct gctctacaac gggcagaaga 5100
   cggacggcaa gggggacttc gtgtcgctgg cactgcggga ccgccgcctg gagttccgct 5160
   acgacctggg caagggggca gcggtcatca ggagcaggga gccagtcacc ctgggagcct 5220
   ggaccagggt ctcactggag cgaaacggcc gcaagggtgc cctgcgtgtg ggcgacggcc 5280
   cccgtgtgtt gggggagtcc ccggttccgc acaccgtcct caacctgaag gagccgctct 5340
   acgtaggggg cgctcccgac ttcagcaagc tggcccgtgc tgctgccgtg tcctctggct 5400
   tcgacggtgc catccagctg gtctccctcg gaggccgcca gctgctgacc ccggagcacg 5460
   tgctgcggca ggtggacgtc acgtcctttg caggtcaccc ctgcacccgg gcctcaggcc 5520
   acccctgcct caatggggcc tcctgcgtcc cgagggaggc tgcctatgtg tgcctgtgtc 5580
   ccgggggatt ctcaggaccg cactgcgaga aggggctggt ggagaagtca gcgggggacg 5640
   tggatacctt ggcctttgac gggcggacct ttgtcgagta cctcaacgct gtgaccgaga 5700
   gcgagaaggc actgcagagc aaccactttg aactgagcct gcgcactgag gccacgcagg 5760
   ggctggtgct ctggagtggc aaggccacgg agcgggcaga ctatgtggca ctggccattg 5820
   tggacgggca cctgcaactg agctacaacc tgggctccca gcccgtggtg ctgcgttcca 5880
   ccgtgcccgt caacaccaac cgctggttgc gggtcgtggc acatagggag cagagggaag 5940
   gttccctgca ggtgggcaat gaggcccctg tgaccggctc ctccccgctg ggcgccacgc 6000
   agctggacac tgatggagcc ctgtggcttg ggggcctgcc ggagctgccc gtgggcccag 6060
   cactgcccaa ggcctacggc acaggctttg tgggctgctt gcgggacgtg gtggtgggcc 6120
   ggcacccgct gcacctgctg gaggacgccg tcaccaagcc agagctgcgg ccctgcccca 6180
   ccccatgagc tggcaccaga gccccgcgcc cgctgtaatt attttctatt tttgtaaact 6240
   tgttgctttt tgatatgatt ttcttgcctg agtgttggcc ggagggactg ctggcccggc 6300
   ctcccttccg tccaggcagc cgtgctgcag acagacctag tgccgaggga tggacaggcg 6360
   aggtggcagc gtggagggct cggcgtggat ggcagcctca ggacacacac ccctgcctca 6420
   aggtgctgag cccccgcctt gcactgcgcc tgccccacgg tgtccccgcc gggaagcagc 6480
   cccggctcct gaatcaccct cgctccgtca ggcgggactc gtgtcccaga gaggaagggg 6540
   ctgctgaggt ctgatggggc ccttcctccg ggtgacccca cagggccttt ccaagccccc 6600
   atttgagctg ctccttcctg tgtgtgctct gggccctgcc tcggcctcct gcgccaatac 6660
   tgtgacttcc aaacaatgtt actgctgggc acagctctgc gttgctcccg tgctgcctgc 6720
   gccagcccca ggctgctgag gagcagaggc cagaccaggg ccgatctggg tgtcctgacc 6780
   ctcagctggc cctgcccagc caccctggac gtgaccgtat ccctctgcca caccccaggc 6840
   cctgcgaggg gctatcgaga ggagctcact gtgggatggg gttgacctct gccgcctgcc 6900
   tgggtatctg ggcctggcca tggctgtgtt cttcatgtgt tgattttatt tgacccctgg 6960
   agtggtgggt ctcatctttc ccatctcgcc tgagagcggc tgagggctgc ctcactgcaa 7020
   atcctcccca cagcgtcagt gaaagtcgtc cttgtctcag aatgaccagg ggccagccag 7080
   tgtctgacca aggtcaaggg gcaggtgcag aggtggcagg gatggctccg aagccagaaa 7140
   tgccttaaac tgcaacgtcc cgtcccttcc ccacccccat cccatcccca cccccagccc 7200
   cagcccagtc ctcctaggag caggacccga tgaagcgggc ggcggtgggg ctgggtgccg 7260
   tgttactaac tctagtatgt ttctgtgtca atcgctgtga aataaagtct gaaaacttta 7320
   aaagcaaaaa aaaaaaaaaa aaa                                         7343
<210> 65
   <211> 829
   <212> DNA
   <213> Homo sapiens
<220>
   <223> tumor protein, translationally-controlled 1 transcript variant 2 (TPT1), fortilin, translationally-controlled tumor protein (TCTP), histamine-releasing factor (HRF), p02, p23
<220>
   <221> CDS
   <222> (94)...(612)
   <223> TPT1
<400> 65
   ccccccgagc gccgctccgg ctgcaccgcg ctcgctccga gtttcaggct cgtgctaagc 60
   tagcgccgtc gtcgtctccc ttcagtcgcc atcatgatta tctaccggga cctcatcagc 120
   cacgatgaga tgttctccga catctacaag atccgggaga tcgcggacgg gttgtgcctg 180
   gaggtggagg ggaagatggt cagtaggaca gaaggtaaca ttgatgactc gctcattggt 240
   ggaaatgcct ccgctgaagg ccccgagggc gaaggtaccg aaagcacagt aatcactggt 300
   gtcgatattg tcatgaacca tcacctgcag gaaacaagtt tcacaaaaga agcctacaag 360
   aagtacatca aagattacat gaaatcaatc aaagggaaac ttgaagaaca gagaccagaa 420
   agagtaaaac cttttatgac aggggctgca gaacaaatca agcacatcct tgctaatttc 480
   aaaaactacc agttctttat tggtgaaaac atgaatccag atggcatggt tgctctattg 540
   gactaccgtg aggatggtgt gaccccatat atgattttct ttaaggatgg tttagaaatg 600
   gaaaaatgtt aacaaatgtg gcaattattt tggatctatc acctgtcatc ataactggct 660
   tctgcttgtc atccacacaa caccaggact taagacaaat gggactgatg tcatcttgag 720
   ctcttcattt attttgactg tgatttattt ggagtggagg cattgttttt aagaaaaaca 780
   tgtcatgtag gttgtctaaa aataaaatgc atttaaactc atttgagag 829
<210> 66
   <211> 881
   <212> DNA
   <213> Homo sapiens
<220>
   <223> 60S ribosomal protein L36a transcript variant 1, L36A, ribosomal protein L44, L44, RPL44, L44-like ribosomal protein (L44L), cell growth-inhibiting gene 15 protein, cell migration-inducing gene 6 protein (MIG6)
<220>
   <221> CDS
   <222> (47)...(475)
   <223> RPL36A
<400> 66
   gagtcctctc agccgcccga gggcgctgcg ctgagcctta cactctatga ttgctcctac 60
   cgactcccat gaggaagtgc gatcgggaac ctcctatata cttccgtttg cctcgcggtt 120
   tctttctttc cgcgccgata gcgctcacgc aagcatggtt aacgtcccta aaacccgccg 180
   gactttctgt aagaagtgtg gcaagcacca accccataaa gtgacacagt acaagaaggg 240
   caaggattct ctgtacgccc agggaaagcg gcgttatgac aggaagcaga gtggctatgg 300
   tgggcaaact aagccgattt tccggaaaaa ggctaaaact acaaagaaga ttgtgctaag 360
   gcttgagtgc gttgagccca actgcagatc taagagaatg ctggctatta aaagatgcaa 420
   gcattttgaa ctgggaggag ataagaagag aaagggccaa gtgatccagt tctaagtgtc 480
   atcttttatt atgaagacaa taaaatcttg agtttatgtt cacttcattt gtttgctgtt 540
   catcttttgg gagggaataa gctagagcca tcaatacaat tccgcttgtg gggaaattta 600
   tgcctcttac tggtactact tgttttgcat tgaagctgac tggttgagtt cacatcatat 660
   gttgcaattt tctaatttgg cacttcaatc actaggggcc ttatgaggca gtttgtcatt 720
   atgcaatggt tattggttat catgtgagta gacacatttc aggctaatag ggagaagtca 780
   gtaacacatt catagtgaat atgagatgtc tttgctaaga gttaagtgtc agatctttgt 840
   tataacagtt aatttaataa agaattttgg cattgttctt c 881
<210> 67
   <211> 1351
   <212> DNA
   <213> Homo sapiens
<220>
   <223> adenine nucleotide translocator 2 (fibroblast) (ANT2), solute carrier family 25 (mitochondrial carrier, adenine nucleotide translocator) member 5 (SLC25A5), ADP,ATP carrier protein 2 (AAC2), fibroblast isoform, 2F1, T2, T3
<220>
   <221> CDS
   <222> (117) ... (1013)
   <223> SLC25A5
<400> 67
   agctccggct ccccctatat aaatcggcca tttgcttcgc tccgccccgc agcgccggag 60
   tcaaagccgg ttcccggccc agtcccgtcc tgcagcagtc tgcctcctct ttcaacatga 120
   cagatgccgc tgtgtccttc gccaaggact tcctggcagg tggagtggcc gcagccatct 180
   ccaagacggc ggtagcgccc atcgagcggg tcaagctgct gctgcaggtg cagcatgcca 240
   gcaagcagat cactgcagat aagcaataca aaggcattat agactgcgtg gtccgtattc 300
   ccaaggagca gggagttctg tccttctggc gcggtaacct ggccaatgtc atcagatact 360
   tccccaccca ggctcttaac ttcgccttca aagataaata caagcagatc ttcctgggtg 420
   gtgtggacaa gagaacccag ttttggctct actttgcagg gaatctggca tcgggtggtg 480
   ccgcaggggc cacatccctg tgttttgtgt accctcttga ttttgcccgt acccgtctag 540
   cagctgatgt gggtaaagct ggagctgaaa gggaattccg aggcctcggt gactgcctgg 600
   ttaagatcta caaatctgat gggattaagg gcctgtacca aggctttaac gtgtctgtgc 660
   agggtattat catctaccga gccgcctact tcggtatcta tgacactgca aagggaatgc 720
   ttccggatcc caagaacact cacatcgtca tcagctggat gatcgcacag actgtcactg 780
   ctgttgccgg gttgacttcc tatccatttg acactgttcg ccgccgcatg atgatgcagt 840
   cagggcgcaa aggaactgac atcatgtaca caggcacgct tgactgctgg cggaagattg 900
   ctcgtgatga aggaggcaaa gcttttttca agggtgcatg gtccaatgtt ctcagaggca 960
   tgggtggtgc ttttgtgctt gtcttgtatg atgaaatcaa gaagtacaca taagttattt 1020
   cctaggattt ttccccctgt gaacaggcat gttgtattat ataacatatc ttgagcattc 1080
   ttgacagact cctggctgtc agtttctcag tggcaactat ttactggttg aaaatgggaa 1140
   gcaataatat tcatctgacc agttttctct taaagccatt tccatgatga tgatgatggg 1200
   actcaattgt atttttattt tcagtcactc ctgataaata acaaatttgg agaaataaaa 1260
   atatctaaaa taaattttgt ctgcagtata ttttcatata aaaatgcata tttgagtgct 1320
   acattcgaat aaatactacc tttttagtga a                                 1351
<210> 68
   <211> 2179
   <212> DNA
   <213> Homo sapiens
<220>
   <223> transketolase transcript variant 1 (TKT, TK, TKT1), epididymis luminal protein 107 (HEL107), Wernicke-Korsakoff syndrome
<220>
   <221> CDS
   <222> (173) ... (2044)
   <223> TKT
<400> 68
   gatccgagcc ccgcctcctc cccctgcccc gcctctccca tccccgcccc gccccgcccg 60
   gcgacttaac gcgcccccgc cccgcgcccg gcctcggcag ccgcctgtcg ccgcgggagc 120
   agccgctatc tctgtgtgtc cgcgtgtgcg cccggtcccc gcctgccgca ccatggagag 180
   ctaccacaag cctgaccagc agaagctgca ggccttgaag gacacggcca accgcctacg 240
   tatcagctcc atccaggcca ccactgcggc gggctctggc caccccacgt catgctgcag 300
   cgccgcagag atcatggctg tcctcttttt ccacaccatg cgctacaagt cccaggaccc 360
   ccggaatccg cacaatgacc gctttgtgct ctccaagggc catgcagctc ccatcctcta 420
   cgcggtctgg gctgaagctg gtttcctggc cgaggcggag ctgctgaacc tgaggaagat 480
   cagctccgac ttggacgggc acccggtccc gaaacaagct ttcaccgacg tggccactgg 540
   ctccctgggc cagggcctcg gggccgcttg tgggatggcc tacaccggca aatacttcga 600
   caaggccagc taccgagtct attgcttgct gggagacggg gagctgtcag agggctctgt 660
   atgggaggcc atggccttcg ccagcatcta taagctggac aaccttgtgg ccattctaga 720
   catcaatcgc ctgggccaga gtgacccggc cccactgcag caccagatgg acatctacca 780
   gaagcggtgc gaggccttcg gttggcatgc catcatcgtg gatggacaca gcgtggagga 840
   gctgtgcaag gcctttggcc aggccaagca ccagccaaca gccatcattg ccaagacctt 900
   caagggccga gggatcacgg gggtagaaga taaggagtct tggcatggga agcccctccc 960
   caaaaacatg gctgagcaga tcatccagga gatctacagc cagatccaga gcaaaaagaa 1020
   gatcctggca acccctccac aggaggacgc accctcagtg gacattgcca acatccgcat 1080
   gcccagcctg cccagctaca aagttgggga caagatagcc acccgcaagg cctacgggca 1140
   ggcactggcc aagctgggcc atgccagtga ccgcatcatc gccctggatg gggacaccaa 1200
   aaattccacc ttctcggaga tcttcaaaaa ggagcacccg gaccgcttca tcgagtgcta 1260
   cattgctgag cagaacatgg tgagcatcgc ggtgggctgt gccacccgca acaggacggt 1320
   gcccttctgc agcacttttg cagccttctt cacgcgggcc tttgaccaga ttcgcatggc 1380
   cgccatctcc gagagcaaca tcaacctctg cggctcccac tgcggcgttt ccatcgggga 1440
   agacgggccc tcccagatgg ccctagaaga tctggctatg tttcggtcag tccccacatc 1500
   aactgtcttt tacccaagtg atggcgttgc tacagagaag gcagtggaac tagccgccaa 1560
   tacaaagggt atctgcttca tccggaccag ccgcccagaa aatgccatca tctataacaa 1620
   caatgaggac ttccaggtcg gacaagccaa ggtggtcctg aagagcaagg atgaccaggt 1680
   gaccgttatc ggggctgggg tgaccctgca cgaggccttg gccgctgccg aactgctgaa 1740
   gaaagaaaag atcaacatcc gcgtgctgga ccccttcacc atcaagcccc tggacagaaa 1800
   actcattctc gacagcgctc gtgccaccaa gggcaggatc ctcaccgtgg aggaccatta 1860
   ttatgaaggt ggcattggtg aggctgtgtc cagtgcagta gtgggcgagc ctggcatcac 1920
   tgtcacccac ctggcagtta accgggtacc aagaagtggg aagccggctg agctgctgaa 1980
   gatgtttggt atcgacaggg atgccattgc acaagctgtg aggggcctca tcaccaaggc 2040
   ctagggcggg tatgaagtgt ggggcggggg tctatacatt cctgagattc tgggaaaggt 2100
   gctcaaagat gtactgagag gaggggtaaa tatatgtttt gagaaaaatg aattggccct 2160
   gaaaaaaaaa aaaaaaaaa                                              2179
<210> 69
   <211> 2617
   <212> DNA
   <213> Homo sapiens
<220>
   <223> lipase maturation factor 2 (LMF2), transmembrane protein 153 (TMEM153), transmembrane protein 112B (TMEM112B)
<220>
   <221> CDS
   <222> (32)...(2155)
   <223> LMF2
<400> 69
   gggccctgct ctagcgggcc gcgtagcgga catggcgggc tcccggctcc cgcggcagct 60
   cttcctccag ggcgtggcgg ccgtcttcat gtttgctttc gcttccctct acacgcaaat 120
   cccaggcctg tatggccccg agggcatcct acctgcaagg aggacgctgc ggcctcaggg 180
   caaggggcgc tggcagcagc tgtgggagac cccgacgctg ctgtgggaag cgccgagact 240
   ggggctggac acggcccagg gcctggagct gctgagcctg ctgggtgcac tagtggccct 300
   gggagccctg ctgctgagcc cactgcgcca ccctgtcatc tacttgctgc tttgggccgc 360
   ctacctgtca gcctgccagg tgggccaggt gttcctttat ttccagtggg actccctgct 420
   gctagagact ggcttcctgg ccgtgctggt ggccccgctg aggccagcct cccaccgcaa 480
   ggaggccccc cagggcaggc aggcaggggc cctgccccac gaagacctcc ccttctggct 540
   ggtgcgatgg ctgctgttcc gcctcatgtt cgcctcaggc gtggtcaagc tgaccagccg 600
   ctgccctgcg tggtgggggc tcactgccct cacctaccac tacgagaccc agtgcctgcc 660
   cacgcccgcc gcctggttcg cacaccacct gccggtctgg ctgcacaagc tcagcgtggt 720
   ggccaccttc ctaattgaga tcgctgtgcc gcccctgttc ttcgccccca ttcgacgcct 780
   gcgcttggct gctttctact cgcaggtgct gctgcaggtc ctgattatca tcaccggcaa 840
   ctacaacttc ttcaacctga tgacgctggt gcttaccact gcgctgctgg acgaccagca 900
   cctggctgct gagcctggcc acggcagccg caagaagacg gccacctcct ggcccaaggc 960
   cctgctggcc accctgtcgc tgctgctgga actagccgtc tacgggcttc tggcctatgg 1020
   cactgtgcac tactttggcc tggaggttga ctggcagcag cgcaccatcc actccagaac 1080
   cactttcacc ttccaccagt tttctcagtg gctgaagaca ctgacgctgc ccactgtgtg 1140
   gctgggtgtg gcctccctgg tctgggagct gctgagtgcc ctgtggaggt ggacccaggt 1200
   gcggggctgg ctacggaagc tcagtgctgt agtccaactg tcccttgtgg gcactgcgac 1260
   cgtggccttg ttcctgatta gcctggtgcc gtactcctac gtggagcccg ggacccacgg 1320
   gcgcctctgg accgggcccc accgcctgtt tggtgccgtg gagcacctac agctggccaa 1380
   ctcctacggc ctcttccgcc gcatgactgg gcttggtgga cggcctgagg tggtgctgga 1440
   gggcagttac gacggccacc actggacgga gatcgagttc atgtacaagc ctgggaacct 1500
   gagccggccg cccccggttg tggtgcccca ccagccacgc ctggactggc agatgtggtt 1560
   tgcagccctg ggcccacaca cgcacagccc gtggttcaca agcctggtct tgcgcctgct 1620
   gcagggcaag gagccagtga tccgccttgt ccagagccaa gtggccaggt atcccttcca 1680
   caagcagccg cccacctacg tccgagccca gcgctacaag tactggttct cccagcctgg 1740
   ggagcagggc cagtggtggc ggcgccagtg ggtggaggag ttcttcccat ccgtgtccct 1800
   gggggacccc acgctggaga cgctgctcag gcagtttgga ctacaggaga aaagcccacc 1860
   tcgcacccgc agcgccaaca gcaccctggc ccaggccctc cactggactc gctctcagct 1920
   gtctcccctg gaggcccccg ccctgctctg ggggctcctc atggccgtgg gggctgtcag 1980
   atttgtgcaa gccctgctag caccctgttc tctccggtcc tccccgctgg caccagtcag 2040
   cggggagaag cgcaggccag cctcccagaa agactccgga gctgcctccg aacaggccac 2100
   cgcagccccc aacccctgct ccagtagttc gaggaccacc cggcgaaaga agtagctgtg 2160
   ttctcccagc tgcacgtcct gagagggcca ggtcgccggg agtgctctgg cctccggcag 2220
   gacaggaccc agccactgtg ccttagctga ccctgcaggg ccaggcacag gttggggggc 2280
   tgcccctggg gtttgcaggg tgctgcattg agggctccag gccccacccc cacgccagcc 2340
   atgcccctcc ccaggactcc cactattgcc tctgtgattg gcccaggagg aaaacacgac 2400
   caagctcaag acccttcccc tgccctgggc tgtgggggtc tgagtctaga gcccccaacc 2460
   ctaggccccg tgccagaggg gaagaggctg actcccaggg gaagagggga agcactgtca 2520
   tcttccacgt catcttcaca ccagcccatc ctgcccttta gatctgggca ccaataaagg 2580
   cgtcttttgt gcttggctga aaaaaaaaaa aaaaaaa                         2617
<210> 70
   <211> 4499
   <212> DNA
   <213> Homo sapiens
<220>
   <223> 60S ribosomal protein L13 transcript variant 1, L13, breast basic conserved protein 1 (BBC1), D16S444E, D16S44E, OK/SW-cl.46
<220>
   <221> CDS
   <222> (77)...(712)
   <223> RPL13
<400> 70
   ccagagtgca ttgcggggcc gcttcctttc cgctcggctg ttttcctgcg caggagccgc 60
   agggccgtag gcagccatgg cgcccagccg gaatggcatg gtcttgaagc cccacttcca 120
   caaggactgg cagcggcgcg tggccacgtg gttcaaccag ccggcccgta agatccgcag 180
   acgtaaggcc cggcaagcca aggcgcgccg catcgccccg cgccccgcgt cgggtcccat 240
   ccggcccatc gtgcgctgcc ccacggttcg gtaccacacg aaggtgcgcg ccggccgcgg 300
   cttcagcctg gaggagctca gggtggccgg cattcacaag aaggtggccc ggaccatcgg 360
   catttctgtg gatccgagga ggcggaacaa gtccacggag tccctgcagg ccaacgtgca 420
   gcggctgaag gagtaccgct ccaaactcat cctcttcccc aggaagccct cggcccccaa 480
   gaagggagac agttctgctg aagaactgaa actggccacc cagctgaccg gaccggtcat 540
   gcccgtccgg aacgtctata agaaggagaa agctcgagtc atcactgagg aagagaagaa 600
   tttcaaagcc ttcgctagtc tccgtatggc ccgtgccaac gcccggctct tcggcatacg 660
   ggcaaaaaga gccaaggaag ccgcagaaca ggatgttgaa aagaaaaaat aaagccctcc 720
   tggggacttg gaatcagtcg gcagtcatgc tgggtctcca cgtggtgtgt ttcgtgggaa 780
   caactgggcc tgggatgggg cttcactgct gtgacttcct cctgccaggg gatttggggc 840
   tttcttgaaa gacagtccaa gccctggata atgctttact ttctgtgttg aagcactgtt 900
   ggttgtttgg ttagtgactg atgtaaaacg gttttcttgt ggggaggtta cagaggctga 960
   cttcagagtg gacttgtgtt ttttcttttt aaagaggcaa ggttgggctg gtgctcacag 1020
   ctgtaatccc agcactttga ggttggctgg gagttcaaga ccagcctggc caacatgtca 1080
   gaactactaa aaataaagaa atcagccatg cttggtgctg cacacttgta gttgcagctc 1140
   ctgggaggca gaggtgaggg atcacttaac ccaggaggca gaggctgcac tgagccagga 1200
   tcacgccact gcactctagc ctgggcaaca gtgagactgt ctcaaaaaaa aaaaaagaga 1260
   cagggtcttc ggcacccagg ctggagtaca gtgccacaat catggctcac tgcagtcttg 1320
   aactcatggc ctcaagcagt cctccctcag cctcccaagt agaggggttt ataggcacga 1380
   gaccctgcac ccaacctaga gttgcctttt ttaagcaaag cagtttctag ttaatgtagc 1440
   atcttggact ttggggcgtc attcttaagc ttgttgtgcc cggtaaccat ggtcctcttg 1500
   ctctgattaa cccttccttc aatgggcttc ttcacccaga caccaaggta tgagatggcc 1560
   ctgccaagtg tcggcctctc ctgttaaaca aaaacattct aaagccattg ttcttgcttc 1620
   atggacaaga ggcagccaga gagagtgcca gggtgccctg gtctgagctg gcatccccat 1680
   gtcttctgtg tccgagggca gcatggtttc tcgtgcagtg ctcagacaca gcctgcccta 1740
   gtcctaccag ctcacagcag cacctgctct ccttggcagc tatggccatg acaaccccag 1800
   agaagcagct tcagggaccg agtcagattc tgttttgtct acatgcctct gccgggtgcc 1860
   ggtattgagg cacccaggga gctgttactg gcgtggaaat aggtgatgct gctacctctg 1920
   ctgctgcact cacagccaca cttgatacac gatgacacct tgcttgtttg gaaacatcta 1980
   aacatctagt agatgacttg caggctgttg gctaccagtt tcctgtctga ggtgtatatg 2040
   ttaacttcgt gatcagtttg tatgtttggg actcttgtcc tatgtaaagt taaggtgggc 2100
   cgggtgcagt ggctcacgcc tgtaatccta acactgggag gccgaggcgg gtggatcacc 2160
   tgatggtgaa acctcatctc tactgaaaat acaaaaatta gctgagtggt gacacacgcc 2220
   tgtaatccca gctacttggt aggcttgaac ccaggaggca gagattgcag tgagccgagc 2280
   tgcaccactg tgctccagcc tgggtgacag cgagactcag tctcaaaaaa agttgtacaa 2340
   ggtggatggt tggaagcttg agcctaggct cgaatccctc tcacgtgaga gggcctgaag 2400
   atttctggtg gattccaacc tggctgaaga ctggccgtgg ggggtgcagg ggtctccagc 2460
   gctctgccct ccagcctgct tcctccctgc ccacaccgca ctaggggaag ggcctttcct 2520
   gctgcctgcg gggccgcacc tggagtaggt aatgccatgt ggtgacgtga atggagcaga 2580
   ggtctgtgcc ccatcacacc gccttgctgt ttttactgtg ggacaaaagc actctgatct 2640
   gcgtgttccg gggcccctcc taccagccga cttgacggga agtcagggtt caggtatcat 2700
   ctgtgcacct ggggcggggt agtctgcact gaacctgcca gagtcccctc ctcatttcac 2760
   tgaaagtcac agtctccagg gctgtgttgc taaccttacg ttctctccgt ttgcttaatc 2820
   tattaagagc cctaacagga gaggatgggc tttctctgtt gtctggggcc ctgctgttgg 2880
   ccggtgctct tagcaagagg tcatttttct aggttgcgct gggacattgt gagtttggtg 2940
   agggtcatgg atgtgggctg ggctgggctg ggctgggccg ggctgcctgc tgcctgctgc 3000
   tcccctacct gaaatgcagc tagtgcggct ctgcccttcc tggggctgag gaaggcttct 3060
   gcaggatagc tggggggctg ggcaggtggg tgaggcagcc tccctgctga cactcagtcc 3120
   ttgtagctgg agcaagatct cctgatccag gtacgggcct gtctgctcca agaaagactc 3180
   tgccaccaga tgcaaagggg ccctttgttt taacttagtc cctggggacc gcctgattca 3240
   gcacctgtcg gcccaggata ccccgctggt ggggacaagt gcctgagtgt gggccgtgcc 3300
   cgagtgtggc catccctgag tggggccgtc ctgactagga agtggctttt cagttgtgat 3360
   gtgtgggcct gacctagggg gcgctgtgga acccgggctg gaaccagccc tctgtgccag 3420
   gccgcagaca ggttccgccg gccctgaggg gcagctgcca tggcgtgggt cactgggagc 3480
   tgagaggaag ggcccccacc gcacctcagg caaagcggct ctgggaacac cttgatttcg 3540
   tccatgtgag ccgtcccagg gagggcagcc aagctgtgaa gcctgagaaa ctgacctgtg 3600
   tgccacgagc ttgtggtctg ctgcccggtg gaggaagtgc aggtgcgccc aggctcctca 3660
   ttccgttttg caggattcct tcggggtgtg agcatttcct attcagcctg tcgcccccgg 3720
   ggagcacggg ctggctctgt ggtgcccgtg gccttttgta gaagcgttgg ttttacggca 3780
   ggttcatctc tggggcagcc tcccacagtg ggtggggctt tgccagcagt gcccacgggg 3840
   gtcatggggc caggcgcgct ccggcgcctg cagaactgat cggggatagt ctcaggaggc 3900
   gctagtcacg tgccccggtg atcggggata gtctcagaag gcgctagtct cctgccccgg 3960
   tgatcgggga tagtctcagg aggcacgagt cgcctgcctc ggtgatgcac cgtttctcac 4020
   accggctgct ctggcccgag ctaaagggga agacgtgtgc ggataggagc tgcacacaat 4080
   tttcctccat gtattgttta ttttgctttt tcttttggct agacattagg aatttcagtt 4140
   ttcccaagtt gtatttttcc ttttctattt taaaattatc atgcagggct gggtgaggtc 4200
   gctcacgcct atagtctcaa aactttggga ggctgagggg ggaggatggc atgagcccag 4260
   gagtttaagg ctgcagtgag ccgagatcgc tccactgtcc tccagcctgc atgacagagc 4320
   gagaccctat ctcaggaaaa aaaaaaacaa aactattatg cagtagtttc gaccctggaa 4380
   gacgagtgtg catctttgag ttgtaacacg tgtacctcgc ccatccaggc gtagtttcat 4440
   ttggaatctg gttatcctgt agttgctttg ttaaaaatat atgtaattgc aaatcattt  4499
<210> 71
   <211> 1494
   <212> DNA
   <213> Homo sapiens
<220>
   <223> cathepsin H (CTSH), pro-cathepsin H preproprotein, cathepsin B3, cathepsin BA, N-benzoylarginine-beta-naphthylamide hydrolase, aleurain, ACC-4, ACC-5, CPSB
<220>
   <221> CDS
   <222> (98)...(1105)
   <223> CTSH
<400> 71
   ccacgctcgt gccgctcccc ccccgcgctc ccagttgacg ctctgggccg ccacctccgc 60
   ggaccctgag cgcaagagcc aagccgccag cgctgcgatg tgggccacgc tgccgctgct 120
   ctgcgccggg gcctggctcc tgggagtccc cgtctgcggt gccgccgaac tgtgcgtgaa 180
   ctccttagag aagtttcact tcaagtcatg gatgtctaag caccgtaaga cctacagtac 240
   ggaggagtac caccacaggc tgcagacgtt tgccagcaac tggaggaaga taaacgccca 300
   caacaatggg aaccacacat ttaaaatggc actgaaccaa ttttcagaca tgagctttgc 360
   tgaaataaaa cacaagtatc tctggtcaga gcctcagaat tgctcagcca ccaaaagtaa 420
   ctaccttcga ggtactggtc cctacccacc ttccgtggac tggcggaaaa aaggaaattt 480
   tgtctcacct gtgaaaaatc agggtgcctg cggcagttgc tggactttct ccaccactgg 540
   ggccctggag tctgcgatcg ccatcgcaac cggaaagatg ctgtccttgg cggaacagca 600
   gctggtggac tgcgcccagg acttcaataa tcacggctgc caagggggtc tccccagcca 660
   ggctttcgag tatatcctgt acaacaaggg gatcatgggt gaagacacct acccctacca 720
   gggcaaggat ggttattgca agttccaacc tggaaaggcc atcggctttg tcaaggatgt 780
   agccaacatc acaatctatg acgaggaagc gatggtggag gctgtggccc tctacaaccc 840
   tgtgagcttt gcctttgagg tgactcagga cttcatgatg tatagaacgg gcatctactc 900
   cagtacttcc tgccataaaa ctccagataa agtaaaccat gcagtactgg ctgttgggta 960
   tggagaaaaa aatgggatcc cttactggat cgtgaaaaac tcttggggtc cccagtgggg 1020
   aatgaacggg tacttcctca tcgagcgcgg aaagaacatg tgtggcctgg ctgcctgcgc 1080
   ctcctacccc atccctctgg tgtgagccgt ggcagccgca gcgcagactg gcggagaagg 1140
   agaggaacgg gcagcctggg cctgggtgga aatcctgccc tggaggaagt tgtggggaga 1200
   tccactggga cccccaacat tctgccctca cctctgtgcc cagcctggaa acctacagac 1260
   aaggaggagt tccaccatga gctcacccgt gtctatgacg caaagatcac cagccatgtg 1320
   ccttagtgtc cttcttaaca gactcaaacc acatggacca cgaatattct ttctgtccag 1380
   aagggctact ttccacatat agagctccag ggactgtctt ttctgtattc gctgttcaat 1440
   aaacattgag tgagcacctc cccagatgga gcatgctggt cctggaaaaa aaaa       1494
<210> 72
   <211> 5604
   <212> DNA
   <213> Homo sapiens
<220>
   <223> family with sequence similarity 83, member H, FAM83H variant 1 (FAM83H), AI3
<220>
   <221> CDS
   <222> (70)...(3609)
   <223> FAM83H
<400> 72
   gggcggcggt cggctcctgc tgcccctgtg ccgagacccc gcgcacctgg ccaggcccct 60
   ggccccaaca tggcccgtcg ctctcagagc tcctcgcagg gggacaaccc actggcaccc 120
   gggtacctgc cgcctcacta caaagagtac taccgcctgg cggtggatgc actggccgag 180
   ggtggctcgg aggcctacag ccgcttcctc gctaccgagg gggcaccaga cttcctgtgc 240
   cctgaagagc tggaacatgt gagccgacac cttcggcctc cgcagtatgt tacccgagag 300
   ccacctgaag gcagccttct cgacgtggac atggatggct cctcgggtac atactggcca 360
   gtgaactcag accaggccgt gcctgagctt gatttgggct ggcctctgac cttcggcttc 420
   cagggcaccg aggtgaccac cttggtgcag ccaccgcccc ccgacagccc cagtatcaag 480
   gatgaggccc gcaggatgat ccgttccgcc cagcaggtgg tggccgtggt gatggacatg 540
   ttcactgatg tggacctgct cagcgaagtg ctggaggccg cggcccgtcg ggtcccagtc 600
   tacatcctgc tggatgagat gaacgcgcag cacttcctgg acatggccga caagtgccgt 660
   gtcaacctgc accacgtgga tttcctgcgc gtacggactg tggcgggccc cacctactac 720
   tgccgcactg ggaagtcctt caagggccac gtcaaggaga agttcctgct ggtggactgt 780
   gccgtggtga tgagtgggag ctacagcttc atgtggtcct ttgagaagat ccaccgcagc 840
   ctggcgcacg tgttccaagg agagctggtc tccagcttcg acgaggagtt ccgcatcctc 900
   ttcgcgcagt ccgagccgct tgtgccctcg gccgcggccc tggcccgcat ggacgcctat 960
   gccctggctc cgtatgccgg ggccgggcct ctcgtgggcg tccctggggt cggggcgcca 1020
   acccccttct ccttccctaa acgagcgcac ctcctgttcc cgccaccccg ggaagagggc 1080
   ctgggcttcc cctccttcct cgacccggac cgccacttcc tgtcggcctt ccgccgggag 1140
   gagccgccgc ggatgccggg gggcgcgctg gaaccgcacg cggggctgcg gccgctctcg 1200
   cggcgcctgg aggccgaggc cgggccggct ggggagctcg cgggcgcgcg gggcttcttc 1260
   caggcgcggc acctggagat ggacgccttc aagcggcaca gcttcgcgac cgagggcgcg 1320
   ggcgccgtgg agaacttcgc ggccgcgcgg caggtgtcgc ggcagacgtt cctcagccac 1380
   ggcgacgact tccgcttcca gaccagccac ttccaccgtg accagctcta ccagcagcag 1440
   taccagtggg acccgcagct cacgccggcg cgcccgcaag gcctgttcga gaagcttcgc 1500
   gggggccgcg cgggtttcgc ggacccggat gacttcaccc tgggcgccgg gccccgcttc 1560
   ccggagctcg gacccgacgg gcaccagcgg ctggactacg tgccgtccag cgcgtcccgc 1620
   gaggtgcgcc acggctcgga ccccgccttc gcgcccggac cccgcggcct ggagcccagc 1680
   ggagccccgc gccccaacct gacccagcgc ttcccatgcc aggccgcggc gaggccgggc 1740
   ccagaccccg ctcccgaggc ggagccggag cgcaggggcg ggcccgaggg gcgggcaggg 1800
   ctgcggcgct ggcgtttggc ctcctacttg agcggctgcc acggcgagga tgggggcgac 1860
   gacggcctac cggcgcccat ggaagcggag gcttacgaag acgacgtgct ggctcccggg 1920
   ggccgggcac ctgccggcga cctgctcccc tcggccttcc gcgtcccagc agccttcccc 1980
   accaaggtcc cggtgccagg cccgggcagc ggcggcaacg gcccagagcg cgagggcccg 2040
   gaggagcctg gcctggccaa gcaggactca ttccgctcgc gcctgaaccc cctggtccag 2100
   cgcagctcca ggctgcgctc ctcgctcatc ttcagcacgt cacaggccga gggcgcggcc 2160
   ggggctgcgg cggccactga gaaggtgcag ctgctgcaca aggagcagac ggtcagcgag 2220
   acgctggggc ccggcggaga ggccgtgcgc tccgcggctt ccaccaaggt ggcggagctg 2280
   ctggagaagt acaagggccc agcccgtgat cccggcggcg gcgcgggcgc catcaccgtt 2340
   gccagccaca gcaaggccgt cgtgtcccag gcgtggcggg aagaggtggc ggccccaggt 2400
   gccgtggggg gcgagcgccg cagcctcgag agctgcctgc tggacctgcg cgactccttt 2460
   gcacagcagc tgcaccagga ggcggagcgg cagccgggag ccgcgtcgct caccgcggcg 2520
   cagctgctcg acacactggg ccggagcggc tccgaccgcc tgccttcccg cttcctctct 2580
   gcccagagcc actcaacgtc cccgcaaggg ctggacagcc ctctgccgct ggaagggtcc 2640
   ggagcgcacc aggtgctcca taatgagtca aaagggagcc ccacctcggc ttaccctgag 2700
   cggaagggga gccccacgcc tgggttttcc actcgaagag gaagtccaac tacaggattt 2760
   atcgagcaga aggggagccc cacctcagcc taccccgagc gcaggggtag tccggtgccc 2820
   cccgtgccgg agcgcaggag cagtccggtg ccccccgtgc cggagcgcag gggcagcctc 2880
   acccttacca tctccgggga gtccccgaag gccgggcccg cggaggaggg gccgagcggc 2940
   cccatggaag tcctgcgcaa aggctccttg cgtcttaggc agctgctgag ccccaagggc 3000
   gagcggcgca tggaggatga gggtggcttc ccagtgccgc aggagaacgg ccaacccgag 3060
   agcccgcggc gtctgtcact gggccagggt gacagcacgg aggctgccac agaagagcgg 3120
   ggtccgcggg cgcgcctgtc ctcagccacg gccaacgcct tgtacagcag caaccttcgg 3180
   gatgacacga aggccattct ggagcagatc agtgcccacg gccagaagca ccgtgcggtc 3240
   cctgccccga gccccggccc gacccacaac agccccgagc taggccgtcc accggctgct 3300
   ggcgtcctgg ccccagatat gtccgacaag gacaagtgtt cagccatctt ccgctcggac 3360
   agcttgggga cccagggccg gctgagccgc acgctgccag ccagcgcgga ggagcgcgat 3420
   cggctgctgc gccgcatgga gagcatgcgc aaggagaagc gcgtgtacag ccgcttcgag 3480
   gtcttctgca agaaagagga ggccagcagc cctggggcag gggaaggccc cgcggaggag 3540
   ggcaccaggg acagcaaggt gggcaagttc gtgcccaaga tcctgggcac gttcaaaagc 3600
   aagaagtgag tcttctggcc tggcaaccca ggccagggtg cccgcatcgc tgccccggtc 3660
   atccagaagc cccgcggaac agagagccct gctcatgtgc ttgagcagcg gctgtcaggc 3720
   cacggccgct tggggcttgg ctgagtgcgc cagacctcgg ctccactgga ggctcacctg 3780
   gcagctgccg tctctgcccc ctggcctccc caacgctggg gctgcacccc tcgccaccag 3840
   tgcctttctc ccctcagcac cttcatctct gcaccgtcag ccttgcgtgg cgcagcgtct 3900
   ggctccgcca tctctttgtg cctcagtccc ccccgccccc tttatttttt tgagacctag 3960
   ggctggagtg cagttgagcg gtctgggctc actgcaacct ctgcctcccg ggttccagcg 4020
   attctcctgc ctcagcctcc tgagtagctg ggattacaga tgtatgctac cacgcccagg 4080
   tagtttttgt atttttagta gagacagggt ttcactatgt tggccaggct ggtctccaac 4140
   tcctggcctc aaatgatcag cccgcttcag cctcccaaag tggggggatt acaggcgtga 4200
   gccttgcacc ccgctaagtc ccctatcctc ttgcaagggt ctcgcctctg tgcctcaatt 4260
   cctcattctc tgggcccttc tcctcctcag ggcctcctgt tctcagggcc tcccccctcc 4320
   ccgctccctc cctctctcaa ggtctcctcc ttccctcccc cccccgtctc ccccctcccc 4380
   cgcctgggct tcacttcctt tcctacttgg attctcctgc tcgctgcctc ccagcatctt 4440
   ttttggaggc ccgtctcttg ctgtggggaa gactgggctg gctgcgggca gtttgcaagg 4500
   ggtgggtggg gcgggggggg gagctggacc agaagatgcc ccttggagtg gcaaggaagc 4560
   tggacagggc aggcctctgg ggacgggaca cagggaagcc cgaaggggcg ccttggccag 4620
   gtctgccatc tcctccagcg aggctctggc cagcactggg tgagagtggg gagggggcac 4680
   tggcctttgc agcacagtaa aacatggtcc agacaacctg tggccccggc ctcatgagca 4740
   ccccctgcac aggcccggcc caagccaggc gctagaaggg ctggttgtgg agtgcttatc 4800
   cttgacaggt atggggccag gtgagggcag gggacaaggt gcagctgagg ccgagcccaa 4860
   ctaggtcctg ggcacccctg caggtgggag tggtccttgt cctcctggta tccagcagac 4920
   acccccctct ccccaccagc cccattctca ggtcctttcc tctttgtcac caacaccaag 4980
   aatctgtcca gggttcttgg cttatctttt atctcttttc actcctagag aggaattgca 5040
   attgactcag aatgacacat tttggcacca cgtgtgtaga aagcccccac tgttagatga 5100
   tagcctcgtg aaattcatgt ttctgtattc tcctatttct tttcaaaaac taattttttt 5160
   tttagtgtaa taaatcctaa gagggaactg atttaagaaa caaggccgcc aaacaaaggc 5220
   agcagttccg actccagcag ctgggaaagg aaggaaagtg accccacttt cactcctgca 5280
   cagcccactg gttaccaaaa ccaccgtgca agtcgggatg acagcaggga cttctggcca 5340
   ggtgggaaag gtgcctggaa gcgggatgcg cctgtgcgtc tcttggccat gatgttcttg 5400
   tgggcatgtt attcttggtg ctgcctgggg tgttgctgag cggacaggct ctccagctgg 5460
   agtccatgga gaggccagag gctggcggcc ctgcctgggc cttcggagcc tcctgcctgc 5520
   accctccacc tcttctaaac catgatgtgg cacattttgg tgttaataaa acacaacaca 5580
   caaagtaaaa aaaaaaaaaa aaaa                                        5604
<210> 73
   <211> 735
   <212> DNA
   <213> Homo sapiens
<220>
   <223> 40S ribosomal protein S29 transcript variant 2, S29
<220>
   <221> CDS
   <222> (31)...(234)
   <223> RPS29
<400> 73
   cttttacctc gttgcactgc tgagagcaag atgggtcacc agcagctgta ctggagccac 60
   ccgcgaaaat tcggccaggg ttctcgctct tgtcgtgtct gttcaaaccg gcacggtctg 120
   atccggaaat atggcctcaa tatgtgccgc cagtgtttcc gtcagtacgc gaaggatatc 180
   ggtttcatta agaaagacct gagctgtctt ccttggcact gcctatggag gtgacaccca 240
   tctcctccat catggccatc ctgagaccgc tcgcgaagcc caagatcatc aaaaagagca 300
   ccaagttcac tgggaaccag tcagactgat atgtcaaaat taagggtaac tggtggaaac 360
   acagaggtat taacaacagg gttcatagaa ggtttgaggg ccagatctat gcccaacatt 420
   ggttatggga gaaacaaaaa gacaaagcac atactgccca gtggcttctg gaagttcctg 480
   gtccacaacg ttaaggagct ggaagtactg ctggtgagca acaaatctta ctgtgttgag 540
   atcactcatg atgtttcttc caagaactgc aaagccatct tggaaagagc agcccaggtg 600
   gtcatcagag tcaccaatgc caatgccagc ctgcacagtg cagaaagtga atagacagtg 660
   aatgtgtttg ttttattggg gtttaaataa aaccaataaa actgtaaaaa caaaaacaac 720
   aaaaaaaaaa aaaaa                                                  735
<210> 74
   <211> 594
   <212> DNA
   <213> Homo sapiens
<220>
   <223> 60S ribosomal protein L23, L23, ribosomal protein L17, rpL17
<220>
   <221> CDS
   <222> (91)...(513)
   <223> RPL23
<400> 74
   ggccacgtga ggagggtggg cggggcgtta aagttcatat cccagtgtcc tttgaatcga 60
   cttccttttt tcttttttcc ggcgttcaag atgtcgaagc gaggacgtgg tgggtcctct 120
   ggtgcgaaat tccggatttc cttgggtctt ccggtaggag ctgtaatcaa ttgtgctgac 180
   aacacaggag ccaaaaacct gtatatcatc tccgtgaagg ggatcaaggg acggctgaac 240
   agacttcccg ctgctggtgt gggtgacatg gtgatggcca cagtcaagaa aggcaaacca 300
   gagctcagaa aaaaggtaca tccagcagtg gtcattcgac aacgaaagtc ataccgtaga 360
   aaagatggcg tgtttcttta ttttgaagat aatgcaggag tcatagtgaa caataaaggc 420
   gagatgaaag gttctgccat tacaggacca gtagcaaagg agtgtgcaga cttgtggccc 480
   cggattgcat ccaatgctgg cagcattgca tgattctcca gtatatttgt aaaaaataaa 540
   aaaaaaaact aaacccatta aaaagtattt gtttgcaaaa aaaaaaaaaa aaaa       594
<210> 75
   <211> 514
   <212> DNA
   <213> Homo sapiens
<220>
   <223> 40S ribosomal protein S25, S25
<220>
   <221> CDS
   <222> (64)...(441)
   <223> RPS25
<400> 75
   cttccttttt gtccgacatc ttgacgaggc tgcggtgtct gctgctattc tccgagcttc 60
   gcaatgccgc ctaaggacga caagaagaag aaggacgctg gaaagtcggc caagaaagac 120
   aaagacccag tgaacaaatc cgggggcaag gccaaaaaga agaagtggtc caaaggcaaa 180
   gttcgggaca agctcaataa cttagtcttg tttgacaaag ctacctatga taaactctgt 240
   aaggaagttc ccaactataa acttataacc ccagctgtgg tctctgagag actgaagatt 300
   cgaggctccc tggccagggc agcccttcag gagctcctta gtaaaggact tatcaaactg 360
   gtttcaaagc acagagctca agtaatttac accagaaata ccaagggtgg agatgctcca 420
   gctgctggtg aagatgcatg aataggtcca accagctgta catttggaaa aataaaactt 480
   tattaaatca aaaaaaaaaa aaaaaaaaaa aaaa                             514
<210> 76
   <211> 1794
   <212> DNA
   <213> Homo sapiens
<220>
   <223> tubulin, beta 3 class III transcript variant 1 (TUBB3), class III beta-tubulin, tubulin beta-4 chain (TUBB4), beta-4, CDCBM, CDCBM1, CFEOM3A
<220>
   <221> CDS
   <222> (124) ... (1476)
   <223> TUBB3
<400> 76
   gacatcagcc gatgcgaagg gcggggccgc ggctataaga gcgcgcggcc gcggtccccg 60
   accctcagca gccagcccgg cccgcccgcg cccgtccgca gccgcccgcc agacgcgccc 120
   agtatgaggg agatcgtgca catccaggcc ggccagtgcg gcaaccagat cggggccaag 180
   ttctgggaag tcatcagtga tgagcatggc atcgacccca gcggcaacta cgtgggcgac 240
   tcggacttgc agctggagcg gatcagcgtc tactacaacg aggcctcttc tcacaagtac 300
   gtgcctcgag ccattctggt ggacctggaa cccggaacca tggacagtgt ccgctcaggg 360
   gcctttggac atctcttcag gcctgacaat ttcatctttg gtcagagtgg ggccggcaac 420
   aactgggcca agggtcacta cacggagggg gcggagctgg tggattcggt cctggatgtg 480
   gtgcggaagg agtgtgaaaa ctgcgactgc ctgcagggct tccagctgac ccactcgctg 540
   gggggcggca cgggctccgg catgggcacg ttgctcatca gcaaggtgcg tgaggagtat 600
   cccgaccgca tcatgaacac cttcagcgtc gtgccctcac ccaaggtgtc agacacggtg 660
   gtggagccct acaacgccac gctgtccatc caccagctgg tggagaacac ggatgagacc 720
   tactgcatcg acaacgaggc gctctacgac atctgcttcc gcaccctcaa gctggccacg 780
   cccacctacg gggacctcaa ccacctggta tcggccacca tgagcggagt caccacctcc 840
   ttgcgcttcc cgggccagct caacgctgac ctgcgcaagc tggccgtcaa catggtgccc 900
   ttcccgcgcc tgcacttctt catgcccggc ttcgcccccc tcacagcccg gggcagccag 960
   cagtaccggg ccctgaccgt gcccgagctc acccagcaga tgttcgatgc caagaacatg 1020
   atggccgcct gcgacccgcg ccacggccgc tacctgacgg tggccaccgt gttccggggc 1080
   cgcatgtcca tgaaggaggt ggacgagcag atgctggcca tccagagcaa gaacagcagc 1140
   tacttcgtgg agtggatccc caacaacgtg aaggtggccg tgtgtgacat cccgccccgc 1200
   ggcctcaaga tgtcctccac cttcatcggg aacagcacgg ccatccagga gctgttcaag 1260
   cgcatctccg agcagttcac ggccatgttc cggcgcaagg ccttcctgca ctggtacacg 1320
   ggcgagggca tggacgagat ggagttcacc gaggccgaga gcaacatgaa cgacctggtg 1380
   tccgagtacc agcagtacca ggacgccacg gccgaggaag agggcgagat gtacgaagac 1440
   gacgaggagg agtcggaggc ccagggcccc aagtgaagct gctcgcagct ggagtgagag 1500
   gcaggtggcg gccggggccg aagccagcag tgtctaaacc cccggagcca tcttgctgcc 1560
   gacaccctgc tttcccctcg ccctagggct cccttgccgc cctcctgcag tatttatggc 1620
   ctcgtcctcc ccacctaggc cacgtgtgag ctgctcctgt ctctgtctta ttgcagctcc 1680
   aggcctgacg ttttacggtt ttgtttttta ctggtttgtg tttatatttt cggggatact 1740
   taataaatct attgctgtca gataccctta aaaaaaaaaa aaaaaaaaaa aaaa 1794
<210> 77
   <211> 660
   <212> DNA
   <213> Homo sapiens
<220>
   <223> 40S ribosomal protein S10 transcript variant 2, S10, DBA9
<220>
   <221> CDS
   <222> (95)...(592)
   <223> RPS10
<400> 77
   ggcggggggc gggtccacgc cagcccggaa gagacgcagc accgcgcatg ctccttcctt 60
   tccagccccg gtaccggacc ctgcagccgc agagatgttg atgcctaaga agaaccggat 120
   tgccatttat gaactccttt ttaaggaggg agtcatggtg gccaagaagg atgtccacat 180
   gcctaagcac ccggagctgg cagacaagaa tgtgcccaac cttcatgtca tgaaggccat 240
   gcagtctctc aagtcccgag gctacgtgaa ggaacagttt gcctggagac atttctactg 300
   gtaccttacc aatgagggta tccagtatct ccgtgattac cttcatctgc ccccggagat 360
   tgtgcctgcc accctacgcc gtagccgtcc agagactggc aggcctcggc ctaaaggtct 420
   ggagggtgag cgacctgcga gactcacaag aggggaagct gacagagata cctacagacg 480
   gagtgctgtg ccacctggtg ccgacaagaa agccgaggct ggggctgggt cagcaaccga 540
   attccagttt agaggcggat ttggtcgtgg acgtggtcag ccacctcagt aaaattggag 600
   aggattcttt tgcattgaat aaacttacag ccaaaaaacc ttaaaaaaaa aaaaaaaaaa 660
<210> 78
   <211> 8481
   <212> DNA
   <213> Homo sapiens
<220>
   <223> fatty acid synthase (FASN, FAS), short chain dehydrogenase/reductase family 27X, member 1 (SDR27X1), OA-519
<220>
   <221> CDS
   <222> (118) ... (7653)
   <223> FASN
<400> 78
   gagagacggc agcggccccg gcctccctct ccgccgcgct tcagcctccc gctccgccgc 60
   gctccagcct cgctctccgc cgcccgcacc gccgcccgcg ccctcaccag agcagccatg 120
   gaggaggtgg tgattgccgg catgtccggg aagctgccag agtcggagaa cttgcaggag 180
   ttctgggaca acctcatcgg cggtgtggac atggtcacgg acgatgaccg tcgctggaag 240
   gcggggctct acggcctgcc ccggcggtcc ggcaagctga aggacctgtc taggtttgat 300
   gcctccttct tcggagtcca ccccaagcag gcacacacga tggaccctca gctgcggctg 360
   ctgctggaag tcacctatga agccatcgtg gacggaggca tcaacccaga ttcactccga 420
   ggaacacaca ctggcgtctg ggtgggcgtg agcggctctg agacctcgga ggccctgagc 480
   cgagaccccg agacactcgt gggctacagc atggtgggct gccagcgagc gatgatggcc 540
   aaccggctct ccttcttctt cgacttcaga gggcccagca tcgcactgga cacagcctgc 600
   tcctccagcc tgatggccct gcagaacgcc taccaggcca tccacagcgg gcagtgccct 660
   gccgccatcg tgggggcatc caatgtcctg ctgaagccca acacctccgt gcagttcttg 720
   aggctgggga tgctcagccc cgagggcacc tgcaaggcct tcgacacagc ggggaatggg 780
   tactgccgct cggagggtgt ggtggccgtc ctgctgacca agaagtccct ggcccggcgg 840
   gtgtacgcca ccatcctgaa cgccggcacc aatacagatg gcttcaagga gcaaggcgtg 900
   accttcccct caggggatat ccaggagcag ctcatccgct cgttgtacca gtcggccgga 960
   gtggcccctg agtcatttga atacatcgaa gcccacggca caggcaccaa ggtgggcgac 1020
   ccccaggagc tgaatggcat cacccgagcc ctgtgcgcca cccgccagga gccgctgctc 1080
   atcggctcca ccaagtccaa catggggcac ccggagccag cctcggggct ggcagccctg 1140
   gccaaggtgc tgctgtccct ggagcacggg ctctgggccc ccaacctgca cttccatagc 1200
   cccaaccctg agatcccagc gctgttggat gggcggctgc aggtggtgga ccagcccctg 1260
   cccgtccgtg gcggcaacgt gggcatcaac tcctttggct tcgggggctc caacgtgcac 1320
   atcatcctga ggcccaacac gcagccgccc cccgcacccg ccccacatgc caccctgccc 1380
   cgtctgctgc gggccagcgg acgcacccct gaggccgtgc agaagctgct ggagcagggc 1440
   ctccggcaca gccaggacct ggctttcctg agcatgctga acgacatcgc ggctgtcccc 1500
   gccaccgcca tgcccttccg tggctacgct gtgctgggtg gtgagcgcgg tggcccagag 1560
   gtgcagcagg tgcccgctgg cgagcgcccg ctctggttca tctgctctgg gatgggcaca 1620
   cagtggcgcg ggatggggct gagcctcatg cgcctggacc gcttccgaga ttccatccta 1680
   cgctccgatg aggctgtgaa gccattcggc ctgaaggtgt cacagctgct gctgagcaca 1740
   gacgagagca cctttgatga catcgtccat tcgtttgtga gcctgactgc catccagata 1800
   ggcctcatag acctgctgag ctgcatgggg ctgaggccag atggcatcgt cggccactcc 1860
   ctgggggagg tggcctgtgg ctacgccgac ggctgcctgt cccaggagga ggccgtcctc 1920
   gctgcctact ggaggggaca gtgcatcaaa gaagcccatc tcccgccggg cgccatggca 1980
   gccgtgggct tgtcctggga ggagtgtaaa cagcgctgcc ccccgggcgt ggtgcccgcc 2040
   tgccacaact ccaaggacac agtcaccatc tcgggacctc aggccccggt gtttgagttc 2100
   gtggagcagc tgaggaagga gggtgtgttt gccaaggagg tgcggaccgg cggtatggcc 2160
   ttccactcct acttcatgga ggccatcgca cccccactgc tgcaggagct caagaaggtg 2220
   atccgggagc cgaagccacg ttcagcccgc tggctcagca cctctatccc cgaggcccag 2280
   tggcacagca gcctggcacg cacgtcctcc gccgagtaca atgtcaacaa cctggtgagc 2340
   cctgtgctgt tccaggaggc cctgtggcac gtgcctgagc acgcggtggt gctggagatc 2400
   gcgccccacg ccctgctgca ggctgtcctg aagcgtggcc tgaagccgag ctgcaccatc 2460
   atccccctga tgaagaagga tcacagggac aacctggagt tcttcctggc cggcatcggc 2520
   aggctgcacc tctcaggcat cgacgccaac cccaatgcct tgttcccacc tgtggagttc 2580
   ccagctcccc gaggaactcc cctcatctcc ccactcatca agtgggacca cagcctggcc 2640
   tgggacgtgc cggccgccga ggacttcccc aacggttcag gttccccctc agccgccatc 2700
   tacaacatcg acaccagctc cgagtctcct gaccactacc tggtggacca caccctcgac 2760
   ggtcgcgtcc tcttccccgc cactggctac ctgagcatag tgtggaagac gctggcccgc 2820
   gccctgggcc tgggcgtcga gcagctgcct gtggtgtttg aggatgtggt gctgcaccag 2880
   gccaccatcc tgcccaagac tgggacagtg tccctggagg tacggctcct ggaggcctcc 2940
   cgtgccttcg aggtgtcaga gaacggcaac ctggtagtga gtgggaaggt gtaccagtgg 3000
   gatgaccctg accccaggct cttcgaccac ccggaaagcc ccacccccaa ccccacggag 3060
   cccctcttcc tggcccaggc tgaagtttac aaggagctgc gtctgcgtgg ctacgactac 3120
   ggccctcatt tccagggcat cctggaggcc agcctggaag gtgactcggg gaggctgctg 3180
   tggaaggata actgggtgag cttcatggac accatgctgc agatgtccat cctgggctcg 3240
   gccaagcacg gcctgtacct gcccacccgt gtcaccgcca tccacatcga ccctgccacc 3300
   cacaggcaga agctgtacac actgcaggac aaggcccaag tggctgacgt ggtggtgagc 3360
   aggtggctga gggtcacagt ggccggaggc gtccacatct ccgggctcca cactgagtcg 3420
   gccccgcggc ggcagcagga gcagcaggtg cccatcctgg agaagttttg cttcactccc 3480
   cacacggagg aggggtgcct gtctgagcgc gctgccctgc aggaggagct gcaactgtgc 3540
   aaggggctgg tgcaggcact gcagaccaag gtgacccagc aggggctgaa gatggtggtg 3600
   cccggactgg atggggccca gatcccccgg gacccctcac agcaggaact gccccggctg 3660
   ttgtcggctg cctgcaggct tcagctcaac gggaacctgc agctggagct ggcgcaggtg 3720
   ctggcccagg agaggcccaa gctgccagag gaccctctgc tcagcggcct cctggactcc 3780
   ccggcactca aggcctgcct ggacactgcc gtggagaaca tgcccagcct gaagatgaag 3840
   gtggtggagg tgctggctgg ccacggtcac ctgtattccc gcatcccagg cctgctcagc 3900
   ccccatcccc tgctgcagct gagctacacg gccaccgacc gccaccccca ggccctggag 3960
   gctgcccagg ccgagctgca gcagcacgac gttgcccagg gccagtggga tcccgcagac 4020
   cctgccccca gcgccctggg cagcgccgac ctcctggtgt gcaactgtgc tgtggctgcc 4080
   ctcggggacc cggcctcagc tctcagcaac atggtggctg ccctgagaga agggggcttt 4140
   ctgctcctgc acacactgct ccgggggcac cccctcgggg acatcgtggc cttcctcacc 4200
   tccactgagc cgcagtatgg ccagggcatc ctgagccagg acgcgtggga gagcctcttc 4260
   tccagggtgt cgctgcgcct ggtgggcctg aagaagtcct tctacggctc cacgctcttc 4320
   ctgtgccgcc ggcccacccc gcaggacagc cccatcttcc tgccggtgga cgataccagc 4380
   ttccgctggg tggagtctct gaagggcatc ctggctgacg aagactcttc ccggcctgtg 4440
   tggctgaagg ccatcaactg tgccacctcg ggcgtggtgg gcttggtgaa ctgtctccgc 4500
   cgagagcccg gcgggaaccg cctccggtgt gtgctgctct ccaacctcag cagcacctcc 4560
   cacgtcccgg aggtggaccc gggctccgca gaactgcaga aggtgttgca gggagacctg 4620
   gtgatgaacg tctaccgcga cggggcctgg ggggctttcc gccacttcct gctggaggag 4680
   gacaagcctg aggagccgac ggcacatgcc tttgtgagca ccctcacccg gggggacctg 4740
   tcctccatcc gctgggtctg ctcctcgctg cgccatgccc agcccacctg ccctggcgcc 4800
   cagctctgca cggtctacta cgcctccctc aacttccgcg acatcatgct ggccactggc 4860
   aagctgtccc ctgatgccat cccagggaag tggacctccc aggacagcct gctaggtatg 4920
   gagttctcgg gccgagacgc cagcggcaag cgtgtgatgg gactggtgcc tgccaagggc 4980
   ctggccacct ctgtcctgct gtcaccggac ttcctctggg atgtgccttc caactggacg 5040
   ctggaggagg cggcctcggt gcctgtcgtc tacagcacgg cctactacgc gctggtggtg 5100
   cgtgggcggg tgcgccccgg ggagacgctg ctcatccact cgggctcggg cggcgtgggc 5160
   caggccgcca tcgccatcgc cctcagtctg ggctgccgcg tcttcaccac cgtggggtcg 5220
   gctgagaagc gggcgtacct ccaggccagg ttcccccagc tcgacagcac cagcttcgcc 5280
   aactcccggg acacatcctt cgagcagcat gtgctgtggc acacgggcgg gaagggcgtt 5340
   gacctggtct tgaactcctt ggcggaagag aagctgcagg ccagcgtgag gtgcttggct 5400
   acgcacggtc gcttcctgga aattggcaaa ttcgaccttt ctcagaacca cccgctcggc 5460
   atggctatct tcctgaagaa cgtgacattc cacggggtcc tactggatgc gttcttcaac 5520
   gagagcagtg ctgactggcg ggaggtgtgg gcgcttgtgc aggccggcat ccgggatggg 5580
   gtggtacggc ccctcaagtg cacggtgttc catggggccc aggtggagga cgccttccgc 5640
   tacatggccc aagggaagca cattggcaaa gtcgtcgtgc aggtgcttgc ggaggagccg 5700
   gaggcagtgc tgaagggggc caaacccaag ctgatgtcgg ccatctccaa gaccttctgc 5760
   ccggcccaca agagctacat catcgctggt ggtctgggtg gcttcggcct ggagttggcg 5820
   cagtggctga tacagcgtgg ggtgcagaag ctcgtgttga cttctcgctc cgggatccgg 5880
   acaggctacc aggccaagca ggtccgccgg tggaggcgcc agggcgtaca ggtgcaggtg 5940
   tccaccagca acatcagctc actggagggg gcccggggcc tcattgccga ggcggcgcag 6000
   cttgggcccg tgggcggcgt cttcaacctg gccgtggtct tgagagatgg cttgctggag 6060
   aaccagaccc cagagttctt ccaggacgtc tgcaagccca agtacagcgg caccctgaac 6120
   ctggacaggg tgacccgaga ggcgtgccct gagctggact actttgtggt cttctcctct 6180
   gtgagctgcg ggcgtggcaa tgcgggacag agcaactacg gctttgccaa ttccgccatg 6240
   gagcgtatct gtgagaaacg ccggcacgaa ggcctcccag gcctggccgt gcagtggggc 6300
   gccatcggcg acgtgggcat tttggtggag acgatgagca ccaacgacac gatcgtcagt 6360
   ggcacgctgc cccagcgcat ggcgtcctgc ctggaggtgc tggacctctt cctgaaccag 6420
   ccccacatgg tcctgagcag ctttgtgctg gctgagaagg ctgcggccta tagggacagg 6480
   gacagccagc gggacctggt ggaggccgtg gcacacatcc tgggcatccg cgacttggct 6540
   gctgtcaacc tggacagctc actggcggac ctgggcctgg actcgctcat gagcgtggag 6600
   gtgcgccaga cgctggagcg tgagctcaac ctggtgctgt ccgtgcgcga ggtgcggcaa 6660
   ctcacgctcc ggaaactgca ggagctgtcc tcaaaggcgg atgaggccag cgagctggca 6720
   tgccccacgc ccaaggagga tggtctggcc cagcagcaga ctcagctgaa cctgcgctcc 6780
   ctgctggtga acccggaggg ccccaccctg atgcggctca actccgtgca gagctcggag 6840
   cggcccctgt tcctggtgca cccaatcgag ggctccacca ccgtgttcca cagcctggcc 6900
   tcccggctca gcatccccac ctatggcctg cagtgcaccc gagctgcgcc ccttgacagc 6960
   atccacagcc tggctgccta ctacatcgac tgcatcaggc aggtgcagcc cgagggcccc 7020
   taccgcgtgg ccggctactc ctacggggcc tgcgtggcct ttgaaatgtg ctcccagctg 7080
   caggcccagc agagcccagc ccccacccac aacagcctct tcctgttcga cggctcgccc 7140
   acctacgtac tggcctacac ccagagctac cgggcaaagc tgaccccagg ctgtgaggct 7200
   gaggctgaga cggaggccat atgcttcttc gtgcagcagt tcacggacat ggagcacaac 7260
   agggtgctgg aggcgctgct gccgctgaag ggcctagagg agcgtgtggc agccgccgtg 7320
   gacctgatca tcaagagcca ccagggcctg gaccgccagg agctgagctt tgcggcccgg 7380
   tccttctact acaagctgcg tgccgctgag cagtacacac ccaaggccaa gtaccatggc 7440
   aacgtgatgc tactgcgcgc caagacgggt ggcgcctacg gcgaggacct gggcgcggac 7500
   tacaacctct cccaggtatg cgacgggaaa gtatccgtcc acgtcatcga gggtgaccac 7560
   cgcacgctgc tggagggcag cggcctggag tccatcatca gcatcatcca cagctccctg 7620
   gctgagccac gcgtgagcgt gcgggagggc taggcccgtg cccccgcctg ccaccggagg 7680
   tcactccacc atccccaccc caccccaccc cacccccgcc atgcaacggg attgaagggt 7740
   cctgccggtg ggaccctgtc cggcccagtg ccactgcccc ccgaggctgc tagatgtagg 7800
   tgttaggcat gtcccaccca cccgccgcct cccacggcac ctcggggaca ccagagctgc 7860
   cgacttggag actcctggtc tgtgaagagc cggtggtgcc cgtgcccgca ggaactgggc 7920
   tgggcctcgt gcgcccgtgg ggtctgcgct tggtctttct gtgcttggat ttgcatattt 7980
   attgcattgc tggtagagac ccccaggcct gtccaccctg ccaagactcc tcaggcagcg 8040
   tgtgggtccc gcactctgcc cccatttccc cgatgtcccc tgcgggcgcg ggcagccacc 8100
   caagcctgct ggctgcggcc ccctctcggc caggcattgg ctcagcccgc tgagtggggg 8160
   gtcgtgggcc agtccccgag gagctgggcc cctgcacagg cacacagggc ccggccacac 8220
   ccagcggccc cccgcacagc cacccgtggg gtgctgccct tatgcccggc gccgggcacc 8280
   aactccatgt ttggtgtttg tctgtgtttg tttttcaaga aatgattcaa attgctgctt 8340
   ggattttgaa atttactgta actgtcagtg tacacgtctg gaccccgttt catttttaca 8400
   ccaatttggt aaaaatgctg ctctcagcct cccacaatta aaccgcatgt gatctccaaa 8460
   aaaaaaaaaa aaaaaaaaaa a                                           8481
<210> 79
   <211> 985
   <212> DNA
   <213> Homo sapiens
<220>
   <223> 60S ribosomal protein L17 transcript variant 1, L17, ribosomal protein L23, RPL23, gene encoding putative NFkB activating protein, PD-1
<220>
   <221> CDS
   <222> (371)...(925)
   <223> RPL17
<400> 79
   cctgcctcct cagatctcgt ttcttcggct acgaatctcg cgagaagtca agttctcatg 60
   agttctccca aaatccaccg ctcttcctct ttccctaagc agcctgaggg ttgactggat 120
   tggtgaggcc cgtgtggcta cttctgtgga agcagtgctg tagttactgg aagataaaag 180
   ggaaagcaag cccttggtgg gggaaagtat ggctgcgatg atggcatttc ttaggacacc 240
   tttggattaa taatgaaaac aactactctc tgagcagctg ttcgaatcat ctgatattta 300
   tactgaatga gttactgtaa gtacgtattg acagaattac actgtacttt cctctaggtg 360
   atctgtgaaa atggttcgct attcacttga cccggagaac cccacgaaat catgcaaatc 420
   aagaggttcc aatcttcgtg ttcactttaa gaacactcgt gaaactgctc aggccatcaa 480
   gggtatgcat atacgaaaag ccacgaagta tctgaaagat gtcactttac agaaacagtg 540
   tgtaccattc cgacgttaca atggtggagt tggcaggtgt gcgcaggcca agcaatgggg 600
   ctggacacaa ggtcggtggc ccaaaaagag tgctgaattt ttgctgcaca tgcttaaaaa 660
   cgcagagagt aatgctgaac ttaagggttt agatgtagat tctctggtca ttgagcatat 720
   ccaagtgaac aaagcaccta agatgcgccg ccggacctac agagctcatg gtcggattaa 780
   cccatacatg agctctccct gccacattga gatgatcctt acggaaaagg aacagattgt 840
   tcctaaacca gaagaggagg ttgcccagaa gaaaaagata tcccagaaga aactgaagaa 900
   acaaaaactt atggcacggg agtaaattca gcattaaaat aaatgtaatt aaaaggaaaa 960
   gaaaaaaaaa aaaaaaaaaa aaaaa                                       985
<210> 80
   <211> 3486
   <212> DNA
   <213> Homo sapiens
<220>
   <223> eukaryotic translation initiation factor 2, subunit 3 gamma, 52kDa (EIF2S3, eIF-2gA, EIF2gamma), eukaryotic translation initiation factor 2G (EIF2G), eukaryotic translation initiation factor 2 subunit gamma X (eIF-2gX, eIF-2-gamma X)
<220>
   <221> CDS
   <222> (22)...(1440)
   <223> EIF2S3
<400> 80
   tttccttcct cttttggcaa catggcgggc ggagaagctg gagtgactct agggcagccg 60
   catctttcgc gtcaggatct caccaccttg gatgttacca agttgacgcc actttcacac 120
   gaagttatca gcagacaagc cacaattaac ataggtacaa ttggtcatgt agctcatggg 180
   aaatccacag tcgtcaaagc tatttctgga gttcatactg tcaggttcaa aaatgaacta 240
   gaaagaaata ttacaatcaa gcttggatat gctaatgcta agatttataa gcttgatgac 300
   ccaagttgcc ctcggccaga atgttataga tcttgtggga gcagtacacc tgacgagttt 360
   cctacggaca ttccagggac caaagggaac ttcaaattag tcagacatgt ttcctttgtt 420
   gactgtcctg gccacgatat tttgatggct actatgctga acggtgcagc agtgatggat 480
   gcagctcttc tgttgatagc tggtaatgaa tcttgccctc agcctcagac atcggaacac 540
   ctggctgcta tagagatcat gaaactgaag catattttga ttctacaaaa taaaattgat 600
   ttggtaaaag aaagtcaggc taaagaacaa tacgagcaga tccttgcatt tgtccaaggt 660
   acagtagcag agggagctcc cattattcca atttcagctc agctgaaata caatattgaa 720
   gttgtttgtg agtacatagt aaagaaaatt ccagtacccc caagagactt tacttcagag 780
   ccccggctta ttgttattag atcttttgat gtcaacaaac ctggctgtga agttgatgac 840
   cttaagggag gtgtagctgg tggtagtatc ctaaaaggag tattaaaggt gggccaggag 900
   atagaagtaa gacctggtat tgtttccaaa gatagtgaag gaaaactcat gtgtaaacca 960
   atcttttcca aaattgtatc actttttgcg gagcataatg atctgcaata tgctgctcca 1020
   ggcggtctta ttggagttgg aacaaaaatt gaccccactt tgtgccgggc tgacagaatg 1080
   gtggggcaag tacttggtgc agtcggagct ttacctgaga tattcacaga attggaaatt 1140
   tcctatttcc tgcttagacg gcttctaggt gtacgcactg aaggagacaa gaaagcagca 1200
   aaggttcaaa agctgtctaa gaatgaagtg ctcatggtga acataggatc cctgtcaaca 1260
   ggagggagag ttagtgctgt caaggccgat ttgggtaaaa ttgttttgac caatccagtg 1320
   tgcacagagg taggagaaaa aattgccctt agccgaagag ttgaaaaaca ctggcgttta 1380
   attggttggg gtcagataag aagaggagtg acaatcaagc caacagtaga tgatgactga 1440
   agaataccag ttaaataata cattcggatg gatttggaag ttggaattcc tcttaacaac 1500
   caaggggttt attttcaaag caatattggg gaattgattt cacagttcgt taccttagta 1560
   ggtaacggta aggttattct cttttttttt tttttttttt ttggttatga aaacttaggg 1620
   actaaaatta atataaaaat tggcataatg ttggattgaa tctacatttt ggcagaagtt 1680
   aaacattccc acataatgtc aaaattatac atcatgcagt tctgtttttt tgtttgtttt 1740
   attttgtttt gtttttgagt ctggctctgt cacccaggct ggagtgcagt ggcgtgatct 1800
   gcaacctctg ccccccgggt tcaagcgatt ctcctgcctc agcctcccga gtagctgaga 1860
   ttacaggtgc gcgccaccac acttggctaa tttttgtatt attagtagag acggggtttc 1920
   agcatgttgg ctaggccggt ctctcctgac ctcagggtga tcagcccacc tcggcctcac 1980
   aaagtgctgg gattacaggc gtgagccacc ttgcccagcc cacatcatac agtttgaaat 2040
   gaaactttgc cacaaccagc ctttgctgta gcacacacat atatcactga acctgtttga 2100
   aataaagttt tttttctttt tcatgattcg tctttgagta cctccaggct gaaagactgt 2160
   tgtaccagta aaaacttaaa ggcacaaatt ctccttgaag accttctccc ttttatgtgg 2220
   ccccatattt tatgttgctt tatctttgaa attttgcatg aaaaggaaat gaatggattc 2280
   gaatgaaatt gtcctttaga gcatgattac ttgttcccat ggacaaatat ttttctcccc 2340
   ttgctcttcc tggcctgaaa cacgggaaac cagagtcaaa agttatctcc ctctccctgt 2400
   gatgccttga gatttttttc tgcgttgttt aatgcctgaa atccaagtct tcctccatgg 2460
   gaaaatactg ttataccaaa taattctaga tgagtaacaa agatcttttt aggccttcat 2520
   tttatgtttt ttcttaactg ttatattatg attgtgacat agattatact actactaatt 2580
   tttggatgtt tcaaaaggtc aagaagtaaa agatgttaga aagcaatgag tgagtccttt 2640
   tgatttttaa cttattcccc atgtccctat acttcgtgtg cttttccttt ttttttttga 2700
   gacggaggct cactccgtca cctaggctag agtacagtgg cacgatcttg gctcgctgca 2760
   acctctgcct ccctgattca agtgattctc gtgcttcagc ctcccatgta gctgatatta 2820
   caggcacttg ccaccatacc cggctaattt ttgtattttt agtagagatg gggtttcacc 2880
   atgttgccca ggctggtctt gaactcctaa cctcaggtga tccgcccgcc tctgccttcc 2940
   aaagtgctgg gattacaggc gtgagccact ctgcccggct tatttttctt tatgtttttg 3000
   cttcgtaaga ggttctgttg agcagtgatt tgcaactctt gctgacgttg ctggggaagc 3060
   tttaaaaaaa aaaaagatgc cccacagaga ttctgatttt aattgttctg atttaattgg 3120
   cttggagtag aattcaggca ttgatatctt taaaaactcc ccagtgttga gaaacaaatt 3180
   tagagagttg agaagtaggt atattaaatt acagaatctt actgagtttt ggtagactga 3240
   taatacaatt tgctttgctt ttcttaaatt tgcattgaga tgggatttga agcatattgt 3300
   gctcttgtga atgttgaagt tgcattgtag aagtttagaa gctctggcta tgggttgcct 3360
   aaattgatgt tttgaggaag catattaatg ttataaactt cgctgacttt gaaggttgtg 3420
   ttgtagcatg aggaacacaa ataaaacaat tctaaatcaa actaaaaaaa aaaaaaaaaa 3480
   aaaaaa                                                            3486
<210> 81
   <211> 571
   <212> DNA
   <213> Homo sapiens
<220>
   <223> 60S ribosomal protein L30, L30
<220>
   <221> CDS
   <222> (116)...(463)
   <223> RPL30
<400> 81
   agttccggct ctgccgtgaa gagctttgca ttgtgggaag tctttccttt ctcgttcccc 60
   ggccatctta gcggctgctg ttggttgggg gccgtcccgc tcctaaggca ggaagatggt 120
   ggccgcaaag aagacgaaaa agtcgctgga gtcgatcaac tctaggctcc aactcgttat 180
   gaaaagtggg aagtacgtcc tggggtacaa gcagactctg aagatgatca gacaaggcaa 240
   agcgaaattg gtcattctcg ctaacaactg cccagctttg aggaaatctg aaatagagta 300
   ctatgctatg ttggctaaaa ctggtgtcca tcactacagt ggcaataata ttgaactggg 360
   cacagcatgc ggaaaatact acagagtgtg cacactggct atcattgatc caggtgactc 420
   tgacatcatt agaagcatgc cagaacagac tggtgaaaag taaacctttt cacctacaaa 480
   atttcacctg caaaccttaa acctgcaaaa ttttccttta ataaaatttg cttgttttaa 540
   aaacattgta tctaaaaaaa aaaaaaaaaa a                                571
<210> 82
   <211> 2004
   <212> DNA
   <213> Homo sapiens
<220>
   <223> actin, gamma 1, transcript variant 2 (ACTG1, ACT, ACTG), cytoskeletal gamma-actin, deafness, autosomal dominant 20 (DFNA20), deafness, autosomal dominant 26 (DFNA26), BRWS2
<220>
   <221> CDS
   <222> (140)...(1267)
   <223> ACTG1
<400> 82
   gcgcgccgcc gggccgcgcg ggcgcgccgc ttccgcttaa ataacggcgg gggaggccgc 60
   ggtcggtctc agtcgccgct gccagctctc gcactctgtt cttccgccgc tccgccgtcg 120
   cgtttctctg ccggtcgcaa tggaagaaga gatcgccgcg ctggtcattg acaatggctc 180
   cggcatgtgc aaagctggtt ttgctgggga cgacgctccc cgagccgtgt ttccttccat 240
   cgtcgggcgc cccagacacc agggcgtcat ggtgggcatg ggccagaagg actcctacgt 300
   gggcgacgag gcccagagca agcgtggcat cctgaccctg aagtacccca ttgagcatgg 360
   catcgtcacc aactgggacg acatggagaa gatctggcac cacaccttct acaacgagct 420
   gcgcgtggcc ccggaggagc acccagtgct gctgaccgag gcccccctga accccaaggc 480
   caacagagag aagatgactc agattatgtt tgagaccttc aacaccccgg ccatgtacgt 540
   ggccatccag gccgtgctgt ccctctacgc ctctgggcgc accactggca ttgtcatgga 600
   ctctggagac ggggtcaccc acacggtgcc catctacgag ggctacgccc tcccccacgc 660
   catcctgcgt ctggacctgg ctggccggga cctgaccgac tacctcatga agatcctcac 720
   tgagcgaggc tacagcttca ccaccacggc cgagcgggaa atcgtgcgcg acatcaagga 780
   gaagctgtgc tacgtcgccc tggacttcga gcaggagatg gccaccgccg catcctcctc 840
   ttctctggag aagagctacg agctgcccga tggccaggtc atcaccattg gcaatgagcg 900
   gttccggtgt ccggaggcgc tgttccagcc ttccttcctg ggtatggaat cttgcggcat 960
   ccacgagacc accttcaact ccatcatgaa gtgtgacgtg gacatccgca aagacctgta 1020
   cgccaacacg gtgctgtcgg gcggcaccac catgtacccg ggcattgccg acaggatgca 1080
   gaaggagatc accgccctgg cgcccagcac catgaagatc aagatcatcg cacccccaga 1140
   gcgcaagtac tcggtgtgga tcggtggctc catcctggcc tcactgtcca ccttccagca 1200
   gatgtggatt agcaagcagg agtacgacga gtcgggcccc tccatcgtcc accgcaaatg 1260
   cttctaaacg gactcagcag atgcgtagca tttgctgcat gggttaattg agaatagaaa 1320
   tttgcccctg gcaaatgcac acacctcatg ctagcctcac gaaactggaa taagccttcg 1380
   aaaagaaatt gtccttgaag cttgtatctg atatcagcac tggattgtag aacttgttgc 1440
   tgattttgac cttgtattga agttaactgt tccccttggt atttgtttaa taccctgtac 1500
   atatctttga gttcaacctt tagtacgtgt ggcttggtca cttcgtggct aaggtaagaa 1560
   cgtgcttgtg gaagacaagt ctgtggcttg gtgagtctgt gtggccagca gcctctgatc 1620
   tgtgcagggt attaacgtgt cagggctgag tgttctggga tttctctaga ggctggcaag 1680
   aaccagttgt tttgtcttgc gggtctgtca gggttggaaa gtccaagccg taggacccag 1740
   tttcctttct tagctgatgt ctttggccag aacaccgtgg gctgttactt gctttgagtt 1800
   ggaagcggtt tgcatttacg cctgtaaatg tattcattct taatttatgt aaggtttttt 1860
   ttgtacgcaa ttctcgattc tttgaagaga tgacaacaaa ttttggtttt ctactgttat 1920
   gtgagaacat taggccccag caacacgtca ttgtgtaagg aaaaataaaa gtgctgccgt 1980
   aaccaaaaaa aaaaaaaaaa aaaa                                        2004
<210> 83
   <211> 3455
   <212> DNA
   <213> Homo sapiens
<220>
   <223> collagen, type VI, alpha 2 transcript variant 2C2 (COL6A2), collagen VI, alpha-2 polypeptide, collagen alpha-2(VI) chain, collagen VI alpha-2 C-terminal globular domain, PP3610
<220>
   <221> CDS
   <222> (83)...(3142)
   <223> COL6A2
<400> 83
   gcttactcgg cgcccgcgcc tcgggccgtc gggagcggag cctcctcggg accaggactt 60
   cagggccaca ggtgctgcca agatgctcca gggcacctgc tccgtgctcc tgctctgggg 120
   aatcctgggg gccatccagg cccagcagca ggaggtcatc tcgccggaca ctaccgagag 180
   aaacaacaac tgcccagaga agaccgactg ccccatccac gtgtacttcg tgctggacac 240
   ctcggagagc gtcaccatgc agtcccccac ggacatcctg ctcttccaca tgaagcagtt 300
   cgtgccgcag ttcatcagcc agctgcagaa cgagttctac ctggaccagg tggcgctgag 360
   ctggcgctac ggcggcctgc acttctctga ccaggtggag gtgttcagcc caccgggcag 420
   cgaccgggcc tccttcatca agaacctgca gggcatcagc tccttccgcc gcggcacctt 480
   caccgactgc gcgctggcca acatgacgga gcagatccgg caggaccgca gcaagggcac 540
   cgtccacttc gccgtggtca tcaccgacgg ccacgtcacc ggcagcccct gcgggggcat 600
   caagctgcag gccgagcggg cccgcgagga gggcatccgg ctcttcgccg tggcccccaa 660
   ccagaacctg aaggagcagg gcctgcggga catcgccagc acgccgcacg agctctaccg 720
   caacgactac gccaccatgc tgcccgactc caccgagatc gaccaggaca ccatcaaccg 780
   catcatcaag gtcatgaaac acgaagccta cggagagtgc tacaaggtga gctgcctgga 840
   aatccctggg ccctctggcc ccaagggcta ccgtggacag aagggtgcca agggcaacat 900
   gggtgagccg ggagagcctg gccagaaggg aagacaggga gacccgggca tcgaaggccc 960
   cattggattc ccaggaccca agggcgttcc tggcttcaaa ggagagaagg gtgaatttgg 1020
   agccgacggt cgcaaggggg cccctggcct ggctggcaag aacgggaccg atggacagaa 1080
   gggcaagctg gggcgcatcg gacctcctgg ctgcaaggga gaccctggaa accggggccc 1140
   cgacggttac ccgggggaag cagggagtcc aggggagcga ggagaccaag gcggcaaggg 1200
   ggaccctggc cgcccaggac gcagagggcc cccgggagaa atcggggcca agggaagcaa 1260
   ggggtatcaa ggcaacagtg gagccccagg aagtcctggt gtgaaaggag ccaagggcgg 1320
   gcctgggccc cgcggaccca aaggcgagcc ggggcgcagg ggagaccccg gcaccaaggg 1380
   cagcccaggc agcgatggcc ccaaggggga gaagggggac cctggccctg aggggccccg 1440
   cggcctggct ggagaggttg gcaacaaagg agccaaggga gaccgaggct tgcctggacc 1500
   cagaggcccc cagggagctc ttggggagcc cggaaagcag ggatctcggg gagaccccgg 1560
   tgatgcagga ccccgtggag actcaggaca gccaggcccc aagggagacc ccggcaggcc 1620
   tggattcagc tacccaggac cccgaggagc acccggagaa aaaggcgagc ccggcccacg 1680
   cggccccgag ggaggccgag gcgactttgg cttgaaagga gaacctggga ggaaaggaga 1740
   gaaaggagag cctgcggatc ctggtccccc tggtgagcca ggccctcggg ggccaagagg 1800
   agtcccagga cccgagggtg agcccggccc ccctggagac cccggtctca cggagtgtga 1860
   cgtcatgacc tacgtgaggg agacctgcgg gtgctgcgac tgtgagaagc gctgtggcgc 1920
   cctggacgtg gtcttcgtca tcgacagctc cgagagcatt gggtacacca acttcacact 1980
   ggagaagaac ttcgtcatca acgtggtcaa caggctgggt gccatcgcta aggaccccaa 2040
   gtccgagaca gggacgcgtg tgggcgtggt gcagtacagc cacgagggca cctttgaggc 2100
   catccagctg gacgacgaac gtatcgactc cctgtcgagc ttcaaggagg ctgtcaagaa 2160
   cctcgagtgg attgcgggcg gcacctggac accctcagcc ctcaagtttg cctacgaccg 2220
   cctcatcaag gagagccggc gccagaagac acgtgtgttt gcggtggtca tcacggacgg 2280
   gcgccacgac cctcgggacg atgacctcaa cttgcgggcg ctgtgcgacc gcgacgtcac 2340
   agtgacggcc atcggcatcg gggacatgtt ccacgagaag cacgagagtg aaaacctcta 2400
   ctccatcgcc tgcgacaagc cacagcaggt gcgcaacatg acgctgttct ccgacctggt 2460
   cgctgagaag ttcatcgatg acatggagga cgtcctctgc ccggaccctc agatcgtgtg 2520
   cccagacctt ccctgccaaa cagagctgtc cgtggcacag tgcacgcagc ggcccgtgga 2580
   catcgtcttc ctgctggacg gctccgagcg gctgggtgag cagaacttcc acaaggcccg 2640
   gcgcttcgtg gagcaggtgg cgcggcggct gacgctggcc cggagggacg acgaccctct 2700
   caacgcacgc gtggcgctgc tgcagtttgg tggccccggc gagcagcagg tggccttccc 2760
   gctgagccac aacctcacgg ccatccacga ggcgctggag accacacaat acctgaactc 2820
   cttctcgcac gtgggcgcag gcgtggtgca cgccatcaat gccatcgtgc gcagcccgcg 2880
   tggcggggcc cggaggcacg cagagctgtc cttcgtgttc ctcacggacg gcgtcacggg 2940
   caacgacagt ctgcacgagt cggcgcactc catgcgcaag cagaacgtgg tacccaccgt 3000
   gctggccttg ggcagcgacg tggacatgga cgtgctcacc acgctcagcc tgggtgaccg 3060
   cgccgccgtg ttccacgaga aggactatga cagcctggcg caacccggct tcttcgaccg 3120
   cttcatccgc tggatctgct agcgccgccg cccgggcccc gcagtcgagg gtcgtgagcc 3180
   caccccgtcc atggtgctaa gcgggcccgg gtcccacacg gccagcaccg ctgctcactc 3240
   ggacgacgcc ctgggcctgc acctctccag ctcctcccac ggggtccccg tagccccggc 3300
   ccccgcccag ccccaggtct ccccaggccc tccgcaggct gcccggcctc cctccccctg 3360
   cagccatccc aaggctcctg acctacctgg cccctgagct ctggagcaag ccctgaccca 3420
   ataaaggctt tgaacccata aaaaaaaaaa aaaaa 3455
<210> 84
   <211> 2801
   <212> DNA
   <213> Homo sapiens
<220>
   <223> ubiquitin-60S ribosomal protein L40 precursor transcript variant 2 (UBA52), RPL40, ubiquitin-52 amino acid fusion protein, ubiquitin carboxyl extension protein 52 (CEP52, HUBCEP52)
<220>
   <221> CDS
   <222> (93)...(479)
   <223> UBA52
<400> 84
   gctccgtgcg caagcgcttt cggcggcgat taggtggttt ccggttccgc tatcttcttt 60
   ttcttcagcg aggcggccga gctgacgcaa acatgcagat ctttgtgaag accctcactg 120
   gcaaaaccat cacccttgag gtcgagccca gtgacaccat tgagaatgtc aaagccaaaa 180
   ttcaagacaa ggagggtatc ccacctgacc agcagcgtct gatatttgcc ggcaaacagc 240
   tggaggatgg ccgcactctc tcagactaca acatccagaa agagtccacc ctgcacctgg 300
   tgttgcgcct gcgaggtggc attattgagc cttctctccg ccagcttgcc cagaaataca 360
   actgcgacaa gatgatctgc cgcaagtgct atgctcgcct tcaccctcgt gctgtcaact 420
   gccgcaagaa gaagtgtggt cacaccaaca acctgcgtcc caagaagaag gtcaaataag 480
   gtggttcttt ccttgaaggg cagcctcctg cccaggcccc gtggccctgg agcctcaata 540
   aagtgtccct ttcattgact ggagcagcaa ttggtgtcct catggctgat ctgtccaggg 600
   aggtggctga agagtgggca tctcccttag ggactctact cagcactcca ttctgtgcca 660
   cctgtggggt cttctgtcct agattctgtc acatcggcat tggtccctgc cctatgcccc 720
   tgactctgga tttgtcatct gtaaaactgg agtaaaaacc tcagtcgtgt aattggtggg 780
   actgaggatc agttttgtca ttgctgggat cctgtcaggc actttgaggt gtccctcagg 840
   ccttggccct gaagtgtcta ggtgtgtgga gatgggtaga aaattaggta cacccaatgg 900
   tgtagaacgt tgattctcaa atttttttat tttatacaaa tggggtctca ctatgttgtc 960
   caggctggtc ttgaactcct gggctcaagc catccgccca tctcagcccc tcaaagtgtt 1020
   gggattacaa gcaagaactg ccatgcctga cccagttctc agttttttgt ttgtttgttt 1080
   gtttgtttgt tttgagacgg agtcttgctc tgtcgcccag gctggagtgc agtggcgcag 1140
   tctcggctta ctacaacctc tgcctccggg gttcacatcc ttctcctgcc tcagcctccc 1200
   gagtagctgg gactacaggt gcccgccaca actcctggct aattttttgt atttttagta 1260
   gagacggggt ttcactgggt tagccaggtt ggtctcgatc tcctgacctt gtgatccatt 1320
   cgccttggcc tcccagaatg ctggtattac aggcgtgagc cagcacgcct ggcccagtta 1380
   ctcagttttg aatctgaggc cgtgacatca ctcatggtct gcagtcagtg ctctgcccct 1440
   gagctgtacc ctctcctatg ataatcactc ttaagaaggg caacccttgg tgttttcccc 1500
   ttaaggtcac ccaggctgga atgcagtggt gtggtcatgg ctccctgtac cctggaactc 1560
   aggcttgggt gatcctctct cctttgcctc cgaagtagcc aggactacag gtgtgcaccc 1620
   accaccacac tcagataatt gctttggtgt ttttaaagct tgtaatgatc agtaggctga 1680
   ggtgggcaaa tcataaggtc aagagttttt tagatggggt gagcacagac caattcctgt 1740
   tttatttact gatttaaaat tttgagacag tctcactgtc acccaggttg gggtgcagtg 1800
   gtaggatcat agcttgctgc agccttgatc tcccaggatc ttgcctcagc ctcccgagta 1860
   gctgggactg catgcttgtg ccaccacact cggttaatat tttgtagaga tggggtcttg 1920
   ctatgttgcc caggctggct tcaaactcct gaacttaaaa gcctcctgtt tagttttggt 1980
   tttttatcac tttttttttt tttttttgag atggagcctt gctcccatcg tgcaggctgg 2040
   agtgcggtgg cgcagtctcg gctcactgca gcttctgcct ctcgggttca agcgattctc 2100
   ctttctcagc ctcttgagta gctggaatta ccagtgtgcg ccaccaccac cacgcctggc 2160
   tagtttttct gtttttagta gagacagggt tttgctatgt tggccaggct ggtcttgaac 2220
   tactgacctc ttgtgatcta cctgtcttgg ccttccaaag tgctaggatt acaagcgtaa 2280
   gccacagcgc ctggccttgc tacatttttt tttttttttt tttttttaca gacatggtct 2340
   cgctatgttg cccagaatgg ttttgcactg ggtccaagca gttctgccgc agcctcccaa 2400
   agtgctggga ttacaggggt gaggcacctt gctggcccct gttttgatta gggtgcagtg 2460
   ctggtgaagc cggtgcacga ggccagtgat gcatcctaat gaggggtgga gttggcggga 2520
   cttcctgggc cagtttgggg actttcacaa aagaccccca tgactcaggg ttttgagttc 2580
   ttaactgatc gaatgaagga ttcaaaatta accactccaa ggggggattg aaggaagaac 2640
   cactcttaat ggacaaaaag aaagaaaggg gagggagtaa cagggatatg agctctagcc 2700
   gcccaagcta gcaatggcaa cccttctggg tccccttcca gcatgtggaa gctttccttt 2760
   cgcttcattc aataaacagc tgctgctcaa aaaaaaaaaa a                    2801
<210> 85
   <211> 1331
   <212> DNA
   <213> Homo sapiens
<220>
   <223> alkB, alkylation repair homolog 7 (E. coli), alkylated DNA repair protein alkB homolog 7, alpha-ketoglutarate-dependent dioxygenase alkB homolog 7, mitochondrial (ALKBH7), spermatogenesis associated 11 (SPATA11), spermatogenesis cell proliferation-related protein
<220>
   <221> CDS
   <222> (389) ... (1054)
   <223> ALKBH7
<400> 85
<210> 86
   <211> 1035
   <212> DNA
   <213> Homo sapiens
<220>
   <223> 60S ribosomal protein L5, L5, DBA6, MSTP030
<220>
   <221> CDS
   <222> (79)...(972)
   <223> RPL5
<400> 86
   ggcccttttc ccacccccta gcgccgctgg gcctgcaggt ctctgtcgag cagcggacgc 60
   cggtctctgt tccgcaggat ggggtttgtt aaagttgtta agaataaggc ctactttaag 120
   agataccaag tgaaatttag aagacgacga gagggtaaaa ctgattatta tgctcggaaa 180
   cgcttggtga tacaagataa aaataaatac aacacaccca aatacaggat gatagttcgt 240
   gtgacaaaca gagatatcat ttgtcagatt gcttatgccc gtatagaggg ggatatgata 300
   gtctgcgcag cgtatgcaca cgaactgcca aaatatggtg tgaaggttgg cctgacaaat 360
   tatgctgcag catattgtac tggcctgctg ctggcccgca ggcttctcaa taggtttggc 420
   atggacaaga tctatgaagg ccaagtggag gtgactggtg atgaatacaa tgtggaaagc 480
   attgatggtc agccaggtgc cttcacctgc tatttggatg caggccttgc cagaactacc 540
   actggcaata aagtttttgg tgccctgaag ggagctgtgg atggaggctt gtctatccct 600
   cacagtacca aacgattccc tggttatgat tctgaaagca aggaatttaa tgcagaagta 660
   catcggaagc acatcatggg ccagaatgtt gcagattaca tgcgctactt aatggaagaa 720
   gatgaagatg cttacaagaa acagttctct caatacataa agaacagcgt aactccagac 780
   atgatggagg agatgtataa gaaagctcat gctgctatac gagagaatcc agtctatgaa 840
   aagaagccca agaaagaagt taaaaagaag aggtggaacc gtcccaaaat gtcccttgct 900
   cagaagaagg atcgggtagc tcaaaagaag gcaagcttcc tcagagctca ggagcgggct 960
   gctgagagct aaacccagca attttctatg attttttcag atatagataa taaacttatg 1020
   aacagcaact aaaaa                                                   1035
<210> 87
   <211> 1043
   <212> DNA
   <213> Homo sapiens
<220>
   <223> 6-phosphogluconolactonase (PGLS, 6PGL)
<220>
   <221> CDS
   <222> (51)...(827)
   <223> PGLS
<400> 87
   ctagcgacgg ccgtagggag cgcttcctcc tccccgccgc cgccctcgcc atggccgcgc 60
   cggccccggg cctcatctcg gtgttctcga gttcccagga gctgggtgcg gcgctagcgc 120
   agctggtggc ccagcgcgca gcatgctgcc tggcaggggc ccgcgcccgt ttcgcgctcg 180
   gcctgtcggg cgggagcctc gtctcgatgc tagcccgcga gctacccgcc gccgtcgccc 240
   ctgccgggcc agctagctta gcgcgctgga cgctgggctt ctgcgacgag cgcctcgtgc 300
   ccttcgatca cgccgagagc acgtacggcc tctaccggac gcatcttctc tccagactgc 360
   cgatcccaga aagccaggtg atcaccatta accccgagct gcctgtggag gaggcggctg 420
   aggactacgc caagaagctg agacaggcat tccaagggga ctccatcccg gttttcgacc 480
   tgctgatcct gggggtgggc cccgatggtc acacctgctc actcttccca gaccaccccc 540
   tcctacagga gcgggagaag attgtggctc ccatcagtga ctccccgaag ccaccgccac 600
   agcgtgtgac cctcacacta cctgtcctga atgcagcacg aactgtcatc tttgtggcaa 660
   ctggagaagg caaggcagct gttctgaagc gcattttgga ggaccaggag gaaaacccgc 720
   tgcccgccgc cctggtccag ccccacaccg ggaaactgtg ctggttcttg gacgaggcgg 780
   ccgcccgcct cctgaccgtg cccttcgaga agcattccac tttgtagctg gccagaggga 840
   cgccgcagct gggaccaggc acgcggccca tggggctggg cccctgctgg ccgccactct 900
   ccgggctctc ctttcaaaaa gccacgtcgt gctgctgctg gaagccaaca gcctccggcc 960
   agcagcccta cccggggctc aacacacagg ctgtggctct ggacatccgg atattaaaag 1020
   gagcgttgct ggaaaaaaaa aaa                                         1043
<210> 88
   <211> 1976
   <212> DNA
   <213> Homo sapiens
<220>
   <223> cold shock domain protein A transcript variant 1 (CSDA),cold-shock domain containing A1 (CSDA1), Y box binding protein 3 (YBX3), single-strand DNA-binding protein NF-GMB, DNA-binding protein A (DBPA), ZO-1-associated nucleic acid-binding protein (ZONAB)
<220>
   <221> CDS
   <222> (244)...(1362)
   <223> CSDA
<400> 88
   agaaaaactt gtgcggggcc atttttgggg cagtaagatc gagcgaggag cccaagagcg 60
   agcgcgcagc acgaagctcg agccgcctcc gccgcgcgac cccacctcgg ccgccgccgc 120
   ctgcgccgcg agatccgccc cggcctcccc gagagcgagc cccggccgcc gcgaccacca 180
   gccgcgctaa ccgccgacca accgccaccg aggcgcctga gcgagagcag aggaggagga 240
   ggcatgagtg aggcgggcga ggccaccacc accaccacca ccaccctccc gcaggctccg 300
   acggaggcgg ccgccgcggc tccccaggac cccgcgccca agagcccggt gggcagcggt 360
   gcgccccagg ccgcggcccc ggcgcccgcc gcccacgtcg caggaaaccc cggtggggac 420
   gcggcccccg cagccacggg caccgcggcc gccgcctctt tagccaccgc cgccggcagc 480
   gaagacgcgg agaaaaaagt tctcgccacc aaagtccttg gcactgtcaa atggttcaac 540
   gtcagaaatg gatatggatt tataaatcga aatgacacca aagaagatgt atttgtacat 600
   cagactgcca tcaagaagaa taacccacgg aaatatctgc gcagtgtagg agatggagaa 660
   actgtagagt ttgatgtggt tgaaggagag aagggtgcag aagctgccaa tgtgactggc 720
   ccggatggag ttcctgtgga agggagtcgt tacgctgcag atcggcgccg ttacagacgt 780
   ggctactatg gaaggcgccg tggccctccc cggaattacg ctggggagga ggaggaggaa 840
   gggagcggca gcagtgaagg atttgacccc cctgccactg ataggcagtt ctctggggcc 900
   cggaatcagc tgcgccgccc ccagtatcgc cctcagtacc ggcagcggcg gttcccgcct 960
   taccacgtgg gacagacctt tgaccgtcgc tcacgggtct taccccatcc caacagaata 1020
   caggctggtg agattggaga gatgaaggat ggagtcccag agggagcaca acttcaggga 1080
   ccggttcatc gaaatccaac ttaccgccca aggtaccgta gcaggggacc tcctcgccca 1140
   cgacctgccc cagcagttgg agaggctgaa gataaagaaa atcagcaagc caccagtggt 1200
   ccaaaccagc cgtctgttcg ccgtggatac cggcgtccct acaattaccg gcgtcgcccg 1260
   cgtcctccta acgctccttc acaagatggc aaagaggcca aggcaggtga agcaccaact 1320
   gagaaccctg ctccacccac ccagcagagc agtgctgagt aacaccaggc tcctcaggca 1380
   ccttcaccat cggcaggtga cctaaagaat taatgaccat tcagaaataa agcaaaaagc 1440
   aggccacaac cttaaccaac accaaagaaa catccaagca ataaagtgga agactaacca 1500
   agatttggac attggaatgt ttactgttat tctttaagaa acaactacaa aaagaaaatg 1560
   tcaacaaatt tttccagcaa gctgagaacc tgggaattcc tgcacggaag acaagagagt 1620
   agcctctcca gtttcagcaa ccgctaggtt tctatttttt ttcctggttt ttactgtttt 1680
   ggtaatatat atattgaaac aagaaatatt aataccacat ggggagaacc ccaaccaaag 1740
   aaatctgaaa tatatagtaa atgctttttt ttccgttttt gttcattttg gatgctggtg 1800
   ctaaacctcc aagtgtcatg atttaaaaaa aaaaaaaatt tatgtccttc ttatttattt 1860
   ctaggatgag gggaggataa catttttgct ttcttatgtg actctctttg aaaatgtgca 1920
   gtaagaaatt cctcaaaaat aaaattttta cccttcagag gacagaatgt ttaaaa     1976
<210> 89
   <211> 972
   <212> DNA
   <213> Homo sapiens
<220>
   <223> 60S ribosomal protein L6, L6, neoplasm-related protein C140, tax-responsive enhancer element-binding protein 107 (TAXREB107, TXREB1), DNA-binding protein TAXREB107, SHUJUN-2
<220>
   <221> CDS
   <222> (54)...(920)
   <223> RPL6
<400> 89
   gattgcttat agaccggaag ccgggacctt aattctcttt cccatcttgc aagatggcgg 60
   gtgaaaaagt tgagaagcca gatactaaag agaagaaacc cgaagccaag aaggttgatg 120
   ctggtggcaa ggtgaaaaag ggtaacctca aagctaaaaa gcccaagaag gggaagcccc 180
   attgcagccg caaccctgtc cttgtcagag gaattggcag gtattcccga tctgccatgt 240
   attccagaaa ggccatgtac aagaggaagt actcagccgc taaatccaag gttgaaaaga 300
   aaaagaagga gaaggttctc gcaactgtta caaaaccagt tggtggtgac aagaacggcg 360
   gtacccgggt ggttaaactt cgcaaaatgc ctagatatta tcctactgaa gatgtgcctc 420
   gaaagctgtt gagccacggc aaaaaaccct tcagtcagca cgtgagaaaa ctgcgagcca 480
   gcattacccc cgggaccatt ctgatcatcc tcactggacg ccacaggggc aagagggtgg 540
   ttttcctgaa gcagctggct agtggcttat tacttgtgac tggacctctg gtcctcaatc 600
   gagttcctct acgaagaaca caccagaaat ttgtcattgc cacttcaacc aaaatcgata 660
   tcagcaatgt aaaaatccca aaacatctta ctgatgctta cttcaagaag aagaagctgc 720
   ggaagcccag acaccaggaa ggtgagatct tcgacacaga aaaagagaaa tatgagatta 780
   cggagcagcg caagattgat cagaaagctg tggactcaca aattttacca aaaatcaaag 840
   ctattcctca gctccagggc tacctgcgat ctgtgtttgc tctgacgaat ggaatttatc 900
   ctcacaaatt ggtgttctaa atgtcttaag aacctaatta aatagctgac tacaaaaaaa 960
   aaaaaaaaaa aa                                                     972
<210> 90
   <211> 2820
   <212> DNA
   <213> Homo sapiens
<220>
   <223> 40S ribosomal protein S24 transcript variant d, S24, DBA3
<220>
   <221> CDS
   <222> (143) ... (1012)
   <223> RPS24
<400> 90
   aggcatcggc gcggtcagcc tcgtggcgcg cccacgcccc cacgccggct cttcccgggg 60
   tccttccgtg cgcgttgata tgattggccg gcgaatcgtg gttctctttt cctccttggc 120
   tgtctgaaga tagatcgcca tcatgaacga caccgtaact atccgcacta gaaagttcat 180
   gaccaaccga ctacttcaga ggaaacaaat ggtcattgat gtccttcacc ccgggaaggc 240
   gacagtgcct aagacagaaa ttcgggaaaa actagccaaa atgtacaaga ccacaccgga 300
   tgtcatcttt gtatttggat tcagaactca ttttggtggt ggcaagacaa ctggctttgg 360
   catgatttat gattccctgg attatgcaaa gaaaaatgaa cccaaacata gacttgcaag 420
   acatggcctg tatgagaaga aaaagacctc aagaaagcaa cgaaaggaac gcaagaacag 480
   aatgaagaaa gtcaggggga ctgcaaaggc caatgttggt gctggcaaaa agatgaggga 540
   attggggctt ggagtgcaag cattgggaag aatttcccag gaagagagat gcacagatgt 600
   gaagaactcg aaggcaagag aaagccgggg ggttgtgtgg caggtagaag tgccaggacc 660
   gtggagcgtg tggacatgtg gcagattgcg gaggggctgt ggcaagtatt tacaggtggc 720
   cgttacctgg aggaagactg aaaacagaga acagtgctgc caggcgtgcc ttttggagag 780
   ggcactggtc agaaacggag ccttcatgtc gcctgcctca cctgctcctg ctggttctcc 840
   ccaccctgtg gacggtgact tggtcctcca cttgccagaa gccttgtcag caaccttgac 900
   tctgtcaccc cacatccagg ccatcaacaa gtcttttggc cctttctttg aaatccacca 960
   ggaatccagc tgcttctccc caccttcctg cctctctggt ctgggccact aatgtcactg 1020
   ccatggccgc cttgctgcat ttctgaggat gcttcatctc tccaccttct tctccactca 1080
   gcagccagca gggcactgtg gaaatcggag tcacatgagc tggcacctct gttcagaacc 1140
   ctccagggct ccacatctct ctcacccaaa tgccaaagac ctccccacgc ccccacaatc 1200
   ccccacgacc tggccactgg cctcccacca ccttccagct ccagcggctc ctaccacatt 1260
   taaggctttc cttcctagtt ttaatttttc ctcgtcagca gttgatttta ttattttctt 1320
   gtttattggt attttcccac tagaaatgaa gctgcgtgaa gttagagatt tttttttttg 1380
   gtctgtgttc ctaattagct cattgctata cccctggcgc ccagaacaat gccttggaca 1440
   cagtacgcag tagactaaat aaatacttgt tgaatgactg actgacggaa tgacggctgt 1500
   gtggggagtg gattgggtcg tgaggcagag gctgcggtgg aaactcaggc aggaggtgat 1560
   ggtggttctt ggggctgcgg aatgccaagt ttagaagctc ttcctctgct gtggcacatg 1620
   aaccggtcac tcgagaaggc ttttagattt actttgccta atcccctctt agtgcatgtg 1680
   gggaaactga ggtacacaaa aggaattccc caccaagtta ggggcagaac ctagccccct 1740
   tgtctcccag atggatatct tctttttttt ttgagacgga gtcttgctct gttgcccagg 1800
   ctggagtgca gtggtaccat cttggctcac tgcaacctct gcttcccagg ttcaagcgat 1860
   tctcctgcct cagcctcctg agtgtctgcg attacaggtg cacacaacca cgcctggcta 1920
   atttttgtat ttttagtaga gacggggttt caccgtgttg gtcagggtga cctcaaactc 1980
   ctgacctcat gatccaccca gctcagcctc ccaacgtgct gggattacag gcatgagcca 2040
   ccgtgcctgg ctggacatct tgttattaaa gcttcttctc tctttgtagg ggagggggag 2100
   atgcctctgg tggagaagac cagtgtggca gtgactgtgt ctgttagtga acctggtggc 2160
   tggttgaggg tctgtcgtgg tgactgagga cacatacaaa gtgcttttct cagtggtcac 2220
   cttggtgttg gtgaataagg gtcagaagat ggctcctgtc ctagggcact gccagtcggt 2280
   ttggaagctg aaatgcctgc ttagcagttt gaggaaacac agaccttgga ggatcttctg 2340
   gttgcctctt caagaattca ttctattccc cttctgctcc ccaaatttgc ttttcttggg 2400
   gtgggtcttg gttggcctaa gccaagaaag tatggcatct actccttcca tagcaatagc 2460
   tcaggaatag gcagtgaccc agacctgaac caatcagtgc atggaattac ccctggccaa 2520
   agtggttgat tgaggctggg tgcaagcaga gttgtgagaa ggctcccatt tggtggttgg 2580
   agagatcgca cttgctccag aggtcataat gtgcagatct gaggcttgga actgctgcag 2640
   acattttgct accacaagtg aagccaccct gacgacacag ttgacaattt ggagcagggc 2700
   agagctgaga gaacagcagg gaaacagcca gagtcttgct caagcctccc tgaagtatct 2760
   atacccctgg actctagtta tgggggctaa taaatgttat atactgttta aggtaaaaaa 2820
<210> 91
   <211> 2099
   <212> DNA
   <213> Homo sapiens
<220>
   <223> 60S ribosomal protein L22 proprotein, L22, Epstein-Barr virus small RNA-associated protein, EBER-associated protein (EAP), heparin-binding protein 15 (HBP15, HBp15, HBP15/L22)
<220>
   <221> CDS
   <222> (47)...(433)
   <223> RPL22
<400> 91
   gcgtctgcgt agttcgctca cctccctttc taactccgct gccgccatgg ctcctgtgaa 60
   aaagcttgtg gtgaaggggg gcaaaaaaaa gaagcaagtt ctgaagttca ctcttgattg 120
   cacccaccct gtagaagatg gaatcatgga tgctgccaat tttgagcagt ttttgcaaga 180
   aaggatcaaa gtgaacggaa aagctgggaa ccttggtgga ggggtggtga ccatcgaaag 240
   gagcaagagc aagatcaccg tgacatccga ggtgcctttc tccaaaaggt atttgaaata 300
   tctcaccaaa aaatatttga agaagaataa tctacgtgac tggttgcgcg tagttgctaa 360
   cagcaaagag agttacgaat tacgttactt ccagattaac caggacgaag aagaggagga 420
   agacgaggat taaatttcat ttatctggaa aattttgtat gagttcttga ataaaacttg 480
   ggaaccaaaa tggtggttta tccttgtatc tctgcagtgt ggattgaaca gaaaattgga 540
   aatcatagtc aaagggcttc ccttggttcg ccactcattt atttgtaact tgacttcttt 600
   ttttttctgc ttaaaaattt caattctcgt ggtaatacca gagtagaagg agagggtgac 660
   tttaccgaac tgacagccat tggggaggca gatgcgggtg tggaggtgtg ggctgaaggt 720
   agtgactgtt tgattttaaa aagtgtgact gtcagttgta tctgttgctt ttctcaatga 780
   ttcagggata caaatgggct tctctcattc attaaaagaa aacgcgacat ctttctaaga 840
   ttctctgtgg gaaaatgact gtcaataaaa tgcgggtttc tgggccattc gtcttacttt 900
   catttttgga ttacaaattt ctcttgacgc acacaattat gtctgctaat cctcttcttc 960
   ctagagagag aaactgtgct ccttcagtgt tgctgccata aggggtttgg gggaatcgat 1020
   tgtaaaagtc ccaggttcta aattaactaa atgtgtacag aaatgaacgt gtaagtaatg 1080
   tttctacagg tctttgcaac aaactgtcac tttcgtctcc agcagaggga gctgtaggaa 1140
   tagtgcttcc agatgtggtc tcccgtgtgg ggcccagcaa tgggggcccc tgatgccaag 1200
   agctctggag gttcttgaaa gaggggacac gaaggaggag tgactgggaa gcctcccatg 1260
   ccaaggaggt gggaggtgcc ctggaaatag ctgcctcatg ccacttaggc catgactgga 1320
   tttaatgtca gtggtgtgcc acagtgcaga ggctagacaa ctgaaagggg ctaccaaggc 1380
   tgggaaaaaa atgcaattgt tgctgtgagt gactttgaaa gactctggtg ccttgtggtg 1440
   cccttctgaa attcaaacag taatgcaaaa gtgtctgcat tagaatttac ggtgtctaaa 1500
   attcatgttt ttaaaagagc ttgcctacag atggtttcca cacttgaaat tgtgccctgc 1560
   gagttgcata gctggaagtt caatgctcag tcctaccttg gctcccatta aacatttggt 1620
   gctctgtgga ttgagttgaa cgtgttgagg ctttgcaatt tcacttgtgt taaaggctct 1680
   ggcatttttc catttctatg caaatttctt tgaagcagaa ttgcttgcat atttcttctc 1740
   tgccgtcaca gaaagcagag tttctttcaa acttcactga ggcatcagtt gctctttggc 1800
   aatgtccctt aaccatgatt attaactaag tttgtggctt gagtttacaa attctacttg 1860
   ttgcattgat gttcccatgt agtaagtcat ttttagtttg gttgtgaaaa aaccctgggc 1920
   tgaagttggc atttcagtta aaagaaaaaa agaaactagt cccagatttg aaaacttgta 1980
   ataaaattga aactcactgg ttttctatgt ctttttgaac tcttgtaatc gagttttgat 2040
   catattttct attaaagtgg ctaacacctg gctactctta ctgtaaaaaa aaaaaaaaa  2099
<210> 92
   <211> 3499
   <212> DNA
   <213> Homo sapiens
<220>
   <223> adaptor-related protein complex 2, alpha 1 subunit transcript variant 1 (AP2A1), clathrin-associated/assembly/adaptor protein, large, alpha 1 (CLAPA1), alpha1-adaptin, alpha-adaptin A, AP-2 complex subunit alpha-1, 100 kDa coated vesicle protein A
<220>
   <221> CDS
   <222> (212) ... (3145)
   <223> AP2A1
<400> 92
   cggctcagag ctccggaccg cgggcggagg ggaggggcag ggggcggtgc cacggcctgc 60
   cagcccgccc gcccgcccgc cagccagccc tccccgcggc cggctcggct ccttggcgct 120
   gcctggggtc ctttccgccc ggtccccgct tgccagcccc cgctgctctg tgccctgtcc 180
   ggccaggcct ggagccgaca ccaccgccat catgccggcc gtgtccaagg gcgatgggat 240
   gcgggggctc gcggtgttca tctccgacat ccggaactgt aagagcaaag aggcggaaat 300
   taagagaatc aacaaggaac tggccaacat ccgctccaag ttcaaaggag acaaagcctt 360
   ggatggctac agtaagaaaa aatatgtgtg taaactgctt ttcatcttcc tgcttggcca 420
   tgacattgac tttgggcaca tggaggctgt gaatctgttg agttccaata aatacacaga 480
   gaagcaaata ggttacctgt tcatttctgt gctggtgaac tcgaactcgg agctgatccg 540
   cctcatcaac aacgccatca agaatgacct ggccagccgc aaccccacct tcatgtgcct 600
   ggccctgcac tgcatcgcca acgtgggcag ccgggagatg ggcgaggcct ttgccgctga 660
   catcccccgc atcctggtgg ccggggacag catggacagt gtcaagcaga gtgcggccct 720
   gtgcctcctt cgactgtaca aggcctcgcc tgacctggtg cccatgggcg agtggacggc 780
   gcgtgtggta cacctgctca atgaccagca catgggtgtg gtcacggccg ccgtcagcct 840
   catcacctgt ctctgcaaga agaacccaga tgacttcaag acgtgcgtct ctctggctgt 900
   gtcgcgcctg agccggatcg tctcctctgc ctccaccgac ctccaggact acacctacta 960
   cttcgtccca gcaccctggc tctcggtgaa gctcctgcgg ctgctgcagt gctacccgcc 1020
   tccagaggat gcggctgtga aggggcggct ggtggaatgt ctggagactg tgctcaacaa 1080
   ggcccaggag ccccccaaat ccaagaaggt gcagcattcc aacgccaaga acgccatcct 1140
   cttcgagacc atcagcctca tcatccacta tgacagtgag cccaacctcc tggttcgggc 1200
   ctgcaaccag ctgggccagt tcctgcagca ccgggagacc aacctgcgct acctggccct 1260
   ggagagcatg tgcacgctgg ccagctccga gttctcccat gaagccgtca agacgcacat 1320
   tgacaccgtc atcaatgccc tcaagacgga gcgggacgtc agcgtgcggc agcgggcggc 1380
   tgacctcctc tacgccatgt gtgaccggag caatgccaag cagatcgtgt cggagatgct 1440
   gcggtacctg gagacggcag actacgccat ccgcgaggag atcgtcctga aggtggccat 1500
   cctggccgag aagtacgccg tggactacag ctggtacgtg gacaccatcc tcaacctcat 1560
   ccgcattgcg ggcgactacg tgagtgagga ggtgtggtac cgtgtgctac agatcgtcac 1620
   caaccgtgat gacgtccagg gctatgccgc caagaccgtc tttgaggcgc tccaggcccc 1680
   tgcctgtcac gagaacatgg tgaaggttgg cggctacatc cttggggagt ttgggaacct 1740
   gattgctggg gacccccgct ccagcccccc agtgcagttc tccctgctcc actccaagtt 1800
   ccatctgtgc agcgtggcca cgcgggcgct gctgctgtcc acctacatca agttcatcaa 1860
   cctcttcccc gagaccaagg ccaccatcca gggcgtcctg cgggccggct cccagctgcg 1920
   caatgctgac gtggagctgc agcagcgagc cgtggagtac ctcaccctca gctcagtggc 1980
   cagcaccgac gtcctggcca cggtgctgga ggagatgccg cccttccccg agcgcgagtc 2040
   gtccatcctg gccaagctga aacgcaagaa ggggccaggg gccggcagcg ccctggacga 2100
   tggccggagg gaccccagca gcaacgacat caacgggggc atggagccca cccccagcac 2160
   tgtgtcgacg ccctcgccct ccgccgacct cctggggctg cgggcagccc ctcccccggc 2220
   agcacccccg gcttctgcag gagcagggaa ccttctggtg gacgtcttcg atggcccggc 2280
   cgcccagccc agcctggggc ccacccccga ggaggccttc ctcagcgagc tggagccgcc 2340
   tgcccccgag agccccatgg ctttgctggc tgacccagct ccagctgctg acccaggtcc 2400
   tgaggacatc ggccctccca ttccggaagc cgatgagttg ctgaataagt ttgtgtgtaa 2460
   gaacaacggg gtcctgttcg agaaccagct gctgcagatc ggagtcaagt cagagttccg 2520
   acagaacctg ggccgcatgt atctcttcta tggcaacaag acctcggtgc agttccagaa 2580
   tttctcaccc actgtggttc acccgggaga cctccagact cagctggctg tgcagaccaa 2640
   gcgcgtggcg gcgcaggtgg acggcggcgc gcaggtgcag caggtgctca atatcgagtg 2700
   cctgcgggac ttcctgacgc ccccgctgct gtccgtgcgc ttccggtacg gtggcgcccc 2760
   ccaggccctc accctgaagc tcccagtgac catcaacaag ttcttccagc ccaccgagat 2820
   ggcggcccag gatttcttcc agcgctggaa gcagctgagc ctccctcaac aggaggcgca 2880
   gaaaatcttc aaagccaacc accccatgga cgcagaagtt actaaggcca agcttctggg 2940
   gtttggctct gctctcctgg acaatgtgga ccccaaccct gagaacttcg tgggggcggg 3000
   gatcatccag actaaagccc tgcaggtggg ctgtctgctt cggctggagc ccaatgccca 3060
   ggcccagatg taccggctga ccctgcgcac cagcaaggag cccgtctccc gtcacctgtg 3120
   tgagctgctg gcacagcagt tctgagccct ggactctgcc ccgggggatg tggccggcac 3180
   tgggcagccc cttggactga ggcagttttg gtggatgggg gacctccact ggtgacagag 3240
   aagacaccag ggtttggggg atgcctggga ctttcctccg gccttttgta tttttatttt 3300
   tgttcatctg ctgctgttta cattctgggg ggttaggggg agtccccctc cctccctttc 3360
   ccccccaagc acagagggga gaggggccag ggaagtggat gtctcctccc ctcccacccc 3420
   accctgttgt agcccctcct accccctccc catccagggg ctgtgtatta ttgtgagcga 3480
   ataaacagag agacgctaa 3499
<210> 93
   <211> 5125
   <212> DNA
   <213> Homo sapiens
<220>
   <223> nucleosome assembly protein 1-like 1 transcript variant 2 (NAP1L1, NAP1, NAP1L), NAP-1-related protein, HSP22-like protein interacting protein
<220>
   <221> CDS
   <222> (413) ... (1588)
   <223> NAP1L1
<400> 93
   aaaagatatg gtggggtgct taacagagga ggttagacac cggcgggaac cagaggagcc 60
   caagcgcggc gcctgggcct cggggctgca ggagtcctcg gtgggggtat ggaggtcgcc 120
   ggggaaggag gacggttcag ttgctaggca acccggcctg gacccgcctc tcgctcgcgt 180
   tgctgggaga ctacaaggcc gggaggaggg cggcgaaagg gccctacgtg ctgacgctaa 240
   ttgtatatga gcgcgagcgg cgggctcttg ggtctttttt agcgccatct gctcgcggcg 300
   ccgcctcctg ctcctcccgc tgctgctgcc gctgccgccc tgagtcactg cctgcgcagc 360
   tccggccgcc tggctcccca tactagtcgc cgatatttgg agttcttaca acatggcaga 420
   cattgacaac aaagaacagt ctgaacttga tcaagatttg gatgatgttg aagaagtaga 480
   agaagaggaa actggtgaag aaacaaaact caaagcacgt cagctaactg ttcagatgat 540
   gcaaaatcct cagattcttg cagcccttca agaaagactt gatggtctgg tagaaacacc 600
   aacaggatac attgaaagcc tgcctagggt agttaaaaga cgagtgaatg ctctcaaaaa 660
   cctgcaagtt aaatgtgcac agatagaagc caaattctat gaggaagttc acgatcttga 720
   aaggaagtat gctgttctct atcagcctct atttgataag cgatttgaaa ttattaatgc 780
   aatttatgaa cctacggaag aagaatgtga atggaaacca gatgaagaag atgagatttc 840
   ggaggaattg aaagaaaagg ccaagattga agatgagaaa aaagatgaag aaaaagaaga 900
   ccccaaagga attcctgaat tttggttaac tgtttttaag aatgttgact tgctcagtga 960
   tatggttcag gaacacgatg aacctattct gaagcacttg aaagatatta aagtgaagtt 1020
   ctcagatgct ggccagccta tgagttttgt cttagaattt cactttgaac ccaatgaata 1080
   ttttacaaat gaagtgctga caaagacata caggatgagg tcagaaccag atgattctga 1140
   tcccttttct tttgatggac cagaaattat gggttgtaca gggtgccaga tagattggaa 1200
   aaaaggaaag aatgtcactt tgaaaactat taagaagaag cagaaacaca agggacgtgg 1260
   gacagttcgt actgtgacta aaacagtttc caatgactct ttctttaact tttttgcccc 1320
   tcctgaagtt cctgagagtg gagatctgga tgatgatgct gaagctatcc ttgctgcaga 1380
   cttcgaaatt ggtcactttt tacgtgagcg tataatccca agatcagtgt tatattttac 1440
   tggagaagct attgaagatg atgatgatga ttatgatgaa gaaggtgaag aagcggatga 1500
   ggaaggggaa gaagaaggag atgaggaaaa tgatccagac tatgacccaa agaaggatca 1560
   aaacccagca gagtgcaagc agcagtgaag caggatgtat gtggccttga ggataacctg 1620
   cactgtaata gcctaaacac aactcttatt tacttacagc cttatgtttt tgtattttct 1680
   tggtagacta ggtaattttt ttttaaagga caggaaactg atattttaaa gaccaatttg 1740
   ttctacctag cattttaact agtttttctg ccagctatgt tgaatgcaca aattctgtca 1800
   cgcatgttca ttcattgcta cataatttgg ttcttctgga atatttttat gtagctcttg 1860
   gagtacagct atgaaaatta acaactgtta aaggaaatac cttttttttt tttttgtaat 1920
   tttttccttg aagaaccaaa gtattttttc agctggttgt tgaatagggt taagtccgct 1980
   tggattagct gtgcctttca ttactttgtt acagaaatgc agtgacttat actaagacaa 2040
   tttattgttt aaaaaaaaaa ttggcaagac aactatatgg ttaagaattt ccagtatgac 2100
   cacacccaat aactgttatt agagtgttaa tggattattg tgttttaggt gacatagtta 2160
   actgtaaagt aacctgactc agtatagtta ctggtaccac agtgaggtga ataaaacggg 2220
   attttcagaa gttagcctga atttaactgt atttttaaat ttaacctcca ttaactaagc 2280
   atcttttctt tgtggtaggg tctaccttct gcttccctgg aaaggatgaa tttacatcat 2340
   ttgacaagcc tattttcaag ttatttgttg tttgtttgct tgtttttgtt tttgcagcta 2400
   aaataaaaat ttcaaataca attttagttc ttacaagata atgtcttaat tttgtaccaa 2460
   ttcaggtaga agtagaggcc taccttgaat taagggttat actcagtttt taacacattg 2520
   ttgaagaaaa ggtaccagct ttggaacgag atgctatact aataagcaag tgtaaaaaaa 2580
   aaaaaaaaag aggaagaaaa tcttaagtga ttgatgctgt tttcttttaa aaaaaaaaaa 2640
   aaaaattcat tttctttggg ttagagctag agagaaggcc ccaagcttct atggtttctt 2700
   ctaattctta ttgcttaaag tatgagtatg tcacttaccc gtgcttctgt ttactgtgta 2760
   attaaaatgg gtagtactgt ttacctaact acctcatgga tgtgttaagg catattgagt 2820
   taaatctcat ataatgtttc tcaatcttgt taaaagctca aaattttggg cctatttgta 2880
   atgccagtgt gacactaagc attttgttca caccacgctt tgataactaa actggaaaac 2940
   aaaggtgtta agtacctctg ttctggatct gggcagtcag cactcttttt agatctttgt 3000
   gtggctccta tttttataga agtggaggga tgcactattt cacaaggtcc aagatttgtt 3060
   ttcagatatt tttgatgact gtattgtaaa tactacaggg atagcactat agtattgtag 3120
   tcatgagact taaagtggaa ataagactat ttttgacaaa agatgccatt aaatttcaga 3180
   ctgtagagcc acatttacaa tacctcaggc taattactgt taattttggg gttgaacttt 3240
   tttttgacag tgagggtgga ttattggatt gtcattagag gaaggtctag atttcctgct 3300
   cttaataaaa ttacattgaa ttgattttta gaggtaatga aaacttcctt tctgagaagt 3360
   tagtgttaag gtcttggaat gtgaacacat tgtttgtagt gctatccatt cctctcctga 3420
   gattttaact tactactgga aatccttaac caattataat agcttttttt ctttattttc 3480
   aaaatgattt cctttgcttt gattagacac tatgtgcttt ttttttttaa ccatagttca 3540
   tcgaaatgca gctttttctg aacttcaaag atagaatccc atttttaatg aactgaagta 3600
   gcaaaatcat ctttttcatt ctttaggaaa tagctattgc caaagtgaag gtgtagataa 3660
   tacctagtct tgttacataa aggggatgtg gtttgcagaa gaattttctt tataaaattg 3720
   aagttttaag ggacgtcagt gtttatgcca tttttccagt tccaaaatga ttccattcca 3780
   ttctagaaat ttgaagtatg taacctgaaa tccttaataa aatttggatt taattttata 3840
   aaatgtactg gtgatatttt gggtgttttt ttttaaatga atgtatatac tttttttttg 3900
   aagagtggag agtagtgatg tctagaggga gctattttgt gctgaggcca ctatgttctg 3960
   taaatatata attttaagag caacctcaca atccctgcta agtggagttt attatttgaa 4020
   gactaaaatg gaattccata gttcctgata ggttatattc tgggttatta ttctgagtta 4080
   tctacaaaca tttttgagat ttgtctttac actctgattg tagtttccag cagcccatgc 4140
   acactgccaa gtaagtctca ttttttcctg ttagaaatgg tgaaatatca tataatcact 4200
   tataaagaaa actgatatga aaaaatttta gagttgtttg ctttatggtc actcaagtag 4260
   ggtaagtgtt ccacaaattc cacaagttga tagtttaaca tggatgtctg aaagccacat 4320
   atataatttc ttaggattct taaattagta aatctagctt actgaagcag tattagcatc 4380
   actattttag attgcaaaaa taccttaatt gtgtggaact ggcttgtaga gtggtactta 4440
   agaaaaatgg gattctacct ctatttctgt tttagcacac ttaatcagga aaggatatat 4500
   taactttcat aaaaatattt ttgttgtgtg aataggttaa tgatatggta aggcccctaa 4560
   aataactgaa ttaattgttt attgtaattg taggccattc ccattattaa aaataaagac 4620
   aaaacttgaa gtaactgaaa atcttatcgt gctatgtaga aatattgaac taatattcaa 4680
   atatttgaat gctttggttt cagggattgg tttaaaattg gagtcctttt ttatgggtta 4740
   gtcttacaaa aatttaagcc tttatatttt tgactttaaa tcaaaacaaa tgttatttta 4800
   aatgtacaga atagattggt agtgcagaag agtgtaagtt cttcatagga gctttagaaa 4860
   agagaaatat gtgctaattc agtttttttt taatctgcac tgtacatata tacttggtaa 4920
   ttatgagctt gattttgttt ttggaaatat gtgttcataa tttaggtaat ttgctactta 4980
   aagcactaag tctctgatac ctgaaaagta catgtaaatg gtgatggtga aataatactg 5040
   cagttaactt aatagatgta tactggtgat ttttgtatgc tggattaaaa ctccagatat 5100
   taaaatataa cctggataaa aagcc 5125
<210> 94
   <211> 787
   <212> DNA
   <213> Homo sapiens
<220>
   <223> 40S ribosomal protein S14 transcript variant 2, S14, emetine resistance (EMTB)
<220>
   <221> CDS
   <222> (268)...(723)
   <223> RPS14
<400> 94
   ctccgccccc tcccactctc tctttccggt gtggagtctg gagacgacgt gcaggtagga 60
   ggcccgggcg cgacaatcgg ggggcatcct gcggcgaggg gaccctgtgg ggcttgggac 120
   gagagacggg ggtctttccg tgggaaccga gctaggtgcc gggcaagaga cgcgcggctg 180
   gcccacctgg atcctggcca actcgggatt gagttcgttc ctggtctcag aaggcccgtt 240
   ttgctttcag ggaggagctt gtgaaaaatg gcacctcgaa aggggaagga aaagaaggaa 300
   gaacaggtca tcagcctcgg acctcaggtg gctgaaggag agaatgtatt tggtgtctgc 360
   catatctttg catccttcaa tgacactttt gtccatgtca ctgatctttc tggcaaggaa 420
   accatctgcc gtgtgactgg tgggatgaag gtaaaggcag accgagatga atcctcacca 480
   tatgctgcta tgttggctgc ccaggatgtg gcccagaggt gcaaggagct gggtatcacc 540
   gccctacaca tcaaactccg ggccacagga ggaaatagga ccaagacccc tggacctggg 600
   gcccagtcgg ccctcagagc ccttgcccgc tcgggtatga agatcgggcg gattgaggat 660
   gtcaccccca tcccctctga cagcactcgc aggaaggggg gtcgccgtgg tcgccgtctg 720
   tgaacaagat tcctcaaaat attttctgtt aataaattgc cttcatgtaa actgtttcaa 780
   aaaaaaa                                                           787
<210> 95
   <211> 978
   <212> DNA
   <213> Homo sapiens
<220>
   <223> ethylmalonic encephalopathy 1 (ETHE1), persulfide dioxygenase ETHE1, mitochondrial, sulfur dioxygenase ETHE1, hepatoma subtracted clone one protein (HSCO), YF13H12, protein ETHE1, mitochondrial
<220>
   <221> CDS
   <222> (68)...(832)
   <223> ETHE1
<400> 95
   agtgccgtag cgcccggctc ctgcaggcgc tcggcctccg ctcattcctg accccgcagt 60
   gggcgcgatg gcggaggctg tactgagggt cgcccggcgg cagctgagcc agcgcggcgg 120
   gtctggagcc cccatcctcc tgcggcagat gttcgagcct gtgagctgca ccttcacgta 180
   cctgctgggt gacagagagt cccgggaggc cgttctgatc gacccagtcc tggaaacagc 240
   gcctcgggat gcccagctga tcaaggagct ggggctgcgg ctgctctatg ctgtgaatac 300
   ccactgccac gcggaccaca ttacaggctc ggggctgctc cgttccctcc tccctggctg 360
   ccagtctgtc atctcccgcc ttagtggggc ccaggctgac ttacacattg aggatggaga 420
   ctccatccgc ttcgggcgct tcgcgttgga gaccagggcc agccctggcc acaccccagg 480
   ctgtgtcacc ttcgtcctga atgaccacag catggccttc actggagatg ccctgttgat 540
   ccgtgggtgt gggcggacag acttccagca aggctgtgcc aagaccttgt accactcggt 600
   ccatgaaaag atcttcacac ttccaggaga ctgtctgatc taccctgctc acgattacca 660
   tgggttcaca gtgtccaccg tggaggagga gaggactctg aaccctcggc tcaccctcag 720
   ctgtgaggag tttgtcaaaa tcatgggcaa cctgaacttg cctaaacctc agcagataga 780
   ctttgctgtt ccagccaaca tgcgctgtgg ggtgcagaca cccactgcct gatctcactt 840
   ctgtcagatg ctcccatcca ctattaatgc actaggtggg aggagagggc ggcaatgaca 900
   ctgcacctct cctttcccac cgcattccct ggagctccct aaataaaact ttttttaacg 960
   tgaaaaaaaa aaaaaaaa                                               978
<210> 96
   <211> 1890
   <212> DNA
   <213> Homo sapiens
<220>
   <223> cyclin I (CCNI, CYC1, CYI), cyclin ITI
<220>
   <221> CDS
   <222> (545)...(1678)
   <223> CCNI
<400> 96
   cccgcgagcg gacgcggcag cgcctctgtc tcgctttttc ttatttttcc cccctttccc 60
   ctttcttttt ttttttttct tttcttttct cccctccccc cctttcacca tttcccctcg 120
   gaggcgcttt ccccgggcag gggcagagcc ggtctcaccc cccgcctctc cccggccccc 180
   gccgccctat ggcgagaggg agccccctcc caacccgggc tcgagcggcg gcggcctcag 240
   gccgggggtc atcatggaac taattcgctg accgacccag cggccgcagc cgtgcgtccc 300
   gctcgagcgc cagcgcccgc gcccgcgccc cccgatccgc ttcccctttc tccctcctca 360
   gttggccgag tcgtcccgcg cgcaccgcct ccgcgcgcct atgagaatga ggtggtaacg 420
   ggcccccgga tgaccccgcg tcaccactgt gaggcctaca gctctgccgg ggaggaggag 480
   gaggaggaag aggaggagaa ggtagctaca gcaagctggg tagcaggcag atccaaagga 540
   tatcatgaag tttccagggc ctttggaaaa ccagagattg tctttcctgt tggaaaaggc 600
   aatcactagg gaagcacaga tgtggaaagt gaatgtgcgg aaaatgcctt caaatcagaa 660
   tgtttctcca tcccagagag atgaagtaat tcaatggctg gccaaactca agtaccaatt 720
   caacctttac ccagaaacat ttgctctggc tagcagtctt ttggataggt ttttagctac 780
   cgtaaaggct catccaaaat acttgagttg tattgcaatc agctgttttt tcctagctgc 840
   caagactgtt gaggaagatg agagaattcc agtactaaag gtattggcaa gagacagttt 900
   ctgtggatgt tcctcatctg aaattttgag aatggagaga attattctgg ataagttgaa 960
   ttgggatctt cacacagcca caccattgga ttttcttcat attttccatg ccattgcagt 1020
   gtcaactagg cctcagttac ttttcagttt gcccaaattg agcccatctc aacatttggc 1080
   agtccttacc aagcaactac ttcactgtat ggcctgcaac caacttctgc aattcagagg 1140
   atccatgctt gctctggcca tggttagtct ggaaatggag aaactcattc ctgattggct 1200
   ttctcttaca attgaactgc ttcagaaagc acagatggat agctcccagt tgatccattg 1260
   tcgggagctt gtggcacatc acctttctac tctgcagtct tccctgcctc tgaattccgt 1320
   ttatgtctac cgtcccctca agcacaccct ggtgacctgt gacaaaggag tgttcagatt 1380
   acatccctcc tctgtcccag gcccagactt ctccaaggac aacagcaagc cagaagtgcc 1440
   agtcagaggt acagcagcct tttaccatca tctcccagct gccagtgggt gcaagcagac 1500
   ctctactaaa cgcaaagtag aggaaatgga agtggatgac ttctatgatg gaatcaaacg 1560
   gctctataat gaagataatg tctcagaaaa tgtgggttct gtgtgtggca ctgatttatc 1620
   aagacaagag ggacatgctt ccccttgtcc acctttgcag cctgtttctg tcatgtagtt 1680
   tcaacaagtg ctacctttga gtgtaaacta aggtagacta ctttgggaat gagaacatgc 1740
   aaaatcagga aaggctgtag aaggaaatat accttaacag gctgatttgg agtgagccag 1800
   aaaaaaaaaa taaaactctc attatttgtg tggctaatta taattcagcg ttatttaagc 1860
   acataaagac caaaaaaaaa aaaaaaaaaa                                 1890
<210> 97
   <211> 2856
   <212> DNA
   <213> Homo sapiens
<220>
   <223> metastasis associated 1 transcript variant 1 (MTA1), metastasis-associated protein MTA1 isoform MTA1
<220>
   <221> CDS
   <222> (188) ... (2335)
   <223> MTA1
<400> 97
   gcggccctcc cgtccctgcg cggcctcggc ggcctcggcg gcggcggcgg cggcggcggc 60
   ggcagcagcg cggccccttt aaacgcctgc ggcgcccccc gcccccgcca tcgcgcctcc 120
   attttcccgg ccgcccgcgc cgagcgccgc gcccgccccg ggcccctccg ccgccgccgg 180
   cccggacatg gccgccaaca tgtacagggt cggagactac gtctactttg agaactcctc 240
   cagcaaccca tacctgatcc ggagaatcga ggagctcaac aagacggcca atgggaacgt 300
   ggaggccaaa gtggtgtgct tctaccggag gcgggacatc tccagcaccc tcatcgccct 360
   ggccgacaag cacgcaaccc tgtcagtctg ctataaggcc ggaccggggg cggacaacgg 420
   cgaggaaggg gaaatagaag aggaaatgga gaatccggaa atggtggacc tgcccgagaa 480
   actaaagcac cagctgcggc atcgggagct gttcctctcc cggcagctgg agtctctgcc 540
   cgccacgcac atcaggggca agtgcagcgt caccctgctc aacgagaccg agtcgctcaa 600
   gtcctacctg gagcgggagg atttcttctt ctattctcta gtctacgacc cacagcagaa 660
   gaccctgctg gcagataaag gagagattcg agtaggaaac cggtaccagg cagacatcac 720
   cgacttgtta aaagaaggcg aggaggatgg ccgagaccag tccaggttgg agacccaggt 780
   gtgggaggcg cacaacccac tcacagacaa gcagatcgac cagttcctgg tggtggcccg 840
   ctctgtgggc accttcgcac gggccctgga ctgcagcagc tccgtccgac agcccagcct 900
   gcacatgagc gccgcagctg cctcccgaga catcaccctg ttccacgcca tggatactct 960
   ccacaagaac atctacgaca tctccaaggc catctcggcg ctggtgccgc agggcgggcc 1020
   cgtgctctgc agggacgaga tggaggagtg gtctgcatca gaggccaacc ttttcgagga 1080
   agccctggaa aaatatggga aggatttcac ggacattcag caagattttc tcccgtggaa 1140
   gtcgctgacc agcatcattg agtactacta catgtggaag accaccgaca gatacgtgca 1200
   gcagaaacgc ttgaaagcag ctgaagctga gagcaagtta aagcaagttt atattcccaa 1260
   ctataacaag ccaaatccga accaaatcag cgtcaacaac gtcaaggccg gtgtggtgaa 1320
   cggcacgggg gcgccgggcc agagccctgg ggctggccgg gcctgcgaga gctgttacac 1380
   cacacagtct taccagtggt attcttgggg tccccctaac atgcagtgtc gtctctgcgc 1440
   atcttgttgg acatattgga agaaatatgg tggcttgaaa atgccaaccc ggttagatgg 1500
   agagaggcca ggaccaaacc gcagtaacat gagtccccac ggcctcccag cccggagcag 1560
   cgggagcccc aagtttgcca tgaagaccag gcaggctttc tatctgcaca cgacgaagct 1620
   gacgcggatc gcccggcgcc tgtgccgtga gatcctgcgc ccgtggcacg ctgcgcggca 1680
   cccctacctg cccatcaaca gcgcggccat caaggccgag tgcacggcgc ggctgcccga 1740
   agcctcccag agcccgctgg tgctgaagca ggcggtacgc aagccgctgg aagccgtgct 1800
   tcggtatctt gagacccacc cccgcccccc caagcctgac cccgtgaaaa gcgtgtccag 1860
   cgtgctcagc agcctgacgc ccgccaaggt ggcccccgtc atcaacaacg gctcccccac 1920
   catcctgggc aagcgcagct acgagcagca caacggggtg gacggcaaca tgaagaagcg 1980
   cctcttgatg cccagtaggg gtctggcaaa ccacggacag gccaggcaca tgggaccaag 2040
   ccggaacctc ctgctcaacg ggaagtccta ccccaccaaa gtgcgcctga tccggggggg 2100
   ctccctgccc ccagtcaagc ggcggcggat gaactggatc gacgccccgg atgacgtgtt 2160
   ctacatggcc acagaggaga ccaggaagat ccgcaagctg ctctcatcct cggaaaccaa 2220
   gcgtgctgcc cgccggccct acaagcccat cgccctgcgc cagagccagg ccctgccgcc 2280
   gcggccaccg ccacctgcgc ccgtcaacga cgagcccatc gtcatcgagg actaggggcc 2340
   gcccccacct gcggccgccc cccgcccctc gcccgcccac acggcccctt cccagccagc 2400
   ccgccgcccg cccctcagtt tggtagtgcc ccacctcccg ccctcacctg cagagaaacg 2460
   cgctccttgg cggacactgg gggaggagag gaagaagcgc ggctaactta ttccgagaat 2520
   gccgaggagt tgtcgttttt agctttgtgt ttactttttg gctggagcgg agatgagggg 2580
   ccaccccgtg cccctgtgct gcggggcctt ttgcccggag gccgggccct aaggttttgt 2640
   tgtgttctgt tgaaggtgcc attttaaatt ttatttttat tacttttttt gtagatgaac 2700
   ttgagctctg taacttacac ctggaatgtt aggatcgtgc ggccgcggcc ggccgagctg 2760
   cctggcgggg ttggcccttg tcttttcaag taattttcat attaaacaaa aacaaagaaa 2820
   aaaaatctta taaaaaggaa aaaaaaaaaa aaaaaa                           2856
<210> 98
   <211> 1286
   <212> DNA
   <213> Homo sapiens
<220>
   <223> eukaryotic translation initiation factor 3, subunit H (EIF3H), eukaryotic translation initiation factor 3, subunit 3 gamma, 40kDa (eIF3-gamma, eIF3-p40), eukaryotic translation initiation factor 3, subunit 2 (beta, 36kD)
<220>
   <221> CDS
   <222> (27)...(1085)
   <223> EIF3H
<400> 98
   ctctttcttc ctgtctgctt ggaaagatgg cgtcccgcaa ggaaggtacc ggctctactg 60
   ccacctcttc cagctccacc gccggcgcag cagggaaagg caaaggcaaa ggcggctcgg 120
   gagattcagc cgtgaagcaa gtgcagatag atggccttgt ggtattaaag ataatcaaac 180
   attatcaaga agaaggacaa ggaactgaag ttgttcaagg agtgcttttg ggtctggttg 240
   tagaagatcg gcttgaaatt accaactgct ttcctttccc tcagcacaca gaggatgatg 300
   ctgactttga tgaagtccaa tatcagatgg aaatgatgcg gagccttcgc catgtaaaca 360
   ttgatcatct tcacgtgggc tggtatcagt ccacatacta tggctcattc gttacccggg 420
   cactcctgga ctctcagttt agttaccagc atgccattga agaatctgtc gttctcattt 480
   atgatcccat aaaaactgcc caaggatctc tctcactaaa ggcatacaga ctgactccta 540
   aactgatgga agtttgtaaa gaaaaggatt tttcccctga agcattgaaa aaagcaaata 600
   tcacctttga gtacatgttt gaagaagtgc cgattgtaat taaaaattca catctgatca 660
   atgtcctaat gtgggaactt gaaaagaagt cagctgttgc agataaacat gaattgctca 720
   gccttgccag cagcaatcat ttggggaaga atctacagtt gctgatggac agagtggatg 780
   aaatgagcca agatatagtt aaatacaaca catacatgag gaatactagt aaacaacagc 840
   agcagaaaca tcagtatcag cagcgtcgcc agcaggagaa tatgcagcgc cagagccgag 900
   gagaaccccc gctccctgag gaggacctgt ccaaactctt caaaccacca cagccgcctg 960
   ccaggatgga ctcgctgctc attgcaggcc agataaacac ttactgccag aacatcaagg 1020
   agttcactgc ccaaaactta ggcaagctct tcatggccca ggctcttcaa gaatacaaca 1080
   actaagaaaa ggaagtttcc agaaaagaag ttaacatgaa ctcttgaagt cacaccaggg 1140
   caactcttgg aagaaatata tttgcatatt gaaaagcaca gaggatttct ttagtgtcat 1200
   tgccgatttt ggctataaca gtgtctttct agccataata aaataaaaca aaatcttgac 1260
   tgcttgctca tttgaaaaaa aaaaaa                                      1286
<210> 99
   <211> 766
   <212> DNA
   <213> Homo sapiens
<220>
   <223> 60S ribosomal protein L9 transcript variant 1, L9, NPC-A-16
<220>
   <221> CDS
   <222> (60)...(638)
   <223> RPL9
<400> 99
   acgcgataca agtacgtaat gacgacagac gttctttctt tgctgcgtct actgcgagaa 60
   tgaagactat tctcagcaat cagactgtcg acattccaga aaatgtcgac attactctga 120
   agggacgcac agttatcgtg aagggcccca gaggaaccct gcggagggac ttcaatcaca 180
   tcaatgtaga actcagcctt cttggaaaga aaaaaaagag gctccgggtt gacaaatggt 240
   ggggtaacag aaaggaactg gctaccgttc ggactatttg tagtcatgta cagaacatga 300
   tcaagggtgt tacactgggc ttccgttaca agatgaggtc tgtgtatgct cacttcccca 360
   tcaacgttgt tatccaggag aatgggtctc ttgttgaaat ccgaaatttc ttgggtgaaa 420
   aatatatccg cagggttcgg atgagaccag gtgttgcttg ttcagtatct caagcccaga 480
   aagatgaatt aatccttgaa ggaaatgaca ttgagcttgt ttcaaattca gcggctttga 540
   ttcagcaagc cacaacagtt aaaaacaagg atatcaggaa atttttggat ggtatctatg 600
   tctctgaaaa aggaactgtt cagcaggctg atgaataaga tctaagagtt acctggctac 660
   agaaagaaga tgccagatga cacttaagac ctacttgtga tatttaaatg atgcaataaa 720
   agacctattg atttggacct tcttcttaaa aaaaaaaaaa aaaaaa                766
<210> 100
   <211> 3325
   <212> DNA
   <213> Homo sapiens
<220>
   <223> 40S ribosomal protein S23, S23, homolog of yeast ribosomal protein S28
<220>
   <221> CDS
   <222> (94)...(525)
   <223> RPS23
<400> 100
   ggggtccttg gctgggcggg gcttgctcgc ggtggcttgt ggctccttcc tgcggtgctt 60
   ctctctttcg ctcaggcccg tggcgccgac aggatgggca agtgtcgtgg acttcgtact 120
   gctaggaagc tccgtagtca ccgacgagac cagaagtggc atgataaaca gtataagaaa 180
   gctcatttgg gcacagccct aaaggccaac ccttttggag gtgcttctca tgcaaaagga 240
   atcgtgctgg aaaaagtagg agttgaagcc aaacagccaa attctgccat taggaagtgt 300
   gtaagggtcc agctgatcaa gaatggcaag aaaatcacag cctttgtacc caatgacggt 360
   tgcttgaact ttattgagga aaatgatgaa gttctggttg ctggatttgg tcgcaaaggt 420
   catgctgttg gtgatattcc tggagtccgc tttaaggttg tcaaagtagc caatgtttct 480
   cttttggccc tatacaaagg caagaaggaa agaccaagat cataaatatt aatggtgaaa 540
   acactgtagt aataaatttt catatgccaa aaaatgtttg tatcttactg tcccctgttc 600
   tcaccacgaa gatcatgttc attaccacca ccaccccccc ttattttttt tatcctaaac 660
   cagcaaacgc aggacctgta ccaattttag gagacaataa gacagggttg tttcaggatt 720
   ctctagagtt aataacattt gtaacctggc acagtttccc tcatcctgtg gaataagaaa 780
   atgggataga tctggaataa atgtgcagta ttgtagtatt actttaagaa ctttaaggga 840
   acttcaaaaa ctcactgaaa ttctagtgag atactttctt ttttattctt ggtattttcc 900
   atatcgggtg caacacttca gttaccaaat ttcattgcac atagattatc ttaggtaccc 960
   ttggaaatgc acattcttgt atccatctta caggggccca agatgataaa tagtaaactc 1020
   aaaattgctc cccactctgt ttattattta aaggtgtcag gatctgtgtt gtaatgtgtc 1080
   tacattaatg tgtttaggag aatacaggca ttggatcatt tagttgatgg aagtatatgc 1140
   caggcaaggg agataaggta tacgacaaga ctgatgtttt cagtatcttc tcatgaggtt 1200
   gtcagagacc ttcatgtctt caaagactag tcagcaaatg aagtggttta gtgtagagac 1260
   aagattggtt gtgttttgat aatttaagct aggtattgag tacatgtgga ttttgctgtc 1320
   cacaaatact tgtttcagag ttttcatgga tacagtggca tggttgaaat gaagctgtga 1380
   gccttctgct ttaaatctga tgtaagaaac tcctgttaac aaatagtaag tatgggttaa 1440
   ttagcccttt gatcaaagcc tagctttaca ttgtttagga tctttggaaa acaattggtt 1500
   tggttgccca ctttccgtag gatcaagagc agaacctttc acatggcaca gaagaaccca 1560
   ggttgcgctt catacctgca tattccagcc ttagcctgcc atttctctcc ttggcacttt 1620
   gtgctccagc aacactggtc tcagttggtc atcctcaaac ttgggttcca tatccagcct 1680
   caggacctct gttcctgtta ctatggttcc ttgcatgtcg cctgctctta ctaaagagct 1740
   cgtgtgtttt ccagcacact tcggtttatc tcttgatgat gatgctagtc tctccctccg 1800
   caagggcgga aaggctgcct gttggtttgt accagtgttt cctaacgtgt agctgcagtc 1860
   agtatttggc taagctgttc ccaggggctc aacagatgct ttcggatgag ccttaactga 1920
   cccaatcctt tgtgatgcgg gagagattgc taggcctcgc tcacctggcc agaaccaggg 1980
   aaagaggccg cggttgcagc gcgattccag gccctgggcg tcaggcgcgg ggtgggcagc 2040
   tctccccggg cggtggggcc cttgtgaccg cgaggcgggg cgcaccagga agggagtggg 2100
   acagcgcggg cgcccaggga tgtggcctgg ttacctgcct tctctgatac gtcaagacac 2160
   cttcaacaat ggcttgcagc tgtaccctgt tggctgcacc caggacgccc ttttcactgc 2220
   taagcagtcc tacctgaggc ccaggggctg ccagattgac ccataaataa tctccggcgc 2280
   ctcagatcca gaagctgctg agcctgatct tagtgccttc tcctttctct gtgtggcccc 2340
   ccagcccctt tccccactgc cttgtgtcca aggccctttc cttcatgtat ccatggagga 2400
   gagacaaaaa tacacatcaa taaaataaga tagggaatcc ataaatagac attcagaagt 2460
   atggccaacg gatttatctt aaaaccaatg gaggaagaag agtttcaata aatgttgtgg 2520
   acttccattt gtcaaagacc aaaacaaagg aaccccaacc ttacatgtaa tacaaactta 2580
   actcaaaatg gatcatatat ctaaatgtaa aatggaaagc tataaaactg aaaacagact 2640
   atctttacaa cctaggcgta ggtatagttt ttagacatta caccaaaagc acatgccgta 2700
   aaagaaaaaa tagataaatt ggtggatttc attaaaatta aaaaactttt tctctctgaa 2760
   aaatcctgtt aagctgggcg ctgtggttca tgcctgtaat cccagcactt tgggaggctg 2820
   agttgggaag aaattaatag cttgaggcca ggagttcaag atcatcctgg gcagcaaagt 2880
   catacactct tgagggaaga gagagacctt ctcatattgt tttatattgt tttatactca 2940
   gtacctgttt taagaaaaaa acaaggaagt gaaatcaaag acaggcagcc cggcaccagg 3000
   cctgaaacca gccctgggcc tgcctggcct aaacctagta gttaaaaatc aacttacgac 3060
   ttagaacctg atgttatccg tagattccaa gcattgtata aaaaaattgt gaaactccct 3120
   gttgtgttct gtaccagtgc atgaaacccc tgtcacatat cccctagatt gctcaatcaa 3180
   tcacgaccct ttcatgtgaa atctttagtg ttgtgagccc ttaaaaggga cagaaattgt 3240
   gcacttgagg agctcagatt ttaaggctgt agcttgccga tgctcccagc tgaataaagc 3300
   ccttccttct acaaaaaaaa aaaaa                                       3325
<210> 101
   <211> 829
   <212> DNA
   <213> Homo sapiens
<220>
   <223> 40S ribosomal protein S6, S6, phosphoprotein NP33
<220>
   <221> CDS
   <222> (43)...(792)
   <223> RPS6
<400> 101
   cctcttttcc gtggcgcctc ggaggcgttc agctgcttca agatgaagct gaacatctcc 60
   ttcccagcca ctggctgcca gaaactcatt gaagtggacg atgaacgcaa acttcgtact 120
   ttctatgaga agcgtatggc cacagaagtt gctgctgacg ctctgggtga agaatggaag 180
   ggttatgtgg tccgaatcag tggtgggaac gacaaacaag gtttccccat gaagcagggt 240
   gtcttgaccc atggccgtgt ccgcctgcta ctgagtaagg ggcattcctg ttacagacca 300
   aggagaactg gagaaagaaa gagaaaatca gttcgtggtt gcattgtgga tgcaaatctg 360
   agcgttctca acttggttat tgtaaaaaaa ggagagaagg atattcctgg actgactgat 420
   actacagtgc ctcgccgcct gggccccaaa agagctagca gaatccgcaa acttttcaat 480
   ctctctaaag aagatgatgt ccgccagtat gttgtaagaa agcccttaaa taaagaaggt 540
   aagaaaccta ggaccaaagc acccaagatt cagcgtcttg ttactccacg tgtcctgcag 600
   cacaaacggc ggcgtattgc tctgaagaag cagcgtacca agaaaaataa agaagaggct 660
   gcagaatatg ctaaactttt ggccaagaga atgaaggagg ctaaggagaa gcgccaggaa 720
   caaattgcga agagacgcag actttcctct ctgcgagctt ctacttctaa gtctgaatcc 780
   agtcagaaat aagatttttt gagtaacaaa taaataagat cagactctg            829
<210> 102
   <211> 745
   <212> DNA
   <213> Homo sapiens
<220>
   <223> 60S ribosomal protein S7, S7, DBA8
<220>
   <221> CDS
   <222> (107)...(691)
   <223> RPS7
<400> 102
   gcgctgtttc cgcctcttgc cttcggacgc cggattttga cgtgctctcg cgagatttgg 60
   gtctcttcct aagccggcgc tcggcaagtt ctcccaggag aaagccatgt tcagttcgag 120
   cgccaagatc gtgaagccca atggcgagaa gccggacgag ttcgagtccg gcatctccca 180
   ggctcttctg gagctggaga tgaactcgga cctcaaggct cagctcaggg agctgaatat 240
   tacggcagct aaggaaattg aagttggtgg tggtcggaaa gctatcataa tctttgttcc 300
   cgttcctcaa ctgaaatctt tccagaaaat ccaagtccgg ctagtacgcg aattggagaa 360
   aaagttcagt gggaagcatg tcgtctttat cgctcagagg agaattctgc ctaagccaac 420
   tcgaaaaagc cgtacaaaaa ataagcaaaa gcgtcccagg agccgtactc tgacagctgt 480
   gcacgatgcc atccttgagg acttggtctt cccaagcgaa attgtgggca agagaatccg 540
   cgtcaaacta gatggcagcc ggctcataaa ggttcatttg gacaaagcac agcagaacaa 600
   tgtggaacac aaggttgaaa ctttttctgg tgtctataag aagctcacgg gcaaggatgt 660
   taattttgaa ttcccagagt ttcaattgta aacaaaaatg actaaataaa aagtatatat 720
   tcacagtaaa aaaaaaaaaa aaaaa                                      745
<210> 103
   <211> 748
   <212> DNA
   <213> Homo sapiens
<220>
   <223> 60S ribosomal protein L19, L19
<220>
   <221> CDS
   <222> (63)...(653)
   <223> RPL19
<400> 103
   gcagataatg ggaggagccg ggcccgagcg agctctttcc tttcgctgct gcggccgcag 60
   ccatgagtat gctcaggctt cagaagaggc tcgcctctag tgtcctccgc tgtggcaaga 120
   agaaggtctg gttagacccc aatgagacca atgaaatcgc caatgccaac tcccgtcagc 180
   agatccggaa gctcatcaaa gatgggctga tcatccgcaa gcctgtgacg gtccattccc 240
   gggctcgatg ccggaaaaac accttggccc gccggaaggg caggcacatg ggcataggta 300
   agcggaaggg tacagccaat gcccgaatgc cagagaaggt cacatggatg aggagaatga 360
   ggattttgcg ccggctgctc agaagatacc gtgaatctaa gaagatcgat cgccacatgt 420
   atcacagcct gtacctgaag gtgaagggga atgtgttcaa aaacaagcgg attctcatgg 480
   aacacatcca caagctgaag gcagacaagg cccgcaagaa gctcctggct gaccaggctg 540
   aggcccgcag gtctaagacc aaggaagcac gcaagcgccg tgaagagcgc ctccaggcca 600
   agaaggagga gatcatcaag actttatcca aggaggaaga gaccaagaaa taaaacctcc 660
   cactttgtct gtacatactg gcctctgtga ttacatagat cagccattaa aataaaacaa 720
   gccttaatct gcaaaaaaaa aaaaaaaa                                  748
<210> 104
   <211> 1458
   <212> DNA
   <213> Homo sapiens
<220>
   <223> 60S ribosomal protein L4, L4, ribosomal protein L1
<220>
   <221> CDS
   <222> (66)...(1349)
   <223> RPL4
<400> 104
   aagcacttcc ttttcctgtg gcagcagccg ggctgagagg agcgtggctg tctcctctct 60
   ccgccatggc gtgtgctcgc ccactgatat cggtgtactc cgaaaagggg gagtcatctg 120
   gcaaaaatgt cactttgcct gctgtattca aggctcctat tcgaccagat attgtgaact 180
   ttgttcacac caacttgcgc aaaaacaaca gacagcccta tgctgtcagt gaattagcag 240
   gtcatcagac tagtgctgag tcttggggta ctggcagagc tgtggctcga attcccagag 300
   ttcgaggtgg tgggactcac cgctctggcc agggtgcttt tggaaacatg tgtcgtggag 360
   gccgaatgtt tgcaccaacc aaaacctggc gccgttggca tcgtagagtg aacacaaccc 420
   aaaaacgata cgccatctgt tctgccctgg ctgcctcagc cctaccagca ctggtcatgt 480
   ctaaaggtca tcgtattgag gaagttcctg aacttccttt ggtagttgaa gataaagttg 540
   aaggctacaa gaagaccaag gaagctgttt tgctccttaa gaaacttaaa gcctggaatg 600
   atatcaaaaa ggtctatgcc tctcagcgaa tgagagctgg caaaggcaaa atgagaaacc 660
   gtcgccgtat ccagcgcagg ggcccgtgca tcatctataa tgaggataat ggtatcatca 720
   aggccttcag aaacatccct ggaattactc tgcttaatgt aagcaagctg aacattttga 780
   agcttgctcc tggtgggcat gtgggacgtt tctgcatttg gactgaaagt gctttccgga 840
   agttagatga attgtacggc acttggcgta aagccgcttc cctcaagagt aactacaatc 900
   ttcccatgca caagatgatt aatacagatc ttagcagaat cttgaaaagc ccagagatcc 960
   aaagagccct tcgagcacca cgcaagaaga tccatcgcag agtcctaaag aagaacccac 1020
   tgaaaaactt gagaatcatg ttgaagctaa acccatatgc aaagaccatg cgccggaaca 1080
   ccattcttcg ccaggccagg aatcacaagc tccgggtgga taaggcagct gctgcagcag 1140
   cggcactaca agccaaatca gatgagaagg cggcggttgc aggcaagaag cctgtggtag 1200
   gtaagaaagg aaagaaggct gctgttggtg ttaagaagca gaagaagcct ctggtgggaa 1260
   aaaaggcagc agctaccaag aaaccagccc ctgaaaagaa gcctgcagag aagaaaccta 1320
   ctacagagga gaagaagcct gctgcataaa ctcttaaatt tgattattcc ataaaggtca 1380
   aatcattttg gacagcttct tttgaataaa gacctgatta tacaggcagt gagaaacatg 1440
   aaaaaaaaaa aaaaaaaa                                                1458
<210> 105
   <211> 529
   <212> DNA
   <213> Homo sapiens
<220>
   <223> 40S ribosomal protein S13, S13
<220>
   <221> CDS
   <222> (33)...(488)
   <223> RPS13
<400> 105
   cgctctcctt tcgttgcctg atcgccgcca tcatgggtcg catgcatgct cccgggaagg 60
   gcctgtccca gtcggcttta ccctatcgac gcagcgtccc cacttggttg aagttgacat 120
   ctgacgacgt gaaggagcag atttacaaac tggccaagaa gggccttact ccttcacaga 180
   tcggtgtaat cctgagagat tcacatggtg ttgcacaagt acgttttgtg acaggcaata 240
   aaattttaag aattcttaag tctaagggac ttgctcctga tcttcctgaa gatctctacc 300
   atttaattaa gaaagcagtt gctgttcgaa agcatcttga gaggaacaga aaggataagg 360
   atgctaaatt ccgtctgatt ctaatagaga gccggattca ccgtttggct cgatattata 420
   agaccaagcg agtcctccct cccaattgga aatatgaatc atctacagcc tctgccctgg 480
   tcgcataaat ttgtctgtgt actcaagcaa taaaatgatt gtttaacta             529
<210> 106
   <211> 2836
   <212> DNA
   <213> Homo sapiens
<220>
   <223> GC-rich sequence DNA-binding factor 1 (GCFC, GCFC1), chromosome 21 open reading frame 66 (C21orf66), PAX3 and PAX7 binding protein 1 transcript variant 2 (PAXBP1), functional spliceosome-associated protein 105 (FSAP105), BM020
<220>
   <221> CDS
   <222> (191) ... (2638)
   <223> C21orf66
<400> 106
   ctctctctct ttctccaagt attgagagcg cgtgggagca taggcgcatg cgcgctcgtg 60
   gggtgcgcgg tagcaacaga ggactcgacc cggctggagc tccggagagc gcgcgtgcgc 120
   cgtcacgagc tcggcgctgc cggggccgcg gtgtggaagc gagtattcga ccgccgtgcg 180
   ggccgcgggg atgttccgaa aggcccggcg ggtgaacgtg cgcaagcgga acgactccga 240
   agaggaagag cgggaacgcg atgaggagca ggagccgccg ccgttgttgc cgccgccggg 300
   cacgggcgaa gaggcgggcc ccggtggcgg cgacagggcc cctggcgggg agtcgctgct 360
   gggcccgggg ccgtcgccgc cttccgcgct gaccccgggc ctcggggctg aggccggggg 420
   cggcttcccc ggcggcgcgg agcccggcaa cgggctgaag ccgcgcaaga ggcctcgcga 480
   gaacaaagag gtgccccggg ccagcctgct cagcttccag gacgaggagg aagaaaatga 540
   agaagttttc aaagtgaaga aatcaagtta tagcaaaaag atagtaaaat tgctcaagaa 600
   ggaatataaa gaagatcttg aaaaatcgaa gattaagaca gaactcaact catcagctga 660
   aagtgaacaa cctttggaca aaacaggaca tgttaaggat acaaatcaag aagatggagt 720
   tatcatcagt gaacatggtg aagatgaaat ggatatggaa agtgaaaaag aggaagaaaa 780
   gccaaagact ggtggagctt tttcaaatgc tttatcttca ttgaatgttc ttcgtccagg 840
   agaaattcca gatgcagctt ttatacatgc tgcaaggaaa aagcgccaaa tggcccgaga 900
   attgggagat ttcactcctc atgataatga gcctggtaaa ggccgccttg ttagagaaga 960
   tgagaatgat gccagtgatg atgaagatga cgatgagaaa cgccggatag ttttttctgt 1020
   gaaagaaaag tcacaaagac aaaaaattgc tgaggaaata ggaattgagg ggagtgatga 1080
   tgatgcttta gtaactggag aacaggatga agagctcagc cgatgggaac aggagcagat 1140
   aaggaaagga attaatatcc ctcaggttca agccagtcaa cccgcagaag tgaatatgta 1200
   ctaccagaac acttaccaga caatgcctta cggctcatcc tatggcattc cttatagtta 1260
   tacggcctat ggatcatcag atgccaaatc tcaaaaaaca gataatacag tccctttcaa 1320
   aactcccagt aatgagatga ctcccgttac tattgatttg gtaaagaaac agcttaaaga 1380
   caggttggac tccatgaaag aattgcacaa aacaaatcga cagcagcatg agaaacatct 1440
   gcaaagccga gtggactcta ccagggctat tgaaagatta gaagggtctt ctgggggtat 1500
   tggtgaacgg tataaatttt tgcaagaaat gcgagggtat gtccaagact tgcttgagtg 1560
   tttcagtgaa aaggtgccac tgattaatga acttgaatca gcaatacatc agctgtacaa 1620
   acagcgagct tcccgccttg tccaaagacg acaagatgat attaaagatg aatcttcgga 1680
   gttttcaagc cattcaaaca aagctctgat ggcaccaaat cttgactcct ttggacgcga 1740
   tcgggcactg tatcaagagc atgcaaaacg tcgcattgca gagcgggagg ccaggaggac 1800
   tcgtcgtaga caagccagag aacaaaccgg taagatggca gatcaccttg aaggcctttc 1860
   cagtgatgat gaagaaactt ctacagatat tactaatttc aatctggaaa aagatcgaat 1920
   ttcaaaagaa tccggcaaag tttttgaaga tgtccttgaa agtttctatt caattgactg 1980
   tattaaatca cagtttgaag catggcgttc aaaatactac acatcctaca aagatgctta 2040
   cattggcctt tgtttgccaa aattattcaa ccccctcata cgacttcagc tcctcacttg 2100
   gactcctctt gaggcaaaat gtcgtgactt tgagaatatg ctgtggtttg aatctttgct 2160
   gttttatggt tgtgaagaac gagagcaaga aaaagatgat gtagatgttg ccctactacc 2220
   taccattgtg gaaaaggtga ttcttcctaa actaacagtg atagctgaaa atatgtggga 2280
   ccctttttct acaacacaga cttcaagaat ggtgggaatt acactaaaat taatcaatgg 2340
   atatccttca gtagtgaatg cagaaaataa aaatacacag gtatacctaa aggcactttt 2400
   attgagaatg agaagaactt tagatgatga tgtatttatg cccttatatc ccaaaaatgt 2460
   cttagaaaat aaaaattctg ggccttactt gttttttcaa cgacagtttt ggtcttcagt 2520
   taaggtcata aaacccccat tccagagagg gtcctgcccc atacccagaa ggaaagaatg 2580
   ctgctcagag aggccaagaa gaatctggac ggacaggcct tgtgttgtct tctcttgagc 2640
   agtagatcat acctttttgt ccagtcatgt ttctgcatgc ctgtacatat ttcgttaaac 2700
   ctaaacatag tagacaattt ctcctccatc tttgggtctt cattctgaag gctgtcatgt 2760
   tacataaaac tgtgatcaaa taaatttata tgccttttct cctgttaaaa aaaaaaaaaa 2820
   aaaaaaaaaa aaaaaa                                                 2836
<210> 107
   <211> 1374
   <212> DNA
   <213> Homo sapiens
<220>
   <223> leucine carboxyl methyltransferase 1 transcript variant 1 (LCMT1, LCMT), protein phosphatase methyltransferase 1 (PPMT1), protein-leucine O-methyltransferase,[phosphatase 2A protein]-leucine-carboxy methyltransferase 1, CGI-68
<220>
   <221> CDS
   <222> (159) ... (1163)
   <223> LCMT1
<400> 107
   agccggcgtg ggcggcgtca ctgagccgcg ccagctgagc caggtagggc cctaccctct 60
   tctgttgctt tctccctgtg gctcgcgccg tcccccgccg cccgtcgacc ccgcttccat 120
   gtccctggcg gacacagctc ccaggaacct ccacgcccat ggccactagg cagagggaat 180
   cctctatcac ctcctgctgt tccacctcga gctgcgacgc agacgacgag ggcgtgcgcg 240
   gcacctgcga agatgcttcc ctgtgcaaga ggtttgcagt aagcattggc tactggcatg 300
   acccttacat acagcacttt gtgagactgt ctaaagagag gaaagcccct gaaatcaaca 360
   gaggatattt tgctcgagtc catggtgtca gtcagcttat aaaggcattt ctacggaaga 420
   cagaatgtca ttgtcaaatt gtcaaccttg gggcaggcat ggataccacc ttctggagat 480
   taaaggatga agatcttctc ccaagtaaat attttgaggt tgactttcca atgattgtca 540
   cgagaaagct gcacagtatc aaatgcaagc ctcccctatc cagccccatt ctagaactgc 600
   attcagagga cacacttcag atggatggac acatactgga ttcaaagaga tatgccgtta 660
   ttggagcaga tctccgagac ctgtctgaac tggaagagaa gctaaagaaa tgtaacatga 720
   atacacaatt gccaacactc ctgatagctg aatgtgtgct ggtttacatg actccagagc 780
   agtccgcaaa cctcctgaag tgggcagcca acagttttga gagagccatg ttcataaact 840
   acgaacaggt gaacatgggt gatcggtttg ggcagatcat gattgaaaac ctgcggagac 900
   gccagtgtga cctggcggga gtggagacct gcaagtcatt agagtcacag aaagaacggc 960
   tcctgtcgaa tgggtgggaa acagcatcgg ccgtcgacat gatggagttg tacaacaggt 1020
   tacctcgagc tgaagtgagc aggatagaat cacttgaatt cctggatgaa atggagctgc 1080
   tggagcagct catgcggcat tactgccttt gctgggcaac caaaggagga aatgagcttg 1140
   ggctgaagga gataacttat taatctgtcg aaggcttatg ccgagccaga agccgaagcc 1200
   acttgccctc ctggaggaga cctgcaagct ccctgagcgg tgggcgggcc tcgtccgcag 1260
   gtctcatccc acactcttga gaagccttgg tcactacagt ggtcgcacat gttcctcttc 1320
   ctgttcctgt tgacatgtcg ttgtttaaat aaatctcact tgccaccagt aaaa       1374
<210> 108
   <211> 2091
   <212> DNA
   <213> Homo sapiens
<220>
   <223> eukaryotic translation initiation factor 3, subunit L transcript variant 1 (EIF3L), eukaryotic translation initiation factor 3 subunit 6-interacting protein (EIF3S6IP), eukaryotic translation initiation factor 3 subunit E-interacting protein (EIF3EIP)
<220>
   <221> CDS
   <222> (79)... (1773)
   <223> EIF3L
<400> 108
   gctgaacttc cggcctcagg acgcaggcgc gggccgctca tttcgctctt tccggcggtg 60
   ctcgcaagcg aggcagccat gtcttatccc gctgatgatt atgagtctga ggcggcttat 120
   gacccctacg cttatcccag cgactatgat atgcacacag gagatccaaa gcaggacctt 180
   gcttatgaac gtcagtatga acagcaaacc tatcaggtga tccctgaggt gatcaaaaac 240
   ttcatccagt atttccacaa aactgtctca gatttgattg accagaaagt gtatgagcta 300
   caggccagtc gtgtctccag tgatgtcatt gaccagaagg tgtatgagat ccaggacatc 360
   tatgagaaca gctggaccaa gctgactgaa agattcttca agaatacacc ttggcccgag 420
   gctgaagcca ttgctccaca ggttggcaat gatgctgtct tcctgatttt atacaaagaa 480
   ttatactaca ggcacatata tgccaaagtc agtgggggac cttccttgga gcagaggttt 540
   gaatcctatt acaactactg caatctcttc aactacattc ttaatgccga tggtcctgct 600
   ccccttgaac tacccaacca gtggctctgg gatattatcg atgagttcat ctaccagttt 660
   cagtcattca gtcagtaccg ctgtaagact gccaagaagt cagaggagga gattgacttt 720
   cttcgttcca atcccaaaat ctggaatgtt catagtgtcc tcaatgtcct tcattccctg 780
   gtagacaaat ccaacatcaa ccgacagttg gaggtataca caagcggagg tgaccctgag 840
   agtgtggctg gggagtatgg gcggcactcc ctctacaaaa tgcttggtta cttcagcctg 900
   gtcgggcttc tccgcctgca ctccctgtta ggagattact accaggccat caaggtgctg 960
   gagaacatcg aactgaacaa gaagagtatg tattcccgtg tgccagagtg ccaggtcacc 1020
   acatactatt atgttgggtt tgcatatttg atgatgcgtc gttaccagga tgccatccgg 1080
   gtcttcgcca acatcctcct ctacatccag aggaccaaga gcatgttcca gaggaccacg 1140
   tacaagtatg agatgattaa caagcagaat gagcagatgc atgcgctgct ggccattgcc 1200
   ctcacgatgt accccatgcg tattgatgag agcattcacc tccagctgcg ggagaaatat 1260
   ggggacaaga tgttgcgcat gcagaaaggt gacccacaag tctatgaaga acttttcagt 1320
   tactcctgcc ccaagttcct gtcgcctgta gtgcccaact atgataatgt gcaccccaac 1380
   taccacaaag agcccttcct gcagcagctg aaggtgtttt ctgatgaagt acagcagcag 1440
   gcccagcttt caaccatccg cagcttcctg aagctctaca ccaccatgcc tgtggccaag 1500
   ctggctggct tcctggacct cacagagcag gagttccgga tccagcttct tgtcttcaaa 1560
   cacaagatga agaacctcgt gtggaccagc ggtatctcag ccctggatgg tgaatttcag 1620
   tcagcctcag aggttgactt ctacattgat aaggacatga tccacatcgc ggacaccaag 1680
   gtcgccaggc gttatgggga tttcttcatc cgtcagatcc acaaatttga ggagcttaat 1740
   cgaaccctga agaagatggg acagagacct tgatgatatt cacacacatt caggaacctg 1800
   ttttgatgta ttataggcag gaagtgtttt tgctaccgtg aaacctttac ctagatcagc 1860
   catcagcctg tcaactcagt taacaagtta aggaccgaag tgtttcaagt ggatctcagt 1920
   aaaggatctt tggagccaga tttgtcgtct cattattgta ggagagaatt tgtgggttgt 1980
   ggcagtaata catttcccat gtgtcctgat gctttcagga tacatcagtt gttagtgttt 2040
   aaattgagtt atttttattt tgtgcttttg agatggagtc tcactctgtc t          2091
<210> 109
   <211> 6209
   <212> DNA
   <213> Homo sapiens
<220>
   <223> importin 7 (IPO7, Imp7), RAN-binding protein 7 (RANBP7)
<220>
   <221> CDS
   <222> (143)...(3259)
   <223> IPO7
<400> 109
   gttctatccg gggccttggc gcttctcttt cctttcgcgc cggttgccgc tgcggagcgc 60
   ggcgggtcca tgtgcgcagt gagtggcgct attcctggcc cagtagcacc cgagccccgg 120
   gtttgaccga gtccgcgctg cgatggaccc caacaccatt atcgaggccc tgcggggcac 180
   catggaccca gccctgcgtg aggccgcgga gcgccagctc aatgaagcac acaagtctct 240
   gaattttgtc tcaacactgc tccagattac tatgtcggaa cagctggatt tacctgtgag 300
   acaggcaggt gttatctatc tgaaaaatat gataacacag tattggcctg atcgagaaac 360
   agcaccaggg gatatatccc cttatactat tccagaagaa gatcgccatt gtattcgaga 420
   aaatattgta gaagccatta tccattctcc tgagctcatc agggtacagc ttactacatg 480
   cattcatcac atcatcaaac atgattatcc aagccgctgg actgccattg tggacaaaat 540
   tggcttttat cttcagtccg ataacagtgc ttgttggcta ggaattcttc tttgccttta 600
   tcagcttgtg aaaaattatg agtataaaaa accagaggag cggagtccat tggtagcagc 660
   aatgcagcat tttctgccag ttctaaagga tcgttttatc cagcttcttt ctgaccagtc 720
   tgatcagtct gtcctcatcc agaaacagat attcaagatc ttctatgctc ttgttcagta 780
   tacactacca ctggaactga taaaccaaca gaacctgaca gaatggatag aaattttaaa 840
   gactgttgtg aacagggatg tacctaatga aacacttcaa gttgaagaag atgatcgacc 900
   tgagttacca tggtggaaat gcaagaagtg ggccttacat attttagcaa gactttttga 960
   aagatatgga agccctggca atgtttccaa ggagtataat gaatttgctg aagtatttct 1020
   gaaggcattt gctgttggtg tccagcaagt tttattgaag gtgttatatc agtacaagga 1080
   gaagcaatat atggctcctc gagttttaca acagacatta aattatatta atcaaggagt 1140
   ttctcatgct ctcacctgga agaatctgaa gccccatata caaggcatta tccaagatgt 1200
   tatttttcca ttgatgtgct atacagatgc tgatgaggaa ctttggcaag aagaccctta 1260
   cgaatatata cgcatgaagt ttgatgtgtt tgaagatttc atttctccta ccactgctgc 1320
   ccagacactt ttgtttacag cctgtagtaa gaggaaagag gtactgcaaa agactatggg 1380
   attttgttac cagattctta cagaaccaaa tgctgaccct cgaaaaaaag atggagccct 1440
   gcatatgatt ggctctttag ctgaaatact tctgaagaaa aagatctata aagatcagat 1500
   ggaatacatg ttgcagaatc atgtattccc tctcttcagc agtgaactag gctacatgag 1560
   agcaagggct tgctgggtac ttcactattt ttgtgaagtg aagttcaaaa gtgatcagaa 1620
   ccttcaaaca gccttagagc taacaagaag atgtctgatt gatgatagag aaatgcctgt 1680
   gaaagtggaa gctgccattg cccttcaagt attgatcagc aatcaagaaa aagctaaaga 1740
   atatatcaca ccattcatca gacctgtaat gcaggctctt cttcacatta taagagaaac 1800
   agaaaatgat gaccttacca atgtaattca gaaaatgatc tgtgaatata gtgaagaagt 1860
   tactcctatt gcagtagaaa tgacacaaca tttggcaatg acatttaacc aagtaatcca 1920
   gacggggcca gatgaagaag gtagtgatga caaagcagtt actgctatgg gaattctgaa 1980
   tacaattgat acacttctta gtgtagttga agatcataaa gagataaccc aacagcttga 2040
   gggaatctgc ttacaggtca ttggtactgt tttacaacag catgtcttag aattctatga 2100
   ggagatcttc tctttagcgc acagtttgac atgtcaacaa gtgtctccac agatgtggca 2160
   gctactaccc cttgtatttg aagtctttca gcaagatggc tttgattact ttacagatat 2220
   gatgcccctc cttcataatt atgtaacagt tgatacagac acacttctgt ctgacaccaa 2280
   gtatcttgaa atgatataca gtatgtgcaa aaaggttctt acaggagttg caggagaaga 2340
   tgcagagtgt catgcagcaa aattgttaga ggtcatcatt ctgcagtgca aagggcgtgg 2400
   cattgaccag tgcattccct tattcgtgga agcagcctta gaaagactga caagagaggt 2460
   taagacaagt gaacttcgaa ctatgtgtct gcaagttgca attgcagctt tgtattataa 2520
   tccacaccta ctactcaata ccttagaaaa tcttcgcttc cctaataatg ttgaaccagt 2580
   tacaaatcat tttattacac agtggcttaa tgatgttgac tgtttcttgg ggcttcatga 2640
   cagaaagatg tgtgttctcg gactctgtgc tcttattgat atggaacaga taccccaagt 2700
   tttaaatcag gtttctggac agattttgcc ggcttttatc cttttattta acggattgaa 2760
   aagagcatat gcctgccatg cagaacatga gaatgacagt gatgatgatg atgaagctga 2820
   agatgatgat gaaaccgagg aactggggag tgatgaagat gatattgatg aagatgggca 2880
   agaatatttg gagattctgg ctaagcaggc tggtgaagat ggagatgatg aagattggga 2940
   agaagatgat gctgaagaga ctgctctgga aggctattcc acaatcattg atgatgaaga 3000
   taaccctgtt gatgagtatc agatatttaa agctatcttt caaactattc aaaatcgtaa 3060
   tcctgtgtgg tatcaggcac tgactcacgg tcttaatgaa gaacaaagaa aacagttaca 3120
   ggacatagca actctggctg atcaaagaag agcagcccat gaatccaaaa tgattgagaa 3180
   gcatggagga tacaaattca gtgctccagt tgtgccaagt tctttcaatt ttggaggccc 3240
   agcaccaggg atgaattgag ttatctcttt ctttcctgct gtgtgcttgt agtgaagagc 3300
   ttgtgttcct cctagtagtg gttccagaac tggttcatgt tatctattct aaactaataa 3360
   tcaatagatg gacaaaagaa acaacaaccc caggagatgg gacctgatca tgcaacctgg 3420
   cactggaaaa gaaatcagcg ggattttggg ggtggggggg gatgggaggt accttagagg 3480
   gagtattttc tttatttttt gaagaaagta agatcctgac tctgaagctt caaagtgaca 3540
   ctgtggaaat ctgaaacgag gggatgtcat gaaggcagct tttctttttc tgaggaaaaa 3600
   ataggcatgg gctacaggac tatttaaaat gtctcattta cagtataaaa ctcaaaggta 3660
   gatgtaattt ttacacctat gagtatttgt ccaatttctg tctcttcctc accattgggt 3720
   atctattctt tatatgtaaa taagataagg tcatctgata gccttattca gtcttcatca 3780
   ttttcatcat tgttcctatg tagattattg gacatttatt gtagcactac ataactgatt 3840
   ataaaaatct gtaaatgaat tagcactttc atattgaaac aagcctgcta gcctatgtat 3900
   aaaatagcaa aatgtttgct gtttataaaa agatgtaatg gggtgggggg caggggtaat 3960
   ttcaagttat taatttaaaa atgaactagc aattttgtac ctggtgactt tgtggtgcac 4020
   tcacctctga tagtgacttg aattcggtat gtaaaaaggg gttagtggta tttcattgct 4080
   gctaaaaatg acaactccct ctgtgtcctg tttttcttaa agctgtcagt gtacaagtgg 4140
   gtatttgaat accagacctt actgtaaaaa ataaaaaagg tggtatctag agcatgtaaa 4200
   ttggatataa agttctgctc ttaaagagtt gatctaagag tatggctaaa catctatata 4260
   tgcaatctat taaaagaact taattcggct attatgtctt gatttgattg cagttttttc 4320
   ctaattataa caaatttttc ctcattggcc tgtttttaat cctgtgccta gaaggagtac 4380
   aaaatgcaca ctttacaaaa ttgatattta acacttaccc actccccttt ccccatctct 4440
   tctaccgctc ttgttgatcg tggtatctga tcttgactag ataggctgaa ggcacatggt 4500
   tccctccaaa aaccactatt gataccacta caaaaacaag ccagcaaaaa gatactgtag 4560
   agaggttggc ttgcttccct ctcttcctaa ctgcatgttg aaaaataagc cgttattgat 4620
   cttaaacatc ggtcagatga gtcatacatt gggttatttt ttatatacat gtatacacaa 4680
   aatatttcaa attgaaagca acatcttaat ggattcaaaa ctattacaag ctgttgtcta 4740
   aaacaggtga gaaaaaaatt tataactgta aaaacaaatg cacatattga tatttaaaat 4800
   gcgtaattaa gaaaacccat tgttgttgtg tttttcttgt ataccaataa ttaagccact 4860
   actgttggca ctgtttggtt ttctatttta acactgaagg agtgaaagta tttcctatat 4920
   ttatgaattt actactaaaa tcttggcaaa aaaagaaaaa aattgtctaa cgtgtgtggg 4980
   tgaaaactgt taatcaagtg tttctactcc cccccgaaaa tcccctgaaa gttggacacc 5040
   aactgtatac cctaggttgc ttaaagggat ttcactatta tataaagtca ataaaaatga 5100
   agtagttgta tatatgcaac attgtgtaca gaggggaaat aatgaatagt attaaagaaa 5160
   cattctcgtc ttcctttacc tttaatcccc taatacctag tctacttttt aaattttcag 5220
   acttcactgc tttttgaatt cataattcta attttcacat tattgttaat ggaaaatcat 5280
   atctaataaa ggttttagtt attcccatgc acagtatgaa aattctcatt tgctgaggtt 5340
   ttgtttcaag aaaatgtatt ggcatgtctt tgagaacatg ttttattgtc tcctgtgtca 5400
   tataatccaa actaatctcc gtttacagac tttaacttga aattagacct tataattaaa 5460
   ctatttaaat agtgttcaaa tgatagtttc taatgcatca aatatatacc tcagttttca 5520
   tgatttcctt taacattata atttggtata gatcaagaat cttaacatgt atcagtttct 5580
   agatgaggct gcaggatttt tggaaaactt tttgaatgta tttacaatat tctcttgtaa 5640
   ttagctacat agggacttgt ctttttttct ttttacatac agcttttcct acagttttat 5700
   taccctgtaa ttttttttta gttgtagaag ttaattctga ttttgtgtgg atttcagtat 5760
   ttgtctttgt taatggcaca tattagcata aatcactttt gtaaatgtaa gctttctttt 5820
   tttttcttga aaaagccttt ctatttatca gtattaaata aaggaagtta atctgtttct 5880
   ctgcaggtaa taaaatagtg acacactgta ttaagatagt gactgctata ctcaactctg 5940
   gaagagacta gagtatagag catgagtggc aaaaccacag cccttgggcc atatgctgct 6000
   attcagtccc agatgtagcc cctgaagcaa gcataaagaa aaatgaatta aaaattaaat 6060
   taatatggaa agttaaaaaa tggattacat tagtatgact aaaccatgtc tttggcaaag 6120
   atctaacaca atgtcttaag tataataggt agtctctgtt tgtaaaataa atgacttaaa 6180
   tttaaaacat caaaaaaaaa aaaaaaaaa                                    6209
<210> 110
   <211> 4004
   <212> DNA
   <213> Homo sapiens
<220>
   <223> pyruvate carboxylase transcript variant 1, mitochondrial precursor (PC, PCB), pyruvic carboxylase
<220>
   <221> CDS
   <222> (95)...(3631)
   <223> PC
<400> 110
   gtcagtggag gcagcagcgg tagaggcggc ggcgaggact ggcgacggcg aggagatagt 60
   gtctgccttc tggagagctg accaaacact aaggatgctg aagttccgaa cagtccatgg 120
   gggcctgagg ctcctgggaa tccgccgaac ctccaccgcc cccgctgcct ccccaaatgt 180
   ccggcgcctg gagtataagc ccatcaagaa agtcatggtg gccaacagag gtgagattgc 240
   catccgtgtg ttccgggcct gcacggagct gggcatccgc accgtagcca tctactctga 300
   gcaggacacg ggccagatgc accggcagaa agcagatgaa gcctatctca tcggccgcgg 360
   cctggccccc gtgcaggcct acctgcacat cccagacatc atcaaggtgg ccaaggagaa 420
   caacgtagat gcagtgcacc ctggctacgg gttcctctct gagcgagcgg acttcgccca 480
   ggcctgccag gatgcagggg tccggtttat tgggccaagc ccagaagtgg tccgcaagat 540
   gggagacaag gtggaggccc gggccatcgc cattgctgcg ggtgttcccg ttgtccctgg 600
   cacagatgcc cccatcacgt ccctgcatga ggcccacgag ttctccaaca cctacggctt 660
   ccccatcatc ttcaaggcgg cctatggggg tggagggcgt ggcatgaggg tggtgcacag 720
   ctacgaggag ctggaggaga attacacccg ggcctactca gaggctctgg ccgcctttgg 780
   gaatggggcg ctgtttgtgg agaagttcat cgagaagcca cggcacatcg aggtgcagat 840
   cttgggggac cagtatggga acatcctgca cctgtacgag cgagactgct ccatccagcg 900
   gcggcaccag aaggtggtcg agattgcccc cgccgcccac ctggacccgc agcttcggac 960
   tcggctcacc agcgactctg tgaaactcgc taaacaggtg ggctacgaga acgcaggcac 1020
   cgtggagttc ctggtggaca ggcacggcaa gcactacttc atcgaggtca actcccgcct 1080
   gcaggtggag cacacggtca cagaggagat caccgacgta gacctggtcc atgctcagat 1140
   ccacgtggct gagggcagga gcctacccga cctgggcctg cggcaggaga acatccgcat 1200
   caacgggtgt gccatccagt gccgggtcac caccgaggac cccgcgcgca gcttccagcc 1260
   ggacaccggc cgcattgagg tgttccggag cggagagggc atgggcatcc gcctggataa 1320
   tgcttccgcc ttccaaggag ccgtcatctc gccccactac gactccctgc tggtcaaagt 1380
   cattgcccac ggcaaagacc accccacggc cgccaccaag atgagcaggg cccttgcgga 1440
   gttccgcgtc cgaggtgtga agaccaacat cgccttcctg cagaatgtgc tcaacaacca 1500
   gcagttcctg gcaggcactg tggacaccca gttcatcgac gagaacccag agctgttcca 1560
   gctgcggcct gcacagaacc gggcccaaaa gctgttgcac tacctcggcc atgtcatggt 1620
   aaacggtcca accaccccga ttcccgtcaa ggccagcccc agccccacgg accccgttgt 1680
   ccctgcagtg cccataggcc cgcccccggc tggtttcaga gacatcctgc tgcgagaggg 1740
   gcctgagggc tttgctcgag ctgtgcggaa ccacccgggg ctgctgctga tggacacgac 1800
   cttcagggac gcccaccagt cactgctggc cactcgtgtg cgcacccacg atctcaaaaa 1860
   gatcgccccc tatgttgccc acaacttcag caagctcttc agcatggaga actggggagg 1920
   agccacgttt gacgtcgcca tgcgcttcct gtatgagtgc ccctggcggc ggctgcagga 1980
   gctccgggag ctcatcccca acatcccttt ccagatgctg ctgcgggggg ccaatgctgt 2040
   gggctacacc aactacccag acaacgtggt cttcaagttc tgtgaagtgg ccaaagagaa 2100
   tggcatggat gtcttccgtg tgtttgactc cctcaactac ttgcccaaca tgctgctggg 2160
   catggaggcg gcaggaagtg ccggaggcgt ggtggaggct gccatctcat acacgggcga 2220
   cgtggccgac cccagccgca ccaagtactc actgcagtac tacatgggct tggccgaaga 2280
   gctggtgcga gctggcaccc acatcctgtg catcaaggac atggccgggc tgctgaagcc 2340
   cacggcctgc accatgctgg tcagctccct ccgggaccgc ttccccgacc tcccactgca 2400
   catccacacc cacgacacgt caggggcagg cgtggcagcc atgctggcct gtgcccaggc 2460
   tggagctgat gtggtggatg tggcagctga ttccatgtct gggatgactt cacagcccag 2520
   catgggggcc ctggtggcct gtaccagagg gactcccctg gacacagagg tgcccatgga 2580
   gcgcgtgttt gactacagtg agtactggga gggggctcgg ggactgtacg cggccttcga 2640
   ctgcacggcc accatgaagt ctggcaactc ggacgtgtat gaaaatgaga tcccaggggg 2700
   ccagtacacc aacctgcact tccaggccca cagcatgggg cttggctcca agttcaagga 2760
   ggtcaagaag gcctatgtgg aggccaacca gatgctgggc gatctcatca aggtgacgcc 2820
   ctcctccaag atcgtggggg acctggccca gtttatggtg cagaatggat tgagccgggc 2880
   agaggccgaa gctcaggcgg aagagctgtc ctttccccgc tccgtggtgg agttcctgca 2940
   gggctacatc ggtgtccccc atggggggtt ccccgaaccc tttcgctcta aggtactgaa 3000
   ggacctgcca agggtggagg ggcggcctgg agcctccctc cctcccctgg atctgcaggc 3060
   actggagaag gagctggtag accggcatgg ggaggaggtg acgccggaag atgtgctctc 3120
   agcagctatg taccccgatg tgtttgccca cttcaaggac ttcactgcca cctttggccc 3180
   cctggatagc ctgaatactc gcctcttcct gcagggaccc aagatcgcag aggagtttga 3240
   ggtggagctg gagcggggca agacgctgca catcaaagcc ctggccgtga gcgacctgaa 3300
   ccgggccggc cagaggcagg tcttctttga gctcaatggg cagctgcggt ccatcttggt 3360
   caaggacacc caggccatga aggagatgca cttccacccc aaggccctaa aggacgtgaa 3420
   gggccagatc ggggcgccca tgcctgggaa ggtgatagac atcaaagtgg tggcaggggc 3480
   caaggtggcc aagggccagc ccctgtgtgt gctcagtgcc atgaagatgg agactgtggt 3540
   gacctcaccc atggagggta ctgtccgcaa ggttcatgtg accaaggaca tgacactgga 3600
   aggtgacgac ctcatcctgg agatcgagtg atcttgcccc agaccggcag cctggccatc 3660
   cccaagcctt caacagaagc tgtgctgcca cggcaggccc aggccagcca gtgcccgagg 3720
   ccaggaaggc cgggccgtgg aggtcctgtc cacagctgga caggagagac accgcctgcg 3780
   gtggttcatt cctttcagcc atcgtccttt cctccggcgg acagctgctt acatgttcat 3840
   ctcttgccaa ataagggtcc cctcctcact ggagactaca agtggtgggt caggtggtcc 3900
   taggacccag gggaggttta ggggtcctat ctcctggggg aaggggagat ctaagatgtc 3960
   ccaggtcctg ggaagtttac tcaataaagc tggctttccc ctgc 4004
<210> 111
   <211> 894
   <212> DNA
   <213> Homo sapiens
<220>
   <223> ubiquitin-40S ribosomal protein S27a precursor, S27A, ubiquitin carboxyl extension protein 80 (CEP80, UBCEP80), ubiquitin C (UBC), epididymis luminal protein 112 (HEL112), UBCEP1
<220>
   <221> CDS
   <222> (148)...(618)
   <223> RPS27A
<400> 111
   ggcgttcttc cttttcgatc cgccatctgc ggtgggtgtc tgcacttcgg ctgctctcgg 60
   gttagcaccc tatggtgcct tctcttgtga tccctgacct aacctgtctc ttccttttcc 120
   tcaacctcag gtggagccgc caccaaaatg cagattttcg tgaaaaccct tacggggaag 180
   accatcaccc tcgaggttga accctcggat acgatagaaa atgtaaaggc caagatccag 240
   gataaggaag gaattcctcc tgatcagcag agactgatct ttgctggcaa gcagctggaa 300
   gatggacgta ctttgtctga ctacaatatt caaaaggagt ctactcttca tcttgtgttg 360
   agacttcgtg gtggtgctaa gaaaaggaag aagaagtctt acaccactcc caagaagaat 420
   aagcacaaga gaaagaaggt taagctggct gtcctgaaat attataaggt ggatgagaat 480
   ggcaaaatta gtcgccttcg tcgagagtgc ccttctgatg aatgtggtgc tggggtgttt 540
   atggcaagtc actttgacag acattattgt ggcaaatgtt gtctgactta ctgtttcaac 600
   aaaccagaag acaagtaact gtatgagtta ataaaagaca tgaactaaca tttattgttg 660
   ggttttattg cagtaaaaag aatggttttt aagcaccaaa ttgatggtca caccatttcc 720
   ttttagtagt gctactgcta tcgctgtgtg aatgttgcct ctggggatta tgtgacccag 780
   tggttctgta tacctgccag gtgccaacca cttgtaaagg tcttgatatt ttcaattctt 840
   agactaccta tactttggca gaagttatat ttaatgtaag ttgtctaaat ataa 894
<210> 112
   <211> 2282
   <212> DNA
   <213> Homo sapiens
<220>
   <223> secreted and transmembrane protein 1 precursor (SECTM1), type 1a transmembrane protein, K12
<220>
   <221> CDS
   <222> (399) ... (1145)
   <223> SECTM1
<400> 112
   aaacagcccc gcggggaacg cgcgccgcac gcgagctgga cccgcccagg cacgaactct 60
   cctggaaaaa tgctcccggc ggctttcctg cttcctttag cgtgaaccgc gggtgcggtg 120
   cctcccgtga aaataataaa ttcaccgtca cgcttgttgt gaacgcgggt ggttcccgaa 180
   acttggaggc ttcccgtaaa cccagctcct tcctcatctg ggaggtgggt cccgcgcggg 240
   tccgccgcct cctccctggc ccctccctct cgtgtctttc attttcctgg ggctccgggg 300
   cgcggagaag ctgcatccca gaggagcgcg tccaggagcg gacccgggag tgtttcaaga 360
   gccagtgaca aggaccaggg gcccaagtcc caccagccat gcagacctgc cccctggcat 420
   tccctggcca cgtttcccag gcccttggga ccctcctgtt tttggctgcc tccttgagtg 480
   ctcagaatga aggctgggac agccccatct gcacagaggg ggtagtctct gtgtcttggg 540
   gcgagaacac cgtcatgtcc tgcaacatct ccaacgcctt ctcccatgtc aacatcaagc 600
   tgcgtgccca cgggcaggag agcgccatct tcaatgaggt ggctccaggc tacttctccc 660
   gggacggctg gcagctccag gttcagggag gcgtggcaca gctggtgatc aaaggcgccc 720
   gggactccca tgctgggctg tacatgtggc acctcgtggg acaccagaga aataacagac 780
   aagtcacgct ggaggtttca ggtgcagaac cccagtccgc ccccgacact gggttctggc 840
   ctgtgccagc ggtggtcact gctgtcttca tcctcttggt cgctctggtc atgttcgcct 900
   ggtacaggtg ccgctgttcc cagcaacgcc gggagaagaa gttcttcctc ctagaacccc 960
   agatgaaggt cgcagccctc agagcgggag cccagcaggg cctgagcaga gcctccgctg 1020
   aactgtggac cccagactcc gagcccaccc caaggccgct ggcactggtg ttcaaaccct 1080
   caccacttgg agccctggag ctgctgtccc cccaaccctt gtttccatat gccgcagacc 1140
   catagccgcc tgcaaggcag agaggacaca ggagagccag ccctgagtgc cgaccttggg 1200
   tggcggggcc tgggtctctc gtcccacccg gagggcacag acaccggctt gcttggcagg 1260
   ctgggcctct gtgtcaccca ctcctgggtg cgtgcagacc cttcccctcc accccccagg 1320
   tcttccaagc tctgcttcct cagtttccaa aatggaacca cctcacctcc gcagcacccg 1380
   acttaccagg acgcatgccc ctccctctgc cctcatcaaa cccacagacc cggactccct 1440
   ttctgccacc ccaggctggt ccggccccag gtgtggggtc cgctctctcc actcccaggg 1500
   ctccgcgccc aagtgagggg gcccctgccg gagcctcaga cacactccag ttcagggctg 1560
   tggggggcct tggccacata cctgtccctt ggctatgagc aggctttggg ggcccttccg 1620
   cggcagcccc gggggccgag gtagggtcgg gggcttagag gctgggatgg ctcctggccc 1680
   caccgccagg gggcagcgca ggccgggctg ggaggcggcg gcggcggctc gggctggggg 1740
   gtcaggtgga cgccgccctc cggggctgga cgcgcatccc tcagtccctc ggccacccgg 1800
   gggtcgctcc ctcgtgccca ccgcacctgc cgagcctctt tggacccaga tctgttcatg 1860
   cttttgtctt cgtcactgcg gcggggccct ttgatgtctt catctgtatg gggtggaaaa 1920
   atcaccggga atcccccttc agttctttga aaaagttcca tgactcgaat atctgaaatg 1980
   aagaaaacaa accgactcac aaacctccaa gtagctccaa atgcaatttt taaaatggaa 2040
   aacaaaaatc tgaaagaaac gtctttagtg gctttaagcc ccaaaacgtc cctaaggcgt 2100
   cctcgagatg aagacggggg ggagccccca gccaggtgga gaccccgcag ggacgcggcg 2160
   gcgcccggtg accgaggcct cgcacagccg gccgccctga gggtcgggcc ggagccaggg 2220
   tccaagaggg gcgcgtttgt gtctcgggtt aaaataaggt tccgtccgcg tgctgggtca 2280
   ga                                                                2282
<210> 113
   <211> 593
   <212> DNA
   <213> Homo sapiens
<220>
   <223> 60S ribosomal protein L41 transcript variant 2, L41, homologue of yeast ribosomal protein YL41, HG12 protein
<220>
   <221> CDS
   <222> (199)...(276)
   <223> RPL41
<400> 113
   acccggcgct ccattaaata gccgtagacg gaacttcgcc tttctctcgg ccttagcgcc 60
   atttttttgg gtgagtgttt tttggttcct gcgttgggat tccgtgtaca atccatagac 120
   atctgacctc ggcacttagc atcatcacag caaactaact gtagcctttc tctctttccc 180
   tgtagaaacc tctgcgccat gagagccaag tggaggaaga agcgaatgcg caggctgaag 240
   cgcaaaagaa gaaagatgag gcagaggtcc aagtaaaccg ctagcttgtt gcaccgtgga 300
   ggccacagga gcagaaacat ggaatgccag acgctgggga tgctggtaca agttgtggga 360
   ctgcatgcta ctgtctagag cttgtctcaa tggatctaga acttcatcgc cctctgatcg 420
   ccgatcacct ctgagaccca ccttgctcat aaacaaaatg cccatgttgg tcctctgccc 480
   tggacctgtg acattctgga ctatttctgt gtttatttgt ggccgagtgt aacaaccata 540
   taataaatca cctcttccgc tgttttagct gaagaattaa atcaaaaaaa aaa        593
<210> 114
   <211> 5675
   <212> DNA
   <213> Homo sapiens
<220>
   <223> tuberous sclerosis 2 transcript variant 1 (TSC2), tuberin, LAM, TSC4
<220>
   <221> CDS
   <222> (107) ... (5530)
   <223> TSC2
<400> 114
   ccggcggcgt cccggggcca ggggggtgcg cctttctccg cgtcggggcg gcccggagcg 60
   cggtggcgcg gcgcgggagg ggttttctgg tgcgtcctgg tccaccatgg ccaaaccaac 120
   aagcaaagat tcaggcttga aggagaagtt taagattctg ttgggactgg gaacaccgag 180
   gccaaatccc aggtctgcag agggtaaaca gacggagttt atcatcaccg cggaaatact 240
   gagagaactg agcatggaat gtggcctcaa caatcgcatc cggatgatag ggcagatttg 300
   tgaagtcgca aaaaccaaga aatttgaaga gcacgcagtg gaagcactct ggaaggcggt 360
   cgcggatctg ttgcagccgg agcggccgct ggaggcccgg cacgcggtgc tggctctgct 420
   gaaggccatc gtgcaggggc agggcgagcg tttgggggtc ctcagagccc tcttctttaa 480
   ggtcatcaag gattaccctt ccaacgaaga ccttcacgaa aggctggagg ttttcaaggc 540
   cctcacagac aatgggagac acatcaccta cttggaggaa gagctggctg actttgtcct 600
   gcagtggatg gatgttggct tgtcctcgga attccttctg gtgctggtga acttggtcaa 660
   attcaatagc tgttacctcg acgagtacat cgcaaggatg gttcagatga tctgtctgct 720
   gtgcgtccgg accgcgtcct ctgtggacat agaggtctcc ctgcaggtgc tggacgccgt 780
   ggtctgctac aactgcctgc cggctgagag cctcccgctg ttcatcgtta ccctctgtcg 840
   caccatcaac gtcaaggagc tctgcgagcc ttgctggaag ctgatgcgga acctccttgg 900
   cacccacctg ggccacagcg ccatctacaa catgtgccac ctcatggagg acagagccta 960
   catggaggac gcgcccctgc tgagaggagc cgtgtttttt gtgggcatgg ctctctgggg 1020
   agcccaccgg ctctattctc tcaggaactc gccgacatct gtgttgccat cattttacca 1080
   ggccatggca tgtccgaacg aggtggtgtc ctatgagatc gtcctgtcca tcaccaggct 1140
   catcaagaag tataggaagg agctccaggt ggtggcgtgg gacattctgc tgaacatcat 1200
   cgaacggctc cttcagcagc tccagacctt ggacagcccg gagctcagga ccatcgtcca 1260
   tgacctgttg accacggtgg aggagctgtg tgaccagaac gagttccacg ggtctcagga 1320
   gagatacttt gaactggtgg agagatgtgc ggaccagagg cctgagtcct ccctcctgaa 1380
   cctgatctcc tatagagcgc agtccatcca cccggccaag gacggctgga ttcagaacct 1440
   gcaggcgctg atggagagat tcttcaggag cgagtcccga ggcgccgtgc gcatcaaggt 1500
   gctggacgtg ctgtcctttg tgctgctcat caacaggcag ttctatgagg aggagctgat 1560
   taactcagtg gtcatctcgc agctctccca catccccgag gataaagacc accaggtccg 1620
   aaagctggcc acccagttgc tggtggacct ggcagagggc tgccacacac accacttcaa 1680
   cagcctgctg gacatcatcg agaaggtgat ggcccgctcc ctctccccac ccccggagct 1740
   ggaagaaagg gatgtggccg catactcggc ctccttggag gatgtgaaga cagccgtcct 1800
   ggggcttctg gtcatccttc agaccaagct gtacaccctg cctgcaagcc acgccacgcg 1860
   tgtgtatgag atgctggtca gccacattca gctccactac aagcacagct acaccctgcc 1920
   aatcgcgagc agcatccggc tgcaggcctt tgacttcctg ttgctgctgc gggccgactc 1980
   actgcaccgc ctgggcctgc ccaacaagga tggagtcgtg cggttcagcc cctactgcgt 2040
   ctgcgactac atggagccag agagaggctc tgagaagaag accagcggcc ccctttctcc 2100
   tcccacaggg cctcctggcc cggcgcctgc aggccccgcc gtgcggctgg ggtccgtgcc 2160
   ctactccctg ctcttccgcg tcctgctgca gtgcttgaag caggagtctg actggaaggt 2220
   gctgaagctg gttctgggca ggctgcctga gtccctgcgc tataaagtgc tcatctttac 2280
   ttccccttgc agtgtggacc agctgtgctc tgctctctgc tccatgcttt caggcccaaa 2340
   gacactggag cggctccgag gcgccccaga aggcttctcc agaactgact tgcacctggc 2400
   cgtggttcca gtgctgacag cattaatctc ttaccataac tacctggaca aaaccaaaca 2460
   gcgcgagatg gtctactgcc tggagcaggg cctcatccac cgctgtgcca gccagtgcgt 2520
   cgtggccttg tccatctgca gcgtggagat gcctgacatc atcatcaagg cgctgcctgt 2580
   tctggtggtg aagctcacgc acatctcagc cacagccagc atggccgtcc cactgctgga 2640
   gttcctgtcc actctggcca ggctgccgca cctctacagg aactttgccg cggagcagta 2700
   tgccagtgtg ttcgccatct ccctgccgta caccaacccc tccaagttta atcagtacat 2760
   cgtgtgtctg gcccatcacg tcatagccat gtggttcatc aggtgccgcc tgcccttccg 2820
   gaaggatttt gtccctttca tcactaaggg cctgcggtcc aatgtcctct tgtcttttga 2880
   tgacaccccc gagaaggaca gcttcagggc ccggagtact agtctcaacg agagacccaa 2940
   gagtctgagg atagccagac cccccaaaca aggcttgaat aactctccac ccgtgaaaga 3000
   attcaaggag agctctgcag ccgaggcctt ccggtgccgc agcatcagtg tgtctgaaca 3060
   tgtggtccgc agcaggatac agacgtccct caccagtgcc agcttggggt ctgcagatga 3120
   gaactccgtg gcccaggctg acgatagcct gaaaaacctc cacctggagc tcacggaaac 3180
   ctgtctggac atgatggctc gatacgtctt ctccaacttc acggctgtcc cgaagaggtc 3240
   tcctgtgggc gagttcctcc tagcgggtgg caggaccaaa acctggctgg ttgggaacaa 3300
   gcttgtcact gtgacgacaa gcgtgggaac cgggacccgg tcgttactag gcctggactc 3360
   gggggagctg cagtccggcc cggagtcgag ctccagcccc ggggtgcatg tgagacagac 3420
   caaggaggcg ccggccaagc tggagtccca ggctgggcag caggtgtccc gtggggcccg 3480
   ggatcgggtc cgttccatgt cggggggcca tggtcttcga gttggcgccc tggacgtgcc 3540
   ggcctcccag ttcctgggca gtgccacttc tccaggacca cggactgcac cagccgcgaa 3600
   acctgagaag gcctcagctg gcacccgggt tcctgtgcag gagaagacga acctggcggc 3660
   ctatgtgccc ctgctgaccc agggctgggc ggagatcctg gtccggaggc ccacagggaa 3720
   caccagctgg ctgatgagcc tggagaaccc gctcagccct ttctcctcgg acatcaacaa 3780
   catgcccctg caggagctgt ctaacgccct catggcggct gagcgcttca aggagcaccg 3840
   ggacacagcc ctgtacaagt cactgtcggt gccggcagcc agcacggcca aaccccctcc 3900
   tctgcctcgc tccaacacag tggcctcttt ctcctccctg taccagtcca gctgccaagg 3960
   acagctgcac aggagcgttt cctgggcaga ctccgccgtg gtcatggagg agggaagtcc 4020
   gggcgaggtt cctgtgctgg tggagccccc agggttggag gacgttgagg cagcgctagg 4080
   catggacagg cgcacggatg cctacagcag gtcgtcctca gtctccagcc aggaggagaa 4140
   gtcgctccac gcggaggagc tggttggcag gggcatcccc atcgagcgag tcgtctcctc 4200
   ggagggtggc cggccctctg tggacctctc cttccagccc tcgcagcccc tgagcaagtc 4260
   cagctcctct cccgagctgc agactctgca ggacatcctc ggggaccctg gggacaaggc 4320
   cgacgtgggc cggctgagcc ctgaggttaa ggcccggtca cagtcaggga ccctggacgg 4380
   ggaaagtgct gcctggtcgg cctcgggcga agacagtcgg ggccagcccg agggtccctt 4440
   gccttccagc tccccccgct cgcccagtgg cctccggccc cgaggttaca ccatctccga 4500
   ctcggcccca tcacgcaggg gcaagagagt agagagggac gccttaaaga gcagagccac 4560
   agcctccaat gcagagaaag tgccaggcat caaccccagt ttcgtgttcc tgcagctcta 4620
   ccattccccc ttctttggcg acgagtcaaa caagccaatc ctgctgccca atgagtcaca 4680
   gtcctttgag cggtcggtgc agctcctcga ccagatccca tcatacgaca cccacaagat 4740
   cgccgtcctg tatgttggag aaggccagag caacagcgag ctcgccatcc tgtccaatga 4800
   gcatggctcc tacaggtaca cggagttcct gacgggcctg ggccggctca tcgagctgaa 4860
   ggactgccag ccggacaagg tgtacctggg aggcctggac gtgtgtggtg aggacggcca 4920
   gttcacctac tgctggcacg atgacatcat gcaagccgtc ttccacatcg ccaccctgat 4980
   gcccaccaag gacgtggaca agcaccgctg cgacaagaag cgccacctgg gcaacgactt 5040
   tgtgtccatt gtctacaatg actccggtga ggacttcaag cttggcacca tcaagggcca 5100
   gttcaacttt gtccacgtga tcgtcacccc gctggactac gagtgcaacc tggtgtccct 5160
   gcagtgcagg aaagacatgg agggccttgt ggacaccagc gtggccaaga tcgtgtctga 5220
   ccgcaacctg cccttcgtgg cccgccagat ggccctgcac gcaaatatgg cctcacaggt 5280
   gcatcatagc cgctccaacc ccaccgatat ctacccctcc aagtggattg cccggctccg 5340
   ccacatcaag cggctccgcc agcggatctg cgaggaagcc gcctactcca accccagcct 5400
   acctctggtg caccctccgt cccatagcaa agcccctgca cagactccag ccgagcccac 5460
   acctggctat gaggtgggcc agcggaagcg cctcatctcc tcggtggagg acttcaccga 5520
   gtttgtgtga ggccggggcc ctccctcctg cactggcctt ggacggtatt gcctgtcagt 5580
   gaaataaata aagtcctgac cccagtgcac agacatagag gcacagattg caaaaaaaaa 5640
   aaaaaaaaaa aaaaaaaaaa aaaaaaaaaa aaaaa 5675
<210> 115
   <211> 5436
   <212> DNA
   <213> Homo sapiens
<220>
   <223> collagen, type XVIII, alpha 1 transcript variant 3 (COL18A1), collagen alpha-1(XVIII) chain, endostatin, antiangiogenic agent, multi-functional protein MFP, KNO, KNO1, KS
<220>
   <221> CDS
   <222> (95)...(4114)
   <223> COL18A1
<400> 115
   ctcccagcgg cggcggctgc agcgcggcgg tccgagcggg tgcaccgcgg cggaggaggc 60
   agcatcccgc ggcgctgacg gtcctgggga gagcatggcg ccgaggtgcc cctggccatg 120
   gccgcggcgg cggcgcctcc tggacgtgct cgcgcccctg gtcctgctgc tcggggtccg 180
   cgcggcctcc gcggagccag agcgcatcag cgaggaggtg gggctgctgc agctccttgg 240
   ggaccccccg ccccagcagg tcacccagac ggatgacccc gacgtcgggc tggcctacgt 300
   ctttgggcca gatgccaaca gtggccaagt ggcccggtac cacttcccca gcctcttctt 360
   ccgtgacttc tcactgctgt tccacatccg gccagccaca gagggcccag gggtgctgtt 420
   cgccatcacg gactcggcgc aggccatggt cttgctgggc gtgaagctct ctggggtgca 480
   ggacgggcac caggacatct ccctgctcta cacagaacca ggtgcaggcc agacccacac 540
   agccgccagc ttccggctcc ccgccttcgt cggccagtgg acacacttag ccctcagtgt 600
   ggcaggtggc tttgtggccc tctacgtgga ctgtgaggag ttccagagaa tgccgcttgc 660
   tcggtcctca cggggcctgg agctggagcc tggcgccggg ctcttcgtgg ctcaggcggg 720
   gggagcggac cctgacaagt tccagggggt gatcgctgag ctgaaggtgc gcagggaccc 780
   ccaggtgagc cccatgcact gcctggacga ggaaggcgat gactcagatg gggcatccgg 840
   agactctggc agcgggctcg gggacgcccg ggagcttctc agggaggaga cgggcgcggc 900
   cctaaaaccc aggctccccg cgccaccccc cgtcaccacg ccacccttgg ctggaggcag 960
   cagcacggaa gattccagaa gtgaagaagt cgaggagcag accacggtgg cttcgttagg 1020
   agctcagaca cttcctggct cagattctgt ctccacgtgg gacgggagtg tccggacccc 1080
   tgggggccgc gtgaaagagg gcggcctgaa ggggcagaaa ggggagccag gtgttccggg 1140
   cccacctggc cgggcaggcc ccccaggatc cccatgccta cctggtcccc cgggtctccc 1200
   gtgcccagtg agtcccctgg gtcctgcagg cccagcgttg caaactgtcc ccggaccaca 1260
   aggaccccca gggcctccgg ggagggacgg cacccctgga agggacggcg agccgggcga 1320
   ccccggtgaa gacggaaagc cgggcgacac cgggccacaa ggcttccccg ggactccagg 1380
   ggacgtaggt cccaagggcg acaagggaga ccctggggtt ggagagagag ggcccccagg 1440
   accccaaggg cctccagggc ccccaggacc ctccttcaga cacgacaagc tgaccttcat 1500
   tgacatggag ggatctggct tcgggggcga tctggaggcc ctgcggggtc ctcgaggctt 1560
   ccctggacct cccggacccc ccggtgtccc aggcctgccc ggcgagccag gccgctttgg 1620
   ggtgaacagc tccgacgtcc caggacccgc cggccttcct ggtgtgcctg ggcgcgaggg 1680
   tccccccggg tttcctggcc tcccgggacc cccaggccct ccgggaagag aggggccccc 1740
   aggaaggact gggcagaaag gcagcctggg tgaagcaggc gccccaggac ataaggggag 1800
   caagggagcc cccggtcctg ctggtgctcg tggggagagc ggcctggcag gagcccccgg 1860
   acctgctgga ccaccaggcc cccctgggcc ccctgggccc ccaggaccag gactccccgc 1920
   tggatttgat gacatggaag gctccggggg gcccttctgg tcaacagccc gaagcgctga 1980
   tgggccacag ggacctcccg gcctgccggg acttaagggg gatcctggcg tgcctgggct 2040
   gccgggggcg aagggagaag ttggagcaga tggagtcccc gggttccccg gcctccctgg 2100
   cagagagggc attgctgggc cccaggggcc aaagggagac agaggcagcc ggggagaaaa 2160
   gggagatcca gggaaggacg gagtcgggca gccgggcctc cctggccccc ccggaccccc 2220
   gggacctgtg gtctacgtgt cggagcagga cggatccgtc ctgagcgtgc cgggacctga 2280
   gggccggccg ggtttcgcag gctttcccgg acctgcagga cccaagggca acctgggctc 2340
   taagggcgaa cgaggctccc cgggacccaa gggtgagaag ggtgaaccgg gcagcatctt 2400
   cagccccgac ggcggtgccc tgggccctgc ccagaaagga gccaagggag agccgggctt 2460
   ccgaggaccc ccgggtccat acggacggcc ggggtacaag ggagagattg gctttcctgg 2520
   acggccgggt cgccccggga tgaacggatt gaaaggagag aaaggggagc cgggagatgc 2580
   cagccttgga tttggcatga ggggaatgcc cggcccccca ggacctccag ggcccccagg 2640
   ccctccaggg actcctgttt acgacagcaa tgtgtttgct gagtccagcc gccccgggcc 2700
   tccaggattg ccagggaatc agggccctcc aggacccaag ggcgccaaag gagaagtggg 2760
   cccccccgga ccaccagggc agtttccgtt tgactttctt cagttggagg ctgaaatgaa 2820
   gggggagaag ggagaccgag gtgatgcagg acagaaaggc gaaagggggg agcccggggg 2880
   cggcggtttc ttcggctcca gcctgcccgg cccccccggc cccccaggcc ccccaggccc 2940
   acgtggctac cctgggattc caggtcccaa gggagagagc atccggggcc agcccggccc 3000
   acctggacct cagggacccc ccggcatcgg ctacgagggg cgccagggcc ctcccggccc 3060
   cccaggcccc ccagggcccc cttcatttcc tggccctcac aggcagacta tcagcgttcc 3120
   cggccctccg ggcccccctg ggccccctgg gccccctgga accatgggcg cctcctcagg 3180
   ggtgaggctc tgggctacac gccaggccat gctgggccag gtgcacgagg ttcccgaggg 3240
   ctggctcatc ttcgtggccg agcaggagga gctctacgtc cgcgtgcaga acgggttccg 3300
   gaaggtccag ctggaggccc ggacaccact cccacgaggg acggacaatg aagtggccgc 3360
   cttgcagccc cccgtggtgc agctgcacga cagcaacccc tacccgcggc gggagcaccc 3420
   ccaccccacc gcgcggccct ggcgggcaga tgacatcctg gccagccccc ctcgcctgcc 3480
   cgagccccag ccctaccccg gagccccgca ccacagctcc tacgtgcacc tgcggccggc 3540
   gcgacccaca agcccacccg cccacagcca ccgcgacttc cagccggtgc tccacctggt 3600
   tgcgctcaac agccccctgt caggcggcat gcggggcatc cgcggggccg acttccagtg 3660
   cttccagcag gcgcgggccg tggggctggc gggcaccttc cgcgccttcc tgtcctcgcg 3720
   cctgcaggac ctgtacagca tcgtgcgccg tgccgaccgc gcagccgtgc ccatcgtcaa 3780
   cctcaaggac gagctgctgt ttcccagctg ggaggctctg ttctcaggct ctgagggtcc 3840
   gctgaagccc ggggcacgca tcttctcctt tgacggcaag gacgtcctga ggcaccccac 3900
   ctggccccag aagagcgtgt ggcatggctc ggaccccaac gggcgcaggc tgaccgagag 3960
   ctactgtgag acgtggcgga cggaggctcc ctcggccacg ggccaggcct cctcgctgct 4020
   ggggggcagg ctcctggggc agagtgccgc gagctgccat cacgcctaca tcgtgctctg 4080
   cattgagaac agcttcatga ctgcctccaa gtagccaccg cctggatgcg gatggccgga 4140
   gaggaccggc ggctcggagg aagcccccac cgtgggcagg gagcggccgg ccagcccctg 4200
   gccccaggac ctggctgcca tactttcctg tatagttcac gtttcatgta atcctcaaga 4260
   aataaaagga agccaaagag tgtatttttt taaaagttta aaacagaagc ctgatgctga 4320
   cattcacctg ccccaactct cccctgacct gtgagcccag ctgggtcagg cagggtgcag 4380
   tatcatgccc tgtgcaacct cttggcctga tcagaccacg gctcgatttc tccaggattt 4440
   cctgctttgg gaagccgtgc tcgccccagc aggtgctgac ttcatctccc acctagcagc 4500
   accgttctgt gcacaaaacc cagacctgtt agcagacagg ccccgtgagg caatgggagc 4560
   tgaggccaca ctcagcacaa ggccatctgg gctcctccag ggtgtgtgct cgccctgcgg 4620
   tagatgggag ggaggctcag gtccctgggg ctagggggag ccccttctgc tcagctctgg 4680
   gccattctcc acagcaaccc caggctgaag caggttccca agctcagagg cgcactgtga 4740
   cccccagctc cggcctgtcc tccaacacca agcacagcag cctggggctg gcctcccaaa 4800
   tgagccatga gatgatacat ccaaagcaga cagctccacc ctggccgagt ccaagctggg 4860
   agattcaagg gacccatgag ttggggtctg gcagcctccc atccagggcc cccatctcat 4920
   gcccctggct gggacgtggc tcagccagca cttgtccagc tgagcgccag gatggaacac 4980
   ggccacatca aagaggctga ggctggcaca ggacatgcgg tagccagcac acagggcagt 5040
   gagggagggc tgtcatctgt gcactgccca tggacaggct ggctccagat gcagggcagt 5100
   cattggctgt ctcctaggaa acccatatcc ttaccctcct tgggactgaa ggggaacccc 5160
   ggggtgccca caggccgccc tgcgggtgaa caaagcagcc acgaggtgca acaaggtcct 5220
   ctgtcagtca cagccacccc tgagatccgg caacatcaac ccgagtcatt cgttctgtgg 5280
   agggacaagt ggactcaggg cagcgccagg ctgaccacag cacagccaac acgcacctgc 5340
   ctcaggactg cgacgaaacc ggtggggctg gttctgtaat tgtgtgtgat gtgaagccaa 5400
   ttcagacagg caaataaaag tgacctttta cactga                           5436
<210> 116
   <211> 3230
   <212> DNA
   <213> Homo sapiens
<220>
   <223> calcium binding protein P22 (CHP, CHP1, p22), calcineurin-like EF-hand protein 1, EF-hand calcium-binding domain-containing protein p22, calcineurin B-like protein, SLC9A1 binding protein (SLC9A1BP), Sid470p
<220>
   <221> CDS
   <222> (145)...(732)
   <223> CHP
<400> 116
   accacccctg ggttccctcc cgggtccgca gtggaaacac tgccctctcc cttcttgacc 60
   cctagccctt ccttccctcc ctccttccct cctgtcgccg tctcttctgg cgccgctgct 120
   cccggaggag ctcccggcac ggcgatgggt tctcgggcct ccacgttact gcgggacgaa 180
   gagctcgagg agatcaagaa ggagaccggc ttttcccaca gtcaaatcac tcgcctctac 240
   agccggttca ccagcctgga caaaggagag aatgggactc tcagccggga agatttccag 300
   aggattccag aacttgccat caacccactg ggggaccgga tcatcaatgc cttctttcca 360
   gagggagagg accaggtaaa cttccgtgga ttcatgcgaa ctttggctca tttccgcccc 420
   attgaggata atgaaaagag caaagatgtg aatggacccg aaccactcaa cagccgaagc 480
   aacaaactgc actttgcttt tcgactatat gatttggata aagatgaaaa gatctcccgt 540
   gatgagctgt tacaggtgct acgcatgatg gtcggagtaa atatctcaga tgagcagctg 600
   ggcagcatcg cagacaggac cattcaggag gctgatcagg atggggacag tgccatatct 660
   ttcacagaat ttgttaaggt tttggagaag gtggatgtag aacagaaaat gagcatccga 720
   tttcttcact aaaggagacc aaactgttcc ttgcggtcta gtatttaaga actggaactt 780
   gaaagtcctc cttctaccaa ctccacctcc accccctcat tccccttctc ccaaagtact 840
   actgctgttg catgacaacc ccaaatatgt tctgtcaaca caaacctgcc tttggtgtat 900
   aaacagggca ttacagaatg gtacacccta tatatttctg ttcagtatcc attcactagt 960
   tcttcattta taaatatcat cttccccatt ctgctgctga atgccacaca tccatccagt 1020
   ctgagaaagt gagagaggca atcatgccaa gaacaagcca gcaaagctct ttcaccagat 1080
   gtagactgta gccctgctgc cttccctcca gcgagtctgc cagcatgctt cttcatcctt 1140
   tttatatgtt ctttgcttcc tacttccctg tcttccaaca tactgttcac ttactctggc 1200
   agtctttctg cttttcatta agcctcaaaa tctcctctgt tctacttggc accacaagct 1260
   atgtcctata tatgtatttc tgacttggca ggatagttca ggggtctggc agtttttatt 1320
   taccttcatt attaaatggg cctctgggat gttgcctctt caggagcttt ttggtaatca 1380
   atacttctct cagaagtatg agaccatcct ctgcactctg ctctgtcatc aaaggctgct 1440
   gggtggagat accctttttg aaaggtggcc ttggtgagag gtatggagcc aagtcttcta 1500
   ggttgcttgc ccacatcact ctatctctgg cctctgattc tcaactttgt acctgtgtgg 1560
   ctcctcttgt tagtgcaatg ttgactgttg aaaaagcagc agtatgctta caggtttgct 1620
   tagtttgggg acaccgttac caccagaatg gctgctctga caatatgcct agggactttc 1680
   tcatggcttt tatttaataa ggaggctggg caccctataa agcctcatgc attcacacct 1740
   ttgcagcatg gtttatgcct cagtgttatg tgcactggaa tgttttccac ttcacatttc 1800
   caagtagaaa tattagtgtt acggaagtgc ctaatatccc agtccaaatt tttttttttt 1860
   tttttttttt tttttgagac agagtcttgc tctgtcaccc aggctggagt gcagtggtgc 1920
   gatcgctcac tgcaacctca gcctcctgga tttaagtgat tctcctgcct cagcctccca 1980
   agtagctggg attacaggtg tgcaccacca tgcccggcta attttttgta tttttagtgg 2040
   agacagggtt tcaccatgtt ggccaggctg gtctcgaact cctgacctcg tgatccgcct 2100
   gcctcagcct cccaaagtgc tgggattaca ggtgtgagcc accacgcctg gccccagtcc 2160
   aaaatattta aagattgttt ccttagtgtc ttgaagtttt gcacaaaatt cttttttttg 2220
   agatggagtc tcactctgtc acccaggctg gagtgcagtg gcgtgatctt ggctcactgc 2280
   aacctctgcc tcctgggttc aagcaattct cccacctcag cctcccaagt agctgggatt 2340
   acagacgtgt gccaccatac ctgggtaatt tttgcatttt tagtggagag ggagtttcac 2400
   catgttggcc aggttggtct tgaactcctg acctcaggtg atcctcctgc ctcggcctcc 2460
   caaagtgctg ggattacagg catgagccac cgtgctcagc cgcaaaattc tttatgaatt 2520
   ttacacttgg caaatgttaa tgacggaagc catagtctgc tcctaataca tgtccaaagc 2580
   attgactgtt gtgtcattag ctgcctggtt acattagctc cctggcttct tgtttagacc 2640
   actgctaatc ccttaaaaac aagaggtctg gcactagtag cacaacctaa ggtggcatta 2700
   cagatctttg agcgagccac agcaactttt ctgccaagtc agcttagttt agacttcagt 2760
   gaatcaggct attgctatcc taatgtatgt ctctatgagt gtatttagcc acacatctgc 2820
   ccttggttga ctttctgact cattgcttgc ttgcttgttt ccttgctttg gaaaactatt 2880
   gaagattgct aaaaaatacc actgcaaagt gatggaaaag ggtggagaac aggggagtag 2940
   ccaggctgga tggctcaaat ataaatgaat gaggaattct ttatgaagta tcagtcagat 3000
   tttatgatta agtgatgtaa tataggaatt atgtaaaagg gaagaatgtc tgatactgat 3060
   ctattagaga ggtactttag aggcttcttg attggcataa agttcctaag gttatagatt 3120
   ttcccccctt ttggctgtat agcaaagtgt tttaatccac ggttgtgcct tattgttcca 3180
   ttaaaattgt atcttcgatc catcaataaa tacttgtggt tgaaacaaaa            3230
<210> 117
   <211> 4511
   <212> DNA
   <213> Homo sapiens
<220>
   <223> phosphofurin acidic cluster sorting protein 1 (PACS1), cytosolic sorting protein PACS-1, MRD17
<220>
   <221> CDS
   <222> (135) ... (3026)
   <223> PACS1
<400> 117
   gcagctcgct ggctgctcgc gctcgggcag gcgggctgag gaggctgccg cgcccccgcc 60
   gccgccgccg cgggggaagc ctgggagcca gatcggcgtc gcctcggcct ccgtaacccc 120
   cgcctagccg ggccatggcg gaacgcggag gggcgggcgg tggtcccgga ggcgccgggg 180
   gcggcagcgg ccagcgggga tccggggtcg cccagtcccc tcagcagccg ccgccgcagc 240
   agcagcagca gcagccgccg cagcagccga cgccccccaa gctggcccag gccacctcgt 300
   cgtcctcgtc cacctcggcg gcggctgcct cctcctcgtc ctcgtctacc tccacctcca 360
   tggccgtggc ggtggcctcg ggctccgcgc ctcccggtgg cccggggcca ggccgcaccc 420
   ccgccccggt gcagatgaac ctgtacgcca cctgggaggt ggaccggagc tcgtccagct 480
   gcgtgcctag gctattcagc ttgaccctga agaaactcgt catgctaaaa gaaatggaca 540
   aagatcttaa ctcagtggtc atcgctgtga agctgcaggg ttcaaaaaga attcttcgct 600
   ccaacgagat cgtccttcca gctagtggac tggtggaaac agagctccaa ttaaccttct 660
   cccttcagta ccctcatttc cttaagcgag atgccaacaa gctgcagatc atgctgcaaa 720
   ggagaaaacg ttacaagaat cggaccatct tgggctataa gaccttggcc gtgggactca 780
   tcaacatggc agaggtgatg cagcatccta atgaaggcgc actggtgctt ggcctacaca 840
   gcaacgtgaa ggatgtctct gtgcctgtgg cagaaataaa gatctactcc ctgtccagcc 900
   aacccattga ccatgaagga atcaaatcca agctttctga tcgttctcct gatattgaca 960
   attattctga ggaagaggaa gagagtttct catcagaaca ggaaggcagt gatgatccat 1020
   tgcatgggca ggacttgttc tacgaagacg aagatctccg gaaagtgaag aagacccgga 1080
   ggaaactaac ctcaacctct gccatcacaa ggcaacctaa catcaaacag aagtttgtgg 1140
   ccctcctgaa gcggtttaaa gtttcagatg aggtgggctt tgggctggag catgtgtccc 1200
   gcgagcagat ccgggaagtg gaagaggact tggatgaatt gtatgacagt ctggagatgt 1260
   acaaccccag cgacagtggc cctgagatgg aggagacaga aagcatcctc agcacgccaa 1320
   agcccaagct caagcctttc tttgagggga tgtcgcagtc cagctcccag acggagattg 1380
   gcagcctcaa cagcaaaggc agcctcggaa aagacaccac cagccctatg gaattggctg 1440
   ctctagaaaa aattaaatct acttggatta aaaaccaaga tgacagcttg actgaaacag 1500
   acactctgga aatcactgac caggacatgt ttggagatgc cagcacgagt ctggttgtgc 1560
   cggagaaagt caaaactccc atgaagtcca gtaaaacgga tctccagggc tctgcctccc 1620
   ccagcaaagt ggagggggtg cacacacccc ggcagaagag gagcacgccc ctgaaggagc 1680
   ggcagctctc caagccccta agtgagagga ccaacagttc cgacagcgag cgctccccag 1740
   atctgggcca cagcacgcag attccaagaa aggtggtgta tgaccagctc aatcagatcc 1800
   tggtgtcaga tgcagccctc ccagaaaatg tcattctggt gaacaccact gactggcagg 1860
   gccagtatgt ggctgagctg ctccaggacc agcggaagcc tgtggtgtgc acctgctcca 1920
   ccgtggaggt ccaggccgtg ctgtccgccc tgctcacccg gatccagcgc tactgcaact 1980
   gcaactcttc catgccgagg ccagtgaagg tggctgctgt gggaggccag agctacctga 2040
   gctccatcct caggttcttt gtcaagtccc tggccaacaa gacctccgac tggcttggct 2100
   acatgcgctt cctcatcatc cccctcggtt ctcaccctgt ggccaaatac ttggggtcag 2160
   tcgacagtaa atacagtagt tccttcctgg attctggttg gagagatctg ttcagtcgct 2220
   cggagccacc agtgtcagag caactggacg tggcagggcg ggtgatgcag tacgtcaacg 2280
   gggcagccac gacacaccag cttcccgtgg ccgaagccat gctgacttgc cggcataagt 2340
   tccctgatga agactcctat cagaagttta ttcccttcat tggcgtggtg aaggtgggtc 2400
   tggttgaaga ctctccctcc acagcaggcg atggggacga ttctcctgtg gtcagcctta 2460
   ctgtgccctc cacatcacca ccctccagct cgggcctgag ccgagacgcc acggccaccc 2520
   ctccctcctc cccatctatg agcagcgccc tggccatcgt ggggagccct aatagcccat 2580
   atggggacgt gattggcctc caggtggact actggctggg ccaccccggg gagcggagga 2640
   gggaaggcga caagagggac gccagctcga agaacaccct caagagtgtc ttccgctcag 2700
   tgcaggtgtc ccgcctgccc catagtgggg aggcccagct ttctggcacc atggccatga 2760
   ctgtggtcac caaagaaaag aacaagaaag ttcccaccat cttcctgagc aagaaacccc 2820
   gagaaaagga ggtggattct aagagccagg tcattgaagg catcagccgc ctcatctgct 2880
   cagccaagca gcagcagact atgctgagag tgtccatcga tggggtcgag tggagtgaca 2940
   tcaagttctt ccagctggca gcccagtggc ccacccatgt caagcacttt ccagtgggac 3000
   tcttcagtgg cagcaaggcc acctgaggcc ctgtctccca gccactttcc ctcctggcac 3060
   tgccaccagc ctcaccgcct gcgggcaggg ggaggccagc aggcccgggc ccagcacccc 3120
   ttccctggca ccagggtctg cctctcactc gcccaggtcc cgaaggacac tgccacaggg 3180
   acgccttccc tcccctcccc tccagcccac ccctgcacag cccctcctcc ttcccgcttt 3240
   tccccttctc cctcctgctc caggcccaag gcgtgttggt tttgccttct ggtgcccata 3300
   gtcccctgga ctgagtcccc caggccttcc ttcacccgac ttccaaactc ttccttgtgg 3360
   tatcagtttc cttctcggaa atgagaaagc tggaatcctg gtccccagca ggagagccta 3420
   gtcctccccc agcccctcca gccaccaggg tgtcctctag gatgcagctg ccagatccac 3480
   tcactctgct gcctccagca ggacccaagg ccactttcaa ctcttatggg gttctccacc 3540
   tgccccagag cttctcaagg gagggtaagg gggcaccctg agcccacagg acccctactt 3600
   cacagctcac aggggcagga ggcagctccc ctgcctccag gaccctgttg ctatggtgac 3660
   acagcgtttc taggacagag gggcctccca gtctcccccc accacccgtg cacgacttcc 3720
   tcaccacccc caggttccct gcagatgtcg tgtgtgtcct gagtgtttct ttggttcttt 3780
   gcacgccaag tctcttggtt gtaccatgtg acacaccctg tgcactggtc gctgtcttcg 3840
   tggcttccac ccttgttaat gatgctcctg cctctgcctc ccagcccctc acccagcaca 3900
   gctctgcctg gacttggaga gatgggaggc agacccccac caccatacat gctgtctgtg 3960
   gcccctcaga cattctgttt catctcccat tcatctccct cctcccaccg tgtcagtttt 4020
   tctgcctttc cctgctctgt tcttccccct ccttaggccc cagcctgggc ccagacccat 4080
   cctcccagcc aggtttccct ccagcaggct ccttccctcc ctgtcacctc cctctcacca 4140
   acccggggtc tgagcccctc attcctgacc gtccgtgttc tcaggagtgg ttgaggacac 4200
   agggccccag cccagccctc tgcacccccc agcccggcca tctgcgcccc acagcccctt 4260
   tggagctttt ctcttgtcct ctcactcctt cccagaagtt tttgcacaga acttcatttt 4320
   gaaagtgttt ttctcattct ccatacctcc cccaagctct cctccagccc ttcccagggc 4380
   tcagccctgc tgtcctgagc gtctcctggg ccagagagag gagatggggg tgggagggac 4440
   tgagttgatg ttgggttttt cattcaataa attggtgatt tcttaccgac tgcaaaaaaa 4500
   aaaaaaaaaa a                                                      4511
<210> 118
   <211> 3585
   <212> DNA
   <213> Homo sapiens
<220>
   <223> general transcription factor IIIB (TF3B90, TFIIIB90), BRF1, RNA polymerase III transcription initiation factor 90 kDa subunit transcript variant 3, TATA box binding protein (TBP)-associated factor, RNA polymerase III, GTF3B subunit 2
<220>
   <221> CDS
   <222> (884) ... (2305)
   <223> BRF1
<400> 118
   gtggcatggg gcactggctt tgctgatgag gagaccagac ttcagaggcc gtgtcacctg 60
   gcctgggccg cacagctggt gcatgccaag ctcgcgtgca gacgaagctc cagaggcctg 120
   gctgtcagga gggttcctcc ccgaggacac acacgcaacc tcctggaccg ccaggcttta 180
   gacttttaaa acttccatat gcaggaaacc ccacaggacg cagccagcat ctcgggtcat 240
   gggtgcacct gtcaccgcag tcctgggcta aacagtgaac aggagctctg tgtctctgtg 300
   gtcgttacag ttgtagtgaa tgttccctgg gagagtcctc ttcagaagat atttgcggta 360
   ttttcaacaa aggactctcc aggatggagg agcaactaca agtcaggaag gaaaaggctg 420
   acaacccagt tttttaaaaa acgggcaaag gacgtgaaca gacaccatgc aatggcccag 480
   agtgcacaaa tcatcatttc tcccaggaat ggggtgcccg agcccccagg cggctgaact 540
   tgagacttgt cacgtagtgt tggtggaatg cagagcagct ggagatcacc tgctgcaggt 600
   gggtgcgtaa ctccgcacgg ccaagccgtt tggaaagctg tagcatatgc atgagagtcc 660
   ggaaagttcc gagagaaagt gtgtggatgc acccgccaag gacacaccca ccgaggccac 720
   agctgcgatt tcatagtgtg ggaccccact ggcaaagaca cgctggctgc tgtggccgtg 780
   gtttctgtcc cagccgtctg tttccataga aaaagacccg tgcctgtata ttccacgctt 840
   tgcgcacctg ctggaattcg gggagaagaa ccacgaggtg tccatgactg ccctgaggct 900
   cctacagagg atgaagcggg actggatgca cacaggccgg cgcccctcgg gcctctgcgg 960
   agcagcgctc ctggttgcag ccagaatgca tgacttcagg aggactgtga aggaggtcat 1020
   cagtgtggtc aaagtgtgtg agtccacgct gcggaagagg ctcacggaat ttgaagacac 1080
   ccccaccagt cagttgacca ttgatgagtt catgaagatc gacctggagg aggagtgcga 1140
   ccccccctcg tacacagctg ggcagaggaa gctgcggatg aagcagcttg aacaagtcct 1200
   gtcaaaaaaa ctggaggagg ttgaaggtga aatatccagt taccaggatg caattgagat 1260
   tgaactagaa aacagccggc caaaggccaa ggggggcctg gccagcctgg caaaagatgg 1320
   ctccaccgag gacaccgcgt ccagcttgtg tggcgaggag gacacagagg acgaggagct 1380
   ggaagccgcg gccagccacc tgaacaaaga cttataccgg gagctccttg gtggtgcccc 1440
   cggcagctcg gaagcagcag gaagccccga gtggggcggc agacctccgg ccctggggtc 1500
   cctgctggac cccctcccca ctgcagccag cctgggcatc tcagactcca tccgcgaatg 1560
   catctcctct cagagcagcg accccaaaga tgcttcagga gacggtgagc tggacctcag 1620
   tggcattgat gacctggaga ttgacaggta catcctgaat gagtcggaag cccgcgtgaa 1680
   ggccgagctg tggatgaggg agaacgccga gtacctgcgg gaacagaggg aaaaagaagc 1740
   aagaatagcg aaagagaagg agctcggcat ctacaaggaa cacaagccca agaagtcttg 1800
   caagcgacgg gagccaattc aggccagtac cgccagggag gccatcgaga agatgctgga 1860
   gcagaagaag atctccagca agatcaatta tagcgtgctc cggggcctca gcagcgccgg 1920
   cgggggcagt ccgcacaggg aggatgcaca gcccgagcat agcgccagtg ccaggaagct 1980
   gtcacgaagg aggacgccgg ccagcagaag tggggctgac cctgtgacca gtgtggggaa 2040
   aaggttgagg cctctggtgt ctacgcagcc agcaaagaag gtggccacgg gagaggcttt 2100
   gctcccaagc tctcccaccc tcggagctga gcctgccagg ccccaggcgg tgctggtgga 2160
   gagcgggccc gtgtcatacc acgccgacga ggaggctgac gaggaggagc ctgacgagga 2220
   ggacggggag ccctgcgtca gtgccctgca gatgatgggc agcaacgact atggctgtga 2280
   tggcgatgag gacgacggct actgaagtgt ggcctccagg caggtgatgt cctggcaggg 2340
   ggcctcgcgg gtctcctcag catcagacgg gcttccagga ccgcagcagg caggccccag 2400
   cgccgagact cctggtgaca ggtggcacct gtcccacagc cctcgtccca tgtggaactt 2460
   accattggga ttgtgtttct attcagcaag ggaaaccgga ccaagcgtct gcatgtgtgt 2520
   gatcagatgt gggccgggtg tgtgcagggc tgggtcccgc tgcctgccgt cgactcatcc 2580
   aaggaccctc caaggctggc agtgtggtgt tgctactatt aaggaaacag gcttggggca 2640
   gccccactgc tggtccaagt gtgtggaggg ctgagtgtgc tggccctgtg actcaggacc 2700
   agctctggag tctccagccc accctccgca ccgtcccctc ctgagcagca ctcggcgcca 2760
   gcagcctctg ccagagtgga agccagagcc ctgcaggtgt ccggcgcagc cgtgggagct 2820
   gaggatctgg cacttgagag gcagcagctc cttgaaggtc ctctgcctcc agctgtggcc 2880
   ctgcatccag atacctgcct cgtccgaggc agacaccccc acccctgcct cctccagacc 2940
   cccctccccg ctgcctgcac cgcctggagc agcatggggg tcagacccct gctccagggc 3000
   cacttgagtt gtgggcccag gagccctgcg gctgccggca ggtgaactga gtgcccgaca 3060
   gctgagaccg gcgcccaccc gtcctgagca tagctctgta ggcagtgcgg gcatagcctg 3120
   catagtgtcc tggcgctggg agttccccgt ggacagagcc agagggcagt ggcgctccct 3180
   gtcagagctg gatcaggccc cccatcgagg agggagggca gacggaggcc cgagagcctc 3240
   cccaggcctc ttcgtgggaa ggccccagta ccactcgtag gaggtctcag ctctggcatg 3300
   gctgccccgg atgtggccga gggggcttca ccctgtgtcc ttaggagggg gtggccttga 3360
   ggcagagccg tgcctcactg acccccaggg gcctcatcct ccccatggaa tgggctgtat 3420
   gtcctgcccc aacttggccc gcagcaggcc agacccccct acccccgccc agagctcagt 3480
   agccagcctg ggtcctgcca gggcttctcg agggcttggg ggaagaatag atttagtaaa 3540
   gcaggaagat ctgttgttac ttaacagaaa aaaaaaaaaa aaaaa                 3585
<210> 119
   <211> 2355
   <212> DNA
   <213> Homo sapiens
<220>
   <223> prostaglandin E synthase 2 transcript variant 1 (PTGES2), chromosome 9 open reading frame 15 (C9orf15), GATE-binding factor 1 (GBF-1, GBF1), gamma-interferon-activated transcriptional element-binding factor 1
<220>
   <221> CDS
   <222> (746)...(1879)
   <223> PTGES2
<400> 119
   ctctcgggcg ccgggcgggg ccatcccggg gctgtccgcg gagacgccta tgcggtggag 60
   gctccgggct tcagctaggg cgggggagcc cagcagaagg accgagcagc ctggcacccc 120
   actttgccat cctctccctg gaaatctcgg ggtcggcggg ccggccgctt cgcgtgggcg 180
   aaatcagaga cacgtggttt ccaaggcccc ttcgggttcg ggaaaatttt atggttcggg 240
   tcacagtagg aagcggacaa tgaggcggga gggcagagag aaccgcaaca cctggtgccg 300
   ggtcgggtcg tttccggggc tttcagtggc cggaagtcgc ggcgcctgta ctgactctag 360
   gaagggctgg agttgttttg aatgggcgcc cgtaagagag gtgggcaagt acgtgttaca 420
   gacggccacg ccgcccttta ggcggtcaag gtggggcgag gagacgttcg cccccctgca 480
   gtcggccggg tcactaccca agagcctttg gaggcggaag catggaacgg tctgcaaacg 540
   ttcccgagcg ggcctctgcg gctctggcgg gcgtttcgaa cttgggcgcc gggcacacgc 600
   ccagtcccga gagcgctgag ggttccctta gcgtcgccct caccccggcc aacccgcggg 660
   gcgccagagt cctggccctt taaacgccgc gcgtgcctcg gcgtcttcgt ttcgcgcgcc 720
   cgcccgcggc gccggcggag cgaacatgga cccggctgcg cgggtggtgc gggcgctgtg 780
   gcctggtggg tgcgccttgg cctggaggct gggaggccgc ccccagccgc tgctacccac 840
   gcagagccgg gctggcttcg cgggggcggc gggcggcccg agccccgtgg ctgcagctcg 900
   taaggggagc ccgcggctgc tgggagctgc ggcgctggcc ctggggggag ccctggggct 960
   gtaccacacg gcgcggtggc acctgcgcgc ccaggacctc cacgcagagc gctcagccgc 1020
   gcagctctcc ctgtccagcc gcctgcagct gaccctgtac cagtacaaga cgtgtccctt 1080
   ctgcagcaag gtccgagcct tcctcgactt ccatgccctg ccctaccagg tggtggaggt 1140
   gaaccctgtg cgcagggctg agatcaagtt ctcctcctac agaaaggtgc ccatcctggt 1200
   ggcccaggaa ggagaaagct cgcaacaact aaatgactcc tctgtcatca tcagcgccct 1260
   caagacctac ctggtgtcgg ggcagcccct ggaagagatc atcacctact acccagccat 1320
   gaaggctgtg aacgagcagg gcaaggaggt gaccgagttc ggcaataagt actggctcat 1380
   gctcaacgag aaggaggccc agcaagtgta tggtgggaag gaggccagga cggaggagat 1440
   gaagtggcgg cagtgggcgg acgactggct ggtgcacctg atctccccca atgtgtaccg 1500
   cacgcccacc gaggctctgg cgtcctttga ctacattgtc cgcgagggca agttcggagc 1560
   cgtggagggt gccgtggcca agtacatggg tgcagcggcc atgtacctca tcagcaagcg 1620
   actcaagagc aggcaccgcc tccaggacaa cgtgcgcgag gacctctatg aggctgctga 1680
   caagtgggtg gctgctgtgg gcaaggaccg gcccttcatg gggggccaga agccgaatct 1740
   cgctgatttg gcggtgtatg gcgtgctgcg tgtgatggag gggctggatg cgttcgatga 1800
   cctgatgcag cacacgcaca tccagccctg gtacctgcgg gtggagaggg ccatcaccga 1860
   ggcctcccca gcgcactgaa tgtccccgcg cagagcagag ggaaggcagc ggaagacgcc 1920
   agctgccagg gcctggggcc actgggccag cgcctggcga tactggttgg gggcaggatc 1980
   attctgcccc ttgtccacgc acccccacca gccctctcgc ttctaacaca gggcacctgc 2040
   tggggctcag ggatgttagg gacgagttcc agccctgcca ctgccctggg gcgacccctc 2100
   cctgtccctg cctccctgct ctgccgcccc tcttcctgga ccctcagtgg ctgtcccatg 2160
   gctacatcct gtgggtgggg gccctcgaca ggacagcagg acggtttgtt ttcagtggaa 2220
   tcccatccct gggttcccct ggttcccact cttcccaagc ctcccgggac tgggacatgt 2280
   ttgcaataaa ggaaaggttt gtggcgcctg tcatggcagg catctcatgg aaaaaaaaaa 2340
   aaaaaaaaaa aaaaa                                                  2355
<210> 120
   <211> 602
   <212> DNA
   <213> Homo sapiens
<220>
   <223> hypothetical protein LOC391356, chromosome 2 open reading frame 79 (C2orf79), peptidyl-tRNA hydrolase domain containing 1 (PTRHD1)
<220>
   <221> CDS
   <222> (6)...(428)
   <223> C2orf79
<400> 120
   ccaagatgca ccggggagta ggtccggcct ttcgggtggt caggaagatg gcggcctctg 60
   gggcggagcc gcaggtcctg gtacaatact tggtgttacg aaaggatcta tcacaagctc 120
   cgttctcctg gccggcgggc gcactggtag cgcaggcttg tcacgcggcc accgcggcct 180
   tgcacactca ccgcgaccac ccgcacacag ccgcttacct ccaagagctg gggcgcatgc 240
   gcaaagtggt cctcgaggcc ccagatgaga ccaccctaaa ggagctggcc gagaccctgc 300
   aacagaagaa cattgaccac atgctgtggc ttgagcaacc agagaatatc gccacttgta 360
   ttgctctccg gccctacccc aaggaagaag tgggccagta tttgaagaag ttccgattgt 420
   tcaagtaact gctgctttga tgtgtttgaa tacgcaggcc acccattcca aagcatcatg 480
   tgttccttgc agtgtcagct tgctcccgtc tttcagttgt gacaatttct tgagggttaa 540
   gcacatgttc atattaaagt tgtcattaat aaaaaaaaaa aaaaaaaaaa aaaaaaaaaa 600
   aa                                                                602
<210> 121
   <211> 2487
   <212> DNA
   <213> Homo sapiens
<220>
   <223> phosphoglucomutase 1 transcript variant 1 (PGM1), glucose phosphomutase 1, CDG1T, GSD14
<220>
   <221> CDS
   <222> (214)...(1902)
   <223> PGM1
<400> 121
   cctttcccct cccgccggac ctgccaggag gtgggctggc gcggagggag ggccctgtcc 60
   cctgtccctt taaggaggag ggccaaacgc cggcctagag tgcggcgtag cccccacccg 120
   ccgtgccctc accccagagc agctgcagcc tcagccggcc gcccctccgc cagccaagtc 180
   cgccgctctg acccccggca gcaagtcgcc accatggtga agatcgtgac agttaagacc 240
   caggcgtacc aggaccagaa gccgggcacg agcgggctgc ggaagcgggt gaaggtgttc 300
   cagagcagcg ccaactacgc ggagaacttc atccagagta tcatctccac cgtggagccg 360
   gcgcagcggc aggaggccac gctggtggtg ggcggggacg gccggttcta catgaaggag 420
   gccatccagc tcatcgctcg catcgctgcc gccaacggga tcggtcgctt ggttatcgga 480
   cagaatggaa tcctctccac ccctgctgta tcctgcatca ttagaaaaat caaagccatt 540
   ggtgggatca ttctgacagc cagtcacaac ccagggggcc ccaatggaga ttttggaatc 600
   aaattcaata tttctaatgg aggtcctgct ccagaagcaa taactgataa aattttccaa 660
   atcagcaaga caattgaaga atatgcagtt tgccctgacc tgaaagtaga ccttggtgtt 720
   ctgggaaagc agcagtttga cttggaaaat aagttcaaac ccttcacagt ggaaattgtg 780
   gattcggtag aagcttatgc tacaatgctg agaagcatct ttgatttcag tgcactgaaa 840
   gaactacttt ctgggccaaa ccgactgaag atccgtattg atgctatgca tggagttgtg 900
   ggaccgtatg taaagaagat cctctgtgaa gaactcggtg cccctgcgaa ctcggcagtt 960
   aactgcgttc ctctggagga ctttggaggc caccaccctg accccaacct cacctatgca 1020
   gctgacctgg tggagaccat gaagtcagga gagcatgatt ttggggctgc ctttgatgga 1080
   gatggggatc gaaacatgat tctgggcaag catgggttct ttgtgaaccc ttcagactct 1140
   gtggctgtca ttgctgccaa catcttcagc attccgtatt tccagcagac tggggtccgc 1200
   ggctttgcac ggagcatgcc cacgagtggt gctctggacc gggtggctag tgctacaaag 1260
   attgctttgt atgagacccc aactggctgg aagttttttg ggaatttgat ggacgcgagc 1320
   aaactgtccc tttgtgggga ggagagcttc gggaccggtt ctgaccacat ccgtgagaaa 1380
   gatggactgt gggctgtcct tgcctggctc tccatcctag ccacccgcaa gcagagtgtg 1440
   gaggacattc tcaaagatca ttggcaaaag tatggccgga atttcttcac caggtatgat 1500
   tacgaggagg tggaagctga gggcgcaaac aaaatgatga aggacttgga ggccctgatg 1560
   tttgatcgct cctttgtggg gaagcagttc tcagcaaatg acaaagttta cactgtggag 1620
   aaggccgata actttgaata cagcgaccca gtggatggaa gcatttcaag aaatcagggc 1680
   ttgcgcctca ttttcacaga tggttctcga atcgtcttcc gactgagcgg cactgggagt 1740
   gccggggcca ccattcggct gtacatcgat agctatgaga aggacgttgc caagattaac 1800
   caggaccccc aggtcatgtt ggcccccctt atttccattg ctctgaaagt gtcccagctg 1860
   caggagagga cgggacgcac tgcacccact gtcatcacct aagaagacag gcctgatgtg 1920
   gtacgtccct ccacccccgg acccatccaa gtcatctgat tgaagagcat gacagaaaca 1980
   aaatgtattc accaagcatt ttaggatttg actttttcac taaccagttg acgagcagtg 2040
   catttacaag gcactgccaa acaagatgcc cttgggagct gtgagggaaa gaggacctgc 2100
   gggcttagat caatctcaat tccttttcat gccctcctgc attgctgctg cgtgggtatt 2160
   tgtctcctta gccatcaggt acagtttaca ctacaatgta agctataggt ggagcatcag 2220
   cagtgagtga ggccattctt catccttagg atgtggcaat gaaatgatgg tgcaagttcc 2280
   tttctctttt gtgaatcttt ccccccattt cctgtttaca tgtaacccaa caaaatgcaa 2340
   tttctagtgc cttctgtcca atcagttctt tcctctgagt gagacgtact tggctacaga 2400
   tttctgcctt gttttgcgac attgtcccat tcacacagat attttgggat aataaaggaa 2460
   aataagctac aaaaaaaaaa aaaaaaa                                     2487
<210> 122
   <211> 2873
   <212> DNA
   <213> Homo sapiens
<220>
   <223> solute carrier family 19 (folate transporter), member 1 transcript variant 1 (SLC19A1), reduced folate carrier protein (RFC, REFC, RFC1), folate transporter 1 (FOLT), placental folate transporter, intestinal folate carrier 1 (IFC-1, IFC1)
<220>
   <221> CDS
   <222> (154)...(1929)
   <223> SLC19A1
<400> 122
   agccgcgcgc ccgccgcgcc gccttgtggg cgctgtagtc ccggagtccg cgtgcgcggg 60
   gccgggtccg ggagccccag ggcagccgcc ccgccgagtc gcaggcacag cgtcaccttc 120
   gtcccctccg gagctgcacg tggcctgagc aggatggtgc cctccagccc agcggtggag 180
   aagcaggtgc ccgtggaacc tgggcctgac cccgagctcc ggtcctggcg gcacctcgtg 240
   tgctaccttt gcttctacgg cttcatggcg cagatacggc caggggagag cttcatcacc 300
   ccctacctcc tggggcccga caagaacttc acgcgggagc aggtcacgaa cgagatcacg 360
   ccggtgctgt cgtactccta cctggccgtg ctggtgcccg tgttcctgct caccgactac 420
   ctgcgctaca cgccggtgct gctgctgcag gggctcagct tcgtgtcggt gtggctgctg 480
   ctgctgctgg gccactcggt ggcgcacatg cagctcatgg agctcttcta cagcgtcacc 540
   atggccgcgc gcatcgccta ttcctcctac atcttctctc tcgtgcggcc cgcgcgctac 600
   cagcgtgtgg ccggctactc gcgcgctgcg gtgctgctgg gcgtgttcac cagctccgtg 660
   ctgggccagc tgctggtcac tgtgggccga gtctccttct ccacgctcaa ctacatctcg 720
   ctggccttcc tcaccttcag cgtggtcctc gccctcttcc tgaagcgccc caagcgcagc 780
   ctcttcttca accgcgacga ccgggggcgg tgcgaaacct cggcttcgga gctggagcgc 840
   atgaatcctg gcccaggcgg gaagctggga cacgccctgc gggtggcctg tggggactca 900
   gtgctggcgc ggatgctgcg ggagctgggg gacagcctgc ggcggccgca gctgcgcctg 960
   tggtccctct ggtgggtctt caactcggcc ggctactacc tggtggtcta ctacgtgcac 1020
   atcctgtgga acgaggtgga ccccaccacc aacagtgcgc gggtctacaa cggcgcggca 1080
   gatgctgcct ccacgctgct gggcgccatc acgtccttcg ccgcgggctt cgtgaagatc 1140
   cgctgggcgc gctggtccaa gctgctcatc gcgggcgtca cggccacgca ggcggggctg 1200
   gtcttccttc tggcgcacac gcgccacccg agcagcatct ggctgtgcta tgcggccttc 1260
   gtgctgttcc gcggctccta ccagttcctc gtgcccatcg ccacctttca gattgcatct 1320
   tctctgtcta aagagctctg tgccctggtc ttcggggtca acacgttctt tgccaccatc 1380
   gtcaagacca tcatcacttt cattgtctcg gacgtgcggg gcctgggcct cccggtccgc 1440
   aagcagttcc agttatactc cgtgtacttc ctgatcctgt ccatcatcta cttcttgggg 1500
   gccatgctgg atggcctgcg gcactgccag cggggccacc acccgcggca gcccccggcc 1560
   cagggcctga ggagtgccgc ggaggagaag gcagcacagg cactgagcgt gcaggacaag 1620
   ggcctcggag gcctgcagcc agcccagagc ccgccgcttt ccccagaaga cagcctgggg 1680
   gctgtggggc cagcctccct ggagcagaga cagagcgacc catacctggc ccaggccccg 1740
   gccccgcagg cagctgaatt cctgagccca gtgacaaccc cttccccctg cactctgtgc 1800
   tccgcccaag cctcaggccc tgaggctgca gatgagactt gtccccagct ggctgtccat 1860
   cctcctggtg tcagcaagct gggtttgcag tgtcttccaa gcgacggtgt tcagaatgtg 1920
   aaccagtgac tctcgggcgc ccctgtggta actttgcagg cggccctcag tgcatcccca 1980
   cgacccctgc ctcgagggcc gcctgcctta gcaatggggg cctccgctta tcctgctagc 2040
   aggcccccta ggattccccc tgccctgtgc cgcactctgg cggtggccac agcgtgctgg 2100
   cgacactcag ggcagctgcc tggccatgct gtccctgcac tgtgccccgc gggctttgtt 2160
   gctggaagag gtgggtggtg ggcttctgcg tccaccaggc ctcactggct catgcccctt 2220
   ggggggcttg agacaaatcc tttctgcccc ccagggctag tgaagtggcc tcttggatac 2280
   cagctcaggg gacactggcc ccacaggagt tgtgagccct ctagggcagg gtgggagccg 2340
   ggaccctcag gtgtagctga gctgtgacat tgctggtcat ccttggtgct cttgcttttt 2400
   tgaaagatgc tttttttttt tttaactgac gtagaatgaa gaactgcatg tggcttctct 2460
   gtctctgtgg aaaagccatc tcaggttggc ggcagacaca ttgtcatcag aggggagcag 2520
   cggctctggt cctcggagct ggttcctctc tcccacccta agggcagccc tccatggtcc 2580
   tgtctgtcct tctgaagtgt gtccatcctg acctgcgggt cctcagctgc tcccacactt 2640
   gtgccagccc ggaggggact ggtcccggtc accgcggacg tgctggcctt ggtatgtgcc 2700
   aggcttgcct gggctgggca gccttggggg ggctgccttt gtggtgggcg ctggggaagt 2760
   acgtcccagc ggcctcaggg tctaaggagc gctagtgcct tgcccacagg tgcgggacca 2820
   tctgatgtga tgtgaatact cttcccacat acattaaaca cacttaagtg aga 2873
<210> 123
   <211> 5748
   <212> DNA
   <213> Homo sapiens
<220>
   <223> CD44 antigen transcript variant 1, hematopoietic cell E- and L-selectin ligand (HCELL), chondroitin sulfate proteoglycan 8 (CSPG8), GP90 lymphocyte homing/adhesion receptor, epican, Hermes antigen, extracellular matrix receptor III (ECMR-III)
<220>
   <221> CDS
   <222> (435) ... (2663)
   <223> CD44
<400> 123
   gagaagaaag ccagtgcgtc tctgggcgca ggggccagtg gggctcggag gcacaggcac 60
   cccgcgacac tccaggttcc ccgacccacg tccctggcag ccccgattat ttacagcctc 120
   agcagagcac ggggcggggg cagaggggcc cgcccgggag ggctgctact tcttaaaacc 180
   tctgcgggct gcttagtcac agcccccctt gcttgggtgt gtccttcgct cgctccctcc 240
   ctccgtctta ggtcactgtt ttcaacctcg aataaaaact gcagccaact tccgaggcag 300
   cctcattgcc cagcggaccc cagcctctgc caggttcggt ccgccatcct cgtcccgtcc 360
   tccgccggcc cctgccccgc gcccagggat cctccagctc ctttcgcccg cgccctccgt 420
   tcgctccgga caccatggac aagttttggt ggcacgcagc ctggggactc tgcctcgtgc 480
   cgctgagcct ggcgcagatc gatttgaata taacctgccg ctttgcaggt gtattccacg 540
   tggagaaaaa tggtcgctac agcatctctc ggacggaggc cgctgacctc tgcaaggctt 600
   tcaatagcac cttgcccaca atggcccaga tggagaaagc tctgagcatc ggatttgaga 660
   cctgcaggta tgggttcata gaagggcacg tggtgattcc ccggatccac cccaactcca 720
   tctgtgcagc aaacaacaca ggggtgtaca tcctcacatc caacacctcc cagtatgaca 780
   catattgctt caatgcttca gctccacctg aagaagattg tacatcagtc acagacctgc 840
   ccaatgcctt tgatggacca attaccataa ctattgttaa ccgtgatggc acccgctatg 900
   tccagaaagg agaatacaga acgaatcctg aagacatcta ccccagcaac cctactgatg 960
   atgacgtgag cagcggctcc tccagtgaaa ggagcagcac ttcaggaggt tacatctttt 1020
   acaccttttc tactgtacac cccatcccag acgaagacag tccctggatc accgacagca 1080
   cagacagaat ccctgctacc actttgatga gcactagtgc tacagcaact gagacagcaa 1140
   ccaagaggca agaaacctgg gattggtttt catggttgtt tctaccatca gagtcaaaga 1200
   atcatcttca cacaacaaca caaatggctg gtacgtcttc aaataccatc tcagcaggct 1260
   gggagccaaa tgaagaaaat gaagatgaaa gagacagaca cctcagtttt tctggatcag 1320
   gcattgatga tgatgaagat tttatctcca gcaccatttc aaccacacca cgggcttttg 1380
   accacacaaa acagaaccag gactggaccc agtggaaccc aagccattca aatccggaag 1440
   tgctacttca gacaaccaca aggatgactg atgtagacag aaatggcacc actgcttatg 1500
   aaggaaactg gaacccagaa gcacaccctc ccctcattca ccatgagcat catgaggaag 1560
   aagagacccc acattctaca agcacaatcc aggcaactcc tagtagtaca acggaagaaa 1620
   cagctaccca gaaggaacag tggtttggca acagatggca tgagggatat cgccaaacac 1680
   ccaaagaaga ctcccattcg acaacaggga cagctgcagc ctcagctcat accagccatc 1740
   caatgcaagg aaggacaaca ccaagcccag aggacagttc ctggactgat ttcttcaacc 1800
   caatctcaca ccccatggga cgaggtcatc aagcaggaag aaggatggat atggactcca 1860
   gtcatagtat aacgcttcag cctactgcaa atccaaacac aggtttggtg gaagatttgg 1920
   acaggacagg acctctttca atgacaacgc agcagagtaa ttctcagagc ttctctacat 1980
   cacatgaagg cttggaagaa gataaagacc atccaacaac ttctactctg acatcaagca 2040
   ataggaatga tgtcacaggt ggaagaagag acccaaatca ttctgaaggc tcaactactt 2100
   tactggaagg ttatacctct cattacccac acacgaagga aagcaggacc ttcatcccag 2160
   tgacctcagc taagactggg tcctttggag ttactgcagt tactgttgga gattccaact 2220
   ctaatgtcaa tcgttcctta tcaggagacc aagacacatt ccaccccagt ggggggtccc 2280
   ataccactca tggatctgaa tcagatggac actcacatgg gagtcaagaa ggtggagcaa 2340
   acacaacctc tggtcctata aggacacccc aaattccaga atggctgatc atcttggcat 2400
   ccctcttggc cttggctttg attcttgcag tttgcattgc agtcaacagt cgaagaaggt 2460
   gtgggcagaa gaaaaagcta gtgatcaaca gtggcaatgg agctgtggag gacagaaagc 2520
   caagtggact caacggagag gccagcaagt ctcaggaaat ggtgcatttg gtgaacaagg 2580
   agtcgtcaga aactccagac cagtttatga cagctgatga gacaaggaac ctgcagaatg 2640
   tggacatgaa gattggggtg taacacctac accattatct tggaaagaaa caaccgttgg 2700
   aaacataacc attacaggga gctgggacac ttaacagatg caatgtgcta ctgattgttt 2760
   cattgcgaat cttttttagc ataaaatttt ctactctttt tgttttttgt gttttgttct 2820
   ttaaagtcag gtccaatttg taaaaacagc attgctttct gaaattaggg cccaattaat 2880
   aatcagcaag aatttgatcg ttccagttcc cacttggagg cctttcatcc ctcgggtgtg 2940
   ctatggatgg cttctaacaa aaactacaca tatgtattcc tgatcgccaa cctttccccc 3000
   accagctaag gacatttccc agggttaata gggcctggtc cctgggagga aatttgaatg 3060
   ggtccatttt gcccttccat agcctaatcc ctgggcattg ctttccactg aggttggggg 3120
   ttggggtgta ctagttacac atcttcaaca gaccccctct agaaattttt cagatgcttc 3180
   tgggagacac ccaaagggtg aagctattta tctgtagtaa actatttatc tgtgtttttg 3240
   aaatattaaa ccctggatca gtcctttgat cagtataatt ttttaaagtt actttgtcag 3300
   aggcacaaaa gggtttaaac tgattcataa taaatatctg tacttcttcg atcttcacct 3360
   tttgtgctgt gattcttcag tttctaaacc agcactgtct gggtccctac aatgtatcag 3420
   gaagagctga gaatggtaag gagactcttc taagtcttca tctcagagac cctgagttcc 3480
   cactcagacc cactcagcca aatctcatgg aagaccaagg agggcagcac tgtttttgtt 3540
   ttttgttttt tgtttttttt ttttgacact gtccaaaggt tttccatcct gtcctggaat 3600
   cagagttgga agctgaggag cttcagcctc ttttatggtt taatggccac ctgttctctc 3660
   ctgtgaaagg ctttgcaaag tcacattaag tttgcatgac ctgttatccc tggggcccta 3720
   tttcatagag gctggcccta ttagtgattt ccaaaaacaa tatggaagtg ccttttgatg 3780
   tcttacaata agagaagaag ccaatggaaa tgaaagagat tggcaaaggg gaaggatgat 3840
   gccatgtaga tcctgtttga catttttatg gctgtatttg taaacttaaa cacaccagtg 3900
   tctgttcttg atgcagttgc tatttaggat gagttaagtg cctggggagt ccctcaaaag 3960
   gttaaaggga ttcccatcat tggaatctta tcaccagata ggcaagttta tgaccaaaca 4020
   agagagtact ggctttatcc tctaacctca tattttctcc cacttggcaa gtcctttgtg 4080
   gcatttattc atcagtcagg gtgtccgatt ggtcctagaa cttccaaagg ctgcttgtca 4140
   tagaagccat tgcatctata aagcaacggc tcctgttaaa tggtatctcc tttctgaggc 4200
   tcctactaaa agtcatttgt tacctaaact tatgtgctta acaggcaatg cttctcagac 4260
   cacaaagcag aaagaagaag aaaagctcct gactaaatca gggctgggct tagacagagt 4320
   tgatctgtag aatatcttta aaggagagat gtcaactttc tgcactattc ccagcctctg 4380
   ctcctccctg tctaccctct cccctccctc tctccctcca cttcacccca caatcttgaa 4440
   aaacttcctt tctcttctgt gaacatcatt ggccagatcc attttcagtg gtctggattt 4500
   ctttttattt tcttttcaac ttgaaagaaa ctggacatta ggccactatg tgttgttact 4560
   gccactagtg ttcaagtgcc tcttgttttc ccagagattt cctgggtctg ccagaggccc 4620
   agacaggctc actcaagctc tttaactgaa aagcaacaag ccactccagg acaaggttca 4680
   aaatggttac aacagcctct acctgtcgcc ccagggagaa aggggtagtg atacaagtct 4740
   catagccaga gatggttttc cactccttct agatattccc aaaaagaggc tgagacagga 4800
   ggttattttc aattttattt tggaattaaa tacttttttc cctttattac tgttgtagtc 4860
   cctcacttgg atatacctct gttttcacga tagaaataag ggaggtctag agcttctatt 4920
   ccttggccat tgtcaacgga gagctggcca agtcttcaca aacccttgca acattgcctg 4980
   aagtttatgg aataagatgt attctcactc ccttgatctc aagggcgtaa ctctggaagc 5040
   acagcttgac tacacgtcat ttttaccaat gattttcagg tgacctgggc taagtcattt 5100
   aaactgggtc tttataaaag taaaaggcca acatttaatt attttgcaaa gcaacctaag 5160
   agctaaagat gtaatttttc ttgcaattgt aaatcttttg tgtctcctga agacttccct 5220
   taaaattagc tctgagtgaa aaatcaaaag agacaaaaga catcttcgaa tccatatttc 5280
   aagcctggta gaattggctt ttctagcaga acctttccaa aagttttata ttgagattca 5340
   taacaacacc aagaattgat tttgtagcca acattcattc aatactgtta tatcagagga 5400
   gtaggagaga ggaaacattt gacttatctg gaaaagcaaa atgtacttaa gaataagaat 5460
   aacatggtcc attcaccttt atgttataga tatgtctttg tgtaaatcat ttgttttgag 5520
   ttttcaaaga atagcccatt gttcattctt gtgctgtaca atgaccactg ttattgttac 5580
   tttgactttt cagagcacac ccttcctctg gtttttgtat atttattgat ggatcaataa 5640
   taatgaggaa agcatgatat gtatattgct gagttgaaag cacttattgg aaaatattaa 5700
   aaggctaaca ttaaaagact aaaggaaaca gaaaaaaaaa aaaaaaaa            5748
<210> 124
   <211> 579
   <212> DNA
   <213> Homo sapiens
<220>
   <223> 60S ribosomal protein L24, L24, ribosomal protein L30, epididymis secretory protein Li 310 (HEL-S-310)
<220>
   <221> CDS
   <222> (44)...(517)
   <223> RPL24
<400> 124
   tctttctttt cgccatcttt tgtctttccg tggagctgtc gccatgaagg tcgagctgtg 60
   cagttttagc gggtacaaga tctaccccgg acacgggagg cgctacgcca ggaccgacgg 120
   gaaggttttc cagtttctta atgcgaaatg cgagtcggct ttcctttcca agaggaatcc 180
   tcggcagata aactggactg tcctctacag aaggaagcac aaaaagggac agtcggaaga 240
   aattcaaaag aaaagaaccc gccgagcagt caaattccag agggccatta ctggtgcatc 300
   tcttgctgat ataatggcca agaggaatca gaaacctgaa gttagaaagg ctcaacgaga 360
   acaagctatc agggctgcta aggaagcaaa aaaggctaag caagcatcta aaaagactgc 420
   aatggctgct gctaaggcac ctacaaaggc agcacctaag caaaagattg tgaagcctgt 480
   gaaagtttca gctccccgag ttggtggaaa acgctaaact ggcagattag atttttaaat 540
   aaagattgga ttataactct agaaaaaaaa aaaaaaaaa                     579
<210> 125
   <211> 3749
   <212> DNA
   <213> Homo sapiens
<220>
   <223> nicalin precursor (NCLN), nicastrin-like protein; nicalin homolog (zebrafish), NET59
<220>
   <221> CDS
   <222> (155) ... (1846)
   <223> NCLN
<400> 125
   gcggtgccca caggacctca gggcgagtgc gggctgcccc gcgcggcgcc cgcaggaccc 60
   cggcggctac ccatgccgag gtgagtccgc gggagccgcc gccgccgccg tcccgtccca 120
   gctgccgccc cgcgcggccc cgccgccggc caggatgctg gaggaagcgg gcgaggtgct 180
   ggagaacatg ctgaaggcgt cttgtctgcc gctcggcttc atcgtcttcc tgcccgctgt 240
   gctgctgctg gtggcgccgc cgctgcctgc cgccgacgcc gcgcacgagt tcaccgtgta 300
   ccgcatgcag cagtacgacc tgcagggcca gccctacggc acacggaatg cagtgctgaa 360
   cacggaggcg cgcacgatgg cggcggaggt gctgagccgc cgctgcgtgc tcatgcggct 420
   actggacttc tcctacgagc agtaccagaa ggccctgcgg cagtcggcgg gcgccgtggt 480
   catcatcctg cccagggcca tggccgccgt gccccaggac gtcgtccggc aattcatgga 540
   gatcgagccg gagatgctgg ccatggagac cgccgtcccc gtgtactttg ccgtggagga 600
   cgaggccctg ctgtctatct acaagcagac ccaggctgcc tccgcctccc agggctccgc 660
   ctctgctgct gaagtactgc tgcgcacggc cactgccaac ggcttccaga tggtcaccag 720
   cggggtacag agcaaggccg tgagtgactg gctgattgcc agcgtggagg ggcggctgac 780
   ggggctgggc ggagaggacc ttcccaccat cgtcatcgtg gcccactacg acgcctttgg 840
   agtggccccc tggctgtcgc tgggcgcgga ctccaacggg agcggcgtct ctgtgctgct 900
   ggagctggca cgcctcttct cccggctcta cacctacaag cgcacgcacg ccgcctacaa 960
   cctcctgttc tttgcgtctg gaggaggcaa gtttaactac cagggaacca agcgctggct 1020
   ggaagacaac ctggaccaca cagactccag cctgcttcag gacaatgtgg ccttcgtgct 1080
   gtgcctggac accgtgggcc ggggcagcag cctgcacctg cacgtgtcca agccgcctcg 1140
   ggagggcacc ctgcagcacg ccttcctgcg ggagctggag acggtggccg cgcaccagtt 1200
   ccctgaggta cggttctcca tggtgcacaa gcggatcaac ctggcggagg acgtgctggc 1260
   ctgggagcac gagcgcttcg ccatccgccg actgcccgcc ttcacgctgt cccacctgga 1320
   gagccaccgt gacggccagc gcagcagcat catggacgtg cggtcccggg tggattctaa 1380
   gaccctgacc cgtaacacga ggatcattgc agaggccctg actcgagtca tctacaacct 1440
   gacagagaag ggacaccccc cagacatgcc ggtgttcaca gagcagatgc agatccagca 1500
   ggagcagctg gactcggtga tggactggct caccaaccag ccgcgggccg cgcagctggt 1560
   ggacaaggac agcaccttcc tcagcacgct ggagcaccac ctgagccgct acctgaagga 1620
   cgtgaagcag caccacgtca aggctgacaa gcgggaccca gagtttgtct tctacgacca 1680
   gctgaagcaa gtgatgaatg cgtacagagt caagccggcc gtctttgacc tgctcctggc 1740
   tgttggcatt gctgcctacc tcggcatggc ctacgtggct gtccagcact tcagcctcct 1800
   ctacaagacc gtccagaggc tgctcgtgaa ggccaagaca cagtgacaca gccaccccca 1860
   cagccggagc cccgccgctc ccacagtccc tggggccgag cacgagtgag tggacactgc 1920
   cccgccgcgg gcggccctgc agggacaggg gccctctccc tccccggcgg tggttggaac 1980
   actgaattac agagcttttt tctgttgctc tccgagactg gggggggatt gtttcttctt 2040
   ttccttgtct ttgaacttcc ttggaggaga gcttgggaga cgtcccgggg ccaggctacg 2100
   gacttgcgga cgagcccccc agtcctggga gccggccgcc ctcggtctgg tgtaagcaca 2160
   catgcacgat taaagaggag acgccgggac cccctgcccg atcgcgcgcg gcctccgccc 2220
   accgcctcct gccgcaaggg gcctggactg caggcctgac ctgctccctg ctccgtgtct 2280
   gtcctaggac gtcccctccc gctccccgat ggtggcgtgg acatggttat ttatctctgc 2340
   tccttcttgc ctggaggagg gcagtgccag ccctggggtt ctgggattcc agccctcctg 2400
   gagccttttg ttccccatgt ggtctcagtg acccgtcccc ctgacagtgg gctcggggag 2460
   ctgcatcacc cagccttccc cttctccgac tgcagggtct gatgtcatca ttgacagcct 2520
   ttgcttcgtg ggggcctggc agggcccctg cctccccgac ccccgaccca ctgcaaatcc 2580
   ccgttcccct gcactcctct tctcccagcc catccctccg gcccctgtgc ctctgcggcc 2640
   ccagcccagc tcccagggcc gtcacctgct tggccctggc ccagctccct gccctgagtc 2700
   ctgagccagt gcctggtgtt tcctgggctc ggtactgggc ccccaggcca tccaggcttt 2760
   gccacggcca gttggtcctc cctggggaac tgggtgcggg tggagtactg ggaggcagga 2820
   ggtggcccgg ggaggccttg tggctcctcc cctcgctcct cgccctgggc ctcagcttcc 2880
   tcatcaatag aaaggatgtg ttcggggtgg gggcgtcagg tgagaacgtt tgctgggaag 2940
   gagaggactt ggggcatggc ctctggggcc acccttcctg gaactcagag aggaaggtcc 3000
   gggccctcgg gaagccttgg acagaaccct ccaccccgca gaccaggcgt cgtgtgtgtg 3060
   tgggagagaa ggaggcccgt gttgagctca gggagacccc ggtgtgtccg ttctttagca 3120
   atataaccta cccagtgcgt gccgagcagg cttggtgggg aagggacttg agctgggcaa 3180
   gtcctggcct ggcacccgca gccgtctccc ttccgtggcc cagggaggtg tttgctgtcc 3240
   gaaggacctg ggccggccca tgggagcctg gggttctgtc cagataggac cagggggtct 3300
   cactttggcc accagttctt cggccagcac ctctgccctc cagaacctgc agcctggagg 3360
   ggtgagggga caaccacccc tctttcctcc aggttggcag gggaccctct tctcccgtct 3420
   gccctgcggg ttgcccgcct cctccagaga cttgcccaag ggcccatcac cactggcctc 3480
   tgggcacttg tgctgagact ctgggaccca ggcagctgcc accttgtcac catgagagaa 3540
   tttggggagt gcttgcatgc tagccagcag gctcctgtct gggtgccacg gggccagcat 3600
   tttggaggga gcttccttcc ttccttcctg gacaggtcgt catgatggat gcactgactg 3660
   accgtctggg gctcaggctg gtgtgggatg cagccggccg atgagaaaat aaagccatat 3720
   tgaatgatcg ccaaaaaaaa aaaaaaaaa 3749
<210> 126
   <211> 2350
   <212> DNA
   <213> Homo sapiens
<220>
   <223> 60S ribosomal protein L15 transcript variant 1, L15, DBA12, EC45, RPL10, RPLY10, RPYL10
<220>
   <221> CDS
   <222> (381)...(995)
   <223> RPL15
<400> 126
   aaagacagcg gctccaccgc ggtacgcggc caccggcttt ggagcctgga ccccaacttg 60
   cctcctctcg cggagagaca gtcgccgacg ctcgcttagc cgccgagacc tcgccgccaa 120
   ctctctcacc tctcgagacg cccaggccgc tcaggctcga atcttgcgga gcagggggcg 180
   ggacaatagc ggccgcggcg ccccactcgg cagaactccg ccaccaggcg cgatgccgga 240
   actacatgtc ccatgacgct ctgggaggcc gcagctttcc accggaaaga gggtggctga 300
   ggtgggggag gagcccaaaa ggcattgtgg gagtacagct ctttcctttc cgtctggcgg 360
   cagccatcag gtaagccaag atgggtgcat acaagtacat ccaggagcta tggagaaaga 420
   agcagtctga tgtcatgcgc tttcttctga gggtccgctg ctggcagtac cgccagctct 480
   ctgctctcca cagggctccc cgccccaccc ggcctgataa agcgcgccga ctgggctaca 540
   aggccaagca aggttacgtt atatatagga ttcgtgttcg ccgtggtggc cgaaaacgcc 600
   cagttcctaa gggtgcaact tacggcaagc ctgtccatca tggtgttaac cagctaaagt 660
   ttgctcgaag ccttcagtcc gttgcagagg agcgagctgg acgccactgt ggggctctga 720
   gagtcctgaa ttcttactgg gttggtgaag attccacata caaatttttt gaggttatcc 780
   tcattgatcc attccataaa gctatcagaa gaaatcctga cacccagtgg atcaccaaac 840
   cagtccacaa gcacagggag atgcgtgggc tgacatctgc aggccgaaag agccgtggcc 900
   ttggaaaggg ccacaagttc caccacacta ttggtggctc tcgccgggca gcttggagaa 960
   ggcgcaatac tctccagctc caccgttacc gctaatataa gtaaagtttg taaaattcat 1020
   acttaataaa caatttagga cagtcatgtc tgcttacagg tgttatttgt ctgttaaaac 1080
   tagtctgcag atgtttcttg aatgctttgt caaattaaga aagttaaagt gcaataatgt 1140
   ttgaagacaa taagtggtgg tgtatcttgt ttctaataag ataaactttt ttgtctttgc 1200
   tttatcttat tagggagttg tatgtcagtg tataaaacat actgtgtggt ataacaggct 1260
   taataaattc tttaaaagga gagaactgaa actagccctg tagatttgtc tggtgcatgt 1320
   gatgaaacct gcagctttat cggagtgatg gcaatgctct gctggtttat tttcaagtgg 1380
   ctgcgttttt tttagtttgg caggtgtaga ctttttaagt tgggctttag aaaatctggg 1440
   ttagcctgaa gaaaattgcc tcagcctcca cagtaccatt ttaaattcac ataaaaggtg 1500
   aaagctcctg gttcagtgcc atggcttcat ggcattcagt gattagtggt aatggtaaac 1560
   actggtgtgt tttgaagttg aatgtgcgat aaaattatta gccttaagat tggtaagcta 1620
   gcaatgaatg ctagggtggg aagctggtga gccagtggcc attagataaa tacctttcaa 1680
   gtgtgagctt agacgtcaac cctaaaatac ttaaccgtaa tgctaattgt gatcattatg 1740
   aatcccttca gtcacattag ggggaaagta gttggctata agtacgtcat tcttagtcca 1800
   gtcagtctta aaaacatctt gggttaccca ctctgtccac tcccataggc tacagaaaaa 1860
   gtcacaagcg catggtttcc aaccatatgt gttttctgca gttatttctc ttgttctggc 1920
   caaacaaccc taaaaatcct taccattcca caaagttgga ccatcacttg tgcacccact 1980
   ttgactatga gtataccacc acattgcatt tctgtttgca ccatgtcttc caggagacta 2040
   gactactgtt gtccagggtc aatttgagtg taaagaaaat gtagacaagg aattgcccaa 2100
   ttttaaattc tgactttgct gacttaattt aaatgctcgt tctgaaccaa ttttctccta 2160
   tcttctctag ggtttcaaag agactcagtt aattgatttc caggaagtac tcatagcaag 2220
   ttcataaaag ttcttgagac ctaaatttct tcacaaaaaa agaaaagatc ttaagtcata 2280
   cattttaatt gtgtagaggt tgttcaactg aaggaataaa tgtctattaa actaaaacaa 2340
   atggaccttc                                                        2350
<210> 127
   <211> 2531
   <212> DNA
   <213> Homo sapiens
<220>
   <223> cleft lip and palate transmembrane protein 1 transcript variant 2 (CLPTM1)
<220>
   <221> CDS
   <222> (55) ... (2064)
   <223> CLPTM1
<400> 127
   gaagtcgggg acggggcggg gctggcggcg ggggcgggga cccggagcgg gaagatggcg 60
   gcggcgcagg aggcggacgg ggcccgcagc gccgtggtgg cggccggggg aggcagctcc 120
   ggtcaggtga ccagcaatgg cagcatcggg agggacccgc cagcggagac ccagcctcag 180
   aacccaccgg cccagccggc acccaatgcc tggcaggtca tcaaaggtgt gctgtttagg 240
   atcttcatca tctgggccat cagcagttgg ttccgccgag ggccggcccc tcaggaccag 300
   gcgggccccg gaggagctcc acgcgtcgcc agccgcaacc tgttccccaa agacacttta 360
   atgaacctgc atgtgtacat ctcagagcac gagcacttta cagacttcaa cgccacgtcg 420
   gcactcttct gggaacagca cgatcttgtg tatggcgact ggactagcgg cgagaactca 480
   gacggctgct acgagcactt tgctgagctc gatatcccac agagcgtcca gcagaacggc 540
   tccatctaca tccacgttta cttcaccaag agtggcttcc acccagaccc ccggcagaag 600
   gccctgtacc gccggcttgc cacagtccac atgtcccgga tgatcaacaa atacaagcgc 660
   agacgatttc agaaaaccaa gaacctgctg acaggagaga cagaagcgga cccagaaatg 720
   atcaagaggg ctgaggacta tgggcctgtg gaggtgatct cccattggca ccccaacatc 780
   accatcaaca tcgtggacga ccacacgccg tgggtgaagg gcagtgtgcc ccctcccctg 840
   gatcaatatg tgaagttcga cgccgtgagc ggtgactact atcccatcat ctacttcaat 900
   gactactgga acctgcagaa ggactactac cccatcaacg agagcctggc cagcctgccg 960
   ctccgcgtct ccttctgccc actctcgctt tggcgctggc agctctatgc tgcccagagc 1020
   accaagtcgc cctggaactt cctgggtgat gagttgtacg agcagtcaga tgaggagcag 1080
   gactcggtga aggtggccct gctggagacc aacccctacc tgctggcgct caccatcatc 1140
   gtgtctatcg ttcacagtgt cttcgagttc ctggccttca agaatgatat ccagttctgg 1200
   aacagccggc agtccctgga gggcctgtcc gtgcgctccg tcttcttcgg cgttttccag 1260
   tcattcgtgg tcctcctcta catcctggac aacgagacca acttcgtggt ccaggtcagc 1320
   gtcttcattg ggtcctcatc cgacctctgg aagatcacca aggtcatgga cgtccggctg 1380
   gaccgagagc acagggtggc aggaatcttc ccccgcctat ccttcaagga caagtccacg 1440
   tatatcgagt cctcgaccaa agtgtatgat gatatggcat tccggtacct gtcctggatc 1500
   ctcttcccgc tcctgggctg ctatgccgtc tacagtcttc tgtacctgga gcacaagggc 1560
   tggtactcct gggtgctcag catgctctac ggcttcctgc tgaccttcgg cttcatcacc 1620
   atgacgcccc agctcttcat caactacaag ctcaagtctg tggcccacct tccctggcgc 1680
   atgctcacct acaaggccct caacacattc atcgacgacc tgttcgcctt tgtcatcaag 1740
   atgcccgtta tgtaccggat cggctgcctg cgggacgatg tggttttctt catctacctc 1800
   taccaacggt ggatctaccg cgtcgacccc acccgagtca acgagtttgg catgagtgga 1860
   gaagacccca cagctgccgc ccccgtggcc gaggttccca cagcagcagg ggccctcacg 1920
   cccacacctg cacccaccac gaccaccgcc accagggagg aggcctccac gtccctgccc 1980
   accaagccca cccagggggc cagctctgcc agcgagcccc aggaagcccc tccaaagcca 2040
   gcagaggaca agaaaaagga ttagtcgaga ctggtcctca cctgctccgg ctcctggcga 2100
   ccactacccc tgcgtcccgg ccccctcgcc tcccctccct gtcgcccttt ccctggacag 2160
   atcaggccgg ggcggtggga ggcccgcctc aggtcagggc ccagcgtgtg atgtaggggc 2220
   cggggcaggc cagggtttgt ttgtggaggc gctgtctgtc cctctgtccc tctgtgtttc 2280
   cagccatctc gccctgccag cccagcacca ctgggaatca tggtgaagct gatgcagcgt 2340
   tgccgagggg gtgggttggg cgggggtggg gccgggcccc cctacgggat gcccacggcc 2400
   gttcatcatc ttgtccctcg tccccctacc acactccccc tcctagaccg ccgcccttta 2460
   acacagtctg gatttaataa attcatatgg gtgtttaact taaactcagc actaaaaaaa 2520
   aaaaaaaaaa a                                                      2531
<210> 128
   <211> 1710
   <212> DNA
   <213> Homo sapiens
<220>
   <223> evolutionarily conserved signaling intermediate in Toll pathway, mitochondrial precursor transcript variant 1 (ECSIT), SITPEC
<220>
   <221> CDS
   <222> (135) ... (1430)
   <223> ECSIT
<400> 128
   gatttgctac ctccctggag ctccctgacc cggacgctct ctgggccaat atggcagcgc 60
   ccagcaacaa gacagagctg gcctggagtc cgcggctggc cgcgtgagta ggtgattgtc 120
   tgacaagcag aggcatgagc tgggtccagg ccaccctact ggcccgaggc ctctgtaggg 180
   cctggggagg cacctgcggg gccgccctca caggaacctc catctctcag gtccctcgcc 240
   ggctccctcg gggcctccac tgcagcgcag ctgcccatag ctctgaacag tccctggttc 300
   ccagcccacc ggaaccccgg cagaggccca ccaaggctct ggtgcccttt gaggacctgt 360
   ttgggcaggc gcctggtggg gaacgggaca aggcgagctt cctgcagacg gtgcagaaat 420
   ttgcggagca cagcgtgcgt aagcggggcc acattgactt catctacctg gccctgcgca 480
   agatgcggga gtatggtgtc gagcgggacc tggctgtgta caaccagctg ctcaacatct 540
   tccccaagga ggtcttccgg cctcgcaaca tcatccagcg catcttcgtc cactaccctc 600
   ggcagcagga gtgtgggatt gctgtcctgg agcagatgga gaaccacggt gtgatgccca 660
   acaaggagac ggagttcctg ctgattcaga tctttggacg caaaagctac cccatgctca 720
   agttggtgcg cctgaagctg tggttccctc gattcatgaa cgtcaacccc ttcccagtgc 780
   cccgggacct gccccaggac cctgtggagc tggccatgtt tggcctgcgg cacatggagc 840
   ctgaccttag tgccagggtc accatctacc aggttccttt gcccaaagac tcaacaggtg 900
   cagcagatcc cccccagccc cacatcgtag gaatccagag tcccgatcag caggccgccc 960
   tggcccgcca caatccagcc cggcctgtct ttgttgaggg ccccttctcc ctgtggctcc 1020
   gcaacaagtg tgtgtattac cacatcctca gagctgactt gctgcccccg gaggagaggg 1080
   aagtggaaga gacgccggag gagtggaacc tctactaccc gatgcagctg gacctggagt 1140
   atgtgaggag tggctgggac aactacgagt ttgacatcaa tgaagtggag gaaggccctg 1200
   tcttcgccat gtgcatggcg ggtgctcatg accaggcgac gatggctaag tggatccagg 1260
   gcctgcagga gaccaaccca accctggccc agatccccgt ggtcttccgc ctcgccgggt 1320
   ccacccggga gctccagaca tcctctgcag ggctggagga gccgcccctg cccgaggacc 1380
   accaggaaga agacgacaac ctgcagcgac agcagcaggg ccagagctag tctgagccgg 1440
   cgcgagggca cgggctgtgg cccgaggagg cggtggactg aaggcatgag atgccctttg 1500
   agtgtacagc aaatcaatgt tttcctgctt ggggctctct tccctcatct ctagcagtat 1560
   ggcatcccct ccccaggatc tcgggctgcc agcgatgggc aggcgagacc cctccagaat 1620
   ctgcaggcgc ctctggttct ccgaattcaa ataaaaaggg gcgggagcgc tgttggttgt 1680
   gcgcatgcgc agtttccaaa aaaaaaaaaa                                 1710
<210> 129
   <211> 854
   <212> DNA
   <213> Homo sapiens
<220>
   <223> eukaryotic translation elongation factor 1 beta 2 transcript variant 2 (EEF1B2), elongation factor 1-beta (EF1B, EEF1B, EF-1-beta), eukaryotic translation elongation factor 1 beta 1 (EEF1B1)
<220>
   <221> CDS
   <222> (113)...(790)
   <223> EEF1B2
<400> 129
   gccggaagtg gccccagcct cgaggccggg cgtcttcggt catctccggc gcttctaggg 60
   ctggttcccg tcatcttcgg gagccgtgga gctctcggat acagccgaca ccatgggttt 120
   cggagacctg aaaagccctg ccggcctcca ggtgctcaac gattacctgg cggacaagag 180
   ctacatcgag gggtatgtgc catcacaagc agatgtggca gtatttgaag ccgtgtccag 240
   cccaccgcct gccgacttgt gtcatgccct acgttggtat aatcacatca agtcttacga 300
   aaaggaaaag gccagcctgc caggagtgaa gaaagctttg ggcaaatatg gtcctgccga 360
   tgtggaagac actacaggaa gtggagctac agatagtaaa gatgatgatg acattgacct 420
   ctttggatct gatgatgagg aggaaagtga agaagcaaag aggctaaggg aagaacgtct 480
   tgcacaatat gaatcaaaga aagccaaaaa acctgcactt gttgccaagt cttccatctt 540
   actagatgtg aaaccttggg atgatgagac agatatggcg aaattagagg agtgcgtcag 600
   aagcattcaa gcagacggct tagtctgggg ctcatctaaa ctagttccag tgggatacgg 660
   aattaagaaa cttcaaatac agtgtgtagt tgaagatgat aaagttggaa cagatatgct 720
   ggaggagcag atcactgctt ttgaggacta tgtgcagtcc atggatgtgg ctgctttcaa 780
   caagatctaa aatccatcct ggatcatggc atttaaataa aagattgaaa gattacaaaa 840
   aaaaaaaaaa aaaa 854
<210> 130
   <211> 743
   <212> DNA
   <213> Homo sapiens
<220>
   <223> prefoldin subunit 5 transcript variant 1 (PFDN5, PFD5), c-myc binding protein, myc modulator-1 (MM-1, MM1)
<220>
   <221> CDS
   <222> (118)...(582)
   <223> PFDN5
<400> 130
   gaggatcata gagctgtctg gcgcagcgag gcctcccggc gccaccgaga cgcgcagagg 60
   acggctagag cgttgctcgc cgagagactt cctcttcgtt aagtcggcct tcccaacatg 120
   gcgcagtcta ttaacatcac ggagctgaat ctgccgcagc tagaaatgct caagaaccag 180
   ctggaccagg aagtggagtt cttgtccacg tccattgctc agctcaaagt ggtacagacc 240
   aagtatgtgg aagccaagga ctgtctgaac gtgctgaaca agagcaacga ggggaaagaa 300
   ttactcgtcc cactgacgag ttctatgtat gtccctggga agctgcatga tgtggaacac 360
   gtgctcatcg atgtgggaac tgggtactat gtagagaaga cagctgagga tgccaaggac 420
   ttcttcaaga ggaagataga ttttctaacc aagcagatgg agaaaatcca accagctctt 480
   caggagaagc acgccatgaa acaggccgtc atggaaatga tgagtcagaa gattcagcag 540
   ctcacagccc tgggggcagc tcaggctact gctaaggcct gagagttttt gcagaaatgg 600
   ggcagaggga caccctttgg gcgtggcttc ctggtgatgg gaagggtctt gtgttttaat 660
   gccaataaat gtgccagctg ggcagaaaaa aaaaaaaaaa aaaaaaaaaa aaaaaaaaaa 720
   aaaaaaaaaa aaaaaaaaaa aaa 743
<210> 131
   <211> 1721
   <212> DNA
   <213> Homo sapiens
<220>
   <223> polynucleotide kinase 3'-phosphatase (PNKP, PNK), bifunctional polynucleotide phosphatase/kinase, DNA 5'-kinase/3'-phosphatase, EIEE10, MCSZ
<220>
   <221> CDS
   <222> (111) ... (1676)
   <223> PNKP
<400> 131
   ccgaggaacc gaccgccgcc ggccgggttg caggcggggc acctcgggca ggacctccct 60
   ggtcggaagt ggccgtgagc ccaagccgcg gtcccgggcc ggcacccagg atgggcgagg 120
   tggaggcccc gggccgcttg tggctcgaga gcccccctgg gggagcgccc cccatcttcc 180
   tgccctcgga cgggcaagcc ctggtcctgg gcaggggacc cctgacccag gttacggacc 240
   ggaagtgctc cagaactcaa gtggagctgg tcgcagatcc tgagacccgg acagtggcag 300
   tgaaacagct gggagttaac ccctcaacta ccgggaccca ggagttgaag ccggggttgg 360
   agggctctct gggggtgggg gacacactgt atttggtcaa tggcctccac ccactgaccc 420
   tgcgctggga agagacccgc acaccagaat cccagccaga tactccgcct ggcacccctc 480
   tggtgtccca agatgagaag agagatgctg agctgccgaa gaagcgtatg cggaagtcaa 540
   accccggctg ggagaacttg gagaagttgc tagtgttcac cgcagctggg gtgaaacccc 600
   agggcaaggt ggctggcttt gatctggacg ggacgctcat caccacacgc tctgggaagg 660
   tctttcccac tggccccagt gactggagga tcttgtaccc agagattccc cgtaagctcc 720
   gagagctgga agccgagggc tacaagctgg tgatcttcac caaccagatg agcatcgggc 780
   gcgggaagct gccagccgag gagttcaagg ccaaggtgga ggctgtggtg gagaagctgg 840
   gggtcccctt ccaggtgctg gtggccacgc acgcaggctt gtaccggaag ccggtgacgg 900
   gcatgtggga ccatctgcag gagcaggcca acgacggcac gcccatatcc atcggggaca 960
   gcatctttgt gggagacgca gccggacgcc cggccaactg ggccccgggg cggaagaaga 1020
   aagacttctc ctgcgccgat cgcctgtttg ccctcaacct tggcctgccc ttcgccacgc 1080
   ctgaggagtt ctttctcaag tggccagcag ccggcttcga gctcccagcc tttgatccga 1140
   ggactgtctc ccgctcaggg cctctctgcc tccccgagtc cagggccctc ctgagcgcca 1200
   gcccggaggt ggttgtcgca gtgggattcc ctggggccgg gaagtccacc tttctcaaga 1260
   agcacctcgt gtcggccgga tatgtccacg tgaacaggga cacgctaggc tcctggcagc 1320
   gctgtgtgac cacgtgtgag acagccctga agcaagggaa acgggtcgcc atcgacaaca 1380
   caaacccaga cgccgcgagc cgcgccaggt acgtccagtg tgcccgagcc gcgggcgtcc 1440
   cctgccgctg cttcctcttc accgccactc tggagcaggc gcgccacaac aaccggtttc 1500
   gagagatgac ggactcctct catatccccg tgtcagacat ggtcatgtat ggctacagga 1560
   agcagttcga ggccccaacg ctggctgaag gcttctctgc catcctggag atcccgttcc 1620
   ggctatgggt ggagccgagg ctggggcggc tgtactgcca gttctccgag ggctgagccc 1680
   cgcccagctc ccctccacaa taaacgctgt ttctccttga g                     1721
<210> 132
   <211> 2889
   <212> DNA
   <213> Homo sapiens
<220>
   <223> septin 8 transcript variant 1 (SEPT8), SEP2
<220>
   <221> CDS
   <222> (239) ... (1690)
   <223> SEPT8
<400> 132
   gcggcggggc tccggctgcg ctcgtggccg ggccgggcgg ggaggccggt cccgcgggcg 60
   ggggcagggg cggctccgcg gcttctcccg ccgccgccgc caaggggagt ttccaggaag 120
   tggccatatt ggatccattc agccgcagcc gcccgggcgg agcgcgtccc gcagccggct 180
   ggtccctgtc gctgcccctg cgctcgtccc agcccacccg cccggtgcgg agctcgccat 240
   ggcggccacc gacctggagc gcttctcgaa tgcagagcca gagccccgga gcctctccct 300
   gggcggccat gtgggtttcg acagcctccc cgaccagctg gtcagcaagt cggtcactca 360
   gggcttcagc ttcaacatcc tctgtgtggg ggagaccggc attggcaaat ccacactgat 420
   gaacacactc ttcaacacga ccttcgagac tgaggaagcc agtcaccatg aggcatgcgt 480
   gcgcctgcgg ccccagacct atgacctcca ggagagcaac gtgcagctca agctgaccat 540
   tgtggatgcc gtgggctttg gggatcagat caataaggat gagagttaca ggcccatagt 600
   tgactacatc gatgcgcagt ttgaaaatta tctgcaggag gagctgaaga tccgccgctc 660
   gctcttcgac taccatgaca caaggatcca cgtttgcctc tacttcatca cgcccacagg 720
   gcactccctg aagtctctag atctagtgac catgaagaaa ctagacagca aggtgaacat 780
   tattcccatc atcgccaagg ctgacaccat ctccaagagc gagctccaca agttcaagat 840
   caagatcatg ggcgagttgg tcagcaacgg ggtccagatc taccagttcc ccacggatga 900
   tgaggctgtt gcagagatta acgcagtcat gaatgcacat ctgccctttg ccgtggtggg 960
   cagcaccgag gaggtgaagg tggggaacaa gctggtccga gcacggcagt acccctgggg 1020
   agtggtgcag gtggagaatg agaatcactg cgacttcgtg aagctgcggg agatgttgat 1080
   ccgggtgaac atggaagacc tccgcgagca gacccacagc cggcactacg agctctaccg 1140
   gcgctgcaag ttggaggaga tgggctttca ggacagcgat ggtgacagcc agcccttcag 1200
   cctacaagag acatacgagg ccaagaggaa ggagttccta agtgagctgc agaggaagga 1260
   ggaagagatg aggcagatgt ttgtcaacaa agtgaaggag acagagctgg agctgaagga 1320
   gaaggaaagg gagctccatg agaagtttga gcacctgaag cgggtccacc aggaggagaa 1380
   gcgcaaggtg gaggaaaagc gccgggaact ggaggaggag accaacgcct tcaatcgccg 1440
   gaaggctgcg gtggaggccc tgcagtcgca ggccttgcac gccacctcgc agcagcccct 1500
   gaggaaggac aaggacaaga agaacagatc agatatagga gcacaccagc cgggcatgag 1560
   cctctccagc tctaaggtga tgatgaccaa ggccagtgtg gagcccttga actgcagcag 1620
   ctggtggccc gccatacagt gctgcagctg cctggtcagg gatgcgacgt ggagggaagg 1680
   attcctctga ggcagcagct ccaacacatg ggccagctcc aggaccacca gggcatggaa 1740
   ctggagacca tggtttttaa tgttagaaca gaaaacgcca tacttttcct atatcaatga 1800
   tcaaaagtgc aaacaattta aatttccatc agggaacatc aaatgttgcc caaccctttt 1860
   cattcctatc catggctccg taaggggctt gaggcttaat gcccatcctg tggccaagct 1920
   gagcttccac tccgggacca aaaaaaaaaa aaagtctgct ttgtgacatc atcgttatga 1980
   gcggaaagta cctagatgac aatgtttcca ttctgaaaaa tagaaacata ctattcaaga 2040
   ccaaggtagc agaaaagtta cttgtatctg cttatcataa gacgaaactc tgcaacttgg 2100
   caacggtggc cagttttcgt aatgaaacag tctttagtaa tttaatcttc atgcttcata 2160
   acaaaccaaa accccatgag atttccacat tgcataattt tgccttacta acagaatcat 2220
   atccttaagg atgaccatca ttcccccaac taaaacaaat acaaactaat gtatgatatt 2280
   tttttaagtg ccagatcaat atggtctaaa gcttcaataa ggattgtgtg taggtgaata 2340
   aagacagcta agtgaatgtg tgtaaagtgt agcaaaagca gacagatatt tatgtacagt 2400
   attcatagaa tggaaagtta aatatttttg cagtgtgtat ttaaaagaga aactcaccat 2460
   aatagtgccg tctaaaaatc tttgtaaagt taatttaatg tcctttagaa gtgggagtct 2520
   ggtggaactg tgttggattt aagatacctt ttcactcttc cgtatgtcat gagccttgtg 2580
   cgtcacctca ctgtggtgca tgtgcaaggg cgtgtgcacg cctgtgcttt gccatcccat 2640
   gttgtaaaca gctgttccaa aggcacaaac gagtttaggg tagactctgt aaacacctcc 2700
   ttactcacta tagtcaagaa gtccagcggc gtcccaatat agaggtccca gtgcagtctg 2760
   tccagaatag ccagctccat cctcagcagc tcattcgggg aatagtcaga gccatagtgc 2820
   tttgtgaagt cttttacttg tggaataaac tgtaaaaaga aaataaagag gccaaagccc 2880
   tacatcatg                                                          2889
<210> 133
   <211> 1397
   <212> DNA
   <213> Homo sapiens
<220>
   <223> cold inducible RNA binding protein transcript variant 1 (CIRBP, CIRP), glycine-rich RNA binding protein, A18 hnRNP
<220>
   <221> CDS
   <222> (150)...(668)
   <223> CIRBP
<400> 133
   aggatgtgta gggggcgggg cccggcggaa gcgtatataa ggccgggctc ggggacgccc 60
   ccccctcact cgcgcgttag gaggctcggg tcgttgtggt gcgctgtctt cccgcttgcg 120
   tcagggacct gcccgactca gtggccgcca tggcatcaga tgaaggcaaa ctttttgttg 180
   gagggctgag ttttgacacc aatgagcagt cgctggagca ggtcttctca aagtacggac 240
   agatctctga agtggtggtt gtgaaagaca gggagaccca gagatctcgg ggatttgggt 300
   ttgtcacctt tgagaacatt gacgacgcta aggatgccat gatggccatg aatgggaagt 360
   ctgtagatgg acggcagatc cgagtagacc aggcaggcaa gtcgtcagac aaccgatccc 420
   gtgggtaccg tggtggctct gccgggggcc ggggcttctt ccgtgggggc cgaggacggg 480
   gccgtgggtt ctctagagga ggaggggacc gaggctatgg ggggaaccgg ttcgagtcca 540
   ggagtggggg ctacggaggc tccagagact actatagcag ccggagtcag agtggtggct 600
   acagtgaccg gagctcgggc gggtcctaca gagacagtta tgacagttac gctacacaca 660
   acgagtaaaa acccttcctg ctcaagatcg tccttccaat ggctgtgtgt ttaaagattg 720
   tgggagcttc gctgaacgtt aatgtgtagt aaatgcacct ccttgtattc ccactttcgt 780
   agtcatttcg gttctgatct tgtcaaaccc agcctgaccg cttctgacgc cgggatggcc 840
   tcgttactag acttttcttt ttaaggaagt gctgtttttt tttgagggtt ttcaaaacat 900
   tttgaaaagc atttactttt ttgaccacga gccatgagtt ttcaaaaaaa tcgggggttg 960
   tgtgggtttt tggtttttgt tttagttttt ggttgcgttg cctttttttt tttagtgggg 1020
   ttggccccat gaagtgggtg ccccactcac ttctctgaga tcgaacggac tgtgaatccg 1080
   ctctttgtcg gaagctgagc aagctgtggc ttttttccaa ctccgtgtga cgtttctgag 1140
   tgtagtgtgg taggaccccg gcgggtgtgg cagcaactgc cctggagccc cagcccctgc 1200
   gtccatctgt gctgtgcgcc ccacagtaga cgtgcagacg tccctgagag gttcttgaag 1260
   atgtttattt atattgtcct tttttactgg aagacgtacg catactccat cgatgttgta 1320
   tttgcagtgg ctgaggaatt cttgtacgca gttttctttg gctttacgaa gccgattaaa 1380
   agaccgtgtg aaatgaa                                                1397
<210> 134
   <211> 2528
   <212> DNA
   <213> Homo sapiens
<220>
   <223> ATP-binding cassette, sub-family B (MDR/TAP), member 7, mitochondrial transcript variant 1 (ABCB7), ABC transporter 7 protein, ATP-binding cassette transporter 7, ASAT; Atmlp; EST140535
<220>
   <221> CDS
   <222> (69)...(2330)
   <223> ABCB7
<400> 134
   aggtagccga attcagtccg ccagtgtccc ataatcctct tctctcggtt cctctttcct 60
   cgctcaagat ggcgctgctc gcgatgcatt cttggcgctg ggcggccgcg gcggctgctt 120
   tcgaaaagcg ccggcactcc gcgattctga tccggccttt agtctctgtt agcggctcag 180
   gtccgcagtg gaggccacat caactcggcg ccttgggaac cgctcgagcc taccagcaga 240
   ttccagagtc attaaaaagt atcacatggc agagattggg aaaaggcaat tcaggacagt 300
   tcttagatgc tgcaaaggct ctccaggtat ggccactgat agaaaagagg acatgttggc 360
   atggtcatgc aggaggagga ctccacacag acccaaaaga agggttaaaa gatgttgata 420
   ctcggaaaat cataaaagca atgctttctt atgtgtggcc caaagacagg ccagatctac 480
   gagctagagt tgccatttcg ctgggatttt tgggtggtgc aaaggccatg aatattgtgg 540
   ttcccttcat gtttaaatat gctgtagaca gcctcaacca gatgtcggga aacatgctga 600
   acctgagtga tgcaccaaat acagttgcaa ccatggcaac agcagttctg attggctatg 660
   gtgtatcaag agctggagct gcttttttta acgaagttcg aaatgcagta tttggcaagg 720
   tagcccagaa ttcaatccga agaatagcca aaaatgtctt tctccatctt cacaacctgg 780
   atctgggttt tcacctgagc agacagacgg gagctttatc taaggctatt gacagaggaa 840
   caaggggtat cagttttgtc ctgagtgctt tggtatttaa tcttcttccc atcatgtttg 900
   aagtgatgct tgtcagtggt gttttgtatt acaaatgcgg tgcccagttt gctttggtaa 960
   cccttggaac acttggtaca tacacagcat tcacagttgc agtcacacgg tggagaacta 1020
   gatttagaat agaaatgaac aaagcagata atgatgcagg taatgctgct atagactcac 1080
   tgctgaatta tgaaactgtg aagtatttta ataatgaaag atatgaagca cagagatatg 1140
   atggattttt gaagacgtat gagactgctt cattgaaaag tacctctact ctggctatgc 1200
   tgaactttgg tcaaagtgct attttcagtg tcggtttaac agctataatg gtgctcgcca 1260
   gtcagggaat tgtggcaggt acccttactg ttggagatct agtaatggtg aatggactgc 1320
   tttttcagct ttcattaccc ctgaactttc tgggaactgt atatagagag actagacaag 1380
   cactcataga tatgaacacc ttgtttactc tactcaaggt agacacccaa attaaagaca 1440
   aagtgatggc atctcccctt cagatcacac cacagacagc taccgtggcc tttgataatg 1500
   tgcattttga atacattgag ggccagaaag tccttagtgg aatatccttt gaagtccctg 1560
   caggaaagaa agtggccatt gtaggaggta gtgggtcagg gaaaagcaca atagtgaggc 1620
   tattatttcg cttctatgag cctcaaaagg gtagcattta tcttgctggt caaaatatac 1680
   aagatgtgag cctggaaagc cttcggaggg cagtgggagt ggtacctcag gatgctgtcc 1740
   tcttccataa tactatttat tacaacctct tatatggaaa catcagtgct tcacctgagg 1800
   aagtgtatgc agtggcaaaa ttagctggac ttcatgatgc aattcttcga atgccacatg 1860
   gatatgacac ccaagtaggg gaacgaggac tcaagctttc aggaggagaa aagcaaagag 1920
   tagcaattgc aagagccatt ttgaaggacc ccccagtcat actctatgat gaagctactt 1980
   catcgttaga ttcgattact gaagagacta ttcttggtgc catgaaggat gtggtcaaac 2040
   acagaacttc tattttcatt gcacacagat tgtcaacagt ggttgatgca gatgaaatca 2100
   ttgtcttgga tcagggtaag gtagccgaac gtggtaccca ccatggtttg cttgctaacc 2160
   ctcatagtat ctattcagaa atgtggcata cacagagcag ccgtgtgcag aaccatgata 2220
   accccaaatg ggaagcaaag aaagaaaata tatccaaaga ggaggaaaga aagaaactac 2280
   aagaagaaat tgtcaatagt gtgaaaggct gtggaaactg ttcgtgctaa gtcacataag 2340
   acatttcttt tttttgttgt tttggactac atatttgcac tgaagcagaa ttgttttatt 2400
   aaaaaaatca tacattccca ttttctataa tccttctttt agataagatt tatttaaaag 2460
   gggatttgag ttttacatct ttcatagtct atttaatgtg gcatctgtat ttatccccaa 2520
   attatttt                                                          2528
<210> 135
   <211> 1136
   <212> DNA
   <213> Homo sapiens
<220>
   <223> alpha-N-acetyltransferase 1A, N-terminal acetyltransferase complex ARD1 subunit homolog A transcript variant 1 (ARD1A, ARD1), N-alpha-acetyltransferase 10, NatA catalytic subunit, ARD1P, DXS707, MCOPS1, NATD, TE2
<220>
   <221> CDS
   <222> (251)...(958)
   <223> ARD1A
<400> 135
   gaccggctcc gcgcattggc ccgccccgtg cgctggcctg tcccgcgcat tggccaagcg 60
   gccgggagga cgggcccggc ggccgtgcac ttccggccgg tggccggccc ggcgcgcacc 120
   gccccttccg ccgtcgccca gcgagcccag ctccggtccc agccccggcc gtcccggcgt 180
   cgcttcggag cgcggcggca gctgactgcg ccttcacgat ccgctgggac ccgcgagccc 240
   cgccgccgtt atgaacatcc gcaatgcgag gccagaggac ctaatgaaca tgcagcactg 300
   caacctcctc tgcctgcccg agaactacca gatgaaatac tacttctacc atggcctttc 360
   ctggccccag ctctcttaca ttgctgagga cgagaatggg aagattgtgg ggtatgtcct 420
   ggccaaaatg gaagaggacc cagatgatgt gccccatgga catatcacct cattggctgt 480
   gaagcgttcc caccggcgcc tcggtctggc tcagaaactg atggaccagg cctctcgagc 540
   catgatagag aacttcaatg ccaaatatgt ctccctgcat gtcaggaaga gtaaccgggc 600
   cgccctgcac ctctattcca acaccctcaa ctttcagatc agtgaagtgg agcccaaata 660
   ctatgcagat ggggaggacg cctatgccat gaagcgggac ctcactcaga tggccgacga 720
   gctgaggcgg cacctggagc tgaaagagaa gggcaggcac gtggtgctgg gtgccatcga 780
   gaacaaggtg gagagcaaag gcaattcacc tccgagctca ggagaggcct gtcgcgagga 840
   gaagggcctg gctgccgagg atagtggtgg ggacagcaag gacctcagcg aggtcagcga 900
   gaccacagag agcacagatg tcaaggacag ctcagaggcc tccgactcag cctcctagag 960
   cctgccccat cccctcctca ccccacgagc tttcacaata aattcgctcc gtggcactgg 1020
   ggaactttgt gtgtgagcgc gcgcacattt agagggtgtg tttctccagg tcctctggtg 1080
   gggatgtgag ccttggcctt ttgacccaga gcatcctgaa aaaaaaaaaa aaaaaa     1136
<210> 136
   <211> 6587
   <212> DNA
   <213> Homo sapiens
<220>
   <223> envoplakin (EVPL, EVPK), 210 kDa paraneoplastic pemphigus antigen, 210 kDa cornified envelope precursor protein
<220>
   <221> CDS
   <222> (229) ... (6330)
   <223> EVPL
<400> 136
   agtgtggccg ttgtgggtgc atgcgcgtca ggcctgggac ccggccgccc gcccgctgcc 60
   tcacctgcaa ggaggggcct cccagaaact cccttcccca gtgcccagcc gccccacctc 120
   gccagactta gctgaccagc cagtgaggac gcccgctgcc tcccacctgc cctcctgccg 180
   tctttcgcca gccaagccca gcctgagcca gcacttgcct ttacgaccat gttcaagggg 240
   ctgagcaaag gctcccaggg gaaggggtcc cccaagggct cccccgccaa ggggtccccc 300
   aaaggctccc ccagcaggca cagccgggct gccacccagg agctggccct tctcatctcc 360
   cgcatgcaag ccaacgccga ccaggtggag cgggacatcc tggagacgca gaagaggctg 420
   cagcaggacc ggctgaacag tgagcagagc caggccctgc agcaccagca ggagacgggc 480
   cgcagcctga aggaggctga ggtgctgctc aaggacctct tcctggacgt ggacaaggcc 540
   cggcggctca agcacccgca ggctgaggag attgagaagg acatcaagca gctgcacgag 600
   cgggtgaccc aggagtgtgc ggagtaccgt gccctgtacg agaagatggt gctgcccccc 660
   gacgtgggac ccagggtcga ctgggcacgc gtgctggagc agaaacagaa gcaggtctgc 720
   gcaggccagt acgggccggg catggcggag ctggagcaac agatcgccga gcacaacatc 780
   ctgcagaagg agatcgacgc ctatgggcag cagctgcgga gcctcgtggg gccggatgca 840
   gccaccatcc ggagccaata ccgagaccta ctgaaggcgg cgtcgtggcg cgggcagagc 900
   ctgggcagcc tgtacacgca cctccagggc tgcacgcggc agctgagcgc cctggctgag 960
   cagcagcgcc gcatcctgca gcaggactgg agcgacctca tggccgaccc tgcgggcgtg 1020
   cggcgggagt acgagcactt caagcagcac gagctgctga gccaggagca gagcgtgaac 1080
   cagctggagg acgacggcga gcgcatggtg gagctgcggc accccgcggt ggggcccatc 1140
   caggcccacc aggaggccct gaagatggag tggcagaact tcctgaacct gtgtatctgc 1200
   caggagaccc agctgcagca cgtggaggac taccgccggt tccaggaaga ggccgactca 1260
   gtcagccaga ccctggcgaa gctcaactcc aacttggatg ccaagtacag ccctgcacct 1320
   gggggccccc ctggcgcccc cacagagctg ctgcaacagc tggaggcaga ggaaaaacgg 1380
   ctggccgtca ccgagagggc cactggggac ctgcagcggc gaagccggga tgtggcccct 1440
   ctgccacagc gaagaaaccc ccctcagcag cccctgcacg tggacagcat ctgcgactgg 1500
   gactcaggag aagtgcagct gctgcagggt gagcggtata agctggtaga taacactgac 1560
   ccgcacgcct gggtcgtgca gggccctggc ggggagacca agcgtgctcc cgccgcctgc 1620
   ttctgcatcc cagcaccaga ccctgatgct gtggccaggg cctcccggct ggcctcagag 1680
   ctgcaggccc tgaagcagaa attggccaca gtccagagcc gcctgaaggc cagtgctgtg 1740
   gagtctcttc ggcccagcca gcaggctcca tctggctcag acctggccaa cccacaggcc 1800
   cagaagctcc tgacacagat gacccggctg gatggagacc tgggacagat agagaggcag 1860
   gtgctggcct gggcgcgggc cccgctgagc cgccccacac ccttggagga cttggagggc 1920
   cgcatccaca gccatgaggg cacagcccag cgcctgcaga gcctgggaac ggagaaggag 1980
   acagcccaga aggagtgcga ggcgtttctg tccacgcggc ccgtgggccc cgctgccctg 2040
   cagctgcccg tagccctcaa cagcgtgaag aacaagttca gtgacgtgca ggttctgtgc 2100
   agcctctacg gggagaaagc caaggctgcc ctggatctgg agcggcagat ccaggatgcg 2160
   gacagggtca tccgaggctt cgaggccacc ctggtgcagg aggcccccat ccctgctgaa 2220
   ccgggggctc tgcaggagag ggtcagcgag ctgcagcgcc agcggaggga gctgctggaa 2280
   cagcagacct gcgtgctgcg gctacaccgc gcgctgaagg cctcggagca cgcatgcgct 2340
   gccctgcaga acaacttcca ggagttctgc caagacctgc ctcgccagca gcgccaggtg 2400
   cgagccctca ccgaccgcta ccacgccgta ggggaccagc tggacctgcg ggagaaggtg 2460
   gtgcaggatg ccgccctcac ctaccagcag ttcaagaact gcaaggataa cctgagctcc 2520
   tggctggagc acctgccccg cagccaggtg cggcccagcg acggccccag ccagatcgcc 2580
   tacaagctgc aggcgcagaa gaggctgacg caggagatcc agagccgaga gcgggacagg 2640
   gccacagcat cccacctctc ccaggccctg caggcagcgc tccaggacta tgagctccag 2700
   gcagacacct accgctgctc tttggagccc accctggcag tgtcagcccc caagagaccc 2760
   cgagtggctc ccctgcaaga gagcatccaa gcccaggaga agaaccttgc aaaggcctat 2820
   actgaggttg cagcagcaca gcagcagctg ctccagcagc tggagtttgc tagaaaaatg 2880
   ctggagaaga aggagctcag tgaggacatc cgaaggaccc atgatgcaaa gcagggctcc 2940
   gagagccctg cccaagcagg gagagagtca gaggccctga aggcccagct ggaagaggag 3000
   aggaagcggg tggcccgggt gcagcatgag ctggaggcgc agaggagcca actgctgcag 3060
   ctgaggaccc agcggccctt ggagaggctg gaggagaagg aagtggtaga gttctaccgg 3120
   gacccccagc tggagggcag cctgtccagg gtgaaggccc aggtggagga ggagggcaag 3180
   cggcgggctg gcctgcaggc agacctggaa gtggcagccc agaaggtcgt gcagctggaa 3240
   agcaagagga agaccatgca gcctcatctg ctgaccaagg aggtcaccca ggtggagagg 3300
   gaccccggcc tggacagcca ggcggcccag ctcaggatcc agatccagca gctccgcggg 3360
   gaggatgccg tcatctcggc ccggctggaa gggctgaaga aggagctact ggcccttgag 3420
   aagagggagg tggacgtgaa ggagaaggtc gtggtgaaag aggtagtcaa ggtggagaag 3480
   aatctggaaa tggtcaaggc agcccaggct ctgaggctgc agatggagga ggatgctgcg 3540
   cggaggaagc aggcggagga ggctgtggcc aagctacagg ctcgcatcga agacctggag 3600
   cgggctatca gctcggtgga gcccaaggtc atcgtgaagg aggtgaagaa ggtggagcag 3660
   gacccagggc tcctccagga gtcctccagg ctgaggagcc tcctcgagga ggagaggacc 3720
   aagaacgcga cgctggccag ggagctgagc gacctgcaca gcaagtacag cgtggtggag 3780
   aagcagaggc ccaaagtgca gctccaggag cgcgtccacg agatcttcca ggtggatccg 3840
   gagacagagc aggagatcac tcggctcaag gccaagctgc aggagatggc gggcaagagg 3900
   agcggtgtgg agaaggaggt ggagaagctg ctgcccgacc tggaggtcct gcgggcccag 3960
   aagcccacgg tggagtacaa ggaggtgacc caggaggtgg tgaggcatga gaggagcccc 4020
   gaggtgctgc gtgagatcga ccgcctgaag gctcagctca acgagctcgt caacagccac 4080
   gggcgctccc aggagcagct catccgcctg cagggtgagc gcgacgagtg gaggcgcgag 4140
   cgggccaagg tggagaccaa gacggtgagc aaggaggtgg tgcgccacga gaaggacccg 4200
   gtgctggaga aagaagcaga gcggctccgc caggaggtgc gggaggcggc ccagaagagg 4260
   cgggccgcgg aggacgcggt gtacgagctg cagagcaagc gcctgctgct ggagaggagg 4320
   aagcccgagg agaaggtggt ggtgcaggag gtggtggtca cccagaagga cccgaagctg 4380
   cgcgaggagc acagccggct gagcgggagc ctggatgagg aggtgggccg gcggcgccag 4440
   ctagagcttg aggtgcagca gctgcgggcc ggcgtggagg agcaggaggg cctgctcagc 4500
   ttccaggagg accgcagcaa gaagctggcc gtggagaggg agctgcggca gctgaccttg 4560
   aggatccagg agctcgagaa gcggcctccc acggtgcagg agaagatcat catggaggaa 4620
   gtggtcaagc tggagaagga cccggacctg gagaagtcca cggaagccct gcggtgggac 4680
   ctggaccagg agaagaccca ggtaaccgag ctgaatcggg agtgcaagaa cctgcaggtc 4740
   cagattgacg tcctccagaa agccaaatcg caggagaaga ccatctacaa ggaagtgatc 4800
   cgggtgcaga aggaccgcgt cctggaagat gagcgggccc gcgtgtggga gatgctcaac 4860
   agggagcgca cggcccggca ggcccgggag gaggaggcac ggcgcctgcg ggagcgcatt 4920
   gaccgggccg agacgctggg gagaacctgg tcccgggagg agtccgagct gcagagggcc 4980
   cgggaccagg ccgaccagga gtgtgggcgg ctgcagcagg agctgcgggc tctggagagg 5040
   cagaagcagc agcagacact gcagctgcag gaggagtcga agctgctcag ccagaagacg 5100
   gagagcgagc gacagaaggc ggcccagcgg ggccaggagc tctcgcggct ggaggcggcc 5160
   atcctccgcg agaaggacca gatctacgag aaggagcgga cgctccggga cctccacgcc 5220
   aaggtgagcc gggaggagct cagccaggag acccagacgc gagagaccaa cctttccacc 5280
   aagatctcca tcctggaacc cgagacgggg aaggacatgt ccccatacga ggcctacaag 5340
   aggggcatca tcgacagggg ccagtacttg cagctgcagg agctcgagtg tgactgggag 5400
   gaggtcacca cctcggggcc ctgtggggag gagtctgtgc tcctggaccg caagagcggg 5460
   aagcagtact ccatcgaggc cgccctccgc tgccggcgca tctctaagga ggagtaccat 5520
   ctgtacaagg acggccacct gcccatctcc gagtttgcgc tgcttgtagc tggggagacc 5580
   aagccaagct cctcactctc catcggctct atcatctcca agtccccgct cgcctccccg 5640
   gccccccaga gcaccagttt cttctctccc agcttctctc tcgggctcgg tgatgacagc 5700
   ttccctatcg ccgggatcta tgacacaacc acagacaaca agtgcagcat caagacggcc 5760
   gtggccaaga acatgctgga ccccatcact gggcagaagc tactggaggc ccaggcggcc 5820
   acagggggca tcgtggacct gctcagccgt gagcgctact ctgtgcacaa ggcgatggag 5880
   aggggcctga tcgagaacac ctccacacag aggctgctta acgcccagaa ggccttcacc 5940
   ggcatcgagg accccgtcac caagaagagg ctctcggtgg gcgaggccgt ccagaagggc 6000
   tggatgcccc gggagagcgt gctcccacac ctgcaggtgc agcacctgac cggggggctc 6060
   atcgacccca agaggacagg ccgcatcccc atccagcagg ccctcctctc cgggatgatc 6120
   agtgaagagc tggcccagct cctgcaggac gagtccagct acgagaagga tttgacagac 6180
   cccatctcca aggaacggct gagctacaag gaggccatgg gccgctgccg caaagacccc 6240
   ctgagcggcc tgctgctcct gccagcggca ctggaggggt accgctgcta ccgctccgcc 6300
   tcccccaccg tcccgcgctc ccttcgctga cacgggccaa ggagccagtg gggaagtgcg 6360
   tgtgttgggc caggtaggat acgtacacct cttgcctcag agcagcctca tcccaggcag 6420
   tgggtcttcc ctctgtccaa ccactgtttt attattttac taacatggtg atgggctccc 6480
   tcccctaacc ttggtgcctg atccatcccc agaccaggac agcagccact cagttcttcc 6540
   tccacctcca cccagtgatc ccaataaacg aattctgtct ccccgtg                6587
<210> 137
   <211> 11426
   <212> DNA
   <213> Homo sapiens
<220>
   <223> laminin alpha-5 chain (LAMA5), laminin-10 subunit alpha, laminin-11 subunit alpha, laminin-15 subunit alpha
<220>
   <221> CDS
   <222> (68)...(11155)
   <223> LAMA5
<400> 137
   agacccgccg ggctcccgcc gcgcgcgctg tccctggagc tcggggacgc ggcccggagc 60
   cgggaagatg gcgaagcggc tctgcgcggg gagcgcactg tgtgttcgcg gcccccgggg 120
   ccccgcgccg ctgctgctgg tcgggctggc gctgctgggc gcggcgcggg cgcgggagga 180
   ggcgggcggc ggcttcagcc tgcacccgcc ctacttcaac ctggccgagg gcgcccgcat 240
   cgccgcctcc gcgacctgcg gagaggaggc cccggcgcgc ggctccccgc gccccaccga 300
   ggacctttac tgcaagctgg tagggggccc cgtggccggc ggcgacccca accagaccat 360
   ccggggccag tactgtgaca tctgcacggc tgccaacagc aacaaggcac accccgcgag 420
   caatgccatc gatggcacgg agcgctggtg gcagagtcca ccgctgtccc gcggcctgga 480
   gtacaacgag gtcaacgtca ccctggacct gggccaggtc ttccacgtgg cctacgtcct 540
   catcaagttt gccaactcac cccggccgga cctctgggtg ctggagcggt ccatggactt 600
   cggccgcacc taccagccct ggcagttctt tgcctcctcc aagagggact gtctggagcg 660
   gttcgggcca cagacgctgg agcgcatcac acgggacgac gcggccatct gcaccaccga 720
   gtactcacgc atcgtgcccc tggagaacgg agagatcgtg gtgtccctgg tgaacggacg 780
   tccgggcgcc atgaatttct cctactcgcc gctgctacgt gagttcacca aggccaccaa 840
   cgtccgcctg cgcttcctgc gtaccaacac gctgctgggc catctcatgg ggaaggcgct 900
   gcgggacccc acggtcaccc gccggtatta ttacagcatc aaggatatca gcatcggagg 960
   ccgctgtgtc tgccacggcc acgcggatgc ctgcgatgcc aaagacccca cggacccgtt 1020
   caggctgcag tgcacctgcc agcacaacac ctgcgggggc acctgcgacc gctgctgccc 1080
   cggcttcaat cagcagccgt ggaagcctgc gactgccaac agtgccaacg agtgccagtc 1140
   ctgtaactgc tacggccatg ccaccgactg ttactacgac cctgaggtgg accggcgccg 1200
   cgccagccag agcctggatg gcacctatca gggtgggggt gtctgtatcg actgccagca 1260
   ccacaccacc ggcgtcaact gtgagcgctg cctgcccggc ttctaccgct ctcccaacca 1320
   ccctctcgac tcgccccacg tctgccgccg ctgcaactgc gagtccgact tcacggatgg 1380
   cacctgcgag gacctgacgg gtcgatgcta ctgccggccc aacttctctg gggagcggtg 1440
   tgacgtgtgt gccgagggct tcacgggctt cccaagctgc tacccgacgc cctcgtcctc 1500
   caatgacacc agggagcagg tgctgccagc cggccagatt gtgaattgtg actgcagcgc 1560
   ggcagggacc cagggcaacg cctgccggaa ggacccaagg gtgggacgct gtctgtgcaa 1620
   acccaacttc caaggcaccc attgtgagct ctgcgcgcca gggttctacg gccccggctg 1680
   ccagccctgc cagtgttcca gccctggagt ggccgatgac cgctgtgacc ctgacacagg 1740
   ccagtgcagg tgccgagtgg gcttcgaggg ggccacatgt gatcgctgtg cccccggcta 1800
   ctttcacttc cctctctgcc agttgtgtgg ctgcagccct gcaggaacct tgcccgaggg 1860
   ctgcgatgag gccggccgct gcctatgcca gcctgagttt gctggacctc attgtgaccg 1920
   gtgccgccct ggctaccatg gtttccccaa ctgccaagca tgcacctgcg accctcgggg 1980
   agccctggac cagctctgtg ggcgggaggg tttgtgccgc tgccgccccg gctacacagg 2040
   cactgcctgc caggaatgca gccccggctt tcacggcttc cccagctgtg tcccctgcca 2100
   ctgctctgct gaaggctccc tgcacgcagc ctgtgacccc cggagtgggc agtgcagctg 2160
   ccggccccgt gtgacggggc tgcggtgtga cacatgtgtg cccggtgcct acaacttccc 2220
   ctactgcgaa gctggctctt gccaccctgc cggtctggcc ccagtggatc ctgcccttcc 2280
   tgaggcacag gttccctgta tgtgccgggc tcacgtggag gggccgagct gtgaccgctg 2340
   caaacctggg ttctggggac tgagccccag caaccccgag ggctgtaccc gctgcagctg 2400
   cgacctcagg ggcacactgg gtggagttgc tgagtgccag ccgggcaccg gccagtgctt 2460
   ctgcaagccc cacgtgtgcg gccaggcctg cgcgtcctgc aaggatggct tctttggact 2520
   ggatcaggct gactattttg gctgccgcag ctgccggtgt gacattggcg gtgcactggg 2580
   ccagagctgt gaaccgagga cgggcgtctg ccggtgccgc cccaacaccc agggccccac 2640
   ctgcagcgag cctgcgaggg accactacct cccggacctg caccacctgc gcctggagct 2700
   ggaggaggct gccacacctg agggtcacgc cgtgcgcttt ggcttcaacc ccctcgagtt 2760
   cgagaacttc agctggaggg gctacgcgca gatggcacct gtccagccca ggatcgtggc 2820
   caggctgaac ctgacctccc ctgacctttt ctggctcgtc ttccgatacg tcaaccgggg 2880
   ggccatgagt gtgagcgggc gggtctctgt gcgagaggag ggcaggtcgg ccacctgcgc 2940
   caactgcaca gcacagagtc agcccgtggc cttcccaccc agcacggagc ctgccttcat 3000
   caccgtgccc cagaggggct tcggagagcc ctttgtgctg aaccctggca cctgggccct 3060
   gcgtgtggag gccgaagggg tgctcctgga ctacgtggtt ctgctgccta gcgcatacta 3120
   cgaggcggcg ctcctgcagc tgcgggtgac tgaggcctgc acataccgtc cctctgccca 3180
   gcagtctggc gacaactgcc tcctctacac acacctcccc ctggatggct tcccctcggc 3240
   cgccgggctg gaggccctgt gtcgccagga caacagcctg ccccggccct gccccacgga 3300
   gcagctcagc ccgtcgcacc cgccactgat cacctgcacg ggcagtgatg tggacgtcca 3360
   gcttcaagtg gcagtgccac agccaggccg ctatgcccta gtggtggagt acgccaatga 3420
   ggatgcccgc caggaggtgg gcgtggccgt gcacacccca cagcgggccc cccagcaggg 3480
   gctgctctcc ctgcacccct gcctgtacag caccctgtgc cggggcactg cccgggatac 3540
   ccaggaccac ctggctgtct tccacctgga ctcggaggcc agcgtgaggc tcacagccga 3600
   acaggcacgc ttcttcctgc acggggtcac tctggtgccc attgaggagt tcagcccgga 3660
   gttcgtggag ccccgggtca gctgcatcag cagccacggc gcctttggcc ccaacagtgc 3720
   cgcctgtctg ccctcgcgct tcccaaagcc gccccagccc atcatcctca gggactgcca 3780
   ggtgatcccg ctgccgcccg gcctcccgct gacccacgcg caggatctca ctccagccat 3840
   gtccccagct ggaccccgac ctcggccccc caccgctgtg gaccctgatg cagagcccac 3900
   cctgctgcgt gagccccagg ccaccgtggt cttcaccacc catgtgccca cgctgggccg 3960
   ctatgccttc ctgctgcacg gctaccagcc agcccacccc accttccccg tggaagtcct 4020
   catcaacgcc ggccgcgtgt ggcagggcca cgccaacgcc agcttctgtc cacatggcta 4080
   cggctgccgc accctggtgg tgtgtgaggg ccaggccctg ctggacgtga cccacagcga 4140
   gctcactgtg accgtgcgtg tgcccaaggg ccggtggctc tggctggatt atgtactcgt 4200
   ggtccctgag aacgtctaca gctttggcta cctccgggag gagcccctgg ataaatccta 4260
   tgacttcatc agccactgcg cagcccaggg ctaccacatc agccccagca gctcatccct 4320
   gttctgccga aacgctgctg cttccctctc cctcttctat aacaacggag cccgtccatg 4380
   tggctgccac gaagtaggtg ctacaggccc cacgtgtgag cccttcgggg gccagtgtcc 4440
   ctgccatgcc catgtcattg gccgtgactg ctcccgctgt gccaccggat actggggctt 4500
   ccccaactgc aggccctgtg actgcggtgc ccgcctctgt gacgagctca cgggccagtg 4560
   catctgcccg ccacgcacca tcccgcccga ctgcctgctg tgccagcccc agacctttgg 4620
   ctgccacccc ctggtcggct gtgaggagtg taactgctca gggcccggca tccaggagct 4680
   cacagaccct acctgtgaca cagacagcgg ccagtgcaag tgcagaccca acgtgactgg 4740
   gcgccgctgt gatacctgct ctccgggctt ccatggctac ccccgctgcc gcccctgtga 4800
   ctgtcacgag gcgggcactg cgcctggcgt gtgtgacccc ctcacagggc agtgctactg 4860
   taaggagaac gtgcagggcc ccaaatgtga ccagtgcagc cttgggacct tctcactgga 4920
   tgctgccaac cccaaaggtt gcacccgctg cttctgcttt ggggccacgg agcgctgccg 4980
   gagctcgtcc tacacccgcc aggagttcgt ggatatggag ggatgggtgc tgctgagcac 5040
   tgaccggcag gtggtgcccc acgagcggca gccagggacg gagatgctcc gtgcagacct 5100
   gcggcacgtg cctgaggctg tgcccgaggc tttccccgag ctgtactggc aggccccacc 5160
   ctcctacctg ggggaccggg tgtcatccta cggtgggacc ctccgttatg aactgcactc 5220
   agagacccag cggggagatg tctttgtccc catggagagc aggccggatg tggtgctgca 5280
   gggcaaccag atgagcatca cattcctgga gccggcatac cccacgcctg gccacgttca 5340
   ccgtgggcag ctgcagctgg tggaggggaa cttccggcat acggagacgc gcaacactgt 5400
   gtcccgcgag gagctcatga tggtgctggc cagcctggag cagctgcaga tccgtgccct 5460
   cttctcacag atctcctcgg ctgtcttcct gcgcagggtg gcactggagg tggccagccc 5520
   agcaggccag ggggccctgg ccagcaatgt ggagctgtgc ctgtgccccg ccagctaccg 5580
   gggggactca tgccaggaat gtgcccccgg cttctatcgg gacgtcaaag gtctcttcct 5640
   gggccgatgt gtcccttgtc agtgccatgg acactcagac cgctgcctcc ctggctctgg 5700
   cgtctgtgtg gactgccagc acaacaccga aggggcccac tgtgagcgct gccaggctgg 5760
   cttcgtgagc agcagggacg accccagcgc cccctgtgtc agctgcccct gccccctctc 5820
   agtgccttcc aacaacttcg ccgagggctg tgtcctgcga ggcggccgca cccagtgcct 5880
   ctgcaaacct ggttatgcag gtgcctcctg cgagcggtgt gcgcccggat tctttgggaa 5940
   cccactggtg ctgggcagct cctgccagcc atgcgactgc agcggcaacg gtgaccccaa 6000
   cttgctcttc agcgactgcg accccctgac gggcgcctgc cgtggctgcc tgcgccacac 6060
   cactgggccc cgctgcgaga tctgtgcccc cggcttctac ggcaacgccc tgctgcccgg 6120
   caactgcacc cggtgcgact gtaccccatg tgggacagag gcctgcgacc cccacagcgg 6180
   gcactgcctg tgcaaggcgg gcgtgactgg gcggcgctgt gaccgctgcc aggagggaca 6240
   ttttggtttc gatggctgcg gggctgccgc cccgtgtgct tgtggaccgg ccgccgaggg 6300
   ctccgagtgc cacccccaga gcggacagtg ccactgccga ccagggacca tgggacccca 6360
   gtgccgcgag tgtgcccctg gctactgggg gctccctgag cagggctgca ggcgctgcca 6420
   gtgccctggg ggccgctgtg accctcacac gggccgctgc aactgccccc cggggctcag 6480
   cggggagcgc tgcgacacct gcagccagca gcatcaggtg cctgttccag gcgggcctgt 6540
   gggccacagc atccactgtg aagtgtgtga ccactgtgtg gtcctgctcc tggatgacct 6600
   ggaacgggcc ggcgccctcc tccccgccat tcacgagcaa ctgcgtggca tcaatgccag 6660
   ctccatggcc tgggcccgtc tgcacaggct gaacgcctcc atcgctgacc tgcagagcca 6720
   gctccggagc cccctgggcc cccgccatga gacggcacag cagctggagg tgctggagca 6780
   gcagagcaca agcctcgggc aggacgcacg gcggctaggc ggccaggccg tggggacccg 6840
   agaccaggcg agccaattgc tggccggcac cgaggccaca ctgggccatg cgaagacgct 6900
   gttggcggcc atccgggctg tggaccgcac cctgagcgag ctcatgtccc agacgggcca 6960
   cctggggctg gccaatgcct cggctccatc aggtgagcag ctgctccgga cactggccga 7020
   ggtggagcgg ctgctctggg agatgcgggc ccgggacctg ggggccccgc aggcagcagc 7080
   tgaggctgag ttggctgcag cacagagatt gctggcccgg gtgcaggagc agctgagcag 7140
   cctctgggag gagaaccagg cactggccac acaaacccgc gaccggctgg cccagcacga 7200
   ggccggcctc atggacctgc gagaggcttt gaaccgggca gtggacgcca cacgggaggc 7260
   ccaggagctc aacagccgca accaggagcg cctggaggaa gccctgcaaa ggaagcagga 7320
   gctgtcccgg gacaatgcca ccctgcaggc cactctgcat gcggctaggg acaccctggc 7380
   cagcgtcttc agattgctgc acagcctgga ccaggctaag gaggagctgg agcgcctcgc 7440
   cgccagcctg gatggggctc ggaccccact gctgcagagg atgcagacct tctccccggc 7500
   gggcagcaag ctgcgtctag tggaggccgc cgaggcccac gcacagcagc tgggccagct 7560
   ggcactcaat ctgtccagca tcatcctgga cgtcaaccag gaccgcctca cccagagggc 7620
   catcgaggcc tccaacgcct acagccgcat cctgcaggcc gtgcaggctg ccgaggatgc 7680
   tgctggccag gccctgcagc aggcggacca cacgtgggcg acggtggtgc ggcagggcct 7740
   ggtggaccga gcccagcagc tcctggccaa cagcactgca ctagaagagg ccatgctcca 7800
   ggaacagcag aggctgggcc ttgtgtgggc tgccctccag ggtgccagga cccagctccg 7860
   agatgtccgg gccaagaagg accagctgga ggcgcacatc caggcggcgc aggccatgct 7920
   tgccatggac acagacgaga caagcaagaa gatcgcacat gccaaggctg tggctgctga 7980
   agcccaggac accgccaccc gtgtgcagtc ccagctgcag gccatgcagg agaatgtgga 8040
   gcggtggcag ggccagtacg agggcctgcg gggccaggac ctgggccagg cagtgcttga 8100
   cgcaggccac tcagtgtcca ccctggagaa gacgctgccc cagctgctgg ccaagctgag 8160
   catcctggag aaccgtgggg tgcacaacgc cagcctggcc ctgtccgcca gcattggccg 8220
   cgtgcgagag ctcattgccc aggcccgggg ggctgccagt aaggtcaagg tgcccatgaa 8280
   gttcaacggg cgctcagggg tgcagctgcg caccccacgg gatcttgccg accttgctgc 8340
   ctacactgcc ctcaagttct acctgcaggg cccagagcct gagcctgggc agggtaccga 8400
   ggatcgcttt gtgatgtaca tgggcagccg ccaggccact ggggactaca tgggtgtgtc 8460
   tctgcgtgac aagaaggtgc actgggtgta tcagctgggt gaggcgggcc ctgcagtcct 8520
   aagcatcgat gaggacattg gggagcagtt cgcagctgtc agcctggaca ggactctcca 8580
   gtttggccac atgtccgtca cagtggagag acagatgatc caggaaacca agggtgacac 8640
   ggtggcccct ggggcagagg ggctgctcaa cctgcggcca gacgacttcg tcttctacgt 8700
   cggggggtac cccagtacct tcacgccccc tcccctgctt cgcttccccg gctaccgggg 8760
   ctgcatcgag atggacacgc tgaatgagga ggtggtcagc ctctacaact tcgagaggac 8820
   cttccagctg gacacggctg tggacaggcc ttgtgcccgc tccaagtcga ccggggaccc 8880
   gtggctcacg gacggctcct acctggacgg caccggcttc gcccgcatca gcttcgacag 8940
   tcagatcagc accaccaagc gcttcgagca ggagctgcgg ctcgtgtcct acagcggggt 9000
   gctcttcttc ctgaagcagc agagccagtt cctgtgcttg gccgtgcaag aaggcagcct 9060
   cgtgctgttg tatgactttg gggctggcct gaaaaaggcc gtcccactgc agcccccacc 9120
   gcccctgacc tcggccagca aggcgatcca ggtgttcctg ctggggggca gccgcaagcg 9180
   tgtgctggtg cgtgtggagc gggccacggt gtacagcgtg gagcaggaca atgatctgga 9240
   gctggccgac gcctactacc tggggggcgt gccgcccgac cagctgcccc cgagcctgcg 9300
   acggctcttc cccaccggag gctcagtccg tggctgcgtc aaaggcatca aggccctggg 9360
   caagtatgtg gacctcaagc ggctgaacac gacaggcgtg agcgccggct gcaccgccga 9420
   cctgctggtg gggcgcgcca tgactttcca tggccacggc ttccttcgcc tggcgctctc 9480
   gaacgtggca ccgctcactg gcaacgtcta ctccggcttc ggcttccaca gcgcccagga 9540
   cagtgccctg ctctactacc gggcgtcccc ggatgggcta tgccaggtgt ccctgcagca 9600
   gggccgtgtg agcctacagc tcctgaggac tgaagtgaaa actcaagcgg gcttcgccga 9660
   tggtgccccc cattacgtcg ccttctacag caatgccacg ggagtctggc tgtatgtcga 9720
   tgaccagctc cagcagatga agccccaccg gggaccaccc cccgagctcc agccgcagcc 9780
   tgaggggccc ccgaggctcc tcctgggagg cctgcctgag tctggcacca tttacaactt 9840
   cagtggctgc atcagcaacg tcttcgtgca gcggctcctg ggcccacagc gcgtatttga 9900
   tctgcagcag aacctgggca gcgtcaatgt gagcacgggc tgtgcacccg ccctgcaagc 9960
   ccagaccccg ggcctggggc ctagaggact gcaggccacc gcccggaagg cctcccgccg 10020
   cagccgtcag cccgcccggc atcctgcctg catgctgccc ccacacctca ggaccacccg 10080
   agactcctac cagtttgggg gttccctgtc cagtcacctg gagtttgtgg gcatcctggc 10140
   ccgacatagg aactggccca gtctctccat gcacgtcctc ccgcgaagct cccgaggcct 10200
   cctcctcttc actgcccgtc tgaggcccgg cagcccctcc ctggcgctct tcctgagcaa 10260
   tggccacttc gttgcacaga tggaaggcct cgggactcgg ctccgcgccc agagccgcca 10320
   gcgctcccgg cctggccgct ggcacaaggt ctccgtgcgc tgggagaaga accggatcct 10380
   gctggtgacg gacggggccc gggcctggag ccaggagggg ccgcaccggc agcaccaggg 10440
   ggcagagcac ccccagcccc acaccctctt tgtgggcggc ctcccggcca gcagccacag 10500
   ctccaaactt ccggtgaccg tcgggttcag cggctgtgtg aagagactga ggctgcacgg 10560
   gaggcccctg gggcccccca cacggatggc aggggtcaca ccctgcatct tgggccccct 10620
   ggaggcgggc ctgttcttcc caggcagcgg gggagttatc actttagacc tcccaggagc 10680
   tacactgcct gatgtgggcc tggaactgga ggtgcggccc ctggcagtca ccggactgat 10740
   cttccacttg ggccaggccc ggacgccccc ctacttgcag ttgcaggtga ccgagaagca 10800
   agtcctgctg cgggcggatg acggagcagg ggagttctcc acgtcagtga cccgcccctc 10860
   agtgctgtgt gatggccagt ggcaccggct agcggtgatg aaaagcggga atgtgctccg 10920
   gctggaggtg gacgcgcaga gcaaccacac cgtgggcccc ttgctggcgg ctgcagctgg 10980
   tgccccagcc cctctgtacc tcgggggcct gcctgagccc atggccgtgc agccctggcc 11040
   ccccgcctac tgcggctgca tgaggaggct ggcggtgaac cggtcccccg tcgccatgac 11100
   tcgctctgtg gaggtccacg ggcagtgggg ggccagtggc tgcccagccg cctaggacac 11160
   agccaacccc ggcccctggt caggcccctg cagctgcctc acaccgcccc ttgtgctcgc 11220
   ctcataggtg tctatttgga ctctaagctc tacgggtgac agatcttgtt tctgaagatg 11280
   gtttaagtta tagcttctta aacgaaagaa taaaatactg caaaatgttt ttatatttgg 11340
   cccttccacc catttttaat tgtgagagat ttgtcaccaa tcatcactgg ttcctcctta 11400
   aaaattaaaa agtaacttct gtgtaa                                      11426
<210> 138
   <211> 6930
   <212> DNA
   <213> Homo sapiens
<220>
   <223> myosin, heavy chain 14, non-muscle transcript variant 3 (MYH14), nonmuscle myosin heavy chain II-C (NMHC II-C, NMHC-II-C), myosin-14, MYH14 variant protein, DFNA4, DFNA4A, FP17425, MHC16, MYH17, PNMHH
<220>
   <221> CDS
   <222> (48) ... (6158)
   <223> MYH14
<400> 138
   ctctttctcc ccaggccgaa gcctcgggac ggccctggaa gccgaccatg gcagccgtga 60
   ccatgtcggt gcccgggcgg aaggcgcccc ccaggccggg cccagtgccc gaggcggccc 120
   agccgttcct gttcacgccc cgcgggccca gcgcgggtgg cgggcctggc tcgggcacct 180
   ccccgcaggt ggagtggacg gcccggcgtc tcgtgtgggt gccttcggag cttcacgggt 240
   tcgaggcggc ggcgctgcgg gacgaaggcg aggaggaggc ggaggtggag ctggcggaga 300
   gcgggaggcg gctgcgactg ccgcgggacc agatccagcg catgaacccg cccaagttca 360
   gcaaggccga ggacatggcc gagctgacct gcctcaacga ggcctcggtc ctgcacaacc 420
   tccgggagcg gtactactcc ggcctcatct acacgtactc cggccttttc tgtgtggtca 480
   tcaacccgta caagcagctt cccatctaca cagaagccat tgtggagatg taccggggca 540
   agaagcgcca cgaggtgcca ccccacgtgt acgcagtgac cgagggggcc tatcggagca 600
   tgctgcagga tcgtgaggac cagtccattc tctgcactgg agagtctgga gctgggaaga 660
   cggaaaacac caagaaggtc atccagtacc tcgcccacgt ggcgtcgtct ccaaagggca 720
   ggaaggagcc gggtgtcccc gcctccgtca gcaccgtgtc ttatggtgag ctggagcggc 780
   agctgcttca ggccaacccc atcctagagg cctttggcaa tgccaagaca gtgaagaatg 840
   acaactcctc ccgattcggc aaattcatcc gcatcaactt tgatgttgcc gggtacatcg 900
   tgggcgccaa cattgagacc tacctgctgg agaagtcgcg ggccatccgc caggccaagg 960
   acgagtgcag cttccacatc ttctaccagc tgctgggggg cgctggagag cagctcaaag 1020
   ccgacctcct cctcgagccc tgctcccact accggttcct gaccaacggg ccgtcatcct 1080
   ctcccggcca ggagcgggaa ctcttccagg agacgctgga gtcgctgcgg gtcctgggat 1140
   tcagccacga ggaaatcatc tccatgctgc ggatggtctc agcagttctc cagtttggca 1200
   acattgcctt gaagagagaa cggaacaccg atcaagccac catgcctgac aacacagctg 1260
   cacagaagct ctgccgcctc ttgggactgg gggtgacgga tttctcccga gccttgctca 1320
   cccctcgcat caaagttggc cgagactatg tgcagaaagc ccagactaag gaacaggctg 1380
   acttcgcgct ggaggccctg gccaaggcca cctacgagcg cctcttccgc tggctggttc 1440
   tgcgcctcaa ccgggccttg gaccgcagcc cccgccaagg cgcctccttc ctgggcatcc 1500
   tggacatcgc gggctttgag atcttccagc tgaactcctt cgagcagctc tgcatcaact 1560
   acaccaacga gaagctgcag cagctcttca accacaccat gttcgtgctg gagcaggagg 1620
   agtaccagcg tgagggcatc ccctggacct tcctcgactt tggcctcgac ctgcagccct 1680
   gcatcgacct catcgagcgg ccggccaacc cccctggact cctggccctg ctggatgagg 1740
   agtgctggtt cccgaaggcc acagacaagt cgtttgtgga gaaggtagcc caggagcagg 1800
   gcggccaccc caagttccag cggccgaggc acctgcggga tcaggccgac ttcagtgttc 1860
   tccactacgc gggcaaggtc gactacaagg ccaacgagtg gctgatgaaa aacatggacc 1920
   ctctgaatga caacgtcgca gccttgctcc accagagcac agaccggctg acggcagaga 1980
   tctggaaaga cgaacatggg ggcttccagc agttctcttt ccttggctcc ttcccaccgt 2040
   cgcccccagg atctgcagag aggtgcagct ctgctatttc tccgccaggg gtggagggca 2100
   tcgtggggct ggaacaggtg agcagcctgg gcgacggccc accaggtggc cgcccccgtc 2160
   ggggtatgtt ccggacagtg ggacagctct acaaggagtc cctgagccgc ctcatggcca 2220
   cactcagcaa caccaacccc agttttgtcc gctgcattgt ccccaaccac gagaagaggg 2280
   ccgggaagct ggagccacgg ctggtgctgg accagcttcg ctgcaacggg gtcctggagg 2340
   gcatccgcat ctgtcgccag ggcttcccca accgcatcct cttccaggag ttccggcagc 2400
   gatacgagat cctgacaccc aatgccatcc ccaagggctt catggatggg aagcaggcct 2460
   gtgaaaagat gatccaggcg ctggaactgg accccaacct ctaccgcgtg ggacagagca 2520
   agatcttctt ccgggctggg gtcctggccc agctggaaga ggagcgagac ctgaaggtca 2580
   ccgacatcat cgtctccttc caggcagctg cccggggata cctggctcgc agggccttcc 2640
   agaagcgcca gcagcagcag agcgccctga gggtgatgca gcggaactgc gcggcctacc 2700
   tcaagctgag acactggcag tggtggcggc tgtttaccaa ggtgaagcca ctgctgcagg 2760
   tgacgcggca ggatgaggtg ctgcaggcac gggcccagga gctgcagaaa gtgcaggagc 2820
   tacagcagca gagcgcccgc gaagttgggg agctccaggg ccgagtggca cagctggaag 2880
   aggagcgcgc ccgcctggca gagcaattgc gagcagaggc agaactgtgt gcagaggccg 2940
   aggagacgcg ggggaggctg gcagcccgca agcaggagct ggagctggtg gtgtcagagc 3000
   tggaggctcg cgtgggcgag gaggaggagt gcagccgtca aatgcaaacc gagaagaaga 3060
   ggctgcagca gcacatacag gagctagagg cccaccttga ggctgaggag ggtgcgcggc 3120
   agaagctgca gctggagaag gtgacgacag aggcaaaaat gaagaaattt gaagaggacc 3180
   tgctgctcct ggaagaccag aattccaagc tgagcaagga gcggaagctg ctggaagatc 3240
   gtctggccga gttctcatcc caggcagctg aggaggagga gaaggtcaag agcctcaata 3300
   agctacggct caaatatgag gccacaatcg cagacatgga ggaccgccta cggaaggagg 3360
   agaagggtcg ccaggagctg gagaagctga agcggaggct ggatggggag agctcagagc 3420
   tgcaggagca gatggtggag cagcaacagc gggcagagga gctgcgggcc cagctgggcc 3480
   ggaaggagga ggagctgcag gctgccctgg ccagggcaga agacgagggt ggggcccggg 3540
   cccagctgct gaaatccctg cgggaggctc aagcagccct ggccgaggcc caggaggacc 3600
   tggagtctga gcgtgtggcc aggaccaagg cggagaagca gcgccgggac ctgggcgagg 3660
   agctggaggc gctgcggggc gagctggagg acacgctgga ctccaccaac gcacagcagg 3720
   agctccggtc caagagggaa caggaggtga cggagctgaa gaagactctg gaggaggaga 3780
   ctcgcatcca cgaggcggca gtgcaggagc tgaggcagcg ccacggccag gccctggggg 3840
   agctggcgga gcagctggag caggcccgga ggggcaaagg tgcatgggag aagacccggc 3900
   tggccctgga ggccgaggtg tccgagctgc gggcagaact gagcagcctg cagactgcac 3960
   gtcaggaggg tgagcagcgg aggcgccgcc tggagttaca gctgcaggag gtgcagggcc 4020
   gggctggtga tggggagagg gcacgagcgg aggctgctga gaagctgcag cgagcccagg 4080
   ctgaactgga gaatgtgtct ggggcgctga acgaggctga gtccaaaacc atccgtctta 4140
   gcaaggagct gagcagcaca gaagcccagc tgcacgatgc ccaggagctg ctgcaggagg 4200
   agaccagggc gaaattggcc ttggggtccc gggtgcgagc catggaggct gaggcagccg 4260
   ggctgcgtga gcagctggag gaggaggcag ctgccaggga acgggcgggc cgtgaactgc 4320
   agactgccca ggcccagctt tccgagtggc ggcggcgcca ggaggaggag gcaggggcac 4380
   tggaggcagg ggaggaggca cggcgccggg cagcccggga ggccgaggcc ctgacccagc 4440
   gcctggcaga aaagacagag accgtggatc ggctggagcg gggccgccgc cggctgcagc 4500
   aggagctgga cgacgccacc atggacctgg agcagcagcg gcagcttgtg agcaccctgg 4560
   agaagaagca gcgcaagttt gaccagcttc tggcagagga gaaggcagct gtacttcggg 4620
   cagtggagga acgtgagcgg gccgaggcag agggccggga gcgtgaggct cgggccctgt 4680
   cactgacacg ggcactggag gaggagcagg aggcacgtga ggagctggag cggcagaacc 4740
   gggccctgcg ggctgagctg gaggcactgc tgagcagcaa ggatgacgtc ggcaagagcg 4800
   tgcatgagct ggaacgagcc tgccgggtag cagaacaggc agccaatgat ctgcgagcac 4860
   aggtgacaga actggaggat gagctgacag cggccgagga tgccaagctg cgtctggagg 4920
   tgactgtgca ggctctcaag actcagcatg agcgtgacct gcagggccgt gatgaggctg 4980
   gtgaagagag gcggaggcag ctggccaagc agctgagaga tgcagaggtg gagcgggatg 5040
   aggagcggaa gcagcgcact ctggccgtgg ctgcccgcaa gaagctggag ggagagctgg 5100
   aggagctgaa ggctcagatg gcctctgccg gccagggcaa ggaggaggcg gtgaagcagc 5160
   ttcgcaagat gcaggcccag atgaaggagc tatggcggga ggtggaggag acacgcacct 5220
   cccgggagga gatcttctcc cagaatcggg aaagtgaaaa gcgcctcaag ggcctggagg 5280
   ctgaggtgct gcggctgcag gaggaactgg ccgcctcgga ccgtgctcgg cggcaggccc 5340
   agcaggaccg ggatgagatg gcagatgagg tggccaatgg taaccttagc aaggcagcca 5400
   ttctggagga gaagcgtcag ctggaggggc gcctggggca gttggaggaa gagctggagg 5460
   aggagcagag caactcggag ctgctcaatg accgctaccg caagctgctc ctgcaggtag 5520
   agtcactgac cacagagctg tcagctgagc gcagtttctc agccaaggca gagagcgggc 5580
   ggcagcagct ggaacggcag atccaggagc tacggggacg cctgggtgag gaggatgctg 5640
   gggcccgtgc ccgccacaag atgaccattg ctgcccttga gtctaagttg gcccaggctg 5700
   aggagcagct agagcaagag accagagagc gcatcctctc tggaaagctg gtgcgcagag 5760
   ctgagaagcg gcttaaagag gtggtgctcc aggtggagga ggagcggagg gtggctgacc 5820
   agctccggga ccagctggag aagggaaacc ttcgagtcaa gcagctgaag cggcagctgg 5880
   aggaggccga ggaggaggca tcccgggctc aggccggccg ccggaggctg cagcgtgagc 5940
   tggaagatgt cacagagtcg gccgagtcca tgaaccgtga agtgaccaca ctgaggaacc 6000
   ggcttcgacg cggccccctc accttcacca cccgcacggt gcgccaggtc ttccgactag 6060
   aggagggcgt ggcatccgac gaggaggcag aggaagcaca gcctgggtct gggccatccc 6120
   cggagcctga ggggtcccca ccagcccacc cccagtgacc ctaccctgtc cccagatgca 6180
   ctaacagatg gggcccagcc cccttcctcc ctggacccca cgggcccctg tcccaggaac 6240
   cccgccctct gacttcttgc cctttggaaa tggtgcagca ctctggcatt tatcaccccc 6300
   acctgggtcc cctgcaacct cccatcaaag gatgacccct aaacacagag gagcggggca 6360
   ggcagggagg caatgactgg agctaccttg cttgttgggg gactgggtac agttggcaag 6420
   ctgtgtttcc atcagctccc tgtcctcctt tcttccctcg ttattgatct atagacatta 6480
   ggaagggagt gagacggctc ctccaccatc ctcagccagt gcaacccatt ccctctgctt 6540
   ctctctctct ctctctctct ccctccctct ccttccctac cctctcacca tctttcttgg 6600
   cctctctgag ggtctctctg tgcatctttt taggaatctc gctctcactc tctacgtagc 6660
   cactctcctt cccccatttc tgcgtccacc cctgaactcc tgagcgacag aagccccagg 6720
   cctccaccag ccttgaaccc ttgcaaaggg gcaggacaag gggacccctc tcactcctgc 6780
   tgctgcccat gctctgccct cccttctggt tgctctgagg gttcggagct tccctctggg 6840
   actaaaggag tgtcctttac cctcccagcc tccaggctct ggcagaaata aactccaacc 6900
   cgactggacc ataaaaaaaa aaaaaaaaaa                                  6930
<210> 139
   <211> 1531
   <212> DNA
   <213> Homo sapiens
<220>
   <223> Rab geranylgeranyltransferase, beta subunit (RABGGTB, GGTB), geranylgeranyl transferase type-2 subunit beta, type II protein geranyl-geranyltransferase subunit beta, GGTase-II-beta; rab GGTase beta; rab GG transferase beta
<220>
   <221> CDS
   <222> (79) ... (1074)
   <223> RABGGTB
<400> 139
   gagaggcgca tctgcgcagg cgcccggctc ctaagtctac ccaggaactg accctgctct 60
   ctcctttccc tgttagacat gggcactcca cagaaggatg ttattatcaa gtcagatgca 120
   ccggacactt tgttattgga gaaacatgca gattatatcg catcctatgg ctcaaagaaa 180
   gatgattatg aatactgtat gtctgagtat ttgagaatga gtggcatcta ttggggtctg 240
   acagtaatgg atctcatggg acaacttcat cgcatgaata gagaagagat tctggcattt 300
   attaagtctt gccaacatga atgtggtgga ataagtgcta gtatcggaca tgatcctcat 360
   cttttataca ctcttagtgc tgtccagatt cttacgctgt atgacagtat taatgttatt 420
   gacgtaaata aagttgtgga atatgttaaa ggtctacaga aagaagatgg ttcttttgct 480
   ggagatattt ggggagaaat tgacacaaga ttctcttttt gtgcggtggc aactttggct 540
   ttgttgggga agcttgatgc tattaatgtg gaaaaggcaa tcgaatttgt tttatcctgt 600
   atgaactttg acggtggatt tggttgcaga ccaggttctg aatcccatgc tgggcagatc 660
   tattgttgca caggatttct ggcaattaca agtcagttgc atcaagtaaa ttctgattta 720
   cttggctggt ggctttgtga acgacaatta ccctcaggcg ggctcaatgg aaggccggag 780
   aagttaccag atgtatgcta ctcatggtgg gtcctggctt ccctaaagat aattggaaga 840
   cttcattgga ttgatagaga gaaactgcgt aatttcattt tagcatgtca agatgaagaa 900
   acggggggat ttgcagacag gccaggagat atggtggatc cttttcatac cttatttgga 960
   attgctggat tgtcactttt gggagaagaa cagattaaac ctgttaatcc tgtcttttgc 1020
   atgcctgaag aagtgcttca gagagtgaat gttcagcctg agctagtgag ctagattcat 1080
   tgaattgaaa gttgcatagt atagttttgc cattttaaca tttctgtatt tgaagtgctt 1140
   atcgaatcta aaagtgacta ctgttaatat tttgtatatt gtgttaaatt aattttaata 1200
   aattatataa ttatacatat tgtaaaataa agaccggtat tttattttct gctttttatt 1260
   ctgaagtcct gttattctga ctacagttct ttgtgtatac ttctgtgtct gttatgttca 1320
   ataactgagc taacataaaa taactctagg tttctacttg atttttcccc catgtatacc 1380
   tttcatctgt tctatagcaa gttgatgtaa attggtttgt caacaagaat gttaactgat 1440
   gaaagtggat agaacccata catgaattaa atgatgcaca aaataaatgg ctgttgaaat 1500
   ttggaaatga ttgaaaaaaa aaaaaaaaaa a                                1531
<210> 140
   <211> 514
   <212> DNA
   <213> Homo sapiens
<220>
   <223> 60S ribosomal protein L27, L27
<220>
   <221> CDS
   <222> (46) ... (456)
   <223> RPL27
<400> 140
   tccttctttc ctttttgctg gtagggccgg gtggttgctg ccgaaatggg caagttcatg 60
   aaacctggga aggtggtgct tgtcctggct ggacgctact ccggacgcaa agctgtcatc 120
   gtgaagaaca ttgatgatgg cacctcagat cgcccctaca gccatgctct ggtggctgga 180
   attgaccgct acccccgcaa agtgacagct gccatgggca agaagaagat cgccaagaga 240
   tcaaagataa aatcttttgt gaaagtgtat aactacaatc acctaatgcc cacaaggtac 300
   tctgtggata tccccttgga caaaactgtc gtcaataagg atgtcttcag agatcctgct 360
   cttaaacgca aggcccgacg ggaggccaag gtcaagtttg aagagagata caagacaggc 420
   aagaacaagt ggttcttcca gaaactgcgg ttttagatgc tttgttttga tcattaaaaa 480
   ttataaagaa aaaaaaaaaa aaaaaaaaaa aaaa                             514
<210> 141
   <211> 531
   <212> DNA
   <213> Homo sapiens
<220>
   <223> 40S ribosomal protein S15, S15, homolog of rat insulinoma (RIG), insulinoma protein
<220>
   <221> CDS
   <222> (63) ... (500)
   <223> RPS15
<400> 141
   ggcagtctcg cgataactgc gcaggcgcgg accaaagcga tctcttctga ggatccggca 60
   agatggcaga agtagagcag aagaagaagc ggaccttccg caagttcacc taccgcggcg 120
   tggacctcga ccagctgctg gacatgtcct acgagcagct gatgcagctg tacagtgcgc 180
   gccagcggcg gcggctgaac cggggcctgc ggcggaagca gcactccctg ctgaagcgcc 240
   tgcgcaaggc caagaaggag gcgccgccca tggagaagcc ggaagtggtg aagacgcacc 300
   tgcgggacat gatcatccta cccgagatgg tgggcagcat ggtgggcgtc tacaacggca 360
   agaccttcaa ccaggtggag atcaagcccg agatgatcgg ccactacctg ggcgagttct 420
   ccatcaccta caagcccgta aagcatggcc ggcccggcat cggggccacc cactcctccc 480
   gcttcatccc tctcaagtaa tggctcagct aataaaggcg cacatgactc c          531
<210> 142
   <211> 1712
   <212> DNA
   <213> Homo sapiens
<220>
   <223> inosine-5'-monophosphate dehydrogenase 2, inosine monophosphate dehydrogenase type II, IMP (inosine monophosphate) dehydrogenase 2, IMP (inosine monophosphate) dehydrogenase 2, IMP oxireductase 2 (IMPDH2, IMPD2, IMPDH-II, IMPD 2; IMPDH 2)
<220>
   <221> CDS
   <222> (93) ... (1637)
   <223> IMPDH2
<400> 142
   cgaaatcggc tggtttatat tggcgcggcc cagacggcag aggtctctgc ggcgcggtcc 60
   tcggagacac gcggcggtgt cctgtgttgg ccatggccga ctacctgatt agtgggggca 120
   cgtcctacgt gccagacgac ggactcacag cacagcagct cttcaactgc ggagacggcc 180
   tcacctacaa tgactttctc attctccctg ggtacatcga cttcactgca gaccaggtgg 240
   acctgacttc tgctctgacc aagaaaatca ctcttaagac cccactggtt tcctctccca 300
   tggacacagt cacagaggct gggatggcca tagcaatggc gcttacaggc ggtattggct 360
   tcatccacca caactgtaca cctgaattcc aggccaatga agttcggaaa gtgaagaaat 420
   atgaacaggg attcatcaca gaccctgtgg tcctcagccc caaggatcgc gtgcgggatg 480
   tttttgaggc caaggcccgg catggtttct gcggtatccc aatcacagac acaggccgga 540
   tggggagccg cttggtgggc atcatctcct ccagggacat tgattttctc aaagaggagg 600
   aacatgactg tttcttggaa gagataatga caaagaggga agacttggtg gtagcccctg 660
   caggcatcac actgaaggag gcaaatgaaa ttctgcagcg cagcaagaag ggaaagttgc 720
   ccattgtaaa tgaagatgat gagcttgtgg ccatcattgc ccggacagac ctgaagaaga 780
   atcgggacta cccactagcc tccaaagatg ccaagaaaca gctgctgtgt ggggcagcca 840
   ttggcactca tgaggatgac aagtataggc tggacttgct cgcccaggct ggtgtggatg 900
   tagtggtttt ggactcttcc cagggaaatt ccatcttcca gatcaatatg atcaagtaca 960
   tcaaagacaa ataccctaat ctccaagtca ttggaggcaa tgtggtcact gctgcccagg 1020
   ccaagaacct cattgatgca ggtgtggatg ccctgcgggt gggcatggga agtggctcca 1080
   tctgcattac gcaggaagtg ctggcctgtg ggcggcccca agcaacagca gtgtacaagg 1140
   tgtcagagta tgcacggcgc tttggtgttc cggtcattgc tgatggagga atccaaaatg 1200
   tgggtcatat tgcgaaagcc ttggcccttg gggcctccac agtcatgatg ggctctctcc 1260
   tggctgccac cactgaggcc cctggtgaat acttcttttc cgatgggatc cggctaaaga 1320
   aatatcgcgg tatgggttct ctcgatgcca tggacaagca cctcagcagc cagaacagat 1380
   atttcagtga agctgacaaa atcaaagtgg cccagggagt gtctggtgct gtgcaggaca 1440
   aagggtcaat ccacaaattt gtcccttacc tgattgctgg catccaacac tcatgccagg 1500
   acattggtgc caagagcttg acccaagtcc gagccatgat gtactctggg gagcttaagt 1560
   ttgagaagag aacgtcctca gcccaggtgg aaggtggcgt ccatagcctc cattcgtatg 1620
   agaagcggct tttctgaaaa gggatccagc acacctcctc ggtttttttt tcaataaaag 1680
   tttagaaaga aaaaaaaaaa aaaaaaaaaa aa                               1712
<210> 143
   <211> 1655
   <212> DNA
   <213> Homo sapiens
<220>
   <223> sigma non-opioid intracellular receptor 1 transcript variant 1, sigma 1-type opioid receptor (SIGMAR1, hSigmaR1, SIG-1R, sigma1R), SR31747 binding protein 1 (SR-BP1, SR-BP, SRBP), aging-associated gene 8 protein, ALS16
<220>
   <221> CDS
   <222> (75) ... (746)
   <223> SIGMAR1
<400> 143
   ctccgaggcc gtgagcgcaa agcctcaggc cccggctccc tcctgagctg cgccgtgcca 60
   ggccgcccgc cgggatgcag tgggccgtgg gccggcggtg ggcgtgggcc gcgctgctcc 120
   tggctgtcgc agcggtgctg acccaggtcg tctggctctg gctgggtacg cagagcttcg 180
   tcttccagcg cgaagagata gcgcagttgg cgcggcagta cgctgggctg gaccacgagc 240
   tggccttctc tcgtctgatc gtggagctgc ggcggctgca cccaggccac gtgctgcccg 300
   acgaggagct gcagtgggtg ttcgtgaatg cgggtggctg gatgggcgcc atgtgccttc 360
   tgcacgcctc gctgtccgag tatgtgctgc tcttcggcac cgccttgggc tcccgcggcc 420
   actcggggcg ctactgggct gagatctcgg ataccatcat ctctggcacc ttccaccagt 480
   ggagagaggg caccaccaaa agtgaggtct tctacccagg ggagacggta gtacacgggc 540
   ctggtgaggc aacagctgtg gagtgggggc caaacacatg gatggtggag tacggccggg 600
   gcgtcatccc atccaccctg gccttcgcgc tggccgacac tgtcttcagc acccaggact 660
   tcctcaccct cttctatact cttcgctcct atgctcgggg cctccggctt gagctcacca 720
   cctacctctt tggccaggac ccttgaccag ccaggcctga aggaagacct gcggatggac 780
   aggagcgggc aggcccgcac atatccactt gctggagccc atgtttacag acagggacat 840
   acaccatgca gatcctgagt tcctgctgta tgagcaggga tatccatgct tatgtatcca 900
   aacacagaga cccatgggaa caaatgagac acatatagat actgagacct gtgtgtacag 960
   taggaccatg cactcacacc catctggaga gggagccccc ggtataccaa gggagccagt 1020
   tgtgttcaga cacacacatc acagcttgac tcactaactg aggcctttcc atagctccac 1080
   agcttcccac ctcctcccca ccaaaccggg gttctagagt taaggatggg ggagggtatt 1140
   atactgcctc agtctgactc ctcaacccag cagcaatttg aggggatgag ggggaagagg 1200
   agctgccttt tggaggcccc cttcacctgc agctatgatg cccttcccct tctcccctgt 1260
   cctcaccata tgccttatcc ccattctact cccctgctat gcaagtgccc ctgtggcttg 1320
   tccccaaccc cctcagcaac aaagctcagc tggggaacga gagtaatttg aagaatgctt 1380
   gaagtcagcg tcttccattc cagaaagacc cccattcttc ctttgggggt atgatgtgga 1440
   agctggtttc agcccaggac ccaccactga ggagaggatc tagacaggtg ggcctaattc 1500
   caaggggccc ttcctggcct ggagaaggcc ttttacacac acacaacaca tacacacaca 1560
   cacacacaca cacatatcac agttttcaca cagcccctgc tgcattctct gtccatctgt 1620
   ctgtttctat taataaagat ttgttgatct gttcc                            1655
<210> 144
   <211> 898
   <212> DNA
   <213> Homo sapiens
<220>
   <223> ATP synthase, H+ transporting, mitochondrial F0 complex, subunit C2 (subunit 9) transcript variant 2, ATP synthase F(0) complex subunit C2, mitochondrial precursor (ATP5G2, ATP5A), ATP synthase lipid-binding protein, mitochondrial, ATP synthase proteolipid P2
<220>
   <221> CDS
   <222> (134) ... (730)
   <223> ATP5G2
<400> 144
   ccacgttacg gatcggctta ctccgcggag ttggcctcat ttctgcagtc ggcgctccct 60
   gtagtttctc ctctcgaacg ccaggtggag caaccggccg gataccgcca cagccctggc 120
   aggcggcgct gtgatgcctg agctgatcct gtatgttgca atcactctat ccgtggctga 180
   gcgactcgtt ggcccgggtc acgcatgcgc tgagccttcc tttcgctctt cccgctgctc 240
   cgcccctctc tgtcttctct gcagtgggag cagctctcct gccacagctc ctcaccccct 300
   gaaaatgttc gcctgctcca agtttgtctc cactccctcc ttggtcaaga gcacctcaca 360
   gctgctgagc cgtccgctat ctgcagtggt gctgaaacga ccggagatac tgacagatga 420
   gagcctcagc agcttggcag tctcatgtcc ccttacctca cttgtctcta gccgcagctt 480
   ccaaaccagc gccatttcaa gggacatcga cacagcagcc aagttcattg gagctggggc 540
   tgccacagtt ggggtggctg gttctggggc tgggattgga actgtgtttg ggagcctcat 600
   cattggttat gccaggaacc cttctctgaa gcaacagctc ttctcctacg ccattctggg 660
   ctttgccctc tcggaggcca tggggctctt ttgtctgatg gtagcctttc tcatcctctt 720
   tgccatgtga aggagccgtc tccacctccc atagttctcc cgcgtctggt tggccccgtg 780
   tgttcctttt cctatacctc cccaggcagc ctggggaacg tggttggctc agggtttgac 840
   agagaaaaga caaataaata ctgtattaat aagatgtttc ttgaaaaaaa aaaaaaaa   898
<210> 145
   <211> 15
   <212> RNA
   <213> Artificial Sequence
<220>
   <223> synthetic 5' untranslated region (5'UTR) consensus sequence pyrimidine rich translational element (PRTE)
<400> 145
   cuuccuuucc cugcu 15
<210> 146
   <211> 26
   <212> DNA
   <213> Artificial Sequence
<220>
   <223> synthetic YB1 5'UTR cloning oligonucleotide deletion (20-34) forward
<400> 146
   gctacaagct tgggcttatc ccgcct 26
<210> 147
   <211> 26
   <212> DNA
   <213> Artificial Sequence
<220>
   <223> synthetic YB1 5'UTR cloning oligonucleotide deletion (20-34) reverse
<400> 147
   tcgatccatg gggttgcggt gatggt 26
<210> 148
   <211> 40
   <212> DNA
   <213> Artificial Sequence
<220>
   <223> synthetic site-directed mutagenesis oligonucleotide deletion (20-34) forward
<400> 148
   tgggcttatc ccgcctgtcc ttcgatcggt agcgggagcg 40
<210> 149
   <211> 40
   <212> DNA
   <213> Artificial Sequence
<220>
   <223> synthetic site-directed mutagenesis oligonucleotide deletion (20-34) reverse
<400> 149
   cgctcccgct accgatcgaa ggacaggcgg gataagccca 40
<210> 150
   <211> 55
   <212> DNA
   <213> Artificial Sequence
<220>
   <223> synthetic site-directed mutagenesis oligonucleotide transversion (20-34) forward
<400> 150
   tgggcttatc ccgcctgtcc gcggtaagag cgatcttcga tcggtagcgg gagcg 55
<210> 151
   <211> 55
   <212> DNA
   <213> Artificial Sequence
<220>
   <223> synthetic site-directed mutagenesis oligonucleotide transversion (20-34) reverse
<400> 151
   cgctcccgct accgatcgaa gatcgctctt accgcggaca ggcgggataa gccca 55
<210> 152
   <211> 20
   <212> DNA
   <213> Artificial Sequence
<220>
   <223> synthetic human qPCR oligonucleotide beta-actin forward
<400> 152
   gcaaagacct gtacgccaac 20
<210> 153
   <211> 20
   <212> DNA
   <213> Artificial Sequence
<220>
   <223> synthetic human qPCR oligonucleotide beta-actin reverse
<400> 153
   agtacttgcg ctcaggagga 20
<210> 154
   <211> 20
   <212> DNA
   <213> Artificial Sequence
<220>
   <223> synthetic human qPCR oligonucleotide CD44 forward
<400> 154
   caacaacaca aatggctggt 20
<210> 155
   <211> 24
   <212> DNA
   <213> Artificial Sequence
<220>
   <223> synthetic human qPCR oligonucleotide CD44 reverse
<400> 155
   ctgaggtgtc tgtctctttc atct 24
<210> 156
   <211> 20
   <212> DNA
   <213> Artificial Sequence
<220>
   <223> synthetic human qPCR oligonucleotide vimentin forward
<400> 156
   ggcccagctg taagttggta 20
<210> 157
   <211> 18
   <212> DNA
   <213> Artificial Sequence
<220>
   <223> synthetic human qPCR oligonucleotide vimentin reverse
<400> 157
   ggagcgagag tggcagag 18
<210> 158
   <211> 19
   <212> DNA
   <213> Artificial Sequence
<220>
   <223> synthetic human qPCR oligonucleotide Snail1 forward
<400> 158
   cactatgccg cgctctttc 19
<210> 159
   <211> 25
   <212> DNA
   <213> Artificial Sequence
<220>
   <223> synthetic human qPCR oligonucleotide Snail1 reverse
<400> 159
   gctggaaggt aaactctgga ttaga 25
<210> 160
   <211> 21
   <212> DNA
   <213> Artificial Sequence
<220>
   <223> synthetic human qPCR oligonucleotide YB1 forward
<400> 160
   tcgccaaaga cagcctagag a 21
<210> 161
   <211> 22
   <212> DNA
   <213> Artificial Sequence
<220>
   <223> synthetic human qPCR oligonucleotide YB1 reverse
<400> 161
   tctgcgtcgg taattgaagt tg 22
<210> 162
   <211> 21
   <212> DNA
   <213> Artificial Sequence
<220>
   <223> synthetic human qPCR oligonucleotide MTA1 forward
<400> 162
   caaagtggtg tgcttctacc g 21
<210> 163
   <211> 21
   <212> DNA
   <213> Artificial Sequence
<220>
   <223> synthetic human qPCR oligonucleotide MTA1 reverse
<400> 163
   cggccttata gcagactgac a 21
<210> 164
   <211> 20
   <212> DNA
   <213> Artificial Sequence
<220>
   <223> synthetic human qPCR oligonucleotide PLAU forward
<400> 164
   ttgctcacca caacgacatt 20
<210> 165
   <211> 20
   <212> DNA
   <213> Artificial Sequence
<220>
   <223> synthetic human qPCR oligonucleotide PLAU reverse
<400> 165
   ggcaggcaga tggtctgtat 20
<210> 166
   <211> 19
   <212> DNA
   <213> Artificial Sequence
<220>
   <223> synthetic human qPCR oligonucleotide FGFBP1 forward
<400> 166
   actggatccg tgtgctcag 19
<210> 167
   <211> 20
   <212> DNA
   <213> Artificial Sequence
<220>
   <223> synthetic human qPCR oligonucleotide FGFBP1 reverse
<400> 167
   gagcagggtg aggctacaga 20
<210> 168
   <211> 23
   <212> DNA
   <213> Artificial Sequence
<220>
   <223> synthetic human qPCR oligonucleotide ARID5B forward
<400> 168
   tggactcaac ttcaaagacg ttc 23
<210> 169
   <211> 20
   <212> DNA
   <213> Artificial Sequence
<220>
   <223> synthetic human qPCR oligonucleotide ARID5B reverse
<400> 169
   acgttcgttt cttcctcgtc 20
<210> 170
   <211> 20
   <212> DNA
   <213> Artificial Sequence
<220>
   <223> synthetic human qPCR oligonucleotide CTGF forward
<400> 170
   ctcctgcagg ctagagaagc 20
<210> 171
   <211> 21
   <212> DNA
   <213> Artificial Sequence
<220>
   <223> synthetic human qPCR oligonucleotide CTGF reverse
<400> 171
   gatgcacttt ttgcccttct t 21
<210> 172
   <211> 19
   <212> DNA
   <213> Artificial Sequence
<220>
   <223> synthetic human qPCR oligonucleotide RND3 forward
<400> 172
   aaaaactgcg ctgctccat 19
<210> 173
   <211> 21
   <212> DNA
   <213> Artificial Sequence
<220>
   <223> synthetic human qPCR oligonucleotide RND3 reverse
<400> 173
   tcaaaactgg ccgtgtaatt c 21
<210> 174
   <211> 20
   <212> DNA
   <213> Artificial Sequence
<220>
   <223> synthetic human qPCR oligonucleotide KLF6 forward
<400> 174
   aaagctccca cttgaaagca 20
<210> 175
   <211> 21
   <212> DNA
   <213> Artificial Sequence
<220>
   <223> synthetic human qPCR oligonucleotide KLF6 reverse
<400> 175
   ccttcccatg agcatctgta a 21
<210> 176
   <211> 18
   <212> DNA
   <213> Artificial Sequence
<220>
   <223> synthetic human qPCR oligonucleotide BCL6 forward
<400> 176
   ttccgctaca agggcaac 18
<210> 177
   <211> 20
   <212> DNA
   <213> Artificial Sequence
<220>
   <223> synthetic human qPCR oligonucleotide BCL6 reverse
<400> 177
   tgcaacgata gggtttctca 20
<210> 178
   <211> 20
   <212> DNA
   <213> Artificial Sequence
<220>
   <223> synthetic human qPCR oligonucleotide FOXA1 forward
<400> 178
   agggctggat ggttgtattg 20
<210> 179
   <211> 18
   <212> DNA
   <213> Artificial Sequence
<220>
   <223> synthetic human qPCR oligonucleotide FOXA1 reverse
<400> 179
   accgggacgg aggagtag 18
<210> 180
   <211> 18
   <212> DNA
   <213> Artificial Sequence
<220>
   <223> synthetic human qPCR oligonucleotide GDF15 forward
<400> 180
   ccggatactc acgccaga 18
<210> 181
   <211> 20
   <212> DNA
   <213> Artificial Sequence
<220>
   <223> synthetic human qPCR oligonucleotide GDF15 reverse
<400> 181
   agagatacgc aggtgcaggt 20
<210> 182
   <211> 18
   <212> DNA
   <213> Artificial Sequence
<220>
   <223> synthetic human qPCR oligonucleotide HBP1 forward
<400> 182
   gctggtggtg ttgtcgtg 18
<210> 183
   <211> 22
   <212> DNA
   <213> Artificial Sequence
<220>
   <223> synthetic human qPCR oligonucleotide HBP1 reverse
<400> 183
   catgttatgg tgctctgact gc 22
<210> 184
   <211> 20
   <212> DNA
   <213> Artificial Sequence
<220>
   <223> synthetic human qPCR oligonucleotide Twist1 forward
<400> 184
   catcctcaca cctctgcatt 20
<210> 185
   <211> 20
   <212> DNA
   <213> Artificial Sequence
<220>
   <223> synthetic human qPCR oligonucleotide Twist1 reverse
<400> 185
   ttcctttcag tggctgattg 20
<210> 186
   <211> 23
   <212> DNA
   <213> Artificial Sequence
<220>
   <223> synthetic human qPCR oligonucleotide LEF1 forward
<400> 186
   ccttggtgaa cgagtctgaa atc 23
<210> 187
   <211> 20
   <212> DNA
   <213> Artificial Sequence
<220>
   <223> synthetic human qPCR oligonucleotide LEF1 reverse
<400> 187
   gaggtttgtg cttgtctggc 20
<210> 188
   <211> 20
   <212> DNA
   <213> Artificial Sequence
<220>
   <223> synthetic human qPCR oligonucleotide rpS19 forward
<400> 188
   gctggccaaa cataaagagc 20
<210> 189
   <211> 20
   <212> DNA
   <213> Artificial Sequence
<220>
   <223> synthetic human qPCR oligonucleotide rpS19 reverse
<400> 189
   ctgggtctga caccgtttct 20
<210> 190
   <211> 19
   <212> DNA
   <213> Artificial Sequence
<220>
   <223> synthetic human qPCR oligonucleotide 5S rRNA forward
<400> 190
   gcccgatctc gtctgatct 19
<210> 191
   <211> 20
   <212> DNA
   <213> Artificial Sequence
<220>
   <223> synthetic human qPCR oligonucleotide 5S rRNA reverse
<400> 191
   agcctacagc acccggtatt 20
<210> 192
   <211> 20
   <212> DNA
   <213> Artificial Sequence
<220>
   <223> synthetic human qPCR oligonucleotide firefly luciferase forward
<400> 192
   aatcaaagag gcgaactgtg 20
<210> 193
   <211> 20
   <212> DNA
   <213> Artificial Sequence
<220>
   <223> synthetic human qPCR oligonucleotide firefly luciferase reverse
<400> 193
   ttcgtcttcg tcccagtaag 20
<210> 194
   <211> 20
   <212> DNA
   <213> Artificial Sequence
<220>
   <223> synthetic mouse qPCR oligonucleotide beta actin forward
<400> 194
   ctaaggccaa ccgtgaaaag 20
<210> 195
   <211> 20
   <212> DNA
   <213> Artificial Sequence
<220>
   <223> synthetic mouse qPCR oligonucleotide beta actin reverse
<400> 195
   accagaggca tacagggaca 20
<210> 196
   <211> 20
   <212> DNA
   <213> Artificial Sequence
<220>
   <223> synthetic mouse qPCR oligonucleotide Yb1 forward
<400> 196
   gggttacaga ccacgattcc 20
<210> 197
   <211> 18
   <212> DNA
   <213> Artificial Sequence
<220>
   <223> synthetic mouse qPCR oligonucleotide Yb1 reverse
<400> 197
   ggcgataccg acgttgag 18
<210> 198
   <211> 20
   <212> DNA
   <213> Artificial Sequence
<220>
   <223> synthetic mouse qPCR oligonucleotide vimentin forward
<400> 198
   tccagcagct tcctgtaggt 20
<210> 199
   <211> 20
   <212> DNA
   <213> Artificial Sequence
<220>
   <223> synthetic mouse qPCR oligonucleotide vimentin reverse
<400> 199
   ccctcacctg tgaagtggat 20
<210> 200
   <211> 20
   <212> DNA
   <213> Artificial Sequence
<220>
   <223> synthetic mouse qPCR oligonucleotide Cd44 forward
<400> 200
   acagtacctt acccaccatg 20
<210> 201
   <211> 20
   <212> DNA
   <213> Artificial Sequence
<220>
   <223> synthetic mouse qPCR oligonucleotide Cd44 reverse
<400> 201
   ggatgaatcc tcggaattac 20
<210> 202
   <211> 20
   <212> DNA
   <213> Artificial Sequence
<220>
   <223> synthetic mouse qPCR oligonucleotide Mta1 forward
<400> 202
   agtgcgccta atccgtggtg 20
<210> 203
   <211> 22
   <212> DNA
   <213> Artificial Sequence
<220>
   <223> synthetic mouse qPCR oligonucleotide Mta1 reverse
<400> 203
   ctgaggatga gagcagcttt cg 22
<210> 204
   <211> 19
   <212> RNA
   <213> Artificial Sequence
<220>
   <223> synthetic siRNA/shRNA Control (D-001810-01) sequence
<400> 204
   ugguuuacau gucgacuaa 19
<210> 205
   <211> 19
   <212> RNA
   <213> Artificial Sequence
<220>
   <223> synthetic siRNA/shRNA vimentin (L-003551) sequence
<400> 205
   ucacgaugac cuugaauaa 19
<210> 206
   <211> 19
   <212> RNA
   <213> Artificial Sequence
<220>
   <223> synthetic siRNA/shRNA vimentin (L-003551) sequence
<400> 206
   ggaaauggcu cgucaccuu 19
<210> 207
   <211> 19
   <212> RNA
   <213> Artificial Sequence
<220>
   <223> synthetic siRNA/shRNA vimentin (L-003551) sequence
<400> 207
   gagggaaacu aaucuggau 19
<210> 208
   <211> 19
   <212> RNA
   <213> Artificial Sequence
<220>
   <223> synthetic siRNA/shRNA vimentin (L-003551) sequence
<400> 208
   uuaagacggu ugaaacuag 19
<210> 209
   <211> 19
   <212> RNA
   <213> Artificial Sequence
<220>
   <223> synthetic siRNA/shRNA YB1 (L-10213) sequence
<400> 209
   cugaguaaau gccggcuua 19
<210> 210
   <211> 19
   <212> RNA
   <213> Artificial Sequence
<220>
   <223> synthetic siRNA/shRNA YB1 (L-10213) sequence
<400> 210
   cgacgcagac gcccagaaa 19
<210> 211
   <211> 19
   <212> RNA
   <213> Artificial Sequence
<220>
   <223> synthetic siRNA/shRNA YB1 (L-10213) sequence
<400> 211
   guaaggaacg gauaugguu 19
<210> 212
   <211> 19
   <212> RNA
   <213> Artificial Sequence
<220>
   <223> synthetic siRNA/shRNA YB1 (L-10213) sequence
<400> 212
   gcggaggcag caaauguua 19
<210> 213
   <211> 19
   <212> RNA
   <213> Artificial Sequence
<220>
   <223> synthetic siRNA/shRNA MTA1 (L-004127) sequence
<400> 213
   ucacggacau ucagcaaga 19
<210> 214
   <211> 19
   <212> RNA
   <213> Artificial Sequence
<220>
   <223> synthetic siRNA/shRNA MTA1 (L-004127) sequence
<400> 214
   ggaccaaacc gcaguaaca 19
<210> 215
   <211> 19
   <212> RNA
   <213> Artificial Sequence
<220>
   <223> synthetic siRNA/shRNA MTA1 (L-004127) sequence
<400> 215
   gcaucuuguu ggacauauu 19
<210> 216
   <211> 19
   <212> RNA
   <213> Artificial Sequence
<220>
   <223> synthetic siRNA/shRNA MTA1 (L-004127) sequence
<400> 216
   ccagcaucau ugaguacua 19
<210> 217
   <211> 19
   <212> RNA
   <213> Artificial Sequence
<220>
   <223> synthetic siRNA/shRNA CD44 (L-009999) sequence
<400> 217
   gaauauaacc ugccgcuuu 19
<210> 218
   <211> 19
   <212> RNA
   <213> Artificial Sequence
<220>
   <223> synthetic siRNA/shRNA CD44 (L-009999) sequence
<400> 218
   caaguggacu caacggaga 19
<210> 219
   <211> 19
   <212> RNA
   <213> Artificial Sequence
<220>
   <223> synthetic siRNA/shRNA CD44 (L-009999) sequence
<400> 219
   cgaagaaggu gugggcaga 19
<210> 220
   <211> 19
   <212> RNA
   <213> Artificial Sequence
<220>
   <223> synthetic siRNA/shRNA CD44 (L-009999) sequence
<400> 220
   gaucaacagu ggcaaugga 19
<210> 221
   <211> 19
   <212> RNA
   <213> Artificial Sequence
<220>
   <223> synthetic siRNA/shRNA 4EBP1 (L-003005) sequence
<400> 221
   cugauggagu gucggaacu 19
<210> 222
   <211> 19
   <212> RNA
   <213> Artificial Sequence
<220>
   <223> synthetic siRNA/shRNA 4EBP1 (L-003005) sequence
<400> 222
   caucuaugac cggaaauuc 19
<210> 223
   <211> 19
   <212> RNA
   <213> Artificial Sequence
<220>
   <223> synthetic siRNA/shRNA 4EBP1 (L-003005) sequence
<400> 223
   gcaauagccc agaagauaa 19
<210> 224
   <211> 19
   <212> RNA
   <213> Artificial Sequence
<220>
   <223> synthetic siRNA/shRNA 4EBP1 (L-003005) sequence
<400> 224
   gagauggaca uuuaaagca 19
<210> 225
   <211> 19
   <212> RNA
   <213> Artificial Sequence
<220>
   <223> synthetic siRNA/shRNA 4EBP2 (L-018671) sequence
<400> 225
   gcagcuaccu caugacuau 19
<210> 226
   <211> 19
   <212> RNA
   <213> Artificial Sequence
<220>
   <223> synthetic siRNA/shRNA 4EBP2 (L-018671) sequence
<400> 226
   ggaggaacuc gaaucauuu 19
<210> 227
   <211> 19
   <212> RNA
   <213> Artificial Sequence
<220>
   <223> synthetic siRNA/shRNA 4EBP2 (L-018671) sequence
<400> 227
   gcaauucucc cauggcuca 19
<210> 228
   <211> 19
   <212> RNA
   <213> Artificial Sequence
<220>
   <223> synthetic siRNA/shRNA 4EBP2 (L-018671) sequence
<400> 228
   uugaacaacu ugaacaauc 19
<210> 229
   <211> 19
   <212> RNA
   <213> Artificial Sequence
<220>
   <223> synthetic siRNA/shRNA rictor (LL-016984) sequence
<400> 229
   gacacaagca cuucgauua 19
<210> 230
   <211> 19
   <212> RNA
   <213> Artificial Sequence
<220>
   <223> synthetic siRNA/shRNA rictor (LL-016984) sequence
<400> 230
   gaagauuuau ugaguccua 19
<210> 231
   <211> 19
   <212> RNA
   <213> Artificial Sequence
<220>
   <223> synthetic siRNA/shRNA rictor (LL-016984) sequence
<400> 231
   gcgagcugau guagaauua 19
<210> 232
   <211> 19
   <212> RNA
   <213> Artificial Sequence
<220>
   <223> synthetic siRNA/shRNA rictor (LL-016984) sequence
<400> 232
   gggaauacaa cuccaaaua 19
<210> 233
   <211> 19
   <212> RNA
   <213> Artificial Sequence
<220>
   <223> synthetic siRNA/shRNA PTEN (SH-003023-01-10) sequence
<400> 233
   gctaagagag gtttccgaa 19
<210> 234
   <211> 19
   <212> RNA
   <213> Artificial Sequence
<220>
   <223> synthetic siRNA/shRNA PTEN (SH-003023-02-10) sequence
<400> 234
   agactgatgt gtatacgta 19
<210> 235
   <211> 1692
   <212> DNA
   <213> Homo sapiens
<220>
   <223> general transcription factor IIA, 1-like transcript variant 1 (GTF2A1L), TFIIA-alpha and beta-like factor (ALF)
<220>
   <221> CDS
   <222> (78) ... (1514)
   <223> GTF2A1L
<400> 235
   aaaacgtgcc cagtgtttcc ctccgagggc actgcgtgcc gcgcaggcgc aaagggccag 60
   gtgctggagg tgctgtcatg gcctgcctca acccggtgcc taaactctac agatctgtaa 120
   ttgaagatgt aattgaagga gttcggaatc tatttgctga agaaggtata gaggaacaag 180
   ttttaaaaga cttgaagcag ctctgggaaa ccaaggtttt gcagtctaaa gcaacagaag 240
   acttcttcag aaatagcatc caatcacctc tgtttactct tcagttgccg cacagcttgc 300
   accaaacatt gcaatcgtca acagcatcat tagttattcc tgctggtaga actcttccaa 360
   gttttaccac agcagaactg ggcacttcaa actccagtgc aaactttact tttcctggtt 420
   atcccattca tgtaccagca ggtgtgacac tacagactgt atctggtcac ctttataaag 480
   tcaatgtacc aattatggtg acagagactt ctggaagagc aggtattctt cagcatccaa 540
   ttcagcaagt atttcaacag cttggccagc cttcagtaat acaaactagt gttccacaat 600
   tgaatccatg gtctcttcaa gcaactactg aaaaatcaca gagaattgaa accgtgctac 660
   agcaacccgc aattctacct tctgggccag tagataggaa acacttagaa aatgccacca 720
   gtgatatact tgtatctcct ggaaatgagc ataaaatcgt gcctgaagct ttgttgtgtc 780
   atcaggaaag ttctcactat atcagtcttc caggtgttgt attttctcca caggtctctc 840
   aaacaaattc taatgtggag tcagtgctca gtggttcagc tagcatggct caaaatctgc 900
   atgatgagtc cctctccaca agccctcatg ggctctccca ccagcacgtg actgatattc 960
   agcttcatat tcttaaaaat aggatgtatg gatgtgattc tgtaaagcaa ccaagaaata 1020
   tagaggaacc cagcaacata cctgtatcag agaaggattc taattctcag gtggatttaa 1080
   gcattcgggt tactgatgat gatattggtg aaataattca agtagatgga agcggtgata 1140
   catcttccaa tgaagaaata ggaagtacaa gagatgcaga tgagaatgaa tttctaggga 1200
   atattgacgg gggagatctg aaggtacctg aagaagaagc tgacagtatt tcaaatgagg 1260
   attcagccac aaacagtagt gataatgaag accctcaagt aaacattgta gaagaggacc 1320
   ctttaaattc tggagatgat gttagtgaac aggatgtgcc agacctgttt gacacggata 1380
   atgttattgt ctgtcagtat gataagattc atcgaagcaa gaacaaatgg aaattctatt 1440
   tgaaagatgg tgttatgtgt tttggaggga gagactatgt atttgcaaaa gccattggtg 1500
   atgcagagtg gtaaaccttg tgagctcagt acatctattt tgtgaacatc agttggatta 1560
   tattgcatat tgtgaattca tttttatttt gaatatagtc cagcacagag ctgttcaaat 1620
   ttttagttca ctgtatggaa tttaataaaa ttataattca gatgcagata caattacaca 1680
   aaaaaaaaaa aa                                                     1692
<210> 236
   <211> 6860
   <212> DNA
   <213> Homo sapiens
<220>
   <223> semaphorin-6A precursor, semaphorin VIA, semaphorin-6A-1 (SEMA6A, sema VIa, SEMA6A-1, SEMA, SEMA6A1, SEMAQ), HT018, VIA
<220>
   <221> CDS
   <222> (710) ... (3802)
   <223> SEMA6A
<400> 236
   ctccgcccgg gagcggcggc ctctccggtg acagaggagt agtgagcggc acggggtgag 60
   gctgcagcca actccgctcc ccgcgcactc ggctgcccag gcgctcggaa cccagcagcg 120
   gcgctcctcc gcggtgccgg tcgcccgcga tgcccgctta gcagcgtgta gcagcggcca 180
   gcatcaccac acccgcggca ccgcgctgcc ggccgcagag ccgggccaga gccttgcacc 240
   cctgccccag cccccacccc gccccccgcc ctgaaatgac ttgttaatcg gcgcagacac 300
   caccaagggg actcaccgaa gtggaatcca agtggaattt ggatttggag aagagtttct 360
   tgaacattta ccctcttcct tgttggtttt ctttttcttt ttcttctttt tttttttggc 420
   ttcttttttc ctctcccctt ctccgctcgt cattggagat gaacacatcg cgtttgcatc 480
   ccagaaagta gtcgccgcga ctatttcccc caaagagaca agcacacatg taggaatgac 540
   aaaggcttgc gaaggagaga gcgcagcccg cggcccggag agatcccctc gataatggat 600
   tactaaatgg gatacacgct gtaccagttc gctccgagcc ccggccgcct gtccgtcgat 660
   gcaccgaaaa gggtgaagta gagaaataaa gtctccccgc tgaactacta tgaggtcaga 720
   agccttgctg ctatatttca cactgctaca ctttgctggg gctggtttcc cagaagattc 780
   tgagccaatc agtatttcgc atggcaacta tacaaaacag tatccggtgt ttgtgggcca 840
   caagccagga cggaacacca cacagaggca caggctggac atccagatga ttatgatcat 900
   gaacggaacc ctctacattg ctgctaggga ccatatttat actgttgata tagacacatc 960
   acacacggaa gaaatttatt gtagcaaaaa actgacatgg aaatctagac aggccgatgt 1020
   agacacatgc agaatgaagg gaaaacataa ggatgagtgc cacaacttta ttaaagttct 1080
   tctaaagaaa aacgatgatg cattgtttgt ctgtggaact aatgccttca acccttcctg 1140
   cagaaactat aagatggata cattggaacc attcggggat gaattcagcg gaatggccag 1200
   atgcccatat gatgccaaac atgccaacgt tgcactgttt gcagatggaa aactatactc 1260
   agccacagtg actgacttcc ttgccattga cgcagtcatt taccggagtc ttggagaaag 1320
   ccctaccctg cggaccgtca agcacgattc aaaatggttg aaagaaccat actttgttca 1380
   agccgtggat tacggagatt atatctactt cttcttcagg gaaatagcag tggagtataa 1440
   caccatggga aaggtagttt tcccaagagt ggctcaggtt tgtaagaatg atatgggagg 1500
   atctcaaaga gtcctggaga aacagtggac gtcgttcctg aaggcgcgct tgaactgctc 1560
   agttcctgga gactctcatt tttatttcaa cattctccag gcagttacag atgtgattcg 1620
   tatcaacggg cgtgatgttg tcctggcaac gttttctaca ccttataaca gcatccctgg 1680
   gtctgcagtc tgtgcctatg acatgcttga cattgccagt gtttttactg ggagattcaa 1740
   ggaacagaag tctcctgatt ccacctggac accagttcct gatgaacgag ttcctaagcc 1800
   caggccaggt tgctgtgctg gctcatcctc cttagaaaga tatgcaacct ccaatgagtt 1860
   ccctgatgat accctgaact tcatcaagac gcacccgctc atggatgagg cagtgccctc 1920
   catcttcaac aggccatggt tcctgagaac aatggtcaga taccgcctta ccaaaattgc 1980
   agtggacaca gctgctgggc catatcagaa tcacactgtg gtttttctgg gatcagagaa 2040
   gggaatcatc ttgaagtttt tggccagaat aggaaatagt ggttttctaa atgacagcct 2100
   tttcctggag gagatgagtg tttacaactc tgaaaaatgc agctatgatg gagtcgaaga 2160
   caaaaggatc atgggcatgc agctggacag agcaagcagc tctctgtatg ttgcgttctc 2220
   tacctgtgtg ataaaggttc cccttggccg gtgtgaacga catgggaagt gtaaaaaaac 2280
   ctgtattgcc tccagagacc catattgtgg atggataaag gaaggtggtg cctgcagcca 2340
   tttatcaccc aacagcagac tgacttttga gcaggacata gagcgtggca atacagatgg 2400
   tctgggggac tgtcacaatt cctttgtggc actgaatggg cattccagtt ccctcttgcc 2460
   cagcacaacc acatcagatt cgacggctca agaggggtat gagtctaggg gaggaatgct 2520
   ggactggaag catctgcttg actcacctga cagcacagac cctttggggg cagtgtcttc 2580
   ccataatcac caagacaaga agggagtgat tcgggaaagt tacctcaaag gccacgacca 2640
   gctggttccc gtcaccctct tggccattgc agtcatcctg gctttcgtca tggggggcgt 2700
   cttctcgggc atcaccgtct actgcgtctg tgatcatcgg cgcaaagacg tggctgtggt 2760
   gcagcgcaag gagaaggagc tcacccactc gcgccggggc tccatgagca gcgtcaccaa 2820
   gctcagcggc ctctttgggg acactcaatc caaagaccca aagccggagg ccatcctcac 2880
   gccactcatg cacaacggca agctcgccac tcccggcaac acggccaaga tgctcattaa 2940
   agcagaccag caccacctgg acctgacggc cctccccacc ccagagtcaa ccccaacgct 3000
   gcagcagaag cggaagccca gccgcggcag ccgcgagtgg gagaggaacc agaacctcat 3060
   caatgcctgc acaaaggaca tgccccccat gggctcccct gtgattccca cggacctgcc 3120
   cctgcgggcc tcccccagcc acatccccag cgtggtggtc ctgcccatca cgcagcaggg 3180
   ctaccagcat gagtacgtgg accagcccaa aatgagcgag gtggcccaga tggcgctgga 3240
   ggaccaggcc gccacactgg agtataagac catcaaggaa catctcagca gcaagagtcc 3300
   caaccatggg gtgaaccttg tggagaacct ggacagcctg ccccccaaag ttccacagcg 3360
   ggaggcctcc ctgggtcccc cgggagcctc cctgtctcag accggtctaa gcaagcggct 3420
   ggaaatgcac cactcctctt cctacggggt tgactataag aggagctacc ccacgaactc 3480
   gctcacgaga agccaccagg ccaccactct caaaagaaac aacactaact cctccaattc 3540
   ctctcacctc tccagaaacc agagctttgg caggggagac aacccgccgc ccgccccgca 3600
   gagggtggac tccatccagg tgcacagctc ccagccatct ggccaggccg tgactgtctc 3660
   gaggcagccc agcctcaacg cctacaactc actgacaagg tcggggctga agcgtacgcc 3720
   ctcgctaaag ccggacgtac cccccaaacc atcctttgct cccctttcca catccatgaa 3780
   gcccaatgat gcgtgtacat aatcccaggg ggagggggtc aggtgtcgaa ccagcaggca 3840
   aggcgaggtg cccgctcagc tcagcaaggt tctcaactgc ctcgagtacc caccagacca 3900
   agaaggcctg cggcagagcc gaggacgctg ggtcctcctc tctgggacac aggggtactc 3960
   acgaaaactg ggccgcgtgg tttggtgaag gtttgcaacg gcggggactc accttcattc 4020
   tcttccttca ctttccccca caccctacaa caggtcggac ccacaaaaga cttcagttat 4080
   catcacaaac atgagccaaa agcacatacc taccccatcc cccaccccca cacacacaca 4140
   cacacacaca tgcacacaac acatacacac acacgcacag aggtgaacag aaactgaaac 4200
   attttgtcca caacttcacg ggacgtggcc agactgggtt tgcgttccaa cctgcaaaac 4260
   acaaatacat tttttaaaat caagaaaatt taaaaagaca aaaaaaaaag aattcattga 4320
   taattctaac tcagacttta acaatggcag aagtttacta tgcgcaaata ctgtgaaatg 4380
   cccgccagtg ttacagcttt ctgttgcagc agataaatgc catgttgggc aactatgtca 4440
   tagatttctg ctcctcctct cttttaatga aataacgtga ccgttaacgc aagtaactct 4500
   ttatttattg ttcacccttt ttttccttaa ggaaaggact cttccaaata tcatcctatg 4560
   aacagctctt cagaaagccc attgaaagtt aaactattta acgtgaaatc cattaactgg 4620
   aataattgag tttctttatt tttacaataa attcactgag taaataagtt ggagctggaa 4680
   ttctgagctt tgtgtttgga ctgtctggtc tgtagctaaa ggaaaaagtt aggaggggaa 4740
   tatttattta aatctatcag ttcattgcct ggtcaggtct ctggcctata aacatgcaaa 4800
   aaaactaatt catgttaaag tccttccaga tcctaatttc taaagcaatc aggagagaaa 4860
   gcttttagaa tggcacatca tatctcattg atgccaacat ggttttgtcc atggttctga 4920
   ctttctgtga aggcaccagc ttgcaatatg ccatcccatt tcaccttgca tgtgagacag 4980
   caaacaaaat ccacaaatgg tgtgaactaa tatgctggct gctaccttgc ataaattaat 5040
   gatttgatca cacgggttct tcgtggggtt acatctgtga atagcctgtt ttccacatgt 5100
   aaatttgtgc cttacacctt gagttgtgta cacttgtaaa ctctttatga tcaactgttc 5160
   ccccttttga aataagtgca gatatttatt taaccctccc ttccccaccc tctgccccac 5220
   ttccagccct ctgaaagatt ggagtcaagc agatggaaga atgcagtggt gatagttgtc 5280
   atgcgacagc ctgagaacgc tgggcagcac cacaccctcc aattcacact gccttctagt 5340
   tgtgccaact ggaaccaccc tttggctgtg ctgcgaagca tggaccccag tgttgttgtg 5400
   ggtgtgtcaa atcccctttc atcctcaaga gctccctgct tcccttagat tatttcaata 5460
   cggtgatatc cttatttgct agcagaaaag ggactaacgt cccattcctc ttttctgctg 5520
   cgtccactgg ctagagagca ggcggtgcgc ggttgggcag acacctggga ggagtctcca 5580
   agccatgtgc acagcacaca cgtgcagtgc acacaaagaa atgacatgga aatagatgca 5640
   ggcaggctgg tccctgctgt gattaacgag taactccaag tacaaggccg accacaatgg 5700
   atgctgcaaa aacgttgact ggggcaaagg atttttattt ttatttttta ttttgttaat 5760
   tatgttttta ggggatggga ggtggtgtgt gtttttctcc tcttggtttt catttgctca 5820
   agcacacaaa agggactttt gtttactcta tcatgaacaa aggaactgtc cacatactgt 5880
   aaaccatgag cagtgttgtt gttgtttttt taaacagtat atttggtggt ctctttgtct 5940
   gcttttattt ccagttcgat cttctgggtt tagttttgtc tttaaaaaga attcaaaaaa 6000
   agactgacaa tgacagtttt gagttggata gtgaaaaagt ggagcctcca taatcagtgt 6060
   ggttgccttc agacctgagt acttagctga gggtgggtga gagccctttg ttccaaaagt 6120
   ccattagttt tgctgttgtt taggagtagg tggttgttgt tgtttttact tttttttttt 6180
   ttggcttttg taatggaatc catgttcaca tcctgtgaac tctgtctcct gaaaccctga 6240
   agtatttcct agaacctgaa atattgtttt cttccttgaa ttttctctag aaatgcagaa 6300
   attaggaagg tgatgggtct gtatccccct gcccccttct ttcccatgct tgactcccga 6360
   gagtactggc agtgcagcac ccatcattga cgtaagcggc tgtcttgtcc agtgtttgta 6420
   ggcatcactg cctcagctgt taaggagacc tgtgtcaaaa cttacatcca cattcctaca 6480
   cccccacaac ccatcacttc tggtgttaac cctaaaatac ccacatgtat tgagctggtc 6540
   ttctgcattt aagtatttct ccccagtttt tttccccact gtgtgtgggg ggagggtcca 6600
   taaacccgag tgtgcctttg ctttccaccc ttgctagaca ctggtagatg caacaaactc 6660
   agatttatat ttgttgtaaa gttgtaaaaa tattgtgatg tcaccaattt tccttccatc 6720
   tccacatccc ctaacatctg attcacgact taatgtatgt tgtaagaaaa gaaaaaagaa 6780
   aagaaaaaaa gggaaaaaag aaaagcaagg aaaaggctct ttattactta aaagtaataa 6840
   aacctgactg ttctatatta                                             6860
<210> 237
   <211> 3152
   <212> DNA
   <213> Homo sapiens
<220>
   <223> hydrocephalus-inducing protein homolog transcript variant 3 (HYDIN, HYDIN1; HYDIN2), axonemal central pair apparatus protein, protein phosphatase 1, regulatory subunit 31 (PPP1R31), CILD5
<220>
   <221> CDS
   <222> (36) ... (2888)
   <223> HYDIN
<400> 237
   accgtggcaa cggacggcaa cagctcgggc ggcgcatgga gagtgcgggc ggcttcaagc 60
   tgggtatgga gcccctcagc ggcggcgggg tctgtgagaa aaaaaaatta ctaaaaatga 120
   caagtagaag acttgaggag tccatggggg ctgttcagat gggattggtc aatatgttca 180
   aaggatttca aagcaaggtt ttgccacccc tgagtccaaa ggtggttaca gaagaagaag 240
   taaaccgaat gcttacaccc tcagagttcc tgaaggaaat gtccctgacc accgagcaga 300
   gactggcaaa aacacgtttg atgtgccgac cacagatcat cgaactctta gatatggggg 360
   aaacaacaca tcagaagttt tcaggaattg acctggatca ggcattattc cagccctttc 420
   catcagaaat tatatttcag aactacactc cctgtgaagt ctatgaagtt ccactgattt 480
   tgaggaacaa tgacaaaatt ccaaggttgg tgaaagttgt ggaagaaagt tcgccttact 540
   ttaaagtaat cagccccaaa gatattggcc acaaagtggc tcctggagtg ccttccatat 600
   tccgaatcct ctttactcca gaggagaaca aggattacgc ccatacgttg acctgtgtta 660
   ctgaaagaga aaagtttatt gtacccatca aagctagagg ggcacgagcc attctcgatt 720
   ttcctgacaa gctgaatttt tccacttgtc ctgtcaaata cagcacccag aagattctgc 780
   tggtacgaaa cattggcaac aaaaatgctg tatttcacat caaaacttgt aggcctttct 840
   ctatagaacc agctattgga actcttaatg tgggagagtc catgcaactg gaagtggagt 900
   ttgagccaca gagtgtgggc gatcacagtg gaagacttat cgtgtgttat gacacaggtg 960
   aaaaggtgtt tgtatctctc tatggagctg ccatagacat gaatataagg ctggataaga 1020
   attccttgac catcgagaaa acctacatat ctctggccaa tcagcgaact ataaccattc 1080
   acaatcgcag taatatcatt gcccatttcc tgtggaaggt atttgctacc cagcaagaag 1140
   aggacagaga aaaatatagg gcctgtgatg atctgatcaa agaggagaag gatgagactg 1200
   atgagttttt tgaagagtgc attactgatc ctttactccg agaacatctt tctgttctgt 1260
   cccgaacctt tgcgaatcag aggaggctgg tgcagggaga cagcaagctg ttcttcaata 1320
   acgttttcac tgtggagccc ctggaaggtg atgtctggcc caactcatca gctgaaatca 1380
   ccgtgtactt taacccacta gaagccaagc tctatcaaca gaccatttac tgcgacattt 1440
   taggccgaga aatccgtctg cccctccgaa tcaaagggga aggcatggga cctaagattc 1500
   acttcaactt tgaattgctg gatattggga aagttttcac tggatctgca cattgttatg 1560
   aggcgatact gtacaacaaa ggcagcatcg atgctctctt caacatgacc cctccaactt 1620
   cagctttggg ggcctgcttt gttttcagtc ccaaggaagg catcattgaa ccaagtggag 1680
   tccaagctat ccagatctcc ttcagctcta ccatcctggg aaactttgaa gaagagttcc 1740
   tggtcaatgt caatgggtca cctgagcctg tgaaactgac cattagaggc tgtgtcattg 1800
   gacctacctt ccattttaat gttccagctc tgcactttgg tgatgtttcc tttgggtttc 1860
   ctcatacctt gatatgttcc ctcaataata cctctttgat ccccatgact tacaaactgc 1920
   gtatccctgg ggatggcctt ggccataaaa gcatttcata ttgtgagcag catgtggact 1980
   acaaaagacc atcttggacc aaggaagaaa tatcctcaat gaaaccaaaa gaattcacca 2040
   tctctcctga ctgtggcacc attcgccccc agggatttgc tgctatcagg gtgacattat 2100
   gctccaacac tgtgcagaaa tacgagctgg cactcgtggt ggacgtggag ggcatcggag 2160
   aagaggtgct ggcgctctta attacagcaa ggtgtgttgt acctgccctc cacctggtca 2220
   atacagaggt ggactttggg cactgcttcc tgaagtaccc gtatgagaaa acactccagc 2280
   ttgccaatca agatgacctc ccaggattct atgaggtcca gcctcaggtg tgtgaggagg 2340
   tgcctactgt gctgttttcc agccccaccc ccagcggggt catctcccca agcagcacca 2400
   tccacatacc actggtcctg gagacccagg tcactggaga acacagatcc acggtttaca 2460
   tctcaatctt tgggagccag gacccccctt tggtatgtca cttaaagagc gctggagaag 2520
   gcccagttat ctacgtccat cccaatcaag tggacttcgg gaatatctac gtcctaaaag 2580
   actcttccag gattctcaac ctatgcaacc agtccttcat tcccgcattt ttccaggcac 2640
   acatggcaca caaaaaatcc ctttggacga ttgaacccaa tgaaggcatg gttcctccag 2700
   aaactgatgt tcaactggca ctgaccgcca acctgaatga cacactgaca ttcaaggact 2760
   gtgttatttt ggacattgaa aatagcagta cctatcggat tcctgttcag gcttccggaa 2820
   ctggttccac tattgtttca gataagccct ttgctccaga actcaatttg ggggcacatt 2880
   ttaggtaagg aaatctcatg gaccagtttt tatagatatc acatatgtta catatgctga 2940
   gcatcacctc tactttagaa aattatttta agtaattaaa actagccacc tcagtccctc 3000
   tcctttccca tcctcactct tccacctcct gatgtgtatg tcagcaacaa gaagacattt 3060
   tctagccata acttggttgc attctctaat ccgtgtccct ctacatccga gaagaatatg 3120
   gtttgtagga aatgcattct tttctcaaac tg                               3152
<210> 238
   <211> 1885
   <212> DNA
   <213> Homo sapiens
<220>
   <223> killer cell immunoglobulin-like receptor 3DL2 transcript variant 1 (KIR3DL2), natural killer cell inhibitory receptor, CD158 antigen-like family member K (CD158K)
<220>
   <221> CDS
   <222> (34) ... (1401)
   <223> KIR3DL2
<400> 238
   ggggcgcggc ctcctgtctg caccggcagc accatgtcgc tcacggtcgt cagcatggcg 60
   tgcgttgggt tcttcttgct gcagggggcc tggccactca tgggtggtca ggacaaaccc 120
   ttcctgtctg cccggcccag cactgtggtg cctcgaggag gacacgtggc tcttcagtgt 180
   cactatcgtc gtgggtttaa caatttcatg ctgtacaaag aagacagaag ccacgttccc 240
   atcttccacg gcagaatatt ccaggagagc ttcatcatgg gccctgtgac cccagcacat 300
   gcagggacct acagatgtcg gggttcacgc ccacactccc tcactgggtg gtcggcaccc 360
   agcaaccccc tggtgatcat ggtcacagga aaccacagaa aaccttccct cctggcccac 420
   ccagggcccc tgctgaaatc aggagagaca gtcatcctgc aatgttggtc agatgtcatg 480
   tttgagcact tctttctgca cagagagggg atctctgagg acccctcacg cctcgttgga 540
   cagatccatg atggggtctc caaggccaac ttctccatcg gtcccttgat gcctgtcctt 600
   gcaggaacct acagatgtta tggttctgtt cctcactccc cctatcagtt gtcagctccc 660
   agtgaccccc tggacatcgt gatcacaggt ctatatgaga aaccttctct ctcagcccag 720
   ccgggcccca cggttcaggc aggagagaac gtgaccttgt cctgtagctc ctggagctcc 780
   tatgacatct accatctgtc cagggaaggg gaggcccatg aacgtaggct ccgtgcagtg 840
   cccaaggtca acagaacatt ccaggcagac tttcctctgg gccctgccac ccacggaggg 900
   acctacagat gcttcggctc tttccgtgcc ctgccctgcg tgtggtcaaa ctcaagtgac 960
   ccactgcttg tttctgtcac aggaaaccct tcaagtagtt ggccttcacc cacagaacca 1020
   agctccaaat ctggtatctg cagacacctg catgttctga ttgggacctc agtggtcatc 1080
   ttcctcttca tcctcctcct cttctttctc ctttatcgct ggtgctccaa caaaaagaat 1140
   gctgctgtaa tggaccaaga gcctgcgggg gacagaacag tgaataggca ggactctgat 1200
   gaacaagacc ctcaggaggt gacgtacgca cagttggatc actgcgtttt catacagaga 1260
   aaaatcagtc gcccttctca gaggcccaag acacccctaa cagataccag cgtgtacacg 1320
   gaacttccaa atgctgagcc cagatccaaa gttgtctcct gcccacgagc accacagtca 1380
   ggtcttgagg gggttttcta gggagacaac agccctgtct caaaaccagg ttgccagatc 1440
   caatgaacca gcagctggaa tctgaaggca tcagtctgca tcttagggga tcgctcttcc 1500
   tcacaccacg aatctgaaca tgcctctctc ttgcttacaa atgcctaagg tcgccactgc 1560
   ctgctgcaga gaaaacacac tcctttgctt agcccacaag tatctatttc acttgacccc 1620
   tgcccacctc tccaacctaa ctggcttact tcctagtcct acttgaggct gcaatcacac 1680
   tgaggaactc acaattccaa acatacaaga ggctccctct taacacggca cttacacact 1740
   tgctgttcca ccttccctca tgctgttcca cctcccctca gactatcttt cagccttctg 1800
   tcatcagtaa aatttataaa ttttttttat aacttcagtg tagctctctc ctcttcaaat 1860
   aaacatgtct gccctcatgg tttcg                                      1885
<210> 239
   <211> 2005
   <212> DNA
   <213> Homo sapiens
<220>
   <223> bystin-like (BYSL), bystin, by the ribosomal protein s6 gene, drosophila, homolog-like
<220>
   <221> CDS
   <222> (337) ... (1650)
   <223> BYSL
<400> 239
   gcggattccc gccccgaggt ttctaaatcc agacattccc gtttggctga gcactctagg 60
   cctacatcca tgaagctagg agagcgacac tcaaagactg cactattgag agaagctaac 120
   gttaaaggca gtgaatatat tcgggagtcc agctccggaa cccgggagct cttttagtgg 180
   gaggggcggc gctgatggcg cttctggcct ccgaatgcta gggggcgctg tgatcgccgg 240
   gcggcctctt gggcgctggg agtccaccgc gcaagcgcat cctggccttt cttcagtccc 300
   cacgtgcgat ccttcccggc aactttttcg agaaaaatgc ccaaattcaa ggcggcccgt 360
   ggggtggggg gtcaggaaaa acatgcgccc ctggccgatc agatcctggc tgggaatgcg 420
   gtgcgggcgg gggtccggga gaagcggcgg ggtcgcggga caggagaagc ggaggaagag 480
   tatgtggggc cccggctgag ccgacggatt ttgcagcaag cacggcagca acaggaggaa 540
   ctcgaggccg agcatgggac tggggacaag cccgcggcgc cgcgggaacg caccacgcgg 600
   ctgggtccaa gaatgcctca ggatggatca gatgacgagg acgaggagtg gcccaccctg 660
   gagaaggctg ccacaatgac agcagcgggc catcatgcag aggtggttgt ggaccctgag 720
   gatgagcgtg ccatagagat gttcatgaac aagaaccctc ctgccaggcg caccctggct 780
   gacatcatca tggagaagct gactgagaag cagacagagg ttgagacagt catgtcagag 840
   gtgtcgggct tccctatgcc ccagctggac ccccgggtcc tagaagtgta caggggggtc 900
   cgggaggtat tatctaagta ccgcagtgga aaactgccca aggcatttaa gatcatccct 960
   gcactctcca actgggagca aatcctctac gtcacagagc cggaggcctg gactgcagct 1020
   gccatgtacc aggccaccag gatttttgcc tctaacctga aggaacgcat ggcccagcgc 1080
   ttctacaacc ttgtcctgct ccctcgagta cgagatgacg ttgctgaata caaacgactc 1140
   aacttccatc tctacatggc tctcaagaag gcccttttca aacctggagc ctggttcaaa 1200
   gggatcctga ttccactgtg cgagtctggc acttgtaccc tccgggaagc catcattgtg 1260
   ggtagcatca tcaccaagtg ctccatccct gtgttgcact ccagtgcggc catgctgaaa 1320
   attgctgaga tggaatacag cggtgccaac agcatcttcc tgcgactgct gctggataag 1380
   aagtatgcac tgccttaccg ggtgctggat gccctagtct tccacttcct ggggttccgg 1440
   acagagaagc gtgaactgcc tgtgctgtgg caccagtgcc tcctgacttt ggtccagcgc 1500
   tacaaggccg acttggccac agaccagaaa gaggccctct tagaactgct ccggctgcag 1560
   ccccatccac agctatcgcc cgaaatcagg cgtgagcttc agagtgcagt cccccgcgat 1620
   gtggaagatg ttcccatcac cgtggagtga ggaaaacagt cagctgtcct ggccaaaggg 1680
   gtttggaagg acaccaagac ccccgttggt gactgaagat gacactgagc tttaatggct 1740
   gaagacccag atcagggcag tgacagatca cagggacatc tgtggctccc agtccaggac 1800
   aggaaggact gagggtctgg ctggttccct cttccattct aggcccttat ccctgtttag 1860
   ttctgagagc caacttgaga taccatatgc tagcattccc agtccccagc tggggcttgg 1920
   tgtgagtact ttttctatgg ctattgtgtc aggtcactgt ggataaaggc aaagacagat 1980
   atttattgaa aaaaaaaaaa aaaaa                                      2005
<210> 240
   <211> 3392
   <212> DNA
   <213> Homo sapiens
<220>
   <223> ELOVL family member 6, elongation of long chain fatty acids (FEN1/Elo2, SUR4/Elo3-like, yeast), ELOVL fatty acid elongase 6 (ELOVL6), fatty acyl-CoA elongase, 3-keto acyl-CoA synthase ELOVL6, very-long-chain 3-oxoacyl-CoA synthase 6
<220>
   <221> CDS
   <222> (330) ... (1127)
   <223> ELOVL6
<400> 240
   gagatttccc tgtagacgag taaaaagggt gatggacaaa cgtgcgggca ctaagaccgc 60
   aaggcattca tttcctccta cggtggatgc ggacgccggg aggaggagag ccccagagag 120
   aggagctggg agcggaggcg caggcaatgc tcagccctgg atgtagctga gaggctggga 180
   gaagagacga ccgctggaga ccgagcggcg tggggaagac ctaggggggt gggtggggga 240
   agcagacagg agaacactcg aaatcaagcg ctttacagat tattttattt tgtatagaga 300
   acacgtagcg actccgaaga tcagccccaa tgaacatgtc agtgttgact ttacaagaat 360
   atgaattcga aaagcagttc aacgagaatg aagccatcca atggatgcag gaaaactgga 420
   agaaatcttt cctgttttct gctctgtatg ctgcctttat attcggtggt cggcacctaa 480
   tgaataaacg agcaaagttt gaactgagga agccattagt gctctggtct ctgacccttg 540
   cagtcttcag tatattcggt gctcttcgaa ctggtgctta tatggtgtac attttgatga 600
   ccaaaggcct gaagcagtca gtttgtgacc agggttttta caatggacct gtcagcaaat 660
   tctgggctta tgcatttgtg ctaagcaaag cacccgaact aggagataca atattcatta 720
   ttctgaggaa gcagaagctg atcttcctgc actggtatca ccacatcact gtgctcctgt 780
   actcttggta ctcctacaaa gacatggttg ccgggggagg ttggttcatg actatgaact 840
   atggcgtgca cgccgtgatg tactcttact atgccttgcg ggcggcaggt ttccgagtct 900
   cccggaagtt tgccatgttc atcaccttgt cccagatcac tcagatgctg atgggctgtg 960
   tggttaacta cctggtcttc tgctggatgc agcatgacca gtgtcactct cactttcaga 1020
   acatcttctg gtcctcactc atgtacctca gctaccttgt gctcttctgc catttcttct 1080
   ttgaggccta catcggcaaa atgaggaaaa caacgaaagc tgaatagtgt tggaactgag 1140
   gaggaagcca tagctcaggg tcatcaagaa aaataataga caaaagaaaa tggcacaagg 1200
   aatcacacgt ggtgcagcta aaacaaaaca aaacatgagc aaacacaaaa cccaaggcag 1260
   cttagggata attaggttga tttaacccag taagtttatg atccttttag ggtgaggact 1320
   cactgagtgc acctccatct ccaagcactg ctgctggaag accccattcc ctctttatct 1380
   atcaactcta ggacaaggga gaacaaaagc aagccagaag cagaggagac taatcaaagg 1440
   caaacaaagg ctattaacac ataggaaaaa atgtatttac taagtgtcac atttctctaa 1500
   gatgaaagat ttttactcta gaaactgtgc gagcacaaca cacacaatcc tttctaactt 1560
   tatggacact aaactggagc caatagaaaa gacaaaaatg aaagagacac agggtgtata 1620
   tctagaacga taatgctttt gcagaaacta aagccttttt aagaaatgcc agctgctgta 1680
   gaccccatga gaaaagatgt cttaatcatc cttatgaaaa cagatgtaaa caactatatt 1740
   tcaactaact tcatcttcac tgcatagcct caggctagtg agtttgccaa aaccaaaggg 1800
   ggtgaatact tccccaagat tcttcctggg aggatggaaa cagtgcagcc caggtcccat 1860
   gggggcagct ccatcccaga gcatttctga tagttgaact gtaatttcta ctcttaagtg 1920
   agatatgaag cattatcctt ttgttcagtt gccccgggct tttgaacaga agagtaaata 1980
   cagaattgaa aaagataaac actcaaccaa acaatgtgaa aacgggttct gtagtatttg 2040
   taaaaaggcc cggcccagga ccactgtgag ctggaaaagg gagaaaggca gtgggaaaag 2100
   aggtgagccg aagatcaatt cgacagacag atggtgtgta tgcccctccc tgtttgactt 2160
   cacacacact cataactttc caaatgaaac cccacagtat agcgcatatt ttcgatattt 2220
   ttgtgaattc caaaaggaaa tcacagggct gttcgaaata ttgggggaac actgtgtttc 2280
   tgcatcatct gcatttgctc cccaagcaat gtagaggtgt ttaaagggcc ctctgctggc 2340
   tgagtggcaa tactacaaca aacttcaagg caagtttggc tgaaaacagt tgacaacaaa 2400
   gggcccccat acacttatcc ctcaaatttt aagtgatatg aaatacttgt catgtctttg 2460
   gccaaatcag aagatattca tcctgcttca agtcagcttc agaaatgttt taaaagggac 2520
   tttagctctg gaactcaaaa tcaatttatt aagagccata ttctttaaaa aaaaaaagct 2580
   ggataatatt ctctgtaata tttcagtcct ttacaagcca aatacatgtg tcaatgtttc 2640
   tagtatttca aagaagcaat tatgtaaagt tgttcaatgt gacataatag tattataatt 2700
   ggttaagtag cttaatgatt aggcaaacta gatgaaaaga ttaggggctt ccacactgca 2760
   tagattacac gcacatagcc acgcatacac acacagacac acagatgtgg ggtacactga 2820
   acttcaaagc ccaaatgaat agaaacacat tttctggcta gcagaaaaaa acaaaacaaa 2880
   actgttgttt ctctttcttg ctttgagagt gtacagtaaa agggattttt tcgaattatt 2940
   tttatattat tttagcttta attgtgctgt cgttcatgaa acagagctgc tctgcttttc 3000
   tgtcagagat ggcaagggct ttttcagcat ctcgtttatg tgtggaattt aaaaagaata 3060
   aagttttatt ccattctgtg tgaatggttt gagcagtgaa caaagaatcc atgcaaacca 3120
   agtcatgact ggaagaaatg gggagtgggg gacagtgcag gggaggaaag ctgttacatc 3180
   tatgttaaat gattgtgagc agtctataat gtcttgatgt tggttcatag gtgagacaca 3240
   actgatggag gaatgtatta taaattatgc aaaatctgac atgagctgaa aagacaggtc 3300
   cttttataag agctaataga tgcttaggat gcaaatgtta ttttgagctt cgcaaataca 3360
   tgataccgtg caataagcct tttttcctta ta                               3392
<210> 241
   <211> 2873
   <212> DNA
   <213> Homo sapiens
<220>
   <223> solute carrier family 1 (neutral amino acid transporter), member 5 transcript variant 1 (SLC1A5), neutral amino acid transporter B(0), RD114/simian type D retrovirus receptor, sodium-dependent neutral amino acid transporter type 2, AAAT, ASCT2, M7VS1, RDRC
<220>
   <221> CDS
   <222> (621) ... (2246)
   <223> SLC1A5
<400> 241
   cggcacgccc gggaggcttt ctctggctgg taaccgctac tcccggacac cagaccaccg 60
   ccttccgtac acaggggccc gcatcccacc ctcccggacc taagagcctg ggtcccctgt 120
   ttccggaggt ccgcttcccg gcccccagat tctggcatcc cagccctcag tgtccaagac 180
   ccaggcagcc cgggtccccg cctcccggat ccaggcgtcc gggatctgcg ccaccagaac 240
   ctagcctcct gcagacctcc gccatctggg ggcactcaac ctcctggagc caagggcccc 300
   acgtcccacc cagagaaact ctcgtattcc cagctcctag ggccaaggaa cccgggcgct 360
   ccgaactccc agctttcgga catctggcac acggggcaga gcagagaagc ctcagcgccc 420
   agcctgggga atttaaacac tccagcttcc aagagccaag gaacttcagt gctgtgaact 480
   cacaactcta aggagccctc caaagttcca gtctccaggt gctgttactc aactcagtcc 540
   taggaacgtc gggtcctggg aaggagccca agcgctccca gccagcttcc aggcgctaag 600
   aaaccccggt gcttcccatc atggtggccg atcctcctcg agactccaag gggctcgcag 660
   cggcggagcc caccgccaac gggggcctgg cgctggcctc catcgaggac caaggcgcgg 720
   cagcaggcgg ctactgcggt tcccgggacc aggtgcgccg ctgccttcga gccaacctgc 780
   ttgtgctgct gacagtggtg gccgtggtgg ccggcgtggc gctgggactg ggggtgtcgg 840
   gggccggggg tgcgctggcg ttgggcccgg agcgcttgag cgccttcgtc ttcccgggcg 900
   agctgctgct gcgtctgctg cggatgatca tcttgccgct ggtggtgtgc agcttgatcg 960
   gcggcgccgc cagcctggac cccggcgcgc tcggccgtct gggcgcctgg gcgctgctct 1020
   ttttcctggt caccacgctg ctggcgtcgg cgctcggagt gggcttggcg ctggctctgc 1080
   agccgggcgc cgcctccgcc gccatcaacg cctccgtggg agccgcgggc agtgccgaaa 1140
   atgcccccag caaggaggtg ctcgattcgt tcctggatct tgcgagaaat atcttccctt 1200
   ccaacctggt gtcagcagcc tttcgctcat actctaccac ctatgaagag aggaatatca 1260
   ccggaaccag ggtgaaggtg cccgtggggc aggaggtgga ggggatgaac atcctgggct 1320
   tggtagtgtt tgccatcgtc tttggtgtgg cgctgcggaa gctggggcct gaaggggagc 1380
   tgcttatccg cttcttcaac tccttcaatg aggccaccat ggttctggtc tcctggatca 1440
   tgtggtacgc ccctgtgggc atcatgttcc tggtggctgg caagatcgtg gagatggagg 1500
   atgtgggttt actctttgcc cgccttggca agtacattct gtgctgcctg ctgggtcacg 1560
   ccatccatgg gctcctggta ctgcccctca tctacttcct cttcacccgc aaaaacccct 1620
   accgcttcct gtggggcatc gtgacgccgc tggccactgc ctttgggacc tcttccagtt 1680
   ccgccacgct gccgctgatg atgaagtgcg tggaggagaa taatggcgtg gccaagcaca 1740
   tcagccgttt catcctgccc atcggcgcca ccgtcaacat ggacggtgcc gcgctcttcc 1800
   agtgcgtggc cgcagtgttc attgcacagc tcagccagca gtccttggac ttcgtaaaga 1860
   tcatcaccat cctggtcacg gccacagcgt ccagcgtggg ggcagcgggc atccctgctg 1920
   gaggtgtcct cactctggcc atcatcctcg aagcagtcaa cctcccggtc gaccatatct 1980
   ccttgatcct ggctgtggac tggctagtcg accggtcctg taccgtcctc aatgtagaag 2040
   gtgacgctct gggggcagga ctcctccaaa attacgtgga ccgtacggag tcgagaagca 2100
   cagagcctga gttgatacaa gtgaagagtg agctgcccct ggatccgctg ccagtcccca 2160
   ctgaggaagg aaaccccctc ctcaaacact atcgggggcc cgcaggggat gccacggtcg 2220
   cctctgagaa ggaatcagtc atgtaaaccc cgggagggac cttccctgcc ctgctggggg 2280
   tgctctttgg acactggatt atgaggaatg gataaatgga tgagctaggg ctctgggggt 2340
   ctgcctgcac actctgggga gccaggggcc ccagcaccct ccaggacagg agatctggga 2400
   tgcctggctg ctggagtaca tgtgttcaca agggttactc ctcaaaaccc ccagttctca 2460
   ctcatgtccc caactcaagg ctagaaaaca gcaagatgga gaaataatgt tctgctgcgt 2520
   ccccaccgtg acctgcctgg cctcccctgt ctcagggagc aggtcacagg tcaccatggg 2580
   gaattctagc ccccactggg gggatgttac aacaccatgc tggttatttt ggcggctgta 2640
   gttgtggggg gatgtgtgtg tgcacgtgtg tgtgtgtgtg tgtgtgtgtg tgtgtgtgtg 2700
   tgttctgtga cctcctgtcc ccatggtacg tcccaccctg tccccagatc ccctattccc 2760
   tccacaataa cagaaacact cccagggact ctggggagag gctgaggaca aatacctgct 2820
   gtcactccag aggacatttt ttttagcaat aaaattgagt gtcaactatt taa        2873
<210> 242
   <211> 4567
   <212> DNA
   <213> Homo sapiens
<220>
   <223> carbohydrate synthase 1, chondroitin sulfate synthase 1 (CHSY1, CHSY, ChSy-1, CSS1), chondroitin glucuronyltransferase II, glucuronosyl-N-acetylgalactosaminyl-proteoglycan 4-beta-N-acetylgalactosaminyltransferase 1, TPBS
<220>
   <221> CDS
   <222> (477) ... (2885)
   <223> CHSY1
<400> 242
   ggcgagctaa gccggaggat gtgcagctgc ggcggcggcg ccggctacga agaggacggg 60
   gacaggcgcc gtgcgaaccg agcccagcca gccggaggac gcgggcaggg cgggacggga 120
   gcccggactc gtctgccgcc gccgtcgtcg ccgtcgtgcc ggccccgcgt ccccgcgcgc 180
   gagcgggagg agccgccgcc acctcgcgcc cgagccgccg ctagcgcgcg ccgggcatgg 240
   tcccctctta aaggcgcagg ccgcggcggc gggggcgggc gtgcggaaca aagcgccggc 300
   gcggggcctg cgggcggctc gggggccgcg atgggcgcgg cgggcccgcg gcggcggcgg 360
   cgctgcccgg gccgggcctc gcggcgctag ggcgggctgg cctccgcggg cgggggcagc 420
   gggctgaggg cgcgcggagc ctgcggcggc ggcccggcgg gcggagcggc gcgggcatgg 480
   ccgcgcgcgg ccggcgcgcc tggctcagcg tgctgctcgg gctcgtcctg ggcttcgtgc 540
   tggcctcgcg gctcgtcctg ccccgggctt ccgagctgaa gcgagcgggc ccacggcgcc 600
   gcgccagccc cgagggctgc cggtccgggc aggcggcggc ttcccaggcc ggcggggcgc 660
   gcggcgatgc gcgcggggcg cagctctggc cgcccggctc ggacccagat ggcggcccgc 720
   gcgacaggaa ctttctcttc gtgggagtca tgaccgccca gaaatacctg cagactcggg 780
   ccgtggccgc ctacagaaca tggtccaaga caattcctgg gaaagttcag ttcttctcaa 840
   gtgagggttc tgacacatct gtaccaattc cagtagtgcc actacggggt gtggacgact 900
   cctacccgcc ccagaagaag tccttcatga tgctcaagta catgcacgac cactacttgg 960
   acaagtatga atggtttatg agagcagatg atgacgtgta catcaaagga gaccgtctgg 1020
   agaacttcct gaggagtttg aacagcagcg agcccctctt tcttgggcag acaggcctgg 1080
   gcaccacgga agaaatggga aaactggccc tggagcctgg tgagaacttc tgcatggggg 1140
   ggcctggcgt gatcatgagc cgggaggtgc ttcggagaat ggtgccgcac attggcaagt 1200
   gtctccggga gatgtacacc acccatgagg acgtggaggt gggaaggtgt gtccggaggt 1260
   ttgcaggggt gcagtgtgtc tggtcttatg agatgcagca gcttttttat gagaattacg 1320
   agcagaacaa aaaggggtac attagagatc tccataacag taaaattcac caagctatca 1380
   cattacaccc caacaaaaac ccaccctacc agtacaggct ccacagctac atgctgagcc 1440
   gcaagatatc cgagctccgc catcgcacaa tacagctgca ccgcgaaatt gtcctgatga 1500
   gcaaatacag caacacagaa attcataaag aggacctcca gctgggaatc cctccctcct 1560
   tcatgaggtt tcagccccgc cagcgagagg agattctgga atgggagttt ctgactggaa 1620
   aatacttgta ttcggcagtt gacggccagc cccctcgaag aggaatggac tccgcccaga 1680
   gggaagcctt ggacgacatt gtcatgcagg tcatggagat gatcaatgcc aacgccaaga 1740
   ccagagggcg catcattgac ttcaaagaga tccagtacgg ctaccgccgg gtgaacccca 1800
   tgtatggggc tgagtacatc ctggacctgc tgcttctgta caaaaagcac aaagggaaga 1860
   aaatgacggt ccctgtgagg aggcacgcgt atttacagca gactttcagc aaaatccagt 1920
   ttgtggagca tgaggagctg gatgcacaag agttggccaa gagaatcaat caggaatctg 1980
   gatccttgtc ctttctctca aactccctga agaagctcgt cccctttcag ctccctgggt 2040
   cgaagagtga gcacaaagaa cccaaagata aaaagataaa catactgatt cctttgtctg 2100
   ggcgtttcga catgtttgtg agatttatgg gaaactttga gaagacgtgt cttatcccca 2160
   atcagaacgt caagctcgtg gttctgcttt tcaattctga ctccaaccct gacaaggcca 2220
   aacaagttga actgatgaga gattaccgca ttaagtaccc taaagccgac atgcagattt 2280
   tgcctgtgtc tggagagttt tcaagagccc tggccctgga agtaggatcc tcccagttta 2340
   acaatgaatc tttgctcttc ttctgcgacg tcgacctcgt gtttactaca gaattccttc 2400
   agcgatgtcg agcaaataca gttctgggcc aacaaatata ttttccaatc atcttcagcc 2460
   agtatgaccc aaagattgtt tatagtggga aagttcccag tgacaaccat tttgccttta 2520
   ctcagaaaac tggcttctgg agaaactatg ggtttggcat cacgtgtatt tataagggag 2580
   atcttgtccg agtgggtggc tttgatgttt ccatccaagg ctgggggctg gaggatgtgg 2640
   accttttcaa caaggttgtc caggcaggtt tgaagacgtt taggagccag gaagtaggag 2700
   tagtccacgt ccaccatcct gtcttttgtg atcccaatct tgaccccaaa cagtacaaaa 2760
   tgtgcttggg gtccaaagca tcgacctatg ggtccaccca gcagctggct gagatgtggc 2820
   tggaaaaaaa tgatccaagt tacagtaaaa gcagcaataa taatggctca gtgaggacag 2880
   cctaatgtcc agctttgctg gaaaagacgt ttttaattat ctaatttatt tttcaaaaat 2940
   tttttgtatg atcagttttt gaagtccgta tacaaggata tattttacaa gtggttttct 3000
   tacataggac tcctttaaga ttgagctttc tgaacaagaa ggtgatcagt gtttgccttt 3060
   gaacacatct tcttgctgaa cattatgtag cagacctgct taactttgac ttgaaatgta 3120
   cctgatgaac aaaacttttt taaaaaaatg ttttcttttg agaccctttg ctccagtcct 3180
   atggcagaaa acgtgaacat tcctgcaaag tattattgta acaaaacact gtaactctgg 3240
   taaatgttct gttgtgattg ttaacattcc acagattcta ccttttgtgt tttgtttttt 3300
   ttttttacaa ttgttttaaa gccatttcat gttccagttg taagataagg aaatgtgata 3360
   atagctgttt catcattgtc ttcaggagag ctttccagag ttgatcattt cctctcatgg 3420
   tactctgctc agcatggcca cgtaggtttt ttgtttgttt tgttttgttc tttttttgag 3480
   acggagtctc actctgttac ccaggctgga atgcagtggc gcaatcttgg ctcactttaa 3540
   cctccacttc cctggttcaa gcaattcccc tgcctttgcc tcccgagtag ctgggattac 3600
   aggcacacac caccacgccc agctagtttt tttgtatttt tagtagagac ggggtttcac 3660
   catgcaagcc cagctggcca cgtaggtttt aaagcaaggg gcgtgaagaa ggcacagtga 3720
   ggtatgtggc tgttctcgtg gtagttcatt cggcctaaat agacctggca ttaaatttca 3780
   agaaggattt ggcattttct cttcttgacc cttctcttta aagggtaaaa tattaatgtt 3840
   tagaatgaca aagatgaatt attacaataa atctgatgta cacagactga aacatacaca 3900
   catacaccct aatcaaaacg ttggggaaaa atgtatttgg ttttgttcct ttcatcctgt 3960
   ctgtgttatg tgggtggaga tggttttcat tctttcatta ctgttttgtt ttatcctttg 4020
   tatctgaaat acctttaatt tatttaatat ctgttgttca gagctctgcc atttcttgag 4080
   tacctgttag ttagtattat ttatgtgtat cgggagtgtg tttagtctgt tttatttgca 4140
   gtaaaccgat ctccaaagat ttccttttgg aaacgctttt tcccctcctt aatttttata 4200
   ttccttactg ttttactaaa tattaagtgt tctttgacaa ttttggtgct catgtgtttt 4260
   ggggacaaaa gtgaaatgaa tctgtcatta taccagaaag ttaaattctc agatcaaatg 4320
   tgccttaata aatttgtttt catttagatt tcaaacagtg atagacttgc cattttaata 4380
   cacgtcattg gagggctgcg tatttgtaaa tagcctgatg ctcatttgga aaaataaacc 4440
   agtgaacaat atttttctat tgtacttttc gaaccatttt gtctcattat tcctgtttta 4500
   gctgaagaat tgtattacat ttggagagta aaaaacttaa acacgattca aaaaaaaaaa 4560
   aaaaaaa                                                           4567
<210> 243
   <211> 1718
   <212> DNA
   <213> Homo sapiens
<220>
   <223> interleukin 8 (IL8), small inducible cytokine subfamily B, member 8, tumor necrosis factor-induced gene 1, chemokine (C-X-C motif) ligand 8 (CXCL8), emoctakin, granulocyte chemotactic protein 1, lymphocyte derived neutrophil activating peptide (NAP-1, NAP1)
<220>
   <221> CDS
   <222> (154) ... (453)
   <223> IL8
<400> 243
   gagggtgcat aagttctcta gtagggtgat gatataaaaa gccaccggag cactccataa 60
   ggcacaaact ttcagagaca gcagagcaca caagcttcta ggacaagagc caggaagaaa 120
   ccaccggaag gaaccatctc actgtgtgta aacatgactt ccaagctggc cgtggctctc 180
   ttggcagcct tcctgatttc tgcagctctg tgtgaaggtg cagttttgcc aaggagtgct 240
   aaagaactta gatgtcagtg cataaagaca tactccaaac ctttccaccc caaatttatc 300
   aaagaactga gagtgattga gagtggacca cactgcgcca acacagaaat tattgtaaag 360
   ctttctgatg gaagagagct ctgtctggac cccaaggaaa actgggtgca gagggttgtg 420
   gagaagtttt tgaagagggc tgagaattca taaaaaaatt cattctctgt ggtatccaag 480
   aatcagtgaa gatgccagtg aaacttcaag caaatctact tcaacacttc atgtattgtg 540
   tgggtctgtt gtagggttgc cagatgcaat acaagattcc tggttaaatt tgaatttcag 600
   taaacaatga atagtttttc attgtaccat gaaatatcca gaacatactt atatgtaaag 660
   tattatttat ttgaatctac aaaaaacaac aaataatttt taaatataag gattttccta 720
   gatattgcac gggagaatat acaaatagca aaattgaggc caagggccaa gagaatatcc 780
   gaactttaat ttcaggaatt gaatgggttt gctagaatgt gatatttgaa gcatcacata 840
   aaaatgatgg gacaataaat tttgccataa agtcaaattt agctggaaat cctggatttt 900
   tttctgttaa atctggcaac cctagtctgc tagccaggat ccacaagtcc ttgttccact 960
   gtgccttggt ttctccttta tttctaagtg gaaaaagtat tagccaccat cttacctcac 1020
   agtgatgttg tgaggacatg tggaagcact ttaagttttt tcatcataac ataaattatt 1080
   ttcaagtgta acttattaac ctatttatta tttatgtatt tatttaagca tcaaatattt 1140
   gtgcaagaat ttggaaaaat agaagatgaa tcattgattg aatagttata aagatgttat 1200
   agtaaattta ttttatttta gatattaaat gatgttttat tagataaatt tcaatcaggg 1260
   tttttagatt aaacaaacaa acaattgggt acccagttaa attttcattt cagataaaca 1320
   acaaataatt ttttagtata agtacattat tgtttatctg aaattttaat tgaactaaca 1380
   atcctagttt gatactccca gtcttgtcat tgccagctgt gttggtagtg ctgtgttgaa 1440
   ttacggaata atgagttaga actattaaaa cagccaaaac tccacagtca atattagtaa 1500
   tttcttgctg gttgaaactt gtttattatg tacaaataga ttcttataat attatttaaa 1560
   tgactgcatt tttaaataca aggctttata tttttaactt taagatgttt ttatgtgctc 1620
   tccaaatttt ttttactgtt tctgattgta tggaaatata aaagtaaata tgaaacattt 1680
   aaaatataat ttgttgtcaa agtaaaaaaa aaaaaaaa                        1718
<210> 244
   <211> 2158
   <212> DNA
   <213> Homo sapiens
<220>
   <223> FBJ murine osteosarcoma viral (v-fos) oncogene homolog (FOS), cellular oncogene c-fos, activator protein 1 (AP-1), G0/G1 switch regulatory protein 7, p55
<220>
   <221> CDS
   <222> (206) ... (1348)
   <223> FOS
<400> 244
   attcataaaa cgcttgttat aaaagcagtg gctgcggcgc ctcgtactcc aaccgcatct 60
   gcagcgagca tctgagaagc caagactgag ccggcggccg cggcgcagcg aacgagcagt 120
   gaccgtgctc ctacccagct ctgctccaca gcgcccacct gtctccgccc ctcggcccct 180
   cgcccggctt tgcctaaccg ccacgatgat gttctcgggc ttcaacgcag actacgaggc 240
   gtcatcctcc cgctgcagca gcgcgtcccc ggccggggat agcctctctt actaccactc 300
   acccgcagac tccttctcca gcatgggctc gcctgtcaac gcgcaggact tctgcacgga 360
   cctggccgtc tccagtgcca acttcattcc cacggtcact gccatctcga ccagtccgga 420
   cctgcagtgg ctggtgcagc ccgccctcgt ctcctccgtg gccccatcgc agaccagagc 480
   ccctcaccct ttcggagtcc ccgccccctc cgctggggct tactccaggg ctggcgttgt 540
   gaagaccatg acaggaggcc gagcgcagag cattggcagg aggggcaagg tggaacagtt 600
   atctccagaa gaagaagaga aaaggagaat ccgaagggaa aggaataaga tggctgcagc 660
   caaatgccgc aaccggagga gggagctgac tgatacactc caagcggaga cagaccaact 720
   agaagatgag aagtctgctt tgcagaccga gattgccaac ctgctgaagg agaaggaaaa 780
   actagagttc atcctggcag ctcaccgacc tgcctgcaag atccctgatg acctgggctt 840
   cccagaagag atgtctgtgg cttcccttga tctgactggg ggcctgccag aggttgccac 900
   cccggagtct gaggaggcct tcaccctgcc tctcctcaat gaccctgagc ccaagccctc 960
   agtggaacct gtcaagagca tcagcagcat ggagctgaag accgagccct ttgatgactt 1020
   cctgttccca gcatcatcca ggcccagtgg ctctgagaca gcccgctccg tgccagacat 1080
   ggacctatct gggtccttct atgcagcaga ctgggagcct ctgcacagtg gctccctggg 1140
   gatggggccc atggccacag agctggagcc cctgtgcact ccggtggtca cctgtactcc 1200
   cagctgcact gcttacacgt cttccttcgt cttcacctac cccgaggctg actccttccc 1260
   cagctgtgca gctgcccacc gcaagggcag cagcagcaat gagccttcct ctgactcgct 1320
   cagctcaccc acgctgctgg ccctgtgagg gggcagggaa ggggaggcag ccggcaccca 1380
   caagtgccac tgcccgagct ggtgcattac agagaggaga aacacatctt ccctagaggg 1440
   ttcctgtaga cctagggagg accttatctg tgcgtgaaac acaccaggct gtgggcctca 1500
   aggacttgaa agcatccatg tgtggactca agtccttacc tcttccggag atgtagcaaa 1560
   acgcatggag tgtgtattgt tcccagtgac acttcagaga gctggtagtt agtagcatgt 1620
   tgagccaggc ctgggtctgt gtctcttttc tctttctcct tagtcttctc atagcattaa 1680
   ctaatctatt gggttcatta ttggaattaa cctggtgctg gatattttca aattgtatct 1740
   agtgcagctg attttaacaa taactactgt gttcctggca atagtgtgtt ctgattagaa 1800
   atgaccaata ttatactaag aaaagatacg actttatttt ctggtagata gaaataaata 1860
   gctatatcca tgtactgtag tttttcttca acatcaatgt tcattgtaat gttactgatc 1920
   atgcattgtt gaggtggtct gaatgttctg acattaacag ttttccatga aaacgtttta 1980
   ttgtgttttt aatttattta ttaagatgga ttctcagata tttatatttt tattttattt 2040
   ttttctacct tgaggtcttt tgacatgtgg aaagtgaatt tgaatgaaaa atttaagcat 2100
   tgtttgctta ttgttccaag acattgtcaa taaaagcatt taagttgaat gcgaccaa   2158
<210> 245
   <211> 4814
   <212> DNA
   <213> Homo sapiens
<220>
   <223> UDP-Gal:betaGlcNAc beta 1,4- galactosyltransferase, polypeptide 6, UDP-galactose:beta-N-acetylglucosamine beta-1,4-galactosyl transferase 6 (B4GALT6, B4Gal-T6, beta4Gal-T6, beta4GalT-VI), UDP-Gal:glucosylceramide beta-1,4-galactosyltransferase
<220>
   <221> CDS
   <222> (298) ... (1446)
   <223> B4GALT6
<400> 245
   ccggctttct tccgtcctgc cgtccccggg cccctagccc ccagtcccct ctcccccctc 60
   cacctcttgt ccctcggcct ccgcgcctcc cctcccccga ctctctggcc tcccattcat 120
   tccgtccgct ccctcccatt tcctcttttc cgctcgggcc ctctccccag agcctccccg 180
   cgcggacccc gctgcagtct cggggttctc agcacattta tggaagttta gggcctggac 240
   agtggccccg agtccggcct gagagcgcag cctgggctgc tggcagggaa gaggaagatg 300
   tctgtgctca ggcggatgat gcgggtttcc aatcgctctc tcctcgcctt catcttcttc 360
   ttctccctct cttcgtcctg tctgtacttc atctatgtgg ccccaggcat cgccaacaca 420
   tatctcttta tggtacaagc tcgaggtata atgttgagag aaaatgtgaa aacaataggt 480
   catatgatca ggctgtacac aaataaaaac agtacgctca acggtacaga ttatcccgaa 540
   ggcaataatt caagtgatta tcttgttcaa acaacaacgt atctcccgga aaacttcaca 600
   tactcaccat acctcccctg tccagaaaag ctgccttata tgcgaggatt cctcaatgtc 660
   aatgtaagcg aagtcagttt tgatgaaatt catcaactct tctccaagga tttagatatt 720
   gagccagggg gtcattggag gccaaaagac tgtaaaccca gatggaaggt ggcagttctc 780
   attcctttcc gtaatcgcca tgaacatctt ccaatttttt tcttacatct gattccaatg 840
   ctccagaagc agcggctgga atttgcgttt tatgtcattg aacagactgg cacacaacct 900
   tttaaccgtg cgatgctttt caatgtgggc ttcaaagagg ccatgaaaga cagtgtctgg 960
   gactgtgtaa tcttccacga tgtggatcat ctacctgaaa atgaccggaa ctattacgga 1020
   tgtggagaaa tgccacgtca ttttgctgca aagctggata aatacatgta tattcttcca 1080
   tataaagaat tttttggtgg tgtaagtggg ctgacagtgg aacaatttag aaagatcaat 1140
   ggttttccta atgccttctg ggatggggga ggagaagatg atgacctttg gaacagagtt 1200
   cactatgctg gatataatgt aaccagacca gagggagact taggaaaata caagtcaatt 1260
   cctcatcacc atagaggtga agtccagttt ttaggacggt ataaattact aaggtattcc 1320
   aaggagcgtc agtacatcga tggactgaac aatttaatat ataggccaaa aatactggtt 1380
   gataggttgt atacaaacat atctgtaaac ctcatgccag agttagctcc aatcgaagac 1440
   tattaaaaga agtggctgtc gtggcaaggt agaccacaat gctggatcat aaacttggag 1500
   cgcgctctta gtggaggtca gtgattggct gtgtcacagt gcccttttct ctgagaagag 1560
   ccagcagtcc acagtgttta cagagtggga ctatatacag tcacccttct ctcacccgtc 1620
   ctccctgctc tgagacacac ccctgtggcg agcaccgcag aaacgggaag ccactactta 1680
   agatcggaag ttaagagagc tccctccgaa gagaaaattt ttatactaaa atctataatt 1740
   taattcaaga gaatgctttt atttccgttt aaacatattt tgtatatatg tgatataaat 1800
   taatgtgtgc aaattgttta aaaattagat gtgttgcagt tttgcatgta atcggttata 1860
   cctttattgg acttttatag acatttttta tttgcatgaa aaaaactcac taaatttaca 1920
   tcactaaaca aaggttaacc cttgtgtgaa atgaaggaac tgtcaataat tgacagccaa 1980
   ctaatacagt aaactgttat actagttttg agctttagac ctcagccttt tgtgtggaag 2040
   aagtcacagc tttcttaggc tttaaaggaa aagaaggaag gacttaaata gcttttcttc 2100
   ctaccgggat tacctatgtt tttccttgct tgcaatctca tctgattttg ctagaaatca 2160
   caaccatatt gtttatgcat attgcatgag tattaccaag aaaaatctta aaagttgtga 2220
   tgtgacatga tataaaggat ctctttatgt taaatgtctt tccatgtacc tctggtgtgt 2280
   cagggatttt gtgcctcaaa aaatgtttcc aaggttgtgt gtttatactg tgtatttttt 2340
   ttaaattcac ggtgaacagc acttttatta tttccagttc agaagagcca aaaaaaagta 2400
   tcttcattta aaaagaaatc aagtcagttt ttttaaaaga atgatctatg gaagaaaacc 2460
   caatagtact tataatatac aaatgaatta tactattaga taagaattaa atattctatt 2520
   tttataaaga taaatactta gtagataaaa acaaatttta agtttagttt tcagacatag 2580
   aatttcactt ttggagggga cccttgaatg ttagtttcat tcaaactata ttttgttttt 2640
   ttcactttta agagattata tctctggtgt taattactaa aattttcttt ctaaaagtat 2700
   aatttaatag aattatctta aaatacatga tcagtattca gtgcgctgat attataatgt 2760
   cttctatatg tagtatctaa aagcataatt taatagcatt gtcttaaaat acgtgatgag 2820
   ttatttggtg cactgatgcc ataatgtctt ctatatgtaa tatcttacat gtttgcatga 2880
   gtgtcagggc tttgtccagt tatcttcata tgactgttct ctgaaaaaag gtcttactac 2940
   ctgatatcaa gtacatatct taattttgga tgaatttttc cacagtaaag ccgaacagta 3000
   ttcttttttg aaaattagca attataaaga ttgtttaaaa tatcttgata ttttcacaaa 3060
   ttttaaacaa aaaattaaat atctataaag tgaatgttca acaacaatga tgagcaatag 3120
   aaatctatat atgagaaacg tttggataat atttgcctca aggattgttt acctaaaaca 3180
   gaactatggt tatttatctc agattgtaag ttttattttg aagtgtcatt tttatggcaa 3240
   tattgataac acaaatgacc acaaactaaa tcataaaata attgaatcct tacactttga 3300
   gaaaatggat agcttctgtg ttatgatacc aaacaaaata ttttcatatt tatttcttag 3360
   aactatttcc aaaatattta tatttcagtt tttttatggt actcgaaaaa tcaaaccttc 3420
   tgaaaattat gatatttgta cttcatttgt gtattagttc attttctgtt tctattacat 3480
   agtttttaaa gtcaatgaaa gagattgaga atgttaacat ttgttgatca atgtggagct 3540
   gtccacattc ggtatctttt catttttata tatattttac agtttcaaga aaacttagta 3600
   ctactagtat ctgccttatt ttcttaagtc tgatgttaat ttttgtttca ggagtgagat 3660
   ataaacattt tgcttttttt cagttttgaa atatagtttg tcatcctgtg aaaaccacct 3720
   cgtttgcttt ttgtttgttt gttttgcttt tttagtgtag acctaaaatt atttttgcct 3780
   ataaacagtg aacttaatat gtttctgtga gagtgacaga gctgtgcccg tttatcttca 3840
   ttgtattaat tattacataa agattaacaa atacattgca aatgcaaatt ataggatgtt 3900
   tggaggattt attaatgagc atgtattgat tgcctattcc atatccagtg caatgctagg 3960
   tatgtctatg gaaggagtat tattacttca tttctaaaca aatgaagtca agcaaattga 4020
   gatctttctg aaattatgaa gacatcttct gataacttat ctgttgtatt tcctagtctc 4080
   aaagtcaaga aacccgtata tgtggaccta gggctaatag gaggtcccta tggcactaac 4140
   tgggaaaatt acatggtgct tttcaactac agaaagaagt aatgactaat gggcctgtct 4200
   gtagagaaga aaaggagaag agtacacatt tgctggaact atatataatt cacagtggag 4260
   atagttaatt ctttgctaaa actagggttt tatgagtatt ttttaaatct acatttaagt 4320
   ttttgttttt gtgaaaagtt attcagtaag aaaactttat aatattaagt agatacagtt 4380
   atgtattaaa aatttcctta tatacccttt tgccagccaa gatactgata ttttaaaaca 4440
   aaatggaaaa atgtccaata caatattctc tttctggagg cttcctacaa gtgaaagcag 4500
   attttgtcat tttatttggc ctttgcttgt ctttgatata tttctttatg tgtttttcaa 4560
   atacatactg ttgtagattt attgtcttgt atttgcaagt atgccggttg agagaactaa 4620
   aatggttata gtattgttta tcagaaagag gaagtgtact taagattatt ttatttattt 4680
   agatgaaagg cttctacata aatgcactga aataaatgct agtaatttct tgtatcccac 4740
   ttcttttttt gcttctcatg gaaatgtttg tactttttta tcattgtctt ttatgaaata 4800
   taatgttcta aaga 4814
<210> 246
   <211> 4304
   <212> DNA
   <213> Homo sapiens
<220>
   <223> cyclin D1 (CCND1), PRAD1 oncogene, parathyroid adenomatosis 1 (PRAD1), B-cell CLL/lymphoma 1, BCL-1 oncogene, B-cell lymphoma 1 protein (BCL1), D11S287E, U21B31
<220>
   <221> CDS
   <222> (210) ... (1097)
   <223> CCND1
<400> 246
   cacacggact acaggggagt tttgttgaag ttgcaaagtc ctggagcctc cagagggctg 60
   tcggcgcagt agcagcgagc agcagagtcc gcacgctccg gcgaggggca gaagagcgcg 120
   agggagcgcg gggcagcaga agcgagagcc gagcgcggac ccagccagga cccacagccc 180
   tccccagctg cccaggaaga gccccagcca tggaacacca gctcctgtgc tgcgaagtgg 240
   aaaccatccg ccgcgcgtac cccgatgcca acctcctcaa cgaccgggtg ctgcgggcca 300
   tgctgaaggc ggaggagacc tgcgcgccct cggtgtccta cttcaaatgt gtgcagaagg 360
   aggtcctgcc gtccatgcgg aagatcgtcg ccacctggat gctggaggtc tgcgaggaac 420
   agaagtgcga ggaggaggtc ttcccgctgg ccatgaacta cctggaccgc ttcctgtcgc 480
   tggagcccgt gaaaaagagc cgcctgcagc tgctgggggc cacttgcatg ttcgtggcct 540
   ctaagatgaa ggagaccatc cccctgacgg ccgagaagct gtgcatctac accgacaact 600
   ccatccggcc cgaggagctg ctgcaaatgg agctgctcct ggtgaacaag ctcaagtgga 660
   acctggccgc aatgaccccg cacgatttca ttgaacactt cctctccaaa atgccagagg 720
   cggaggagaa caaacagatc atccgcaaac acgcgcagac cttcgttgcc ctctgtgcca 780
   cagatgtgaa gttcatttcc aatccgccct ccatggtggc agcggggagc gtggtggccg 840
   cagtgcaagg cctgaacctg aggagcccca acaacttcct gtcctactac cgcctcacac 900
   gcttcctctc cagagtgatc aagtgtgacc cggactgcct ccgggcctgc caggagcaga 960
   tcgaagccct gctggagtca agcctgcgcc aggcccagca gaacatggac cccaaggccg 1020
   ccgaggagga ggaagaggag gaggaggagg tggacctggc ttgcacaccc accgacgtgc 1080
   gggacgtgga catctgaggg cgccaggcag gcgggcgcca ccgccacccg cagcgagggc 1140
   ggagccggcc ccaggtgctc ccctgacagt ccctcctctc cggagcattt tgataccaga 1200
   agggaaagct tcattctcct tgttgttggt tgttttttcc tttgctcttt cccccttcca 1260
   tctctgactt aagcaaaaga aaaagattac ccaaaaactg tctttaaaag agagagagag 1320
   aaaaaaaaaa tagtatttgc ataaccctga gcggtggggg aggagggttg tgctacagat 1380
   gatagaggat tttatacccc aataatcaac tcgtttttat attaatgtac ttgtttctct 1440
   gttgtaagaa taggcattaa cacaaaggag gcgtctcggg agaggattag gttccatcct 1500
   ttacgtgttt aaaaaaaagc ataaaaacat tttaaaaaca tagaaaaatt cagcaaacca 1560
   tttttaaagt agaagagggt tttaggtaga aaaacatatt cttgtgcttt tcctgataaa 1620
   gcacagctgt agtggggttc taggcatctc tgtactttgc ttgctcatat gcatgtagtc 1680
   actttataag tcattgtatg ttattatatt ccgtaggtag atgtgtaacc tcttcacctt 1740
   attcatggct gaagtcacct cttggttaca gtagcgtagc gtgcccgtgt gcatgtcctt 1800
   tgcgcctgtg accaccaccc caacaaacca tccagtgaca aaccatccag tggaggtttg 1860
   tcgggcacca gccagcgtag cagggtcggg aaaggccacc tgtcccactc ctacgatacg 1920
   ctactataaa gagaagacga aatagtgaca taatatattc tatttttata ctcttcctat 1980
   ttttgtagtg acctgtttat gagatgctgg ttttctaccc aacggccctg cagccagctc 2040
   acgtccaggt tcaacccaca gctacttggt ttgtgttctt cttcatattc taaaaccatt 2100
   ccatttccaa gcactttcag tccaataggt gtaggaaata gcgctgtttt tgttgtgtgt 2160
   gcagggaggg cagttttcta atggaatggt ttgggaatat ccatgtactt gtttgcaagc 2220
   aggactttga ggcaagtgtg ggccactgtg gtggcagtgg aggtggggtg tttgggaggc 2280
   tgcgtgccag tcaagaagaa aaaggtttgc attctcacat tgccaggatg ataagttcct 2340
   ttccttttct ttaaagaagt tgaagtttag gaatcctttg gtgccaactg gtgtttgaaa 2400
   gtagggacct cagaggttta cctagagaac aggtggtttt taagggttat cttagatgtt 2460
   tcacaccgga aggtttttaa acactaaaat atataattta tagttaaggc taaaaagtat 2520
   atttattgca gaggatgttc ataaggccag tatgatttat aaatgcaatc tccccttgat 2580
   ttaaacacac agatacacac acacacacac acacacacaa accttctgcc tttgatgtta 2640
   cagatttaat acagtttatt tttaaagata gatcctttta taggtgagaa aaaaacaatc 2700
   tggaagaaaa aaaccacaca aagacattga ttcagcctgt ttggcgtttc ccagagtcat 2760
   ctgattggac aggcatgggt gcaaggaaaa ttagggtact caacctaagt tcggttccga 2820
   tgaattctta tcccctgccc cttcctttaa aaaacttagt gacaaaatag acaatttgca 2880
   catcttggct atgtaattct tgtaattttt atttaggaag tgttgaaggg aggtggcaag 2940
   agtgtggagg ctgacgtgtg agggaggaca ggcgggagga ggtgtgagga ggaggctccc 3000
   gaggggaagg ggcggtgccc acaccgggga caggccgcag ctccattttc ttattgcgct 3060
   gctaccgttg acttccaggc acggtttgga aatattcaca tcgcttctgt gtatctcttt 3120
   cacattgttt gctgctattg gaggatcagt tttttgtttt acaatgtcat atactgccat 3180
   gtactagttt tagttttctc ttagaacatt gtattacaga tgcctttttt gtagtttttt 3240
   ttttttttat gtgatcaatt ttgacttaat gtgattactg ctctattcca aaaaggttgc 3300
   tgtttcacaa tacctcatgc ttcacttagc catggtggac ccagcgggca ggttctgcct 3360
   gctttggcgg gcagacacgc gggcgcgatc ccacacaggc tggcgggggc cggccccgag 3420
   gccgcgtgcg tgagaaccgc gccggtgtcc ccagagacca ggctgtgtcc ctcttctctt 3480
   ccctgcgcct gtgatgctgg gcacttcatc tgatcggggg cgtagcatca tagtagtttt 3540
   tacagctgtg ttattctttg cgtgtagcta tggaagttgc ataattatta ttattattat 3600
   tataacaagt gtgtcttacg tgccaccacg gcgttgtacc tgtaggactc tcattcggga 3660
   tgattggaat agcttctgga atttgttcaa gttttgggta tgtttaatct gttatgtact 3720
   agtgttctgt ttgttattgt tttgttaatt acaccataat gctaatttaa agagactcca 3780
   aatctcaatg aagccagctc acagtgctgt gtgccccggt cacctagcaa gctgccgaac 3840
   caaaagaatt tgcaccccgc tgcgggccca cgtggttggg gccctgccct ggcagggtca 3900
   tcctgtgctc ggaggccatc tcgggcacag gcccaccccg ccccacccct ccagaacacg 3960
   gctcacgctt acctcaacca tcctggctgc ggcgtctgtc tgaaccacgc gggggccttg 4020
   agggacgctt tgtctgtcgt gatggggcaa gggcacaagt cctggatgtt gtgtgtatcg 4080
   agaggccaaa ggctggtggc aagtgcacgg ggcacagcgg agtctgtcct gtgacgcgca 4140
   agtctgaggg tctgggcggc gggcggctgg gtctgtgcat ttctggttgc accgcggcgc 4200
   ttcccagcac caacatgtaa ccggcatgtt tccagcagaa gacaaaaaga caaacatgaa 4260
   agtctagaaa taaaactggt aaaaccccaa aaaaaaaaaa aaaa                    4304
<210> 247
   <211> 4102
   <212> DNA
   <213> Homo sapiens
<220>
   <223> ets variant 5 , ETS translocation variant 5 (ETV5), ets-related molecule; ets-related protein ERM
<220>
   <221> CDS
   <222> (248) ... (1780)
   <223> ETV5
<400> 247
   agcctggttg gcagctgcgg cgcagagtcc agccgctggt gcgcggagcg gttcaccgtc 60
   ttcggagcgg ttcggcccag cctttcgccc aggcgcccag gcccgctgcg cgcgtgcgtg 120
   agcgcgcctg cgccgccggg gccgctgcaa ggggaggaga gaggccgcct caggaggatc 180
   ccttttcccc cagaaattac tcaatgctga aacctctcaa agtggtatta gagacgctga 240
   aagcaccatg gacgggtttt atgatcagca agtccctttt atggtcccag ggaaatctcg 300
   atctgaggaa tgcagagggc ggcctgtgat tgacagaaag aggaagtttt tggacacaga 360
   tctggctcac gattctgaag agctatttca ggatctcagt caacttcaag aggcttggtt 420
   agctgaagca caagttcctg atgatgaaca gtttgtccca gattttcagt ctgataacct 480
   ggtgcttcat gccccacctc caaccaagat caaacgggag ctgcacagcc cctcctctga 540
   gctgtcgtct tgtagccatg agcaggctct tggtgctaac tatggagaaa agtgcctcta 600
   caactattgt gcctatgata ggaagcctcc ctctgggttc aagccattaa cccctcctac 660
   aacccccctc tcacccaccc atcagaatcc cctatttccc ccacctcagg caactctgcc 720
   cacctcaggg catgcccctg cagctggccc agttcaaggt gtgggccccg cccccgcccc 780
   ccattcgctt ccagagcctg gaccacagca gcaaacattt gcggtccccc gaccaccaca 840
   tcagcccctg cagatgccaa agatgatgcc tgaaaaccag tatccatcag aacagagatt 900
   tcagagacaa ctgtctgaac cctgccaccc cttccctcct cagccaggag ttcctggaga 960
   taatcgcccc agttaccatc ggcaaatgtc agaacctatt gtccctgcag ctcccccgcc 1020
   ccctcaggga ttcaaacaag aataccatga cccactctat gaacatgggg tcccgggcat 1080
   gccagggccc ccagcacacg ggttccagtc accaatggga atcaagcagg agcctcggga 1140
   ttactgcgtc gattcagaag tgcctaactg ccagtcatcc tacatgagag ggggttattt 1200
   ctccagcagc catgaaggtt tttcatatga aaaagatccc cgattatact ttgacgacac 1260
   ttgtgttgtg cctgagagac tggaaggcaa agtcaaacag gagcctacca tgtatcgaga 1320
   ggggccccct taccagaggc gaggttccct tcagctgtgg cagttcctgg tcacccttct 1380
   tgatgaccca gccaatgccc acttcattgc ctggacaggt cgaggcatgg agttcaagct 1440
   gatagaaccg gaagaggttg ctcggcgctg gggcatccag aagaaccggc cagccatgaa 1500
   ctatgacaag ctgagccgct ctctccgcta ttactatgaa aagggcatca tgcagaaggt 1560
   ggctggagag cgatacgtct acaaatttgt ctgtgaccca gatgccctct tctccatggc 1620
   tttcccggat aaccagcgtc cgttcctgaa ggcagagtcc gagtgccacc tcagcgagga 1680
   ggacaccctg ccgctgaccc actttgaaga cagccccgct tacctcctgg acatggaccg 1740
   ctgcagcagc ctcccctatg ccgaaggctt tgcttactaa gtttctgagt ggcggagtgg 1800
   ccaaacccta gagctagcag ttcccattca ggcaaacaag ggcagtggtt ttgtttgtgt 1860
   ttttggttgt tcctaaagct tgccctttga gtattatctg gagaacccaa gctgtctctg 1920
   gattggcacc cttaaagaca gatacattgg ctggggagtg ggaacaggga ggggcagaaa 1980
   accaccaaaa ggccagtgcc tcaactcttg attctgatga ggtttctggg aagagatcaa 2040
   aatggagtct ccttaccatg gacaatacat gcaaagcaat atcttgttca ggttagtacc 2100
   cgcaaaacgg gacatagtat gtgacaatct gcatcgatca tggactacta aatgccttta 2160
   catagaaggg ctctgatttg cacaatttgt tgaaaaatca caaacccata gaaaagtaag 2220
   taggctaagt tggggaggct caaaccatta agggttaaaa atacatctta aacattggaa 2280
   agctcttcta gctgaatctg aaatattacc ccttgtctag aaaaaggggg gcagtcagaa 2340
   cagctgttcc ccactccgtg gttctcaaaa tcataaacca tggctactct tgggaaccac 2400
   ccggccatgt ggtcgccaag tagagcaagc cccctttctc ttcccaatca cgtggctgag 2460
   tgtggatgac ttttatttta ggagaagggc gattaacact tttgacagta ttttgttttg 2520
   ccctgatttg ggggattgtt ttgttttggt ggttgttttg gaaaaacagt ttataaactg 2580
   atttttgtag ttttggtatt taaagcaaaa aaacgaaaaa caaaaaacaa aaacaaacct 2640
   tttggtaact gtgcactgtg tcctttagcc agggccgtgc caacttatga agacactgca 2700
   gcttgagagg ggctttgctg aggcttcccc ttggccatgt gaaagcccgc cttgttgcct 2760
   gctttgtgct ttctgcacca gacaacctga tggaacattt gcacctgagt tgtacatttt 2820
   tgaagtgtgc agggcagcct ggacacaagc ttagattctc tatgtatagt tccccgtgtt 2880
   cactaacatg ccctctctgg aaagcatatg tatataacat gtgtcatgtc ctttggaaac 2940
   ctggtcacct ggtgaaaacc cttgggattc ttccctgggc atgactgatg acaatttcca 3000
   tttcatcagt ttgttttgtt ttcctttttc tttaaatctt ggactttaaa ccctacctgt 3060
   gtgattcagt agggtttgag acttacgtgt gatactgaca ggtaagcaac agtgctagca 3120
   ttctagattc ctgccttttt ttaaaaagaa attattctca ttgctgtatt atattggaaa 3180
   agttttaaac aaccaagcta aagctatgtg aaagttgagc tcaaagtaga ggaaaagtta 3240
   ctggtggtac cttgctgcct gctctgctgg tagaattctg tgctccccgt gacacttagt 3300
   acattaagaa tgactacact gttcctcgta tgtgaaggag gcagtgctga ctccgtgagt 3360
   gtgagacacg tgctttgaac tgcttttcta ttcatggagc actccatagt ctcaaactgt 3420
   cccccttatg accaacagca catttgtgaa gaggttcgca gggataaggg gtgcacttta 3480
   tagctatgga aacatgagat tctcctctat tggaagctaa ttagcccaca aaggtggtaa 3540
   acctgtagat tgggccttaa ttagcattgt actctaatca aaggactctt tctaaaccat 3600
   atttatagct ttcttaacct acacatagtc tatacataga tgcatatttt acccccagct 3660
   ggctagagat ttatttgttg taaatgctgt atagatttgg ttttcctttc tttacttacc 3720
   ctggtttgga tttttttttt ttttcttttg aatggattta tgctgtctta gcaatatgac 3780
   aataatcctc tgtagcttga gctacccctc ccctgctgta acttacgtga cctgtgctgt 3840
   cactgggcat aggacagcgg catcacggtt gcattcccat tggactcatg cacctcccgg 3900
   atggtttttg tttttttcgg gggttctttg gggtttgttt gtttgcttct tttccagagt 3960
   gtggaaagtc tacagtgcag aaaggcttga acctgccagc tgatttgaaa tactttcccc 4020
   tgcgcagggc cgtatgcatc ctgccaagct gcgttatatt ctgtactgtg tacaataaag 4080
   aagtttgctt ttcgtttacc aa                                          4102
<210> 248
   <211> 2298
   <212> DNA
   <213> Homo sapiens
<220>
   <223> ets variant 4 (E1A enhancer-binding protein, E1AF) transcript variant 1 (ETV4), EWS protein/E1A enhancer binding protein chimera, adenovirus E1A enhancer-binding protein, polyomavirus enhancer activator 3 homolog (PEA3, PEAS3)
<220>
   <221> CDS
   <222> (129) ... (1583)
   <223> ETV4
<400> 248
   cacgttctta tgtaaccgag cccgggtaaa gcagggctgc agaaagcaga aacggcgagc 60
   ccggctcctg ggagcaggtc tcggcccccg cttggggccc cggccgtgcg gccggaggga 120
   gcggccggat ggagcggagg atgaaagccg gatacttgga ccagcaagtg ccctacacct 180
   tcagcagcaa atcgcccgga aatgggagct tgcgcgaagc gctgatcggc ccgctgggga 240
   agctcatgga cccgggctcc ctgccgcccc tcgactctga agatctcttc caggatctaa 300
   gtcacttcca ggagacgtgg ctcgctgaag ctcaggtacc agacagtgat gagcagtttg 360
   ttcctgattt ccattcagaa aacctagctt tccacagccc caccaccagg atcaagaagg 420
   agccccagag tccccgcaca gacccggccc tgtcctgcag caggaagccg ccactcccct 480
   accaccatgg cgagcagtgc ctttactcca gtgcctatga cccccccaga caaatcgcca 540
   tcaagtcccc tgcccctggt gcccttggac agtcgcccct acagcccttt ccccgggcag 600
   agcaacggaa tttcctgaga tcctctggca cctcccagcc ccaccctggc catgggtacc 660
   tcggggaaca tagctccgtc ttccagcagc ccctggacat ttgccactcc ttcacatctc 720
   agggaggggg ccgggaaccc ctcccagccc cctaccaaca ccagctgtcg gagccctgcc 780
   caccctatcc ccagcagagc tttaagcaag aataccatga tcccctgtat gaacaggcgg 840
   gccagccagc cgtggaccag ggtggggtca atgggcacag gtacccaggg gcgggggtgg 900
   tgatcaaaca ggaacagacg gacttcgcct acgactcaga tgtcaccggg tgcgcatcaa 960
   tgtacctcca cacagagggc ttctctgggc cctctccagg tgacggggcc atgggctatg 1020
   gctatgagaa acctctgcga ccattcccag atgatgtctg cgttgtccct gagaaatttg 1080
   aaggagacat caagcaggaa ggggtcggtg catttcgaga ggggccgccc taccagcgcc 1140
   ggggtgccct gcagctgtgg caatttctgg tggccttgct ggatgaccca acaaatgccc 1200
   atttcattgc ctggacgggc cggggaatgg agttcaagct cattgagcct gaggaggtcg 1260
   ccaggctctg gggcatccag aagaaccggc cagccatgaa ttacgacaag ctgagccgct 1320
   cgctccgata ctattatgag aaaggcatca tgcagaaggt ggctggtgag cgttacgtgt 1380
   acaagtttgt gtgtgagccc gaggccctct tctctttggc cttcccggac aatcagcgtc 1440
   cagctctcaa ggctgagttt gaccggcctg tcagtgagga ggacacagtc cctttgtccc 1500
   acttggatga gagccccgcc tacctcccag agctggctgg ccccgcccag ccatttggcc 1560
   ccaagggtgg ctactcttac tagcccccag cggctgttcc ccctgccgca ggtgggtgct 1620
   gccctgtgta catataaatg aatctggtgt tggggaaacc ttcatctgaa acccacagat 1680
   gtctctgggg cagatcccca ctgtcctacc agttgcccta gcccagactc tgagctgctc 1740
   accggagtca ttgggaagga aaagtggaga aatggcaagt ctagagtctc agaaactccc 1800
   ctgggggttt cacctgggcc ctggaggaat tcagctcagc ttcttcctag gtccaagccc 1860
   cccacacctt ttccccaacc acagagaaca agagtttgtt ctgttctggg ggacagagaa 1920
   ggcgcttccc aacttcatac tggcaggagg gtgaggaggt tcactgagct ccccagatct 1980
   cccactgcgg ggagacagaa gcctggactc tgccccacgc tgtggccctg gagggtcccg 2040
   gtttgtcagt tcttggtgct ctgtgttccc agaggcaggc ggaggttgaa gaaaggaacc 2100
   tgggatgagg ggtgctgggt ataagcagag agggatgggt tcctgctcca agggaccctt 2160
   tgcctttctt ctgccctttc ctaggcccag gcctgggttt gtacttccac ctccaccaca 2220
   tctgccagac cttaataaag gcccccactt ctcccattaa aaaaaaaaaa aaaaaaaaaa 2280
   aaaaaaaaaa aaaaaaaa                                               2298
<210> 249
   <211> 7767
   <212> DNA
   <213> Homo sapiens
<220>
   <223> solute carrier family 4, sodium bicarbonate cotransporter, member 7 transcript variant 1 (SLC4A7), sodium bicarbonate cotransporter 2b (SBC2), electroneutral Na/HCO(3) cotransporter, NBC2, NBC3, NBCN1, SLC4A6
<220>
   <221> CDS
   <222> (72) ... (3716)
   <223> SLC4A7
<400> 249
   ctcactgtcc ctggaatctt caagggagtt actgcattac atcatcagaa acaaggcatt 60
   tctatattac tatggaaaga tttcgtctgg agaagaagtt acctggtcct gatgaagaag 120
   ctgttgtgga tcttggcaaa actagctcaa ctgtgaacac caagtttgaa aaagaagaac 180
   tagaaagtca tagagctgta tatattggtg ttcacgtccc gtttagtaaa gagagtcgtc 240
   ggcgtcatag gcatcgcgga cacaaacatc accaccggag aagaaaagat aaagaatcag 300
   ataaagaaga tggacgggaa tctccttctt atgatacacc atcccagaga gttcagttta 360
   tccttggtac tgaagatgat gatgaagaac atattcccca tgatctcttc acggaaatgg 420
   atgaactgtg ttacagagat ggagaagaat atgaatggaa agaaactgct agatggctga 480
   aatttgaaga ggatgttgaa gatggcggtg accgatggag taaaccttat gtggcaactc 540
   tctctttgca cagtcttttt gaactaagga gttgcatcct caatggaaca gtcatgctgg 600
   atatgagagc aagcactcta gatgaaatag cagatatggt attagacaac atgatagctt 660
   ctggccaatt agacgagtcc atacgagaga atgtcagaga agctcttctg aagagacatc 720
   atcatcagaa tgagaaaaga ttcaccagtc ggattcctct tgttcgatct tttgcagata 780
   taggcaagaa acattctgac cctcacttgc ttgaaaggaa tggggaaggc ctttcagcct 840
   cccgccactc tttgcgaaca ggtctgtctg cctcaaacct ttccttgaga ggagaatcac 900
   ctttatctct tcttcttggt catcttcttc cttcttcaag agctggaacc cctgcaggct 960
   caaggtgtac aaccccagta cccacccctc aaaacagtcc tccttctagc cctagcatca 1020
   gccgcctgac ctccagaagt tcccaagaga gtcagcgtca ggccccagaa ctactggttt 1080
   cacctgccag tgatgatatt cccacagtag taattcatcc gcctgaggaa gacttagaag 1140
   cagcgctgaa aggcgaggag cagaagaatg aggaaaatgt tgacttaact ccaggtattt 1200
   tggcctctcc ccagtctgct cctggaaact tggacaatag taaaagtgga gaaattaaag 1260
   gtaatggaag tggtggaagc agagaaaata gtactgttga cttcagcaag gttgatatga 1320
   atttcatgag aaaaattcct acgggtgctg aggcatccaa cgtcctggtg ggcgaagtag 1380
   actttttgga aaggccaata attgcatttg tgagactggc tcctgctgtc ctccttacag 1440
   ggttgactga ggtccctgtt ccaaccaggt ttttgttttt gttattgggt ccagcgggca 1500
   aggcaccaca gtaccatgaa attggacgat caatagccac tctcatgaca gatgagattt 1560
   tccatgatgt agcttataaa gcaaaagaca gaaatgacct cttatctgga attgatgaat 1620
   ttttagatca agtaactgtc ctacctccag gagagtggga tccttctata cgcatagaac 1680
   caccaaaaag tgtcccttct caggaaaaga gaaagattcc tgtgtttcac aatggatcta 1740
   cccccacact gggtgagact cctaaagagg ccgctcatca tgctgggcct gagctacaga 1800
   ggactggacg gctttttggt ggtttgatac ttgacatcaa aaggaaagca ccttttttct 1860
   tgagtgactt caaggatgca ttaagcctgc agtgcctggc ctcgattctt ttcctatact 1920
   gtgcctgtat gtctcctgta atcacttttg gagggctgct tggagaagct acagaaggca 1980
   gaataagtgc aatagagtct ctttttggag catcattaac tgggattgcc tattcattgt 2040
   ttgctgggca acctctaaca atattgggga gcacaggtcc agttctagtg tttgaaaaaa 2100
   ttttatataa attctgcaga gattatcaac tttcttatct gtctttaaga accagtattg 2160
   gtctgtggac ttcttttttg tgcattgttt tggttgcaac agatgcaagc agccttgtgt 2220
   gttatattac tcgatttaca gaagaggctt ttgcagccct tatttgcatc atattcatct 2280
   acgaggcttt ggagaagctc tttgatttag gagaaacata tgcatttaat atgcacaaca 2340
   acttagataa actgaccagc tactcatgtg tatgtactga acctccaaac cccagcaatg 2400
   aaactctagc acaatggaag aaagataata taacagcaca caatatttcc tggagaaatc 2460
   ttactgtttc tgaatgtaaa aaacttcgtg gtgtattctt ggggtcagct tgtggtcatc 2520
   atggacctta tattccagat gtgctctttt ggtgtgtcat cttgtttttc acaacatttt 2580
   ttctgtcttc attcctcaag caatttaaga ccaagcgtta ctttcctacc aaggtgcgat 2640
   cgacaatcag tgattttgct gtatttctca caatagtaat aatggttaca attgactacc 2700
   ttgtaggagt tccatctcct aaacttcatg ttcctgaaaa atttgagcct actcatccag 2760
   agagagggtg gatcataagc ccactgggag ataatccttg gtggacctta ttaatagctg 2820
   ctattcctgc tttgctttgt accattctca tctttatgga tcaacaaatc acagctgtaa 2880
   ttataaacag aaaggaacac aaattgaaga aaggagctgg ctatcacctt gatttgctca 2940
   tggttggcgt tatgttggga gtttgctctg tcatgggact tccatggttt gtggctgcaa 3000
   cagtgttgtc aataagtcat gtcaacagct taaaagttga atctgaatgt tctgctccag 3060
   gggaacaacc caagtttttg ggaattcgtg aacagcgggt tacagggcta atgattttta 3120
   ttctaatggg cctctctgtg ttcatgactt cagtcctaaa gtttattcca atgcctgttc 3180
   tgtatggtgt tttcctttat atgggagttt cctcattaaa aggaatccag ttatttgacc 3240
   gtataaaatt atttggaatg cctgctaagc atcagcctga tttgatatac ctccgttatg 3300
   tgccgctctg gaaggtccat attttcacag tcattcagct tacttgtttg gtccttttat 3360
   gggtgataaa agtttcagct gctgcagtgg tttttcccat gatggttctt gcattagtgt 3420
   ttgtgcgcaa actcatggac ctgtgtttca cgaagagaga acttagttgg cttgatgatc 3480
   ttatgccaga aagtaagaaa aagaaagaag atgacaaaaa gaaaaaagag aaagaggaag 3540
   ctgaacggat gcttcaagat gatgatgata ctgtgcacct tccatttgaa gggggaagtc 3600
   tcttgcaaat tccagtcaag gccctaaaat atagtcctga taaacctgtg agtgtgaaaa 3660
   taagttttga agatgaacca agaaagaaat acgtggatgc tgaaacttca ttatagaatt 3720
   gaaccaagag gcattataca tatagatata tacatatgta atgtgtgcgt atcatgtcac 3780
   tatatataag aatattgtat gtcatgctgt ttatgtgtga ctaccgggtt tttaaaagta 3840
   gtgtctggag tttgtaatga gcaccgtgga gactatgtat ttaatgaaat gctctctttg 3900
   aagtgaggta catggttctt aactattcaa atatttattc tgttagaaaa aaaaattttc 3960
   tgttttgcaa tagaaggatg tggagaaatg ctttcagtct acttttctta aatctctgtt 4020
   catcagtggc aattcgtaaa aaccttaagt gatactttgt ttatatgttt ataattttta 4080
   ggtgtttcct gaaattttca catattattt cacttttgtt agtgctttat gggaagaata 4140
   gggagtctat accagtgctg tgggaaaaat ggtaacattt cagggcttct ctatttgtgt 4200
   ttcatttctg tagatgtcca tcgtgtttca ctaactggcg ttttcttagc catagagatg 4260
   actgtagaac aatagaaact ttaataatga tagtttttaa cttttatgtt taaatttttt 4320
   ttaaatctta aaaccttcat atctaggtat ccattgtgac agacaagtaa aattgcaggt 4380
   gatttgataa ttaagcaccc ataccattta taacttctga atttaaaaag ttatacaatg 4440
   ccagtttgca atagttgatt ttgatgcctt tgtagaatat tttttctgaa tccttatgct 4500
   cttttaaagc caatgattcc cactctgttc tctgccttgt ctctctttgt cttaaaatgc 4560
   tttagtttcc atcaggttca agttcttgac tattattctc ttataagtag taggcgtaaa 4620
   taatcaggag ttagaattct ctcagaaggg tctatgatca gtattacttt attaagaatt 4680
   acctttcatt ttctctttat gttattcttt cacttttgta gattacattt aatagcttgt 4740
   tacctgtgat tttattttaa aatatttatt ttgatatgat gcttaaatat tatataaaca 4800
   tttggaaaag taacaaatag agtaaattgt taatgtaaat agttggtgct tactttcatt 4860
   tatgtttatt atcttaaagc aattgataga ttttacatct ttgatataaa gcactgccat 4920
   atttatattt taaaaggaaa ttagacattt tatatgtagc tcagattaat gacattttta 4980
   ttttgtgtat tagtttttgc ttttctgagc tttttaaagt ctaacaaatc tttttagtca 5040
   tcttttatat acttttagtt ccacatgaaa taaatgttgt taagcctgta ggactggatg 5100
   aatggggttg tgaaactgat ttgacaagag aataatttac aaaaatcaaa tgagtattga 5160
   gaagccacag aaatatcaaa aatggtgaca tcactattag gatgaaaatt ttatgaaatt 5220
   ccaatgctcc ctttaaccat ataattaaat tacaaaggta tacataatta atctaataaa 5280
   ggatcatgag gaatgccaaa agctgaaatt ttagcaagtc tggtgtttta aattcattat 5340
   aaactattta ttaactaatg taatgtcctc ctctaagacc atattgtaag agtcttgtta 5400
   aaatgatgga ttcaacttct gcctcggatg agaggttgga aatgtagctg cttttcttta 5460
   gaaaatatgt agctgtcatc atttggtact gcctaaaaag agttagactc tattggcaat 5520
   tgatagagta ttacccacgg tgcttattat tgtttatgag cttccattgt aatgattcct 5580
   ttttatttgt agcagcataa ttatttccaa agaccccagt atttgctgct atttttaaaa 5640
   ctcccctgat gtacctgaac aagaggattt cctcacatca tttccttgtg tctggacatc 5700
   aggggtaaca actgtactta ctttacagga agaaatttta aaactgaaaa ctacctggga 5760
   tcatagtgtt tctgtgattt tatttaattg tgtatagtaa ttatccagtg ccagaaaaac 5820
   cgtcacttgc aaaatacttt gcactcaaaa tgtttttaca atgcttctaa atgttactgg 5880
   tttctgcttt cttttgacta cttgactgac aaaatgatct gactactcca tttaagagca 5940
   aaggtaactc atgtttaggt aattaactgc ttgttttaag tgattatatt tcttccactg 6000
   tttttgaaaa ataatcaaag atagcattca ttgagagaca gtgacagata taatttacta 6060
   catatattga tttttaaata aagttgcctt aaataagtgt atgtaagcag tagtagttgc 6120
   tatgtactga tttacctcaa ggtgcaaaat aattaaacct gtacatattc catttacaaa 6180
   ataaattcag ccctgcactt tctttagatg ccttgatttc cagaatggag cttagtgcta 6240
   ctgaataccc tggccacaga gccacctcag gatattcttt tctccaccct agtttattta 6300
   tttatagata tctgtttaca aagtctgtag taaatcctga tgctgaccat ctgaaatgta 6360
   ctttttttct gaatgctgtt tcaatctaaa atagcagctt ttgagaaaac aatgatgtaa 6420
   attccttatg ataaaaggat gattctatat attctttaat gatattaaat atgccgaagc 6480
   caagcacaca gtctttctaa agtgtgtgta tgtttgtgtg aatgtgaatg atactgatct 6540
   tatatctgtt aaaagttgtt ttaaaaagct gtggcatccc attgttcata tttgccaagt 6600
   cttctgtaaa gatgtctagg acgaaatatt ttatgtgcta atgcatgtat ttgtaaacca 6660
   gatttgttta ccactcaaaa ttaacttgtt ttcttcatcc aaaaaagttt atttcttcca 6720
   cgtacttaaa ttttctgtgt gggtataata tagctttcta atttttttct ttcacaaagg 6780
   caggttcaaa attctgttga aagaaaaatg ctttctgaaa ctgaggtata acaccagagc 6840
   ttgctgttta aaggattata tgatgtacat cagttctata aatgtgctca gcagtttaac 6900
   atgtgaatcc tgttttaaag tgctcagatt tcaactgtgt aagccattga tataacgctg 6960
   taattaaaaa tgtttatatg aaataactta atgttttaaa tttatttatg tagatcacat 7020
   catttttatc gtatgcagtg caaatatgtg aaatgtcttt tggtttattc caacaattat 7080
   ttattttaga aagtaagttt aaagacttta aggacattca aagtttaaaa tagtgttcaa 7140
   attgcaaaat ttggcaatct tcatataaat tggtttcttt tctaactttt caaaaactaa 7200
   cattaaatgt caattatagg aaaacatagt tggaaatgta atcatccaaa gatcattttt 7260
   aaaatgaaat ttaattagca catattgaac atttgactta attgttaaac cccagttttg 7320
   ttttgttttt ttaatcagat ttttgcacac tgattagttt ttgtgttgtg gcttttgttg 7380
   ctttattatt caaggttttt tttttttttc ttccccatgg gggagattgt cttccaatgt 7440
   ttaactacgt ttaaataaat aaaaattgaa ttttattgtt catttatata aaatctgata 7500
   ccttgatgta atttcacaat acagttccaa tttttatggc ttttataatt acaatgatat 7560
   tttcttctat aataaaatcc aaagtaaaca tttaaattgt agaactgata tttttcattt 7620
   atatgaagta taagcctcta ctgggtctat attgtgaatc atcctgcctt tcaaatttgt 7680
   ttcataattg ttagatgaaa actatttttt tggagatgtt actgaagttg attgagcaat 7740
   aaaagtctac ttaattagta aaaaaaa 7767
<210> 250
   <211> 6824
   <212> DNA
   <213> Homo sapiens
<220>
   <223> ets variant 1 transcript variant 1 (ETV1), ETS translocation variant 1; ets-related protein 81 (ER81)
<220>
   <221> CDS
   <222> (740) ... (2173)
   <223> ETV1
<400> 250
   aaggaaacca gcccaatagt ggacctaaca ccccaataat gggaactggc gactcgaaac 60
   acacggtttc agtatcccca ggcgccttaa caataagcag cgggttacag aggaaatcga 120
   gtctctccgt ggcgcttcca ggctgaggac ccagcgccta ccggccgagc accccctagg 180
   gcagaggggc gagggagacc cgcgggaagg ggcagcaagc cgcctgattg acagcccgaa 240
   atctgatctt gtgaaagaga cgcggagcca atgggaggca gcgatccatc agtttggatt 300
   ggaggcccct ggaggagaag tagaggaaaa accaccgaat tgagctctgt cagaggcgct 360
   ttcggcttcc aagggggaag tgctgggcta taattaatgt ttttattaaa tttggaggga 420
   agtttttgca gcctttcgcc tagcgtggcc ttcagtcccg ttaggtgcaa agcaagtctc 480
   gttgatcgcc attgctagtt ttgcacacgt ttgcgaatca gagctgcccg gggtacaccg 540
   accgcgcagg gaaacatcga gagtgtaaat aaatacatcg cctcttgttc ggatttttgc 600
   tactaccgaa aatatgtaaa ttgtgaactc tgttggctct ctttggatcc aggttgatag 660
   aagtccagat cctgaggaaa tctccagcta aatgctcaaa atataaaata ctgagctgag 720
   atttgcgaag agcagcagca tggatggatt ttatgaccag caagtgcctt acatggtcac 780
   caatagtcag cgtgggagaa attgtaacga gaaaccaaca aatgtcagga aaagaaaatt 840
   cattaacaga gatctggctc atgattcaga agaactcttt caagatctaa gtcaattaca 900
   ggaaacatgg cttgcagaag ctcaggtacc tgacaatgat gagcagtttg taccagacta 960
   tcaggctgaa agtttggctt ttcatggcct gccactgaaa atcaagaaag aaccccacag 1020
   tccatgttca gaaatcagct ctgcctgcag tcaagaacag ccctttaaat tcagctatgg 1080
   agaaaagtgc ctgtacaatg tcagtgccta tgatcagaag ccacaagtgg gaatgaggcc 1140
   ctccaacccc cccacaccat ccagcacgcc agtgtcccca ctgcatcatg catctccaaa 1200
   ctcaactcat acaccgaaac ctgaccgggc cttcccagct cacctccctc catcgcagtc 1260
   cataccagat agcagctacc ccatggacca cagatttcgc cgccagcttt ctgaaccctg 1320
   taactccttt cctcctttgc cgacgatgcc aagggaagga cgtcctatgt accaacgcca 1380
   gatgtctgag ccaaacatcc ccttcccacc acaaggcttt aagcaggagt accacgaccc 1440
   agtgtatgaa cacaacacca tggttggcag tgcggccagc caaagctttc cccctcctct 1500
   gatgattaaa caggaaccca gagattttgc atatgactca gaagtgccta gctgccactc 1560
   catttatatg aggcaagaag gcttcctggc tcatcccagc agaacagaag gctgtatgtt 1620
   tgaaaagggc cccaggcagt tttatgatga cacctgtgtt gtcccagaaa aattcgatgg 1680
   agacatcaaa caagagccag gaatgtatcg ggaaggaccc acataccaac ggcgaggatc 1740
   acttcagctc tggcagtttt tggtagctct tctggatgac ccttcaaatt ctcattttat 1800
   tgcctggact ggtcgaggca tggaatttaa actgattgag cctgaagagg tggcccgacg 1860
   ttggggcatt cagaaaaaca ggccagctat gaactatgat aaacttagcc gttcactccg 1920
   ctattactat gagaaaggaa ttatgcaaaa ggtggctgga gagagatatg tctacaagtt 1980
   tgtgtgtgat ccagaagccc ttttctccat ggcctttcca gataatcagc gtccactgct 2040
   gaagacagac atggaacgtc acatcaacga ggaggacaca gtgcctcttt ctcactttga 2100
   tgagagcatg gcctacatgc cggaaggggg ctgctgcaac ccccacccct acaacgaagg 2160
   ctacgtgtat taacacaagt gacagtcaag cagggcgttt ttgcgctttt ccttttttct 2220
   gcaagataca gagaattgct gaatctttgt tttatttctg ttgtttgtat tttattttta 2280
   aataataata cacaaaaagg ggcttttcct gttgcattat tctatggtct gccatggact 2340
   gtgcacttta tttgagggtg ggtgggagta atctaaacat ttattctgtg taacaggaag 2400
   ctaatgggtg aatgggcaga gggatttggg gattactttt tacttaggct tgggatgggg 2460
   tcctacaagt tttgagtatg atgaaactat atcatgtctg tttgatttca taacaacata 2520
   agataatgtt tattttatcg gggtatctat ggtacagtta atttcacgtt gtgtaaatat 2580
   ccacttggag actatttgcc ttgggcattt tcccctgtca tttatgagtc tctgcaggtg 2640
   tacaaaaaaa ccccaatcta ctgtaaatgg cagtttaatt gttagaaatg actgtttttg 2700
   caccacttgt aaaaaggtat ttagcgattg catttgctgt ttgttgtttt attttgcttt 2760
   atatatgact tgcagaggat aaccataaaa tgggtaattc tctctgaagt tgaataatca 2820
   ccatgactgt aaatgagggg cacaattttg gactctggcg ccaaactgag tcataggcca 2880
   gtagcattac gtgtatctgg tgccaccttg ctgtttagat acaaatcata ccgtctttta 2940
   aatattttga agcccatttc agttaaataa tgacatgtca tggtcctttg gaatcttcat 3000
   ttaaatgtta aatctggaat caaaatgaag caaaaaatat ctgtctcctt ttcactttct 3060
   tcagtacata aatacattat ttaatcaata agaattaact gtactaaatc atgtattatg 3120
   ctgttctagt tacagcaaac actctttaag aaaaatatcc aatacactaa ataggtacta 3180
   tagtaatttt tagacatggt acccattgat atgcatttaa accttttact gctgtgttat 3240
   gttgataaca tatataaata ttagataatg ctaatgcttc tgctgctgtc ttttctgtaa 3300
   tattctcttt catgctgaat ttactatgac catttataag cagtgcagtt aactacagat 3360
   agcatttcag gacaaaatag atgactcaaa ccatttattg cttaaaaaat agcttacgcc 3420
   atgctatgct ataagcagct tttatgcaca ttgacaaatg aagagtaagc ttcagcttgc 3480
   taaaggaaac tgtggaacct tttgtaactt ttggtgatat ggaaaattat ttacaaaccg 3540
   tcaaagaata tgaggaagtt gctgtatgac atagtgctgg cactgatatt atccatcatc 3600
   tctttttgga cacttctgta aatgtgattg gattgtttga aagaagattt aaagtttcaa 3660
   agttttttgt tctgtttttg ctttgcattt ggagaaaata ttgaaagcag ggtatgttgt 3720
   ttcattcacc ttgaaaaaac catgagtaaa tggggatata gaatctctga atagctcgct 3780
   aaaagattca agcaagggac atgaattttg ttccatctat caataatatc cagaagaaca 3840
   acttttttaa agagtctata gcaaaaagca aaaaaaaaaa aaaattctaa acacaaagtc 3900
   aaaataaacc tattgtaaaa gcatttcgtg atgagcatga aaaagattgt ttaaagatga 3960
   tccccccagc tacccatttt ccaaaactac acagatcaca gctcatttct ctaagtggag 4020
   cagttatcaa gaaacccaaa caccaaaatt gctactcttc acatttaatc ctacaaaaag 4080
   tactccaatt tcaaaatatg tatgtaacct gcgatttcaa tgattgttgt tcatatacat 4140
   catgtattat tttggcccat tttgggccta aaaaagaaaa ctatgcctta aaaatcagaa 4200
   ccttttctcc ccactatgct tatgtggcca tctacagcac ttagaataaa aacagatgtt 4260
   aaaatattca gtgaaagttt tattggaaaa aggaattgag atatataatt gagatttggt 4320
   gaaattgaag gagaaaattt aagtgagtct ttaaaatata ttctgaatga aaactgtatt 4380
   gaggattcat ttttgttcct tttttttctt tttctctttt ctcctttttc ttctttttaa 4440
   tagtctagtt ttagtcagtc agtgaggaag aattgggcca tgctaacgtt atcacaagag 4500
   aacaatggca gaaatggtat tagttatata atatttaagg acaaactata tgttttgctg 4560
   ttttaacgta gtgactcact gaactaaata cataattgac caacattaag tgtatttcca 4620
   atacagaagg gttgaaaata ttacattata aactcttttg aaaaatgtat ctaaaatttt 4680
   ttaagttctg ttttgattcc actttttggt tgagttttta tgtttttgtt ttcaggtaga 4740
   ttaataaatc tggcagctga tttctgcaag attcttgtgt tttgaatttc tcattgaatt 4800
   ggctactcaa acatagaaat catttgttaa tgatgtaatg tcttctctca gcttttatct 4860
   tcactgctgt ttgctgtctc ttgatgatga catgttaata cccaatagat taattgcaac 4920
   aaacacttat actcaaataa ctaagtaaaa ataatttttc ttgttatgtc catgaaaagt 4980
   gcttcagaat aaaaatccac aagactgaca gtgcagaaca tttttctcaa atcatgggcg 5040
   gatcttggag gtctagtttc ccgtagatgc tgtaaccaat taccacaact tcagtaattt 5100
   acacaaattt atcttatagt tctggaggca gaagttcaaa agaagcctta agagactaaa 5160
   accaagatgt ccttaggtct ggttccttct ggaggctcca ggggagattc ttccagcttt 5220
   cacttctaga gtctgctgac attccttggc tcctggctac atcacttcaa tctctgcttc 5280
   catggtcaca tactcttcta ctatagtcaa atttccttcc tgcctcttat aaggatgctt 5340
   gtgattacat ttaggggatg ctcagataat ccaggacaat ctctccatct caagatcctt 5400
   aacttaatga cgtgtgccaa gtccctttgg ctagataatt attcataggt cccagggatt 5460
   aggacatgga tgtaaggggt gagggcaggg ctgttattca gaacaccgca cggaggagga 5520
   agactgtgta gcaaagactc taattgattt actcaggaac agtggagttc tgctgaggga 5580
   tctaggattt gaaagtacta gagtttgctt ttatttacca ctgagatatt ttccccttat 5640
   tctgcataaa taattttgaa aactttctat attaaatttc aactattcca ctaaaatgtc 5700
   tggtaatcac atcaagcctt tagattattc aaatccttcc ccagccccca ggaaaacact 5760
   aagtcatgaa acagaaaaac agaaggtatg ataataatag taataacagt taaatcagtg 5820
   gtctaatcca gattttattt tttaatacat ttcttttggt gttaatatgg gttactatgt 5880
   gatcttatca tttgctagtg attattactt attaggtaag aacaatgtgt aaaatatgtc 5940
   tattactcaa aagaacaatt gcaaaatgag tcaacttatc tttatataac caggaaagaa 6000
   atatattgcc agaagctaca gaattttgcc agatgatagg gatttctaaa atgagccact 6060
   ttgtctatca tgcagccttt tcagagcttg taatgagaaa acattacaga ggagaaggtc 6120
   atttggatgt ttgttacttg gaatcctaga aaacaaaaac taaaatttaa aaataagaag 6180
   tgagtaagct attttccatt tgcgatttgg tatggagaag agaggaaata gaattattaa 6240
   aaaaatacaa attgggtaaa agtgatggtg gaaaaaatat aaagaaggca aatgtacata 6300
   ttaagcaatt ctactaagaa ttggaaaaat caagtttcaa aaagatggta atagttgggc 6360
   atgatactag aaaatttcac ccagtttatt cagagctcaa ctagtacttt taggacttct 6420
   ttttttatat acatgagact cactttgaca tacttaaaaa aaaaacagtt tatggaaagt 6480
   acagtttaag aggagaattt gattagacta agtggatatc tttatagaaa tattaatgat 6540
   ttcagaattt tcagttacaa gtgtatatac cgtggctatt gtttatggat tcatatgtaa 6600
   ggtagggtct tttttgcata tagactccag tattagttac tttcattcta aaattatatt 6660
   tatgcttcta tggggaagaa aatttttaat tcacttggtt gtattaaaat tatacttacg 6720
   gtttgagaaa acatgctatg aaaatcatga ttatagcaaa ttaaatatgc tcaaaattta 6780
   aatctaaaat aaaagcccag aaactgaaaa aaaaaaaaaa aaaa                  6824
<210> 251
   <211> 2476
   <212> DNA
   <213> Homo sapiens
<220>
   <223> v-maf avian musculoaponeurotic fibrosarcoma oncogene homolog F transcript variant 3 (MAFF, hMafF), transcription factor MAFF, U-MAF
<220>
   <221> CDS
   <222> (339) ... (833)
   <223> MAFF
<400> 251
   cgggtcggtg acgtcaccgc atgactgggt ttttatgaat gaaaggaatc ctgtgagtga 60
   gtaattccgg gaagctcgcc ttacaactcc gcgcggcctc ggccccctgc gccgcccgcc 120
   ccacaacaaa actcagcgca gcgctcccgg gcgcccggtt cagagcgacc tgcggctcag 180
   agcggagggg agactgaccg gagcgcggat cgggacagcg gccgggacag cggcgagacg 240
   cgcgtgtgtg agcgcgccgg accaagcggg cccagaagcg ggtgaggaac gcgggaccag 300
   gaggacggtc tgcagcccag agggcacctt ctgcaaacat gtctgtggat cccctatcca 360
   gcaaagctct aaagatcaag cgagagctga gcgagaacac gccgcacctg tcggacgagg 420
   cgctgatggg gctgtcggtg cgcgagctga accggcatct gcgcgggctc tccgccgagg 480
   aggtgacacg gctcaagcag cggcgccgca cactcaaaaa ccgtggctac gccgccagct 540
   gccgcgtgaa gcgcgtgtgc cagaaggagg agctgcagaa gcagaagtcg gagctggagc 600
   gcgaggtgga caagctggcg cgcgagaacg ccgccatgcg cctggagctc gacgcgctgc 660
   gcggcaagtg cgaggcgctg cagggcttcg cgcgctccgt ggccgccgcc cgcgggcccg 720
   ccacgctcgt ggcgccggcc agcgtcatca ccatcgtcaa gtccaccccg ggctcggggt 780
   ctggccccgc ccacggcccg gaccccgccc acggcccggc ctcctgctcc tagtgcccgc 840
   ccccgccatg cctcagccac gcccctccgg cctcagctcc ctccccaaag tgcctgagcg 900
   ccgcctctgt gcccaggtcc catttctctg cagcactggc cccttggtgc acacacattc 960
   ccttcgtggg ccctgtcttc ctcttgcagc cccccaaact gggaccgaat gaccctggga 1020
   aggggaactt gggtaggttg gggatggggc agaggtctgg atctgggatc gcccttggct 1080
   gaaagtttag cctttttaga ttgagagata cagagccggc ttagagaaca gctgttgggg 1140
   gagaagaggg cacccctcat cttggaaact gctcttattg tgccaatatg ccctccaaac 1200
   cctcccagga ttcaaagcta ggtttggctg tctgtgactt acgggaccgt cctgctgaga 1260
   aattgcactg aagagatgcc cccacctctg gttgggcctg gggtgcctgg gccttccgaa 1320
   actaaaagag tgggtgggaa gactagtgaa acccagttca cggatgggga aacaggcctg 1380
   aggtcacatt tcacttagtg gttgtgttgg gaccaaaacc tgggtgtcct cactgctgcc 1440
   ctgagtccag ccatggtttt cagggggaca gtggacaggg actcagaaat gtggtgggag 1500
   ggcctccctg gcttgggaga ccgctctctg caagggaggg ggagagaagc agagggagag 1560
   agaaggtgac acggatggaa gagtgggaag gagctggcct ggctcagccc taggctgtcc 1620
   ctgcagccag ggtgtccggg ggctggccag tcagagaaag ggggccatgg actgctgtgg 1680
   caaataggga gacaaggaga cagaccctgc agtcctacta cagtctggag tggggtccta 1740
   agaagaaggg tcccacctca acccctgtca gtgtccactg tggggtgggg gctgacccct 1800
   gcctttgatt gtcattctcc tgggaagccc agtctcagtc cctcccccaa cactgtccac 1860
   actgcccctc cccactgttt atttattgca cggatctaag ttattctccc cagccagagc 1920
   ccgagctcct gctccctggg aaaagtggcg tatggccctg agctgggctt tatattttat 1980
   atctgcaaat aaatcacatt ttatcttata tttagggaaa gccggagagc aacaacaaaa 2040
   aatgtttaag ccgggcgcgg tggctcacat ctgtaatccc agcactttgg gagtccaagg 2100
   agggggatcg cttgagtcca ggagtttgag accagcctgg acaacatggt gaaaccccat 2160
   ctctacaaaa aatacaaaaa ttagccatgc atggtggctc atgcctgtag tcccagctac 2220
   ttgggaggct gaggcaggag gatcacttaa gcccagaagg cagaggttgt agtgagctga 2280
   gatcgcacca ctgcactcca gcctgggcaa catagcaaaa tcctgtctca aaaaaaaagt 2340
   taaaaaatat tgcccggctc ctagaattta tttatttcct gacttacagc aagcgagtta 2400
   tcgtcttctg tattttgtag actttctaaa taaagtcaaa ttctttcttt ttccacagag 2460
   aataaaaaaa aaaaaa                                                  2476
<210> 252
   <211> 1254
   <212> DNA
   <213> Homo sapiens
<220>
   <223> immediate early response 3 (IER3), immediate early protein GLY96, radiation-inducible immediate-early gene IEX-1, anti-death protein, differentiation-dependent gene 2 protein (DIF-2, DIF2), PACAP-responsive gene 1 protein (PRG1)
<220>
   <221> CDS
   <222> (33) ... (503)
   <223> IER3
<400> 252
   ctcacttggc cttacactcc gctcggctca ccatgtgtca ctctcgcagc tgccacccga 60
   ccatgaccat cctgcaggcc ccgaccccgg ccccctccac catcccggga ccccggcggg 120
   gctccggtcc tgagatcttc accttcgacc ctctcccgga gcccgcagcg gcccctgccg 180
   ggcgccccag cgcctctcgc gggcaccgaa agcgcagccg cagggttctc taccctcgag 240
   tggtccggcg ccagctgcca gtcgaggaac cgaacccagc caaaaggctt ctctttctgc 300
   tgctcaccat cgtcttctgc cagatcctga tggctgaaga gggtgtgccg gcgcccctgc 360
   ctccagagga cgcccctaac gccgcatccc tggcgcccac ccctgtgtcc gccgtcctcg 420
   agccctttaa tctgacttcg gagccctcgg actacgctct ggacctcagc actttcctcc 480
   agcaacaccc ggccgccttc taactgtgac tccccgcact ccccaaaaag aatccgaaaa 540
   accacaaaga aacaccaggc gtacctggtg cgcgagagcg tatccccaac tgggacttcc 600
   gaggcaactt gaactcagaa cactacagcg gagacgccac ccggtgcttg aggcgggacc 660
   gaggcgcaca gagaccgagg cgcatagaga ccgaggcaca gcccagctgg ggctaggccc 720
   ggtgggaagg agagcgtcgt taatttattt cttattgctc ctaattaata tttatatgta 780
   tttatgtacg tcctcctagg tgatggagat gtgtacgtaa tatttatttt aacttatgca 840
   agggtgtgag atgttccccc tgctgtaaat gcaggtctct tggtatttat tgagctttgt 900
   gggactggtg gaagcaggac acctggaact gcggcaaagt aggagaagaa atggggagga 960
   ctcgggtggg ggaggacgtc ccggctggga tgaagtctgg tggtgggtcg taagtttagg 1020
   aggtgactgc atcctccagc atctcaactc cgtctgtcta ctgtgtgaga cttcggcgga 1080
   ccattaggaa tgagatccgt gagatccttc catcttcttg aagtcgcctt tagggtggct 1140
   gcgaggtaga gggttggggg ttggtgggct gtcacggagc gactgtcgag atcgcctagt 1200
   atgttctgtg aacacaaata aaattgattt actgtctgca aaaaaaaaaa aaaa       1254
<210> 253
   <211> 3987
   <212> DNA
   <213> Homo sapiens
<220>
   <223> leukemia inhibitory factor transcript variant 1 (LIF), cholinergic differentiation factor (CDF), hepatocyte-stimulating factor III, melanoma-derived LPL inhibitor (MLPLI), differentiation-inducing factor (DIF)
<220>
   <221> CDS
   <222> (157) ... (765)
   <223> LIF
<400> 253
   cttcctggac tggggatccc ggctaaatat agctgtttct gtcttacaac acaggctcca 60
   gtatataaat caggcaaatt ccccatttga gcatgaacct ctgaaaactg ccggcatctg 120
   aggtttcctc caaggccctc tgaagtgcag cccataatga aggtcttggc ggcaggagtt 180
   gtgcccctgc tgttggttct gcactggaaa catggggcgg ggagccccct ccccatcacc 240
   cctgtcaacg ccacctgtgc catacgccac ccatgtcaca acaacctcat gaaccagatc 300
   aggagccaac tggcacagct caatggcagt gccaatgccc tctttattct ctattacaca 360
   gcccaggggg agccgttccc caacaacctg gacaagctat gtggccccaa cgtgacggac 420
   ttcccgccct tccacgccaa cggcacggag aaggccaagc tggtggagct gtaccgcata 480
   gtcgtgtacc ttggcacctc cctgggcaac atcacccggg accagaagat cctcaacccc 540
   agtgccctca gcctccacag caagctcaac gccaccgccg acatcctgcg aggcctcctt 600
   agcaacgtgc tgtgccgcct gtgcagcaag taccacgtgg gccatgtgga cgtgacctac 660
   ggccctgaca cctcgggtaa ggatgtcttc cagaagaaga agctgggctg tcaactcctg 720
   gggaagtata agcagatcat cgccgtgttg gcccaggcct tctagcagga ggtcttgaag 780
   tgtgctgtga accgagggat ctcaggagtt gggtccagat gtgggggcct gtccaagggt 840
   ggctggggcc cagggcatcg ctaaacccaa atgggggctg ctggcagacc ccgagggtgc 900
   ctggccagtc cactccactc tgggctgggc tgtgatgaag ctgagcagag tggaaacttc 960
   catagggagg gagctagaag aaggtgcccc ttcctctggg agattgtgga ctggggagcg 1020
   tgggctggac ttctgcctct acttgtccct ttggcccctt gctcactttg tgcagtgaac 1080
   aaactacaca agtcatctac aagagccctg accacagggt gagacagcag ggcccagggg 1140
   agtggaccag cccccagcaa attatcacca tctgtgcctt tgctgcccct taggttggga 1200
   cttaggtggg ccagaggggc taggatccca aaggactcct tgtcccctag aagtttgatg 1260
   agtggaagat agagaggggc ctctgggatg gaaggctgtc ttcttttgag gatgatcaga 1320
   gaacttgggc ataggaacaa tctggcagaa gtttccagaa ggaggtcact tggcattcag 1380
   gctcttgggg aggcagagaa gccaccttca ggcctgggaa ggaagacact gggaggagga 1440
   gaggcctgga aagctttggt aggttcttcg ttctcttccc cgtgatcttc cctgcagcct 1500
   gggatggcca gggtctgatg gctggacctg cagcaggggt ttgtggaggt gggtagggca 1560
   ggggcaggtt gctaagtcag gtgcagaggt tctgagggac ccaggctctt cctctgggta 1620
   aaggtctgta agaaggggct ggggtagctc agagtagcag ctcacatctg aggccctggg 1680
   aggccttgtg aggtcacaca gaggtacttg agggggactg gaggccgtct ctggtcccca 1740
   gggcaaggga acagcagaac ttagggtcag ggtctcaggg aaccctgagc tccaagcgtg 1800
   ctgtgcgtct gacctggcat gatttctatt tattatgata tcctatttat attaacttat 1860
   tggtgctttc agtggccaag ttaattcccc tttccctggt ccctactcaa caaaatatga 1920
   tgatggctcc cgacacaagc gccagggcca gggcttagca gggcctggtc tggaagtcga 1980
   caatgttaca agtggaataa gccttacggg tgaagctcag agaagggtcg gatctgagag 2040
   aatggggagg cctgagtggg agtggggggc cttgctccac ccccccccat cccctactgt 2100
   gacttgcttt agggtgtcag ggtccaggct gcaggggctg ggccaatttg tggagaggcc 2160
   gggtgccttt ctgtcttgat tccagggggc tggttcacac tgttcttggg cgccccagca 2220
   ttgtgttgtg aggcgcactg ttcctggcag atattgtgcc ccctggagca gtgggcaaga 2280
   cagtccttgt ggcccaccct gtccttgttt ctgtgtcccc atgctgcctc tgaaatagcg 2340
   ccctggaaca accctgcccc tgcacccagc atgctccgac acagcaggga agctcctcct 2400
   gtggcccgga cacccataga cggtgcgggg ggcctggctg ggccagaccc caggaaggtg 2460
   gggtagactg gggggatcag ctgcccattg ctcccaagag gaggagaggg aggctgcaga 2520
   tgcctgggac tcagaccagg aagctgtggg ccctcctgct ccacccccat cccactccca 2580
   cccatgtctg ggctcccagg cagggaaccc gatctcttcc tttgtgctgg ggccaggcga 2640
   gtggagaaac gccctccagt ctgagagcag gggagggaag gaggcagcag agttggggca 2700
   gctgctcaga gcagtgttct ggcttcttct caaaccctga gcgggctgcc ggcctccaag 2760
   ttcctccgac aagatgatgg tactaattat ggtacttttc actcactttg cacctttccc 2820
   tgtcgctctc taagcacttt acctggatgg cgcgtgggca gtgtgcaggc aggtcctgag 2880
   gcctggggtt ggggtggagg gtgcggcccg gagttgtcca tctgtccatc ccaacagcaa 2940
   gacgaggatg tggctgttga gatgtgggcc acactcaccc ttgtccagga tgcagggact 3000
   gccttctcct tcctgcttca tccggcttag cttggggctg gctgcattcc cccaggatgg 3060
   gcttcgagaa agacaaactt gtctggaaac cagagttgct gattccaccc ggggggcccg 3120
   gctgactcgc ccatcacctc atctccctgt ggacttggga gctctgtgcc aggcccacct 3180
   tgcggccctg gctctgagtc gctctcccac ccagcctgga cttggcccca tgggacccat 3240
   cctcagtgct ccctccagat cccgtccggc agcttggcgt ccaccctgca cagcatcact 3300
   gaatcacaga gcctttgcgt gaaacagctc tgccaggccg ggagctgggt ttctcttccc 3360
   tttttatctg ctggtgtgga ccacacctgg gcctggccgg aggaagagag agtttaccaa 3420
   gagagatgtc tccgggccct tatttattat ttaaacattt ttttaaaaag cactgctagt 3480
   ttacttgtct ctcctcccca tcgtccccat cgtcctcctt gtccctgact tggggcactt 3540
   ccaccctgac ccagccagtc cagctctgcc ttgccggctc tccagagtag acatagtgtg 3600
   tggggttgga gctctggcac ccggggaggt agcatttccc tgcagatggt acagatgttc 3660
   ctgccttaga gtcatctcta gttccccacc tcaatcccgg catccagcct tcagtcccgc 3720
   ccacgtgcta gctccgtggg cccaccgtgc ggccttagag gtttccctcc ttcctttcca 3780
   ctgaaaagca catggccttg ggtgacaaat tcctctttga tgaatgtacc ctgtggggat 3840
   gtttcatact gacagattat ttttatttat tcaatgtcat atttaaaata tttatttttt 3900
   ataccaaatg aatacttttt tttttaagaa aaaaaagaga aatgaataaa gaatctactc 3960
   ttggctggca aaaaaaaaaa aaaaaaa 3987
<210> 254
   <211> 4992
   <212> DNA
   <213> Homo sapiens
<220>
   <223> sprouty homolog 4 (Drosophila) transcript variant 1 (SPRY4, spry-4), HH17
<220>
   <221> CDS
   <222> (242) ... (1210)
   <223> SPRY4
<400> 254
   attcataaaa aagccatttt cccaggcagt ggttgcaaca tcgccgcgga ggtagcgagc 60
   tgagctgaca gcgcggagct ggcgctgtgg agcgcaggga gccttgccgg ttcctccgac 120
   cggcgtctgc gagtacagcg gcggctaacc tgccccggct tcaggattta cacagacgtg 180
   gggcgatgct tgtgaccctg cagctcctca aaccagcctg tattgagcgg tttgcagcct 240
   gatgctcagc cccctcccca cagggcccct agaagcctgt ttctccgtac agtccaggac 300
   ctccagcccc atggagcccc cgatcccaca gagcgccccc ttgactccca actcagtcat 360
   ggtccagccc cttcttgaca gccggatgtc ccacagccgg ctccagcacc cactcaccat 420
   cctacccatt gaccaggtga agaccagcca tgtggagaat gactacatag acaaccctag 480
   cctggccctg accaccggcc caaagcggac ccggggcggg gccccagagc tggccccgac 540
   gcccgcccgc tgtgaccagg atgtcaccca ccattggatc tccttcagcg ggcgccccag 600
   ctctgtgagc agcagcagca gcacatcctc tgaccaacgg ctcttagacc acatggcacc 660
   accacccgtg gctgaccagg cctcaccaag ggctgtgcgc atccagccca aggtggtcca 720
   ctgccagccg ctggacctca agggcccggc ggtcccaccc gagctggaca agcacttctt 780
   gctgtgcgag gcctgtggga agtgtaaatg caaggagtgt gcatcccccc ggacgttgcc 840
   ttcctgctgg gtctgcaacc aggagtgcct gtgctcagcc cagactctgg tcaactatgg 900
   cacgtgcatg tgtttggtgc agggcatctt ctaccactgc acgaatgagg acgatgaggg 960
   ctcctgcgct gaccacccct gctcctgctc ccgctccaac tgctgcgccc gctggtcctt 1020
   catgggtgct ctctccgtgg tgctgccctg cctgctctgc tacctgcctg ccaccggctg 1080
   cgtgaagctg gcccagcgtg gctacgaccg tctgcgccgc cctggttgcc gctgcaagca 1140
   cacgaacagc gtcatctgca aagcagccag cggggatgcc aagaccagca ggcccgacaa 1200
   gcctttctga cagtttgtgt cgaagcccca gtgctctgcc tggaaacctg gttctcttct 1260
   gacatctaag aagactgcag caaggtcaga ggttttagcc tcctgaggct gaccttgcta 1320
   gtctgcccac tccctacccc cagcttcgga aaatacagag accaccacca cgtaccctgt 1380
   attccccaag gtgatgaaga agcactttgg ggcttttttt cagggtcctg aaactttgtg 1440
   tcaaacagac aatgcagggg cagggtgtgg tttgggggga aatttttctt tttcagaaga 1500
   cagaacacag atgtggacac atatccggaa actgcagctg cttgaatgcc ttcccagccc 1560
   ctccttctcc ctccctccct ccgccccccc ccccttcctc ttttccattg tctttggctc 1620
   tcacaggagc tagctgcctg ggaggaattg ttaactgagt accagggtac ctttaaagaa 1680
   gacccttgga gtcttctata ccttcttctc cttccccatc tcactccacc ccactttgtc 1740
   cctgatgtct tggggaaggt gtagaacacc ctagcagttc ctattgtata tacttgggag 1800
   ccactgagaa cagaggacgg ccagtgagtc caagcctcgt tcctccttct gcctccccgg 1860
   agccacagga tggatttagg agccactgct cagtgcactt ctcccttcca actgcatcaa 1920
   ctaactctcg ggggtgttct gctcaccaca ccgtccttcg gttcttactg agtcacagac 1980
   tcgcctgccc actacgtgtc ctgggttctc tctactcaga tcccttccag aaactttata 2040
   tgggtagagg aagccagggc ggcaaatgcg agaccaaata tcattttgcc aatgagtctg 2100
   aggctgtggt ctctggatcc agtcattatg tttttataga ataattaaac cggatgctaa 2160
   cggtgtttta aaaaataata ataaaacaac ttgtttcctt ttggccaccc ccaggaaggg 2220
   ctgatttcaa aatctggggg cgagcaacct caaggaacac aatttccctc cctatcaaca 2280
   agaggatttt aacagcaaag aagagaggca gcacctccca ttggcagaat gaccgctgag 2340
   ccaggctggg tttgggtttc ttctcttctg attctgctgc tcactgtcat agccttttgt 2400
   gtatagtgat gtgtctgtat ctttaatgta aatagagaga tgatgaaaaa agagtctatt 2460
   ttagtgttag gaagccccag caggggagtc ggaagagctt ggaagagctg gggagagggt 2520
   aggggaaagg tttttccagg ggccactggg tttgagccct gcttctgtgc acagccacac 2580
   caccctctcc cgacagccct caaagacgta gcaactcttt ctctcaaggt gctaaaggac 2640
   tcagaaggtg cagcacgtcc agtgggtagg tacttgttgc atgcaaaagc tgtagtgtat 2700
   ctggtccttc ctccccagct tttgtgtggg gttcttgctt tgtgtggtat tttgttttcc 2760
   cctctaatga gagggcatgg cctgagtcag aagagctacc ccaggtgaaa ctggaagtgc 2820
   atgaggcaga gcgtccgtag catttccagt ttgttctgta taggaacaga ggtgcctccg 2880
   ggaaggaggc agcgaggtag gtagctatga taggcaccta atgcttctca aggacttatt 2940
   ttttccttct tgaagactag tagtaacatc ttatgattta gagtaagttg attgtaacca 3000
   taggtattta ttgattggag gaagggaggg tcatattatt ttcggcttta tttatgtaac 3060
   atttgctagc ttgtaaaagg cgaatgtgaa atattgcatc tgcattttcc aaggctgatt 3120
   cgtgtagcta cccttgccac agttgtgacg gatgtatgga tgttcttgaa catttcagaa 3180
   ggagtggtag aaaaaaacac acattcagcc aaccacttat atgaattgaa tgtatcagaa 3240
   gtgtactgaa gggactggag atggttttcc tcagatgagg gggccccaaa attgatagtg 3300
   cacatctgca cgctttctgc gaggcctcag aacttcccag ggcccctccc tcaaattgtc 3360
   tccatgggaa acttgaccca gtggcaagtt gcactttggt gatcttggtg gtctacacac 3420
   ccgttctgtg gagagtcgat ttacataagc tgtgtataca cacacacaca cacacacaca 3480
   cacccctacc ccacactgac tgtctaccga caaagaccct atttcctggc aaacggcctc 3540
   ctgaaccctg actttttgtg tacatacttg taaacacgga tttttctggg ttttggtttg 3600
   ctttttcctt ttttccccct gcccctgttc tagcttgttc ttcttggttt gctttcaacc 3660
   tgcttgatgg atgtctgcag agtgctctct aagagtccac ctcagtgcct cgtgtgctca 3720
   gtggtcatgg gaaaggagcg aaggaaccat ccttggttct cccagcttgg ttgtgtagca 3780
   atccctcagc attgtttttc tcagcttctt ggcaaaaatt aaaacaacaa caacaacaac 3840
   aacaacaaca acaaacagaa ggataaactg gcttgcctgt ggaccctccc cggctctggg 3900
   gccagtcgag agccactgag ggacccagca ctcagagaca caacacacat gtgtagctgc 3960
   ttctggctga gtgtgtttcc tgtcaccaat ggcctgtttg gctggacgat gcctcggctt 4020
   gacctttttt gaaaagtgct ggttagttcc cgcccctggt aaacctgggg taggtggggg 4080
   ttctgtctta actcgagggg cacctgggat ccaggacgct tctagggggc tctggctgcc 4140
   cgtgttaatg aaggacagcg cttccgcgag caccctggga actgggtctt gggtagcaaa 4200
   gccctcccag agaaaagatt gggcacaact aaggctttcc tgagcaggaa gggggtgaag 4260
   accaatccct tcctttggtc ctttggtacg caccccctca gagctgagat ggaagacatg 4320
   gctagttctt ttcagccttg tggagcctgt cagtcgccat catacctcga gtgaggccca 4380
   gctagataat gacttgtcca agatggcaca cgtggaaagt tgatctgcac cagaacccgg 4440
   atgactgtca ccttgaagca tcctgttctc cttctgtgct gtcccaggaa gtgtctggcg 4500
   ggcgtgggca gcacagctct acactgtacg attcactagg gcatcctgcg agcctcacta 4560
   gccttctggt tcatgccttt gacaagcatt tttgtgcccc ctctgcttac tgtgacagtc 4620
   gatgatgaat cttgcgttgc cattttctgc tgtgggtaac tgcgtgcagt gtcttgcctt 4680
   gctttctctt cttactgtcc cacagcttgg tttcatgtta caaacagaaa agctcgaggc 4740
   tcccaccccg ccacatccca acttcatttc cccctcactg tagcccattt ccaccccacc 4800
   acaaagttgc cacaggtttt ctttgtatag aatatttatt ttgaagctct attttaatag 4860
   tatttatttt agaaagtcta ctattgtaag agttcttctg tttgtgaaga aaaaaacaag 4920
   ttaaaaactg aatgtactga tttagaaaat atatataaat atatattgtt aaatatacac 4980
   gggactgccg tt                                                     4992
<210> 255
   <211> 5625
   <212> DNA
   <213> Homo sapiens
<220>
   <223> dual specificity phosphatase 4 transcript variant 1 (DUSP4), serine/threonine specific protein phosphatase, mitogen-activated protein kinase phosphatase 2, MAP kinase phosphatase 2 (MKP-2, MKP2), TYP
<220>
   <221> CDS
   <222> (473) ... (1657)
   <223> DUSP4
<400> 255
   acctagcgcc ccctcccccg ggagcgcgga ggagcattaa taaacctcta agccgaggag 60
   aaaactctgg ctggggcagt gcgctgagcg ccggaggagc gtaggcaggg cagcgctggc 120
   gccagtggcg acaggagccg cgcgaccggc aaaaatacac gggaggccgt cgccgaaaag 180
   agtccgcggt cctctctcgt aaacacactc tcctccaccg gcgcctcccc ctccgctctg 240
   cgcgccgccc ggctgggcgc ccgaggccgc tccgactgct atgtgaccgc gaggctgcgg 300
   gaggaagggg acagggaaga agaggctctc ccgcgggagc ccttgaggac caagtttgcg 360
   gccacttctg caggcgtccc ttcttagctc tcgcccgccc ctttctgcag cctaggcggc 420
   ccgggttctc ttctcttcct cgcgcgccca gccgcctcgg ttcccggcga ccatggtgac 480
   gatggaggag ctgcgggaga tggactgcag tgtgctcaaa aggctgatga accgggacga 540
   gaatggcggc ggcgcgggcg gcagcggcag ccacggcacc ctggggctgc cgagcggcgg 600
   caagtgcctg ctgctggact gcagaccgtt cctggcgcac agcgcgggct acatcctagg 660
   ttcggtcaac gtgcgctgta acaccatcgt gcggcggcgg gctaagggct ccgtgagcct 720
   ggagcagatc ctgcccgccg aggaggaggt acgcgcccgc ttgcgctccg gcctctactc 780
   ggcggtcatc gtctacgacg agcgcagccc gcgcgccgag agcctccgcg aggacagcac 840
   cgtgtcgctg gtggtgcagg cgctgcgccg caacgccgag cgcaccgaca tctgcctgct 900
   caaaggcggc tatgagaggt tttcctccga gtacccagaa ttctgttcta aaaccaaggc 960
   cctggcagcc atcccacccc cggttccccc cagtgccaca gagcccttgg acctgggctg 1020
   cagctcctgt gggaccccac tacacgacca ggggggtcct gtggagatcc ttcccttcct 1080
   ctacctcggc agtgcctacc atgctgcccg gagagacatg ctggacgccc tgggcatcac 1140
   ggctctgttg aatgtctcct cggactgccc aaaccacttt gaaggacact atcagtacaa 1200
   gtgcatccca gtggaagata accacaaggc cgacatcagc tcctggttca tggaagccat 1260
   agagtacatc gatgccgtga aggactgccg tgggcgcgtg ctggtgcact gccaggcggg 1320
   catctcgcgg tcggccacca tctgcctggc ctacctgatg atgaagaaac gggtgaggct 1380
   ggaggaggcc ttcgagttcg ttaagcagcg ccgcagcatc atctcgccca acttcagctt 1440
   catggggcag ctgctgcagt tcgagtccca ggtgctggcc acgtcctgtg ctgcggaggc 1500
   tgctagcccc tcgggacccc tgcgggagcg gggcaagacc cccgccaccc ccacctcgca 1560
   gttcgtcttc agctttccgg tctccgtggg cgtgcactcg gcccccagca gcctgcccta 1620
   cctgcacagc cccatcacca cctctcccag ctgttagagc cgccctgggg gccccagaac 1680
   cagagctggc tcccagcaag ggtaggacgg gccgcatgcg ggcagaaagt tgggactgag 1740
   cagctgggag caggcgaccg agctccttcc ccatcatttc tccttggcca acgacgaggc 1800
   cagccagaat ggcaataagg actccgaata cataataaaa gcaaacagaa cactccaact 1860
   tagagcaata acggctgccg cagcagccag ggaagacctt ggtttggttt atgtgtcagt 1920
   ttcacttttc cgatagaaat ttcttacctc atttttttaa gcagtaaggc ttgaagtgat 1980
   gaaacccaca gatcctagca aatgtgccca accagcttta ctaaaggggg aggaagggag 2040
   ggcaaaggga tgagaagaca agtttcccag aagtgcctgg ttctgtgtac ttgtcccttt 2100
   gttgtcgttg ttgtagttaa aggaatttca ttttttaaaa gaaatcttcg aaggtgtggt 2160
   tttcatttct cagtcaccaa cagatgaata attatgctta ataataaagt atttattaag 2220
   actttcttca gagtatgaaa gtacaaaaag tctagttaca gtggatttag aatatattta 2280
   tgttgatgtc aaacagctga gcaccgtagc atgcagatgt caaggcagtt aggaagtaaa 2340
   tggtgtcttg tagatatgtg caaggtagca tgatgagcaa cttgagtttg ttgccactga 2400
   gaagcaggcg ggttgggtgg gaggaggaag aaagggaaga attaggtttg aattgctttt 2460
   taaaaaaaaa agaaaagaaa aagacagcat ctcactatgt tgccaaggct catcttgaga 2520
   agcaggcggg ttgggtggga ggaggaagaa agggaagaat taggtttgaa ttgctttttt 2580
   aaaaaaaaag aaaagaaaaa aaaagacagc atctcactat gttgccaagg ctcatctcaa 2640
   gctcttgggc tcaagagatc ctcccacctc ggcctcctga gtagctggga ctgcaggtgt 2700
   gtgtcatcat gaccaatgtg aattgctttt gaagctggtt catgggcatg taggccaccg 2760
   aagcaatttt agaccacagt aagtcaagct tttttccctc cgatgatcac tgggtggttg 2820
   cagcattttt tgcataaacc tgcctaagac ttgtctatcg tctgtgatca atatgccata 2880
   ttacactaag gtgctcctgg aaaattgggt gcagttcaaa ttttcctaca gcaaatcatt 2940
   tggcaaggcc agccattggg gaaaccagac aactagagat aaccctgaaa tgaatccttt 3000
   tgtaaattga agcaccatct tttctttttt tgcataaatt ggaggtttta attttagggc 3060
   agttacctga agtgaaatat accaacaatt tcttgtgttc tttaaattcc tagttaggtg 3120
   aatatttttg aaggtcctct tttgaataaa gaggggaatg gacaccacat ttcaggtctt 3180
   ctcgaagtgt ggaagggcaa gagagcatca gtgagctgat ggtggattgc ttacatcgga 3240
   ttccattggt atgaatttcc caaactggaa atcaaagcgc cagggtgggg ttggggctga 3300
   ctgctggtga gggggctggc cgctggctcc cgtgacgtgc gtcatgggca cgcaggcgcc 3360
   attttgaatc tatcgtcggc acgtgggtgc cattttgaat ccttagttgg gcctttctaa 3420
   atggagaatg gctttggagg gagacacgtt ttctgtgggg agggtttggg ggggagggag 3480
   gagggaacaa gctacatgct attttgtttg tagtattgtg gaacagtctt gttatggagt 3540
   gccagcttag aggttgttgc aaacttgtct agaagtgaga gcatggtttt ttttagccct 3600
   ttgagagtct acatctaatg aacattcttg ctcacccata aataacgtca agcctcaatg 3660
   tcaccgtcac gttgggatac tctttctcat ctggcatcct agacaggaca aggttggtta 3720
   cctttccttc catgaaccat gaacctgtga cggcatcatt catcctgact tcaccaagct 3780
   ccgcctgtgg gtgaggccag agctcccact ggcaattttt agaagagcca gaggctccct 3840
   gcttcctcta gaaataacag ttcagggtga agcatggagg gtttcagttc ccagacaatg 3900
   gaaccattta gagacaacac agttggacat ttccactttt tccttgattc ctggaagtcc 3960
   agtgggttct gcagctgaaa aagccctggg tcccagcagc agagagacag gacagagggg 4020
   atgcttgggc ggggagggac ggtaacctgc agaacagatt ccatttttat agaacgagta 4080
   cacgtttgct aaaacagtcc tgctttccca gactggattc ccaccacagg gacagtcgga 4140
   actcaggact agctccagcg acatctttcc tccgaattca agccttctat cacaatgtca 4200
   aaacagctat ttataaagcc attttcattg tacttgataa cagcacgagt cccaaaactt 4260
   ttagaaataa aataggacat tggcttgatt gaaaagaggg actttttaaa aattgttctt 4320
   tcgtcagaag ccttttggat gacttacaat agctctgatg aagataccac cccagcgtca 4380
   gtccaatagg tcagtgagtt tcaacaggca tccatccctc ccatgaaggg attctggtga 4440
   tgggaagttt ctgtaatgac aggaaagcat tgaccctcat tgattgtcaa ctttggtatt 4500
   agccatgaaa gacaggatgc tcattgggtg ttctgtagag tgaggaatgc tgcctattcc 4560
   ctcccagaac gtctgaccca ggggtgtgtg ttgaggagcc ctgggggaaa tggaccaagt 4620
   tttcccacag agcagtatta ggctgaagag caggtgactg gtaggcccca gctcccatca 4680
   ttccctccca aagccatttt gttcagttgc tcatccacgc tggattccag agagttttcc 4740
   aatttgggaa gccatgagaa aggtttttaa atcttgggaa gatggagaga gggacatagg 4800
   atagttgact ccaacatgac aggaagaggc tggagattgg gaattggcca tcaaccaagc 4860
   ctgtagtagt aaagccatgg tcccgcattg gaattacttg gggaacttat acagttctga 4920
   tacccaggct ctcctagacc agttcaacca attctaggtg ggggactcag gcatcagtgt 4980
   gtttcgtagc tccccgggtg ttttccctgt gcagccgagc ttgggaaact gccatgcttt 5040
   ttggatgtca aggcgctgtt ggaggctggg tgtgacagca cagagccagg ttgtcttgtg 5100
   gaaaccacag ccacgggttt gccactggct cagcatggcc tcactgccag tcccagcctg 5160
   gctgagggac aagatggttt ctcttgggag ttcctgagtg gagcaccctt ccaggctttt 5220
   tgaaagccag ctgatctgtg gagccttgtt aagggactca atacggtgtt tggatattga 5280
   tgtttttcct tgagactgtc ttgtccatca ataaagatgg aggatgtctc ctctttgaac 5340
   cccgcttccc caccagtact ctctctccct tagagtttat gagttattca aggaggagac 5400
   ttcttaaaga cagcaacgca attcttgtaa cttgtgtaaa tagccccatc tttcagagtg 5460
   ataccatttc tacatttgat aatgcctgta ttcctgtagg atgtatatag tttaggggat 5520
   tttttttttg tttggttttg ttttttagaa gtcaatatgt ctggttttat ttattgcttg 5580
   aaaaagatca tttgaaaaaa ataaatacat tttcaaccac taaaa                5625
<210> 256
   <211> 5425
   <212> DNA
   <213> Homo sapiens
<220>
   <223> SH2B adaptor protein 3 (SH2B3), signal transduction protein Lnk, lymphocyte-specific adapter protein Lnk, IDDM20
<220>
   <221> CDS
   <222> (358) ... (2085)
   <223> SH2B3
<400> 256
   cccgggccac cgcctccgcc cggctgcccg cccggactgt cgcggcccgc ggtggcgacg 60
   gcggccgctg caaagtttcc ccggcggcgg cggcccgggg gcgcatcctc ccgcaactgt 120
   caagcgctgg cggcggaaat gatgaggcgc tggccatttt ccgagcccgg gtttcctgcc 180
   tgagccccgc tcgagcgagc cgcgagcgag gagccggcgg gcgggagagg acgcgcccag 240
   ggcgggggcc cgcccgcccc ctcgggattt cgagggcccg ggggcgcgcg acgccatggg 300
   ccggccgggc ccagagctcc tgtctctcag cccggccgca ccacctgggt ctccgccatg 360
   aacgggcctg ccctgcagcc ctcctcgccc tcttccgcgc cctcagcctc cccggcggcg 420
   gccccgcggg gctggagcga gttctgtgag ttgcacgccg tagcggcggc ccgggagctg 480
   gcccgccagt actggctgtt cgcccgggag catccgcagc acgcgccgct gcgcgccgag 540
   ctggtgtcgc tgcagttcac cgacctcttc cagcgctact tctgccgcga ggtgcgcgac 600
   ggacgggcgc cgggccgcga ctaccgggac acaggccgtg ggcccccagc caaggccgag 660
   gcgtccccgg agccaggccc cggccccgcc gcccctggcc tgcccaaggc ccgcagctct 720
   gaggagctgg ccccgccgcg gccgcccggg ccctgctcct tccagcactt tcgccgcagc 780
   ctccgccaca tcttccgccg ccgctcggcc ggggagctgc cagcggccca caccgctgcc 840
   gcccccggga cccccggaga ggctgctgag acccccgccc ggcctggcct ggccaagaag 900
   ttcctgccct ggagcctggc ccgggagccg ccacccgagg cgctgaagga ggcggtgctg 960
   cgctacagcc tggccgacga ggcctccatg gacagcgggg cacgctggca gcgcgggagg 1020
   ctggcgctgc gccgggcccc gggccccgat ggccccgacc gcgtgctgga gctcttcgac 1080
   ccacccaaga gttcaaggcc caagctacaa gcagcttgct ccagcatcca ggaggtccgg 1140
   tggtgcacac ggcttgagat gcctgacaac ctttacacct ttgtgctgaa ggtgaaggac 1200
   cggacagaca tcatctttga ggtgggagac gagcagcagc tgaattcatg gatggctgag 1260
   ctctcggagt gcacaggccg agggctggag agcacagaag cagagatgca tattccctca 1320
   gccctagagc ctagcacgtc cagctcccca aggggcagca cagattccct taaccaaggt 1380
   gcttctcctg gggggctgct ggacccggcc tgccagaaga cggaccattt cctgtcctgc 1440
   tacccctggt tccacggccc catctccaga gtgaaagcag ctcagctggt tcagctgcag 1500
   ggccctgatg ctcatggagt gttcctggtg cggcagagcg agacgcggcg tggggaatac 1560
   gtgctcactt tcaactttca ggggatagcc aagcacctgc gcctgtcgct gacagagcgg 1620
   ggccagtgcc gtgtgcagca cctccacttt ccctcggtcg tggacatgct ccaccacttc 1680
   cagcgctcgc ccatcccact cgagtgcggc gccgcctgtg atgtccggct ctccagctac 1740
   gtggtagtcg tctcccaacc accaggttcc tgcaacacgg tcctcttccc tttctccctt 1800
   cctcactggg attcagagtc ccttcctcac tggggttcag agttgggcct tccccacctt 1860
   agttcttctg gctgtccccg ggggctcagc ccagagggtc tcccagggcg atcctcaccc 1920
   cccgagcaga tcttccacct ggtgccttcg cccgaagaac tggccaacag cctgcagcac 1980
   ctggagcatg agcctgtgaa tcgagcccgg gactcggact acgaaatgga ctcatcctcc 2040
   cggagccacc tgcgggccat agacaatcag tacacacctc tctgaccagt gaggaattcc 2100
   aggcctcaac agctgccctt gaggagcaca ggcagaagtg tgaacttgtg aatgtaattg 2160
   atctttcctt ccttccagag aaagatttaa gggacactgt taactgctcg tgccagtttg 2220
   gaagtgaccc ttctattagg cctgttgaag ggccctcctg taggtttcat ctatccacct 2280
   ggctttctcc ttattgttta cagatgtagt tcttgttaga ggatgccgct agctcctgcc 2340
   cggggtccct atgcccagtc cccgttactc ttagagaaag gagttggggt gagggccaga 2400
   gctggcagtg gaaacttgtt ctctttttca ctgacactgt cacagcggat gacagacttt 2460
   ctacggggag gaggggggga tcatcaggaa gcccagaaca ctaacaagcg gttctcccat 2520
   ctaccgtcag tccacatggc aggtctgctg tgtccacacc acagatgacc acatctaatc 2580
   ctgcttctac tctcagcttt aggacaaaag ctctgtcaga ggcacaagct gaaggtcaaa 2640
   aatgatttaa aacattttac ctcagactaa tttctttaaa ggattcaggt tcaaaactta 2700
   accactgctt atttcagtgc actgtttcaa ctaacaccca tgctattttt gtagtcagaa 2760
   acagctatgc aaaccctacc taatttacag tctgagccag catgctggct tgtctactgc 2820
   atcctcggga cagtcacctg ccactgagtg gccactgtcc ttcctaaatg tcaagaagtg 2880
   aagtatgtca ccctttcagg gaaattcagg caattactga aataggaggg tggcaagaac 2940
   agttctatcc tggtgcctta cgaataaaaa actggattct ggtttacagc agctttacag 3000
   tgatagttaa attaactggg gctaggggaa gagcaagcaa aaagggaaga aggactccta 3060
   ggccctttct agtaaatcct tcagcaacaa ggctggcttg gtgccctcca agcatctaat 3120
   ggcttattaa attatcccac aagtgggttt taggctcctt ttttgagcca aaatggaagc 3180
   tgggaatctg gtgccataac taatgagaaa ctcctttaat agcccacaat cagtgttctg 3240
   ttctagctgg ctactgcttc actggattga gaatctatct atctccttgc acacatgggc 3300
   acacacaatc tccaccatcc agggaggtcc tgaagtcaaa tctctatcta tacaagtgat 3360
   acaattcata gggggctggc tcctcccaga acctgtctgg aggctcagaa acgggggcag 3420
   tgacagtgga gtcagctgct cttgggtgcc agcagagcca ttcagtacaa cccccaggct 3480
   cacagcagtg gcttctagga aactgggagt ttagatcagc tttacagata catcgatcag 3540
   aggctaaaat gaaacctcag cctaaaactc ataggactga ctgcctggga ggagggttag 3600
   gtctgcttct tccacttata cttagtctct gtgctccaag aggtcaaatt tttgcttcta 3660
   gaatttcctt ggggtctttc agagggtggg ggaacaaacc cctatgcact tttctttttt 3720
   ttttttttga gatggagttt ctcttgtcaa ccgggctgga gtgcagtggt gcaatcttgg 3780
   ctcactgcaa cctccacctt cctggttcaa gcgattctgc ctcgacctct caagtagctg 3840
   ggattacaag caccagccac catgcctggc taattttgta tttttagtag agacagggtt 3900
   tcaccatgtt ggccaggctg gtctcgaatg tctgacctca ggtgatccac ccgccttggc 3960
   ctcccaaagt gctgggatta caggcgcgag ccaccgcgcc cagcctacac cacttttagt 4020
   accaacactc ttgggtgatt tcatggaccc taaagcagac ctgacactga tccagatttg 4080
   cagtccattt ttaaggacac ctgtctttat ttcctcaaag tcaagcagct ttctctggaa 4140
   aatgaatgct aattagtgtg aaccaaaaga gtaagtaaga gtctgaagtt tttttaaagg 4200
   agaaagctta ttatggaaag tcactggtcc tcccctccgc acaggaaagg tacccagtag 4260
   ataatgaacc aaattaagtt ccctccctcc agccagaagt taaacatctg ggatatgacg 4320
   tcttcatgcc aggggcactc atttcttagc agcctctcta catacatctc tcaggtggtg 4380
   ccaagaggca caccaggtag agcaaactta gcagctctga ctaacaggct gcaaagtgca 4440
   agttcagatt ctgtggcaga gatttggaag gcacccacct ccagactgct tcccgtccaa 4500
   gttaccagga cagctcaaaa acatgctgac agaaaactcc catggctcta ggaagaagtg 4560
   acactaagcc aacacctttc tttatgtggg agcagaatca gctgatgaag gggtgggcag 4620
   cagtgtgggg caggcacccc actggctgca gctagcccac cataggcaca gcacatccca 4680
   ccactctcct tccagtcctg accaggcccc agccggcaac ttctaccgag agccatggct 4740
   caacaccaaa ctggacagta gacatcatga tccctccagt tagctctaat tacagacccc 4800
   accagtacag cttgacagct cccggcacca tcccttcctt catctgactt attgaacttt 4860
   tacaaactaa cagtcaccag caccaaagaa ttaagtcaac taacctgcct tgaattttag 4920
   accagcaatc catatggctt tatctggtat aaatcttctg cctttgatca tttctggacc 4980
   gtaggaaaaa ggaatagcaa tcattaaaat cttgggccag agaacactat ttttacataa 5040
   cagtttctta acctaaagtc aaggccttgg actcttccct gagggttgcc tgagattcct 5100
   tcatgctttc tattcaggac taagtccctt actgcaaatg tgttagctct aacatctccc 5160
   acaagctaga ggaacttgcg agtatattaa caaggacaca tctgacatcc tgtgtttggt 5220
   tagaatatac agcacattgt gataacataa agtggattca tcttgtatca ttataggcag 5280
   aaggtatttg gcaaattttt atgtattgtt ttatgtactg tacaagtaac ttattcttga 5340
   ataatgcaaa ttttgctata atgtacaaat tgctatatgt gaattaaaaa gttttcagaa 5400
   tcttgaaaaa aaaaaaaaaa aaaaa 5425
<210> 257
   <211> 2145
   <212> DNA
   <213> Homo sapiens
<220>
   <223> G protein-coupled receptor 3 (GPR3), ACCA orphan receptor, adenylate cyclase constitutive activator
<220>
   <221> CDS
   <222> (96) ... (1088)
   <223> GPR3
<400> 257
   cgcgggggtt tctcggggtc cacgcacgcc ctgcgccgcc aggacccgag cggagcctcc 60
   ccgcggcccg gccgcgcctg gtcctgagcg gtaccatgat gtggggtgca ggcagccctc 120
   tggcctggct ctcagctggc tcaggcaacg tgaatgtaag cagcgtgggc ccagcagagg 180
   ggcccacagg tccagccgca ccactgccct cgcctaaggc ctgggatgtg gtgctctgca 240
   tctcaggcac cctggtgtcc tgcgagaatg cgctagtggt ggccatcatc gtgggcactc 300
   ctgccttccg tgcccccatg ttcctgctgg tgggcagcct ggccgtggca gacctgctgg 360
   caggcctggg cctggtcctg cactttgctg ctgtcttctg catcggctca gcggagatga 420
   gcctggtgct ggttggcgtg ctggcaatgg cctttaccgc cagcatcggc agtctactgg 480
   ccatcactgt cgaccgctac ctttctctgt acaatgccct cacctactat tcagagacaa 540
   cagtgacacg gacctatgtg atgctggcct tagtgtgggg aggtgccctg ggcctggggc 600
   tgctgcctgt gctggcctgg aactgcctgg atggcctgac cacatgtggc gtggtttatc 660
   cactctccaa gaaccatctg gtagttctgg ccattgcctt cttcatggtg tttggcatca 720
   tgctgcagct ctacgcccaa atctgccgca tcgtctgccg ccatgcccag cagattgccc 780
   ttcagcggca cctgctgcct gcctcccact atgtggccac ccgcaagggc attgccacac 840
   tggccgtggt gcttggagcc tttgccgcct gctggttgcc cttcactgtc tactgcctgc 900
   tgggtgatgc ccactctcca cctctctaca cctatcttac cttgctccct gccacctaca 960
   actccatgat caaccctatc atctacgcct tccgcaacca ggatgtgcag aaagtgctgt 1020
   gggctgtctg ctgctgctgt tcctcttcca agatcccctt ccgatcccgc tcccccagtg 1080
   atgtctagct gagtcttcat gacccttcaa ccctgattac tacagaattc cagaatgtta 1140
   ggctctccag ggcttctttc caaaccccca gctccacacc ccccagaccc agctggttct 1200
   ggagttctag gacattgggt gtttcaaggt tctgttcaga tccctatggg ggcccagctg 1260
   gctccacggt tccagaatgt tcaggtggtc agtgttctac tcagaaatgt ctcacagccc 1320
   agctgggttg caattccaga atgctgggag ttttacagtg ccattccaag tcccagatgt 1380
   ccctcttccc ccaaacttga ccttgaccat gtcactttac gtttgaattt ctgagctaaa 1440
   gagtcagaga gattagtcac atagttgcct aaataggaga gagaaagatt atatatgcac 1500
   atatacaaag acagtgtcta tttatgattg atttatttat ttataaattt acttatgggt 1560
   ggtaaggggc aaaaaagagg cccacacctt gatatccagg ccataccagg gtatcccttg 1620
   tcccttcacc cccatttctg acctcagttc ctggaggggg gaaagggtga aagagaaacc 1680
   acgtattttg ttattatttt ggattatttt ttatcgaaga gatcatagaa accagagcct 1740
   tctccccagg cctgccctcc tcgggtttgg aaggggaaca caccagcctc tggtttttta 1800
   tttttttaag aagccatcac ctgagcaacc aaaaattcct ctgcgctggg gtccgactgc 1860
   cctctggtgg ccatttgggg aaaactgcag cccggccagg cagctgggac cagaatgcaa 1920
   ccccagctcc actccagcct ggcgtccagg gccacagcca tggcctgggg gccaagcctc 1980
   accctgcggt gccctaaagg agggggggca cgagccaaca ccccacccct ctgccaaccg 2040
   gggtatggcc cccagtgcat tccctgttcc cgtctccaac ccaactcaat aaaaaatgat 2100
   tttgtcataa aaaaaaaaaa aaaaaaaaaa aaaaaaaaaa aaaaa 2145
<210> 258
   <211> 8605
   <212> DNA
   <213> Homo sapiens
<220>
   <223> tenascin C (TNC, TN-C, TN), hexabrachion (HXB), cytotactin, myotendinous antigen, glioma-associated-extracellular matrix antigen (GMEM), deafness, autosomal dominant 56 (DFNA56), neuronectin, GP 150-225
<220>
   <221> CDS
   <222> (413) ... (7018)
   <223> TNC
<400> 258
   aattcgccaa ctgaaaaagt gggaaaggat gtctggaggc gaggcgtccc attacagagg 60
   aaggagctcg ctatataagc cagccaaagt tggctgcacc ggccacagcc tgcctactgt 120
   cacccgcctc tcccgcgcgc agatacacgc ccccgcctcc gtgggcacaa aggcagcgct 180
   gctggggaac tcgggggaac gcgcacgtgg gaaccgccgc agctccacac tccaggtact 240
   tcttccaagg acctaggtct ctcgcccatc ggaaagaaaa taattctttc aagaagatca 300
   gggacaactg atttgaagtc tactctgtgc ttctaaatcc ccaattctgc tgaaagtgag 360
   ataccctaga gccctagagc cccagcagca cccagccaaa cccacctcca ccatgggggc 420
   catgactcag ctgttggcag gtgtctttct tgctttcctt gccctcgcta ccgaaggtgg 480
   ggtcctcaag aaagtcatcc ggcacaagcg acagagtggg gtgaacgcca ccctgccaga 540
   agagaaccag ccagtggtgt ttaaccacgt ttacaacatc aagctgccag tgggatccca 600
   gtgttcggtg gatctggagt cagccagtgg ggagaaagac ctggcaccgc cttcagagcc 660
   cagcgaaagc tttcaggagc acacagtgga tggggaaaac cagattgtct tcacacatcg 720
   catcaacatc cccgccgggg cctgtggctg tgccgcagcc cctgatgtta aggagctgct 780
   gagcagactg gaggagctgg agaacctggt gtcttccctg agggagcaat gtactgcagg 840
   agcaggctgc tgtctccagc ctgccacagg ccgcttggac accaggccct tctgtagcgg 900
   tcggggcaac ttcagcactg aaggatgtgg ctgtgtctgc gaacctggct ggaaaggccc 960
   caactgctct gagcccgaat gtccaggcaa ctgtcacctt cgaggccggt gcattgatgg 1020
   gcagtgcatc tgtgacgacg gcttcacggg cgaggactgc agccagctgg cttgccccag 1080
   cgactgcaat gaccagggca agtgcgtaaa tggagtctgc atctgtttcg aaggctacgc 1140
   cggggctgac tgcagccgtg aaatctgccc agtgccctgc agtgaggagc acggcacatg 1200
   tgtagatggc ttgtgtgtgt gccacgatgg ctttgcaggc gatgactgca acaagcctct 1260
   gtgtctcaac aattgctaca accgtggacg atgcgtggag aatgagtgcg tgtgtgatga 1320
   gggtttcacg ggcgaagact gcagtgagct catctgcccc aatgactgct tcgaccgggg 1380
   ccgctgcatc aatggcacct gctactgcga agaaggcttc acaggtgaag actgcgggaa 1440
   acccacctgc ccacatgcct gccacaccca gggccggtgt gaggaggggc agtgtgtatg 1500
   tgatgagggc tttgccggtg tggactgcag cgagaagagg tgtcctgctg actgtcacaa 1560
   tcgtggccgc tgtgtagacg ggcggtgtga gtgtgatgat ggtttcactg gagctgactg 1620
   tggggagctc aagtgtccca atggctgcag tggccatggc cgctgtgtca atgggcagtg 1680
   tgtgtgtgat gagggctata ctggggagga ctgcagccag ctacggtgcc ccaatgactg 1740
   tcacagtcgg ggccgctgtg tcgagggcaa atgtgtatgt gagcaaggct tcaagggcta 1800
   tgactgcagt gacatgagct gccctaatga ctgtcaccag cacggccgct gtgtgaatgg 1860
   catgtgtgtt tgtgatgacg gctacacagg ggaagactgc cgggatcgcc aatgccccag 1920
   ggactgcagc aacaggggcc tctgtgtgga cggacagtgc gtctgtgagg acggcttcac 1980
   cggccctgac tgtgcagaac tctcctgtcc aaatgactgc catggccagg gtcgctgtgt 2040
   gaatgggcag tgcgtgtgcc atgaaggatt tatgggcaaa gactgcaagg agcaaagatg 2100
   tcccagtgac tgtcatggcc agggccgctg cgtggacggc cagtgcatct gccacgaggg 2160
   cttcacaggc ctggactgtg gccagcactc ctgccccagt gactgcaaca acttaggaca 2220
   atgcgtctcg ggccgctgca tctgcaacga gggctacagc ggagaagact gctcagaggt 2280
   gtctcctccc aaagacctcg ttgtgacaga agtgacggaa gagacggtca acctggcctg 2340
   ggacaatgag atgcgggtca cagagtacct tgtcgtgtac acgcccaccc acgagggtgg 2400
   tctggaaatg cagttccgtg tgcctgggga ccagacgtcc accatcatcc aggagctgga 2460
   gcctggtgtg gagtacttta tccgtgtatt tgccatcctg gagaacaaga agagcattcc 2520
   tgtcagcgcc agggtggcca cgtacttacc tgcacctgaa ggcctgaaat tcaagtccat 2580
   caaggagaca tctgtggaag tggagtggga tcctctagac attgcttttg aaacctggga 2640
   gatcatcttc cggaatatga ataaagaaga tgagggagag atcaccaaaa gcctgaggag 2700
   gccagagacc tcttaccggc aaactggtct agctcctggg caagagtatg agatatctct 2760
   gcacatagtg aaaaacaata cccggggccc tggcctgaag agggtgacca ccacacgctt 2820
   ggatgccccc agccagatcg aggtgaaaga tgtcacagac accactgcct tgatcacctg 2880
   gttcaagccc ctggctgaga tcgatggcat tgagctgacc tacggcatca aagacgtgcc 2940
   aggagaccgt accaccatcg atctcacaga ggacgagaac cagtactcca tcgggaacct 3000
   gaagcctgac actgagtacg aggtgtccct catctcccgc agaggtgaca tgtcaagcaa 3060
   cccagccaaa gagaccttca caacaggcct cgatgctccc aggaatcttc gacgtgtttc 3120
   ccagacagat aacagcatca ccctggaatg gaggaatggc aaggcagcta ttgacagtta 3180
   cagaattaag tatgccccca tctctggagg ggaccacgct gaggttgatg ttccaaagag 3240
   ccaacaagcc acaaccaaaa ccacactcac aggtctgagg ccgggaactg aatatgggat 3300
   tggagtttct gctgtgaagg aagacaagga gagcaatcca gcgaccatca acgcagccac 3360
   agagttggac acgcccaagg accttcaggt ttctgaaact gcagagacca gcctgaccct 3420
   gctctggaag acaccgttgg ccaaatttga ccgctaccgc ctcaattaca gtctccccac 3480
   aggccagtgg gtgggagtgc agcttccaag aaacaccact tcctatgtcc tgagaggcct 3540
   ggaaccagga caggagtaca atgtcctcct gacagccgag aaaggcagac acaagagcaa 3600
   gcccgcacgt gtgaaggcat ccactgaaca agcccctgag ctggaaaacc tcaccgtgac 3660
   tgaggttggc tgggatggcc tcagactcaa ctggaccgca gctgaccagg cctatgagca 3720
   ctttatcatt caggtgcagg aggccaacaa ggtggaggca gctcggaacc tcaccgtgcc 3780
   tggcagcctt cgggctgtgg acataccggg cctcaaggct gctacgcctt atacagtctc 3840
   catctatggg gtgatccagg gctatagaac accagtgctc tctgctgagg cctccacagg 3900
   ggaaactccc aatttgggag aggtcgtggt ggccgaggtg ggctgggatg ccctcaaact 3960
   caactggact gctccagaag gggcctatga gtactttttc attcaggtgc aggaggctga 4020
   cacagtagag gcagcccaga acctcaccgt cccaggagga ctgaggtcca cagacctgcc 4080
   tgggctcaaa gcagccactc attataccat caccatccgc ggggtcactc aggacttcag 4140
   cacaacccct ctctctgttg aagtcttgac agaggaggtt ccagatatgg gaaacctcac 4200
   agtgaccgag gttagctggg atgctctcag actgaactgg accacgccag atggaaccta 4260
   tgaccagttt actattcagg tccaggaggc tgaccaggtg gaagaggctc acaatctcac 4320
   ggttcctggc agcctgcgtt ccatggaaat cccaggcctc agggctggca ctccttacac 4380
   agtcaccctg cacggcgagg tcaggggcca cagcactcga ccccttgctg tagaggtcgt 4440
   cacagaggat ctcccacagc tgggagattt agccgtgtct gaggttggct gggatggcct 4500
   cagactcaac tggaccgcag ctgacaatgc ctatgagcac tttgtcattc aggtgcagga 4560
   ggtcaacaaa gtggaggcag cccagaacct cacgttgcct ggcagcctca gggctgtgga 4620
   catcccgggc ctcgaggctg ccacgcctta tagagtctcc atctatgggg tgatccgggg 4680
   ctatagaaca ccagtactct ctgctgaggc ctccacagcc aaagaacctg aaattggaaa 4740
   cttaaatgtt tctgacataa ctcccgagag cttcaatctc tcctggatgg ctaccgatgg 4800
   gatcttcgag acctttacca ttgaaattat tgattccaat aggttgctgg agactgtgga 4860
   atataatatc tctggtgctg aacgaactgc ccatatctca gggctacccc ctagtactga 4920
   ttttattgtc tacctctctg gacttgctcc cagcatccgg accaaaacca tcagtgccac 4980
   agccacgaca gaggccctgc cccttctgga aaacctaacc atttccgaca ttaatcccta 5040
   cgggttcaca gtttcctgga tggcatcgga gaatgccttt gacagctttc tagtaacggt 5100
   ggtggattct gggaagctgc tggaccccca ggaattcaca ctttcaggaa cccagaggaa 5160
   gctggagctt agaggcctca taactggcat tggctatgag gttatggtct ctggcttcac 5220
   ccaagggcat caaaccaagc ccttgagggc tgagattgtt acagaagccg aaccggaagt 5280
   tgacaacctt ctggtttcag atgccacccc agacggtttc cgtctgtcct ggacagctga 5340
   tgaaggggtc ttcgacaatt ttgttctcaa aatcagagat accaaaaagc agtctgagcc 5400
   actggaaata accctacttg cccccgaacg taccagggac ataacaggtc tcagagaggc 5460
   tactgaatac gaaattgaac tctatggaat aagcaaagga aggcgatccc agacagtcag 5520
   tgctatagca acaacagcca tgggctcccc aaaggaagtc attttctcag acatcactga 5580
   aaattcggct actgtcagct ggagggcacc cacagcccaa gtggagagct tccggattac 5640
   ctatgtgccc attacaggag gtacaccctc catggtaact gtggacggaa ccaagactca 5700
   gaccaggctg gtgaaactca tacctggcgt ggagtacctt gtcagcatca tcgccatgaa 5760
   gggctttgag gaaagtgaac ctgtctcagg gtcattcacc acagctctgg atggcccatc 5820
   tggcctggtg acagccaaca tcactgactc agaagccttg gccaggtggc agccagccat 5880
   tgccactgtg gacagttatg tcatctccta cacaggcgag aaagtgccag aaattacacg 5940
   cacggtgtcc gggaacacag tggagtatgc tctgaccgac ctcgagcctg ccacggaata 6000
   cacactgaga atctttgcag agaaagggcc ccagaagagc tcaaccatca ctgccaagtt 6060
   cacaacagac ctcgattctc caagagactt gactgctact gaggttcagt cggaaactgc 6120
   cctccttacc tggcgacccc cccgggcatc agtcaccggt tacctgctgg tctatgaatc 6180
   agtggatggc acagtcaagg aagtcattgt gggtccagat accacctcct acagcctggc 6240
   agacctgagc ccatccaccc actacacagc caagatccag gcactcaatg ggcccctgag 6300
   gagcaatatg atccagacca tcttcaccac aattggactc ctgtacccct tccccaagga 6360
   ctgctcccaa gcaatgctga atggagacac gacctctggc ctctacacca tttatctgaa 6420
   tggtgataag gctgaggcgc tggaagtctt ctgtgacatg acctctgatg ggggtggatg 6480
   gattgtgttc ctgagacgca aaaacggacg cgagaacttc taccaaaact ggaaggcata 6540
   tgctgctgga tttggggacc gcagagaaga attctggctt gggctggaca acctgaacaa 6600
   aatcacagcc caggggcagt acgagctccg ggtggacctg cgggaccatg gggagacagc 6660
   ctttgctgtc tatgacaagt tcagcgtggg agatgccaag actcgctaca agctgaaggt 6720
   ggaggggtac agtgggacag caggtgactc catggcctac cacaatggca gatccttctc 6780
   cacctttgac aaggacacag attcagccat caccaactgt gctctgtcct acaaaggggc 6840
   tttctggtac aggaactgtc accgtgtcaa cctgatgggg agatatgggg acaataacca 6900
   cagtcagggc gttaactggt tccactggaa gggccacgaa cactcaatcc agtttgctga 6960
   gatgaagctg agaccaagca acttcagaaa tcttgaaggc aggcgcaaac gggcataaat 7020
   tccagggacc actgggtgag agaggaataa ggcccagagc gaggaaagga ttttaccaaa 7080
   gcatcaatac aaccagccca accatcggtc cacacctggg catttggtga gagtcaaagc 7140
   tgaccatgga tccctggggc caacggcaac agcatgggcc tcacctcctc tgtgatttct 7200
   ttctttgcac caaagacatc agtctccaac atgtttctgt tttgttgttt gattcagcaa 7260
   aaatctccca gtgacaacat cgcaatagtt ttttacttct cttaggtggc tctgggaatg 7320
   ggagaggggt aggatgtaca ggggtagttt gttttagaac cagccgtatt ttacatgaag 7380
   ctgtataatt aattgtcatt atttttgtta gcaaagatta aatgtgtcat tggaagccat 7440
   cccttttttt acatttcata caacagaaac cagaaaagca atactgtttc cattttaagg 7500
   atatgattaa tattattaat ataataatga tgatgatgat gatgaaaact aaggattttt 7560
   caagagatct ttctttccaa aacatttctg gacagtacct gattgtattt tttttttaaa 7620
   taaaagcaca agtacttttg agtttgttat tttgctttga attgttgagt ctgaatttca 7680
   ccaaagccaa tcatttgaac aaagcgggga atgttgggat aggaaaggta agtagggata 7740
   gtggtcaagt gggaggggtg gaaaggagac taaagactgg gagagaggga agcacttttt 7800
   ttaaataaag ttgaacacac ttgggaaaag cttacaggcc aggcctgtaa tcccaacact 7860
   ttgggaggcc aaggtgggag gatagcttaa ccccaggagt ttgagaccag cctgagcaac 7920
   atagtgagaa cttgtctcta cagaaaaaaa aaaaaaaaaa aatttaatta ggcaagcgtg 7980
   gtagtgcgca cctgtcgtcc cagctactca ggaggctgag gtaggaaaat cactggagcc 8040
   caggagttag aggttacagt gagctatgat cacactactg cactccagcc tgggcaacag 8100
   agggagaccc tgtctctaaa taaaaaaaga aaagaaaaaa aaagcttaca acttgagatt 8160
   cagcatcttg ctcagtattt ccaagactaa tagattatgg tttaaaagat gcttttatac 8220
   tcattttcta atgcaactcc tagaaactct atgatatagt tgaggtaagt attgttacca 8280
   cacatgggct aagatcccca gaggcagact gcctgagttc aattcttggc tccaccattc 8340
   ccaagttccc taacctctct atgcctcagt ttcctcttct gtaaagtagg gacactcata 8400
   cttctcattt cagaacattt ttgtgaagaa taaattatgt tatccatttg aggcccttag 8460
   aatggtaccc ggtgtatatt aagtgctagt acatgttagc tatcatcatt atcactttat 8520
   atgagatgga ctggggttca tagaaaccca atgacttgat tgtggctact actcaataaa 8580
   taatagaatt tggatttaaa aaaaa                                       8605
<210> 259
   <211> 3285
   <212> DNA
   <213> Homo sapiens
<220>
   <223> polymerase (RNA) III (DNA directed) polypeptide G (32kD), DNA-directed RNA polymerase III subunit G (POLR3G), RNA polymerase III subunit C7 (RPC7), RNA polymerase III 32 kDa subunit (RPC32)
<220>
   <221> CDS
   <222> (201) ... (872)
   <223> POLR3G
<400> 259
   actccgcggt cctggtgccg cgtgcaggtc ggtgcgcgct tctcccgagg tggaacgggc 60
   ggcagtcaag cgccggcgtt ctctgccgtc accctttcct tgccggccgg cacttcggct 120
   gcagagtttt gcccacgctt cgagacttag ggagcagtgc ctttcagaat ttgcccactc 180
   atctggtata actggttctg atggctggga ataaaggaag aggacgtgct gcttatacct 240
   ttaatattga ggctgttgga tttagcaaag gtgaaaagtt acctgatgta gtgttgaaac 300
   cacccccact atttcctgat acagattata aaccagtgcc actgaaaaca ggagaaggtg 360
   aagaatatat gctggctttg aaacaggagt tgagagaaac aatgaaaaga atgccttatt 420
   ttattgaaac acctgaagaa agacaagata ttgaaaggta tagtaaaaga tacatgaagg 480
   tatacaagga agaatggata ccagattgga gaagacttcc aagagagatg atgccaagaa 540
   ataaatgtaa aaaagcaggc ccaaaaccca aaaaggcaaa agacgcaggc aaaggcacac 600
   cactcactaa tactgaagat gtgttgaaaa aaatggagga attggaaaaa agaggtgatg 660
   gtgaaaaatc agatgaggaa aatgaagaga aagaaggaag caaagagaaa agtaaagaag 720
   gtgatgatga cgatgacgat gatgccgcag aacaggagga atatgatgaa gaagagcaag 780
   aagaggaaaa tgactacatt aattcatact ttgaagatgg agatgatttt ggcgcagaca 840
   gtgatgacaa catggatgag gcaacctatt aggcatgaaa tttttcaaaa aatattttta 900
   tgatgcagct tctgaacatt tggacagact tgatttgtat tttatttctg ataaggaata 960
   agtacttgtt tctgttgttt tggacaaata gttgttacca aaatattcaa aaccactttg 1020
   agtttacata ctagttacct taaaaattat tccctgacac tctgacattc cttctaaaca 1080
   cctctgccat ctctcttatg tactctcatg ggtttttttg tatgatttga atatgaatgt 1140
   gcctaaagaa tttttgctct cttaatctat gtatacatac ttgaacaaat cattcttgct 1200
   taactgctga tcttttgtaa aactattgct agtcataatg gatttctata acctgaaagt 1260
   tgatataata cagcacgtgg agcactttaa aaacaaagaa acttgaaaat aattttattt 1320
   tttaccttta catggtgtgt tctagactac tgcattatga ctacataaac ctacctgagc 1380
   tcttacaagt acagaattgt gagacagagg ttttgtgatg gaaaacatac acatagggaa 1440
   aaataccaaa ctgagcaata taaatttcac agctcacatc ttagccagta caaagaaact 1500
   ttcacatggg gtgaatatta taatacttga atttgagact taatactgta tatgaattaa 1560
   tcatcccact gtaataactg acatcttcaa atagttgggc ttatgaaaaa tttgactaag 1620
   acattttatt ttatacttta tgtaaatgtg aaataagttc tggtcatata aatatgtaga 1680
   atgcatgtgt gtgaactcta tgaagtgaag tgtggtggcc ttttatgttt cagagtactc 1740
   aagtgtacaa ttgatttata taaaaatgca aaatgaactc aggtcatttt gaaattaata 1800
   ttaatatttt tgttgtttaa ctttaaagct agctccccta actttatata ttttttggga 1860
   aaaaatacct aaaaacctca gcctacaaag actctcagaa atgccaaaga ttttcaagga 1920
   aattcatatt atatatttca aaaatgattt atcaatgtta tctaccaaaa gaaataattt 1980
   tatttttccc ctttggggag atattatcct ctaatatgag aatcagatcc ctagattcta 2040
   tttctaccta tactattaaa ctcaaccttg aacctgagct tggttgtttc tgtgccttag 2100
   tttctccact cgtaaacaat ttcctataaa aattgtttaa acaccaacca aaatttacca 2160
   tatgtattca ttctcttgcc ttaaagatca cttcttaact tacatttggc tctcaccctc 2220
   taaaaatttg tagtttggag agtaactgaa agcacaattt taagaaacat gttttcctca 2280
   caaaatatat tgtaatctga ttttcctatc ttgtattcat gcagaaaatt ggagaaaatg 2340
   tgtattgtct tgtgccattc tgcaatatga atttctcagg aaaaagatgt ttcaatggca 2400
   atactttctt aaataattca agatgggtgt gaggacctat cttattagtt tagtgctgtg 2460
   ttaaccattt gtgaattact tcttgtttaa aggaagttgt caaaaaattt taagattcta 2520
   aatggaatac acgaataaag taagccttag aatagaaacc tcacaaaaag ttgtcatttg 2580
   cggttgataa ttaacatagg tgttttattc tctcaactcc ttagtaccag agccaaatac 2640
   aatttagttt tcagaaatct tatcaatatt gttctattta cctacttata aatctgcaga 2700
   taaccacttg aaccaggcaa atacactgaa gataaaggtt atttcttttt ttagctttga 2760
   atttgtcatg accattttag tcttgcagat agcagggcag ccccttgggg caaggattta 2820
   ctctgggagg taccgttaag agccttcttt ccccctttga aagatccttt tacaatgtta 2880
   aagtatacta gttgcaagaa caagcaggat ttgcaggttg ctttaccagc atgagtctca 2940
   tttttctggc ttaaaatctg ggactgtgaa attattccat aggaaagtga atgttatttt 3000
   gcagaattag cctcttacat aaaagtattt gttgaagtgt ctttaaaatt gctatcatga 3060
   gcaaaactgg ttgctgtaat gcttgttttt ctgtatttat ttacacatta aattcttaca 3120
   aaacaaaatg tgttcgtttg ttttatagta agatgtttta ttttgaactt atttaaatgt 3180
   ttatttgtta gagaaaacta atttgttaat atagatctta actagtaact attaagcagg 3240
   gatgtttgtt ccaaatattc ttgaataaac aatcaccaca aacaa                 3285
<210> 260
   <211> 3877
   <212> DNA
   <213> Homo sapiens
<220>
   <223> WD repeat domain 3 (WDR3), DIP2, UTP12, dJ776P7.2
<220>
   <221> CDS
   <222> (67) ... (2898)
   <223> WDR3
<400> 260
   ggtccaatcg gctgggagcc tcgtggaggc tgagattgct tcacctgtgg tatcagacat 60
   cacaacatgg ggctcaccaa gcagtaccta cgctatgttg ctagtgcggt ctttggcgtt 120
   atcggcagcc aaaaaggtaa tattgtcttt gtgacacttc gtggtgagaa aggacgttat 180
   gtggcagtac cagcttgtga acacgttttc atctgggact taaggaaagg agagaagatt 240
   cttatccttc aggggcttaa acaagaagtt acttgcttat gcccctcccc agatgggcta 300
   cacttagctg ttgggtatga ggatgggtcg atccgaatct tcagtctcct gagtggggaa 360
   ggaaatgtga ccttcaatgg tcacaaagca gctatcacta ccttgaagta tgatcagcta 420
   ggaggcagac tggcatctgg gtccaaggac acagatatta ttgtatggga tgtgatcaat 480
   gaaagtggtc tgtaccgtct aaaggggcac aaggatgcca tcacacaagc attgtttcta 540
   cgagaaaaga acctgctagt tactagtggg aaagatacca tggtgaaatg gtgggacctt 600
   gatactcagc actgctttaa aacaatggtt ggccaccgga ctgaggtatg ggggttggtt 660
   ctgttgtcag aagaaaagcg actcatcact ggggcctcag acagtgaact gagggtatgg 720
   gacatagctt atctgcaaga gattgaagac ccggaagaac cagaccccaa gaaaatcaaa 780
   ggatcttctc ctggaataca agatactctt gaggcagagg atggtgcctt tgagacggat 840
   gaagcccctg aggatcgaat cctttcatgc agaaaagctg gttccataat gcgggaagga 900
   agagacagag ttgtaaacct tgcagtcgac aagacaggca ggattcttgc ttgccatgga 960
   actgactctg tgctagaatt gttttgtatc ctttccaaaa aggaaattca gaagaaaatg 1020
   gataagaaga tgaagaaagc tagaaagaaa gcaaaattac attctagcaa aggagaggag 1080
   gaagatcctg aggttaatgt tgaaatgagt ctgcaagatg aaatccagcg ggtgactaat 1140
   ataaaaactt ctgccaaaat caagtccttt gacttgattc attcacctca cggagagtta 1200
   aaggctgtct tcctgctgca gaacaacctg gtggaattgt attcactgaa tccatccttg 1260
   cctactcctc agcctgtcag gacaagcaga atcactattg ggggtcatcg cagtgatgtg 1320
   cggactttgt cattcagctc agacaatatt gctgttcttt cagctgcagc tgattccatt 1380
   aaaatatgga acaggtctac actgcagtgt attcgcacaa tgacctgtga atatgcactt 1440
   tgctcattct ttgtacctgg tgatagacag gtagtcatag gaacaaagac agggaagctg 1500
   cagctttatg acttggcttc agggaatctg ctggagacaa tagatgcaca tgatggagct 1560
   ttgtggtcca tgtccctctc tccagatcag cgtggctttg tgacaggtgg tgcagataaa 1620
   tctgtcaaat tctgggattt tgagttagtg aaagatgaaa atagtaccca aaagagactt 1680
   tctgtgaagc aaacccgaac tttgcaacta gatgaagatg ttctgtgtgt cagttactct 1740
   cccaatcaaa agctattggc tgtgtctttg ctggactgta ctgtgaaaat tttctacgtt 1800
   gatactttaa agtttttctt gtcactgtat ggacacaaac tgcctgttat atgcatggac 1860
   atctctcatg atggagcact catagcaact ggctccgctg ataggaatgt gaaaatctgg 1920
   ggtttggact ttggggactg ccacaagtct ctctttgcac atgatgacag tgtgatgtac 1980
   ctacagtttg tacccaagtc tcacctcttc ttcactgccg gaaaagatca taagattaaa 2040
   cagtgggatg cagacaaatt tgaacacata cagactctgg agggtcatca ccaggaaata 2100
   tggtgtttgg ctgtaagccc cagtggagac tatgttgtat catcgtccca tgacaaatct 2160
   ctgagacttt gggagagaac aagggagcct cttattcttg aggaagaaag ggagatggaa 2220
   agagaagcag aatatgagga gagtgtggcc aaagaagacc aaccagcagt tccaggagag 2280
   actcaaggtg acagttactt tactggaaag aaaactattg aaacagtgaa agcagctgag 2340
   aggattatgg aggctattga gttgtaccga gaagaaactg caaaaatgaa ggaacacaaa 2400
   gccatttgta aagctgcagg gaaagaggtt ccacttccca gcaaccccat cctaatggct 2460
   tatggcagta tctcaccttc agcttatgta ttagagattt ttaaagggat caagtcgagt 2520
   gagctggaag aatctctact tgtgctgcct ttctcttatg tcccagacat tcttaaactc 2580
   tttaacgaat tcattcagct gggctctgat gttgaactta tatgccggtg cctcttcttc 2640
   ctccttagga ttcactttgg acagatcact agcaatcaaa tgcttgtgcc agtgatagaa 2700
   aaattaaggg aaacaactat ttcaaaagtc agccaagtcc gggatgttat cggcttcaat 2760
   atggctggtc ttgattatct caagagggaa tgcgaggcaa aaagtgaagt tatgtttttt 2820
   gctgatgcta ctagccactt ggaagagaag aagaggaaga ggaaaaagag ggagaagttg 2880
   attctaacgt tgacttagaa ctgaaatgtg gtatcttttt ttttttcaac tttttccttt 2940
   aaaggactcc taaactaagc acagaagagt tggcgtcatc ttaaaaatac caaataacag 3000
   aagatcgcat tgcagatgat atcaggatgt ggtttccagc tttgcctgag ggaattccaa 3060
   catgagatta tgggctggct ccatttcttg gacttaaaat gcattattag tttaaaaatc 3120
   tttctgtgct ctcaaagctt gagccttgca gctcaagctt gttgttccct ttatattcta 3180
   gcagggaata aataattgtt ttaattaggt atttgtttca ttggagttga aattaacatt 3240
   tcaaaagttt ttcgtatttt tttatggcag atgatttgtc atttatttat attaggtttt 3300
   actgcctatt gagacaacca ggtgcataat tgattgccct ttggccataa aaatgcagtg 3360
   tcatggatct tagagctaaa aaggactgta aaaattaccc agaacagcgt cctcagactt 3420
   aaccttctgc aagttatgtc tgtatataag aagattctaa ttgctaactg tttatacttt 3480
   tctgaataaa atagttgttt ctaattaaaa agtagccaag ctaagatgcc tggctgggct 3540
   tctgaggaat taatacactc gtgtgtgtgt gtgtgtgtgt gtgtgtgtgt gtgtgtgtat 3600
   gtgtatgtgt atgtacacat acatatatat agttgacact tgaaaaatgc aggtggtagg 3660
   ggcactgacc cacccctacg ccccgcacag tcaaaaatct gcatataact tttgattccc 3720
   caaagtctta agtactagta gcctgttatt gactgggagc ctgactgatg tattatatat 3780
   attgtatact gtattcttaa agtaagctag agaaaagaaa atgttactta gaaaatcata 3840
   aggaagagaa aatatattta ctgtgtttat caatact                          3877
<210> 261
   <211> 1303
   <212> DNA
   <213> Homo sapiens
<220>
   <223> brix domain containing 2 (BRIX1, BXDC2), ribosome biogenesis protein BRX1 homolog, BRX1, biogenesis of ribosomes, homolog (S. cerevisiae)
<220>
   <221> CDS
   <222> (25) ... (1086)
   <223> BRIX1
<400> 261
   caagagcgcg ggcggcgagg caagatggcg gcaaccaaga ggaaacggcg tggaggcttt 60
   gcagttcagg cgaagaagcc aaaaagaaac gaaatagatg cggagccgcc agctaagcgg 120
   cacgccacag cagaggaggt ggaggaagaa gagagggacc ggatcccagg ccccgtttgc 180
   aagggaaagt ggaaaaataa ggaacggatt ctcatctttt cttccagagg aataaatttt 240
   agaacaagac atttaatgca ggacttgaga atgttgatgc ctcattctaa agcagatact 300
   aaaatggatc gtaaggataa gctatttgtg attaacgagg tttgtgaaat gaagaactgt 360
   aataaatgca tctattttga agctaagaaa aaacaggatc tctatatgtg gctttcaaat 420
   tcacctcacg gaccatctgc taaattcctt gttcaaaata ttcataccct cgctgaactg 480
   aagatgactg gaaactgttt gaaaggttct cggccccttt tgtcttttga ccctgctttt 540
   gatgaattac cacattatgc tttgttaaaa gaactcttaa ttcagatctt tagtacacca 600
   cggtatcatc ccaaaagcca accatttgtg gaccacgtgt ttactttcac cattttggat 660
   aataggatat ggtttcggaa ctttcagatc atagaagaag atgctgctct tgtagaaata 720
   ggacctcgtt ttgtcttaaa tctcataaag attttccagg gaagttttgg aggaccaact 780
   ttatatgaaa atcctcacta ccagtcacca aacatgcatc ggcgtgtcat aagatccatc 840
   acagctgcaa aatacagaga gaaacagcaa gtgaaagatg tgcaaaaact gagaaagaaa 900
   gagccgaaga ctcttcttcc acatgatccc actgcagatg tttttgtaac accagctgag 960
   gagaaaccaa tagaaataca gtgggtaaaa ccagagccaa aagttgattt gaaagcaaga 1020
   aagaaacgga tttacaaaag gcaaagaaaa atgaaacaga ggatggacag tgggaaaaca 1080
   aaataagtca atggaaacct gatttgtttt tcagttactt tatatttatt ttgtattcaa 1140
   tgtgtaaata cttttattat ctaatactat cttacgtcta attagtgtag catttacaag 1200
   aaagaaaaat taagatctta aaatcagtga ttatcttttt ctaaataaaa tatcaccaga 1260
   attcatcagt taaaaaaaaa aaaaaaaaaa aaaaaaaaaa aaa                   1303
<210> 262
   <211> 3286
   <212> DNA
   <213> Homo sapiens
<220>
   <223> CD3e antigen, epsilon polypeptide associated protein (CD3EAP), antisense to ERCC-1 protein (ASE-1, ASE1), DNA-directed RNA polymerase I subunit RPA34m, polymerase I-associated factor PAF49, CAST
<220>
   <221> CDS
   <222> (489) ... (2021)
   <223> CD3EAP
<400> 262
   aagttctgaa cttgtgaggc atctgggcct ccccagaaga catttaacac agaaagcaca 60
   gccctactaa ctagtattct tacctgtctc ttcaagaatt tcagaccaat cgaccgtcct 120
   gtctctttaa ggcttaggaa gagcagtgtg gctgcccctt taaggaggcg ttgcaacaaa 180
   ccatattgga cagacgatgg gggcgaccca tcgggacccg acgggcctct gactccagca 240
   atacagcgaa tcagcggctt tcgggaatac atttttcgga aaaagacttc ttcctcggtt 300
   ttctgctctg cacacgttga aattttcccc agtttttcct gcagatcggg agtcgagcaa 360
   tgcctacccc cgcgctcccg caccagttgg gcgctcccgg atgatgccct acccctttgg 420
   atccacgtgg tctgcaacct ggtgcgagca gcccgggcta cagggttgcc tgaggtgtgg 480
   gtcccaggat ggaggagccc caggccggcg atgctgctcg gttctcttgt ccccccaact 540
   ttaccgcgaa gcccccagcc tcagagtccc ctcgtttctc cttggaggcg ctgacgggtc 600
   cagatacgga gctgtggctt attcaggccc ctgcagactt tgccccagaa tgcttcaatg 660
   ggcggcatgt gcctctctct ggctcccaga tcgtcaaggg caaattggca ggcaagcggc 720
   accgctatcg agtcctcagc agctgtcccc aagctggaga agcgaccctg ctggccccct 780
   caacggaggc aggaggtgga ctcacctgtg cctcagcccc ccagggcacc ctaaggatcc 840
   ttgagggtcc ccagcaatcc ctgtcaggga gccctctgca gcccatccca gcaagtcccc 900
   caccacagat ccctcctggc ctgaggcctc ggttctgtgc ctttgggggc aacccaccag 960
   tcacagggcc taggtcagcc ttggccccca acctgctcac ctcagggaag aagaaaaagg 1020
   agatgcaggt gacagaggcc ccagtcactc aggaggcagt gaatgggcac ggggccctgg 1080
   aggtggacat ggctttgggg tcgccagaaa tggatgtgcg gaagaagaag aagaaaaaaa 1140
   atcagcagct gaaagaacca gaggcagcag ggcctgtggg gacagagccc acagtggaga 1200
   cactggagcc tctgggagtg ctgttcccgt ccaccaccaa gaagaggaag aagcccaaag 1260
   ggaaagaaac cttcgagcca gaagacaaga cagtgaagca ggaacagatt aacactgagc 1320
   ctctagaaga cacagtcctg tccccgacca aaaagagaaa gaggcaaaag gggacggaag 1380
   ggatggagcc agaggagggg gtgacagttg agtctcagcc acaggtgaag gtggagccac 1440
   tggaggaagc catccctctg ccccctacga agaagaggaa aaaagaaaag ggacagatgg 1500
   caatgatgga gccagggacg gaggcgatgg agccagtgga gccggagatg aagcctctgg 1560
   agtccccagg ggggaccatg gcgcctcaac agccagaagg agcgaagcct caggcccagg 1620
   cagctctggc agctcccaaa aagaagacga agaaagaaaa acagcaagat gccacagtgg 1680
   agccagagac agaggtggtg gggcctgagc tgccggatga ccttgagcct caggcagctc 1740
   ccacatccac caagaagaag aagaagaaga aagagagagg tcacacagtg actgagccaa 1800
   ttcagccact agagcctgaa ctgccagggg agggacagcc tgaagccagg gcaactccgg 1860
   gatccaccaa gaagaggaag aagcagagtc aggaaagccg gatgccagag acagtgcccc 1920
   aagaggagat gccagggccg ccactgaatt cagagtctgg ggaggaggct cccacaggcc 1980
   gggacaagaa gcggaagcag cagcagcagc agcctgtgta gtctgccccc gggaaactga 2040
   ggaactaaag aaagctgaag gtgcccacct gggccaccag aaggtgacac ccccagaatc 2100
   cctccccaga gactgcacca gcgcagccag caggagcctg gcctgggagg acgatttatt 2160
   attacactgg gggtttcctt ggcagctggg gtcatcaggg tactttcaag aagggctcgt 2220
   gcaggacatc aaacagcctc cgggcctgga tgggagggag aaaaaaatga ggaaccagtc 2280
   attaaaggag ctgtttcctg ggtaaatcta gagtggggtt ttggttcttt attttcccct 2340
   ataccctcaa gcatttatcc attgagttac aaacaatcca gttacaatct ttttaagtta 2400
   ttattattat tattattttt tttttttttg agatggagtc tcgctctgtc gcccaggttg 2460
   gagtgcagtg gcgcaatctc ggctcactgc aagctccgcc tcccgggttc acgccattct 2520
   cctgcctcag cctcctgagt agctgggact acaggcccct gcccagctaa ttttttgtat 2580
   ttttttttag tagagatggg gtttcaccac gttagccagg atggtctcga tctcctgacc 2640
   tcctgatgcg cctgcctcag cctcccagtg ctgggattat aggtgtgagc cactgcgcct 2700
   ggctaagtta ttattatttt tttgagacag tctcctggtg tcacccaggc tggagtgcag 2760
   tggtgtgatc ttggctcact gcaacctccg cctcctgggt tccaacgatt ctcctgcctc 2820
   agcctcccga gtagctgggc ctaaaggtgc ccaccactat acccggctaa tttttgtatt 2880
   tttagtagag acaggggttt caccatattg gccaggctgg tctcgaactc ctgacctcgt 2940
   gatccacctg ccttgacctc ccaaagtgct aggataacag gtgtgagcca ccgcaccctg 3000
   ccaagttatt ttaaaatgta ccattattat tgactatagt cacctggttg tgttatcaaa 3060
   tagtatgtct tattcattct ttctttgtgt gtgtgtgtgt gtgtgtgtgt gtgtgtggta 3120
   cccattaacc ttccccatct ccctgccagc ccctaactac cctccccagc ctccaggaac 3180
   tatccatcca ctcttatctc catgagttca attgttttga tttttagata cacaaataaa 3240
   taagaacatg caatgtttgt ctttctgtgc ctggcttatt tcactt 3286
<210> 263
   <211> 3136
   <212> DNA
   <213> Homo sapiens
<220>
   <223> early growth response 1 (EGR1), transcription factor ETR103, transcription factor Zif268, nerve growth factor-induced protein A (NGFI-A), zinc finger protein 225 (ZNF225), zinc finger protein Krox-24 (KROX-24), AT225, G0S30, TIS8
<220>
   <221> CDS
   <222> (271) ... (1902)
   <223> EGR1
<400> 263
   gcgcagaact tggggagccg ccgccgccat ccgccgccgc agccagcttc cgccgccgca 60
   ggaccggccc ctgccccagc ctccgcagcc gcggcgcgtc cacgcccgcc cgcgcccagg 120
   gcgagtcggg gtcgccgcct gcacgcttct cagtgttccc cgcgccccgc atgtaacccg 180
   gccaggcccc cgcaactgtg tcccctgcag ctccagcccc gggctgcacc cccccgcccc 240
   gacaccagct ctccagcctg ctcgtccagg atggccgcgg ccaaggccga gatgcagctg 300
   atgtccccgc tgcagatctc tgacccgttc ggatcctttc ctcactcgcc caccatggac 360
   aactacccta agctggagga gatgatgctg ctgagcaacg gggctcccca gttcctcggc 420
   gccgccgggg ccccagaggg cagcggcagc aacagcagca gcagcagcag cgggggcggt 480
   ggaggcggcg ggggcggcag caacagcagc agcagcagca gcaccttcaa ccctcaggcg 540
   gacacgggcg agcagcccta cgagcacctg accgcagagt cttttcctga catctctctg 600
   aacaacgaga aggtgctggt ggagaccagt taccccagcc aaaccactcg actgcccccc 660
   atcacctata ctggccgctt ttccctggag cctgcaccca acagtggcaa caccttgtgg 720
   cccgagcccc tcttcagctt ggtcagtggc ctagtgagca tgaccaaccc accggcctcc 780
   tcgtcctcag caccatctcc agcggcctcc tccgcctccg cctcccagag cccacccctg 840
   agctgcgcag tgccatccaa cgacagcagt cccatttact cagcggcacc caccttcccc 900
   acgccgaaca ctgacatttt ccctgagcca caaagccagg ccttcccggg ctcggcaggg 960
   acagcgctcc agtacccgcc tcctgcctac cctgccgcca agggtggctt ccaggttccc 1020
   atgatccccg actacctgtt tccacagcag cagggggatc tgggcctggg caccccagac 1080
   cagaagccct tccagggcct ggagagccgc acccagcagc cttcgctaac ccctctgtct 1140
   actattaagg cctttgccac tcagtcgggc tcccaggacc tgaaggccct caataccagc 1200
   taccagtccc agctcatcaa acccagccgc atgcgcaagt accccaaccg gcccagcaag 1260
   acgccccccc acgaacgccc ttacgcttgc ccagtggagt cctgtgatcg ccgcttctcc 1320
   cgctccgacg agctcacccg ccacatccgc atccacacag gccagaagcc cttccagtgc 1380
   cgcatctgca tgcgcaactt cagccgcagc gaccacctca ccacccacat ccgcacccac 1440
   acaggcgaaa agcccttcgc ctgcgacatc tgtggaagaa agtttgccag gagcgatgaa 1500
   cgcaagaggc ataccaagat ccacttgcgg cagaaggaca agaaagcaga caaaagtgtt 1560
   gtggcctctt cggccacctc ctctctctct tcctacccgt ccccggttgc tacctcttac 1620
   ccgtccccgg ttactacctc ttatccatcc ccggccacca cctcataccc atcccctgtg 1680
   cccacctcct tctcctctcc cggctcctcg acctacccat cccctgtgca cagtggcttc 1740
   ccctccccgt cggtggccac cacgtactcc tctgttcccc ctgctttccc ggcccaggtc 1800
   agcagcttcc cttcctcagc tgtcaccaac tccttcagcg cctccacagg gctttcggac 1860
   atgacagcaa ccttttctcc caggacaatt gaaatttgct aaagggaaag gggaaagaaa 1920
   gggaaaaggg agaaaaagaa acacaagaga cttaaaggac aggaggagga gatggccata 1980
   ggagaggagg gttcctctta ggtcagatgg aggttctcag agccaagtcc tccctctcta 2040
   ctggagtgga aggtctattg gccaacaatc ctttctgccc acttcccctt ccccaattac 2100
   tattcccttt gacttcagct gcctgaaaca gccatgtcca agttcttcac ctctatccaa 2160
   agaacttgat ttgcatggat tttggataaa tcatttcagt atcatctcca tcatatgcct 2220
   gaccccttgc tcccttcaat gctagaaaat cgagttggca aaatggggtt tgggcccctc 2280
   agagccctgc cctgcaccct tgtacagtgt ctgtgccatg gatttcgttt ttcttggggt 2340
   actcttgatg tgaagataat ttgcatattc tattgtatta tttggagtta ggtcctcact 2400
   tgggggaaaa aaaaaaaaga aaagccaagc aaaccaatgg tgatcctcta ttttgtgatg 2460
   atgctgtgac aataagtttg aacctttttt tttgaaacag cagtcccagt attctcagag 2520
   catgtgtcag agtgttgttc cgttaacctt tttgtaaata ctgcttgacc gtactctcac 2580
   atgtggcaaa atatggtttg gtttttcttt tttttttttt ttgaaagtgt tttttcttcg 2640
   tccttttggt ttaaaaagtt tcacgtcttg gtgccttttg tgtgatgcgc cttgctgatg 2700
   gcttgacatg tgcaattgtg agggacatgc tcacctctag ccttaagggg ggcagggagt 2760
   gatgatttgg gggaggcttt gggagcaaaa taaggaagag ggctgagctg agcttcggtt 2820
   ctccagaatg taagaaaaca aaatctaaaa caaaatctga actctcaaaa gtctattttt 2880
   ttaactgaaa atgtaaattt ataaatatat tcaggagttg gaatgttgta gttacctact 2940
   gagtaggcgg cgatttttgt atgttatgaa catgcagttc attattttgt ggttctattt 3000
   tactttgtac ttgtgtttgc ttaaacaaag tgactgtttg gcttataaac acattgaatg 3060
   cgctttattg cccatgggat atgtggtgta tatccttcca aaaaattaaa acgaaaataa 3120
   agtagctgcg attggg                                                 3136
<210> 264
   <211> 937
   <212> DNA
   <213> Homo sapiens
<220>
   <223> pleckstrin homology-like domain, family A, member 2 (PHLDA2), tumor suppressing subchromosomal transferable fragment cDNA 3 (TSSC3), Beckwith-Wiedemann syndrome chromosomal region 1 candidate gene C protein (BWR1C), HLDA2, IPL
<220>
   <221> CDS
   <222> (57) ... (515)
   <223> PHLDA2
<400> 264
   agagccggcg ccgtcaccgc ccgcattgcc gctcccagtc ccgcgctcgg cacgacatga 60
   aatcccccga cgaggtgcta cgcgagggcg agttggagaa gcgcagcgac agcctcttcc 120
   agctatggaa gaagaagcgc ggggtgctca cctccgaccg cctgagcctg ttccccgcca 180
   gcccccgcgc gcgccccaag gagctgcgct tccactccat cctcaaggtg gactgcgtgg 240
   agcgcacggg caagtacgtg tacttcacca tcgtcaccac cgaccacaag gagatcgact 300
   tccgctgcgc gggcgagagc tgctggaacg cggccatcgc gctggcgctc atcgatttcc 360
   agaaccgccg cgccctgcag gactttcgca gccgccagga acgcaccgca cccgccgcac 420
   ccgccgagga cgccgtggct gccgcggccg ccgcaccctc cgagccctcg gagccctcca 480
   ggccatcccc gcagcccaaa ccccgcacgc catgagcccg ccgcgggcca tacgctggac 540
   gagtcggacc gaggctagga cgtggccggc gctctccagc cctgcagcag aagaacttcc 600
   cgtgcgcgcg gatcctcgct ccgttgcacg ggcgccttaa gttattggac tatctaatat 660
   ctatgtattt atttcgctgg ttctttgtag tcacatattt tatagtctta atatcttgtt 720
   tttgcatcac tgtgcccatt gcaaataaat cacttggcca gtttgctttt ctaccatccg 780
   gctgtggctc agtgagactc ctgctgggag ggtggaggcc caggaatggg cgggcaggac 840
   accctcatcc agtcctgcgg ggctggtgtg aaaggcgctg ggaaccggct ttgaatgaat 900
   aaatgaatcg tgtcatctgc aaaaaaaaaa aaaaaaa                          937
<210> 265
   <211> 2223
   <212> DNA
   <213> Homo sapiens
<220>
   <223> AT rich interactive domain 5A (MRF1-like), ARID domain-containing protein 5A (ARID5A), modulator recognition factor 1 (MRF-1, MRF1), RFVG5814, RP11-363D14
<220>
   <221> CDS
   <222> (101) ... (1885)
   <223> ARID5A
<400> 265
   ccggcgggcc gcgccgcgag ccagtatctc agagagcgcg gggtccggac agccgcgcgc 60
   tgagggtctc ggggcgggcg ccgcgggacc tctccgggcc atggcagccc ctgtcaaagg 120
   gaacaggaag cagtccacgg agggtgacgc cctagaccca cctgcatccc ccaaacctgc 180
   tggcaagcag aacggaatcc agaaccccat ctcgctggag gactcccccg aggcaggcgg 240
   ggagcgggag gaggagcagg agcgggagga ggagcaggcc ttcctggtca gcctctacaa 300
   gttcatgaag gagcgacaca cgcccatcga gagggtgccc catctcggct tcaagcagat 360
   taacctgtgg aagatctaca aagcagtgga gaagctgggg gcctatgagc tggtgaccgg 420
   gcgccgcctc tggaagaacg tgtacgacga gctggggggc agcccaggca gcaccagcgc 480
   ggccacgtgc acgcgccgcc actacgagag gctggtcctg ccatacgtgc ggcacctgaa 540
   gggggaggat gacaagccgc tgcccacctc caagcccagg aaacagtaca agatggctaa 600
   ggagaacagg ggggatgatg gggccaccga gaggccgaag aaggccaagg aggagcggcg 660
   catggaccag atgatgccag gaaagaccaa agcagatgct gctgacccag caccacttcc 720
   cagccaggag ccccccagga acagcacaga acagcagggc ctggcctctg ggtcttctgt 780
   gtcctttgtg ggtgccagcg gctgtcctga ggcctacaag cggctcctat ccagcttcta 840
   ctgcaagggg acacacggca tcatgtcacc actggccaaa aagaagctcc tggcccaggt 900
   gagcaaggtg gaggccttgc agtgccagga ggagggctgc cgccatgggg cagagcccca 960
   ggcgtcccca gctgttcacc tcccagagag tccccagagc cccaaagggc tgactgagaa 1020
   ctccaggcac cggctgaccc ctcaggaggg attgcaggcc ccaggtggca gcctcagaga 1080
   ggaggcgcag gcaggcccct gcccggcagc ccccatcttc aagggctgct tctacaccca 1140
   ccccaccgag gtgctgaagc ctgtcagcca gcaccccagg gacttcttct ctagacttaa 1200
   agatggggtg ctattggggc ctcctggcaa agaggggctg tcagtgaaag agccccagct 1260
   ggtgtggggc ggagacgcta accgcccttc tgcgttccat aaaggtggct ccagaaaggg 1320
   catcctctac cccaagccca aagcctgctg ggtgtccccc atggccaagg tcccagccga 1380
   gagccccacg ctcccgccca ccttccccag tagcccaggc ctgggcagca agcgcagcct 1440
   ggaggaagag ggtgctgccc acagtgggaa gagactgcgg gccgtgtctc cctttcttaa 1500
   ggaggcggat gccaagaagt gtggggccaa acctgcaggg tccggcctgg tctcctgcct 1560
   tctgggccca gccctggggc ctgtgccccc agaggcctac aggggcacca tgctgcactg 1620
   cccgctgaac ttcactggca ccccgggccc cttgaagggc caggctgcac tccccttcag 1680
   ccccctggtc atcccggcct tcccggccca cttcctggcc accgcaggcc cctcgcccat 1740
   ggccgctggc ctgatgcact tccccccaac gtccttcgac agtgccctcc gccacagact 1800
   ttgcccggcc tcatctgcct ggcacgcacc accagtcaca acctatgcag cgccccactt 1860
   cttccacctc aacaccaagc tgtaggccag cccatggtgt tgtgtacact gtggagtcga 1920
   caggggccta caacaggcag gtactgctgc cagggggctc tgaactagtg cctgctaccc 1980
   aggacacccg ggccatgccc ctggctgggc agcctggcac aagtgaagaa gaaggcagtg 2040
   ggaaaactgg gtttatctca aggcagcagc ctgagcccag gagcagagga cccagttgtt 2100
   ataaggcgct gggagaggat gggcagctcc cactgcccca gagcggagct cgaagcaccc 2160
   aggttgccca cggaaaatcc aataaaaaga caccagtgtg aatccaaaaa aaaaaaaaaa 2220
   aaa                                                               2223
<210> 266
   <211> 2842
   <212> DNA
   <213> Homo sapiens
<220>
   <223> dual specificity phosphatase 6 transcript variant 1 (DUSP6), serine/threonine specific protein phosphatase, mitogen-activated protein kinase phosphatase 3 (MKP3), dual specificity protein phosphatase PYST1, HH19
<220>
   <221> CDS
   <222> (481) ... (1626)
   <223> DUSP6
<400> 266
   gagacgctcg ctgtttgtat ccattgagga gctgcctcgc gcagggggtg tgcgaggctg 60
   agtccaagag atagcaaatc gagtcttaaa taatccgggg agaaagacgc ccgggtagat 120
   ttgaggtgca gccttggagg gagggattag aagccgctag actttttttc ctcccctctc 180
   agtagcacgg agtccgaatt aattggattt cattcactgg ggaggaacaa aaactatctg 240
   ggcagcttca ttgagagaga ttcattgaca ctaagagcca gcggctgcag ctgggtgcag 300
   agagaacctc cggctttact tctgtctcgt ctgccccaac cgctagcctc ggcttgggta 360
   aggcgaggcg gaattaaacc ccgctccgag agcggcagct tcgcgcgcgg tgcgctcggc 420
   ctatgcctgc cccgaggggc gtctggtagg caccccgccc tctcccgcag ctcgaccccc 480
   atgatagata cgctcagacc cgtgcccttc gcgtcggaaa tggcgatcag caagacggtg 540
   gcgtggctca acgagcagct ggagctgggc aacgagcggc tgctgctgat ggactgccgg 600
   ccgcaggagc tatacgagtc gtcgcacatc gagtcggcca tcaacgtggc catcccgggc 660
   atcatgctgc ggcgcctgca gaagggtaac ctgccggtgc gcgcgctctt cacgcgcggc 720
   gaggaccggg accgcttcac ccggcgctgt ggcaccgaca cagtggtgct ctacgacgag 780
   agcagcagcg actggaacga gaatacgggc ggcgagtcgg tgctcgggct gctgctcaag 840
   aagctcaagg acgagggctg ccgggcgttc tacctggaag gtggcttcag taagttccaa 900
   gccgagttct ccctgcattg cgagaccaat ctagacggct cgtgtagcag cagctcgccg 960
   ccgttgccag tgctggggct cgggggcctg cggatcagct ctgactcttc ctcggacatc 1020
   gagtctgacc ttgaccgaga ccccaatagt gcaacagact cggatggtag tccgctgtcc 1080
   aacagccagc cttccttccc agtggagatc ttgcccttcc tctacttggg ctgtgccaaa 1140
   gactccacca acttggacgt gttggaggaa ttcggcatca agtacatctt gaacgtcacc 1200
   cccaatttgc cgaatctctt tgagaacgca ggagagttta aatacaagca aatccccatc 1260
   tcggatcact ggagccaaaa cctgtcccag tttttccctg aggccatttc tttcatagat 1320
   gaagcccggg gcaagaactg tggtgtcttg gtacattgct tggctggcat tagccgctca 1380
   gtcactgtga ctgtggctta ccttatgcag aagctcaatc tgtcgatgaa cgatgcctat 1440
   gacattgtca aaatgaaaaa atccaacata tcccctaact tcaacttcat gggtcagctg 1500
   ctggacttcg agaggacgct gggactcagc agcccatgtg acaacagggt tccagcacag 1560
   cagctgtatt ttaccacccc ttccaaccag aatgtatacc aggtggactc tctgcaatct 1620
   acgtgaaaga ccccacaccc ctccttgctg gaatgtgtct ggcccttcag cagtttctct 1680
   tggcagcatc agctgggctg ctttctttgt gtgtggcccc aggtgtcaaa atgacaccag 1740
   ctgtctgtac tagacaaggt taccaagtgc ggaattggtt aatactaaca gagagatttg 1800
   ctccattctc tttggaataa caggacatgc tgtatagata caggcagtag gtttgctctg 1860
   tacccatgtg tacagcctac ccatgcaggg actgggattc gaggacttcc aggcgcatag 1920
   ggtagaacca aatgataggg taggagcatg tgttctttag ggccttgtaa ggctgtttcc 1980
   ttttgcatct ggaactgact atataattgt cttcaatgaa gactaattca attttgcata 2040
   tagaggagcc aaagagagat ttcagctctg tatttgtggt atcagtttgg aaaaaaaaat 2100
   ctgatactcc atttgattat tgtaaatatt tgatcttgaa tcacttgaca gtgtttgttt 2160
   gaattgtgtt tgttttttcc tttgatgggc ttaaaagaaa ttatccaaag ggagaaagag 2220
   cagtatgcca cttcttaaaa cagaacaaaa caaaaaaaga aaattgtgct cttttctaat 2280
   ccaaagggta tatttgcagc atgcttgact ttaccaattc tgatgacatc tttacggaca 2340
   ctattatcac taagaccttg ttatggcgaa gtctttagtc tttttcatgt attttcctca 2400
   tgattttttc tctttatgta gtttgactat gccttacctt tgtaaatatt tttgcttgtg 2460
   ttgtcgcaaa ggggataatc tgggaaagac accaaatcat gggctcactt taaaaaaaga 2520
   aagaataaaa aaaccttcag ctgtgctaaa cagtatatta cctctgtata aaattcttca 2580
   gggagtgtca cctcaaatgc aatactttgg gttggtttct ttcctttaaa aaaatttgta 2640
   taaaactgga agtgtgtgtg tgtgagcatg ggtacccatt tgataagaga aatgcatttg 2700
   attgtgaaga agggagagtt aaattctcca ttatgttcgt ggtgtaaagt ttagagctgg 2760
   aatttattat aagaatgtaa aaccttaaat tattaataaa taactatttt ggctattgaa 2820
   aaaaaaaaaa aaaaaaaaaa aa 2842
<210> 267
   <211> 2126
   <212> DNA
   <213> Homo sapiens
<220>
   <223> sprouty homolog 2 (Drosophila) (SPRY2, spry-2, hSPRY2)
<220>
   <221> CDS
   <222> (382) ... (1329)
   <223> SPRY2
<400> 267
   gtaaggccgt tttcttttcc cattcgctca tctgccagga aaagggactt gccgttggcg 60
   cttcggcctc ttgttcattg agaaaaaaga ggaaatactc cgcgtgcgct tgtagaaggg 120
   gagtcgtctc cagctccgaa ccccggagtg ttcatcagcg gggaatctgg ctccgaattc 180
   tctttttttc tcccgccgat tgctcggaag ttggtctaaa gcagaggttg gaaagaaagg 240
   aaaaaagttt gcatcgagac tggatttatt tgcacatcgc agaaagaaga gaatccaagg 300
   gagaggggtt ggtgcaaagc cgcgatcacg gagttcagat gtgttctaag cctgctggag 360
   tgaccacact tccaagacct gatggaggcc agagctcaga gtggcaacgg gtcgcagccc 420
   ttgctgcaga cgccccgtga cggtggcaga cagcgtgggg agcccgaccc cagagacgcc 480
   ctcacccagc aggtacatgt cttgtctctg gatcagatca gagccatccg aaacaccaat 540
   gagtacacag aggggcctac tgtcgtccca agacctgggc tcaagcctgc tcctcgcccc 600
   tccactcagc acaaacacga gagactccac ggtctgcctg agcaccgcca gcctcctagg 660
   ctccagcact cgcaggtcca ttcttctgca cgagcccctc tgtccagatc cataagcacg 720
   gtcagctcag ggtcgcggag cagtacgagg acaagtacca gcagcagctc ctctgaacag 780
   agactgctag gatcatcctt ctcctccggg cctgttgctg atggcataat ccgggtgcaa 840
   cccaaatctg agctcaagcc aggtgagctt aagccactga gcaaggaaga tttgggcctg 900
   cacgcctaca ggtgtgagga ctgtggcaag tgcaaatgta aggagtgcac ctacccaagg 960
   cctctgccat cagactggat ctgcgacaag cagtgccttt gctcggccca gaacgtgatt 1020
   gactatggga cttgtgtatg ctgtgtgaaa ggtctcttct atcactgttc taatgatgat 1080
   gaggacaact gtgctgacaa cccatgttct tgcagccagt ctcactgttg tacacgatgg 1140
   tcagccatgg gtgtcatgtc cctctttttg ccttgtttat ggtgttacct tccagccaag 1200
   ggttgcctta aattgtgcca ggggtgttat gaccgggtta acaggcctgg ttgccgctgt 1260
   aaaaactcaa acacagtttg ctgcaaagtt cccactgtcc cccctaggaa ctttgaaaaa 1320
   ccaacatagc atcattaatc aggaatatta cagtaatgag gattttttct ttcttttttt 1380
   aatacacata tgcaaccaac taaacagtta taatcttggc actgttaata gaaaggtggg 1440
   atagtctttg ctgtttgcgg tgaaatgctt tttgtccatg tgccgtttta actgatatgc 1500
   ttgttagaac tcagctaatg gagctcaaag tatgagatac agaacttggt gacccatgta 1560
   ttgcataagc taaagcaaca cagacactcc taggcaaagt ttttgtttgt gaatagtact 1620
   tgcaaaactt gtaaattagc agatgacttt tttccattgt tttctccaga gagaatgtgc 1680
   tatatttttg tatatacaat aatatttgca actgtgaaaa acaagttgtg ccatactaca 1740
   tggcacagac acaaaatatt atactaatat gttgtacatt cggaagaatg tgaatcaatc 1800
   agtatgtttt tagattgtat tttgccttac agaaagcctt tattgtaaga ctctgatttc 1860
   cctttggact tcatgtatat tgtacagtta cagtaaaatt caacctttat tttctaattt 1920
   tttcaacata ttgtttagtg taaagaatat ttatttgaag ttttattatt ttataaaaaa 1980
   gaatatttat tttaagaggc atcttacaaa ttttgcccct tttatgagga tgtgatagtt 2040
   gctgcaaatg aggggttaca gatgcatatg tccaatataa aatagaaaat atattaacgt 2100
   ttgaaattaa aaaaaaaaaa aaaaaa                                       2126
<210> 268
   <211> 4720
   <212> DNA
   <213> Homo sapiens
<220>
   <223> DEAD (Asp-Glu-Ala-Asp) box polypeptide 21 transcript variant 1, DEAD (Asp-Glu-Ala-Asp) box helicase 21 (DDX21), RNA helicase II/Gu alpha (RH-II/GU, RH-II/GuA), nucleolar RNA helicase Gu, nucleolar RNA helicase II, gu-alpha (GUA), GURDB
<220>
   <221> CDS
   <222> (104) ... (2455)
   <223> DDX21
<400> 268
   gctacgggaa gtgacgagag ccgggtggac ccagggtggg gaactacctc ttcctctcca 60
   cgcggttgag aagaccggtc ggcctgggca acctgcgctg aagatgccgg gaaaactccg 120
   tagtgacgct ggtttggaat cagacaccgc aatgaaaaaa ggggagacac tgcgaaagca 180
   aaccgaggag aaagagaaaa aagagaagcc aaaatctgat aagactgaag agatagcaga 240
   agaggaagaa actgttttcc ccaaagctaa acaagttaaa aagaaagcag agccttctga 300
   agttgacatg aattctccta aatccaaaaa ggcaaaaaag aaagaggagc catctcaaaa 360
   tgacatttct cctaaaacca aaagtttgag aaagaaaaag gagcccattg aaaagaaagt 420
   ggtttcttct aaaaccaaaa aagtgacaaa aaatgaggag ccttctgagg aagaaataga 480
   tgctcctaag cccaagaaga tgaagaaaga aaaggaaatg aatggagaaa ctagagagaa 540
   aagccccaaa ctgaagaatg gatttcctca tcctgaaccg gactgtaacc ccagtgaagc 600
   tgccagtgaa gaaagtaaca gtgagataga gcaggaaata cctgtggaac aaaaagaagg 660
   cgctttctct aattttccca tatctgaaga aactattaaa cttctcaaag gccgaggagt 720
   gaccttccta tttcctatac aagcaaagac attccatcat gtttacagcg ggaaggactt 780
   aattgcacag gcacggacag gaactgggaa gacattctcc tttgccatcc ctttgattga 840
   gaaacttcat ggggaactgc aagacaggaa gagaggccgt gcccctcagg tactggttct 900
   tgcacctaca agagagttgg caaatcaagt aagcaaagac ttcagtgaca tcacaaaaaa 960
   gctgtcagtg gcttgttttt atggtggaac tccctatgga ggtcaatttg aacgcatgag 1020
   gaatgggatt gatatcctgg ttggaacacc aggtcgtatc aaagaccaca tacagaatgg 1080
   caaactagat ctcaccaaac ttaagcatgt tgtcctggat gaagtggacc agatgttgga 1140
   tatgggattt gctgatcaag tggaagagat tttaagtgtg gcatacaaga aagattctga 1200
   agacaatccc caaacattgc ttttttctgc aacttgccct cattgggtat ttaatgttgc 1260
   caagaaatac atgaaatcta catatgaaca ggtggacctg attggtaaaa agactcagaa 1320
   aacggcaata actgtggagc atctggctat taagtgccac tggactcaga gggcagcagt 1380
   tattggggat gtcatccgag tatatagtgg tcatcaagga cgcactatca tcttttgtga 1440
   aaccaagaaa gaagcccagg agctgtccca gaattcagct ataaagcagg atgctcagtc 1500
   cttgcatgga gacattccac agaagcaaag ggaaatcacc ctgaaaggtt ttagaaatgg 1560
   tagttttgga gttttggtgg caaccaatgt tgctgcacgt gggttagaca tccctgaggt 1620
   tgatttggtt atacaaagct ctccaccaaa ggatgtagag tcctacattc atcgatccgg 1680
   gcggacaggc agagctggaa ggacgggggt gtgcatctgc ttttatcagc acaaggaaga 1740
   atatcagtta gtacaagtgg agcaaaaagc gggaattaag ttcaaacgaa taggtgttcc 1800
   ttctgcaaca gaaataataa aagcttccag caaagatgcc atcaggcttt tggattccgt 1860
   gcctcccact gccattagtc acttcaaaca atcagctgag aagctgatag aggagaaggg 1920
   agctgtggaa gctctggcag cagcactggc ccatatttca ggtgccacgt ccgtagacca 1980
   gcgctccttg atcaactcaa atgtgggttt tgtgaccatg atcttgcagt gctcaattga 2040
   aatgccaaat attagttatg cttggaaaga acttaaagag cagctgggcg aggagattga 2100
   ttccaaagtg aagggaatgg tttttctcaa aggaaagctg ggtgtttgct ttgatgtacc 2160
   taccgcatca gtaacagaaa tacaggagaa atggcatgat tcacgacgct ggcagctctc 2220
   tgtggccaca gagcaaccag aactggaagg accacgggaa ggatatggag gcttcagggg 2280
   acagcgggaa ggcagtcgag gcttcagggg acagcgggac ggaaacagaa gattcagagg 2340
   acagcgggaa ggcagtagag gcccgagagg acagcgatca ggaggtggca acaaaagtaa 2400
   cagatcccaa aacaaaggcc agaagcggag tttcagtaaa gcatttggtc aataattaga 2460
   aatagaagat ttatatagca aaaagagaat gatgtttggc aatatagaac tgaacattat 2520
   ttttcatgca aagttaaaag cacattgtgc ctccttttga ccacttgcca agtccctgtc 2580
   tctttcagac acagacaagc ttcatttaaa ttatttcatc tgatcattat catttataac 2640
   tttattgtta cttcttcatc agtttttcct tttgaaaggt gtatgaattc attacatttt 2700
   tattctaatg tattatctgt agattagaag ataaaatcaa gcatgtatct gcctatactt 2760
   tgtgagttca cctgtcttta tactcaaaag tgtcccttaa tagtgtcctt ccctgaaata 2820
   aatacctaag ggagtgtaac agtctctgga ggaccacttt gagcctttgg aagttaaggt 2880
   ttcctcagcc acctgccgaa cagtttctca tgtggtccta ttatttgtct actgagactt 2940
   aatactgagc aatgttttga aacaagattt caaactaatc tgggttgtaa tacagtttat 3000
   accagtgtat gctctagact tggaagatgt agtatgtttg atgtggatta cctatactta 3060
   tgttcgtttt gatacatttt tagcttctca ttataaggtg attcatgctt tagtgaattc 3120
   ttcatagata gtatatataa aagtacattt taatagaaag ccagggtttt aaggaatttc 3180
   acatgtataa ggtggctcca tagctttatt tgtaagtagg ctggataaat ggtgcttaaa 3240
   tggtaatgta ctccacttct tcctattgga agattaacat tatttaccaa gaaggactta 3300
   agggagtagg gggcgcagat tagcattgct caagagtatg taaaaaaaaa aaaaaaaaaa 3360
   aagaaccaaa ccactggaaa taatcaaatg caaaaaggta acaaattcat aactggaaag 3420
   caaagagaag aacaagtatg atttggatga taaagcattg ttttaatggt gaaaacttca 3480
   cagatcacta atgtttctag aggttaactt caagtgggca agctggggtt tttaggtagt 3540
   cagtggccta gttcctaaag ccacagtata ggatctgtta aactgaatgt ctgttgaaag 3600
   tttgttttag ctgcttggag gcttcctttt aagacaaact gtatgtgatt aagttgtttt 3660
   gagggaactg aagaacctga tgtagcccct ggccagataa ctgcctgatt tctcagatat 3720
   tatttctctg ggaaacattc tacatagcac aggagcttaa gagtggcatt atcttctcgc 3780
   cttaatttcc agagattatt tctgtactga gaatcctgga actactatgc taggaaattt 3840
   aaagctgcat ggtctgtctt gttttcattt aattattgtg aatacctaga atctttcttg 3900
   gtcctgattt ctcttgctta atccagtctt tatctctaac tgccccttat ttgatcacca 3960
   tgtactagga gctctgatag ccagctcagc tcctaatcct tgaggcaaca ttctttttct 4020
   atttgaactt cagttctgtc cttgaatccc gactagatat ttcttgccct ctggtctcag 4080
   aattctcttg gcttttattc cttgatccac ttgccagttt tatcacttta cccttgttcc 4140
   tcatggcttc ccatcaagcc atgggtatta ggtgacagtg taatttatta gattctggtt 4200
   ttgcccaaat actgggcatg ctttaataat aactgaacca tttcattatt tggataggca 4260
   tgggtacctt atcaagcaga ttaaaaggat atggtacccg tcctttagaa aagaacagct 4320
   aaaaccttgt tgtggattat ggatttagcc taaagaaaaa taatctggca taaattaaga 4380
   gtaagagaga agattaatag aaatttcact tcacataact taaaacatgg ctatttcaat 4440
   aaaggactaa agtttctcct ggatcccaga attcaacctg tatttataaa tgtataatgt 4500
   atttagctac tttttggttt aaatgaactt gttgggttag cttggtaaat gttataattt 4560
   ttactatttt ctacaaagaa aatattttct aatttaagtt ggagctatct gtgcagcagt 4620
   ttctctacag ttgtgcataa atgtttttac tataaaatga gctaatgtat aaaatactgc 4680
   tgtataccat aataaagata gtaatacttg ataaaaaaaa                       4720
<210> 269
   <211> 2537
   <212> DNA
   <213> Homo sapiens
<220>
   <223> GTP binding protein 4 (GTPBP4), G protein-binding protein CRFG, G protein-binding protein NGB, chronic renal failure gene protein (CRFG), NOG1
<220>
   <221> CDS
   <222> (72) ... (1976)
   <223> GTPBP4
<400> 269
   ggaagtccca cctgcgcccg acggcggaag ttccgggagt gccaagtacc cgcgtgcata 60
   cggctgccgg catggcacat tacaacttca agaaaattac ggtggtgccg tccgccaagg 120
   acttcataga cctcacgttg tcgaagactc aacgaaagac tccaaccgtt attcataaac 180
   attaccaaat acatcgcatt agacattttt acatgagaaa agtcaaattt actcaacaga 240
   attaccatga tagactttca caaattctaa cagatttccc caaattggat gatattcatc 300
   cgttctatgc tgatttgatg aatattctct acgacaagga tcattacaag ttggctctgg 360
   ggcaaataaa tattgccaaa aatttagtgg acaatgttgc taaagattat gtgcgactga 420
   tgaagtatgg cgactctctc taccgctgca aacagctgaa gcgtgcggcc ctgggacgga 480
   tgtgcacagt gatcaagagg cagaagcaga gtttggagta tttggagcaa gtgcgtcagc 540
   atttatcccg tttgccaacc attgatccga ataccaggac cctgcttttg tgtgggtacc 600
   caaatgttgg gaagtccagc ttcatcaaca aggtgacgag agcagacgtg gatgtccagc 660
   cctatgcgtt cacaaccaag tctctgtttg ttgggcacat ggattataag tatctacgtt 720
   ggcaggttgt agacactcct gggatcctgg accaccctct ggaggatagg aacaccatcg 780
   agatgcaggc catcactgcc ctggcccacc tccgtgctgc ggtcctgtat gtgatggatt 840
   tgtctgagca gtgtgggcat gggctgaggg agcagctaga actcttccag aacatcagac 900
   ctctcttcat caacaagcct ctcatagttg tagccaacaa atgtgatgtg aagagaatag 960
   ctgaactttc tgaagatgat cagaaaatat ttacagattt gcagtctgaa ggattccctg 1020
   taatagagac cagcaccctg actgaggaag gtgttattaa agttaaaaca gaggcttgcg 1080
   ataggctttt ggctcatcga gtggaaacca aaatgaaggg aaataaagtg aatgaggtgc 1140
   tgaatagact gcacctggct atcccaacca ggagggacga taaggagagg ccccctttca 1200
   tccctgaagg agtggtggct cgcaggaaga ggatggaaac tgaggagtcc aggaagaaga 1260
   gggaacgaga tcttgagctg gaaatgggag atgattatat tttggatctt cagaagtact 1320
   gggatttaat gaatttgtct gaaaaacatg ataagatacc agaaatctgg gaaggccata 1380
   atatagctga ttatattgat ccagccatca tgaagaaatt ggaagaatta gaaaaagaag 1440
   aagagctgag aacagctgct ggagagtatg acagtgtatc tgagagtgaa gacgaagaga 1500
   tgctggaaat ccgacagctg gcaaagcaaa ttcgagagaa aaagaagttg aaaattctgg 1560
   agtccaaaga aaagaataca cagggaccca ggatgccgcg aactgctaag aaggttcaga 1620
   ggacagtttt ggagaaggag atgcgtagtc ttggtgttga catggacgat aaagacgatg 1680
   cccattacgc agtccaggca agaagatccc ggagcatcac taggaaaaga aagcgggaag 1740
   actctgctcc cccgtcctct gtggcccgga gtgggagttg ctctcgaact ccacgtgacg 1800
   tttctggtct tagggatgtc aagatggtga agaaagccaa gactatgatg aagaatgctc 1860
   agaagaagat gaatcggttg gggaagaaag gggaggcgga tagacacgtg tttgatatga 1920
   agcccaagca cttgctgtct gggaagagga aagctggtaa aaaggacagg agatagtatc 1980
   cgtttggttg gcgtggcttc gctagagtgt tgctgtttat ttcctggttt ggcacagtat 2040
   ggtttcatga aattggagct ctgtataaac tgaaaaagac aaaataagta aagcacttgt 2100
   tgctttgctg aaaactatgg ttaaccctat ataggtgtgg gaaatttttg tcactgcata 2160
   atattacaaa tattttgagt agacagtgtt tccacattta atggagtatc agttgcttca 2220
   gattttcaga actgggaaga tttactggtg taactgggtt gtttttgatg gagaaaaacc 2280
   ttattttctt ttgtaagagc tgggagcaaa cacgtttatg agtgtgtcgg aatcccgtgc 2340
   ttaaaatacg ctcttaaatt attttctagt cttattttac aatgtctcat tgtagtctgt 2400
   cttcaactat tttatccaaa ataaacctcc agaagaaagt agttttcatt tacttagctc 2460
   atgttttggt ttagttatag tcgctatgga tttggccaaa taaaaaggca aacaacaaaa 2520
   aaaaaaaaaa aaaaaaa                                                2537
<210> 270
   <211> 3713
   <212> DNA
   <213> Homo sapiens
<220>
   <223> phosphoribosyl pyrophosphate amidotransferase (PPAT), glutamine phosphoribosylpyrophosphate amidotransferase (GPAT), amidophosphoribosyltransferase (ATASE), amidophosphoribosyltransferase proprotein
<220>
   <221> CDS
   <222> (160) ... (1713)
   <223> PPAT
<400> 270
   agggacagcg cgcgccgccg cagtagctgg ggccaccctc gggaaggaag agcctgtcgc 60
   gagcgcgagc ttctgagctc gacgggccga gctggcagct ggttggtgct tacaccttgg 120
   ccgcagcggc aggtccttcc acgtgctttc ggcggcgaca tggagctgga ggagttgggg 180
   atccgagagg aatgtggcgt gttcgggtgc atcgcctcag gagagtggcc cacgcagctg 240
   gatgtaccgc atgtgatcac tctgggactc gtggggctgc agcaccgggg tcaggagagt 300
   gctggtattg tgactagtga tgggagttcg gtgccaacat tcaaatcaca caagggaatg 360
   ggtcttgtaa atcacgtctt tactgaagac aatttgaaaa aattatatgt ttcaaatctt 420
   ggaattggac acaccaggta tgccaccaca ggaaaatgtg aactagaaaa ttgtcagccc 480
   ttcgttgttg aaacacttca tgggaagata gctgtggcac ataatggcga attggtaaat 540
   gctgctcgat taaggaaaaa gcttctgcgt catggtattg gtctgtctac aagttctgat 600
   agtgaaatga ttacccagtt actggcgtat acccctcctc aggaacaaga tgacacccca 660
   gactgggtag ccaggattaa aaacttgatg aaggaagcac ccacagcata ctccctgctt 720
   ataatgcaca gagatgttat ttatgcagta cgagatcctt atggaaatcg tcccttatgc 780
   attggtcgtc ttattccagt gtctgatata aatgacaaag agaaaaaaac atcagaaaca 840
   gaaggatggg tggtgtcttc agaatcttgt agcttcttat ctattggtgc aagatattac 900
   cgtgaagtct tgcctggaga aattgtggaa atatccagac acaatgtcca aactcttgat 960
   attatatcaa ggtctgaagg aaacccagtg gctttttgta tctttgaata tgtttatttt 1020
   gcaagaccag acagtatgtt cgaagaccaa atggtttata cagtaagata ccgttgtggc 1080
   cagcagctag cgattgaagc acctgtggat gcagatttgg ttagcactgt tccagaatct 1140
   gctacgcctg ctgctcttgc ttacgcagga aagtgtggac ttccatatgt ggaggtgctg 1200
   tgtaaaaacc ggtatgtagg gagaaccttc attcagccaa acatgaggtt aagacaactt 1260
   ggtgttgcaa aaaaatttgg agtattgtca gacaacttta aaggcaaaag aattgttctt 1320
   gtagatgatt caattgtcag aggcaatacc atctcaccta taataaaact gctcaaagaa 1380
   tctggtgcaa aagaggtaca cattcgagta gcttcaccac caattaaata tccatgcttc 1440
   atgggaataa acattcctac aaaagaagag ctcattgcca ataaaccaga atttgatcac 1500
   cttgcagaat atctaggagc aaacagtgtt gtgtatctgt cagtagaagg actggtttca 1560
   tctgtacaag aagggataaa gtttaaaaaa cagaaagaga aaaagcacga tattatgatc 1620
   caagaaaatg gaaatggtct ggaatgtttt gaaaagagtg gtcattgtac agcttgtctc 1680
   actggaaaat atcctgtaga attagaatgg tagctggtag ggttggatgt gtgtagtttc 1740
   aagatagaaa gttggtcaag aagttatagt ggtcacacct catctattta ctgttactca 1800
   gttggtacaa tgtaaaatgc catgcttatg tttataagtt ttgagatttt ttttttttct 1860
   gaaaaggata ccaaagtgcg ataactgaac atttccaatt gcatataata caacaatatg 1920
   tggtgttctt ttttttacac aagcattggc tagccttttt aacctggtca gagaaggcag 1980
   gtggtcactg acatttccca agtccatgct ttaaagggtt tgcaagaagt tagggttaag 2040
   gagaggtgat gccaacaaga caggtgagtt aaatatacca tttcacacaa agtttgaata 2100
   gaatacatta tacctcatag gtgtctagcc tctacagttc tggctgtagt tatgaccttg 2160
   gcttccctgt ctaactgtag acaaatcttt aaaaaaaaat aataataatc tggtgcctca 2220
   gtttccccac atgtgcaatg ggatacttat taaataatta ataagaatgt gaataagtgt 2280
   catacttttg tgatttgagc catcatttca cttctgattt taagacaact catgattgtt 2340
   agctttcaga aagctaatga ttgttaactt tttgaaatta gtttacaatt aattaagatt 2400
   tcattatgat ggaaggagac ataattggca gatctttgcc atctctcttt gagatgtcct 2460
   aaaaagggtt gtaaaaatct gtgaaaaagt ttttcctaca tttgactaga aaatgtgatc 2520
   catagtattt agtgccctga tactataagc tcagcaagta acctggtaca tttgaaataa 2580
   aaaccaaatt tttagattca aacaatccct ttatccttaa tttaattaat tatcatatgc 2640
   tttttttaat gaagtgcttg atcacttgca aacatatata catgtagatg tacatataca 2700
   tgtacacata cacataaata ttattgcaat taagtgatca agtacagaca caataggggc 2760
   cagttttgtt taaggatcaa agagacaacc actttgggga attagtatca acttacaatc 2820
   caagtccaag tatcatctta taatcacttt tttctactat attaagatct aatgaatttg 2880
   atttcttttt tgaagttttt tcttgtaaca tctgagatta gaagtttaag atgacttgac 2940
   cccaaacctt tgtttatgta agaattttta aacataaaag tgtttgtttc tgttatgtta 3000
   ccataatttg atgtatatag tgtccagatc catttagaaa tttaatattt attaataact 3060
   gaaactgttt gtcttccttt ggtatatagt ctcgcatatt atattatagc aggccaagat 3120
   aaaattttga cagctcttta agcccacatg cagcagtggg tcagataacc ctgtggcagt 3180
   gacacgggca aattggcatt tgaataaagc cctgggacca cctcaacatg cgtagcctct 3240
   tgtcttaaat gtactcccca tggcagcatg gaggaggcaa gacctgtggg tcaattttga 3300
   actggcctta ctttgatttt taaaacaaga gactcaggga aagtactaaa ccaaaatctc 3360
   tgattttact ttgcgttttc tgtagttttt gttttactga gatgcttttg taaaggaaaa 3420
   taatactgtg acagtttagt aattctacag attcttaata tttctccatc atggcctttt 3480
   acttcacaat tttctgaagt ctgaattcaa ttacaatttt tttttaccaa tttaatctca 3540
   aatgttgttt aactgcttta aattcatata cgtagagtat tataaactgc agagatgaaa 3600
   aatgtgtttt cacgggattt atattgtgaa ctaaactaag cctacttttt gtgacttatt 3660
   tgtgatgcct tgttgataaa tatgtgtaat aagtatgttt aaaaaaaaaa aaa        3713
<210> 271
   <211> 1069
   <212> DNA
   <213> Homo sapiens
<220>
   <223> hypothetical protein HSPC111, nucleolar protein 16 homolog transcript variant 3 (NOP16), HBV pre-S2 trans-regulated protein 3, HSPC185
<220>
   <221> CDS
   <222> (224) ... (760)
   <223> NOP16
<400> 271
   gtcatgaaag gggcggggaa acgaataaat tgagccttgt acgcaggcgc aaatgctcgt 60
   tgcatcctgg gagtcgtagt gctcagcacg gtagtgctac aaaaggacta catttcccca 120
   aatgcccgca aagccttgtg cacgccttcc ggaaggagtt tgttacacga ggtctgagag 180
   acagaggcag cgtgtttgag ctgctggtgc ggtggtcagc gcgatgccca aggccaaggg 240
   caaaacccgg aggcagaagt ttggttacag tgtcaaccga aagcgtctga accggaatgc 300
   tcgacggaag gcagcgccgc ggatcgaatg ctcccacatc cgacatgcct gggaccacgc 360
   taaatcggta cggcagaacc tggccgagat ggggttggct gtggacccca acagggcggt 420
   gcccctccgt aagagaaagg tgaaggccat ggaggtggac atagaggaga ggcctaaaga 480
   gcttgtacgg aagccctatg tgctgaatga cctggaggca gaagccagcc ttccagaaaa 540
   gaaaggaaat actctgtctc gggacctcat tgactatgta cgctacatgg tagagaacca 600
   cggggaggac tataaggcca tggcccgtga tgagaagaat tactatcaag ataccccaaa 660
   acagattcgg agtaagatca acgtctataa acgcttttac ccagcagagt ggcaagactt 720
   cctcgattct ttgcagaaga ggaagatgga ggtggagtga ctggtttaca tcacagctgc 780
   cccaggctga ggcgtccccc ggaccagtga agctggagcc agggtgtaag gcaaggaggt 840
   gctgtgtggc tccagaggag ctggccaggt cccatggaat cagaaggtta cacacacgtg 900
   cacactcccc gctctgggga aggaactgtt ctcagaggct ccaatttata ttcatctggg 960
   ggttcacgga aaagccagaa cctgctgttt tcagggtggg tgatgtaaat atagtgtgta 1020
   cataataaag caaatatatt ttacttctct gaaaaaaaaa aaaaaaaaa           1069
<210> 272
   <211> 2379
   <212> DNA
   <213> Homo sapiens
<220>
   <223> v-myc avian myelocytomatosis viral oncogene homolog (MYC), myc-related translation/localization regulatory factor (MRTL), class E basic helix-loop-helix protein 39 (bHLHe39), transcription factor p64, proto-oncogene c-Myc, p67 myc protein
<220>
   <221> CDS
   <222> (526) ... (1890)
   <223> MYC
<400> 272
   gacccccgag ctgtgctgct cgcggccgcc accgccgggc cccggccgtc cctggctccc 60
   ctcctgcctc gagaagggca gggcttctca gaggcttggc gggaaaaaga acggagggag 120
   ggatcgcgct gagtataaaa gccggttttc ggggctttat ctaactcgct gtagtaattc 180
   cagcgagagg cagagggagc gagcgggcgg ccggctaggg tggaagagcc gggcgagcag 240
   agctgcgctg cgggcgtcct gggaagggag atccggagcg aatagggggc ttcgcctctg 300
   gcccagccct cccgctgatc ccccagccag cggtccgcaa cccttgccgc atccacgaaa 360
   ctttgcccat agcagcgggc gggcactttg cactggaact tacaacaccc gagcaaggac 420
   gcgactctcc cgacgcgggg aggctattct gcccatttgg ggacacttcc ccgccgctgc 480
   caggacccgc ttctctgaaa ggctctcctt gcagctgctt agacgctgga tttttttcgg 540
   gtagtggaaa accagcagcc tcccgcgacg atgcccctca acgttagctt caccaacagg 600
   aactatgacc tcgactacga ctcggtgcag ccgtatttct actgcgacga ggaggagaac 660
   ttctaccagc agcagcagca gagcgagctg cagcccccgg cgcccagcga ggatatctgg 720
   aagaaattcg agctgctgcc caccccgccc ctgtccccta gccgccgctc cgggctctgc 780
   tcgccctcct acgttgcggt cacacccttc tcccttcggg gagacaacga cggcggtggc 840
   gggagcttct ccacggccga ccagctggag atggtgaccg agctgctggg aggagacatg 900
   gtgaaccaga gtttcatctg cgacccggac gacgagacct tcatcaaaaa catcatcatc 960
   caggactgta tgtggagcgg cttctcggcc gccgccaagc tcgtctcaga gaagctggcc 1020
   tcctaccagg ctgcgcgcaa agacagcggc agcccgaacc ccgcccgcgg ccacagcgtc 1080
   tgctccacct ccagcttgta cctgcaggat ctgagcgccg ccgcctcaga gtgcatcgac 1140
   ccctcggtgg tcttccccta ccctctcaac gacagcagct cgcccaagtc ctgcgcctcg 1200
   caagactcca gcgccttctc tccgtcctcg gattctctgc tctcctcgac ggagtcctcc 1260
   ccgcagggca gccccgagcc cctggtgctc catgaggaga caccgcccac caccagcagc 1320
   gactctgagg aggaacaaga agatgaggaa gaaatcgatg ttgtttctgt ggaaaagagg 1380
   caggctcctg gcaaaaggtc agagtctgga tcaccttctg ctggaggcca cagcaaacct 1440
   cctcacagcc cactggtcct caagaggtgc cacgtctcca cacatcagca caactacgca 1500
   gcgcctccct ccactcggaa ggactatcct gctgccaaga gggtcaagtt ggacagtgtc 1560
   agagtcctga gacagatcag caacaaccga aaatgcacca gccccaggtc ctcggacacc 1620
   gaggagaatg tcaagaggcg aacacacaac gtcttggagc gccagaggag gaacgagcta 1680
   aaacggagct tttttgccct gcgtgaccag atcccggagt tggaaaacaa tgaaaaggcc 1740
   cccaaggtag ttatccttaa aaaagccaca gcatacatcc tgtccgtcca agcagaggag 1800
   caaaagctca tttctgaaga ggacttgttg cggaaacgac gagaacagtt gaaacacaaa 1860
   cttgaacagc tacggaactc ttgtgcgtaa ggaaaagtaa ggaaaacgat tccttctaac 1920
   agaaatgtcc tgagcaatca cctatgaact tgtttcaaat gcatgatcaa atgcaacctc 1980
   acaaccttgg ctgagtcttg agactgaaag atttagccat aatgtaaact gcctcaaatt 2040
   ggactttggg cataaaagaa cttttttatg cttaccatct tttttttttc tttaacagat 2100
   ttgtatttaa gaattgtttt taaaaaattt taagatttac acaatgtttc tctgtaaata 2160
   ttgccattaa atgtaaataa ctttaataaa acgtttatag cagttacaca gaatttcaat 2220
   cctagtatat agtacctagt attataggta ctataaaccc taattttttt tatttaagta 2280
   cattttgctt tttaaagttg atttttttct attgttttta gaaaaaataa aataactggc 2340
   aaatatatca ttgagccaaa tcttaaaaaa aaaaaaaaa                       2379
<210> 273
   <211> 2319
   <212> DNA
   <213> Homo sapiens
<220>
   <223> mitogen-activated protein kinase kinase 3 transcript variant B (MAP2K3, MKK3), stress-activated protein kinase kinase 2 (SAPKK-2, SAPKK2), MAPK/ERK kinase 3 (MEK3), dual specificity mitogen-activated protein kinase kinase 3, PRKMK3
<220>
   <221> CDS
   <222> (266) ... (1309)
   <223> MAP2K3
<400> 273
<210> 274
   <211> 2048
   <212> DNA
   <213> Homo sapiens
<220>
   <223> guanine nucleotide binding protein-like 3 (nucleolar) transcript variant 1 (GNL3), estradiol-induced nucleotide binding protein, E2-induced gene 3 protein (E2IG3), novel nucleolar protein 47 (NNP47), nucleostemin (NS), nucleolar GTP-binding protein 3
<220>
   <221> CDS
   <222> (174) ... (1823)
   <223> GNL3
<400> 274
   gcggccgcca agcgatccct gctccgcgcg acactgcgtg cccgcgcacg cagagaggcg 60
   gtgacgcact ttacggcggc agcgtaagtg cgtgacgctc gtcagtggct tcagttcaca 120
   cgtggcgcca gcggaggcag gttgatgtgt ttgtgcttcc ttctacagcc aatatgaaaa 180
   ggcctaagtt aaagaaagca agtaaacgca tgacctgcca taagcggtat aaaatccaaa 240
   aaaaggttcg agaacatcat cgaaaattaa gaaaggaggc taaaaagcgg ggtcacaaga 300
   agcctaggaa agacccagga gttccaaaca gtgctccctt taaggaggct cttcttaggg 360
   aagctgagct aaggaaacag aggcttgaag aactaaaaca gcagcagaaa cttgacaggc 420
   agaaggaact agaaaagaaa agaaaacttg aaactaatcc tgatattaag ccatcaaatg 480
   tggaacctat ggaaaaggag tttgggcttt gcaaaactga gaacaaagcc aagtcgggca 540
   aacagaattc aaagaagctg tactgccaag aacttaaaaa ggtgattgaa gcctccgatg 600
   ttgtcctaga ggtgttggat gccagagatc ctcttggttg cagatgtcct caggtagaag 660
   aggccattgt ccagagtgga cagaaaaagc tggtacttat attaaataaa tcagatctgg 720
   taccaaagga gaatttggag agctggctaa attatttgaa gaaagaattg ccaacagtgg 780
   tgttcagagc ctcaacaaaa ccaaaggata aagggaagat aaccaagcgt gtgaaggcaa 840
   agaagaatgc tgctccattc agaagtgaag tctgctttgg gaaagagggc ctttggaaac 900
   ttcttggagg ttttcaggaa acttgcagca aagccattcg ggttggagta attggtttcc 960
   caaatgtggg gaaaagcagc attatcaata gcttaaaaca agaacagatg tgtaatgttg 1020
   gtgtatccat ggggcttaca aggagcatgc aagttgtccc cttggacaaa cagatcacaa 1080
   tcatagatag tccgagcttc atcgtatctc cacttaattc ctcctctgcg cttgctctgc 1140
   gaagtccagc aagtattgaa gtagtaaaac cgatggaggc tgccagtgcc atcctttccc 1200
   aggctgatgc tcgacaggta gtactgaaat atactgtccc aggctacagg aattctctgg 1260
   aattttttac tgtgcttgct cagagaagag gtatgcacca aaaaggtgga atcccaaatg 1320
   ttgaaggtgc tgccaaactg ctgtggtctg agtggacagg tgcctcatta gcttactatt 1380
   gccatccccc tacatcttgg actcctcctc catattttaa tgagagtatt gtggtagaca 1440
   tgaaaagcgg cttcaatctg gaagaactgg aaaagaacaa tgcacagagc ataagagcca 1500
   tcaagggccc tcatttggcc aatagcatcc ttttccagtc ttccggtctg acaaatggaa 1560
   taatagaaga aaaggacata catgaagaat tgccaaaacg gaaagaaagg aagcaggagg 1620
   agagggagga tgacaaagac agtgaccagg aaactgttga tgaagaagtt gatgaaaaca 1680
   gctcaggcat gtttgctgca gaagagacag gggaggcact gtctgaggag actacagcag 1740
   gtgaacagtc tacaaggtct tttatcttgg ataaaatcat tgaagaggat gatgcttatg 1800
   acttcagtac agattatgtg taacagaaca atggcttttt atgatttttt ttttaacatt 1860
   ttaagcagac tgctaaactg ttctctgtat aagttatggt atgcatgagc tgtgtaaatt 1920
   ttgtgaatat gtattatatt aaaaccaggc aacttggaat ccctaaattc tgtaaaaaga 1980
   caattcatct cattgtgagt ggaagtagtt atctggaata aaaaaagaag atacctattg 2040
   aaaaaaaa                                                           2048
<210> 275
   <211> 1724
   <212> DNA
   <213> Homo sapiens
<220>
   <223> RRS1 ribosome biogenesis regulator homolog (S. cerevisiae) (RRS1), homolog of yeast ribosome biogenesis regulatory protein RRS1
<220>
   <221> CDS
   <222> (105) ... (1202)
   <223> RRS1
<400> 275
   gcatcccggc atctgcacgt ggttatgctg ccggagtttg ggccgccact gtaggaaaag 60
   taacttcagc tgcagcccca aagcgagtga gccgagccgg agccatggag ggccagagcg 120
   tggaggagct gctcgcaaag gcagagcagg acgaggcaga gaagttgcaa cgcatcacgg 180
   tgcacaagga gctggagctg cagtttgacc tgggcaacct gctggcgtcg gaccggaacc 240
   ccccgaccgg gctgcggtgc gccggaccca cgccggaggc cgagctacag gccctggcgc 300
   gggacaacac gcaactgctc atcaaccagc tgtggcagct gcccacggag cgcgtggaag 360
   aggcgatagt ggcgcggctg ccggagccca ccacacgcct gccgcgagag aagcctctgc 420
   cccgaccgcg gccacttaca cgctggcagc agttcgcgcg cctcaagggc atccgtccca 480
   agaagaagac caacctggtg tgggacgagg tgagtggcca gtggcggcgg cgctggggct 540
   accagcgcgc ccgggacgac accaaagaat ggctgattga ggtgcccggc aatgccgacc 600
   ccttggagga ccagttcgcc aagcggattc aggccaagaa ggaaagggtg gccaagaacg 660
   agctgaaccg gctgcgtaac ctggcccgcg cgcacaagat gcagctgccc agcgcggccg 720
   gcttgcaccc taccggacac cagagtaagg aggagctggg ccgcgccatg caagtggcca 780
   aggtctccac cgcctctgtg gggcgctttc aggagcgcct ccccaaggag aaggtgcccc 840
   ggggctccgg caagaaaagg aagtttcaac cccttttcgg ggactttgca gccgagaaaa 900
   agaaccagtt ggagctgctt cgtgtcatga acagcaagaa gcctcagctg gatgtgacta 960
   gggccaccaa taagcagatg agggaggagg accaggagga ggccgccaag aggaggaaaa 1020
   tgagccagaa gggcaagaga aagggaggcc ggcaggggcc tgggggcaag aggaaagggg 1080
   gcccgcccag ccagggaggg aagaggaaag ggggcttggg aggcaagatg aattctgggc 1140
   cgcctggctt gggtggcaag agaaaaggag gacagcgccc aggaggaaag aggaggaagt 1200
   aatagtttct aactgtcgga cccgtctgta aaccaaggac tatgaatact aaatgttaag 1260
   ttctaggcaa ttatacgggg actcagaagg acctggccgc tgccttcatt gagtttaaag 1320
   ggacaggatt gcccttccgt caagaaagta tgtaagtgtt ggactgcaca aattaatgtt 1380
   tttcccacaa ccgagacttt ggagattaag aacttatttg aggatttaag aattagggaa 1440
   ataatttggt ggaaaccggg aatgagttct attcttaaac agcctttttt tttcttttta 1500
   atgttggata tacggcgagg tagagttggc catatttcag agacttagat tgacgtatat 1560
   gtttctgcat tatttttaca acaagtttgt gtatcagagc gggagtgcgg gggagggaaa 1620
   gaaaacaaac agtttcagaa ttgaataggc aagtgactgt tttaaagatt aagtaataaa 1680
   gatgtcttat ctagtgtgac ttttaaaaaa aaaaaaaaaa aaaa                  1724
<210> 276
   <211> 1668
   <212> DNA
   <213> Homo sapiens
<220>
   <223> FOS-like antigen 1 (FOSL1), fos-related antigen 1 (FRA, fra-1, FRA1)
<220>
   <221> CDS
   <222> (188) ... (1003)
   <223> FOSL1
<400> 276
   acgggccaag gcggcgcgtc tcgggggtgg agcctggagg tgaccgcgcc gctgcaacgc 60
   ccccaccccc cgcggtcgca gtggttcagc ccgagaactt ttcattcata aaaagaaaag 120
   actccgcacg gcgcgggtga gtcagaaccc agcagccgtg taccccgcag agccgccagc 180
   cccgggcatg ttccgagact tcggggaacc cggcccgagc tccgggaacg gcggcgggta 240
   cggcggcccc gcgcagcccc cggccgcagc gcaggcagcc cagcagaagt tccacctggt 300
   gccaagcatc aacaccatga gtggcagtca ggagctgcag tggatggtac agcctcattt 360
   cctggggccc agcagttacc ccaggcctct gacctaccct cagtacagcc ccccacaacc 420
   ccggccagga gtcatccggg ccctggggcc gcctccaggg gtacgtcgaa ggccttgtga 480
   acagatcagc ccggaggaag aggagcgccg ccgagtaagg cgcgagcgga acaagctggc 540
   tgcggccaag tgcaggaacc ggaggaagga actgaccgac ttcctgcagg cggagactga 600
   caaactggaa gatgagaaat ctgggctgca gcgagagatt gaggagctgc agaagcagaa 660
   ggagcgccta gagctggtgc tggaagccca ccgacccatc tgcaaaatcc cggaaggagc 720
   caaggagggg gacacaggca gtaccagtgg caccagcagc ccaccagccc cctgccgccc 780
   tgtaccttgt atctcccttt ccccagggcc tgtgcttgaa cctgaggcac tgcacacccc 840
   cacactcatg accacaccct ccctaactcc tttcaccccc agcctggtct tcacctaccc 900
   cagcactcct gagccttgtg cctcagctca tcgcaagagt agcagcagca gcggagaccc 960
   atcctctgac ccccttggct ctccaaccct cctcgctttg tgaggcgcct gagccctact 1020
   ccctgcagat gccaccctag ccaatgtctc ctccccttcc cccaccggtc cagctggcct 1080
   ggacagtatc ccacatccaa ctccagcaac ttcttctcca tccctctaat gagactgacc 1140
   atattgtgct tcacagtaga gccagcttgg ggccaccaaa gctgcccact gtttctcttg 1200
   agctggcctc tctagcacaa tttgcactaa atcagagaca aaatatttcc catttgtgcc 1260
   agaggaatcc tggcagccca gagactttgt agatccttag aggtcctctg gagccctaac 1320
   cccttccaga tcactgccac actctccatc accctcttcc tgtgatccac ccaaccctat 1380
   ctcctgacag aaggtgccac tttacccacc tagaacacta actcaccagc cccactgcca 1440
   gcagcagcag gtgattggac caggccattc tgccgccccc tcctgaaccg cacagctcag 1500
   gaggcgccct tggcttctgt gatgagctga tctgcggatc tcagctttga gaagccttca 1560
   gctccaggga atccaagcct ccacagcgag ggcagctgct atttattttc ctaaagagag 1620
   tatttttata caaacctacc aaaatggaat aaaaggcttg aagctgtg             1668
<210> 277
   <211> 4890
   <212> DNA
   <213> Homo sapiens
<220>
   <223> TSR1, 20S rRNA accumulation, homolog (S. cerevisiae) (TSR1), pre-rRNA-processing protein TSR1 homolog
<220>
   <221> CDS
   <222> (958) ... (3372)
   <223> TSR1
<400> 277
   cgccacgggg gtcgctgtca tggcaacgca cccgctcccg catggagggc gcgaagccgc 60
   attgtgagcg ctggccctgg agcccgcggc cccgcggctg agagcgagcc tcggtcccgc 120
   tgaagctccc ctgacttctg cctgctccct ctcccgacgg gacggaaagg gccggggctc 180
   gggcatgggg tcccctgcgg tccgagcctg cccacggcga agccggtgca gcctggcccc 240
   gtgacccctc tgaatccccg tccggccgtc gccgtgcagt tgtcaagttt ctgggaagat 300
   tagtgggagc gcagccgccc gagcccgacg gccggagggt cgcggccagt cccccttgcc 360
   gggtcgccac gtcccacaag gtccttcccg gccgctccgt gggaggcggg gcgcaggttg 420
   gagtaacagg gacgggcttg agctggtacg gaaccgggca aggtgagaga agtgtcttcc 480
   acgcgattcc caagcctcgc tagggaatcg agggagaaga aaggttgctc agagtgataa 540
   agaacatgcc gttgcaaacg gaagagcaac gtgcccgaat ctaggtgaca ggaagggctt 600
   aggccttttc ggcacgaatg gagccttcca gtcaacatgc ttctacgtga tgcagcttta 660
   tagcgcaaaa aaatgacaat gcaaggggta caaacagccc tgctagacca acagcccgag 720
   cctcgacagt ctacatccgg cgccttcaag cccccaacca cacactgcga agtgggaaaa 780
   gttcccagcg cggcttggcg aacccgctaa cacggcgctc tcacgagctg ctggaaaaga 840
   ccttcgcgag agggcgcagg gagtcccgct ccctggaggg cccgccccct tggaggggac 900
   cggaagcgct tgtcctggcc cggaagcagt gctgactccg tacacgcgcg ctgcggcatg 960
   gcggcccacc gccccggccc gctcaagcag cagaataaag ctcataaagg cggacggcat 1020
   cggggtcggg gatctgcaca gcgggacggc aagggccgtc tggcactgaa aaccctaagc 1080
   aagaaggtga gaaaagaact cagcagagtc gaccagaggc atcgcgccag ccagctccga 1140
   aagcagaaga aggaggcggt tctggcagag aagagacagc tgggtggcaa ggatggccct 1200
   cctcatcagg tactggtggt gcccctgcac agcagaattt ccctgccaga ggccatgcag 1260
   ctgcttcaag atagggacac tggaacagta cacttgaatg aattgggaaa cacccagaac 1320
   tttatgctgc tgtgcccccg cttgaaacat cggtggtttt tcacctcagc aaggccaggg 1380
   gatctgcacg ttgtgttaga catggctaaa gtagctgata ccatcctgtt cctccttgat 1440
   ccactagaag gctgggacag caccggtgat tactgtcttt cctgcctctt tgctcagggc 1500
   cttccgacct atacactagc tgtccagggg atttctggcc tcccactgaa gaaacaaata 1560
   gataccagga agaagctaag taaagcagtg gagaagcgct ttccgcatga caaactcctc 1620
   ttgttagaca ctcaacagga ggcagggatg ctgcttaggc agttggctaa ccagaagcaa 1680
   cagcatcttg cttttcgaga tcggcgggcc tacctatttg cccatgctgt tgattttgtt 1740
   cctagtgaag agaataactt ggtgggcacc ttgaaaattt caggctatgt tcgagggcag 1800
   actctgaatg tcaataggtt gctgcatatc gttggatatg gtgatttcca gatgaaacag 1860
   atagatgccc ccggagaccc tttcccttta aatcctagag gaattaaacc ccaaaaggac 1920
   ccagacatgg caatggagat ttgtgctacg gatgctgtag atgatatgga agaaggtctt 1980
   aaagtcctaa tgaaggcaga ccctggtaga caggaatcct tgcaagcaga ggttatccca 2040
   gatccaatgg agggagagca aacctggccc actgaggagg agctgagcga ggcaaaggat 2100
   ttcttgaagg aaagttctaa ggtggtaaag aaggtcccca aaggaacatc cagttaccaa 2160
   gctgaatgga ttttggatgg tggcagccaa agtggtgggg aaggagatga atatgaatat 2220
   gatgatatgg aacatgagga ttttatggag gaggaatctc aggatgagag tagtgaagaa 2280
   gaggaagaat atgaaactat gactattggg gagtctgtgc atgatgatct gtatgataag 2340
   aaagtagatg aagaagctga ggcaaaaatg ttggagaaat ataaacaaga aagactggaa 2400
   gagatgtttc cagatgaagt ggacacgccc cgtgatgtgg ctgctcgaat tcgatttcag 2460
   aaatacagag gccttaagag cttccggaca tctccatggg atcctaagga aaaccttcct 2520
   caagattatg ctcgaatatt tcagtttcag aactttacta acactaggaa aagcatcttt 2580
   aaagaggttg aagaaaaaga ggttgaagga gctgaggttg gctggtatgt cacacttcat 2640
   gtctctgaag tccccgtctc agtggtcgag tgcttcaggc aaggaacacc cttgattgca 2700
   ttttctttac tacctcatga acagaagatg tcagtattga atatggtggt gaggcgtgac 2760
   cctggcaaca ctgaacctgt gaaagccaag gaagagctca tatttcactg tggattcagg 2820
   cgcttccgag cctcaccttt attctctcag cacactgcag cggacaaaca taaattgcag 2880
   agattcctga ctgctgacat ggccctggtg gcgacagtct atgcgccaat cacttttcct 2940
   cctgcatctg tgctgctttt caagcaaaaa agcaatggaa tgcacagcct cattgctaca 3000
   ggccatctta tgtcagtaga tccagacaga atggtcatca agagagttgt tctgagtggt 3060
   catcctttca aaatttttac taagatggca gtagtacgtt acatgttctt caacagagag 3120
   gatgtgctgt ggtttaaacc agtggaactg agaacgaagt ggggccggag aggacatatc 3180
   aaggaacctt taggtaccca tggccacatg aaatgcagct ttgatgggaa gctaaaatct 3240
   caagacacag tactgatgaa cctgtataaa cgagtcttcc ccaaatggac ttatgatcca 3300
   tatgtaccag aaccagtacc ctggctgaaa agtgagattt cttcaacagt gcctcaaggg 3360
   ggcatggagt aatggattca aagagattct gtcttaccgg tgccagtcag tactccaggg 3420
   atgggaggca caagttgtga ttgggcaaag tttattttct atgtcagcct gtcagtccac 3480
   tgccccattt tgcaagactt ttttttagcc ttgacaaaat gtctcagtta agtataaaag 3540
   tttttccact acttagtcca aaaaaaacta ttaaatctta atgaaatagc cactctccat 3600
   taagaatctg ataactaaga gttgacaata ctaggaaaca aaatgtcttc agtatctaaa 3660
   gacactgaat tcccaccatt tccttttctg taaattaaac agaaacagac tgataagaag 3720
   ctggcctttc agcctgcttc acccaagtta tggaggaggt taagtctaca tttccaccac 3780
   tgagcacaat acaaatgttc tttacttctg gggaaacagt ttgaaaatgt tgagacagca 3840
   cagcagccac tccaacacca gctgtaggtt caatgagtag tttcatcctc tcccacacca 3900
   gctgggttgc acactgttga tatggaaggg agtaggaatt agtgaaaggg gagtctagtt 3960
   ggtttaccta taaagttcag atgagaatac gtgtccagtt tgaattttct tatcactgtg 4020
   tacacacgct tcccaaagct tttctttaat tctgactctg cttctgtcaa gagggttgtc 4080
   attaatacat ctagggtacc ctcccatgtc atgaagttac ctagtcaggt tcccgtgggt 4140
   cacttgctac tcctaaggga agtttcttct gcctaagaag ttacaccatg ctttgctatt 4200
   tcttttcttg ctggagcctc accttaattt catcctctgt gacagtgaag atatcatcca 4260
   caaggtccct gataataggc caggtgttca agccaatgct ggatttgaca ccatctgcta 4320
   tggtttctgg aggataaaga ttgggcatca gtttcccctt cagcttggac tggtagcagt 4380
   catctgcatt tgagggttca gcagcatata ccttcacact aggtttcaga gcctgggaag 4440
   aggaatatta agacatctta acaacaaacc acaacattgc ctgcatgtct aaaagaaaat 4500
   atgggcctgg tgtggtggct cacacctgta atcccaacac tttgggagcc cgaggcgggt 4560
   ggatcagctg aggtcaggag ttcaagacca gcctggccaa cctggtgaaa tgcagtgtca 4620
   actaaaaata ggaaaatcag ccaggcgtgg tggcagaccc ctgtaatccc agctactcaa 4680
   gaggctgagg taggagaatc acttgaactt gggaggggta ggttgcagtg agctgagatc 4740
   acaccaaaat ggggtggggc gcagtggctc acgcctgtaa tcccagcact ttgggaggcc 4800
   gaggtgggcg gatcacgagg tagggagatc aagaccatcc tggctaacac ggtgaaaccc 4860
   cgtctctact aaaaatacaa aaaaaaaaaa 4890
<210> 278
   <211> 4457
   <212> DNA
   <213> Homo sapiens
<220>
   <223> sprouty-related, EVH1 domain containing 2 transcript variant 1 (SPRED2, Spred-2), sprouty protein with EVH-1 domain 2, related sequence, sprouty-related, EVH1 domain-containing protein 2
<220>
   <221> CDS
   <222> (536) ... (1792)
   <223> SPRED2
<400> 278
   atccaatatg gagtaaatcg gtggagtcga gagatggggc tcggacgggg cgccctgcac 60
   cgcctcccag gcgcacctcc cccggccgcc gaccgctccc cggaaccgtg attggcccgg 120
   ccccccgggg gcgaccccgg actgagcccc cccacccgcg gccggcgctc tcctcctcct 180
   cgcagcagtc cctgccgctc cgaggcgcgc ttgtttttct cctgctctcc cccaccgccg 240
   tccccttccc aaatctcgcc ccctctgccc gctcccgagc ccccggagcc tccgcccgcc 300
   tctccccacg gcgctgcggc gttcactccc cgcgcagcct gcctttgcac cccttccccc 360
   aaaccctatc ccgcgccctg cttccccttc tgctgcggcg ccctcttcat ctctagccgc 420
   cccccctccc caaatcaggc gatctccgga gatgtgaaga aggggggcga gcggacagga 480
   agatgaaggg agcaaagctg cccgccgcgg gacaggcgtc taggtgaaca agaaaatgac 540
   cgaagaaaca cacccagacg atgacagcta tattgtgcgt gtcaaggctg tggttatgac 600
   cagagatgac tccagcgggg gatggttccc acaggaagga ggcgggatca gtcgcgtcgg 660
   ggtctgtaag gtcatgcacc ccgaaggcaa tggacgaagc ggctttctca tccatggtga 720
   acgacagaaa gacaaactgg tggtattgga atgctatgta agaaaggact tggtctacac 780
   caaagccaat ccaacgtttc atcactggaa ggtcgataat aggaagtttg gacttacttt 840
   ccaaagccct gctgatgccc gagcctttga caggggagta aggaaagcaa tcgaagacct 900
   tatagaaggt tcaacaacgt catcttccac catccataat gaagctgagc ttggcgatga 960
   tgacgttttt acaacagcta cagacagttc ttctaattcc tctcagaaga gagagcaacc 1020
   tactcggaca atctcctctc ccacatcctg tgagcaccgg aggatttata ccctgggcca 1080
   cctccacgac tcatacccca cagaccacta tcacctcgat cagccgatgc caaggcccta 1140
   ccgccaggtg agcttcccgg acgacgacga ggagatcgtg cgcatcaacc cccgggagaa 1200
   gatctggatg acggggtacg aggattaccg gcacgcaccc gtcaggggca agtacccgga 1260
   cccctcggag gacgcggact cctcctacgt gcgcttcgcc aagggcgagg tccccaagca 1320
   tgactacaac tacccctacg tggactcctc agactttggc ctaggcgagg accccaaagg 1380
   ccgcgggggc agcgtgatca agacgcagcc ctcccggggc aagtcgcggc ggcggaagga 1440
   ggacggagag cgctcgcggt gcgtgtactg cagggacatg ttcaaccacg aggagaaccg 1500
   ccggggccac tgccaggacg cgcccgactc cgtgagaact tgcatccgcc gggtgagctg 1560
   catgtggtgc gcggacagca tgctctatca ctgtatgtcg gaccccgagg gagactatac 1620
   agacccttgc tcgtgcgata ctagcgacga gaagttttgc ctccggtgga tggctcttat 1680
   tgccttgtct ttcctggccc cctgtatgtg ctgttacctg ccccttcggg cctgctacca 1740
   ctgcggagtg atgtgcaggt gctgtggcgg gaagcacaaa gcggccgcgt gactcagttt 1800
   ccctcccttc tccctccatc cgcagccaca ggggaactcg tctcttacat actctcatct 1860
   tctcccccgc tcccttccac tccaaggagc gaggagggca agcggcctcc cagctccctg 1920
   gtacctcgag gcaccattcc agccagggac gctgccgggt agactctcca ctccccctgc 1980
   cgcccacact gcagcagcca catccataca cacacgctcg cacagtgttc tgaggaagga 2040
   accttcgcca cagactcctg tactattaac aatctgtaac caagctaact gtctcatcca 2100
   tgtgttgatt tcctgtttcc tcctcccccg cctcttccag ttcaaaggag tctgcaattg 2160
   gaactgctga ttttcggtgg gttttgtagt tgatttttcc aagagcgtcg aagactctct 2220
   ttctcttggt tcaccttgcc tgtcgctagc aagcatctgg ttcagcggaa atgggatgtg 2280
   agaatgatga aacccgacag aagtatctca gcctgcagtc agttattatg tataggaggt 2340
   gagctagtta acaaacttgt accacaaaac aatatcgctt taacttttct aaagccaaat 2400
   ttcccatgta agctgcagtt tctatcttta gcccatcatc attttctgcc cccccaaaat 2460
   ctgttgaaat gattcactga tgcaaaacat tcacccgtaa tagactgaga attgagcctt 2520
   aacttcagat taacttgtga gaatcaggaa aattccttca actgcattgc atcctcttgg 2580
   accagggcta gaatggggat ttcaggtttc tatgagcctc cccattaccc ctaaagtaga 2640
   acatttttta aattgtgttg ccaccacttc taaaaattta gcaatattag aataggatac 2700
   tagtgaagta agaaattttg cttgttgttt tgcaaaccaa atagtttcct caaacacaaa 2760
   tcagtgttcc cacgaacaag ttccagttga gaaacactaa ggttatggtg aataaaacca 2820
   tagggagctt cttcccccca acccctggct attttatatt aatggggaga ggggattttt 2880
   aaatgtcata aatttgaaga gtggtgggtt gcattttctt catgggttta tgttttcgtt 2940
   tcattttgga ctcaatttca catcaccaaa ttcctcattt atacttgggg aaaaaacaag 3000
   gccatatgta aaaacccttc caatgcctaa gtgtctttct cctgcaactc caaacccaga 3060
   ctcgccactt tgggtgcaca ggtggttagg tcagccaact ggttctgcct gtcgccttgc 3120
   cacggaggag gcttctaatt agctggaaaa gagtattttt ctaatacgtt gcaaggatta 3180
   gccaaatctt cttattgaag aaagaagaaa agtgaagagt ggttacctat acctagcata 3240
   gtacaatcag aacctcgtgg agaccaccgg ggacaggctt gcggacgccg gctgttcttc 3300
   ccgccacgat ctttctgtgg tagcggccag cagaagacat ggcctgctcc ccactccctt 3360
   tccccactcc ttctttattg cacacaggac accagtcttc aaggaaaggg acttttttcc 3420
   agtctgccaa tcatattggg aaagtgctag ctgtgctcac cttcatgggg ctgtttccag 3480
   ctcgtccaca agctcatcga ttttctttag tagattaccg gtgtaaatac ccagtgtgct 3540
   tatgagtcag ttagtagacg tcttcattca ttggagtaac tggtttaggc tttccagttt 3600
   ggaaaaggag cagagagctg tccatctgga ttgatggaag aaaagagaac ctcatccatg 3660
   cctggagaac atctagaaaa ccttcagcca gcctccagtg ctgtcgagag accaccttcc 3720
   cccgacccgg aggcacttcc ttggggtctt tctctagggt ctcttctcta caaagcacaa 3780
   cactaatgtt cgtttcctta gacctcagtt caagtgcccc tatttatttc aataagaacg 3840
   cacatatccc agctgttttt tgtttgtcac ctctatttag ttgttacctg tttctctctt 3900
   ctttcacccc ttgtcctttt ccaccctttt aagagttacg ctagcagatc ttactccacg 3960
   tatacttttt ggtttgtgaa ggcatcggtt aagggcacaa agacagccat ggggacattt 4020
   atgtaaatac gtctctaatt gccacactgc agctgaacag tgtgtagtat tttcccagtc 4080
   agctttgcca tactgacgtc aatcatttga gagaaattat tcagatttta tttttgtatc 4140
   tgtggtaaca aaacattaac caaaagattt tctgtccaga agcctccccg accccccaag 4200
   ctatttgctc acattaacaa attaaagtgc ctgaagcata attcattctt tacctgtata 4260
   ctaaaaaccc tgttgtattg atttttttat aataagcctt tttacctctg tgtaaaaaat 4320
   atatatacaa gtgtatgatg tacattttag ttcttaactt ttttttatgg tttctaatat 4380
   gtatgaccaa tgtagccatt gctttaaaat gtaccgtgta aatataaaca catcctatca 4440
   gaaaaaaaaa aaaaaaa                                                4457
<210> 279
   <211> 4150
   <212> DNA
   <213> Homo sapiens
<220>
   <223> high mobility group AT-hook 2 transcript variant 1 (HMGA2), high-mobility group protein HMGI-C, high-mobility group (nonhistone chromosomal) protein isoform I-C (HMGI-C, HMGIC), BABL, LIPO, STQTL9
<220>
   <221> CDS
   <222> (812) ... (1141)
   <223> HMGA2
<400> 279
   cttgaatctt ggggcaggaa ctcagaaaac ttccagcccg ggcagcgcgc gcttggtgca 60
   agactcagga gctagcagcc cgtccccctc cgactctccg gtgccgccgc tgcctgctcc 120
   cgccacccta ggaggcgcgg tgccacccac tactctgtcc tctgcctgtg ctccgtgccc 180
   gaccctatcc cggcggagtc tccccatcct cctttgcttt ccgactgccc aaggcacttt 240
   caatctcaat ctcttctctc tctctctctc tctctctctc tctctctctc tctctctctc 300
   tctctctctc gcagggtggg gggaagagga ggaggaattc tttccccgcc taacatttca 360
   agggacacaa ttcactccaa gtctcttccc tttccaagcc gcttccgaag tgctcccggt 420
   gcccgcaact cctgatccca acccgcgaga ggagcctctg cgacctcaaa gcctctcttc 480
   cttctccctc gcttccctcc tcctcttgct acctccacct ccaccgccac ctccacctcc 540
   ggcacccacc caccgccgcc gccgccaccg gcagcgcctc ctcctctcct cctcctcctc 600
   ccctcttctc tttttggcag ccgctggacg tccggtgttg atggtggcag cggcggcagc 660
   ctaagcaaca gcagccctcg cagcccgcca gctcgcgctc gccccgccgg cgtccccagc 720
   cctatcacct catctcccga aaggtgctgg gcagctccgg ggcggtcgag gcgaagcggc 780
   tgcagcggcg gtagcggcgg cgggaggcag gatgagcgca cgcggtgagg gcgcggggca 840
   gccgtccact tcagcccagg gacaacctgc cgccccagcg cctcagaaga gaggacgcgg 900
   ccgccccagg aagcagcagc aagaaccaac cggtgagccc tctcctaaga gacccagggg 960
   aagacccaaa ggcagcaaaa acaagagtcc ctctaaagca gctcaaaaga aagcagaagc 1020
   cactggagaa aaacggccaa gaggcagacc taggaaatgg ccacaacaag ttgttcagaa 1080
   gaagcctgct caggaggaaa ctgaagagac atcctcacaa gagtctgccg aagaggacta 1140
   gggggcgcca acgttcgatt tctacctcag cagcagttgg atcttttgaa gggagaagac 1200
   actgcagtga ccacttattc tgtattgcca tggtctttcc actttcatct ggggtggggt 1260
   ggggtggggt gggggagggg ggggtggggt ggggagaaat cacataacct taaaaaggac 1320
   tatattaatc accttctttg taatcccttc acagtcccag gtttagtgaa aaactgctgt 1380
   aaacacaggg gacacagctt aacaatgcaa cttttaatta ctgttttctt ttttcttaac 1440
   ctactaatag tttgttgatc tgataagcaa gagtgggcgg gtgagaaaaa ccgaattggg 1500
   tttagtcaat cactgcactg catgcaaaca agaaacgtgt cacacttgtg acgtcgggca 1560
   ttcatatagg aagaacgcgg tgtgtaacac tgtgtacacc tcaaatacca ccccaaccca 1620
   ctccctgtag tgaatcctct gtttagaaca ccaaagataa ggactagata ctactttctc 1680
   tttttcgtat aatcttgtag acacttactt gatgattttt aactttttat ttctaaatga 1740
   gacgaaatgc tgatgtatcc tttcattcag ctaacaaact agaaaaggtt atgttcattt 1800
   ttcaaaaagg gaagtaagca aacaaatatt gccaactctt ctatttatgg atatcacaca 1860
   tatcagcagg agtaataaat ttactcacag cacttgtttt caggacaaca cttcattttc 1920
   aggaaatcta cttcctacag agccaaaatg ccatttagca ataaataaca cttgtcagcc 1980
   tcagagcatt taaggaaact agacaagtaa aattatcctc tttgtaattt aatgaaaagg 2040
   tacaacagaa taatgcatga tgaactcacc taattatgag gtgggaggag cgaaatctaa 2100
   atttcttttg ctatagttat acatcaattt aaaaagcaaa aaaaaaaaag gggggggcaa 2160
   tctctctctg tgtctttctc tctctctctt cctctccctc tctcttttca ttgtgtatca 2220
   gtttccatga aagacctgaa taccacttac ctcaaattaa gcatatgtgt tacttcaagt 2280
   aatacgtttt gacataagat ggttgaccaa ggtgcttttc ttcggcttga gttcaccatc 2340
   tcttcattca aactgcactt ttagccagag atgcaatata tccccactac tcaatactac 2400
   ctctgaatgt tacaacgaat ttacagtcta gtacttatta catgctgcta tacacaagca 2460
   atgcaagaaa aaaacttact gggtaggtga ttctaatcat ctgcagttct ttttgtacac 2520
   ttaattacag ttaaagaagc aatctcctta ctgtgtttca gcatgactat gtatttttct 2580
   atgttttttt aattaaaaat ttttaaaata cttgtttcag cttctctgct agatttctac 2640
   attaacttga aaatttttta accaagtcgc tcctaggttc ttaaggataa ttttcctcaa 2700
   tcacactaca catcacacaa gatttgactg taatatttaa atattaccct ccaagtctgt 2760
   acctcaaatg aattctttaa ggagatggac taattgactt gcaaagacct acctccagac 2820
   ttcaaaagga atgaacttgt tacttgcagc attcatttgt tttttcaatg tttgaaatag 2880
   ttcaaactgc agctaaccct agtcaaaact atttttgtaa aagacatttg atagaaagga 2940
   acacgttttt acatactttt gcaaaataag taaataataa ataaaataaa agccaacctt 3000
   caaagaaact tgaagctttg taggtgagat gcaacaagcc ctgcttttgc ataatgcaat 3060
   caaaaatatg tgtttttaag attagttgaa tataagaaaa tgcttgacaa atattttcat 3120
   gtattttaca caaatgtgat ttttgtaata tgtctcaacc agatttattt taaacgcttc 3180
   ttatgtagag tttttatgcc tttctctcct agtgagtgtg ctgacttttt aacatggtat 3240
   tatcaactgg gccaggaggt agtttctcat gacggctttt gtcagtatgg cttttagtac 3300
   tgaagccaaa tgaaactcaa aaccatctct cttccagctg cttcagggag gtagtttcaa 3360
   aggccacata cctctctgag actggcagat cgctcactgt tgtgaatcac caaaggagct 3420
   atggagagaa ttaaaactca acattactgt taactgtgcg ttaaataagc aaataaacag 3480
   tggctcataa aaataaaagt cgcattccat atctttggat gggcctttta gaaacctcat 3540
   tggccagctc ataaaatgga agcaattgct catgttggcc aaacatggtg caccgagtga 3600
   tttccatctc tggtaaagtt acacttttat ttcctgtatg ttgtacaatc aaaacacact 3660
   actacctctt aagtcccagt atacctcatt tttcatactg aaaaaaaaag cttgtggcca 3720
   atggaacagt aagaacatca taaaattttt atatatatag tttatttttg tgggagataa 3780
   attttatagg actgttcttt gctgttgttg gtcgcagcta cataagactg gacatttaac 3840
   ttttctacca tttctgcaag ttaggtatgt ttgcaggaga aaagtatcaa gacgtttaac 3900
   tgcagttgac tttctccctg ttcctttgag tgtcttctaa ctttattctt tgttctttat 3960
   gtagaattgc tgtctatgat tgtactttga atcgcttgct tgttgaaaat atttctctag 4020
   tgtattatca ctgtctgttc tgcacaataa acataacagc ctctgtgatc cccatgtgtt 4080
   ttgattcctg ctctttgtta cagttccatt aaatgagtaa taaagtttgg tcaaaacaga 4140
   aaaaaaaaaa                                                        4150
<210> 280
   <211> 2369
   <212> DNA
   <213> Homo sapiens
<220>
   <223> polo-like kinase 3 (PLK3), proliferation-related kinase (PRK), cytokine-inducible kinase (CNK), FGF-inducible kinase, cytokine-inducible serine/threonine-protein kinase
<220>
   <221> CDS
   <222> (101) ... (2041)
   <223> PLK3
<400> 280
   cctgggcgcc agcgcagcgt agcaaatcca ggcagcgcca cgcgcggccg gggccgggcg 60
   gaaccgagaa gccgggaccg cgctgcgacg cgccggccgc atggagcctg ccgccggttt 120
   cctgtctccg cgccccttcc agcgtgcggc cgccgcgccc gctcccccgg ccgggcccgg 180
   gccgcctccg agtgccttgc gcggacctga gctggagatg ctggccgggc taccgacgtc 240
   agaccccggg cgcctcatca cggacccgcg cagcggccgc acctacctca aaggccgctt 300
   gttgggcaag gggggcttcg cccgctgcta cgaggccact gacacagaga ctggcagcgc 360
   ctacgctgtc aaagtcatcc cgcagagccg cgtcgccaag ccgcatcagc gcgagaagat 420
   cctaaatgag attgagctgc accgagacct gcagcaccgc cacatcgtgc gtttttcgca 480
   ccactttgag gacgctgaca acatctacat tttcttggag ctctgcagcc gaaagtccct 540
   ggcccacatc tggaaggccc ggcacaccct gttggagcca gaagtgcgct actacctgcg 600
   gcagatcctt tctggcctca agtacttgca ccagcgcggc atcttgcacc gggacctcaa 660
   gttgggaaat tttttcatca ctgagaacat ggaactgaag gtgggggatt ttgggctggc 720
   agcccggttg gagcctccgg agcagaggaa gaagaccatc tgtggcaccc ccaactatgt 780
   ggctccagaa gtgctgctga gacagggcca cggccctgag gcggatgtat ggtcactggg 840
   ctgtgtcatg tacacgctgc tctgcgggag ccctcccttt gagacggctg acctgaagga 900
   gacgtaccgc tgcatcaagc aggttcacta cacgctgcct gccagcctct cactgcctgc 960
   ccggcagctc ctggccgcca tccttcgggc ctcaccccga gaccgcccct ctattgacca 1020
   gatcctgcgc catgacttct ttaccaaggg ctacaccccc gatcgactcc ctatcagcag 1080
   ctgcgtgaca gtcccagacc tgacaccccc caacccagct aggagtctgt ttgccaaagt 1140
   taccaagagc ctctttggca gaaagaagaa gagtaagaat catgcccagg agagggatga 1200
   ggtctccggt ttggtgagcg gcctcatgcg cacatccgtt ggccatcagg atgccaggcc 1260
   agaggctcca gcagcttctg gcccagcccc tgtcagcctg gtagagacag cacctgaaga 1320
   cagctcaccc cgtgggacac tggcaagcag tggagatgga tttgaagaag gtctgactgt 1380
   ggccacagta gtggagtcag ccctttgtgc tctgagaaat tgtatagcct tcatgccccc 1440
   agcggaacag aacccggccc ccctggccca gccagagcct ctggtgtggg tcagcaagtg 1500
   ggttgactac tccaataagt tcggctttgg gtatcaactg tccagccgcc gtgtggctgt 1560
   gctcttcaac gatggcacac atatggccct gtcggccaac agaaagactg tgcactacaa 1620
   tcccaccagc acaaagcact tctccttctc cgtgggtgct gtgccccggg ccctgcagcc 1680
   tcagctgggt atcctgcggt acttcgcctc ctacatggag cagcacctca tgaagggtgg 1740
   agatctgccc agtgtggaag aggtagaggt acctgctccg cccttgctgc tgcagtgggt 1800
   caagacggat caggctctcc tcatgctgtt tagtgatggc actgtccagg tgaacttcta 1860
   cggggaccac accaagctga ttctcagtgg ctgggagccc ctccttgtga cttttgtggc 1920
   ccgaaatcgt agtgcttgta cttacctcgc ttcccacctt cggcagctgg gctgctctcc 1980
   agacctgcgg cagcgactcc gctatgctct gcgcctgctc cgggaccgca gcccagccta 2040
   ggacccaagc cctgaggcct gaggcctgtg cctgtcaggc tctggccctt gcctttgtgg 2100
   ccttccccct tcctttggtg cctcactggg ggctttgggc cgaatccccc agggaatcag 2160
   ggaccagctt tactggagtt gggggcggct tgtcttcgct ggctcctacc ccatctccaa 2220
   gataagcctg agccttagct cccagctagg gggcgttatt tatggaccac ttttatttat 2280
   tgtcagacac ttatttattg ggatgtgagc cccagggggg cctcctccta ggataataaa 2340
   caattttgca gaattggaaa aaaaaaaaa                                   2369
<210> 281
   <211> 1871
   <212> DNA
   <213> Homo sapiens
<220>
   <223> yrdC domain-containing protein, mitochondrial (YRDC), yrdC N(6)-threonylcarbamoyltransferase domain containing, dopamine receptor interacting protein 3 (DRIP3), ischemia/reperfusion inducible protein (IRIP), RP11-109P14.4, SUA5
<220>
   <221> CDS
   <222> (14) ... (853)
   <223> YRDC
<400> 281
   cgggcctggg cggatgtctc cggcgcgtcg gtgcaggggg atgagggccg cggtggctgc 60
   cagcgtgggg ttgagcgagg ggcctgctgg ctcccggagc ggtcgcctct tccgcccgcc 120
   gagtcccgct ccggcggccc ccggcgcccg gctgttgcgg ctcccgggga gcggggccgt 180
   gcaggccgcg agcccggagc gcgccggctg gaccgaggcg ctgcgggccg ccgtggccga 240
   gctgcgcgcc ggcgccgtgg tggccgtccc caccgatacg ctgtacggcc tggcctgcgc 300
   ggcgagctgc tcggcggctc tgcgcgctgt gtaccgcctc aagggtcgca gcgaggccaa 360
   gcctctggcc gtatgcctcg gccgcgtggc cgacgtctac agatactgcc gtgtgagagt 420
   acctgagggg ctcctgaaag acctactgcc aggaccagtg accctggtga tggaacgctc 480
   ggaggagctc aacaaggacc taaacccttt tacgcctctt gtaggcattc ggattcctga 540
   tcatgctttt atgcaagact tggctcagat gtttgagggt ccgcttgctc tcactagtgc 600
   caacctcagc tcccaggcca gttctctgaa tgtcgaggag ttccaggatc tctggcctca 660
   gttgtccttg gttattgatg ggggacaaat tggggatggc cagagccccg agtgtcgcct 720
   tggctcaact gtggttgatt tgtctgtgcc cggaaagttt ggcatcattc gtccaggctg 780
   tgccctggaa agtactacag ccatcctcca acagaagtac ggactgctcc cctcacatgc 840
   gtcctacctg tgaaactctg ggaagcagga aggcccaaga cctggtgctg gatactatgt 900
   gtctgtccac tgacgactgt caaggcctca tttgcagagg ccaccggagc tagggcacta 960
   gcctgacttt taaggcagtg tgtctttctg agcactgtag accaagccct tggagctgct 1020
   ggtttagcct tgcacctggg gaaaggatgt atttatttgt attttcatat atcagccaaa 1080
   agctgaatgg aaaagttaag aacattccta ggtggcctta ttctaataag tttcttctgt 1140
   ctgttttgtt tttcaattga aaagtaatta aataacagat ttagaatcta gtgagagcct 1200
   cctctctggt gggtggtggc atttaaggtt caaaccagcc agaagtgctg gtgctgttta 1260
   aaaagtctca ggtggctgcg tgtggtggct catgcctgta atcccaacat tctgggaggc 1320
   ccaggcggga gaactgcttg agcccaggag ttcagaatca gcctgggcaa catagcaata 1380
   ctccgtctca taaaaattaa taaataaaaa gtctcaggtg accaaaggct cctgaagcta 1440
   gaaccaggtt tggataaaga ttgaagagcc acaggccact cttccctctg agccattggg 1500
   cctagtggtg tcatgtattg taattgctcg cagggagagc agtctttttg gtgtaatagt 1560
   gggatgtctg cttagttggc aggggttcag tccaaatgga agaatattgg gaaataaacc 1620
   tccactatcc tttatagcca gggacttttt tcttatttat tcataaaata aattatagtt 1680
   aattataccc ataacacctt tatttaaatc cagtgttctc cgcagccttt tgtctattta 1740
   tatgtgtacc aagtgttaaa cataattatt attgggcatt tgaactttgt ttttctttaa 1800
   agaaatgctg ctattaaaca tatttgtaaa tggtttaaaa aaaaaaaaaa aaaaaaaaaa 1860
   aaaaaaaaaa a                                                      1871
<210> 282
   <211> 1353
   <212> DNA
   <213> Homo sapiens
<220>
   <223> polymerase (RNA) I polypeptide C, 30kDa (POLR1C), RNA polymerases I and III subunit AC1 (RPAC1), DNA-directed RNA polymerases I and III 40 kDa polypeptide (RPA40), DNA-directed RNA polymerase I subunit C, AC40, RP3-337H4.4, RPA39, TCS3
<220>
   <221> CDS
   <222> (72) ... (1112)
   <223> POLR1C
<400> 282
   cacggagacg gagcagggct actaggacgc acgcgcgaga tagaacctct agtctcgtgg 60
   agagattgaa gatggcggct tctcaggcgg tggaggaaat gcggagccgc gtggttctgg 120
   gggagtttgg ggttcgcaat gtccatacta ctgactttcc cggtaactat tccggttatg 180
   atgatgcctg ggaccaggac cgcttcgaga agaatttccg tgtggatgta gtacacatgg 240
   atgaaaactc actggagttt gacatggtgg gaattgacgc agccattgcc aatgcttttc 300
   gacgaattct gctagctgag gtgccaacta tggctgtgga gaaggtcctg gtgtacaata 360
   atacatccat tgttcaggat gagattcttg ctcaccgtct ggggctcatt cccattcatg 420
   ctgatccccg tctttttgag tatcggaacc aaggagatga agaaggcaca gagatagata 480
   ctctacagtt tcgtctccag gtcagatgca ctcggaaccc ccatgctgct aaagattcct 540
   ctgaccccaa cgaactgtac gtgaaccaca aagtgtatac caggcatatg acatggatcc 600
   ccctggggaa ccaggctgat ctctttccag agggcactat ccgaccagtg catgatgata 660
   tcctcatcgc tcagctgcgg cctggccaag aaattgacct gctcatgcac tgtgtcaagg 720
   gcattggcaa agatcatgcc aagttttcac cagtggcaac agccagttac aggctcctgc 780
   cagacatcac cctgcttgag cccgtggaag gggaggcagc tgaggagttg agcaggtgct 840
   tctcacctgg tgttattgag gtgcaggaag tccaaggtaa aaaggtggcc agagttgcca 900
   acccccggct ggataccttc agcagagaaa tcttccggaa tgagaagcta aagaaggttg 960
   tgaggcttgc ccgggttcga gatcattata tcttctctgt tgagtcaacg ggggtgttgc 1020
   caccagatgt gctggtgagt gaagccatca aagtactgat ggggaagtgc cggcgcttct 1080
   tggatgaact agatgcggtt cagatggact gagcttggat gcttctgagg caagctgaag 1140
   ctttgggttc tgactgaccc accctacagg actgctgaac agagagccca gtgtgactag 1200
   ggatcctgag ttttctggga caattccagc tttaatcaat acattttgtt aaatgtgcca 1260
   taaaatgaga ctttttacgc ctttataagg ccttagatgt aaataaactc acccaaacaa 1320
   aaaaaaaaaa aaaaaaaaaa aaaaaaaaaa aaa                               1353
<210> 283
   <211> 1745
   <212> DNA
   <213> Homo sapiens
<220>
   <223> peter pan homolog (Drosophila) (PPAN), second-step splicing factor 1, suppressor of sterile four 1 (SSF, SSF-1, SSF1, SSF2), brix domain-containing protein 3 (BXDC3), suppressor of SWI4 1 homolog
<220>
   <221> CDS
   <222> (173) ... (1594)
   <223> PPAN
<400> 283
   gtgtctccat agcaaccgct ccatctgatt gcccctcccc gctcccagag gccacctagt 60
   gcgcaggccc gcctgatgtc gtcccacgcc gtgccggctc tcaggcgccg gaagtgagct 120
   gcgcagcgcc ggaagcggcg gacgcaggag gcctcgtgga ggacacagca gcatgggaca 180
   gtcagggagg tcccggcacc agaagcgcgc ccgcgcccag gcgcagctcc gcaacctcga 240
   ggcctatgcc gcgaacccgc actcgttcgt gttcacgcga ggctgcacgg gtcgcaacat 300
   ccggcagctc agcctggacg tgcggcgggt catggagccg ctcactgcca gccgtctgca 360
   ggttcgtaag aagaactcgc tgaaggactg cgtggcagtg gctgggcccc tcggggtcac 420
   acactttctg atcctgagca aaacagagac caatgtctac tttaagctga tgcgcctccc 480
   aggaggcccc accttgacct tccaggtcaa gaagtactcg ctggtgcgtg atgtggtctc 540
   ctcactgcgc cggcaccgca tgcacgagca gcagtttgcc cacccacccc tcctggtact 600
   caacagcttt ggcccccatg gtatgcatgt gaagctcatg gccaccatgt tccagaacct 660
   gttcccctcc atcaacgtgc acaaggtgaa cctgaacacc atcaagcgct gcctcctcat 720
   cgactacaac cccgactccc aggagctgga cttccgccac tatagcatca aagttgttcc 780
   tgtgggcgcg agtcgcggga tgaagaagct gctccaggag aagttcccca acatgagccg 840
   cctgcaggac atcagcgagc tgctggccac gggcgcgggg ctgtcggaga gcgaggcaga 900
   gcctgacggc gaccacaaca tcacagagct gcctcaggct gtcgctggcc gtggcaacat 960
   gcgggcccag cagagtgcag tgcggctcac cgagatcggc ccgcggatga cactgcagct 1020
   catcaaggtc caggagggcg tcggggaggg caaagtgatg ttccacagtt ttgtgagcaa 1080
   gacggaggag gagctgcagg ccatcctgga agccaaggag aagaagctgc ggctgaaggc 1140
   gcagaggcag gcccagcagg cccagaatgt gcagcgcaag caggagcagc gggaggccca 1200
   cagaaagaag agcctggagg gcatgaagaa ggcacgggtc gggggtagtg atgaagaggc 1260
   ctctgggatc ccttcaagga cggcgagcct ggagttgggt gaggacgatg atgaacagga 1320
   agatgatgac atcgagtatt tctgccaggc ggtgggcgag gcgcccagtg aggacctgtt 1380
   ccccgaggcc aagcagaaac ggcttgccaa gtctccaggg cggaagcgga agcggtggga 1440
   aatggatcga ggcaggggtc gcctttgtga ccagaagttt cccaagacca aggacaagtc 1500
   ccagggagcc caggccaggc gggggcccag aggggcttcc cgggatggtg ggcgaggccg 1560
   gggccggggc cgcccaggga agagagtggc ctgagcccaa gccgcaccgg agcagcggct 1620
   ggattgaacg ccccagattg gggcccgaga tgtggccctc ggtttccttt cataaaggag 1680
   ttgtgtcccc agcccttcca ctccagtaaa gaactgaatt ggccagggaa aaaaaaaaaa 1740
   aaaaa                                                             1745
<210> 284
   <211> 2678
   <212> DNA
   <213> Homo sapiens
<220>
   <223> pyrroline-5-carboxylate reductase-like (PYCRL), pyrroline-5-carboxylate reductase 3 (P5CR 3, P5C reductase 3)
<220>
   <221> CDS
   <222> (31) ... (891)
   <223> PYCRL
<400> 284
   ctagatctgt gggcggggcg cggcctgtgg atgggcggtg agcgcagcgg cgtccgaggc 60
   aacaagatgg cagctgcgga gccgtctccg cggcgcgtgg gcttcgtggg cgcgggccgc 120
   atggcggggg ccatcgcgca gggcctcatc agagcaggaa aagtggaagc tcagcacata 180
   ctggccagtg caccaacaga caggaaccta tgtcactttc aagctctggg ttgccggacc 240
   acgcactcca accaggaggt gctgcagagc tgcctgctcg tcatctttgc caccaagcct 300
   catgtgctgc cagctgtcct ggcagaggtg gctcctgtgg tcaccactga acacatcttg 360
   gtgtccgtgg ctgctggggt gtctctgagc accctggagg agctgctgcc cccaaacaca 420
   cgggtgctgc gggtcttgcc caacctgccc tgtgtggtcc aggaaggggc catagtgatg 480
   gcgcggggcc gccacgtggg gagcagcgag accaagctcc tgcagcatct gctggaggcc 540
   tgtgggcggt gtgaggaggt gcctgaagcc tacgtcgaca tccacactgg cctcagtggc 600
   agtggcgtgg ccttcgtgtg tgcattctcc gaggccctgg ctgaaggagc cgtcaagatg 660
   ggcatgccca gcagcctggc ccaccgcatc gctgcccaga ccctgctggg gacggccaag 720
   atgctgctgc acgagggcca acacccagcc cagctgcgct cagacgtgtg caccccgggt 780
   ggcaccacca tctatggact ccacgccctg gagcagggcg ggctgcgagc agccaccatg 840
   agcgccgtgg aggctgccac ctgccgggcc aaggagctca gcagaaagta ggctgggctc 900
   tggccatcct ttcctgcctc tgtgcccctg cctctccctg tgtcccttcc cctgaggact 960
   gcggctccct ccctcctgca tgagggtctc ctactgctcc ttctcccctt gcacagggaa 1020
   atgcaggggg caggacttgg gaggttccag caggcggggg agccccgacc agtggggaca 1080
   ctcctccctc cccagtgagc agaaggcacc gtggtggtgg ctctgcccct tgctgcagtg 1140
   agcccacctt gctgcaacat tggttctgag gggcccaaga gatggcgtct tggtcatttg 1200
   cccgcatggt tgggcagttg gttgaggcca tgaacagaac ttacggtaac aggcacggct 1260
   ggcccaatgc ctggtctgga gctggagctt gcctttggct ttccaggtgg ctccgtgcag 1320
   ctacagccag gccggctgcc tcatctcagc tctagggggc acgagccata tggggtctgc 1380
   acaagagacc ctctcccctg cagtaaagcc aggggccctg gcctgatggg gcccccatgg 1440
   ggagctggag cctgccctgc agcctggaga agagggtggc tgtggtgggc gtgctcatcc 1500
   cctgctaagg agcaggagct gctgggccag gtctgcggca gtgctggggt ggcaccaggt 1560
   gggcagtggt aggtggggtg gcttgaggtc tgggagggtg gccctggcca gccaggacac 1620
   atgcagaccc ctggctttag tctggataca ggctccctct ttcctcccaa tcctaagctc 1680
   ctgacaagtg gccaggtggc tctgggccct cctgccccgt gcctaggtca ggggtcctgg 1740
   aataccccgt agctctggca ccaccacact ggcctctgat ggcaagactt ggcccctcca 1800
   cctgtcccta acggacggca ggtcaggaaa gccaggactc aggggagaag caaaccccca 1860
   ggattgaagg ctagggttct agggcctttg ggtggggagg gcccgggccg gacagcctca 1920
   gctccgtccc ctgccccaca agattcacct gggcctccag tcccacgctg gccccaactg 1980
   ctgcagctct cggcttccgc ccaacagcct ctggaggtga ggcgggagca tgccctcagc 2040
   gaggctgggc ggcgggtcct gctgtgccat ctccctgtgc gcctgagcag atcaatccac 2100
   cagtgcaaaa cagggctaac ggcacctgca ggacagcagc acgctccatc cctcatgctc 2160
   agctgcctct gcggccacgg acttctgccc ttcatctgct ctctcttact ctcctgagcc 2220
   tagcccgtcc gtaagctccc tcccctgcct ggttcccagg gcaggctgac tcagttgact 2280
   gcttggtcca agcctggccc tggcacttgt cagggtcagc ctaaggagat gggaataaag 2340
   aggccagaga gcaccaagtg agctcatgtt tcaccagagg tggataaaac caagttctgg 2400
   gctgggccca gtggctcaca tctgtaattc cagcactttg ggaggccgag gcgggcagat 2460
   cacaaagtca ggagtttgag accaacctga ccaacatgga gaaaccccat ctctactaaa 2520
   aatacaaaat tagctggatg tggtggtgcg tgcctgtaat cccagctact tgggaggctg 2580
   aggcaggaga attgcttgaa tccaggaggc ggagattgca gagagccaag atcatgccac 2640
   tgcactccag cctgggtgac agcaagactc tgtcttgg                         2678
<210> 285
   <211> 3757
   <212> DNA
   <213> Homo sapiens
<220>
   <223> gem (nuclear organelle) associated protein 4 (GEMIN4), component of gems 4, HCC-associated protein 1 (HCAP1), HC56, HHRF-1, p97
<220>
   <221> CDS
   <222> (120) ... (3296)
   <223> GEMIN4
<400> 285
   aggcctaagc gcggccgccg tggctcctgc gtctcccatc gtgccgtgcg tcccgcgccg 60
   cgttcgagtt ctcggagggg agggggcgtt agccccgcgc agccgccggc gtcgccgcca 120
   tggacctagg acccttgaac atctgtgaag aaatgactat tctgcatgga ggcttcttgc 180
   tggccgagca gctgttccac cctaaggcac tggcagaatt aacaaagtct gactgggaac 240
   gtgttggacg gcccatcgtg gaggccttaa gggagatctc ctcggctgca gcacactccc 300
   agccctttgc ctggaagaag aaagccctga tcatcatctg ggccaaggtt ctgcagccgc 360
   accccgtgac cccgtccgac acagagacac ggtggcagga agacctgttc ttctcggtgg 420
   gcaacatgat ccccaccatc aaccacacca tcctcttcga gctgctcaaa tccctggaag 480
   cttctggact ctttatccag ctcctgatgg ccctgcccac caccatctgc catgcagaac 540
   tagagcgctt tctggaacat gtgaccgttg acacttctgc cgaagacgtg gccttcttcc 600
   tggacgtctg gtgggaggtg atgaagcaca agggtcaccc gcaggacccc ctgctctccc 660
   agtttagtgc aatggcccat aagtacctgc ctgccttaga tgagttcccc catcctccaa 720
   agaggcttag gtcagaccca gacgcgtgcc ccaccatgcc cctgttggcc atgctgctcc 780
   gcgggctgac acagatccag agtcggatcc tgggcccggg gaggaagtgc tgtgcgctgg 840
   ccaacctggc tgacatgctg actgtgtttg cgctgacaga ggacgacccc caggaggtgt 900
   ctgcaaccgt gtatctggac aaactggcca cggtgatctc tgtgtggaac tcggacaccc 960
   agaatcccta ccaccagcag gcgctggcag agaaggtgaa ggaggcagaa cgggatgtca 1020
   gcctgacctc gctggccaaa ctccccagtg agaccatttt cgtgggctgc gagttcctgc 1080
   accacctgct gcgggagtgg ggggaggagt tgcaggccgt gctccgcagc agccagggga 1140
   caagttacga cagctaccgg ctgtgcgaca gtctgacttc cttcagccag aacgcgacgc 1200
   tctacctgaa ccgcaccagc ctgtccaagg aggacaggca ggtggtctct gagctggcgg 1260
   agtgtgtcag ggacttcctg aggaaaacga gcacggtgct gaagaacagg gccttggagg 1320
   atatcacagc ttccattgcc atggccgtca tccagcagaa gatggaccgc catatggaag 1380
   tgtgctacat ttttgcctct gagaagaagt gggccttctc ggacgagtgg gtagcctgcc 1440
   tggggagtaa cagggccctc ttccgacagc cagacttggt gttgaggctg ctggaaacag 1500
   tgatagacgt cagcacagct gacagagcca tccctgagtc tcagatccgg caggtgatcc 1560
   acctgatcct ggaatgttac gcagacctct ccctgccagg taaaaataaa gtccttgcag 1620
   gtatcctgcg ttcctggggg cgaaagggcc tctctgaaaa gttgctggct tatgtggagg 1680
   gttttcagga agacctcaat acaactttta accagctcac tcagagtgcc tccgaacagg 1740
   gcttggcaaa agctgtggcc tccgtggccc gcctggtcat agtgcacccg gaagtcacgg 1800
   tgaagaaaat gtgcagcctg gctgtggtca atctcggcac ccacaagttc ctggcccaga 1860
   ttctcactgc cttccctgcc cttaggtttg tggaagagca gggtcccaat tcatctgcca 1920
   ctttcatggt gtcatgcctc aaagaaaccg tctggatgaa gttctctaca cccaaggaag 1980
   aaaagcaatt tttagagctc ctgaactgcc tgatgagtcc cgtgaaaccc caagggattc 2040
   cagtggctgc tcttcttgag ccagacgagg tgctgaagga atttgtcctg cctttcttga 2100
   ggttagatgt tgaagaggta gacctcagtc tgaggatctt catccagact ctagaggcaa 2160
   acgcgtgccg agaggaatac tggctccaga cctgctcccc gtttccactc ctcttcagct 2220
   tgtgccagct cttggaccgc ttcagcaaat actggcagct tcccaaggag aagcggtgcc 2280
   tctctttgga taggaaggat ctagcgatcc atatcctgga gctcctgtgt gagattgtat 2340
   cagccaatgc tgagaccttc tccccggatg tctggatcaa gtccctgtcc tggctccacc 2400
   gcaagttaga acagctagac tggactgtgg gcctgaggct gaagagcttc ttcgaggggc 2460
   acttcaagtg tgaagtgcca gccacacttt ttgagatctg taagctttca gaagacgagt 2520
   ggacctccca ggcccaccca gggtacgggg ctggcacggg gctcctggcc tggatggagt 2580
   gctgctgcgt ctccagcggc atctcggaga ggatgctgtc tctcttggtg gtggacgtgg 2640
   gcaatcctga ggaggtcaga ctgttcagca aaggctttct ggtggccctg gtgcaagtca 2700
   tgccttggtg cagccctcag gagtggcagc gccttcacca gctgaccagg agactgctgg 2760
   agaagcagct cctccatgtc ccttatagcc tggaatatat tcagtttgtt cccctgctca 2820
   acctgaagcc ctttgcccag gagttgcaac tctccgtcct cttcctgagg actttccagt 2880
   ttctctgcag ccatagctgt cgtgattggc ttcctctgga aggctggaac cacgtggtca 2940
   aactcctctg tggcagtctg acccgcctcc tggactcagt cagggcgata caggcagctg 3000
   gcccttgggt tcaaggacca gagcaggacc tgacccagga agccctgttt gtttacaccc 3060
   aggtgttctg ccatgctctg cacatcatgg ccatgctcca cccggaggtc tgtgagccac 3120
   tctacgtttt agccttggaa accctcacct gctatgagac tttgagcaag accaaccctt 3180
   ctgtcagctc cttgctccag agggcacacg agcagcgctt cttaaagtcc attgctgagg 3240
   gcatcggccc tgaagaacgg cgccaaaccc tgttgcagaa gatgagcagc ttctgacttg 3300
   gcgtggggag ctgggcccca acatggcggg tctgcagaag atcagcagct tcttacctgt 3360
   gcgggagcga aaaagctggg cttcaacatg gcaggtctgt aggggtcaga cccgagcagc 3420
   ctggacttta cagttatgtg aaactgtcca caaaaagtca tggcaataat ggtgtaaaga 3480
   aaatagtttc ttgggtattt gtaacgtaca aactatcata aaaattctcc tctttcgcat 3540
   ctcactttgt ctcttctaag tcggcctcag caatagccca ggattaaata tgctctgaaa 3600
   ttgggtttag tgtcttcaag atcaaatcca gccaggagga acatgttcat aactggactt 3660
   ttccatccta gattttggca aataagccca aagttgaaac catgtgagtg gaaaaagcat 3720
   tacatggtac gtataacccc caaaaaaaaa aaaaaaa                          3757
<210> 286
   <211> 1048
   <212> DNA
   <213> Homo sapiens
<220>
   <223> tumor necrosis factor receptor superfamily, member 12A (TNFRSF12A), type I transmembrane protein Fn14, FGF-inducible 14, fibroblast growth factor-inducible immediate-early response protein 14 (FN14), tweak-receptor (TWEAKR), CD266
<220>
   <221> CDS
   <222> (87) ... (476)
   <223> TNFRSF12A
<400> 286
   aaggcggggg cgggggcggg gcggcggccg tgggtccctg ccggccggcg gcgggcgcag 60
   acagcggcgg gcgcaggacg tgcactatgg ctcggggctc gctgcgccgg ttgctgcggc 120
   tcctcgtgct ggggctctgg ctggcgttgc tgcgctccgt ggccggggag caagcgccag 180
   gcaccgcccc ctgctcccgc ggcagctcct ggagcgcgga cctggacaag tgcatggact 240
   gcgcgtcttg cagggcgcga ccgcacagcg acttctgcct gggctgcgct gcagcacctc 300
   ctgccccctt ccggctgctt tggcccatcc ttgggggcgc tctgagcctg accttcgtgc 360
   tggggctgct ttctggcttt ttggtctgga gacgatgccg caggagagag aagttcacca 420
   cccccataga ggagaccggc ggagagggct gcccagctgt ggcgctgatc cagtgacaat 480
   gtgccccctg ccagccgggg ctcgcccact catcattcat tcatccattc tagagccagt 540
   ctctgcctcc cagacgcggc gggagccaag ctcctccaac cacaaggggg gtggggggcg 600
   gtgaatcacc tctgaggcct gggcccaggg ttcaggggaa ccttccaagg tgtctggttg 660
   ccctgcctct ggctccagaa cagaaaggga gcctcacgct ggctcacaca aaacagctga 720
   cactgactaa ggaactgcag catttgcaca ggggaggggg gtgccctcct tcctagaggc 780
   cctgggggcc aggctgactt ggggggcaga cttgacacta ggccccactc actcagatgt 840
   cctgaaattc caccacgggg gtcaccctgg ggggttaggg acctattttt aacactaggg 900
   ggctggccca ctaggagggc tggccctaag atacagaccc ccccaactcc ccaaagcggg 960
   gaggagatat ttattttggg gagagtttgg aggggaggga gaatttatta ataaaagaat 1020
   ctttaacttt aaaaaaaaaa aaaaaaaa                                    1048
<210> 287
   <211> 14
   <212> PRT
   <213> Artificial Sequence
<220>
   <223> synthetic DEAD box
<400> 287

## Description

This invention was made with government support under Grant No. RO1 CA154916 awarded by the National Institutes of Health. The government has certain rights in the invention.

### BACKGROUND OF THE INVENTION

Gene expression studies have been used to examine mRNA in cell populations under different conditions, *e.g*., for comparing gene expression under different drug treatments or in different cell types. For example, Cheok et al. (Nat Genet. 34:85-90 (2003)) demonstrated that lymphoid leukemia cells of different molecular subtypes share common pathways of genomic response to the same treatment, and that changes in gene expression are treatment-specific and that gene expression can illuminate differences in cellular response to drug combinations versus single agents. However, these types of gene expression studies have many drawbacks. For example, genome-scale predictions of synthesis rates of mRNAs and proteins have been used to demonstrate that cellular abundance of proteins is predominantly controlled at the level of translation. Schwanhausser et al. (Nature 473:337-342(2011)).

The mammalian target of rapamycin (mTOR) kinase is a master regulator of protein synthesis that couples nutrient sensing to cell growth and cancer. However, the downstream translationally regulated nodes of gene expression that may direct cancer development have not been well characterized. Thus, there remains a need for methods of characterizing the translational control of mRNAs in oncogenic mTOR signaling and in cell populations generally. The present invention addresses this need and others.

Hsieh et al (2012) Nature, 485, 55-61 discloses identification of specialised translation of the prostate cancer genome by oncogenic mTOR signalling. Hsieh et al (2010) Cancer Cell, 17, 249-261 discloses genetic dissection of the contribution of the most prominent downstream translational components of mTOR signalling toward Akt-driven lymphomagenesis. Ruggero et al (2012) Cold Spring Harbor Perspective in Biology, 5, a012336 relates to the link between perturbations in translational control and cancer etiology as a primary focus in cancer research. Patel et al (2011) Hepatology, 53, 1809-1818 discloses application of a label-free liquid chromatography mass spectrometry based proteomics discovery platform to pre-treatment sera from a well-characterised and matched training cohort of 55 chronic hepatitis patients and an independent validation set of 41 chronic hepatitis genome type 1 patients with characterised IL28B genotype. Weiss et al (2011) Science, 334, 1509-1510 relates to studies assessing the extent of proteins being synthesized in a given cell.

### BRIEF SUMMARY OF THE INVENTION

In one aspect, the present disclosure relates to methods for identifying an agent that modulates an oncogenic signaling pathway (*e.g*., an agent that inhibits an oncogenic signaling pathway) in a biological sample. In some aspects, the method comprises:
(a) contacting the biological sample with an agent;
(b) determining a first translational profile for the contacted biological sample, wherein the translational profile comprises translational levels for one or more genes having a 5' terminal oligopyrimidine tract (5' TOP) and/or a pyrimidine-rich translational element (PRTE); and
(c) comparing the first translational profile to a second translational profile comprising translational levels for the one or more genes in a control sample that has not been contacted with the agent;
wherein a difference in the translational levels of the one or more genes in the first translation profile as compared to the second translation profile identifies the agent as a modulator of the oncogenic signaling pathway.

In some aspects, the method comprises:
(a) contacting the biological sample with an agent;
(b) determining a first translational profile for the contacted biological sample, wherein the translational profile comprises translational levels for one or more genes selected from the group consisting of SEQ ID NOs:1-144; and
(c) comparing the first translational profile to a second translational profile comprising translational levels for the one or more genes in a control sample that has not been contacted with the agent;
wherein a difference in the translational levels of the one or more genes in the first translation profile as compared to the second translation profile identifies the agent as a modulator of the oncogenic signaling pathway.

In some aspects, the method comprises:
(a) contacting the biological sample with an agent;
(b) determining a first translational profile for the contacted biological sample, wherein the translational profile comprises a measurement of gene translational levels for a substantial portion of the genome;
(c) comparing the first translational profile to a second translational profile comprising a measurement of gene translational levels for the substantial portion of the genome translational levels for the one or more genes in a control sample that has not been contacted with the agent;
(d) identifying in the first translational profile a plurality of genes having decreased translational levels as compared to the translational levels of the plurality of genes in the second translational profile; and
(e) determining whether, for the plurality of genes identified in step (d), there is a common consensus sequence and/or regulatory element in the untranslated regions (UTRs) of the genes that is shared by at least 10% of the plurality of genes identified in step (d);
wherein a decrease in the translational levels of at least 10% of the genes sharing the common consensus sequence and/or UTR regulatory element in the first translational profile as compared to the second translational profile identifies the agent as an inhibitor of an oncogenic signaling pathway.

In some aspects, the one or more genes are selected from the genes listed in Table 1, Table 2, and/or Table 3. In some aspects, the one or more genes are cell invasion and/or metastasis genes. In some aspects, the one or more genes are selected from Y-box binding protein 1 (YB1), vimentin, metastasis associated 1 (MTA1), and CD44.

In some aspects, the oncogenic signaling pathway is the mammalian target of rapamycin (mTOR) pathway, the PI3K pathway, the AKT pathway, the Ras pathway, the Myc pathway, the Wnt pathway, or the BRAF pathway. In some aspects, the oncogenic signaling pathway is the mTOR pathway.

In some aspects, the translational level for the one or more genes is decreased for the first translational profile as compared to the second translational profile, thereby identifying the agent as an inhibitor of the oncogenic signaling pathway. In some aspects, the translational level of the one or more genes in the first translational profile is decreased by at least three-fold as compared to the second translational profile. In some aspects, the translational level for the one or more genes is increased for the first translational profile as compared to the second translational profile, thereby identifying the agent as a potentiator of the oncogenic signaling pathway. In some aspects, the translational level of the one or more genes in the first translational profile is increased by at least three-fold as compared to the second translational profile.

In some aspects, the first and/or second translational profiles are generated using ribosomal profiling. In some aspects, the first and/or second translational profiles are generated using polysome microarray. In some aspects, the first and/or second translational profiles are generated using immunoassay. In some aspects, the first and/or second translational profiles are generated using mass spectrometry analysis.

In some aspects, the first and/or second translation profile comprises measuring the translational levels of at least 500 genes in the sample (*e.g*., at least 500, 600, 700, 800, 900, 1000, 2000, 3000, 4000, 5000, 6000, 7000, 8000, 9000, 10,000, 11,000, 12,000, 13,000, 14,000, or 15,000 genes or more). In some aspects, the first and/or second translational profile comprises a genome-wide measurement of gene translational levels.

In some aspects, the biological sample comprises a cell. In some aspects, the cell is a human cell. In some aspects, the cell is a cancer cell. In some aspects, the cancer is prostate cancer, breast cancer, bladder cancer, lung cancer, renal cell carcinoma, endometrial cancer, melanoma, ovarian cancer, thyroid cancer, or brain cancer.

In some aspects, the identified agent binds to a 5' TOP or PRTE sequence in the one or more genes having a different translational level in the first translational profile as compared to the second translational profile. In some aspects, the identified agent inhibits the activity of a downstream effector of the oncogenic signaling pathway, wherein the effector is 4EBP1, p70S6K1/2, or AKT.

In some aspects, the method further comprises chemically synthesizing a structurally related agent derived from the identified agent. In some aspects, the method further comprises administering the structurally related agent to an animal and determining the oral bioavailability of the structurally related agent. In some aspects, the method further comprises administering the structurally related agent to an animal and determining the potency of the structurally related agent.

In another aspect, the present disclosure relates to a structurally related agent to an agent identified as described herein.

In still another aspect, the present disclosure relates to methods of validating a target for therapeutic intervention. In some aspects, the method comprises:
(a) contacting a biological sample with an agent that modulates the target;
(b) determining a first translational profile for the contacted biological sample, wherein the first translational profile comprises translational levels for a plurality of genes; and
(c) comparing the first translational profile to a second translational profile comprising translational levels for the plurality of genes in a control sample that has not been contacted with the agent;
wherein identifying one or more genes of a biological pathway as differentially translated in the first translational profile as compared to the second translational profile validates the target for therapeutic intervention, wherein said biological pathway is selected from a protein synthesis pathway, a cell invasion/metastasis pathway, a cellular metabolism pathway, a cell division pathway, an apoptosis pathway, a signal transduction pathway, a cellular transport pathway, a post-translational protein modification pathway, a DNA repair pathway, and a DNA methylation pathway.

In some aspects, the one or more genes have a 5' terminal oligopyrimidine tract (5' TOP) and/or a pyrimidine-rich translational element (PRTE). In some aspects, the one or more genes are selected from the group consisting of SEQ ID NOs: 1-144.

In some aspects, the target for therapeutic intervention is part of an oncogenic signaling pathway. In some aspects, the oncogenic signaling pathway is the mammalian target of rapamycin (mTOR) pathway. In some aspects, the target for therapeutic intervention is a protein. In some aspects, the target for therapeutic intervention is a nucleic acid.

In some aspects, one or more genes from each of at least two of the biological pathways is differentially translated in the first translational profile as compared to the second translational profile. In some aspects, one or more genes from each of at least three of the biological pathways is differentially translated in the first translational profile as compared to the second translational profile. In some aspects, there is at least a two-fold difference in translational level for the one or more genes in the first translational profile as compared to the second translational profile.

In some aspects, the first and/or second translational profile comprises a genome-wide measurement of gene translational levels. In some aspects, less than 20% of the genes in the genome are differentially translated by at least two-fold in the first translational profile as compared to the second translational profile. In some aspects, less than 5% of the genes in the genome are differentially translated by at least two-fold in the first translational profile as compared to the second translational profile. In some aspects, less than 1% of the genes in the genome are differentially translated by at least two-fold in the first translational profile as compared to the second translational profile.

In some aspects, the first and/or second translational profiles are generated using ribosomal profiling. In some aspects, the first and/or second translational profiles are generated using polysome microarray. In some aspects, the first and/or second translational profiles are generated using immunoassay. In some aspects, the first and/or second translational profiles are generated using mass spectrometry analysis.

In some aspects, the biological sample comprises a cell. In some aspects, the cell is a human cell. In some aspects, the cell is a cancer cell. In some aspects, the cancer is prostate cancer, breast cancer, bladder cancer, lung cancer, renal cell carcinoma, endometrial cancer, melanoma, ovarian cancer, thyroid cancer, or brain cancer.

In some aspects, the therapeutic intervention is an anti-cancer therapy.

In some aspects, the agent is a peptide, protein, RNA, or small organic molecule. In some aspects, the agent is an inhibitory RNA.

In yet another aspect, the present disclosure relates to methods of identifying a drug candidate molecule. In some aspects, the method comprises:
(a) contacting a biological sample with the drug candidate molecule;
(b) determining a translational profile for the contacted biological sample, wherein the translational profile comprises translational levels for a plurality of genes; and
(c) comparing the first translational profile to a second translational profile comprising translational levels for the plurality of genes in a control sample that has not been contacted with the drug candidate molecule,
wherein the drug candidate molecule is identified as suitable for use in a therapeutic intervention when one or more genes of a biological pathway is differentially translated in the first translational profile as compared to the second translational profile, wherein the biological pathway is selected from a protein synthesis pathway, a cell invasion/metastasis pathway, a cellular metabolism pathway, a cell division pathway, an apoptosis pathway, a signal transduction pathway, a cellular transport pathway, a post-translational protein modification pathway, a DNA repair pathway, and DNA methylation pathway.

In some aspects, the one or more genes have a 5' terminal oligopyrimidine tract (5' TOP) and/or a pyrimidine-rich translational element (PRTE). In some aspects, the one or more genes are selected from the group consisting of SEQ ID NOs: 1-144.

In some aspects, one or more genes from each of at least two of the biological pathways is differentially translated in the first translational profile as compared to the second translational profile. In some aspects, one or more genes from each of at least three of the biological pathways is differentially translated in the first translational profile as compared to the second translational profile. In some aspects, there is at least a two-fold difference in translational level for the one or more genes in the first translational profile as compared to the second translational profile.

In some aspects, the first and/or second translational profile comprises a genome-wide measurement of gene translational levels. In some aspects, less than 20% of the genes in the genome are differentially translated by at least two-fold in the first translational profile as compared to the second translational profile. In some aspects, less than 5% of the genes in the genome are differentially translated by at least two-fold in the first translational profile as compared to the second translational profile. In some aspects, less than 1% of the genes in the genome are differentially translated by at least two-fold in the first translational profile as compared to the second translational profile.

In some aspects, the first and/or second translational profiles are generated using ribosomal profiling. In some aspects, In some aspects, the first and/or second translational profiles are generated using polysome microarray. In some aspects, the first and/or second translational profiles are generated using immunoassay. In some aspects, the first and/or second translational profiles are generated using mass spectrometry analysis.

In some aspects, the method further comprises comparing the translational profile for the contacted biological sample with a control translational profile for a second biological sample that has been contacted with a known therapeutic agent. In some aspects, the known therapeutic agent is a known inhibitor of an oncogenic signaling pathway. In some aspects, the known therapeutic agent is a known inhibitor of the mammalian target of rapamycin (mTOR) pathway.

In still another aspect, the present disclosure relates to methods of identifying a subject as a candidate for treatment with an mTOR inhibitor. In some aspects, the method comprises:
(a) determining a first translational profile in a sample from the subject, wherein the first translational profile comprises translational levels for one or more genes having a 5' terminal oligopyrimidine tract (5' TOP) and/or a pyrimidine-rich translational element (PRTE); and
(b) comparing the first translational profile to a second translational profile comprising translational levels for the one or more genes, wherein the second translational profile is from a control sample, wherein the control sample is from a known responder to the mTOR inhibitor prior to administration of the mTOR inhibitor to the known responder;
wherein a translational level of the one or more genes in the first translational profile that is at least as high as the translational level of the one or more genes in the second translational profile identifies the subject as a candidate for treatment with the mTOR inhibitor.

In some aspects, the method comprises:
(a) determining a first translational profile in a sample from the subject, wherein the first translational profile comprises translational levels for one or more genes selected from the group consisting of SEQ ID NOs: 1-144; and
(b) comparing the first translational profile to a second translational profile comprising translational levels for the one or more genes, wherein the second translational profile is from a control sample, wherein the control sample is from a known responder to the mTOR inhibitor prior to administration of the mTOR inhibitor to the known responder;
wherein a translational level of the one or more genes in the first translational profile that is at least as high as the translational level of the one or more genes in the second translational profile identifies the subject as a candidate for treatment with the mTOR inhibitor.

In some aspects, the one or more genes are selected from the genes listed in Table 1, Table 2, and/or Table 3. In some aspects, the one or more genes are cell invasion and/or metastasis genes. In some aspects, the one or more genes are selected from Y-box binding protein 1 (YB1), vimentin, metastasis associated 1 (MTA1), and CD44.

In some aspects, the method comprises:
(a) determining a first translational profile in a sample from the subject, wherein the first translational profile comprises translational levels for one or more genes of a biological pathway, wherein the biological pathway is selected from a protein synthesis pathway, a cell invasion/metastasis pathway, a cellular metabolism pathway, a cell division pathway, an apoptosis pathway, a signal transduction pathway, a cellular transport pathway, a post-translational protein modification pathway, a DNA repair pathway, and a DNA methylation pathway; and
(b) comparing the first translational profile to a second translational profile comprising translational levels for the one or more genes, wherein the second translational profile is from a control sample, wherein the control sample is from a known responder to the mTOR inhibitor prior to administration of the mTOR inhibitor to the known responder;
wherein a translational level of the one or more genes in the first translational profile that is at least as high as the translational level of the one or more genes in the second translational profile identifies the subject as a candidate for treatment with the mTOR inhibitor.

In some aspects, the translational level of one or more genes from each of at least two of the biological pathways is at least as high in the first translational profile as in the second translational profile. In some aspects, the translational level of one or more genes from each of at least three of the biological pathways is at least as high in the first translational profile as in the second translational profile.

In some aspects, there is at least a two-fold difference in translational level for the one or more genes in the first translational profile as compared to the second translational profile.

In some aspects, the first and/or second translation profile comprises measuring the translational levels of at least 500 genes in the sample (*e.g*., at least 500, 600, 700, 800, 900, 1000, 2000, 3000, 4000, 5000, 6000, 7000, 8000, 9000, 10,000, 11,000, 12,000, 13,000, 14,000, or 15,000 genes or more). In some aspects, the first and/or second translational profile comprises a genome-wide measurement of gene translational levels. In some aspects, the first and second translational profiles are differential profiles from before and after administration of the mTOR inhibitor.

In some aspects, the subject has a cancer. In some aspects, the cancer is prostate cancer, breast cancer, bladder cancer, lung cancer, renal cell carcinoma, endometrial cancer, melanoma, ovarian cancer, thyroid cancer, or brain cancer.

In some aspects, the method further comprises administering an mTOR inhibitor to the subject.

In still another aspect, the present disclosure relates to methods of identifying a subject as a candidate for treatment with a therapeutic agent. In some aspects, the method comprises:
(a) determining a first translational profile in a sample from the subject, wherein the translational profile comprises translational levels for one or more genes of a biological pathway, wherein the biological pathway is selected from a protein synthesis pathway, a cell invasion/metastasis pathway, a cellular metabolism pathway, a cell division pathway, an apoptosis pathway, a signal transduction pathway, a cellular transport pathway, a post-translational protein modification pathway, a DNA repair pathway, and a DNA methylation pathway; and
(b) comparing the first translational profile to a second translational profile comprising translational levels for the one or more genes, wherein the second translational profile is from a control sample, wherein the control sample is from a known responder to the therapeutic agent prior to administration of the therapeutic agent to the known responder;
wherein a translational level of the one or more genes that is at least as high as the translational level of the one or more genes in the second translational profile identifies the subject as a candidate for treatment with the therapeutic agent.

In some aspects, the translational level of one or more genes from each of at least two of the biological pathways is at least as high in the first translational profile as in the second translational profile. In some aspects, the translational level of one or more genes from each of at least three of the biological pathways is at least as high in the first translational profile as in the second translational profile.

In some aspects, the first and second translational profiles are differential profiles from before and after administration of the therapeutic agent.

In some aspects, the subject has a disease. In some aspects, the disease is cancer. In some aspects, the cancer is prostate cancer, breast cancer, bladder cancer, lung cancer, renal cell carcinoma, endometrial cancer, melanoma, ovarian cancer, thyroid cancer, or brain cancer. In some aspects, the biological sample comprises diseased cells.

In yet another aspect, the present disclosure relates to methods of treating a subject having a cancer. In some embodiments, the method comprises:
administering an mTOR inhibitor to a subject that has been selected as having a first translational profile comprising a translational level of one or more genes that is at least as high as the translational level of the one or more genes in a second translational profile from a control sample;
wherein the first and second translational profiles comprise translational levels for one or more genes having a 5' terminal oligopyrimidine tract (5' TOP) and/or a pyrimidine-rich translational element (PRTE); and wherein the control sample is from a known responder to the mTOR inhibitor prior to administration of the mTOR inhibitor to the known responder;
thereby treating the cancer in the subject.

In some aspects, the method of treating a subject having a cancer comprises:
administering an mTOR inhibitor to a subject that has been selected as having a first translational profile comprising a translational level of one or more genes that is at least as high as the translational level of the one or more genes in a second translational profile from a control sample;
wherein the first and second translational profiles comprise translational levels for one or more genes selected from the group consisting of SEQ ID NOs: 1-144; and wherein the control sample is from a known responder to the mTOR inhibitor prior to administration of the mTOR inhibitor to the known responder;
thereby treating the cancer in the subject.

In some aspects, the one or more genes are selected from the genes listed in Table 1, Table 2, and/or Table 3. In some aspects, the one or more genes are cell invasion and/or metastasis genes. In some aspects, the one or more genes are selected from Y-box binding protein 1 (YB1), vimentin, metastasis associated 1 (MTA1), and CD44.

In some aspects, the method of treating a subject having a cancer comprises:
administering an mTOR inhibitor to a subject that has been selected as having a first translational profile comprising a translational level of one or more genes that is at least as high as the translational level of the one or more genes in a second translational profile from a control sample;
wherein the first and second translational profiles comprise translational levels for one or more genes of a biological pathway selected from a protein synthesis pathway, a cell invasion/metastasis pathway, a cellular metabolism pathway, a cell division pathway, an apoptosis pathway, a signal transduction pathway, a cellular transport pathway, a post-translational protein modification pathway, a DNA repair pathway, and a DNA methylation pathway; and wherein the control sample is from a known responder to the mTOR inhibitor prior to administration of the mTOR inhibitor to the known responder;
thereby treating the cancer in the subject.

In some aspects, the translational level of one or more genes from each of at least two of the biological pathways is at least as high in the first translational profile as in the second translational profile. In some aspects, the translational level of one or more genes from each of at least three of the biological pathways is at least as high in the first translational profile as in the second translational profile.

In some aspects, the first and/or second translation profile comprises measuring the translational levels of at least 500 genes in the sample (*e.g*., at least 500, 600, 700, 800, 900, 1000, 2000, 3000, 4000, 5000, 6000, 7000, 8000, 9000, 10,000, 11,000, 12,000, 13,000, 14,000, or 15,000 genes or more). In some aspects, the first and/or second translational profile comprises a genome-wide measurement of gene translational levels. In some aspects, the first and second translational profiles are differential profiles from before and after administration of the mTOR inhibitor.

In some aspects, the subject has a cancer. In some aspects, the cancer is prostate cancer, breast cancer, bladder cancer, lung cancer, renal cell carcinoma, endometrial cancer, melanoma, ovarian cancer, thyroid cancer, or brain cancer. In some aspects, the cancer is an invasive cancer.

In some aspects, the method further comprises monitoring the translational levels of the one or more genes in the subject subsequent to administering the mTOR inhibitor.

In still another aspect, the present disclosure relates to methods of treating a subject in need thereof. In some aspects, the method comprises:
administering a therapeutic agent to a subject that has been selected as having a first translational profile comprising a translational level of one or more genes that is at least as high as the translational level of the one or more genes in a second translational profile;
wherein the first and second translational profiles comprise translational levels for one or more genes of a biological pathway selected from a protein synthesis pathway, a cell invasion/metastasis pathway, a cellular metabolism pathway, a cell division pathway, an apoptosis pathway, a signal transduction pathway, a cellular transport pathway, a post-translational protein modification pathway, a DNA repair pathway, and a DNA methylation pathway; and wherein the control sample is from a known responder to the therapeutic agent prior to administration of the therapeutic agent to the known responder;
thereby treating the subject.

In some aspects, the translational level of one or more genes from each of at least two of the biological pathways is at least as high in the first translational profile as in the second translational profile. In some aspects, the translational level of one or more genes from each of at least three of the biological pathways is at least as high in the first translational profile as in the second translational profile.

In some aspects, the first and second translational profiles are differential profiles from before and after administration of the therapeutic agent.

In some aspects, the subject in need of treatment has a disease. In some embodiments, the disease is cancer. In some aspects, the cancer is prostate cancer, breast cancer, bladder cancer, lung cancer, renal cell carcinoma, endometrial cancer, melanoma, ovarian cancer, thyroid cancer, or brain cancer. In some aspects, the cancer is an invasive cancer. In some aspects, the biological sample comprises diseased cells.

In still another aspect, the present disclosure relates to methods of identifying an agent for normalizing a translational profile in a subject in need thereof. In some embodiments, the method comprises:
(a) determining a first translational profile for a first biological sample from the subject, wherein the first translational profile comprises translational levels for a plurality of genes;
(b) comparing the first translational profile to a second translational profile comprising translational levels for the plurality of genes, wherein the second translational profile is from a control sample, wherein the control sample is from a non-diseased subject;
(c) identifying one or more genes of a biological pathway as differentially translated in the first translational profile as compared to the second translational profile, wherein the biological pathway is selected from a protein synthesis pathway, a cell invasion/metastasis pathway, a cellular metabolism pathway, a cell division pathway, an apoptosis pathway, a signal transduction pathway, a cellular transport pathway, a post-translational protein modification pathway, a DNA repair pathway, and a DNA methylation pathway;
(d) contacting a second biological sample from the subject with the agent;
(e) determining a third translational profile for the second biological sample, wherein the third translational profile comprises translational levels for the one or more genes identified as differentially translated in the first translational profile as compared to the second translational profile; and
(f) comparing the translational levels for the one or more genes in the third translational profile to the translational levels for the one or more genes in the first and second translational profiles;
wherein a translational level for the one or more genes in the third translational profile that is closer to the translational level for the one or more genes in the second translational profile than to the translational level for the one or more genes in the first translational profile identifies the agent as an agent for normalizing the translational profile in the subject.

In yet another aspect, the present disclosure relates to methods of normalizing a translational profile in a subject in need thereof. In some aspects, the method comprises:
administering to the subject an agent that has been selected as an agent that normalizes the translational profile in the subject, wherein the agent is selected by:
   (a) determining a first translational profile for a first biological sample from the subject, wherein the first translational profile comprises translational levels for a plurality of genes;
   (b) comparing the first translational profile to a second translational profile comprising translational levels for the plurality of genes, wherein the second translational profile is from a control sample, wherein the control sample is from a non-diseased subject;
   (c) identifying one or more genes of a biological pathway as differentially translated in the first translational profile as compared to the second translational profile, wherein the biological pathway is selected from a protein synthesis pathway, a cell invasion/metastasis pathway, a cellular metabolism pathway, a cell division pathway, an apoptosis pathway, a signal transduction pathway, a cellular transport pathway, a post-translational protein modification pathway, a DNA repair pathway, and a DNA methylation pathway;
   (d) contacting a second biological sample form the subject with the agent;
   (e) determining a third translational profile for the second biological sample, wherein the third translational profile comprises translational levels for the one or more genes identified as differentially translated in the first translational profile as compared to the second translational profile; and
   (f) comparing the translational levels for the one or more genes in the third translational profile to the translational levels for the one or more genes in the first and second translational profiles; wherein a translational level for the one or more genes in the third translational profile that is closer to the translational level for the one or more genes in the second translational profile than to the translational level for the one or more genes in the
   first translational profile identifies the agent as an agent for normalizing the translational profile in the subject;
thereby normalizing the translational profile in the subject.

In some aspects, one or more genes from each of at least two of the biological pathways is differentially translated in the first translational profile as compared to the second translational profile. In some aspects, one or more genes from each of at least three of the biological pathways is differentially translated in the first translational profile as compared to the second translational profile. In some aspects, there is at least a two-fold difference in translational level for the one or more genes in the first translational profile as compared to the second translational profile.

In some aspects, the first and/or second translation profile comprises measuring the translational levels of at least 500 genes in the sample (*e.g*., at least 500, 600, 700, 800, 900, 1000, 2000, 3000, 4000, 5000, 6000, 7000, 8000, 9000, 10,000, 11,000, 12,000, 13,000, 14,000, or 15,000 genes or more). In some aspects, the first, second, and/or third translational profiles comprise a genome-wide measurement of gene translational levels.

In some aspects, the agent is a peptide, protein, inhibitory RNA, or small organic molecule.

In still another aspect, the present disclosure relates to methods for identifying a candidate therapeutic for treating a disease. In some aspects, the method comprises:
(a) determining a first translational profile for a plurality of genes for a disease sample that has been contacted with a candidate agent;
(b) determining a second translational profile for a plurality of genes for a disease sample that has not been contacted with a candidate agent; and
(c) identifying the agent as a candidate therapeutic for treating the disease when one or more genes are differentially translated in the first translation profile as compared to the second translation profile and when the differential translation results in a biological benefit.

In some aspects, the method for identifying a candidate therapeutic for treating a disease comprises:
(a) determining a first translational profile for a plurality of genes for a disease sample that has been contacted with a candidate agent;
(b) determining a second translational profile for a plurality of genes for a disease sample that has been contacted with a known active compound for treating the disease; and
(c) identifying the agent as a candidate therapeutic for use in treating the disease when the first translational profile is comparable to the second translational profile.

In some aspects, the method for identifying a candidate therapeutic for treating a disease comprises:
(a) determining three independent translational profiles, each for a plurality of genes from a disease sample, wherein (i) a first translational profile is from a sample not contacted with any compound; (ii) a second translational profile is from a sample that has been contacted with a known active compound for treating the disease; and (iii) a third translational profile is from a sample that has been contacted with a candidate agent;
(b) identifying one or more genes as differentially translated in the first translational profile as compared to the second translational profile; and
(c) identifying the agent as a candidate therapeutic for use in treating the disease when the one or more differentially translated genes from step (b) are in the third translational profile and when the translational profile of the one or more genes in the third translational profile is closer to the translational profile of the one or more genes in the second translational profile than to the translational profile of the one or more genes in the first translational profile.

In some aspects, the method for identifying a candidate therapeutic for treating a disease comprises:
(a) determining three independent translational profiles, each for a plurality of genes from a disease sample, wherein (i) a first translational profile is from a sample not contacted with any compound; (ii) a second translational profile is from a sample that has been contacted with a known active compound for treating the disease; and (iii) a third translational profile is from a sample that has been contacted with a candidate agent;
(b) determining a first differential translational profile comprising one or more genes differentially translated in the first translational profile as compared to the second translational profile, and determining a second differential translational profile comprising one or more genes differentially translated in the first translational profile as compared to the third translational profile; and
(c) identifying the agent as a candidate therapeutic for use in treating the disease when the first differential translational profile is comparable to the second differential translational profile.

In yet another aspect, the present disclosure relates to methods for identifying a candidate molecule for normalizing a translational profile associated with a disease. In some aspects, the method comprises:
(a) determining a first translational profile for a plurality of genes from a disease sample that has been contacted with a candidate agent;
(b) determining a second translational profile for a plurality of genes from (i) a control non-diseased sample or (2) a control non-diseased sample that has been contacted with the candidate agent; and
(c) identifying the agent as a candidate therapeutic for normalizing a translational profile associated with the disease when the first translational profile is comparable to the second translational profile.

In some aspects, the method for identifying a candidate molecule for normalizing a translational profile associated with a disease comprises:
(a) determining three independent translational profiles, each for a plurality of genes, wherein (i) a first translational profile is from a disease sample, (ii) a second translational profile is from (1) a control non-diseased sample or (2) a control non-diseased sample that has been contacted with a candidate agent, and (iii) a third translational profile is from a disease sample that has been contacted with the candidate agent;
(b) identifying one or more genes as differentially translated in the first translational profile as compared to the second profile; and
(c) identifying the agent as a candidate therapeutic for normalizing a translational profile associated with the disease when the one or more differentially translated genes from step (b) are in the third translational profile and when the translational profile of the one or more genes in the third translational profile is closer to the translational profile of the one or more genes in the second translational profile than to the translational profile of the one or more genes in the first translational profile.

In some aspects, the method for identifying a candidate molecule for normalizing a translational profile associated with a disease comprises:
(a) determining three independent translational profiles, each for a plurality of genes, wherein (i) a first translational profile is from a disease sample, (ii) a second translational profile is from (1) a control non-diseased sample or (2) a control non-diseased sample that has been contacted with a candidate agent, and (iii) a third translational profile is from a disease sample that has been contacted with the candidate agent;
(b) determining a first differential translational profile comprising one or more genes differentially translated in the first translational profile as compared to the second translational profile, and determining a second differential translational profile comprising one or more genes differentially translated in the first translational profile as compared to the third translational profile; and
(c) identifying the agent as a candidate therapeutic for normalizing a translational profile associated with the disease when the first differential translational profile is comparable to the second differential translational profile.

In still another aspect, the present disclosure provides methods of validating a target for therapeutic intervention in disease. In some aspects, the method comprises:
(a) determining a first translational profile for a plurality of genes from a disease sample that has been contacted with an agent that modulates a disease-associated target;
(b) determining a second translational profile for a plurality of genes from a control disease sample that has not been contacted with the agent; and
(c) validating the target for therapeutic intervention in the disease when one or more genes are differentially translated in the first translational profile as compared to the second translational profile and when the differential translation results in a biological benefit.

In some aspects, the method of validating a target for therapeutic intervention in disease comprises:
(a) determining a first translational profile for a plurality of genes from a disease sample that has been contacted with an agent that modulates a disease-associated target;
(b) determining a second translational profile for a plurality of genes from a control disease sample that has been contacted with a known active compound for treating the disease; and
(c) validating the target as a target for therapeutic intervention in the disease when the first translational profile is comparable to the second translational profile.

In some aspects, the method of validating a target for therapeutic intervention in disease comprises:
(a) determining three independent translational profiles, each for a plurality of genes from a disease sample, wherein (i) a first translational profile is from a sample not contacted with any compound, (ii) a second translational profile is from a sample contacted with an agent that modulates a disease-associated target, and (iii) a third translational profile is from a sample contacted with a known active compound for treating the disease;
(b) identifying one or more genes as differentially translated in the first translational profile as compared to the second translational profile; and
(c) validating the target as a target for therapeutic intervention in the disease when the one or more differentially translated genes from step (b) are in the third translational profile and when the translational profile of the one or more genes in the third translational profile is closer to the translational profile of the one or more genes in the second translational profile than to the translational profile of the one or more genes in the first translational profile.

In some aspects, the method of validating a target for therapeutic intervention in disease comprises:
(a) determining three independent translational profiles, each for a plurality of genes from a disease sample, wherein (i) a first translational profile is from a sample not contacted with any compound, (ii) a second translational profile is from a sample contacted with an agent that modulates a disease-associated target, and (iii) a third translational profile is from a sample contacted with a known active compound for treating the disease;
(b) determining a first differential translational profile comprising one or more genes differentially translated in the first translational profile as compared to the second translational profile, and determining a second differential translational profile comprising one or more genes differentially translated in the first translational profile as compared to the third translational profile; and
(c) validating the target as a target for therapeutic intervention in the disease when the first differential translational profile is comparable to the second differential translational profile.

In still another aspect, the present disclosure provides methods for validating a target for normalizing a translational profile associated with a disease. In some aspects, the method comprises:
(a) determining a first translational profile for a plurality of genes from a disease sample that has been contacted with an agent that modulates a disease-associated target;
(b) determining a second translational profile for a plurality of genes from (i) a control non-diseased sample or (ii) a control non-diseased sample that has been contacted with the agent that modulates a disease-associated target; and
(c) validating the target as a target for normalizing a translational profile associated with the disease when the first translational profile is comparable to the second translational profile.

In some aspects, the method for validating a target for normalizing a translational profile associated with a disease comprises:
(a) determining three independent translational profiles, each for a plurality of genes, wherein (i) a first translational profile is from a disease sample, (ii) a second translational profile is from (1) a control non-diseased sample or (2) a control non-diseased sample that has been contacted with an agent that modulates a disease-associated target, and (iii) a third translational profile is from a disease sample that has been contacted with the agent that modulates the disease-associated target;
(b) identifying one or more genes as differentially translated in the first translational profile as compared to the second translational profile; and
(c) validating the target as a target for normalizing a translational profile associated with the disease when the one or more differentially translated genes from step (b) are in the third translational profile and when the translational profile of the one or more genes in the third translational profile is closer to the translational profile of the one or more genes in the second translational profile than to the translational profile of the one or more genes in the first translational profile.

In some aspects, the method for validating a target for normalizing a translational profile associated with a disease comprises:
(a) determining three independent translational profiles, each for a plurality of genes, wherein (i) a first translational profile is from a disease sample, (ii) a second translational profile is from (1) a control non-diseased sample or (2) a control non-diseased sample that has been contacted with an agent that modulates a disease-associated target, and (iii) a third translational profile is from a disease sample that has been contacted with the agent that modulates the disease-associated target;
(b) determining a first differential translational profile comprising one or more genes differentially translated in the first translational profile as compared to the second translational profile, and determining a second differential translational profile comprising one or more genes differentially translated in the first translational profile as compared to the third translational profile; and
(c) validating the target as a target for normalizing a translational profile associated with the disease when the first differential translational profile is comparable to the second differential translational profile.

The present invention provides a method of identifying a subject as a candidate for treating a disease with a therapeutic agent. The method comprises:
(a) determining by ribosomal profiling a first translational profile for a plurality of genes in a sample from a subject having or suspected of having a disease selected from a cancer, an inflammatory disease, an autoimmune disease, a neurodegenerative disease, a neurodevelopmental disease and a metabolic disease;
(b) determining by ribosomal profiling a second translational profile for a plurality of genes in a control sample, wherein the control sample is from a subject known to respond to the therapeutic agent and wherein the sample has not been contacted with the therapeutic agent; and
(c) identifying the subject as a candidate for treating the disease with the therapeutic agent when the first translational profile is comparable to the second translational profile.

In another aspect, the present disclosure provides methods for treating a disease (*e.g*., a cancer, an inflammatory disease, an autoimmune disease, a neurodegenerative disease, a neurodevelopmental disease, a metabolic disease, or a viral infection) comprising administering a therapeutic agent to a subject identified according to any of the methods described herein.

In still another aspect, the present disclosure provides methods for treating a disease (*e.g*., a cancer, an inflammatory disease, an autoimmune disease, a neurodegenerative disease, a neurodevelopmental disease, a metabolic disease, or a viral infection) comprising administering to a subject having the disease a therapeutic agent identified according to any of the methods described herein.

In still yet another aspect, the present disclosure provides methods for treating a disease (*e.g*., a cancer, an inflammatory disease, an autoimmune disease, a neurodegenerative disease, a neurodevelopmental disease, a metabolic disease, or a viral infection) comprising administering to a subject having the disease an agent that modulates a disease-associated target, wherein the target was validated according to any of the methods described herein.

In yet another aspect, the present disclosure provides methods for treating a disease (*e.g*., a cancer, an inflammatory disease, an autoimmune disease, a neurodegenerative disease, a neurodevelopmental disease, a metabolic disease, or a viral infection) by normalizing the disease translational profile, comprising administering to a subject having the disease a therapeutic agent identified according to any of the methods described herein.

### BRIEF DESCRIPTION OF THE DRAWINGS

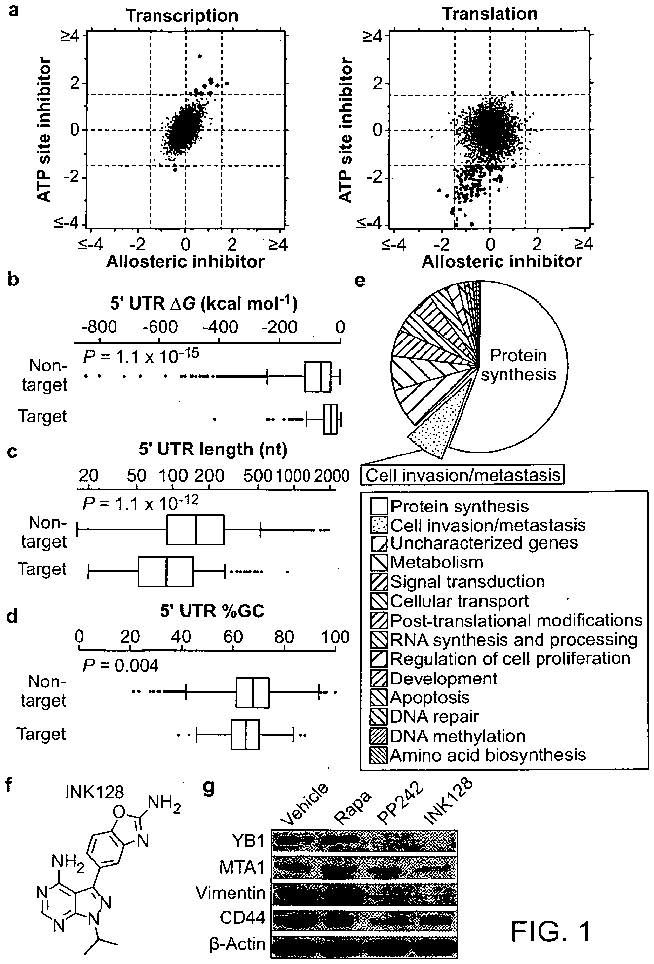

**Figure 1****. Ribosome profiling reveals mTOR-dependent specialized translational control of the prostate cancer genome.** (a) Representative comparison of mRNA abundance and translational efficiency after a 3 hr treatment with an ATP site inhibitor (PP242) versus an allosteric inhibitor (rapamycin). (b-d) Free energy, length and percentage G+C content of the 5' UTRs of mTOR target versus non-target mRNAs (error bars indicate range, non-target n = 5,022, target n = 144, two-sided Wilcoxon). (e) Functional classification of translationally regulated mTOR-responsive mRNAs. (f) Representative Western blot from three independent experiments of mTOR-sensitive invasion genes in PC3 cells after a 48-hr drug treatment. Rapa: rapamycin.

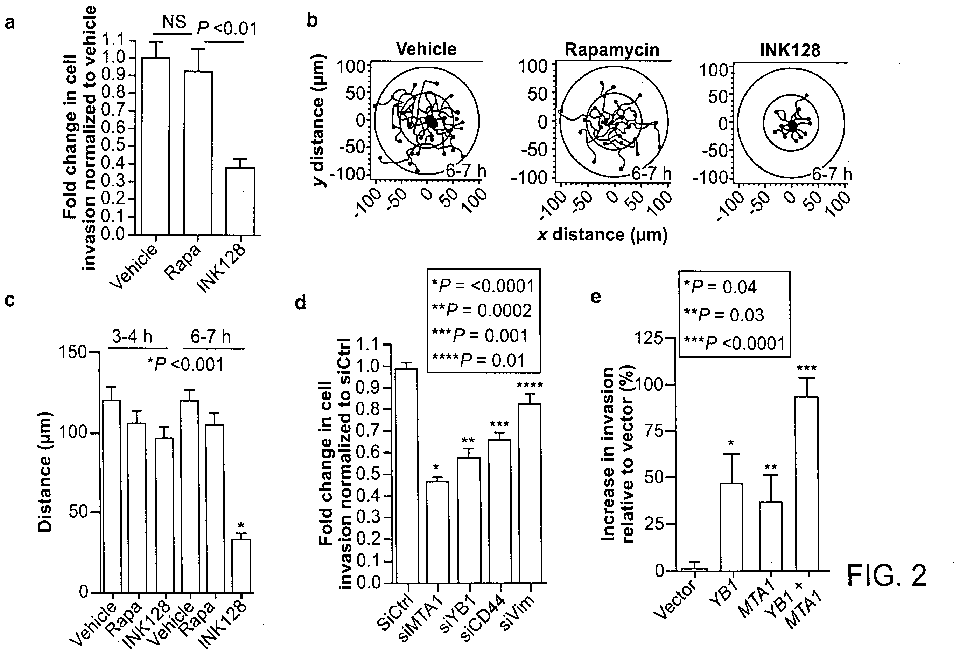

**Figure 2****. mTOR promotes prostate cancer cell migration and invasion through a translationally regulated gene signature.** (a) Matrigel invasion assay in PC3 cells: 6-hr pre-treatment followed by 6 hr of cell invasion (n = 6, ANOVA). (b, c) Migration patterns and average distance traveled by GFP-labeled PC3 cells during hours 3-4 and 6-7 of drug treatment (n =34 cells per condition, ANOVA). (d) Matrigel invasion assay in PC3 cells after 48 hr of knockdown of *YB1, MTA1, CD44,* or vimentin followed by 24 hr of cell invasion (n = 7, t-test). (e) Matrigel invasion assay in BPH-1 cells after 48 hr of overexpression of *YB1* and/or *MTA1,* followed by cell invasion for 24 hr (n = 7, t-test). Rapa: rapamycin. All data represent mean ± s.e.m. NS: not statistically significant.

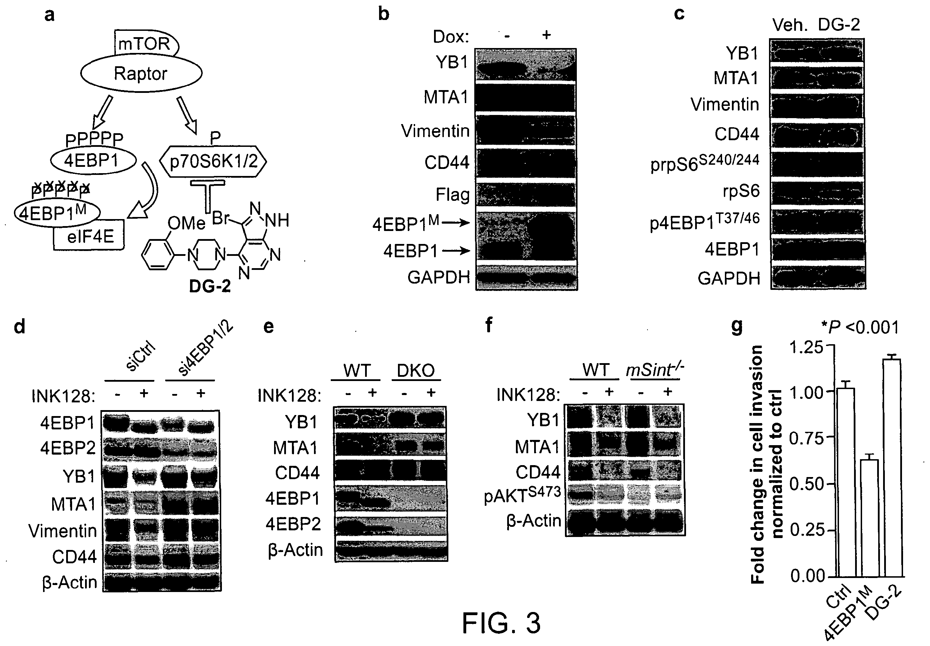

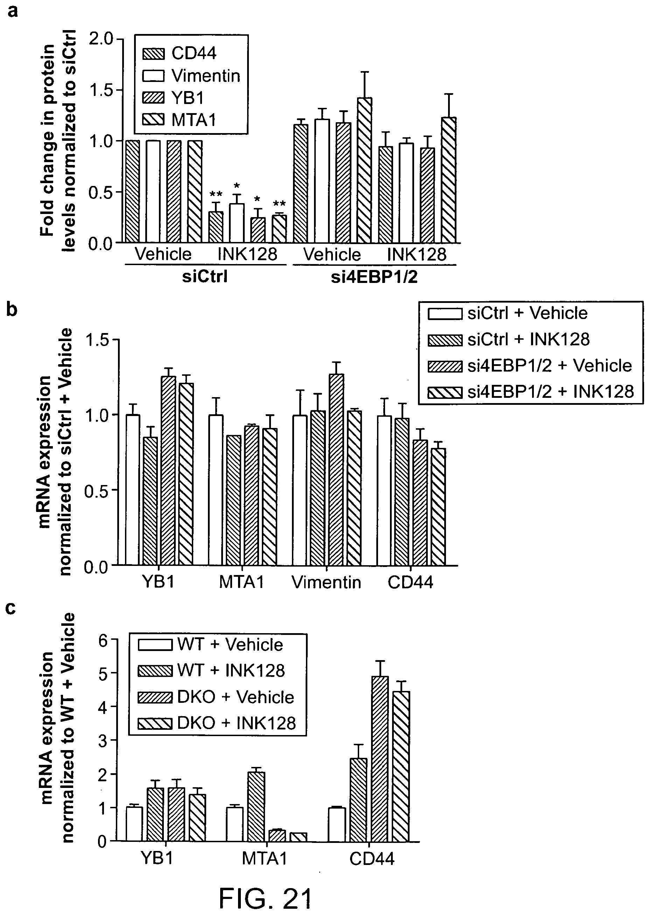

**Figure 3****. The 4EBP1-eIF4E axis controls the post-transcriptional expression of mTOR-sensitive invasion genes.** (a) Schematic of the pharmacogenetic strategy to inhibit p70S6K1/2 or eIF4E hyperactivation. (b) Representative Western blot from three independent experiments of PC3 4EBP1M cells after 48-hr doxycycline induction of 4EBP1^{M}. (c) Representative Western blot from three independent experiments of PC3 cells after 48-hr DG-2 treatment. (d) Representative Western blot from three independent experiments of PC3 cells after 48 h of 4EBP1/4EBP2 knockdown followed by 24-hr treatment with an ATP site inhibitor of mTOR (see quantification of independent experiments in Fig. 21a). (e) Representative Western blot from three independent experiments of wild-type (WT) and *4EBP1*/*4EBP2* double knockout (DKO) MEFs treated with an ATP site inhibitor of mTOR for 24 hr. (f) Representative Western blot from two independent experiments of wild-type and *mSin1*^{*-*/*-*} (also called *Mapkap1^{tm1Bisu}*) MEFs after 24-hr treatment with an ATP site inhibitor of mTOR. (g) Matrigel invasion assay upon 48-hr doxycycline induction of 4EBP1^{M}, or treatment with DG-2 compared to control (n =6 per condition, t-test). All data represent mean ± s.e.m.

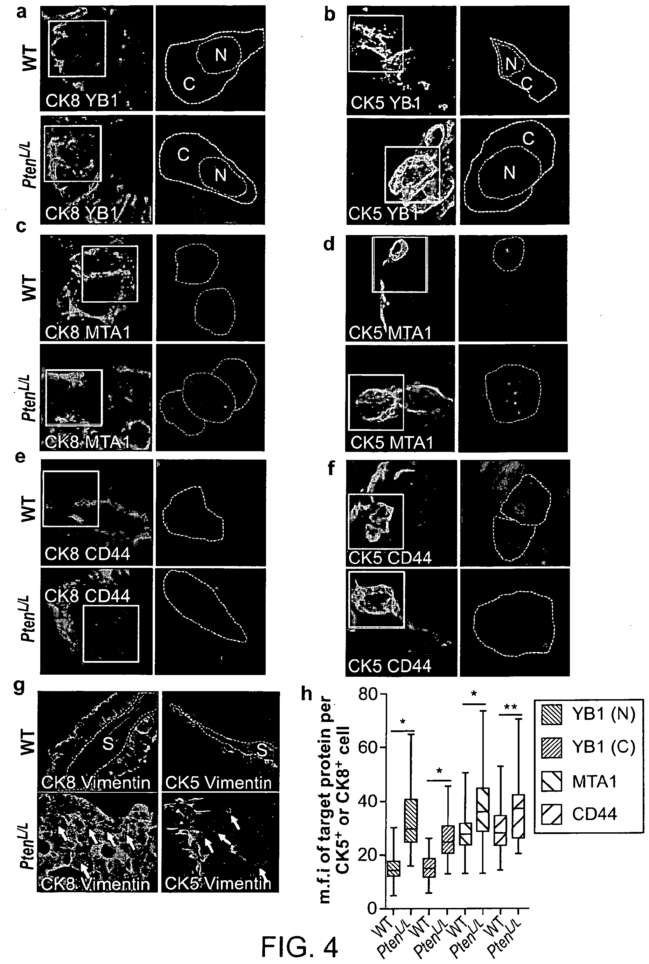

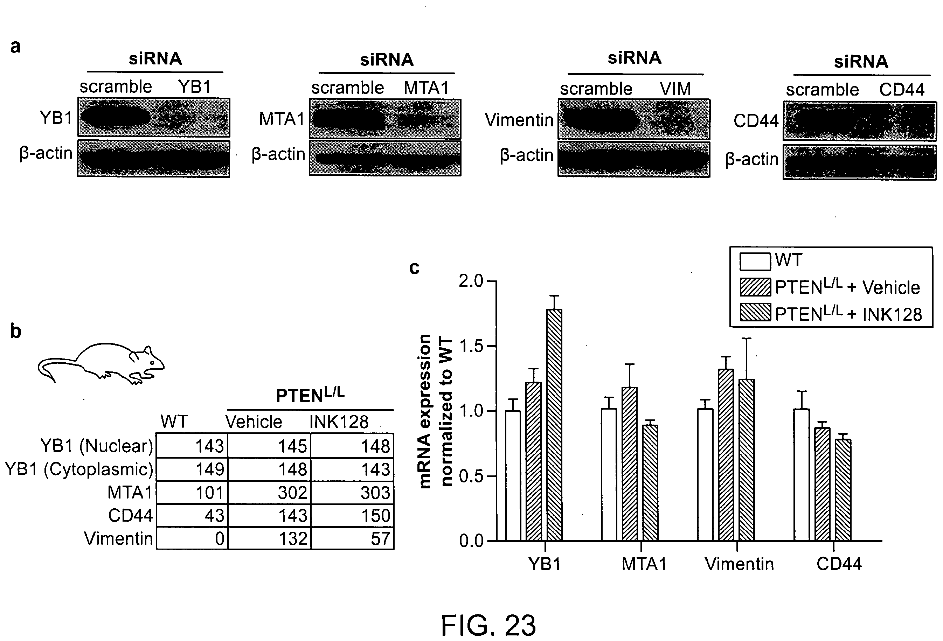

**Figure 4****. mTOR hyperactivation augments translation of *YB1, MTA1, CD44,* and vimentin mRNAs in a subset of pre-invasive prostate cancer cells *in vivo.*** Left: immunofluorescent images of CK8/DAPI or CK5/DAPI with YB1 (a, b), MTA1 (c, d), or CD44 (e, f) co-staining in 14-month-old wild-type and *Pten*^{*L*/*L*} mouse prostate epithelial cells. White boxes outline the area magnified in the right panel. Right: magnified immunofluorescent images of YB1 (a, b), MTA1 (c, d) and CD44 (e, f) co-stained with DAPI in wild-type and *Pten*^{*L*/*L*} mouse prostate epithelial cells. Dotted lines encircle the cytoplasm (C) and/or the nucleus (N). (g) Representative immunofluorescent images of CK5 or CK8 co-staining with vimentin in 14-month-old wild-type and *Pten*^{*L*/*L*}mouse prostate epithelial cells. S: stroma; yellow arrows indicate perinuclear vimentin. (h) Box plot of YB1 (N = nuclear, C = cytoplasmic), MTA1, and CD44 mean fluorescence intensity (m.f.i.) per CK5⁺ orCK8⁺ prostate epithelial cell in wild-type and *Pten*^{*L*/*L*} mice (three mice per arm, n = 43-303 cells quantified per target gene, error bars indicate range (see Fig. 23b); *P <0.0001, **P = 0.0004, t-test).

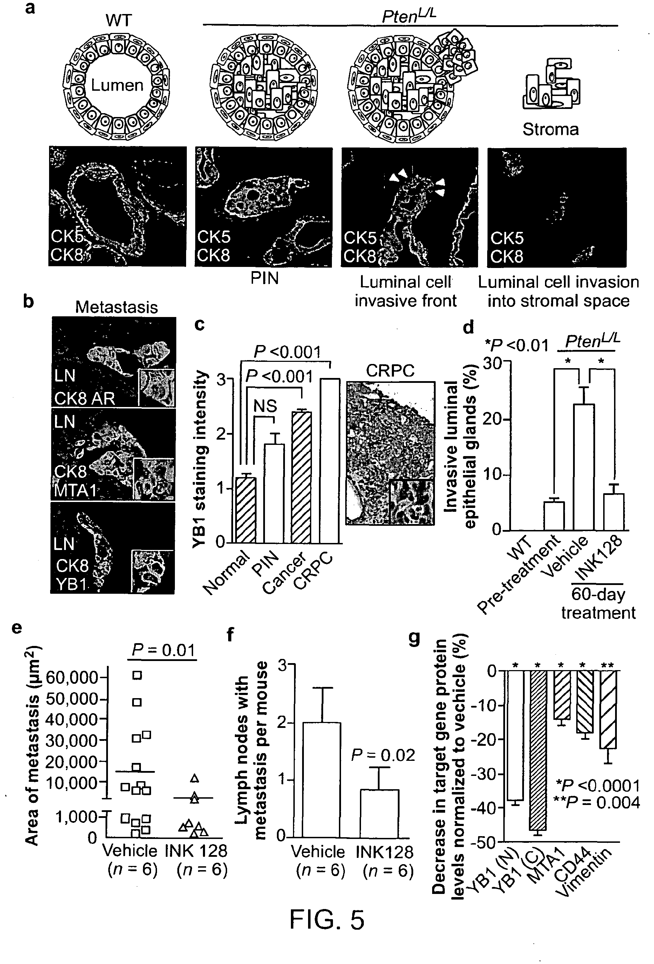

**Figure 5****. Complete mTOR inhibition by treatment with an ATP site inhibitor of mTOR prevents prostate cancer invasion and metastasis *in vivo.*** (a) Diagram and images of normal prostate gland, pre-invasive PIN, and invasive prostate cancer. CK8/CK5, luminal/basal epithelial cells, respectively. Yellow arrowheads indicate invasive front. (b) Immunofluorescent images of 14-month-old *Pten*^{*L*/*L*} lymph node (LN) metastasis co-stained with CK8/androgen receptor (AR), CK8/YB1, and CK8/MTA1. (c) Left: human tissue microarray of YB1 protein levels in normal (n = 59), PIN (n = 5), cancer (n = 99), and CRPC (n = 3) (ANOVA). Right: immunohistochemistry of YB1 in human CRPC demarcated by the red line (inset shows nuclear and cytoplasmic YB1). (d) Quantification of invasive prostate glands in wild-type and *Pten*^{*L*/*L*} mice before (12-months old) and after (14-months old) 60 days of treatment with an ATP site inhibitor of mTOR (n = 6 mice per arm, ANOVA). (e, f) Area and number of CK8/AR+ metastases in draining lymph nodes in 14-month-old *Pten*^{*L*/*L*} mice after 60 days of treatment with an ATP site inhibitor of mTOR (n = 6 mice per arm, t-test). (g) Percentage decrease of YB1 (N = nuclear, C = cytoplasmic), MTA1, CD44, or vimentin protein levels (determined by quantitative immunofluorescence, see Fig. 23b) in CK8⁺ or CK5⁺ prostate cells (CK8⁺ only for vimentin) in ATP site inhibitor of mTOR-treated 14-month-old *Pten*^{*L*/*L*} mice normalized to vehicle-treated mice (n = 3 mice per arm, t-test). All data represent mean ± s.e.m.

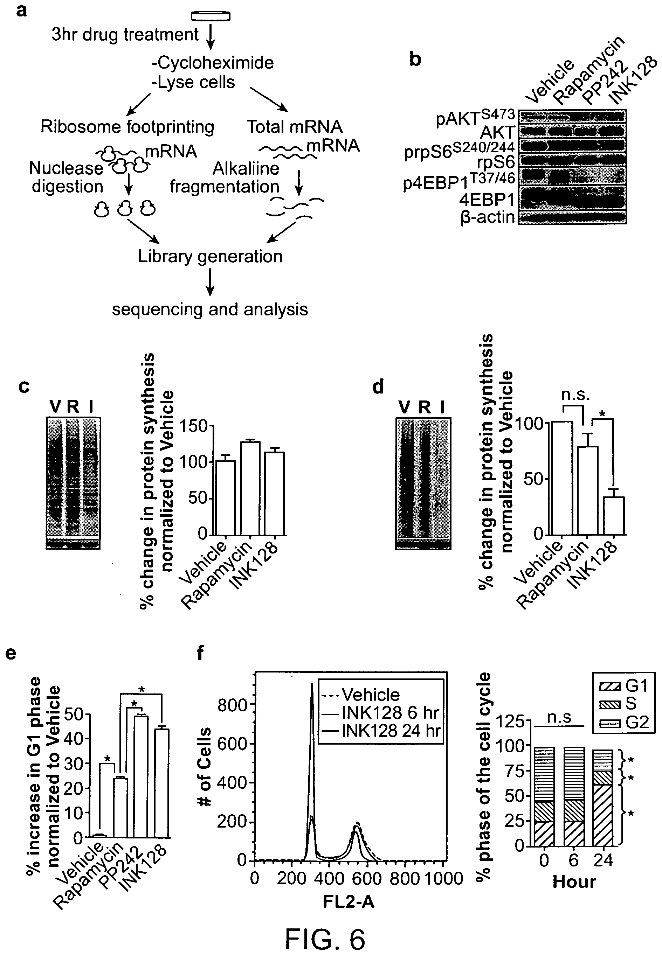

**Figure 6****. Validation of mTOR inhibitors in PC3 prostate cancer cell line.** (a) Schematic of ribosome profiling of human prostate cancer cells. (b) Representative Western blot analysis from 3 independent experiments of PC3 prostate cancer cells treated with rapamycin (50 nM), PP242 (2.5 µM), or ATP site inhibitor of mTOR (200 nM) for 3 hours. (c) Representative [³⁵S]-methionine incorporation in PC3 cells after 6-hour treatment with rapamycin (50 nM) or an ATP site inhibitor of mTOR (200 nM) (left panel). Quantification of [³⁵S]-methionine incorporation (right panel, n = 4, mean + SEM). (d) Representative [³⁵S]-methionine incorporation in PC3 cells after 14-hour treatment with rapamycin (50 nM) or an ATP site inhibitor of mTOR (200 nM) (left panel). Quantification of [³⁵S]-methionine incorporation (right panel, n = 4, mean + SEM, *P<0.05 ANOVA). (e) Cell cycle analysis of PC3 cells after treatment with rapamycin (50 nM), PP242 (2.5 µM), or an ATP site inhibitor of mTOR (200 nM) for 48 hours (mean + SEM, n = 3, *P<0.001 ANOVA). (f) Cell cycle analysis of PC3 cells after 0-, 6-, or 24-hour treatment with an ATP site inhibitor of mTOR (200 nM) (mean + SEM, n = 3, *P<0.001 ANOVA). n.s.: not statistically significant. V: vehicle; R: rapamycin; I: ATP site inhibitor of mTOR.

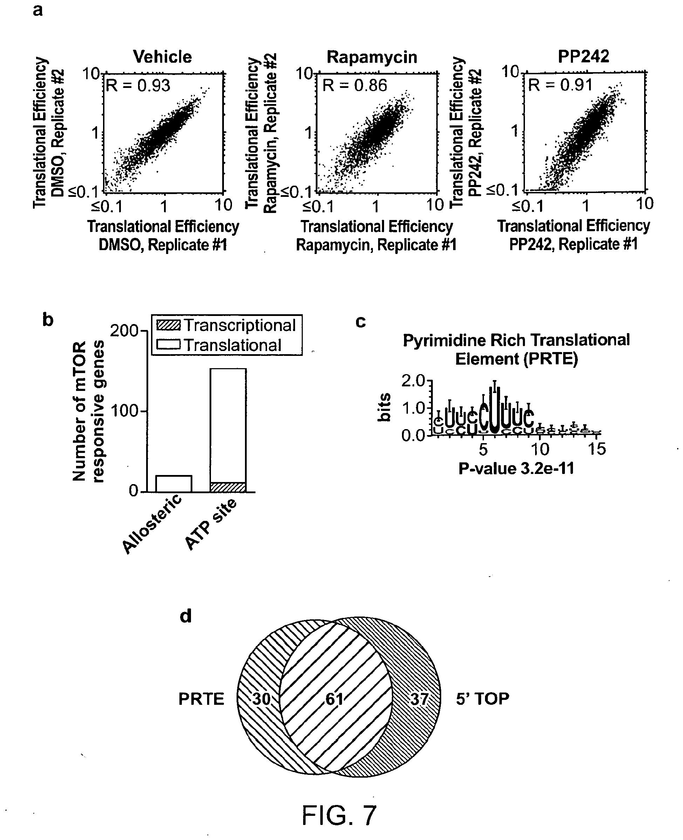

**Figure 7****. Inter-experimental correlation of ribosome profiling per treatment condition and tally of mTOR responsive genes.** (a) Correlation plots from 2 independent ribosome profiling experiments after a 3-hour treatment with rapamycin (50 nM) or PP242 (2.5 µM). (b) Number of translationally and transcriptionally regulated mRNA targets of mTOR after 3-hour drug treatments. (c) The Pyrimidine Rich Translational Element (PRTE) (SEQ ID NO:145) is present within the 5' UTRs of 63% of mTOR-responsive translationally regulated mRNAs. (d) Venn diagram of the number of mTOR sensitive genes that possess a PRTE (red), 5' TOP (green), or both (yellow).

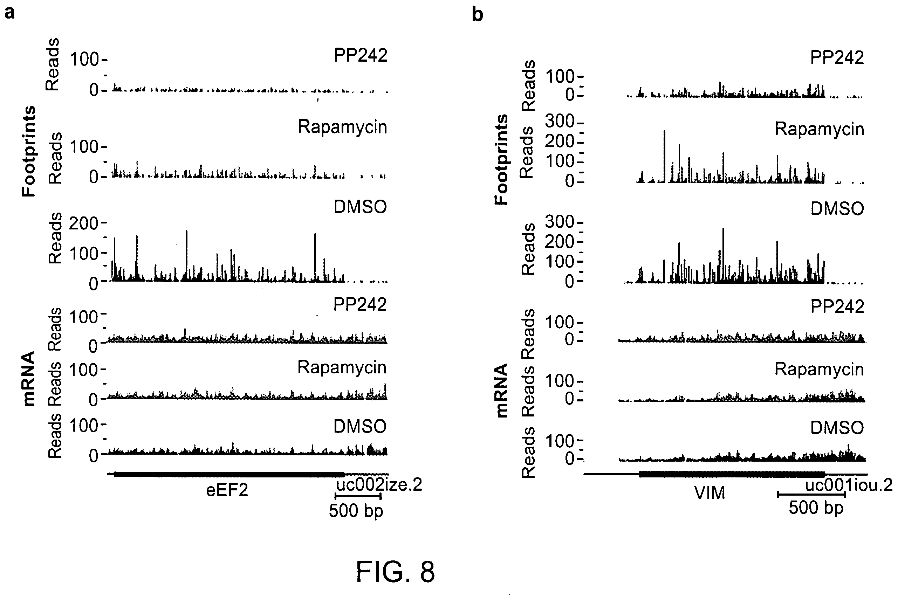

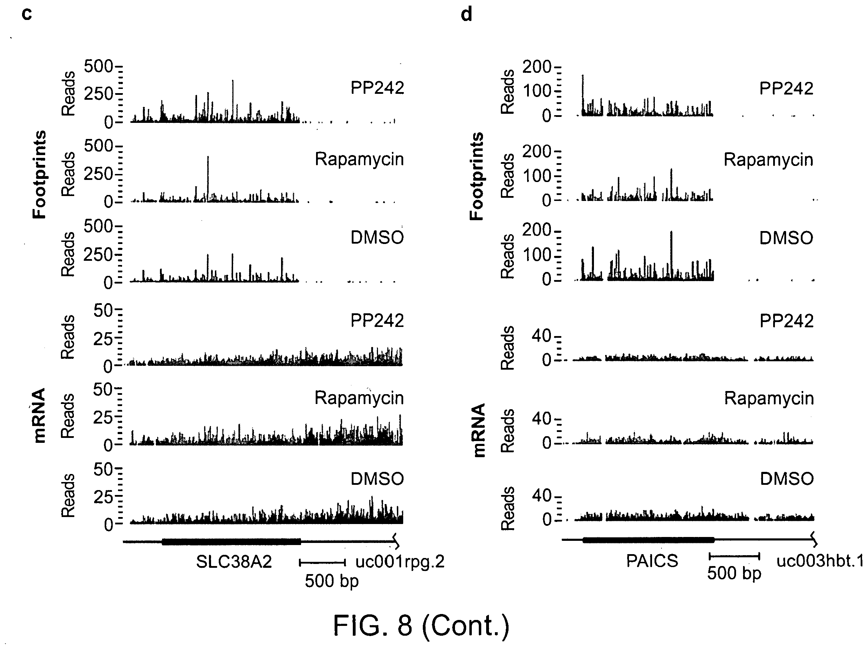

**Figure 8****. Read count profiles for *eEF2,* vimentin, *SLC38A2,* and *PAICS.*** (a) Ribosome footprint and RNA-Seq profiles for *eEF2.* Read count profiles are shown for each nucleotide position in the uc0021ze.2 transcript, with the *eEF2* coding sequence marked. Ribosome footprints were assigned to specific A site nucleotide positions based on their length. (b) Ribosome footprint and RNA-Seq profiles for vimentin. (c) Ribosome footprint and RNA-Seq profiles for *SLC38A2.* (d) Ribosome footprint and RNA-Seq profiles for *PAICS.*

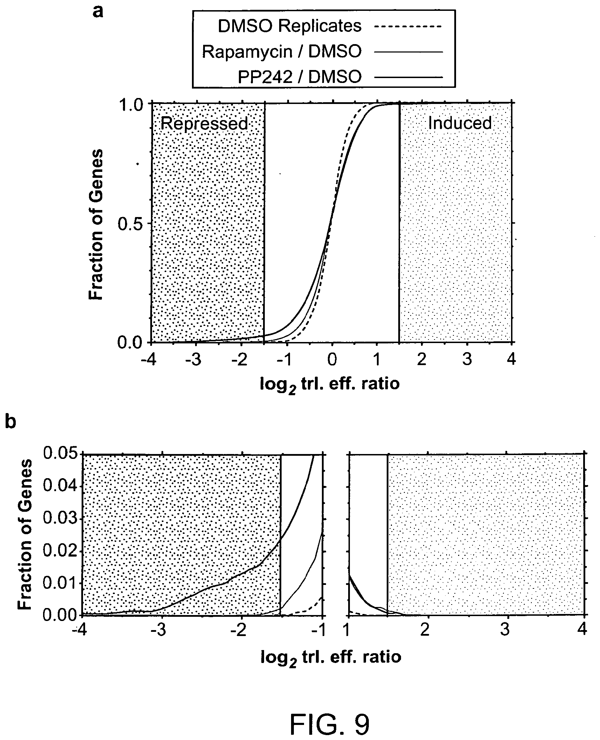

**Figure 9****. False Discovery Rate computation.** (a) The cumulative distribution of log₂ fold-change values is shown for three comparisons, considering only genes passing the minimum read count criterion in that comparison. The DMSO replicate represents a comparison of full biological replicates of the control DMSO-only treatment condition. The rapamycin and PP242 conditions show the ratio of drug-treated to DMSO-treated samples within a single experiment. The fold-change threshold chosen based on PP242 translational repression, described below, is shown. (b) The extremes of the log₂ fold-change cumulative distributions, showing the complementary cumulative distribution function for positive extreme values on the right. The cumulative distribution of fold-change values between the DMSO replicates was used as an estimate of the error distribution for measurements in drug treatment comparisons. That is, the fraction of genes above a given absolute value fold-change level in the comparison of biological replicates should reflect the fraction of genes above that level by chance in any measurement. At a cutoff of log₂ fold-change of +/- 1.5, we detect 2.5% (95% CI, 2.1% - 2.9% by Agresti-Coull) of genes in the PP242 / DMSO comparison and only 0.044% (95% CI, 0.001% - 0.172%) of genes in the DMSO replicate comparison. The estimated false discovery rate is therefore q = 0.018 in the PP242 / DMSO comparison at this fold-change threshold.

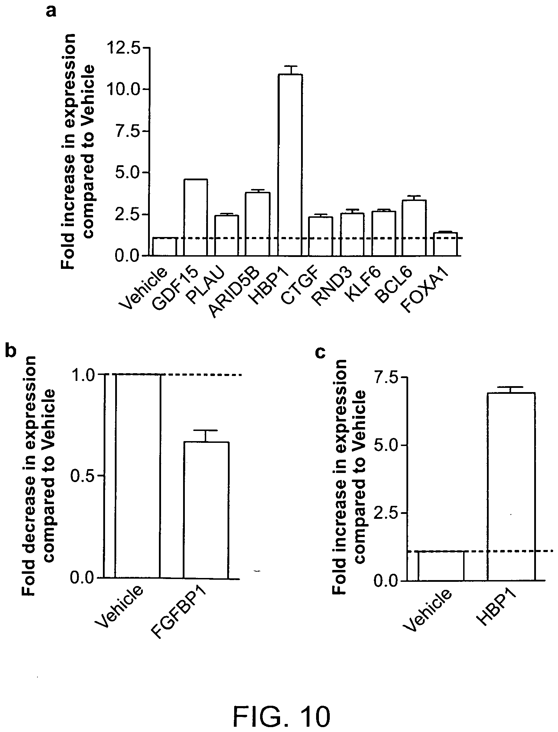

**Figure 10****. Transcriptionally regulated mTOR targets.** (a and b) qPCR validation of up-regulated or down-regulated transcripts identified by RNA-Seq upon 3-hour PP242 treatment (2.5 µM) in PC3 cells (mean + SEM, n = 3). (c) qPCR validation of up-regulated transcript identified by RNA-Seq upon 3-hour rapamycin treatment (50 nM) in PC3 cells (mean + SEM, n = 3).

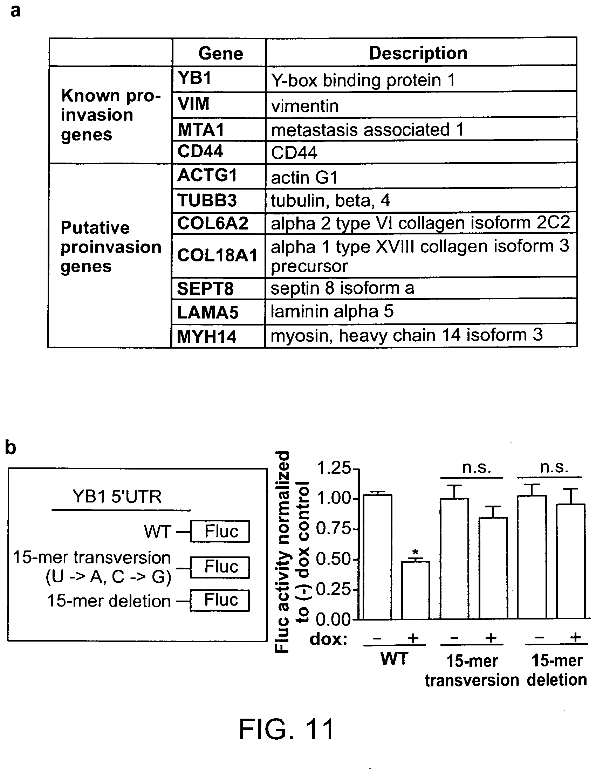

**Figure 11****. mTOR-sensitive translationally regulated gene invasion signature.** Mutation of the PRTE abrogates sensitivity to eIF4E. (a) 4 known pro-invasion genes and 7 putative pro-invasion genes discovered through ribosome profiling. (b) Schematic of *YB1 5'* UTR cloning (WT, transversion mutant, and deletion mutant of the PRTE (position +20-34, uc001chs.2)) into pGL3-Promoter (left panel). Firefly luciferase activity in PC3-4EBP1^{M} cells after a 24-hour pre-treatment with 1 µg/ml doxycycline followed by transfection of respective 5' UTR constructs (mean + SEM, n = 7, *P<0.0001, t-test) (right panel). n.s.: not statistically significant.

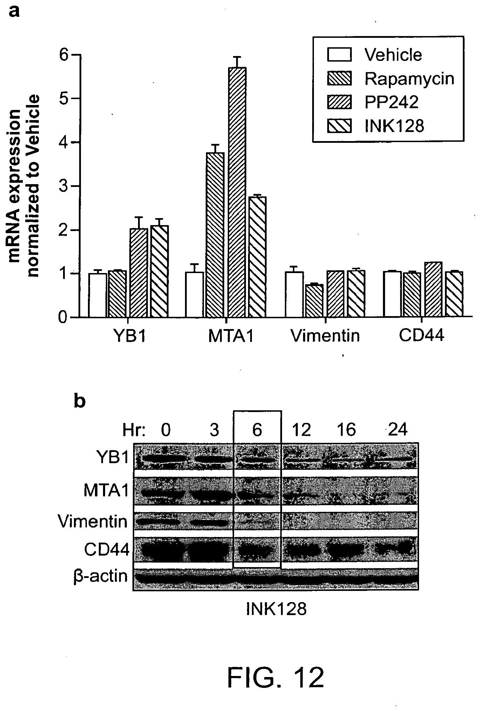

**Figure 12****. ATP site inhibition of mTOR does not reduce transcript levels of the 4 invasion genes.** ATP site inhibitor of mTOR time course. (a) mRNA expression of *YB1, MTA1,* vimentin, and *CD44,* relative to β-actin upon treatment with rapamycin (50 nM), PP242 (2.5 µM), or an ATP site inhibitor of mTOR (200 nM) for 48 hours in PC3 cells (mean + SEM, n = 3). (b) Representative Western blot of 3 independent experiments showing a time course of invasion gene expression before and after treatment with ATP site inhibitor of mTOR (200 nM) in PC3 cells.

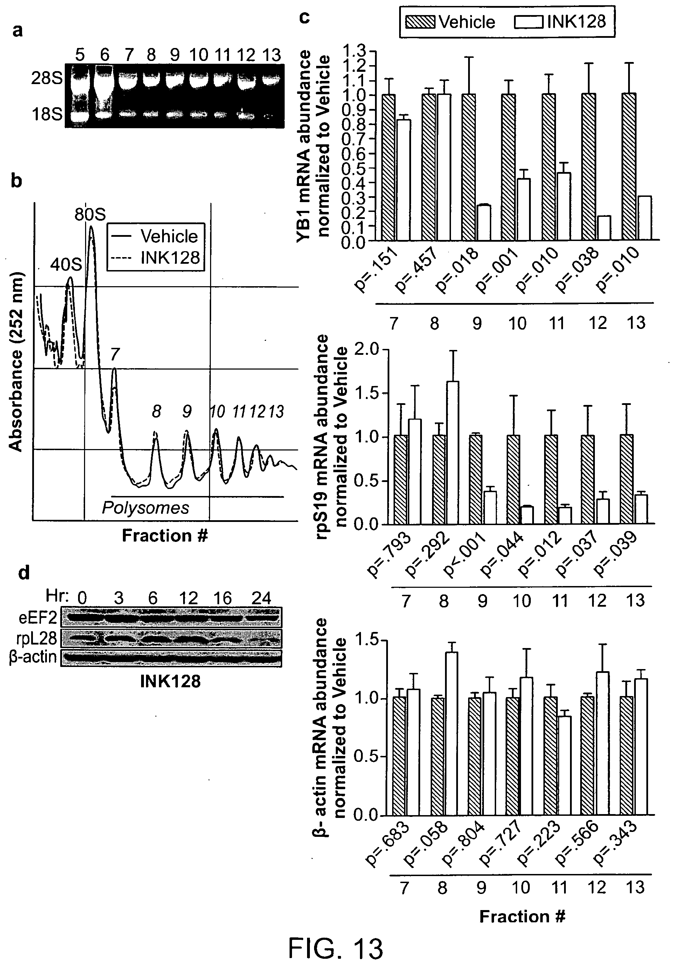

**Figure 13****. Polysome analysis after 3-hour ATP site inhibitor of mTOR treatment.** (a) Ethidium bromide staining of rRNA species in individual fractions. Fractions 7-13 were determined to be polysome-associated fractions. (b) Overlay of polysome profiles from PC3 cells treated with vehicle (solid line) or ATP site inhibitor of mTOR (100 nM) (dotted line). (c) qPCR analysis of *YB1* and *rpS19* mRNAs that show differential association in polysome fractions after ATP site inhibitor of mTOR (100 nM) treatment (mean + SEM, n = 6). The bottom graph shows that there is no change in β-actin mRNA association in polysome fractions between treatments. P-values (t-test) for each polysome fraction are shown. (d) Representative Western blot of 3 independent experiments showing a time course of eEF2 and rpL28 expression before and after treatment with ATP site inhibitor of mTOR (200 nM) in PC3 cells.

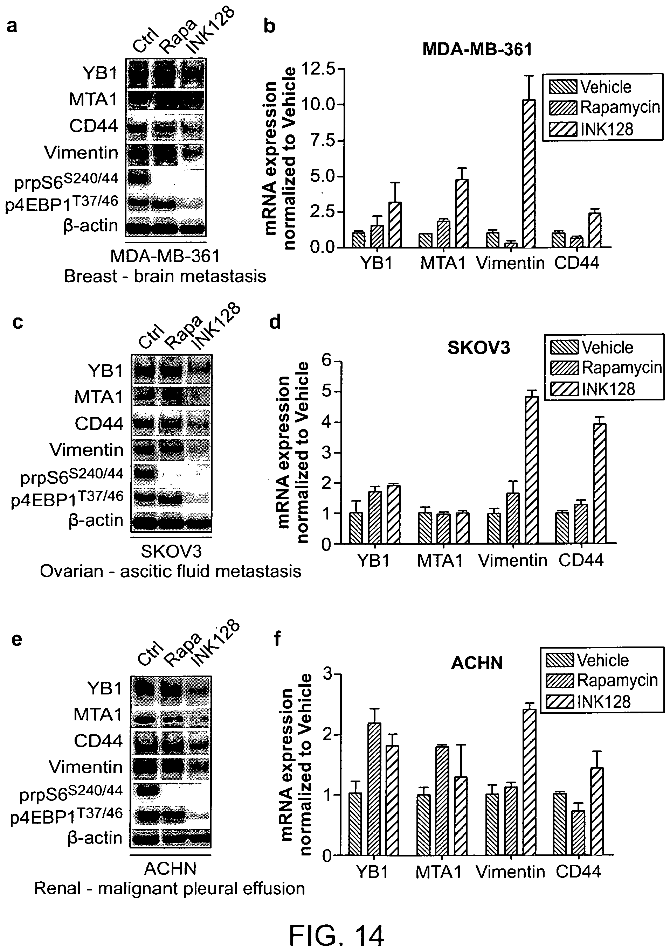

**Figure 14****. 4-gene invasion signature is responsive to ATP site inhibitor of mTOR but not rapamycin in metastatic cell lines.** (a-b) Representative Western blot (a) and qPCR analysis (b) of MDA-MB-361 cells after 48-hour treatment with ATP site inhibitor of mTOR (200 nM). (c-d) Representative Western blot (c) and qPCR analysis (d) of SKOV3 cells after 48-hour treatment with ATP site inhibitor of mTOR (200 nM). (e-f) Representative Western blot (e) and qPCR analysis (f) of ACHN cells after 48-hour treatment with ATP site inhibitor of mTOR (200 nM). Westerns = representative Western blot of 2 independent experiments. qPCR - n = 3. All data represent mean + SEM.

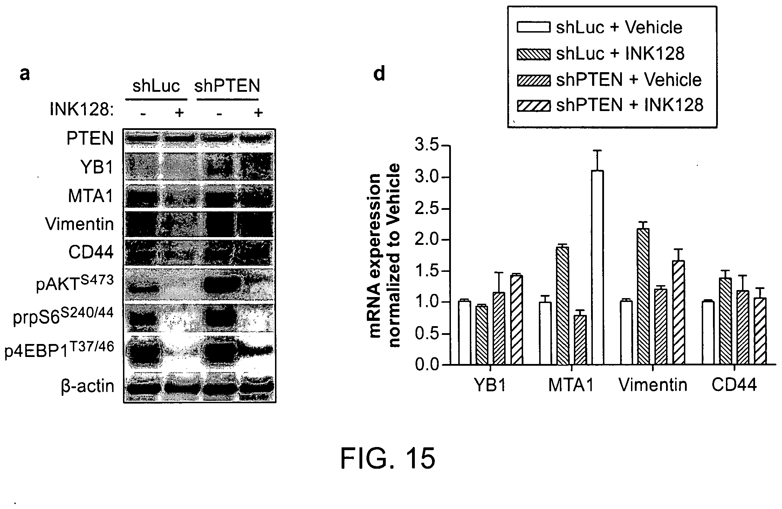

**Figure 15****. PTEN gene silencing in the A498 *PTEN* positive renal carcinoma cell line induces the post-transcriptional expression of the 4-gene invasion signature.** (a-b) Representative Western blot (a) and qPCR analysis (b) of A498 cells after stable silencing of *PTEN* and 24 hour treatment with an ATP site inhibitor of mTOR (200 nM). Western = representative Western blot of 2 independent experiments. qPCR - n = 3. All data represent mean + SEM.

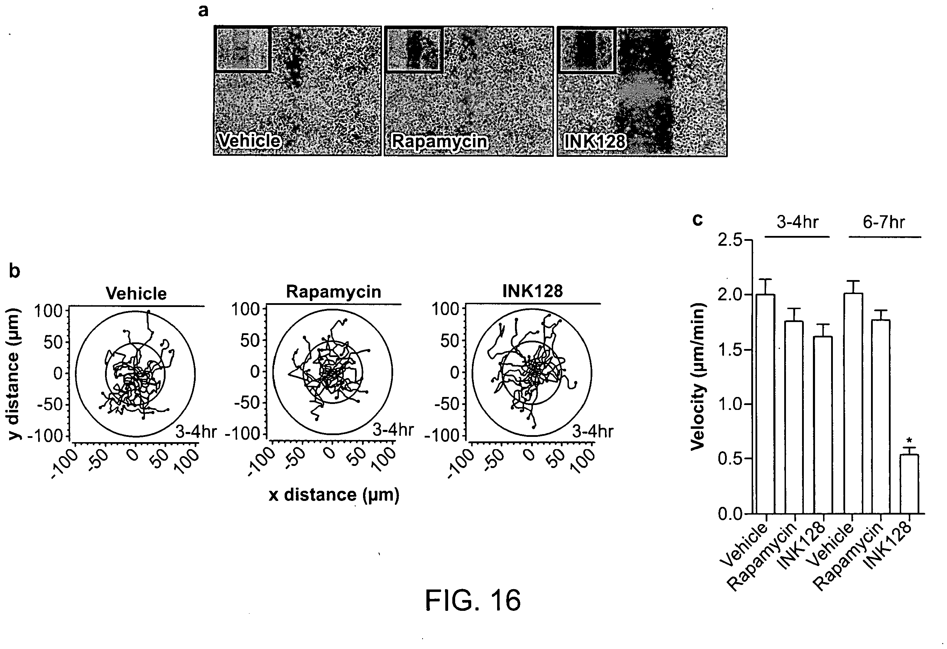

**Figure 16****. ATP site inhibitor of mTOR inhibits cell migration in PC3 prostate cancer cells as early as 6 hours after drug treatment.** (a) Representative wound healing assay of 3 independent experiments in PC3 cells treated with rapamycin (50 nM) or ATP site inhibitor of mTOR (200 nM) for 40 hours. Inset (red box) represents wound at 0 hours. (b) Migration patterns of individual GFP-labeled PC3 cells during hours 3-4 after treatment with rapamycin or ATP site inhibitor of mTOR (34 cells per condition). (c) Average velocity of GFP-labeled PC3 cells during hours 3-4 or 6-7 after treatment with rapamycin (50 nM) or ATP site inhibitor of mTOR (200 nM) (mean + SEM, n = 34 cells per condition, *P<0.001, ANOVA).

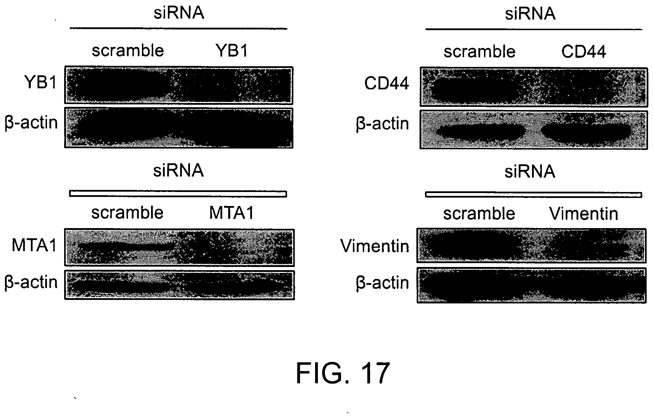

**Figure 17****. Knockdown of the 4 invasion genes in PC3 prostate cancer cells.** YB1, CD44, MTA1, and Vimentin protein levels after 48 hours of gene silencing in PC3 cells.

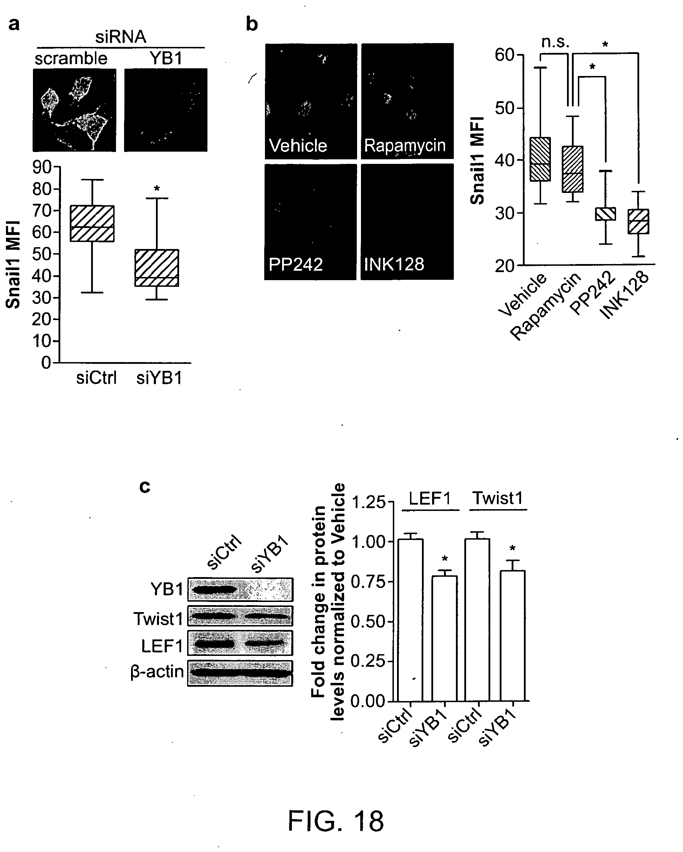

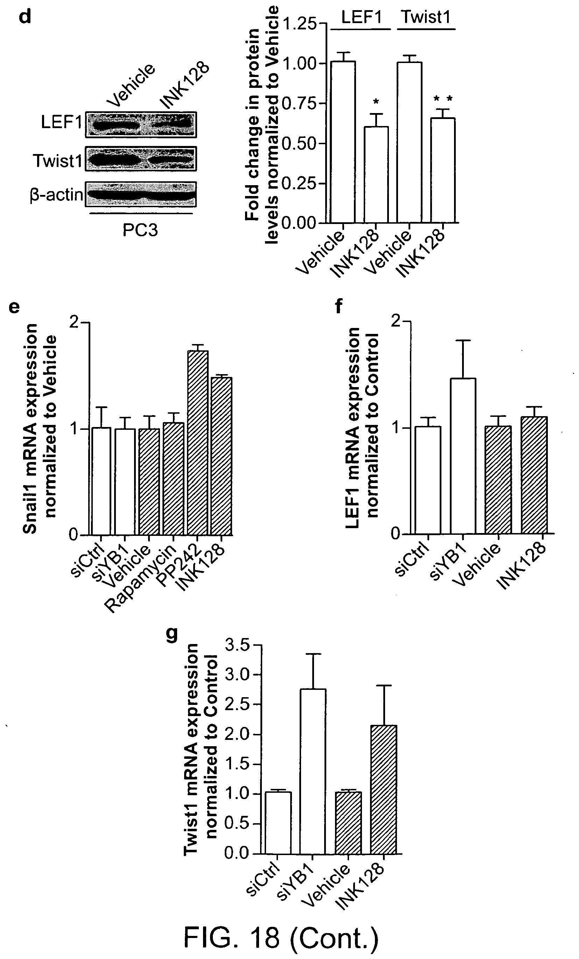

**Figure 18****. *YB1* knockdown and ATP site inhibition of mTOR decreases the protein levels but not mRNA levels of YB1 target genes.** (a) Snail1 immunofluorescence in PC3 cells after 48 hours of *YB1* gene silencing. Representative Snail1 immunofluorescence (top panels), box plot of Snail1 mean fluorescence intensity per cell (MFI)(n = 26 siCtrl cells, n = 15 siYB1 cells, *P = 0.001, t-test) (bottom panel). (b) Snail1 immunofluorescence in PC3 cells after treatment with rapamycin (50 nM), PP242 (2.5 µM), or ATP site inhibitor of mTOR (200 nM). Representative Snail1 immunofluorescence (left panel), box plot of Snail1 mean fluorescence intensity per cell (MFI) (n = 16 vehicle treated cells, n = 26 rapamycin treated cells, n = 28 PP242 treated cells, n = 27 ATP site inhibitor of mTOR treated cells, *P < 0.05, ANOVA) (right panel). (c) Representative Western blot (left panel) and quantification of protein levels (right panel) for LEF1 and Twist1 after *YB1* gene silencing (mean + SEM, n = 6, *P<0.05, t-test). (d) Representative Western blot (left panel) and quantification of protein levels (right panel) for LEF1 and Twist1 after ATP site inhibitor of mTOR treatment (mean + SEM, n = 6, *P<0.005, t-test). (e-g) *Snail1* (e), *LEF1* (f), or *Twist1* (g) mRNA expression normalized to β-actin after *YB1* gene knockdown or treatment with rapamycin (50 nM), PP242 (2.5 µM) or ATP site inhibitor of mTOR (200 nM) in PC3 cells (mean + SEM, n = 3).

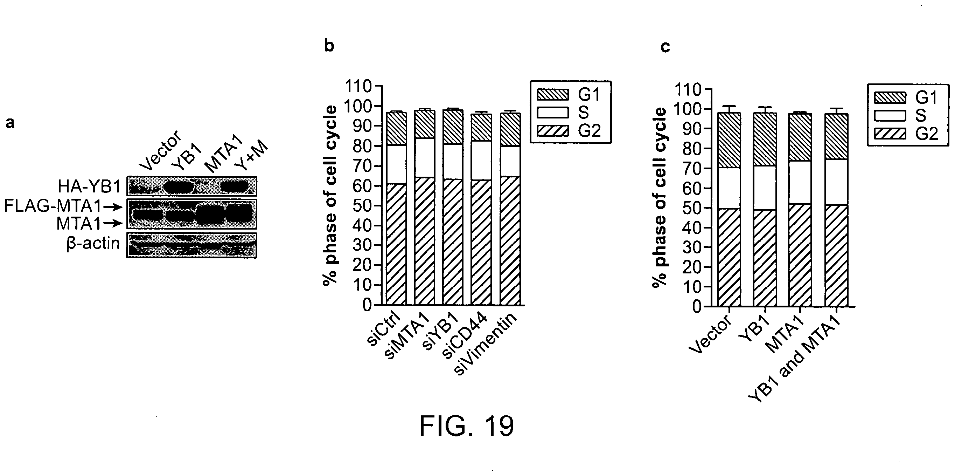

**Figure 19****. Effects of invasion gene knockdown or overexpression in PC3 and BPH-1 cells, respectively, on the cell cycle.** (a) HA-YB1 and Flag-MTAl protein levels after 48 hours of overexpression in non-transformed BPH-1 prostate epithelial cells (Y = YB1, M = MTA1). (b) Cell cycle analysis in PC3 cells after knockdown of respective genes (mean + SEM, n = 3). (c) Cell cycle analysis upon overexpression of YB1 and/or MTA1 in BPH-1 cells (mean + SEM, n = 3).

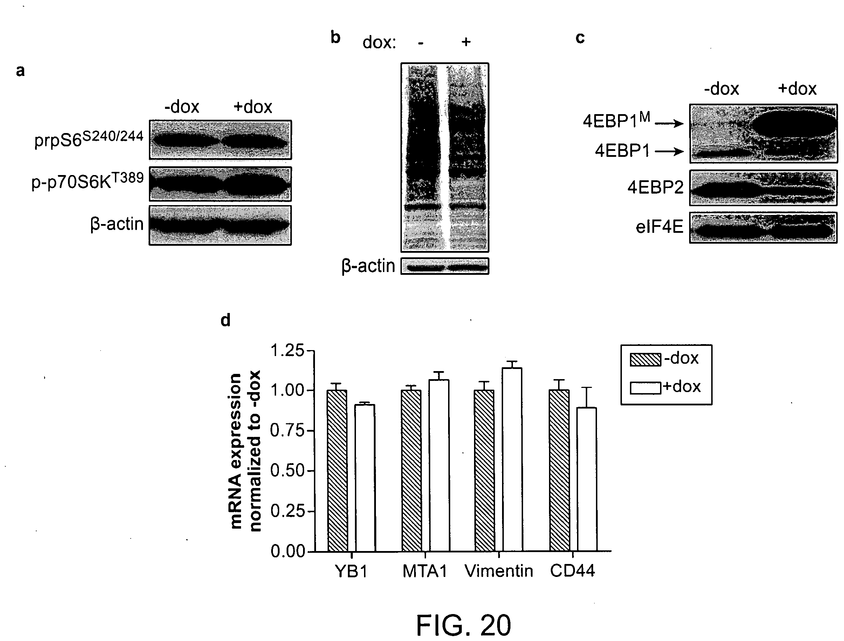

**Figure 20****. The 4EBP1^{M} does not augment mTORC1 function or global protein synthesis in PC3 cells.** (a) Representative Western blot from 3 independent experiments of phospho-p70S6K^{T389} and phospho-rpS6^{S240/244} after a 48-hour treatment with and without 1 µg/ml doxycycline in PC3-4EBP1^{M} cells. (b) Representative [³⁵S]-methionine incorporation from 2 independent experiments in PC3-4EBP1^{M} cells (48 hours, doxycycline 1 µg/mL) (mean + SEM). (c) Representative cap-binding assay from 2 independent experiments after 48-hour treatment with 1 µg/ml doxycycline in PC3-4EBP1^{M} cells. (d) mRNA expression of *YB1, MTA1,* Vimentin, and *CD44* relative to β-actin after 48-hour treatment with 1 µg/ml doxycycline in PC3-4EBP1^{M} cells (mean + SEM, n = 3).
**Figure 21****. The 4EBP/eIF4E axis imparts sensitivity to mTOR ATP site inhibition.** (a) Quantification of Western blots from 3 independent experiments of PC3 cells after 48 hours of *4EBP1*/*4EBP2* knockdown followed by 24-hour treatment with an ATP site inhibitor of mTOR (n = 3, *p<0.05, **p<0.01, ANOVA). (b) mRNA expression of *YB1, MTA1,* vimentin, and *CD44* relative to β-actin after 48 hours of gene silencing of *4EBP1* and *4EBP2* followed by a 24-hour treatment with an ATP site inhibitor of mTOR (200 nM) (mean + SEM, n = 3). (c) mRNA expression of *YB1, MTA1,* and *CD44* in WT and *4EBP1*/*4EBP2* DKO MEFs treated with 200 nM ATP site inhibitor of mTOR for 24 hours (mean + SEM, n = 3).

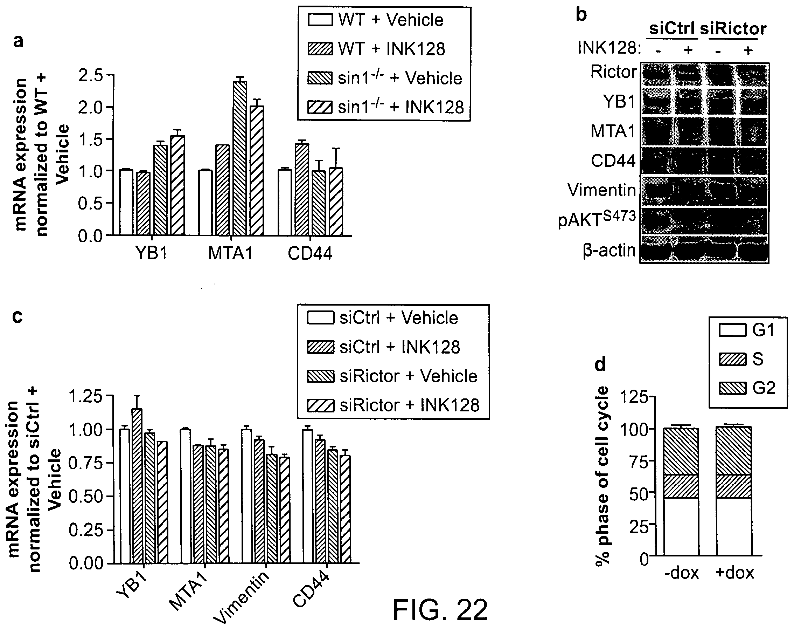

**Figure 22****. mTORC2 does not control the expression of the 4-gene invasion signature.** (a) mRNA expression of *YB1, MTA1,* and *CD44* relative to β-actin after a 24-hour treatment with ATP site inhibitor of mTOR (200 nM) in *mSin1*^{*-*/*-*} MEFs (mean + SEM, n = 3). (b) Representative Western blot analysis from 2 independent experiments of PC3 prostate cancer cells after 48 hours of *rictor* gene silencing followed by a 24-hour treatment with ATP site inhibitor of mTOR (200 nM). (c) mRNA expression of *YB1, MTA1,* vimentin, and *CD44* relative to β-actin in PC3 prostate cancer cells after 48 hours of *rictor* gene silencing followed by a 24-hour treatment with ATP site inhibitor of mTOR (200 nM) in PC3 (mean + SEM, n = 3). (d) Cell cycle analysis of PC3-4EBP1^{M} cells after treatment with 1 µg/ml doxycycline for 48 hours (mean + SEM, n = 3).

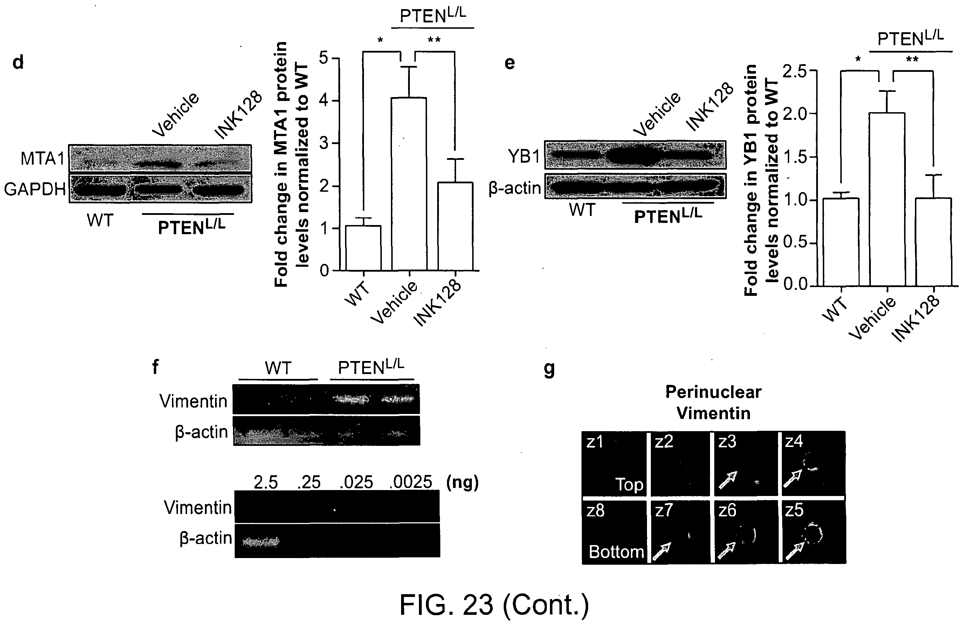

**Figure 23****. Complete mTOR inhibition decreases the expression of the 4-gene invasion signature at the level of translational control *in vivo* in *PTEN*^{*L*/*L*} mice.** (a) Validation of antibodies used for immunofluorescence after 48-hour gene silencing of respective genes in PC3 cells. (b) Number of individual CK5⁺ and/or CK8⁺ cells measured in 3 separate mice for mean fluorescence intensity of respective protein targets in WT and PTEN^{L/L}mouse prostates. (c) mRNA expression of *YB1, MTA1,* vimentin, and *CD44* relative to β-actin in WT and *PTEN*^{*L*/*L*} mice after 28 days of treatment with ATP site inhibitor of mTOR (1 mg/kg daily) (mean + SEM, n = 3 mice per arm). (d) Representative Western blot of MTA1 from whole prostate tissue in WT and *PTEN*^{*L*/*L*} mice after 28 days of treatment with ATP site inhibitor of mTOR (1 mg/kg daily) (left panel) and quantitation relative to β-actin protein levels (right panel) (mean + SEM, n = 3 mice per arm, *P=0.02, **P=0.04, t-test) (e) Representative Western blot of YB1 from whole prostate tissue in WT and *PTEN*^{*L*/}*^{L} mice* after 28 days of treatment with ATP site inhibitor of mTOR (1 mg/kg daily) (left panel) and quantitation relative to β-actin protein levels (right panel) (mean + SEM, n = 4 mice per arm, *P=0.002, **P=0.04, t-test) (f) Semi-quantitative RT-PCR of vimentin and β-actin for WT and *PTEN*^{*L*/*L*} FACS sorted murine prostate luminal epithelial cells (top panel). RT-PCR of a serial dilution of WT prostate luminal epithelial cell (bottom panel) (g) Z-series of perinuclear vimentin in a *PTEN*^{*L*/*L*} CK8⁺ prostate epithelial cell (red: vimentin; blue: DAPI; 0.4 µm per section; yellow arrows point to perinuclear vimentin).

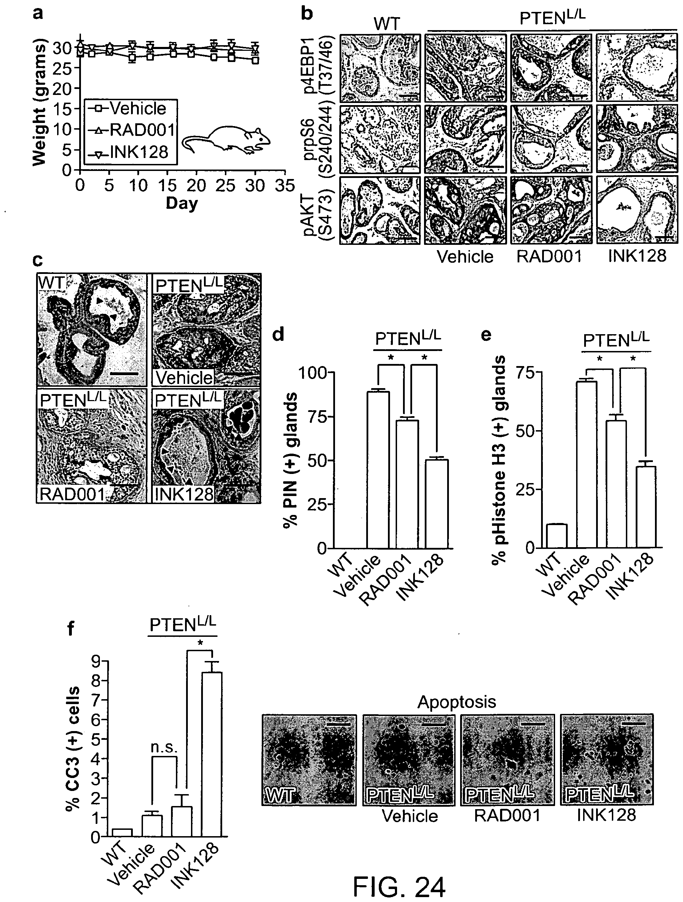

**Figure 24****. Preclinical efficacy of complete mTOR blockade *in vivo.*** (a) Mouse weights measured every 3 days over the course of the preclinical trial (mean + SEM, n = 3 mice per arm). (b) Representative phospho-specific immunohistochemistry of downstream mTOR targets in the ventral prostate (VP) of 9-month-old WT or *PTEN*^{*L*/*L*} mice after 28 days of treatment with ATP site inhibitor of mTOR (1 mg/kg daily) or RAD001 (10 mg/kg daily) (n = 6 mice per treatment arm). Scale bar = 100 µm. (c) Representative histology of 9-month-old WT or *PTEN*^{*L*/*L*} mice VP after 28 days of treatment with vehicle, RAD001 (10 mg/kg daily), or ATP site inhibitor of mTOR (1 mg/kg daily). Yellow dotted lines encircle prostate glands. Black triangles refer to prostatic secretions. Scale bar = 50 µm. (d) Quantification of PIN+ glands in treated mice (mean + SEM, n = 6 mice/arm, *P<0.001, ANOVA). (e) Proliferation measured by phospho-histone H3 positive glands in the prostates of 9-month-old WT or *PTEN*^{*L*/*L*} mice treated with RAD001 (10 mg/kg daily) or ATP site inhibitor of mTOR (1 mg/kg daily) (mean + SEM, n = 3 mice per arm, *P<0.01, ANOVA). (f) Apoptosis measured by cleaved caspase 3 (CC3) positive cells in the prostates of 9-month-old WT or *PTEN*^{*L*/*L*} mice treated with RAD001 (10 mg/kg daily) or ATP site inhibitor of mTOR (1 mg/kg daily) (mean + SEM, n = 3 mice per arm, *P<0.01, ANOVA) (left panel). Representative CC3 images (right panel). Scale bar = 25 µm.

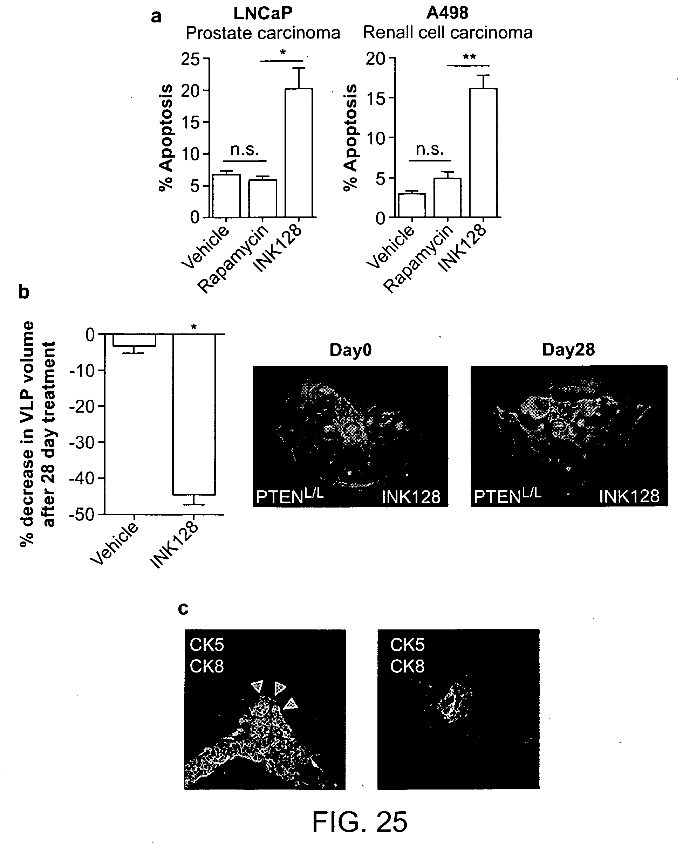

**Figure 25****. An ATP site inhibitor of mTOR induces apoptosis in specific cancer cell lines and decreases primary prostate cancer volume** ***in vivo.*** (a) Apoptosis in LNCaP (n = 3) and A498 (n = 2) cancer cells after treatment with rapamycin (50 nM), or an ATP site inhibitor of mTOR (200 nM) for 48 hours (mean + SEM, *P<0.001, **P<0.05, ANOVA, n.s. = not statistically significant). (b) Percentage decrease in ventral and lateral prostate volume in 9-month-old *PTEN*^{*L*/*L*} after a 28-day treatment with vehicle or the ATP site inhibitor of mTOR (1 mg/kg daily) measured by MRI (left panel) (mean + SEM, n = 4 mice per arm, *P = 0.0008, t-test). Representative MRI images of the *PTEN*^{*L*/*L*} ventral and lateral prostate on day 0 and day 28 of treatment with the ATP site inhibitor of mTOR (right panel) (red dotted lines encircle the ventral and lateral prostate). (c) Additional images of prostate cancer invasion in the *PTEN*^{*L*/*L*} prostate (14-month-old mouse).

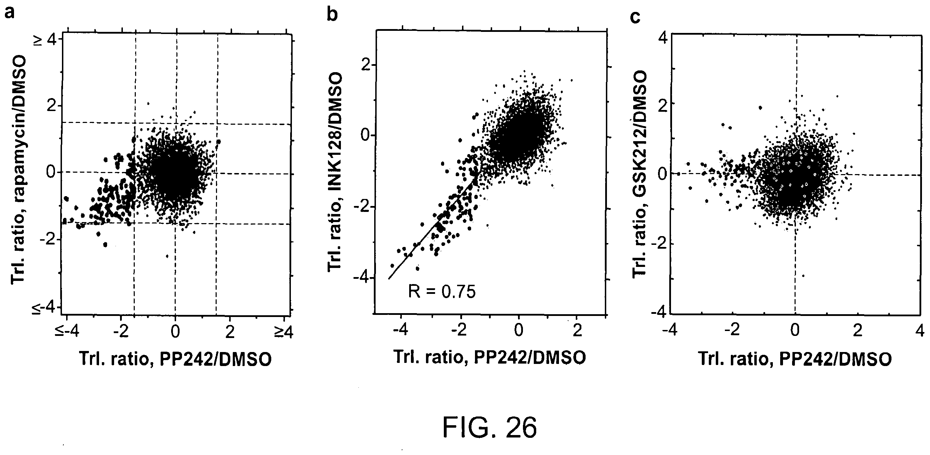

**Figure 26****. Two ATP site inhibitors of mTOR mimic effect on translational profiles.** These correlation plots provide a representative comparison of change in translational efficiency versus DMSO control by (a) the allosteric mTOR inhibitor rapamycin and the ATP site inhibitor PP242 in PC3 cells, (b) the two ATP site inhibitors INK128 and PP242, and (c) the MEK inhibitor GSK212 and mTOR ATP site inhibitor PP242 in SW620 cells. Each data point represents a single gene. In panels (a) and (b), data points highlighted in red have statistically significant changes in translational efficiency for PP242 versus versus DMSO control as described herein. In panel (c), the data points highlighted in red correspond to the 144 genes listed in Table 4 below. The allosteric inhibitor, rapamycin, affects the translational efficiency of similar genes affected by PP242, the magnitude of the rapamycin effect is substantially less than with PP242. In contrast, treatment with the two ATP site inhibitors (PP242 and INK128) alters the same gene set and at the same magnitude of change on a gene by gene basis. Finally, the MEK inhibitor GSK212 has little impact on the set of genes with translational efficiencies modulated by PP242.

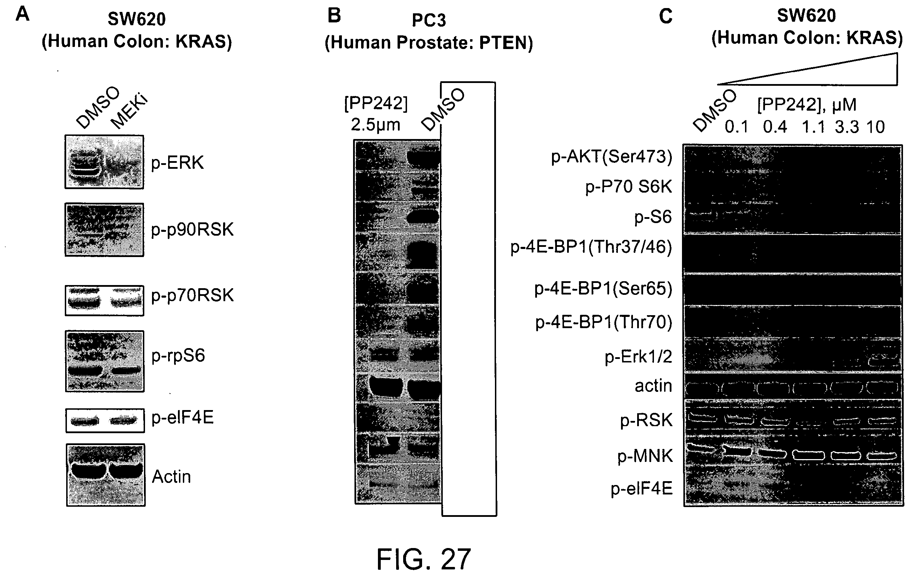

**Figure 27****. Effect of mTOR and MEK inhibitors on phosphorylation of protein translation components.** (a) SW620 cells were treated with DMSO or the MEK inhibitor GSK212 (250 nM) for 8 hrs; (b) PC3 and (c) SW620 cells were treated with DMSO or the mTOR inhibitor PP242 for 3 hrs. Actin was used as a loading control.

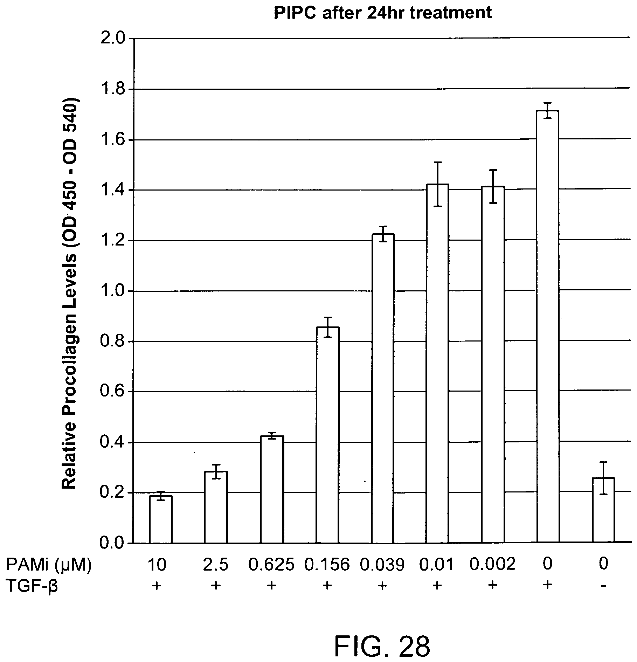

**Figure 28****. Induction of procollagen release from fibroblasts by TGF-β.** Procollagen Type 1 levels (Procollagen Type 1C-Peptide, "PIPC") after 24 hrs of treatment of fibroblasts with various concentrations of a PI3K/AKT/mTOR inhibitor ("PAMi") and 10ng/mL TGF-β. The difference in absorbance at 450 and 540 nm (y-axis) is proportional to the procollagen concentration.

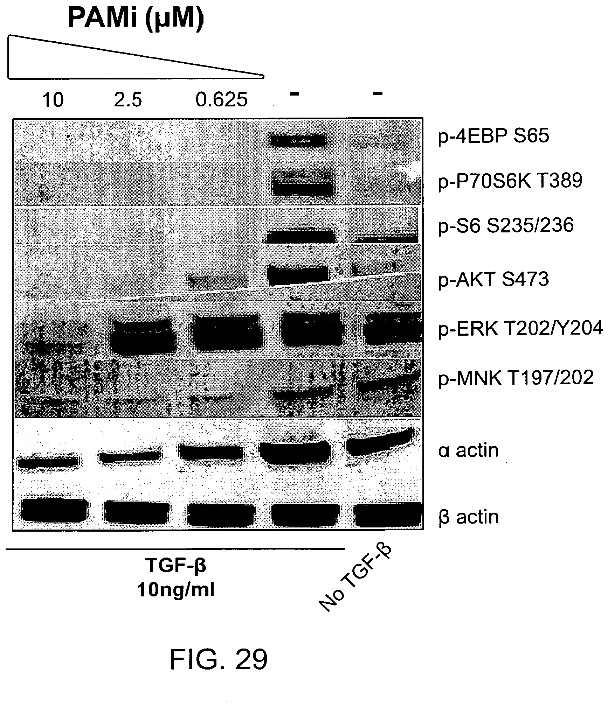

**Figure 29****. Western blot of protein phosphorylation levels during fibroblast transformation.** Western blot analysis of fibroblast transformation as monitored by α-SMA levels after 24hrs of treatment with various concentrations of a PAMi and 10ng/mL TGF-β.

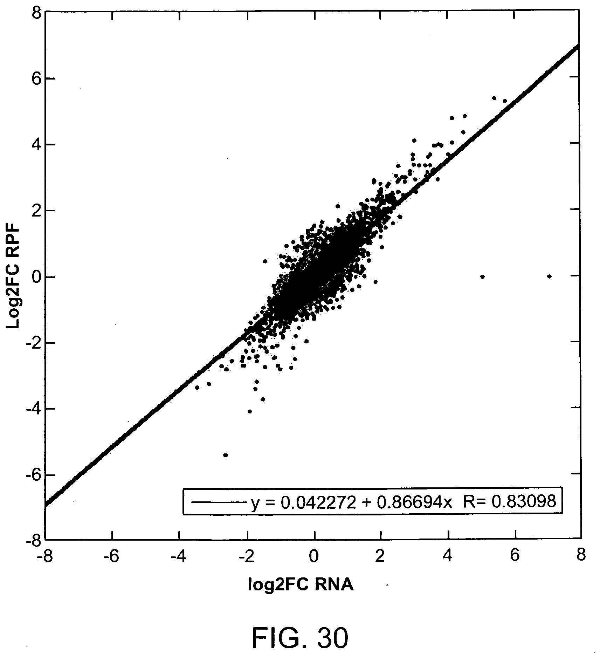

**Figure 30****. Translational and transcriptional profile of fibroblasts treated with TGF-β.** Comparison of changes in mRNA levels (RNA) and translational rate (RPF) in fibroblasts treated with TGF-β. Data points in red have p≤0.05 for changes in translational efficiency.

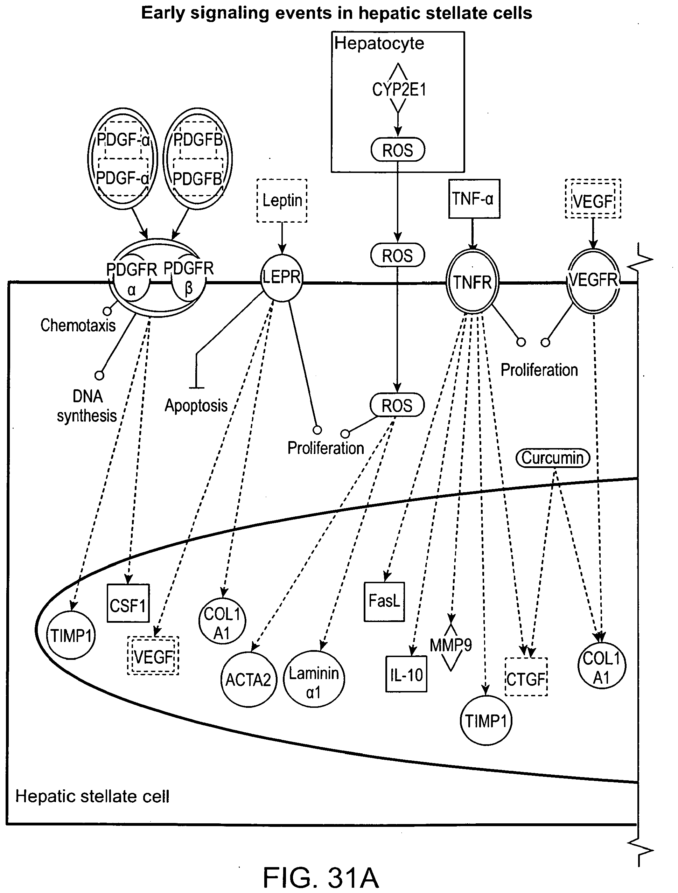

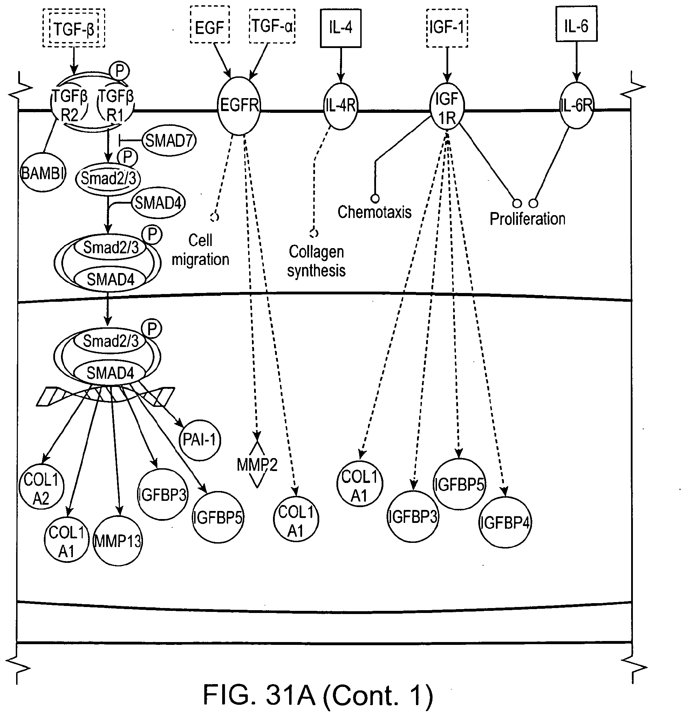

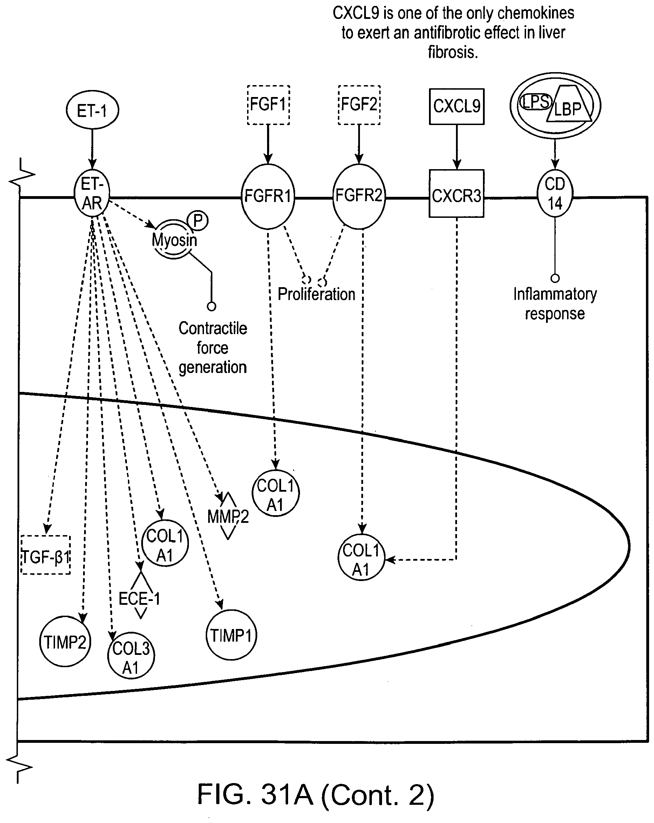

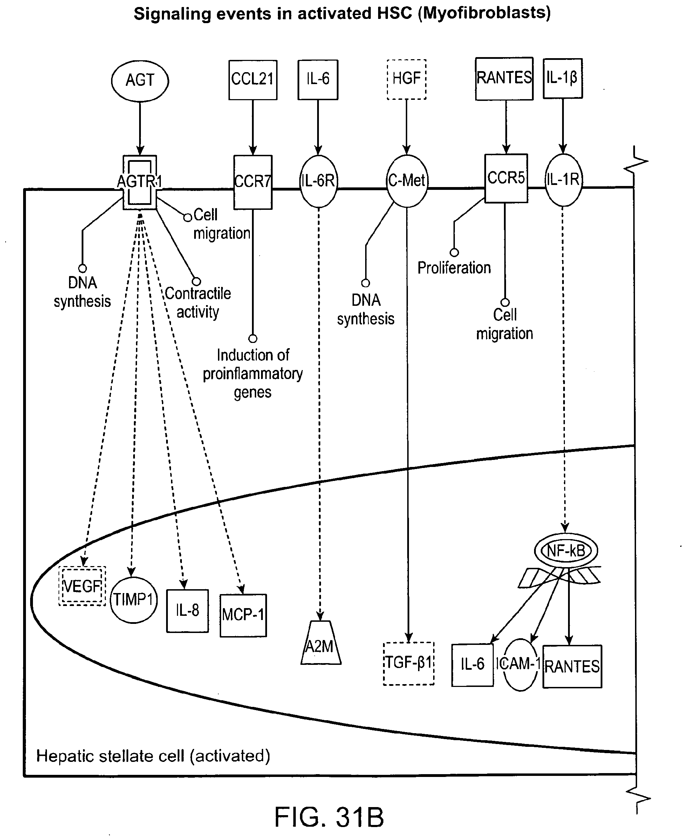

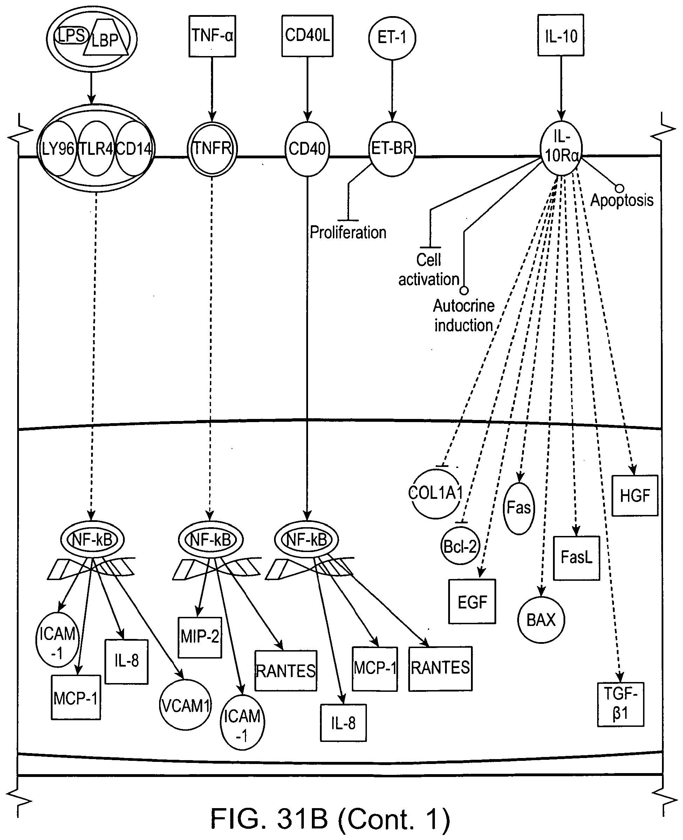

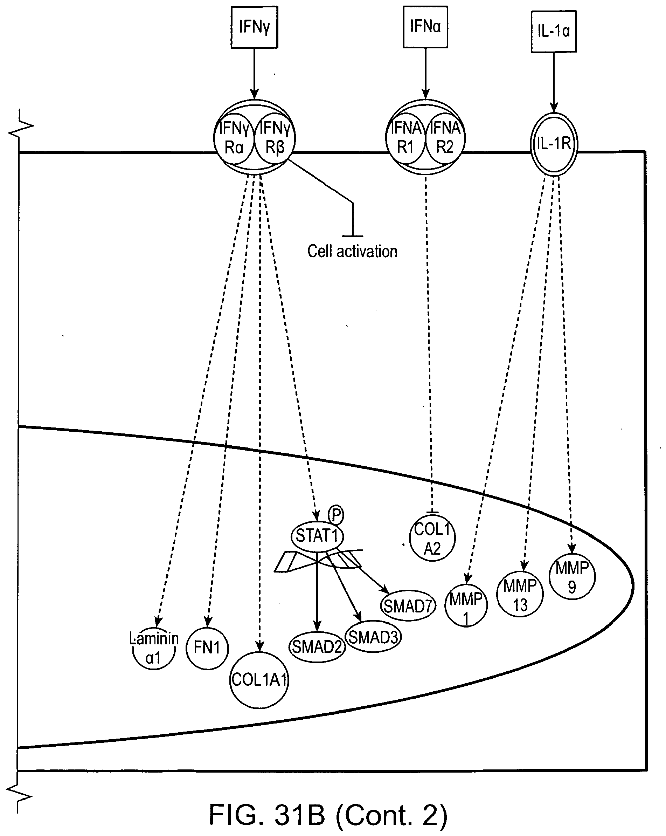

**Figure 31****. Hepatic Fibrosis/Hepatic Stellate cell activation from IPA pathway analysis.** (a) Early signaling events in hepatic stellate cells. (b) Signaling events in activated hepatic stellate cells. Gene list used and gene signature identified in analysis is based on p-value from differential concentrations of protein-coding mRNAs from control and TGF-β treated fibroblasts. Color coding is based on log₂ fold change.

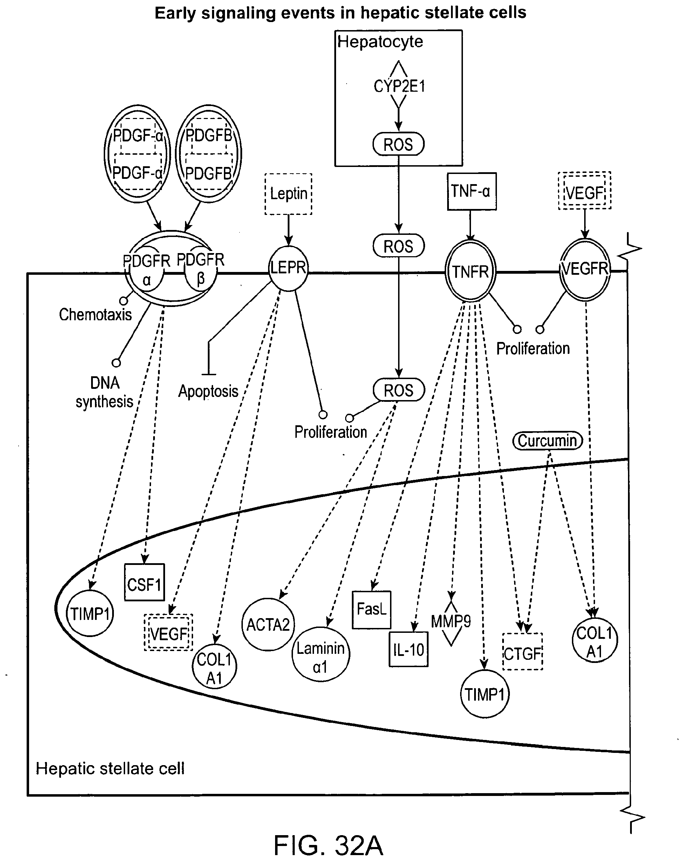

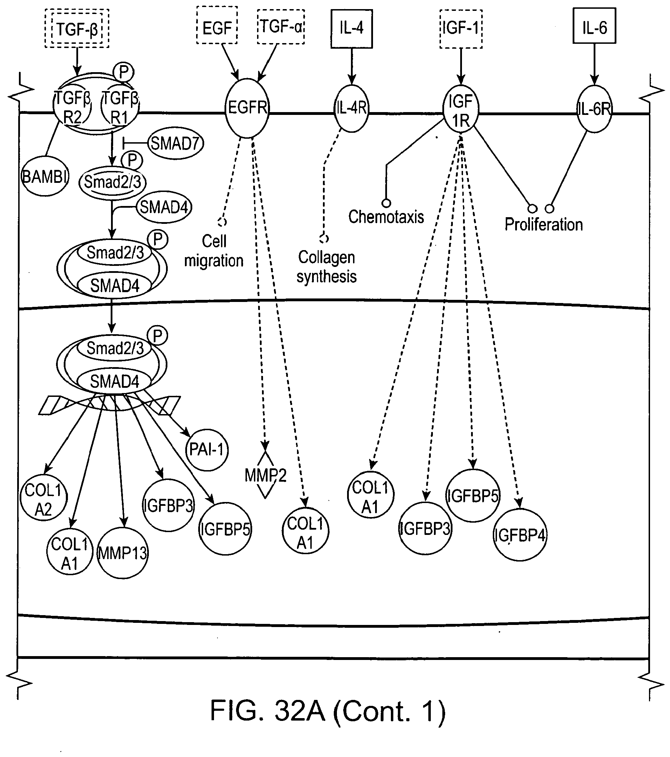

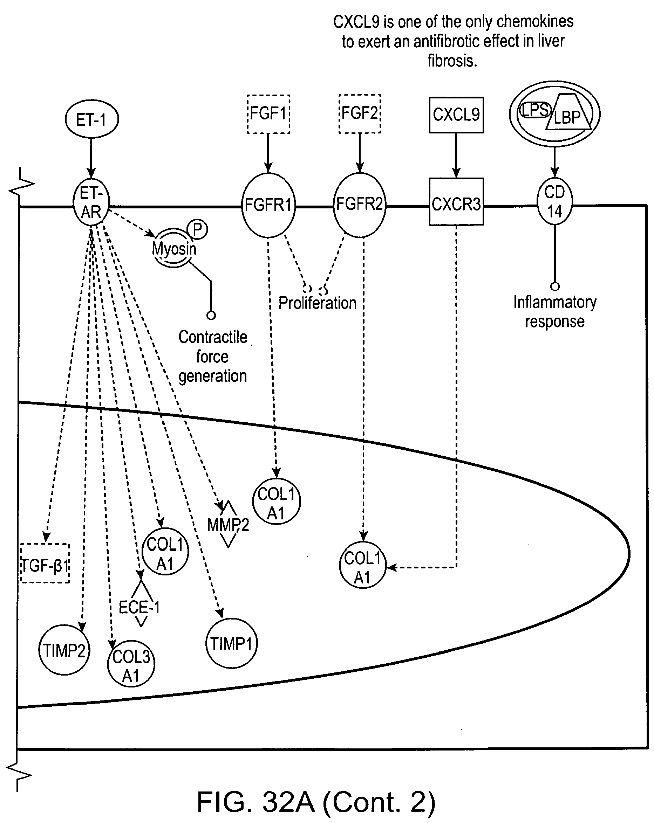

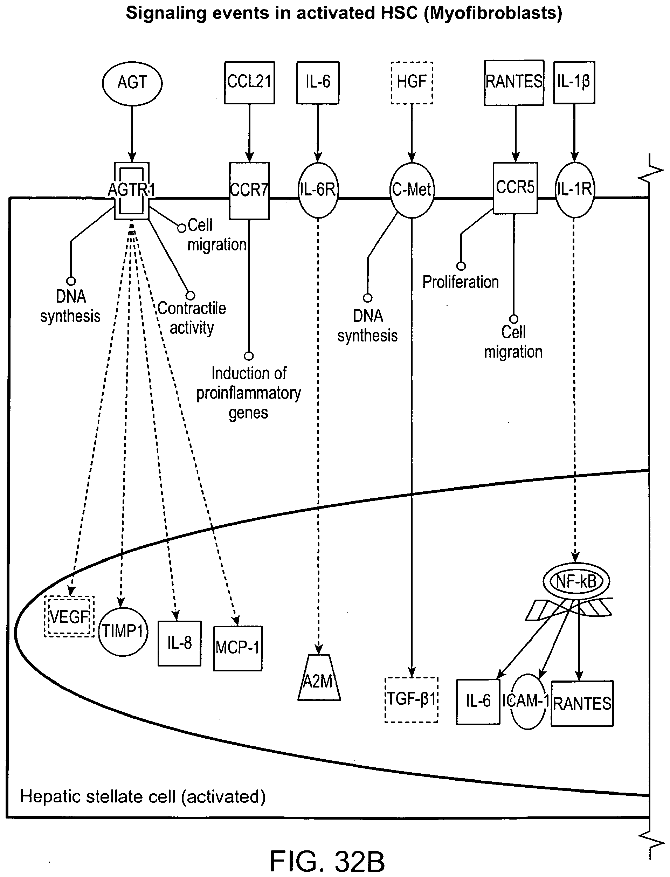

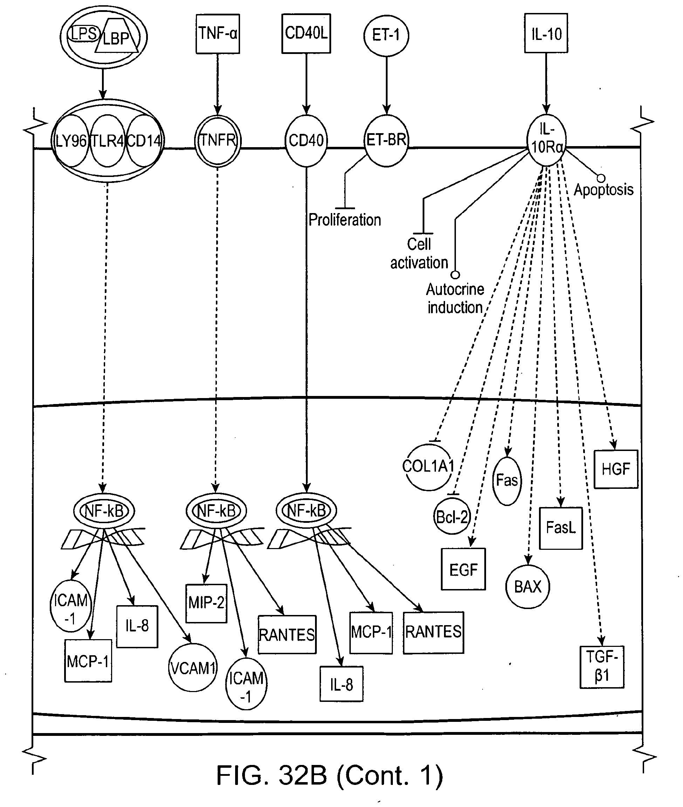

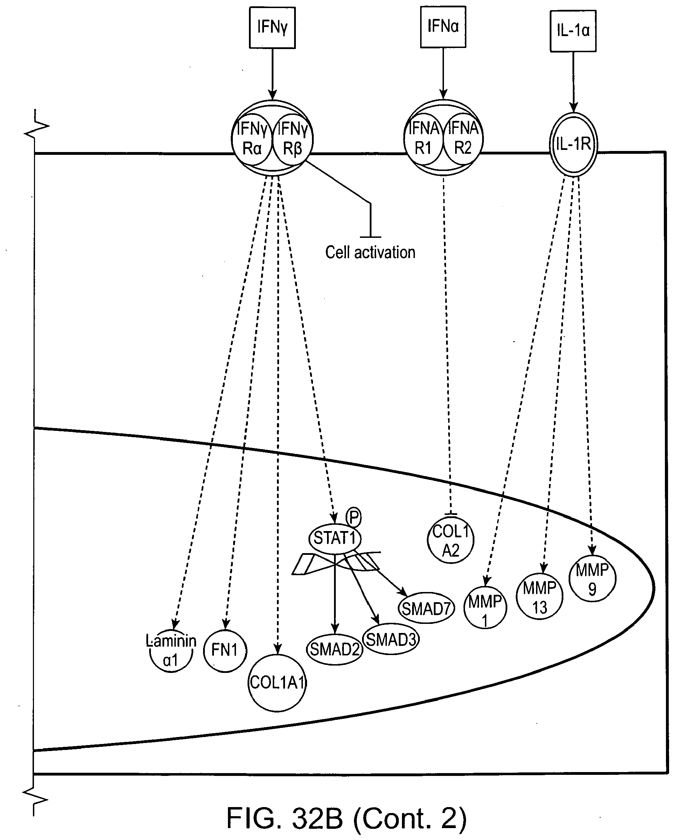

**Figure 32****. Hepatic Fibrosis/Hepatic Stellate cell activation from IPA pathway analysis.** (a) Early signaling events in hepatic stellate cells. (b) Signaling events in activated hepatic stellate cells. Gene list used and gene signature identified in analysis is based on p-value from differential translation rates from control and TGF-β treated fibroblasts. Color coding is based on log₂ fold change.

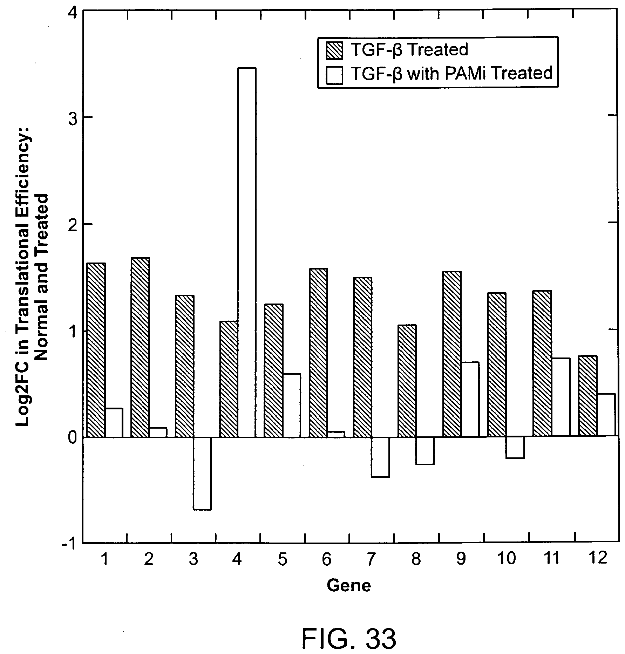

**Figure 33****. Normalization of translational efficiencies of fibrotic disorder-associated gene signature.** The bar graph shows the translational efficiencies of fibrotic disorder-associated gene signature in fibroblasts treated with TGF-β and fibroblasts treated with TGF-β and a PAMi. The normal translational efficiency is set at zero and the p-value upon TGF-β treatment was ≤ 0.05 for these genes having an altered translational efficiency.

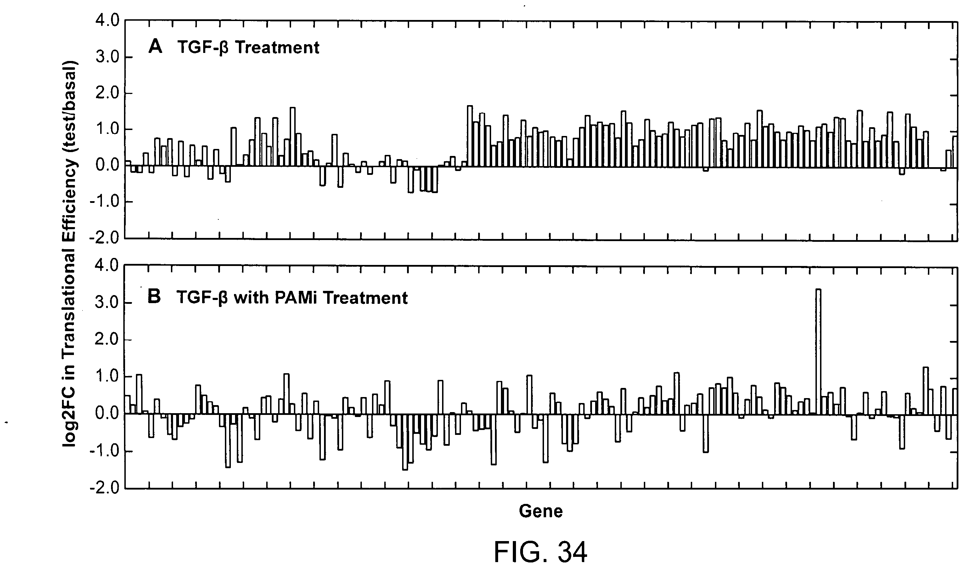

**Figure 34****. Translational profile of genes associated with fibrotic disorder.** The bar graph shows the translational efficiencies of all 141 fibrotic disorder-associated genes showing a differential translational profile in transforming fibroblasts (treated with TGF-β) as compared to untreated (normal) fibroblasts, and how treatment of transforming fibroblasts with a PAMi normalizes most genes (when compared to normal fibroblasts, set at zero). The p-value for change in translational efficiency upon TGF-β treatment was ≤ 0.05 for this gene signature.

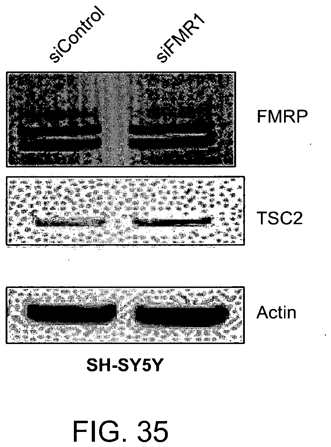

**Figure 35****. Translation levels of proteins associated with a neurodevelopmental disease model.** Western blot analysis of the protein levels of FMRP, TSC2 and β-actin after siRNA knockdown of the FMRP gene. SH-SY5Y cells were transfected with either siControl or siFMR1 at 100 nM for 3 days.

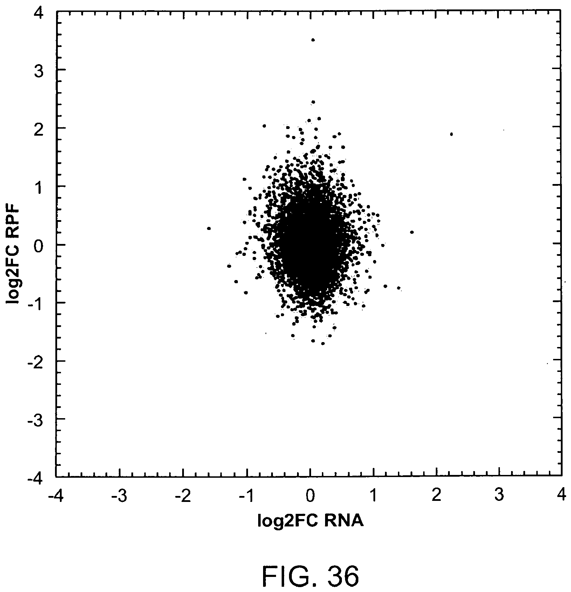

**Figure 36****. Ribosomal profile of a neurodevelopmental disease model.** Comparison of changes in mRNA levels (RNA) and translational rate (RPF) in SH-SY5Y neuronal cells transfected with either a control siRNA or test siFMR1. Data points in red have p≤0.05 for changes in translational efficiency.

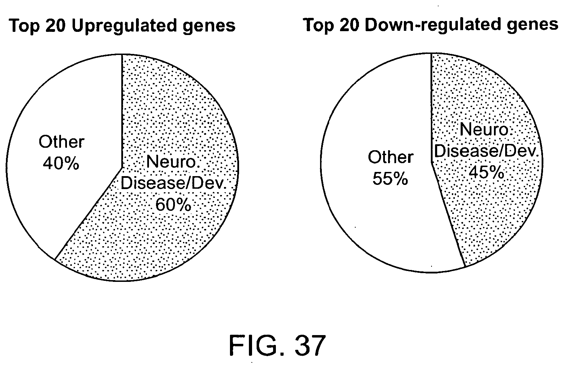

**Figure 37****. Top up and down translationally regulated genes in a neurodevelopmental disease model.** siRNA knockdown of the FMRP gene in SH-SY5Y cells with siFMR1 versus siCONTROL. The top 20 up- or down- differentially translationally regulated genes show a 60 or 45%, respectively, enrichment for association with neurological disease and development (p-value ≤0.05).

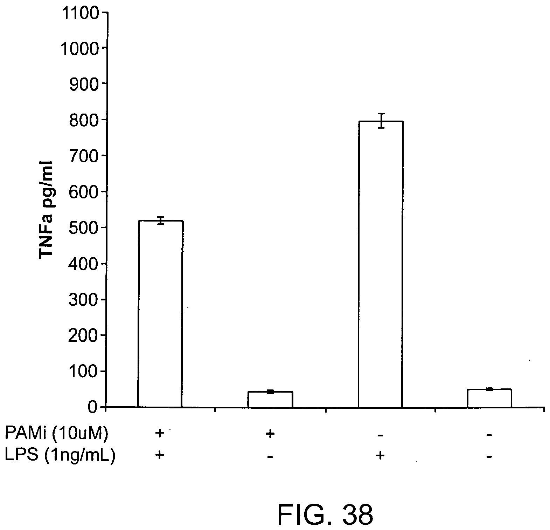

**Figure 38****. Effect of PI3K/AKT/mTOR inhibitors on TNF-α production during presence or absence of an induced inflammatory response.** RAW264.7 macrophages were pre-treated with PI3K/Atk/mTOR inhibitor PAMi (10 µM) or without PAMi for 2 hrs followed by challenge with or without LPS 1 ng/ml for an additional 1 hr. Culture media was collected and TNF-α levels were quantified.

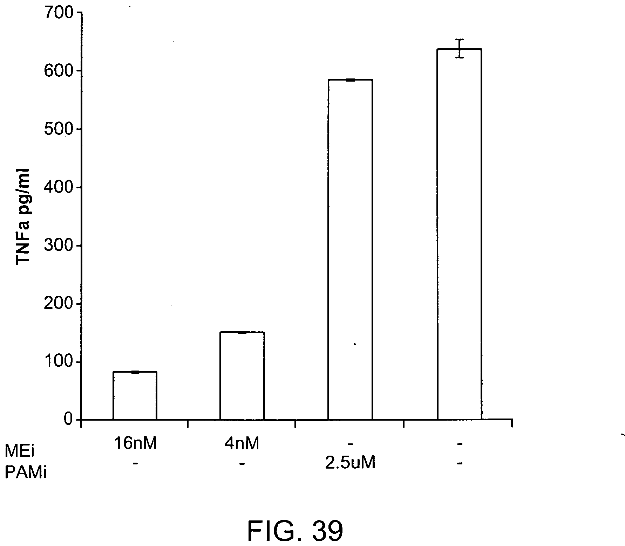

**Figure 39****. Effect of MEK/ERK and PI3K/AKT/mTOR inhibitors on TNF-α production during an induced inflammatory response.** RAW264.7 macrophages were pre-treated with a MEK/ERK pathway inhibitor ("MEi") (16 nM or 4 nM) or PAMi (2.5µM) for 2 hrs followed by challenge with LPS 1 ng/ml for an additional 1 hr. Culture media was collected and TNF-α levels quantified.

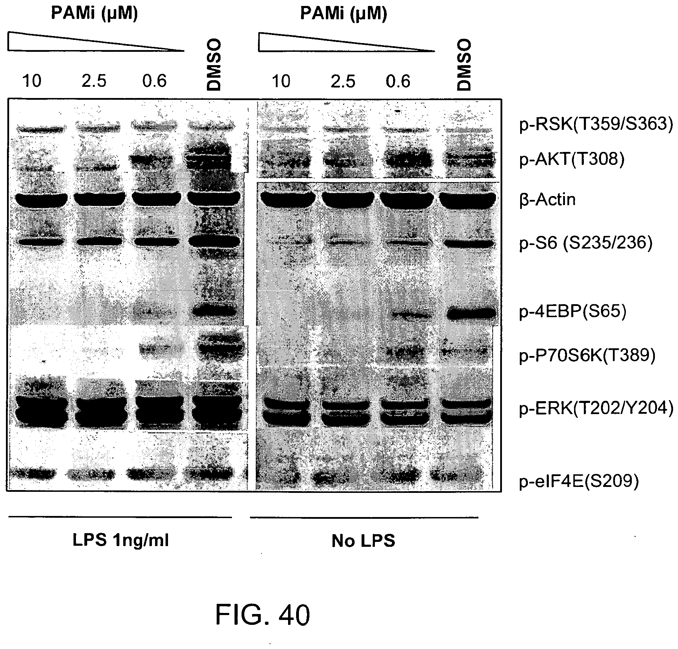

**Figure 40****. Dose-dependent effect of PI3K/AKT/mTOR inhibitor on protein translation components in the presence or absence of induced inflammatory response.** RAW264.7 macrophages were pre-treated with PAMi (10, 2.5, or 0.6 µM) or without PAMi for 2 hrs followed by challenge with or without LPS 1 ng/ml for an additional 1 hr. Culture media was collected and TNF-α levels quantified. Actin was used as a loading control.

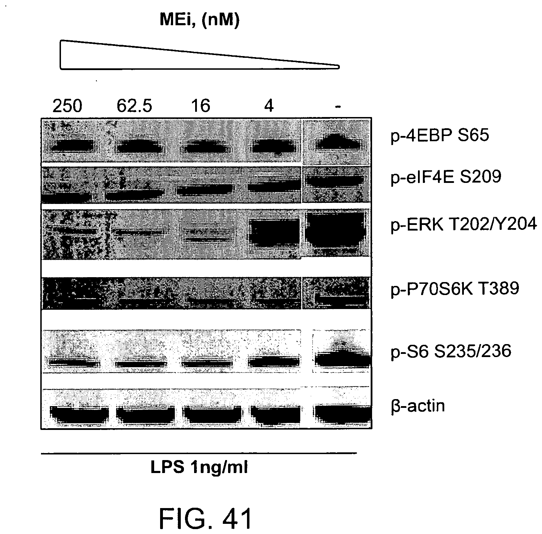

**Figure 41****. Effect of MEK inhibitor on various protein translation components during induced inflammatory response.** Western blot analysis of RAW264.7 macrophages pre-treated with MEi (250, 62.5, 16, or 4 nM), for 2 hrs followed by challenge with LPS (1 ng/ml) for an additional hour. Actin was used as a loading control.

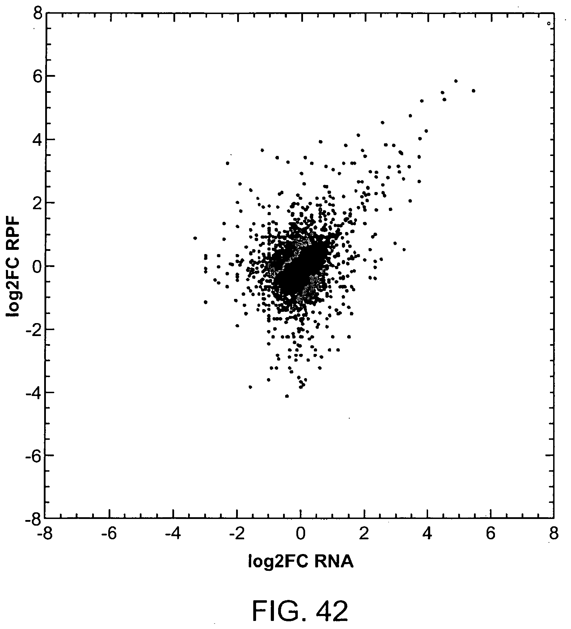

**Figure 42****. Translational and transcriptional profile of macrophages treated with LPS.** Comparison of changes in mRNA levels (RNA) and translational rate (RPF) in macrophages treated with LPS. Data points in red have p≤0.05 for changes in translational efficiency.

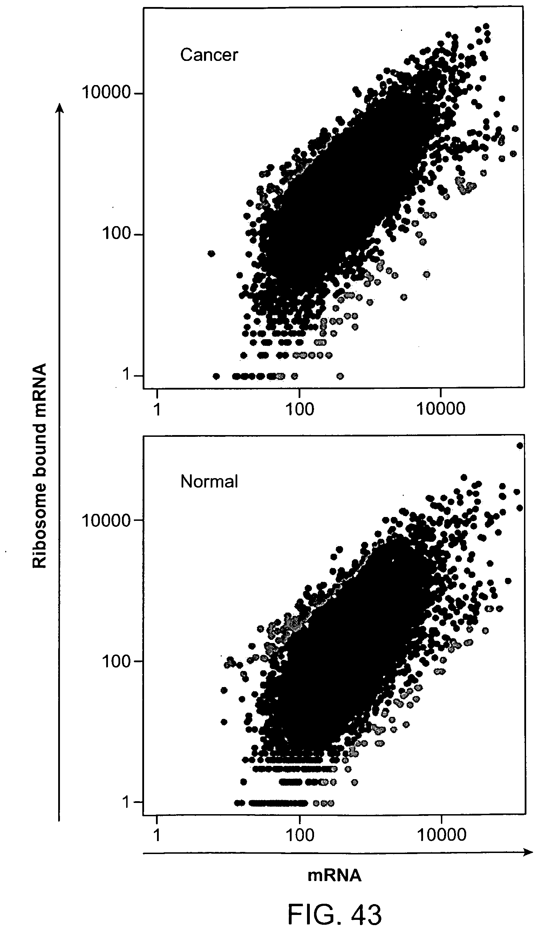

**Figure 43****. Translational profile of genes from primary tissue.** These correlation plots show the translational efficiencies of genes in a healthy (normal) section of prostate tissue (lower panel) and a section of cancer prostate tissue (upper panel) from the same patient.

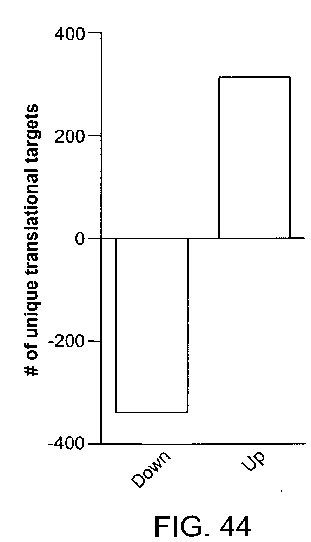

**Figure 44****. Translational efficiency of genes from primary tissue.** This bar graph shows the number of translationally regulated (up and down) mRNA targets in healthy versus cancer prostate tissue.

### DETAILED DESCRIPTION OF THE INVENTION

### I. Introduction

The present disclosure relates to methods of characterizing potential therapeutic agents and validating therapeutic targets using translational profiles from a biological sample. In some aspects, the methods of the present disclosure provide a genome-wide characterization of translationally controlled mRNAs downstream of biological pathways (*e.g*., oncogenic signaling pathways such as the mTOR pathway). The translational profiles that are generated can be used in identifying agents that modulate the biological pathway or in identifying or validating targets for therapeutic intervention.

### II. Definitions

As used herein, the term "translational profile" refers to the amount of protein that is translated (*i.e*., translational level) for each gene in a given set of genes in a biological sample, collectively representing a set of individual translational rate values, translational efficiency values, or both translational rate and translational efficiency values for each of one or more genes in a given set of genes. In the invention, a translational profile comprises translational levels for a plurality of genes in a biological sample (*e.g*., in a cell), *e.g.,* for at least about 2, 3, 4, 5, 6, 7, 8, 9, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, 10,000 genes or more, or for at least about 1%, 2%, 3%, 4%, 5%, 6%, 7%, 8%, 9%, 10%, 15%, 20%, 25% of all genes in the sample or more. In some embodiments, a translational profile comprises a genome-wide measurement of translational levels in a biological sample. In certain embodiments, a translational profile refers to a quantitative measure of the amount of mRNA associated with one or more ribosomes for each gene (*i.e*., translational rate, efficiency or both) in a given set of genes in a biological sample, wherein the amount of ribosome-associated mRNA correlates to the amount of protein that is translated (*i.e*., translational level).

As used herein, "translation rate" or "rate of translation" or "translational rate" refers to the total count of ribosome engagement, association or occupancy of mRNA for a particular gene as compared to the total count of ribosome engagement, association or occupancy of mRNA for at least one other gene or set of genes, wherein the count of total ribosomal occupancy correlates to the level of protein synthesis. Examination of translation rate across individual genes may be quantitative or qualitative, which will reveal differences in translation. In certain embodiments, translational rate provides a measure of protein synthesis for one or more genes, a plurality of genes, or across an entire genome. In particular embodiments, a translation rate is the amount of mRNA fragments protected by ribosomes for a particular gene relative to the amount of mRNA fragments protected by ribosomes for one or more other genes or groups of genes. For example, the mRNA fragments protected by ribosomes may correspond to a portion of the 5'-untranslated region, a portion of the coding region, a portion of a splice variant coding region, or combinations thereof. In further embodiments, the translation rate is a measure of one, a plurality or all mRNA variants of a particular gene. Translation rates can be established for one or more selected genes or groups of genes within a single composition (*e.g*., biological sample), between different compositions, or between a composition that has been split into at least two portions and each portion exposed to different conditions.

As used herein, "mRNA level" refers to the amount, abundance, or concentration of mRNA or portions thereof for a particular gene in a composition (*e.g*., biological sample). In certain embodiments, mRNA level refers to a count of one form, a plurality of forms or all forms of mRNA for a particular gene, including pre-mRNA, mature mRNA, or splice variants thereof. In particular embodiments, an mRNA level for one or more genes or groups of genes corresponds to counts of unique mRNA sequences or portions thereof for a particular gene that map to a 5'-untranslated region, a coding region, a splice variant coding region, or any combination thereof.

As used herein, "translation efficiency" or "translational efficiency" refers to the ratio of the translation rate for a particular gene to the mRNA level for a particular gene in a given set of genes. For example, gene X may produce an equal abundance of mRNA (*i.e*., same or similar mRNA level) in normal and diseased tissue, but the amount of protein X produced may be greater in diseased tissue as compared to normal tissue. In this situation, the message for gene X is more efficiently translated in diseased tissue than in normal tissue (*i.e*., an increased translation rate without an increase in mRNA level). In another example, gene Y may produce half the mRNA level in normal tissue as compared to diseased tissue, and the amount of protein Y produced in normal tissue is half the amount of protein Y produced in diseased tissue. In this second situation, the message for gene Y is translated equally efficiently in normal and diseased tissue (*i.e*., a change in translation rate in diseased tissue that is proportional to the increase in mRNA level and, therefore, the translational efficiency is unchanged). In other words, the expression of gene X is altered at the translational level, while gene Y is altered at the transcriptional level. In certain situations, both the amount of mRNA and protein may change such that mRNA abundance (transcription), translation rate, translation efficiency, or a combination thereof is altered relative to a particular reference or standard.

In certain embodiments, translational efficiency may be standardized by measuring a ratio of ribosome-associated mRNA read density (*i.e*., translation level) to mRNA abundance read density (*i.e*., transcription level) for a particular gene (*see*, Example 6 in the Examples section below). As used herein, "read density" is a measure of mRNA abundance and protein synthesis (*e.g*., ribosome profiling reads) for a particular gene, wherein at least 5, 10, 15, 20, 25, 50, 100, 150, 175, 200, 225, 250, 300 reads or more per unique mRNA or portion thereof is performed in relevant samples to obtain single-gene quantification for one or more treatment conditions. In certain embodiments, translational efficiency is scaled to standardize or normalize the translational efficiency of a median gene to 1.0 after excluding regulated genes (*e.g*., log₂ fold-change ±1.5 after normalizing for the all-gene median), which corrects for differences in the absolute number of sequencing reads obtained for different libraries. In further embodiments, changes in protein synthesis, mRNA abundance and translational efficiency are similarly computed as the ratio of read densities between different samples and normalized to give a median gene a ratio of 1.0, normalized to the mean, normalized to the mean or median of log values, or the like.

As used herein, "gene signature" or "gene cluster" refers to a plurality of genes that exhibit a generally coherent, systematic, coordinated, unified, collective, congruent, or signature expression pattern or translation efficiency. In certain embodiments, a gene signature is a plurality of genes that together comprise at least a detectable or identifiable portion of a biological pathway (*e.g*., 2, 3, 4, 5, or more genes; a cell invasion signature comprising 4 genes is illustrated in Figure 15), comprise a complete set of genes associated with a biological pathway, or comprise a cluster or grouping of independent genes having a recognized pattern of expression (*e.g*., response to a known drug or active compound; related to a disease state such as a cancer, an inflammatory disease, an autoimmune disease, a fibrotic disorder, a neurodegenerative disease, a neurodevelopmental disease, a viral infection). One or more genes from a particular gene signature may be part of a different gene signature (*e.g.*, a cell migration pathway may share a gene with a cell adhesion pathway) - that is, gene signatures may intersect or overlap but each signature can still be independently defined by its unique translation profile.

As used herein, the term "agent" refers to any molecule, either naturally occurring or synthetic, *e.g.,* peptide, protein, oligopeptide (*e.g*., from about 5 to about 25 amino acids in length, preferably from about 10 to 20 or 12 to 18 amino acids in length, preferably 12, 15, or 18 amino acids in length), small organic molecule (*e.g.,* an organic molecule having a molecular weight of less than about 2500 daltons, *e.g.,* less than 2000, less than 1000, or less than 500 daltons), circular peptide, peptidomimetic, antibody, polysaccharide, lipid, fatty acid, inhibitory RNA (*e.g*., siRNA or shRNA), polynucleotide, oligonucleotide, aptamer, drug compound, or other compound.

The terms "polypeptide," "peptide," and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, *e.g*., hydroxyproline, γ-carboxyglutamate, and O-phosphoserine. Amino acid analogs refers to compounds that have the same basic chemical structure as a naturally occurring amino acid, i.e., an α-carbon that is bound to a hydrogen, a carboxyl group, an amino group, and an R group, *e.g.*, homoserine, norleucine, methionine sulfoxide, methionine methyl sulfonium. Such analogs have modified R groups (*e.g*., norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid.

"Nucleic acid" refers to deoxyribonucleotides or ribonucleotides and polymers thereof in either single- or double-stranded form, and complements thereof. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2'-O-methyl ribonucleotides, and peptide-nucleic acids (PNAs).

Unless otherwise indicated, a particular nucleic acid sequence also implicitly encompasses conservatively modified variants thereof (*e.g*., degenerate codon substitutions), complementary sequences, splice variants, and nucleic acid sequences encoding truncated forms of proteins, as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res., 19:5081 (1991); Ohtsuka et al., J. Biol. Chem., 260:2605-2608 (1985); Rossolini et al., Mol. Cell. Probes, 8:91-98 (1994)). The term nucleic acid is used interchangeably with gene, cDNA, mRNA, shRNA, siRNA, oligonucleotide, and polynucleotide.

The term "modulate" or "modulator," as used with reference to modulating an activity of a target gene or signaling pathway, refers to increasing (*e.g*., activating, facilitating, enhancing, agonizing, sensitizing, potentiating, or upregulating) or decreasing (*e.g*., preventing, blocking, inactivating, delaying activation, desensitizing, antagonizing, attenuating, or downregulating) the activity of the target gene or signaling pathway. In certain embodiments, a modulator alters a translational profile at the translational level (*i.e*., increases or decreases translation rate or translation efficiency or both as described herein), at the transcriptional level, or both. In some embodiments, a modulator increases the activity of the target gene or signaling pathway, *e.g*., by at least about 1-fold, 1.5-fold, 2-fold, 2.5-fold, 3-fold, 3.5-fold, 4-fold, 4.5-fold, 5-fold, 6-fold, 7-fold, 8-fold, 9-fold, 10-fold, 15-fold, 20-fold or more. In some embodiments, a modulator decreases the activity of the target gene or signaling pathway, *e.g*., by at least about 1-fold, 1.5-fold, 2-fold, 2.5-fold, 3-fold, 3.5-fold, 4-fold, 4.5-fold, 5-fold, 6-fold, 7-fold, 8-fold, 9-fold, 10-fold, 15-fold, 20-fold or more.

A "biological sample" includes blood and blood fractions or products (*e.g*., serum, plasma, platelets, red blood cells, and the like); sputum or saliva; kidney, lung, liver, heart, brain, nervous tissue, thyroid, eye, skeletal muscle, cartilage, or bone tissue; cultured cells, *e.g.,* primary cultures, explants, and transformed cells, stem cells, stool, urine, *etc.* Such biological samples (*e.g*., disease samples or normal samples) also include sections of tissues such as biopsy and autopsy samples, frozen sections taken for histologic purposes, and cells or other biological material used to model disease or to be representative of a pathogenic state (*e.g.,* TGF-β treated fibroblasts as a model system for fibrosis; LPS treatment of cells as a model system for inflammation, *etc.*). A biological sample is typically obtained from a "subject," *i.e*., a eukaryotic organism, most preferably a mammal such as a primate, *e.g.,* chimpanzee or human; cow; dog; cat; a rodent, *e.g*., guinea pig, rat, or mouse; rabbit; or a bird; reptile; or fish.

As used herein, the terms "administer," "administered," or "administering" refer to methods of delivering agents or compositions to the desired site of biological action. These methods include, but are not limited to, topical delivery, parenteral delivery, intravenous delivery, transdermal delivery, intradermal delivery, transmucosal delivery, intramuscular delivery, oral delivery, nasal delivery, colonical delivery, rectal delivery, intrathecal delivery, ocular delivery, otic delivery, intestinal delivery, or intraperitoneal delivery. Administration techniques that are optionally employed with the agents and methods described herein, include *e.g.,* as discussed in Goodman and Gilman, The Pharmacological Basis of Therapeutics, current ed.; Pergamon; and Remington's, Pharmaceutical Sciences (current edition), Mack Publishing Co., Easton, Pa.

As used herein, the term "normalize" or "normalizing" or "normalization" refers to adjusting the translational level (*i.e*., translational rate and/or translational efficiency) of one or more genes in a biological sample from a subject (*e.g*., a sample from a subject having a disease or condition) to a level that is more similar, closer to, or comparable to the translational level of those one or more genes in a control sample (*e.g*., a biological sample from a non-diseased tissue or subject). In certainaspects, normalization refers to modulation of one or more translational regulators or translational system components to adjust or shift the translational efficiency of one or more genes in a biological sample (*e.g*., diseased, abnormal or other biologically altered condition) to a translational efficiency that is more similar, closer to or comparable to the translational efficiency of those one or more genes in a non-diseased or normal control sample. In some aspects, normalization is evaluated by determining translational levels (*i.e*., translational rate and/or translational efficiency) of one or more genes in a biological sample from a subject (*e.g*., a sample from a subject having a disease or condition) before and after an agent (*e.g*., a therapeutic or known active agent) is administered to the subject and comparing the translational levels before and after administration to the translational levels from a control sample in the absence or presence of the agent. Exemplary methods of evaluating normalization of a translational profile associated with a disease or disorder includes identifying an agent, validating a target, or observing a shift in a gene signature. Further exemplary methods of normalization may be used for evaluating therapeutic intervention in a particular condition, disease or disorder.

As used herein, the term "undruggable target" refers to a gene, or a protein encoded by a gene, for which targeted therapy using a drug compound (*e.g*., a small molecule or antibody) does not successfully interfere with the biological function of the gene or protein encoded by the gene. Typically, an undruggable target is a protein that lacks a binding site for small molecules or for which binding of small molecules does not alter biological function (*e.g*., ribosomal proteins); a protein for which, despite having a small molecule binding site, successful targeting of said site has proven intractable in practice (*e.g*., GTP/GDP proteins); or a protein for which selectivity of small molecule binding has not been obtained due to close homology of the binding site with other proteins, and for which binding of the small molecule to these other proteins obviates the therapeutic benefit that is theoretically achievable with binding to the target protein (*e.g*., protein phosphatases). A target may be undruggable to antibody-based therapeutics for a variety of reasons, such as intracellular location of the target, masking of target antigenicity (*e.g*., due to modification with carbohydrate or other masking modifications) or to escape by competition (*e.g*., by shedding or release of decoy molecules).

In the present description, any concentration range, percentage range, ratio range, or integer range is to be understood to include the value of any integer within the recited range and, when appropriate, fractions thereof (such as one tenth and one hundredth of an integer), unless otherwise indicated. Also, any number range recited herein relating to any physical feature, such as polymer subunits, size or thickness, are to be understood to include any integer within the recited range, unless otherwise indicated. As used herein, the term "about" means ± 20% of the indicated range, value, or structure, unless otherwise indicated. It should be understood that the terms "a" and "an" as used herein refer to "one or more" of the enumerated components. The use of the alternative (*e.g*., "or") should be understood to mean either one, both, or any combination thereof of the alternatives. As used herein, the terms "include," "have" and "comprise" are used synonymously, which terms and variants thereof are intended to be construed as non-limiting.

Additional definitions are set forth throughout this disclosure.

### III. Translational Profiling

The present invention relates to the generation and analysis of translational profiles. A translational profile provides information about the identity of genes being translated in a biological sample (*e.g*., a cell) and/or the amount of protein that is translated (*i.e*., translational level in the form of translational rate, translational efficiency, or both) for each gene in a given set of genes in the biological sample, thereby providing information about the translational landscape in that biological sample. In certain aspects, a translational profile is a biomarker, or comprises one or more biomarker genes, for a particular sample or condition.

In certain embodiments, a translational profile comprises one or more biologically meaningful groupings or clusters of genes, referred to as a "gene signature." For example, a translational profile may comprise a plurality of gene signatures (*e.g*., 2, 3, 4, 5, 6, 7, 8, 9, 10, or more). In still further embodiments, a translational profile comprises one or more gene signatures in combination with one more additional gene not associated with or part of such gene signatures. In any of the aforementioned embodiments, particular genes, gene signatures, groups of genes, groups of gene signatures or any combination thereof comprise a biomarker. In certain embodiments, a translational profile comprises one or more gene signatures or gene clusters, wherein the one or more gene signatures or gene clusters individually or in a particular combination are a biomarker.

The expression pattern of one or more genes in one (*e.g*., a first) translational profile may be altered by an agent, compound, molecule, drug, or the like. In some cases, a test agent, compound, molecule, drug, or the like may mimic the action of an active compound known to have a particular function or induce a particular biological effect or phenotypic change in a cell or a subject. In certain aspects, a test agent, compound, molecule, drug, or the like is identified as a mimic of a known active compound by causing a shift in the translational profile to be comparable or similar to the translational profile induced by the known active compound. In certain aspects, a known active compound causes a translational profile to be more comparable or similar to normal. In other aspects, a known active compound causes a translational profile to be more comparable or similar to a desired phenotype or effect, such as necrosis, apoptosis, or the like.

In the invention, a translational profile comprises translational levels for a plurality of genes in a biological sample, *e.g*., at least 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, 500, 550, 600, 650, 700, 750, 800, 850, 900, 950, 1000, 1500, 2000, 2500, 3000, 3500, 4000, 4500, 5000, 5500, 6000, 6500, 7000, 7500, 8000, 8500, 9000, 9500, 10,000 genes or more. In some aspects, a translational profile comprises translational levels for one or more genes of one or more biological pathways in a biological sample (*e.g*., pathways such as protein synthesis, cell invasion/metastasis, cell division, apoptosis pathway, signal transduction, cellular transport, post-translational protein modification, DNA repair, and DNA methylation pathways). In some embodiments, a translational profile comprises translational levels for a subset of the genome, *e.g*., for about 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50% of the genome or more. In some embodiments, a translational profile comprises a genome-wide measurement of translational levels.

### A. Biological Samples

In some embodiments, a biological sample comprises a cell. In some embodiments, the cell is derived from a tissue or organ (*e.g*., prostate, breast, kidney, lung, liver, heart, brain, nervous tissue, thyroid, eye, skeletal muscle, cartilage, skin, or bone tissue). In some embodiments, the cell is derived from a biological fluid, *e.g*., blood (*e.g*., an erythrocyte), lymph (*e.g*., a monocyte, macrophage, neutrophil, eosinophil, basophil, mast cell, T cell, B cell, and/or NK cell), serum, urine, sweat, tears, or saliva. In some embodiments, the cell is derived from a biopsy (*e.g*., a skin biopsy, a muscle biopsy, a bone marrow biopsy, a liver biopsy, a gastrointestinal biopsy, a lung biopsy, a nervous system biopsy, or a lymph node biopsy). In some aspects, the cell is derived from a cultured cell (*e.g*., a primary cell culture) or a cell line (*e.g*., PC3, HEK293T, NIH3T3, Jurkat, or Ramos). In some aspects, the cell is a stem cell or is derived (*e.g*., differentiated) from a stem cell. In some aspects, the cell is a cancer stem cell.

In some embodiments, the biological sample comprises a cancer cell (*e.g*., a cell obtained or derived from a tumor). In some embodiments, the cancer is prostate cancer, breast cancer, bladder cancer, urogenital cancer, lung cancer, renal cell carcinoma, endometrial cancer, melanoma, ovarian cancer, thyroid cancer, or brain cancer. In some embodiments, the cancer is a metastatic cancer.

In some embodiments, the biological sample is from a human subject. In some embodiments, the biological sample is from a non-human mammal (*e.g*., chimpanzee, dog, cat, pig, mouse, rat, sheep, goat, or horse), avian (*e.g*., pigeon, penguin, eagle, chicken, duck, or goose), reptile (*e.g*., snake, lizard, alligator, or turtle), amphibian (*e.g*., frog, toad, salamander, caecilian, or newt), or fish (*e.g*., shark, salmon, trout, or sturgeon).

### B. Generating Translational Profiles

Various techniques for quantitating translational levels for a given set of genes and generating a translational profile are known in the art and can be used according to the methods of the present disclosure. These techniques include, but are not limited to, ribosomal profiling, polysome microarray, immunoassay, and mass spectrometry analysis, each of which is detailed below.

### Ribosomal Profiling

In the invention, one or more translational profiles are generated by ribosomal profiling. Ribosomal profiling provides a quantitative assessment of translational levels in a sample and can be used to measure translational levels on a genome-wide scale. Generally, ribosomal profiling identifies and/or measures the mRNA associated with ribosomes. Ribosome footprinting is used to measure the density of ribosome occupancy on a given mRNA and to identify the position of active ribosomes on mRNA. Using nuclease digestion, the ribosome position and translated message can be determined by analyzing the approximately 30-nucleotide region that is protected by the ribosome. In some embodiments, ribosome-protected mRNA fragments are analyzed and quantitated by a high-throughput sequencing method. For example, in some embodiments the protected fragments are analyzed by microarray. In some embodiments, the protected fragments are analyzed by deep sequencing; *see, e.g.,* Bentley et al., Nature 456:53-59 (2008). Ribosomal profiling is described, for example, in US 2010/0120625; Ingolia et al., Science 324:218-223 (2009); and Ingolia et al., Nat Protoc 7:1534-1550 (2012).

Ribosome profiling can comprise methods for detecting a plurality of RNA molecules that are bound by at least one ribosome, wherein the plurality of RNA molecules are associated with ribosomes. In some embodiments, the ribosome profile is of a group of ribosomes, for instance from a polysome. In some embodiments, the ribosome profile is from a group of ribosomes from the same cell or population of cells. For example, in some embodiments, a ribosome profile of a tumor sample can be determined.

In some embodiments, the ribosomal profiling comprises detecting a plurality of RNA molecules bound to at least one ribosome, by (a) contacting the plurality of RNA molecules with an enzymatic degradant or a chemical degradant, thereby forming a plurality of RNA fragments, wherein each RNA fragment comprises an RNA portion protected from the enzymatic degradant or the chemical degradant by a ribosome to which the RNA portion is bound; (b) amplifying the RNA fragments to form a detectable number of amplified nucleic acid fragments; and (c) detecting the detectable number of amplified nucleic acid fragments, thereby detecting the plurality of RNA molecules bound to at least one ribosome.

In some embodiments, nucleic acid fragments (*e.g*., mRNA fragments) are detected and/or analyzed by deep sequencing. Deep sequencing enables the simultaneous sequencing of multiple fragments, *e.g*., simultaneous sequencing of at least 500, 1000, 1500, 2000 fragments or more. In a typical deep sequencing protocol, nucleic acids (*e.g*., mRNA fragments) are attached to the surface of a reaction platform (*e.g*., flow cell, microarray, and the like). The attached DNA molecules may be amplified *in situ* and used as templates for synthetic sequencing (*i.e*., sequencing by synthesis) using a detectable label (*e.g*., a fluorescent reversible terminator deoxyribonucleotide). Representative reversible terminator deoxyribonucleotides may include 3'-O-azidomethyl-2'-deoxynucleoside triphosphates of adenine, cytosine, guanine and thymine, each labeled with a different recognizable and removable fluorophore, optionally attached via a linker. Where fluorescent tags are employed, after each cycle of incorporation, the identity of the inserted bases may be determined by excitation (*e.g*., laser-induced excitation) of the fluorophores and imaging of the resulting immobilized growing duplex nucleic acid. The fluorophore, and optionally linker, may be removed by methods known in the art, thereby regenerating a 3' hydroxyl group ready for the next cycle of nucleotide addition. In some embodiments, the ribsome-protected mRNA fragments are detected and/or analyzed by a sequencing method described in US 2010/0120625.

### Polysome Microarray

In some aspects, one or more translational profiles are generated by polysome microarray. In a polysome microarray, mRNA is isolated and separated based on the number of associated ribosomes. Fractions of mRNA associated with several ribosomes are pooled to form a translationally active pool and are compared to cytosolic mRNA levels. Polysome microarray methods are described, for example, in Melamed and Arava, Methods in Enzymology, 431:177-201 (2007); and Larsson and Nadon, Biotech and Genet Eng Rev, 25:77-92 (2008).

In some aspects, polysome fractions having mRNA associated with multiple ribosomes (*e.g*., 3, 4, 5, 10 or more ribosomes) are pooled from a biological sample and RNA is isolated and labeled. The RNA samples from the translationally active pool are hybridized to a microarray with a control RNA sample (*e.g*., an unfractionated RNA sample). Ratios of polysome-to-free RNA are generated for each gene in the microarray to determine the relative levels of ribosomal association for each of the genes.

### Immunoassay

In some aspects, one or more translational profiles are generated by immunoassay. Immunoassay techniques and protocols are generally described in Price and Newman, "Principles and Practice of Immunoassay," 2nd Edition, Grove's Dictionaries, 1997; and Gosling, "Immunoassays: A Practical Approach," Oxford University Press, 2000. A variety of immunoassay techniques, including competitive and non-competitive immunoassays, can be used. *See, e.g*., Self et al., Curr. Opin. Biotechnol., 7:60-65 (1996). The term immunoassay encompasses techniques including, without limitation, enzyme immunoassays (EIA) such as enzyme multiplied immunoassay technique (EMIT), enzyme-linked immunosorbent assay (ELISA), IgM antibody capture ELISA (MAC ELISA), and microparticle enzyme immunoassay (MEIA); capillary electrophoresis immunoassays (CEIA); radioimmunoassays (RIA); immunoradiometric assays (IRMA); fluorescence polarization immunoassays (FPIA); and chemiluminescence assays (CL). If desired, such immunoassays can be automated. Immunoassays can also be used in conjunction with laser induced fluorescence. *See, e.g*., Schmalzing et al., Electrophoresis, 18:2184-93 (1997); Bao, J. Chromatogr. B. Biomed. Sci., 699:463-80 (1997).

A detectable moiety can be used in the assays described herein. A wide variety of detectable moieties can be used, with the choice of label depending on the sensitivity required, ease of conjugation with the antibody, stability requirements, and available instrumentation and disposal provisions. Suitable detectable moieties include, but are not limited to, radionuclides, fluorescent dyes (*e.g.*, fluorescein, fluorescein isothiocyanate (FITC), Oregon Green™, rhodamine, Texas red, tetrarhodimine isothiocynate (TRITC), Cy3, Cy5, *etc.*), fluorescent markers (*e.g*., green fluorescent protein (GFP), phycoerythrin, *etc.*), autoquenched fluorescent compounds that are activated by tumor-associated proteases, enzymes (*e.g*., luciferase, horseradish peroxidase, alkaline phosphatase, *etc*.), nanoparticles, biotin, digoxigenin, and the like.

Useful physical formats comprise surfaces having a plurality of discrete, addressable locations for the detection of a plurality of different sequences. Such formats include microarrays and certain capillary devices. *See, e.g.,* Ng et al., J. Cell Mol. Med., 6:329-340 (2002); U.S. Pat. No. 6,019,944. In these aspects, each discrete surface location may comprise antibodies to immobilize one or more sequences for detection at each location. Surfaces may alternatively comprise one or more discrete particles (*e.g*., microparticles or nanoparticles) immobilized at discrete locations of a surface, where the microparticles comprise antibodies to immobilize one or more sequences for detection. Other useful physical formats include sticks, wells, sponges, and the like.

Analysis can be carried out in a variety of physical formats. For example, the use of microtiter plates or automation could be used to facilitate the processing of large numbers of samples (*e.g*., for determining the translational levels of 100, 500, 1000, 5000, 10,000 genes or more).

### Mass Spectrometry Analysis

In some aspects, one or more translational profiles are generated by mass spectrometry analysis. Mass spectrometry ("MS") generally involves the ionization of the analyte (*e.g*., a translated protein or portion thereof) to generate a charged analyte and measuring the mass-to-charge ratios of said analyte. During the procedure the sample containing the analyte is loaded onto a MS instrument and undergoes vaporization. The components of the sample are then ionized by one of a variety of methods.

As a non-limiting example, during Electrospray-MS (ESI) the analyte is initially dissolved in liquid aerosol droplets. Under the influence of high electromagnetic fields and elevated temperature and/or application of a drying gas the droplets get charged and the liquid matrix evaporates. After all liquid matrix is evaporated the charges remain localized at the analyte molecules that are transferred into the Mass Spectrometer. In matrix assisted laser desorption ionization (MALDI) a mixture of analyte and matrix is irradiated by a laser beam. This results in localized ionization of the matrix material and desorption of analyte and matrix. The ionization of the analyte is believed to happen by charge transfer from the matrix material in the gas phase. For a detailed description of ESI and MALDI, *see, e.g.,* Mano N et al. Anal. Sciences 19 (1) (2003) 3-14. For a description of desorption electrospray ionization (DESI), *see* Takats Z et al. Science 306 (5695) (2004) 471-473. *See also* Karas, M.; Hillencamp, F. Anal. Chem. 60:2301 1988); Beavis, R. C. Org. Mass Spec. 27:653 (1992); and Creel, H. S. Trends Poly. Sci. 1(11):336 (1993).

Ionized sample components are then separated according to their mass-to-charge ratio in a mass analyzer. Examples of different mass analyzers used in LC/MS include, but are not limited to, single quadrupole, triple quadrupole, ion trap, TOF (time of Flight) and quadrupole-time of flight (Q-TOF).

The use of MS for analyzing proteins is also described, for example, in Mann et al., Annu. Rev. Biochem. 70:437-73 (2001).

### C. Differential Translational Profiling

The expression pattern of one or more genes, gene signatures or combinations thereof from a (*e.g*., first) translational profile may differ from the expression pattern observed in one or more genes, gene signatures or combinations thereof from one or more different (*e.g*., second, third, etc.) translational profiles. In such situations, the one or more genes, gene signatures or combinations thereof showing different expression patterns between profiles are considered to be differentially translated. As used herein, the phrase "differentially translated" refers to the change or difference (*e.g*., increase, decrease or a combination thereof) in translation rate, translation efficiency, or both of one gene, a plurality of genes, a set of genes of interest (referred to as "gene markers" or "gene marker set"), one or more gene clusters, or one or more gene signatures under a particular condition as compared to the translation rate, translation efficiency, or both of the same gene, plurality of genes, set of gene markers, gene clusters, or gene signatures under a different condition, which is observed as a difference in expression pattern. For example, a translational profile of a diseased cell may reveal that one or more genes have higher translation rates and/or efficiencies (*e.g*., higher ribosome engagement of mRNA or higher protein abundance) than observed in a normal cell. In some embodiments, one or more gene signatures, gene clusters or sets of gene markers are differentially translated in a first translational profile as compared to one or more other translational profiles. In further embodiments, one or more genes, gene signatures, gene clusters or sets of gene markers in a first translational profile show at least a two-fold translation differential or at least a 1.1 log₂ change (*i.e*., increase or decrease) as compared to the same one or more genes in at least one other different (*e.g*., second, third, etc.) translational profile.

In some embodiments, two or more translational profiles are generated and compared to each other to determine the differences (*i.e*., increases and/or decreases in translational levels, such as translational rate and/or translational efficiency) for each gene in a given set of genes between the two or more translational profiles. The comparison between the two or more translational profiles is referred to as the "differential translational profile." In certain embodiments, a differential translational profile comprises one or more genes, gene signatures (*e.g*., a biological or disease-associated pathway), or combinations thereof. In certain other embodiments, a differential translational profile comprises one or more clusters or groupings of independent genes having a recognized pattern of expression, such as an oncogenic signaling pathway, inflammatory disease-associated pathway, autoimmune disease-associated pathway, neurodegenerative disease-associated pathway, neurocognitive function disorder-associated pathway, fibrotic disorder-associated pathway, metabolic disease-associated pattern, or the like.

In some aspects, methods are provided for identifying a gene signature associated with a disease. In some aspects, the method comprises:
(a) determining a first translational profile for a plurality of genes from a disease sample;
(b) determining a second translational profile for a plurality of genes from a control non-diseased (*e.g*., normal) sample;
(c) identifying a differential translational profile between the first and second translational profiles; and
(d) identifying one or more gene signatures associated with a disease when the disease sample contacted with a known therapeutic has a translational profile for certain genes of a gene cluster or one or more biological pathways found in the differential translational profile that are closer to the translational profile of the same genes in the second translational profile.

The translational profiles that are generated for identifying a gene signature associated with a disease can be generated according to any of the methods described herein. In some aspects, translational profiles are generated by ribosomal profiling, polysome microarray, immunoassay, or combinations thereof. In certain aspects, translational profiles are generated by ribosomal profiling. In some aspects, the disease sample is from a subject having or suspected of having a cancer, an inflammatory disease, an autoimmune disease, a fibrotic disorder, a neurodegenerative disease, a neurodevelopmental disease, a metabolic disease, a viral infection, or cardiomyopathy.

In some aspects, a differential translational profile compares a first translational profile comprising gene translational levels for an experimental biological sample or subject, wherein the experimental biological sample or subject has been contacted with an agent as described herein (*e.g*., a peptide, protein, RNA, drug molecule, or small organic molecule) with a second translational profile comprising gene translational levels for a control biological sample or subject, *e.g*., a corresponding biological sample or subject of the same type that has not been contacted with the agent.

In some aspects, a differential translational profile compares a first translational profile comprising gene translational levels for an experimental biological sample, wherein the experimental biological sample is from a subject having an unknown disease state (*e.g*., a cancer) or an unknown responsiveness to a therapeutic agent, with a second translational profile comprising gene translational levels for a control biological sample, *e.g.,* a biological sample from a subject known to be positive for a disease state (*e.g*., a cancer) or from a subject that is a known responder to the therapeutic agent or from a non-diseased subject or tissue.

In some aspects, differential profiles are generated for each of the first and second translational profiles, *e.g.,* to compare the differences in translational levels for one or more genes in the presence or absence of a condition, or before and after administration of an agent, for the first translational profile (*e.g*., a translational profile from an experimental subject or sample) as compared to the second translational profile (*e.g*., a translational profile from a control subject or sample). For example, in some aspects, differential profiles are generated for an experimental subject or sample (*e.g*., a subject having a cancer) before and after administration of a therapeutic agent and for a control subject or sample (*e.g*., a subject that is a known responder to the therapeutic agent, or a non-diseased (normal) subject or sample) before and after administration of the therapeutic agent. The first differential profile for the first translational profile (from the experimental subject or sample) is compared to the second differential profile for the second translational profile (from the control subject or sample) to determine the similarities in translational levels of one or more genes for the first differential profile as compared to the second differential profile. Based on the similarities between the differential profiles (*e.g*., whether the differential profiles are highly similar or comparable, or whether the translational level for one or more genes in the first differential profile is about the same as the translational level for the one or more genes in the second differential profile), it can be determined whether or not the experimental subject or control is likely to respond to the therapeutic agent.

In certain embodiments, differential translation between genes or translational profiles may involve or result in a biological (*e.g*., phenotypic, physiological, clinical, therapeutic, prophylactic) benefit. For example, when identifying a therapeutic, validating a target, or treating a subject, a "biological benefit" means that the effect on translation rate and/or translation efficiency or on the translation rate and/or translation efficiency of one or more genes of a translational profile allows for intervention or management of a disease, disorder, or condition of a subject (*e.g*., a human or non-human mammal, such as a primate, horse, dog, mouse, rat). In general, one or more differential translations or differential translation profiles indicate that a "biological benefit" will be in the form, for example, of an improved clinical outcome; lessening or alleviation of symptoms associated with disease; decreased occurrence of symptoms; improved quality of life; longer disease-free status; diminishment of extent of disease; stabilization of a disease state; delay of disease progression; remission; survival; or prolonging survival. In certain aspects, a biological benefit comprises normalization of a differential translation profile, or comprises a shift in translational profile to one closer to or comparable to a translational profile induced by a known active compound or therapeutic, or comprises inducing, stimulating or promoting a desired phenotype or outcome (*e.g*., apoptosis, necrosis, cytotoxicity), or reducing, inhibiting or preventing an undesired phenotype or outcome (*e.g*., proliferation, migration).

### IV. Methods of Identifying Agents that Modulate Translation

In one aspect, the present disclosure relates to methods of identifying an agent that modulates translation in a biological pathway (*e.g*., an oncogenic signaling pathway) in a biological sample. In some aspects, the present disclosure relates to methods of identifying an agent that inhibits, antagonizes, or downregulates translation in a biological pathway (*e.g*., an oncogenic signaling pathway) or disease. In some aspects, the present disclosure relates to methods of identifying an agent that modulates, *i.e.,* potentiates, agonizes, inhibits, or upregulates, translation in a biological pathway (*e.g*., an oncogenic signaling pathway) or disease.

### A. Translational Profiles for Identifying Agents that Modulate Translation

In some aspects, a method for identifying an agent that modulates translation in a disease comprises:
(a) determining a first translational profile for a plurality of genes from a disease sample contacted with a candidate agent;
(b) determining a second translational profile for a plurality of genes from a control disease sample not contacted with the agent; and
(c) identifying the agent as a modulator of translation in a disease when one or more genes, one or more gene signatures or combinations thereof are differentially translated in the first translational profile as compared to the second translational profile and when the differential translation results in a biological benefit.

The translational profiles that are generated for identifying an agent that modulates translation in a disease can be generated according to any of the methods described herein. In some aspects, translational profiles are generated by ribosomal profiling, polysome microarray, immunoassay, or combinations thereof. In certain aspects, translational profiles are generated by ribosomal profiling.

In some aspects, translational profiles comprise translational efficiencies, translational rates, or a combination thereof for at least 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, 500, 550, 600, 650, 700, 750, 800, 850, 900, 950, 1000, 1500, 2000, 2500, 3000, 3500, 4000, 4500, 5000, 5500, 6000, 6500, 7000, 7500, 8000, 8500, 9000, 9500, 10,000 genes or more in the biological sample. In some aspects, a first or second translational profile or both comprise translational efficiencies, translational rates, or combinations thereof for at least about 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50% or more of all genes in a biological sample. In some aspects, translational profiles comprise genome-wide measurements of gene translational levels.

In some aspects, an agent that modulates translation in a disease is identified as suitable for use when one or more genes of one or more biological pathways, gene signatures or combinations thereof are differentially translated by at least 1.5-fold or at least 2-fold (*e.g*., at least 1.5-fold, at least 2-fold, at least 2.5-fold, at least 3-fold, at least 3.5-fold, at least 4-fold, at least 4.5-fold, at least 5-fold, at least 6-fold, at least 7-fold, at least 8-fold, at least 9-fold, at least 10-fold or more) in the first translational profile (*e.g*., treated disease sample) as compared to the second translational profile (*e.g*., untreated disease sample). In some aspects, an agent that modulates translation in a disease is identified as suitable for use when the translational rate, translational efficiency or both for one or more genes of one or more biological pathways, gene signatures or combinations thereof are decreased by at least 1.5-fold or at least 2-fold (*e.g*., at least 1.5-fold, at least 2-fold, at least 2.5-fold, at least 3-fold, at least 3.5-fold, at least 4-fold, at least 4.5-fold, at least 5-fold, at least 6-fold, at least 7-fold, at least 8-fold, at least 9-fold, at least 10-fold or more) in the first translational profile as compared to the second translational profile. In some aspects, an agent that modulates translation in a disease is identified as suitable for use when the translational rate, translational efficiency or both for one or more genes of one or more biological pathways, gene signatures or combinations thereof are increased by at least 1.5-fold or at least 2-fold (*e.g*., at least 1.5-fold, at least 2-fold, at least 2.5-fold, at least 3-fold, at least 3.5-fold, at least 4-fold, at least 4.5-fold, at least 5-fold, at least 6-fold, at least 7-fold, at least 8-fold, at least 9-fold, at least 10-fold or more) in the first translational profile as to the second translational profile.

In some aspects, less than about 20% of the genes in the genome are differentially translated by at least 1.5-fold or at least 2-fold in the first translational profile as compared to the second translational profile. In some aspects, less than about 5% of the genes in the genome are differentially translated by at least 1.5-fold or at least 2-fold in the first translational profile as compared to the second translational profile. In some aspects, less than about 1% of the genes in the genome are differentially translated by at least 1.5-fold, at least 2-fold, at least 3-fold, at least 4-fold, or at least 5-fold in the first translational profile as compared to the second translational profile. In some aspects, less than 1% of the genes in the genome are differentially translated by at least 3-fold in the first translational profile as compared to the second translational profile. In some aspects, less than 1% of the genes in the genome are differentially translated by at least 5-fold in the first translational profile as compared to the second translational profile.

In some aspects, the differentially translated genes comprise one or more biological pathways, such as at least two or at least three biological pathways. In certain aspects, the one or more differentially translated genes comprise a plurality of genes and optionally the plurality of differentially translated genes may comprise one or more gene signatures. In further aspects, the one or more genes are differentially translated at least a two-fold or more. In still further aspects, each translational profile comprises at least 100 genes, at least 200 genes, at least 300 genes, at least 400 genes, at least 500 genes, or each translational profile comprises a genome-wide translational profile. For example, less than about 25%, about 20%, about 15%, about 10%, about 5%, about 4% , about 3% , about 2% or about 1% of the genes in the genome are differentially translated in a translational profile from a disease sample treated with a candidate agent as compared to a translational profile of an untreated disease sample.

A disease sample may be obtained from any subject having a disease of interest to identify agents that affect translational profiles in such samples. In certain aspects, the subject has or is suspected of having a disease, such as a cancer, an inflammatory disease, an autoimmune disease, a fibrotic disorder, a neurodegenerative disease, a neurodevelopmental disease, a metabolic disease, a viral infection, or cardiomyopathy.

### B. Translational Profiles for Identifying Agents that Modulate an Oncogenic Signaling Pathway

In some aspects, the method of identifying an agent that modulates an oncogenic signaling pathway comprises:
(a) contacting the biological sample with an agent;
(b) determining a first translational profile for the contacted biological sample, wherein the translational profile comprises translational levels for one or more genes having a 5' terminal oligopyrimidine tract (5' TOP) and/or a pyrimidine-rich translational element (PRTE); and
(c) comparing the first translational profile to a second translational profile comprising translational levels for the one or more genes in a control sample that has not been contacted with the agent;
wherein a difference in the translational levels of the one or more genes in the first translation profile as compared to the second translation profile identifies the agent as a modulator of the oncogenic signaling pathway.

In some aspects, a gene that has a different translational level in the first translational profile as compared to the second translational profile is a gene having a 5' terminal oligopyrimidine tract (5' TOP) sequence. A 5' TOP sequence is a sequence that occurs in the 5' untranslated region (5' UTR) of mRNA. This element is comprised of a cytidine residue at the cap site followed by an uninterrupted stretch of up to 13 pyrimidines. Non-limiting examples of genes having a 5' TOP sequence are shown in Table 1 below. In some aspects, translational levels are compared for the first translational profile and the second translational profile for 1, 2, 3, 4, 5, 6, 7, 8, 9, 10, 11, 12, 13, 14, 15, 16, 17, 18, 19, 20 or more genes selected from the genes listed in Table 1.

**Table 1. Translationally regulated mTOR-responsive genes having a 5' TOP sequence**

| **Gene** | **Description** | **SEQ ID NO** |
|---|---|---|
| AP2A1 | adaptor-related protein complex 2, alpha 1 subunit | 92 |
| CCNI | cyclin I | 96 |
| CD44 | CD44 antigen | 123 |
| CHP | calcineurin-like EF hand protein 1 | 116 |
| CRTAP | cartilage associated protein | 31 |
| EEF1A2 | eukaryotic translation elongation factor 1, alpha 2 | 45 |
| EEF1B2 | eukaryotic translation elongation factor 1, beta 2 | 129 |
| EEF1G | eukaryotic translation elongation factor 1, gamma | 34 |
| EEF2 | eukaryotic translation elongation factor 2 | 1 |
| EIF4B | eukaryotic translation initiation factor 4B | 37 |
| GAPDH | glyceraldehyde-3 -phosphate dehydrogenase | 58 |
| GNB2L1 | guanine nucleotide binding protein (G protein), beta polypeptide 2-like 1 | 22 |
| HNRNPA1 | heterogeneous nuclear ribonucleoprotein A1 | 56 |
| HSPA8 | heat shock 70kDa protein 8 | 42 |
| IPO7 | importin 7 | 109 |
| LCMT1 | leucine carboxyl methyltransferase 1 | 107 |
| NAP1L1 | nucleosome assembly protein 1-like 1 | 93 |
| PABPC1 | poly(A) binding protein, cytoplasmic 1 | 17 |
| PACS1 | phosphofurin acidic cluster sorting protein 1 | 117 |
| PGM1 | phosphoglucomutase 1 | 121 |
| RABGGTB | Rab geranylgeranyltransferase, beta subunit | 139 |
| RPL10 | ribosomal protein L10 | 13 |
| RPL12 | ribosomal protein L12 | 3 |
| RPL13 | ribosomal protein L13 | 70 |
| RPL14 | ribosomal protein L14 | 53 |
| RPL15 | ribosomal protein L15 | 126 |
| RPL17 | ribosomal protein L17 | 79 |
| RPL22 | ribosomal protein L22 | 91 |
| RPL22L1 | ribosomal protein L22 L1 | 35 |
| RPL23 | ribosomal protein L23 | 74 |
| RPL29 | ribosomal protein L29 | 60 |
| RPL31 | ribosomal protein L31 isoform 2 | 49 |
| RPL32 | ribosomal protein L32 | 33 |
| RPL34 | ribosomal protein L34 | 11 |
| RPL36 | ribosomal protein L36 | 63 |
| RPL36A | ribosomal protein L36A | 66 |
| RPL37 | ribosomal protein L37 | 54 |
| RPL37A | ribosomal protein L37A | 18 |
| RPL39 | ribosomal protein L39 | 43 |
| RPL4 | ribosomal protein L4 | 104 |
| RPL41 | ribosomal protein L41 | 113 |
| RPL5 | ribosomal protein L5 | 86 |
| RPL6 | ribosomal protein L6 | 89 |
| RPL8 | ribosomal protein L8 | 59 |
| RPLP0 | ribosomal protein, large, P0 | 28 |
| RPLP2 | ribosomal protein, large, P2 | 38 |
| RPS10 | ribosomal protein S10 | 77 |
| RPS11 | ribosomal protein S11 | 51 |
| RPS14 | ribosomal protein S14 | 94 |
| RPS15A | ribosomal protein S15A | 21 |
| RPS2 | ribosomal protein S2 | 4 |
| RPS20 | ribosomal protein S20 | 24 |
| RPS3A | ribosomal protein S3A | 61 |
| RPS5 | ribosomal protein S5 | 19 |
| RPS6 | ribosomal protein S6 | 101 |
| RPS9 | ribosomal protein S9 | 29 |
| SECTM1 | secreted and transmembrane 1 | 112 |
| TPT1 | tumor protein, translationally-controlled 1 | 65 |
| UBA52 | ubiquitin A-52 residue ribosomal protein fusion product 1 | 84 |
| VIM | vimentin | 40 |
| ABCB7 | ATP-binding cassette, sub-family B (MDR/TAP), member 7 | 134 |
| ALKBH7 | alkB, alkylation repair homolog 7 | 85 |
| ATP5G2 | ATP synthase, H+ transporting, mitochondrial Fo complex, subunit C2 (subunit 9) | 144 |
| EEF1A1 | eukaryotic translation elongation factor 1 alpha 1 | 7 |
| EIF2S3 | eukaryotic translation initiation factor 2, subunit 3 gamma, 52kDa | 80 |
| EIF3H | eukaryotic translation initiation factor 3, subunit H | 98 |
| EIF3L | eukaryotic translation initiation factor 3, subunit L | 108 |
| GLTSCR2 | glioma tumor suppressor candidate region gene 2 | 15 |
| IMPDH2 | IMP (inosine 5'-monophosphate) dehydrogenase 2 | 142 |
| PFDN5 | prefoldin subunit 5 | 130 |
| RPL10A | ribosomal protein L10a | 46 |
| RPL11 | ribosomal protein L11 | 23 |
| RPL13A | ribosomal protein L13a | 5 |
| RPL18 | ribosomal protein L18 | 62 |
| RPL19 | ribosomal protein L19 | 103 |
| RPL21 | ribosomal protein L21 | 20 |
| RPL24 | ribosomal protein L24 | 124 |
| RPL26 | ribosomal protein L26 | 52 |
| RPL27A | ribosomal protein L27A | 12 |
| RPL28 | ribosomal protein L28 | 8 |
| RPL3 | ribosomal protein L3 | 16 |
| RPL30 | ribosomal protein L30 | 81 |
| RPL7A | ribosomal protein L7a | 25 |
| RPLP1 | ribosomal protein, large, P1 | 50 |
| RPS12 | ribosomal protein S12 | 2 |
| RPS13 | ribosomal protein S13 | 105 |
| RPS16 | ribosomal protein S16 | 39 |
| RPS19 | ribosomal protein S19 | 26 |
| RPS21 | ribosomal protein S21 | 27 |
| RPS23 | ribosomal protein S23 | 100 |
| RPS24 | ribosomal protein S24 | 90 |
| RPS25 | ribosomal protein S25 | 75 |
| RPS27 | ribosomal protein S27 | 10 |
| RPS28 | ribosomal protein S28 | 9 |
| RPS29 | ribosomal protein S29 | 73 |
| RPS3 | ribosomal protein S3A | 61 |
| RPS7 | ribosomal protein S7 | 102 |

In some aspects, a gene that has a different translational level in the first translational profile as compared to the second translational profile is a gene having a pyrimidine-rich translational element (PRTE). This element consists of an invariant uridine at its position 6 and does not reside at position +1 of the 5' UTR. *See, e.g.,* Figure 7(c). Non-limiting examples of genes having a PRTE sequence are shown in Table 2 below. In some aspects, translational levels are compared for the first translational profile and the second translational profile for 1, 2, 3, 4, 5, 6, 7, 8, 9, 10, 11, 12, 13, 14, 15, 16, 17, 18, 19, 20 or more genes selected from the genes listed in Table 2.

**Table 2. Translationally regulated mTOR-responsive genes having a PRTE sequence**

| **Gene** | **Description** | **SEQ ID NO** |
|---|---|---|
| EEF2 | eukaryotic translation elongation factor 2 | 1 |
| RPL12 | ribosomal protein L12 | 3 |
| RPS2 | ribosomal protein S2 | 4 |
| RPL18A | ribosomal protein L18a | 6 |
| RPL34 | ribosomal protein L34 | 11 |
| RPL10 | ribosomal protein L10 | 13 |
| EEF1D | eukaryotic translation elongation factor 1 delta | 14 |
| PABPC1 | poly(A) binding protein, cytoplasmic 1 | 17 |
| RPL37A | ribosomal protein L37a | 18 |
| RPS5 | ribosomal protein S5 | 19 |
| RPS15A | ribosomal protein S15a | 21 |
| GNB2L1 | guanine nucleotide binding protein (G protein) | 22 |
| RPS20 | ribosomal protein S20 isoform 1 | 24 |
| RPLP0 | ribosomal protein P0 | 28 |
| RPS9 | ribosomal protein S9 | 29 |
| CRTAP | cartilage associated protein | 31 |
| RPL32 | ribosomal protein L32 | 33 |
| EEF1G | eukaryotic translation elongation factor 1, gamma | 34 |
| RPL22L1 | ribosomal protein L22-like 1 | 35 |
| YB1 | Y-box binding protein 1 | 36 |
| EIF4B | eukaryotic translation initiation factor 4B | 37 |
| RPLP2 | ribosomal protein P2 | 38 |
| VIM | vimentin | 40 |
| HSPA8 | heat shock 70kDa protein 8 isoform 1 | 42 |
| RPL39 | ribosomal protein L39 | 43 |
| AHCY | adenosylhomocysteinase isoform 1 | 44 |
| EEF1A2 | eukaryotic translation elongation factor 1 alpha 2 | 45 |
| PABPC4 | poly A binding protein, cytoplasmic 4 isoform 1 | 47 |
| RPS4X | ribosomal protein S4, X-linked X isoform | 48 |
| RPL31 | ribosomal protein L31 isoform 2 | 49 |
| RPS11 | ribosomal protein S11 | 51 |
| RPL14 | ribosomal protein L14 | 53 |
| RPL37 | ribosomal protein L37 | 54 |
| RPL7 | ribosomal protein L7 | 55 |
| HNRNPA1 | heterogeneous nuclear ribonucleoprotein A1 | 56 |
| RPS8 | ribosomal protein S8 | 57 |
| GAPDH | glyceraldehyde-3 -phosphate dehydrogenase | 58 |
| RPL8 | ribosomal protein L8 | 59 |
| RPL29 | ribosomal protein L29 | 60 |
| RPS3A | ribosomal protein S3a | 61 |
| RPL36 | ribosomal protein L36 | 63 |
| TPT1 | tumor protein, translationally-controlled 1 | 65 |
| RPL36A | ribosomal protein L36a | 66 |
| TKT | transketolase isoform 1 | 68 |
| LMF2 | lipase maturation factor 2 | 69 |
| RPL13 | ribosomal protein L13 | 70 |
| RPL23 | ribosomal protein L23 | 74 |
| TUBB3 | tubulin, beta, 4 | 76 |
| RPS10 | ribosomal protein S10 | 77 |
| FASN | fatty acid synthase | 78 |
| RPL17 | ribosomal protein L17 | 79 |
| ACTG1 | actin, gamma 1 propeptide | 82 |
| COL6A2 | alpha 2 type VI collagen isoform 2C2 | 83 |
| UBA52 | ubiquitin and ribosomal protein L40 precursor | 84 |
| RPL5 | ribosomal protein L5 | 86 |
| PGLS | 6-phosphogluconolactonase | 87 |
| RPL6 | ribosomal protein L6 | 89 |
| RPL22 | ribosomal protein L22 | 91 |
| AP2A1 | adaptor-related protein complex 2, alpha 1 | 92 |
| NAP1L1 | nucleosome assembly protein 1-like 1 | 93 |
| RPS14 | ribosomal protein S14 | 94 |
| CCNI | cyclin I | 96 |
| MTA1 | metastasis associated 1 | 97 |
| RPL9 | ribosomal protein L9 | 99 |
| RPL4 | ribosomal protein L4 | 104 |
| LCMT1 | leucine carboxyl methyltransferase 1 isoform a | 107 |
| IPO7 | importin 7 | 109 |
| PC | pyruvate carboxylase | 110 |
| RPS27A | ubiquitin and ribosomal protein S27a | 111 |
| SECTM1 | secreted and transmembrane 1 precursor | 112 |
| RPL41 | ribosomal protein L41 | 113 |
| TSC2 | tuberous sclerosis 2 isoform 1 | 114 |
| COL18A1 | alpha 1 type XVIII collagen isoform 3 | 115 |
| CHP | calcium binding protein P22 | 116 |
| PACS1 | phosphofurin acidic cluster sorting protein 1 | 117 |
| BRF1 | transcription initiation factor IIIB | 118 |
| PTGES2 | prostaglandin E synthase 2 | 119 |
| PGM1 | phosphoglucomutase 1 | 121 |
| SLC19A1 | solute carrier family 19 member 1 | 122 |
| CD44 | CD44 antigen isoform 1 | 123 |
| RPL15 | ribosomal protein L15 | 126 |
| EEF1B2 | eukaryotic translation elongation factor 1 beta 2 | 129 |
| PNKP | polynucleotide kinase 3' phosphatase | 131 |
| SEPT8 | septin 8 isoform a | 132 |
| EVPL | envoplakin | 136 |
| MYH14 | myosin, heavy chain 14 isoform 3 | 138 |
| RABGGTB | RAB geranylgeranyltransferase, beta subunit | 139 |
| RPL27 | ribosomal protein L27 | 140 |
| SIGMAR1 | sigma non-opioid intracellular receptor 1 | 143 |

In some aspects, a gene that has a different translational level in the first translational profile as compared to the second translational profile is a gene having both a 5' TOP sequence and a PRTE sequence. Non-limiting examples of genes having both a 5' TOP sequence and a PRTE sequence are shown in Table 3 below. In some aspects, translational levels are compared for the first translational profile and the second translational profile for 1, 2, 3, 4, 5, 6, 7, 8, 9, 10, 11, 12, 13, 14, 15, 16, 17, 18, 19, 20 or more genes selected from the genes listed in Table 3.

**Table 3. 5' TOP and PRTE genomic positions in translationally regulated mTOR-responsive genes having both 5' TOP and PRTE**

| **Gene** | **RefSeq ID** | **Chromosome** | **Strand (+/-)** | **5' TOP Position** | **PRTE Position** |
|---|---|---|---|---|---|
| AP2A1 | NM_014203 | 19 | + | 50270268 | 50270306 |
| CCNI | NM_006835 | 4 | - | 77997142 | 77997076 |
| CD44 | NM_000610 | 11 | + | 35160717 | 35160813 |
| CHP | NM_007236 | 15 | + | 41523519 | 41523536 |
| CRTAP | NM_006371 | 3 | + | 33155506/ 33155554 | 33155540 |
| eEF1A2 | NM_001958 | 20 | - | 62130436 | 62129175 |
| eEF1B2 | NM_021121 | 2 | + | 207024619 | 207024665 |
| eEFIG | NM_001404 | 11 | - | 62341490/ 62341335 | 62341383 |
| eEF2 | NM_001961 | 19 | - | 3985461 | 3985423 |
| eIF4B | NM_001417 | 12 | + | 53400240 | 53400250 |
| GAPDH | NM_002046 | 12 | + | 6643684 | 6643717 |
| GNB2L1 | NM_006098 | 5 | - | 180670906 | 180670818 |
| HNRNPA1 | NM_031157 | 12 | + | 54674529 | 54674571 |
| HSPA8 | NM_006597 | 11 | - | 122932844 | 122932806 |
| IP07 | NM_006391 | 11 | + | 9406199 | 9406255 |
| LCMT1 | NM_016309 | 16 | + | 25123101 | 25123114 |
| NAP1L1 | NM_004537 | 12 | - | 76478465 | 76478429 |
| PABPC1 | NM_002568 | 8 | - | 101734315 | 101734151 |
| PACS1 | NM_018026 | 11 | + | 65837839 | 65837922 |
| PGM1 | NM_002633 | 1 | + | 64059078 | 64059107 |
| RABGGTB | NM_004582 | 1 | + | 76251941 | 76251928 |
| RPL10 | NM_006013 | X | + | 153626718 | 153626846 |
| RPL12 | NM_000976 | 9 | - | 130213677 | 130213648 |
| RPL13 | NM_000977/ NM_033251 | 16 | + | 89627090 | 89627102/ 89627202 |
| RPL14 | NM_001034996 | 3 | + | 40498830 | 40498906 |
| RPL15 | NM_002948 | 3 | + | 23958639 | 23958711 |
| RPL17 | NM_000985 | 18 | - | 47018849 | 47017964 |
| RPL22 | NM_000983 | 1 | - | 6259654 | 6259645 |
| RPL22L1 | NM_001099645 | 3 | - | 170587984 | 170587976 |
| RPL23 | NM_000978 | 17 | - | 37009989 | 37010013 |
| RPL29 | NM_000992 | 3 | - | 52029911 | 52029904 |
| RPL31 | NM_001098577 | 2 | + | 101618755 | 101618739 |
| RPL32 | NM_001007074 | 3 | - | 12883040 | 12883002 |
| RPL34 | NM_000995/ NM_033625 | 4 | + | 109541733 | 109541743/ 109541769 |
| RPL36 | NM_033643/ NM_015414 | 19 | + | 5690307 | 5690319/ 5690493 |
| RPL36A | NM_021029 | X | + | 100645999 | 100645981 |
| RPL37 | NM_000997 | 5 | - | 40835324 | 40835314 |
| RPL37A | NM_000998 | 2 | + | 217363567 | 217363526 |
| RPL39 | NM_001000 | X | - | 118925591 | 118925564 |
| RPL4 | NM_000968 | 15 | - | 66797185 | 66797143 |
| RPL41 | NM_001035267 | 12 | + | 56510417 | 56510539 |
| RPL5 | NM_000969 | 1 | + | 93297597 | 93297656 |
| RPL6 | NM_000970 | 12 | - | 112847409 | 112847256 |
| RPL8 | NM_000973/ NM_033301 | 8 | - | 146017775 | 146017709 |
| RPLP0 | NM_053275 | 12 | - | 120638910 | 120638652 |
| RPLP2 | NM_001004 | 11 | + | 809968 | 810006 |
| RPS10 | NM_001014 | 6 | - | 34393846 | 34393715 |
| RPS11 | NM_001015 | 19 | + | 49999690 | 49999677 |
| RPS14 | NM_001025070 | 5 | - | 149829300/ 149829186 | 149829107 |
| RPS15A | NM_001030009 | 16 | - | 18801656 | 18801604 |
| RPS2 | NM_002952 | 16 | - | 2014827 | 2014653 |
| RPS20 | NM_001146227 | 8 | - | 56987065 | 56986992 |
| RPS27A | NM_001177413 | 2 | + | 55459824 | 55459920 |
| RPS3A | NM_001006 | 4 | + | 152020780 | 152020789 |
| RPS5 | NM_001009 | 19 | + | 58898636 | 58898691 |
| RPS6 | NM_001010 | 9 | - | 19380234 | 19380207 |
| RPS9 | NM_001013 | 19 | + | 54704726 | 54704775 |
| SECTM1 | NM_003004 | 17 | - | 80291646 | 80291674/ 80291639 |
| TPT1 | NM_003295 | 13 | - | 45915318 | 45915222 |
| UBA52 | NM_003333 | 19 | + | 18682670 | 18683218 |
| VIM | NM_003380 | 10 | + | 17271277 | 17271358 |

In some aspects, the method comprises:
(a) contacting the biological sample with an agent;
(b) determining a first translational profile for the contacted biological sample, wherein the translational profile comprises translational levels for one or more genes selected from the group consisting of SEQ ID NOs: 1-144; and
(c) comparing the first translational profile to a second translational profile comprising translational levels for the one or more genes in a control sample that has not been contacted with the agent;
wherein a difference in the translational levels of the one or more genes in the first translation profile as compared to the second translation profile identifies the agent as a modulator of the oncogenic signaling pathway.

In some aspects, translational levels are compared for the first translational profile and the second translational profile for 1, 2, 3, 4, 5, 6, 7, 8, 9, 10, 11, 12, 13, 14, 15, 16, 17, 18, 19, 20 or more genes selected from the group consisting of SEQ ID NOs:1-144. SEQ ID NOs:1-144 are listed in Table 4 below:

**Table 4. Translationally regulated mTOR-responsive genes**

| **Gene** | **Description** | **SEQ ID NO** |
|---|---|---|
| EEF2 | eukaryotic translation elongation factor 2 | 1 |
| RPS12 | ribosomal protein S12 | 2 |
| RPL12 | ribosomal protein L12 | 3 |
| RPS2 | ribosomal protein S2 | 4 |
| RPL13A | ribosomal protein L13a | 5 |
| RPL18A | ribosomal protein L18a | 6 |
| EEF1A1 | eukaryotic translation elongation factor 1 alpha 1 | 7 |
| RPL28 | ribosomal protein L28 isoform 1 | 8 |
| RPS28 | ribosomal protein S28 | 9 |
| RPS27 | ribosomal protein S27 | 10 |
| RPL34 | ribosomal protein L34 | 11 |
| RPL27 A | ribosomal protein L27a | 12 |
| RPL10 | ribosomal protein L10 | 13 |
| EEF1D | eukaryotic translation elongation factor 1 delta | 14 |
| GLTSCR2 | glioma tumor suppressor candidate region gene 2 | 15 |
| RPL3 | ribosomal protein L3 isoform a | 16 |
| PABPC1 | poly(A) binding protein, cytoplasmic 1 | 17 |
| RPL37A | ribosomal protein L37a | 18 |
| RPS5 | ribosomal protein S5 | 19 |
| RPL21 | ribosomal protein L21 | 20 |
| RPS15A | ribosomal protein S15a | 21 |
| GNB2L1 | guanine nucleotide binding protein (G protein) | 22 |
| RPL11 | ribosomal protein L11 | 23 |
| RPS20 | ribosomal protein S20 isoform 1 | 24 |
| RPL7A | ribosomal protein L7a | 25 |
| RPS19 | ribosomal protein S19 | 26 |
| RPS21 | ribosomal protein S21 | 27 |
| RPLP0 | ribosomal protein P0 | 28 |
| RPS9 | ribosomal protein S9 | 29 |
| RPS3 | ribosomal protein S3 | 30 |
| CRTAP | cartilage associated protein | 31 |
| FAM128B | hypothetical protein LOC80097 | 32 |
| RPL32 | ribosomal protein L32 | 33 |
| EEF1G | eukaryotic translation elongation factor 1, gamma | 34 |
| RPL22L1 | ribosomal protein L22-like 1 | 35 |
| YB1 | Y-box binding protein 1 | 36 |
| EIF4B | eukaryotic translation initiation factor 4B | 37 |
| RPLP2 | ribosomal protein P2 | 38 |
| RPS16 | ribosomal protein S16 | 39 |
| VIM | vimentin | 40 |
| GAMT | guanidinoacetate N-methyltransferase isoform b | 41 |
| HSPA8 | heat shock 70kDa protein 8 isoform 1 | 42 |
| RPL39 | ribosomal protein L39 | 43 |
| AHCY | adenosylhomocysteinase isoform 1 | 44 |
| EEF1A2 | eukaryotic translation elongation factor 1 alpha 2 | 45 |
| RPL10A | ribosomal protein L10a | 46 |
| PABPC4 | poly A binding protein, cytoplasmic 4 isoform 1 | 47 |
| RPS4X | ribosomal protein S4, X-linked X isoform | 48 |
| RPL31 | ribosomal protein L31 isoform 2 | 49 |
| RPLP1 | ribosomal protein P1 isoform 1 | 50 |
| RPS11 | ribosomal protein S11 | 51 |
| RPL26 | ribosomal protein L26 | 52 |
| RPL14 | ribosomal protein L14 | 53 |
| RPL37 | ribosomal protein L37 | 54 |
| RPL7 | ribosomal protein L7 | 55 |
| HNRNPA1 | heterogeneous nuclear ribonucleoprotein A1 | 56 |
| RPS8 | ribosomal protein S8 | 57 |
| GAPDH | glyceraldehyde-3 -phosphate dehydrogenase | 58 |
| RPL8 | ribosomal protein L8 | 59 |
| RPL29 | ribosomal protein L29 | 60 |
| RPS3A | ribosomal protein S3a | 61 |
| RPL18 | ribosomal protein L18 | 62 |
| RPL36 | ribosomal protein L36 | 63 |
| AGRN | agrin precursor | 64 |
| TPT1 | tumor protein, translationally-controlled 1 | 65 |
| RPL36A | ribosomal protein L36a | 66 |
| SLC25A5 | adenine nucleotide translocator 2 | 67 |
| TKT | transketolase isoform 1 | 68 |
| LMF2 | lipase maturation factor 2 | 69 |
| RPL13 | ribosomal protein L13 | 70 |
| CTSH | cathepsin H isoform b | 71 |
| FAM83H | FAM83H | 72 |
| RPS29 | ribosomal protein S29 isoform 2 | 73 |
| RPL23 | ribosomal protein L23 | 74 |
| RPS25 | ribosomal protein S25 | 75 |
| TUBB3 | tubulin, beta, 4 | 76 |
| RPS10 | ribosomal protein S10 | 77 |
| FASN | fatty acid synthase | 78 |
| RPL17 | ribosomal protein L17 | 79 |
| EIF2S3 | eukaryotic translation initiation factor 2, S3 | 80 |
| RPL30 | ribosomal protein L30 | 81 |
| ACTG1 | actin, gamma 1 propeptide | 82 |
| COL6A2 | alpha 2 type VI collagen isoform 2C2 | 83 |
| UBA52 | ubiquitin and ribosomal protein L40 precursor | 84 |
| ALKBH7 | spermatogenesis associated 11 precursor | 85 |
| RPL5 | ribosomal protein L5 | 86 |
| PGLS | 6-phosphogluconolactonase | 87 |
| CSDA | cold shock domain protein A | 88 |
| RPL6 | ribosomal protein L6 | 89 |
| RPS24 | ribosomal protein S24 isoform d | 90 |
| RPL22 | ribosomal protein L22 | 91 |
| AP2A1 | adaptor-related protein complex 2, alpha 1 | 92 |
| NAP1L1 | nucleosome assembly protein 1-like 1 | 93 |
| RPS14 | ribosomal protein S14 | 94 |
| ETHE1 | ETHE1 protein | 95 |
| CCNI | cyclin I | 96 |
| MTA1 | metastasis associated 1 | 97 |
| EIF3H | eukaryotic translation initiation factor 3, H | 98 |
| RPL9 | ribosomal protein L9 | 99 |
| RPS23 | ribosomal protein S23 | 100 |
| RPS6 | ribosomal protein S6 | 101 |
| RPS7 | ribosomal protein S7 | 102 |
| RPL19 | ribosomal protein L19 | 103 |
| RPL4 | ribosomal protein L4 | 104 |
| RPS13 | ribosomal protein S13 | 105 |
| C21orf66 | GC-rich sequence DNA-binding factor candidate | 106 |
| LCMT1 | leucine carboxyl methyltransferase 1 isoform a | 107 |
| EIF3L | eukaryotic translation initiation factor 3, L | 108 |
| IPO7 | importin 7 | 109 |
| PC | pyruvate carboxylase | 110 |
| RPS27A | ubiquitin and ribosomal protein S27a | 111 |
| SECTM1 | secreted and transmembrane 1 precursor | 112 |
| RPL41 | ribosomal protein L41 | 113 |
| TSC2 | tuberous sclerosis 2 isoform 1 | 114 |
| COL18A1 | alpha 1 type XVIII collagen isoform 3 | 115 |
| CHP | calcium binding protein P22 | 116 |
| PACS1 | phosphofurin acidic cluster sorting protein 1 | 117 |
| BRF1 | transcription initiation factor IIIB | 118 |
| PTGES2 | prostaglandin E synthase 2 | 119 |
| C2orf79 | hypothetical protein LOC391356 | 120 |
| PGM1 | phosphoglucomutase 1 | 121 |
| SLC19A1 | solute carrier family 19 member 1 | 122 |
| CD44 | CD44 antigen isoform 1 | 123 |
| RPL24 | ribosomal protein L24 | 124 |
| NCLN | nicalin | 125 |
| RPL15 | ribosomal protein L15 | 126 |
| CLPTM1 | cleft lip and palate associated transmembrane | 127 |
| ECSIT | evolutionarily conserved signaling intermediate | 128 |
| EEF1B2 | eukaryotic translation elongation factor 1 beta 2 | 129 |
| PFDN5 | prefoldin subunit 5 isoform alpha | 130 |
| PNKP | polynucleotide kinase 3' phosphatase | 131 |
| SEPT8 | septin 8 isoform a | 132 |
| CIRBP | cold inducible RNA binding protein | 133 |
| ABCB7 | ATP-binding cassette, sub-family B, member 7 | 134 |
| ARD1A | alpha-N-acetyltransferase 1A | 135 |
| EVPL | envoplakin | 136 |
| LAMA5 | laminin alpha 5 | 137 |
| MYH14 | myosin, heavy chain 14 isoform 3 | 138 |
| RABGGTB | RAB geranylgeranyltransferase, beta subunit | 139 |
| RPL27 | ribosomal protein L27 | 140 |
| RPS15 | ribosomal protein S15 | 141 |
| IMPDH2 | inosine monophosphate dehydrogenase 2 | 142 |
| SIGMAR1 | sigma non-opioid intracellular receptor 1 | 143 |
| ATP5G2 | ATP synthase, H+ transporting, mitochondrial F0 | 144 |

In some aspects, the first and/or second translational profile comprises 1, 2, 3, 4, 5, 6, 7, 8, 9, 10 or more genes that are functionally classified as a protein synthesis gene, a cell invasion/metastasis gene, a metabolism gene, a signal transduction gene, a cellular transport gene, a post-translational modification gene, an RNA synthesis and processing gene, a regulation of cell proliferation gene, a development gene, an apoptosis gene, a DNA repair gene, a DNA methylation gene, or an amino acid biosynthesis gene. In some embodiments, the first and/or second translational profile comprises 1, 2, 3, 4, 5, 6, 7, 8, 9, 10 or more genes from each of two, three, four, five, or more of these functional categories of genes. In some aspects, first and/or second translational profile comprises 1, 2, 3, 4, 5, 6, 7, 8, 9, 10 more genes that are functionally classified as a cell invasion or metastasis gene. In some aspects, the first and/or second translational profile comprises one or more of the cell invasion/metastasis genes YB1, vimentin, MTA1, and CD44. In some aspects, the first and/or second translational profile comprises YB1, vimentin, MTA1, and CD44.

In some aspects, the method comprises:
(a) contacting the biological sample with an agent;
(b) determining a first translational profile for the contacted biological sample, wherein the translational profile comprises a measurement of gene translational levels for a substantial portion of the genome;
(c) comparing the first translational profile to a second translational profile comprising a measurement of gene translational levels for the substantial portion of the genome translational levels for the one or more genes in a control sample that has not been contacted with the agent;
(d) identifying in the first translational profile a plurality of genes having decreased translational levels as compared to the translational levels of the plurality of genes in the second translational profile; and
(e) determining whether, for the plurality of genes identified in step (d), there is a common consensus sequence and/or regulatory element in the untranslated regions (UTRs) of the genes that is shared by at least 10% of the plurality of genes identified in step (d);
wherein a decrease in the translational levels of at least 10% of the genes sharing the common consensus sequence and/or UTR regulatory element in the first translational profile as compared to the second translational profile identifies the agent as an inhibitor of an oncogenic signaling pathway.

As used herein, the term "substantial portion of the genome," with reference to a biological sample, can refer to an empirical number of genes being measured in the biological sample or to a percentage of the genes in the genome being measured in the biological sample. In some aspects, a substantial portion of the genome comprises at least 500 genes, *e.g.,* at least 500, 600, 700, 800, 900, 1000, 2000, 3000, 4000, 5000, 6000, 7000, 8000, 9000, 10,000, 11,000, 12,000, 13,000, 14,000, or 15,000 genes or more. In some aspects, a substantial portion of the genome comprises at least about 0.01%, at least about 0.05%, at least about 0.1%, at least about 0.5%, at least about 1%, at least about 2%, at least about 3%, at least about 4%, at least about 5%, at least about 6%, at least about 7%, at least about 8%, at least about 9%, at least about 10%, at least about 11%, at least about 12%, at least about 13%, at least about 14%, at least about 15%, at least about 16%, at least about 17%, at least about 18%, at least about 19%, at least about 20%,at least about 25%, at least about 30%, at least about 35%, at least about 40%, at least about 45%, or at least about 50% of all genes in the genome for the biological sample.

In some aspects, the oncogenic signaling pathway that is modulated is the mammalian target of rapamycin (mTOR) pathway, the PI3K pathway, the AKT pathway, the Ras pathway, the Myc pathway, the Wnt pathway, or the BRAF pathway. In some aspects, the oncogenic signaling pathway that is modulated is the mTOR pathway.

In some aspects, there is at least a 1.5-fold or at least 2-fold (*e.g.,* at least 1.5-fold, at least 2-fold, at least 2.5-fold, at least 3-fold, at least 3.5-fold, at least 4-fold, at least 4.5-fold, at least 5-fold, at least 6-fold, at least 7-fold, at least 8-fold, at least 9-fold, at least 10-fold difference or more) in translational level for the one or more genes in the first translational profile as compared to the second translational profile. In some aspects, there is at least a 1.5-fold or at least a 2-fold difference in translational level for 1, 2, 3, 4, 5, 6, 7, 8, 9, 10, 11, 12, 13, 14, 15, 16, 17, 18, 19, 20, 30, 40, 50, 60, 70, 80, 90, 100 or more genes in the first translational profile as compared to the second translational profile. In some aspects, the translational level of one or more genes is decreased in the first translational profile as compared to the second translational profile. In some aspects, the translational level of one or more genes in the first translational profile is decreased by at least 1.5-fold, at least two-fold, at least three-fold, at least four-fold, at least five-fold, at least six-fold, at least seven-fold, at least eight-fold, at least nine-fold, at least ten-fold or more as compared to the second translational profile. In some aspects, the translational level of one or more genes is increased in the first translational profile as compared to the second translational profile. In some aspects, the translational level of one or more genes in the first translational profile is increased by at least 1.5-fold, at least two-fold, at least three-fold, at least four-fold, at least five-fold, at least six-fold, at least seven-fold, at least eight-fold, at least nine-fold, at least ten-fold or more as compared to the second translational profile. In some aspects, the translational level of one or more genes is decreased (*e.g.,* by at least 1.5-fold, at least two-fold, at least three-fold, at least four-fold, at least five-fold, at least six-fold, at least seven-fold, at least eight-fold, at least nine-fold, at least ten-fold or more) in the first translational profile, while the translational level of another one or more genes is increased (*e.g*., by at least 1.5-fold, at least two-fold, at least three-fold, at least four-fold, at least five-fold, at least six-fold, at least seven-fold, at least eight-fold, at least nine-fold, at least ten-fold or more) in the first translational profile, as compared to the second translational profile.

### C. Agents

In some embodiments, an agent that can be used according to the methods of the present invention is a peptide, protein, oligopeptide, circular peptide, peptidomimetic, antibody, polysaccharide, lipid, fatty acid, inhibitory RNA (*e.g.*, siRNA, miRNA, or shRNA), polynucleotide, oligonucleotide, aptamer, small organic molecule, or drug compound. The agent can be either synthetic or naturally-occurring.

In some embodiments, the agent acts as a specific regulator of translational machinery or a component of translational machinery that alters the program of protein translation in cells (*e.g.,* a small molecule inhibitor or inhibitory RNA). In some embodiments, the agent binds at the active site of a protein (*e.g.,* an ATP site inhibitor of mTOR).

In some embodiments, multiple agents (*e.g.,* 2, 3, 4, 5, or more agents) are used. In some aspects, multiple agents are administered to a subject or contacted to a biological sample sequentially. In some aspects, multiple agents are administered to a subject or contacted to a biological sample concurrently.

The agents described herein can be used at varying concentrations. In some aspects, an agent is administered to a subject or contacted to a biological sample at a concentration that is known or expected to be a therapeutic dose. In some aspects, an agent is administered to a subject or contacted to a biological sample at a concentration that is known or expected to be a sub-therapeutic dose. In some aspects, an agent is administered to a subject or contacted to a biological sample at a concentration that is lower than a concentration that would typically be administered to an organism or applied to a sample, *e.g.,* at a concentration that is 2, 3, 4, 5, 6, 7, 8, 9, 10, 15, 20, 25, 30, 35, 40, 45, 50, 60, 70, 80, 90, or 100 times less than the concentration that would typically be administered to an organism or applied to a sample.

In some embodiments, an agent can be identified from a library of agents. In some embodiments, the library of agents comprises at least about 5, 10, 15, 20, 25, 30, 35, 40, 45, 50, 60, 70, 80, 90, 100, 200, 300, 400, 500, 600, 700, 800, 900, 1000, 5000, 10,000, 20,000, 30,000, 40,000, 50,000 agents or more. It will be appreciated that there are many suppliers of chemical compounds, including Sigma (St. Louis, MO), Aldrich (St. Louis, MO), Sigma-Aldrich (St. Louis, MO), Fluka Chemika-Biochemica Analytika (Buchs Switzerland), as well as providers of small organic molecule and peptide libraries ready for screening, including Chembridge Corp. (San Diego, CA), Discovery Partners International (San Diego, CA), Triad Therapeutics (San Diego, CA), Nanosyn (Menlo Park, CA), Affymax (Palo Alto, CA), ComGenex (South San Francisco, CA), and Tripos, Inc. (St. Louis, MO). In some embodiments, the library is a combinatorial chemical or peptide library. A combinatorial chemical library is a collection of diverse chemical compounds generated by either chemical synthesis or biological synthesis, by combining a number of chemical "building blocks" such as reagents. For example, a linear combinatorial chemical library such as a polypeptide library is formed by combining a set of chemical building blocks (amino acids) in every possible way for a given compound length (*i.e.,* the number of amino acids in a polypeptide compound). Millions of chemical compounds can be synthesized through such combinatorial mixing of chemical building blocks. The preparation and screening of chemical libraries is well known to those of skill in the art *(see, e.g.,* Beeler et al., Curr Opin Chem Biol., 9:277 (2005); and Shang et al., Curr Opin Chem Biol., 9:248 (2005)).

In some embodiments, an agent for use in the methods of the present invention (e.g., an agent that modulates an oncogenic signaling pathway) can be identified by screening a library containing a large number of potential therapeutic compounds. The library can be screened in one or more assays, as described herein, to identify those library members that display a desired characteristic activity. The compounds thus identified can serve as conventional "lead compounds" (*e.g*., for identifying other potential therapeutic compounds) or can themselves be used as potential or actual therapeutics. Libraries of use in the present invention can be composed of amino acid compounds, nucleic acid compounds, carbohydrates, or small organic compounds. Carbohydrate libraries have been described in, for example, Liang et al., Science, 274:1520-1522 (1996); and U.S. Patent No. 5,593,853.

Representative amino acid compound libraries include, but are not limited to, peptide libraries *(see, e.g.,* U.S. Patent Nos. 5,010,175; 6,828,422; and 6,844,161; Furka, Int. J. Pept. Prot. Res., 37:487-493 (1991); Houghton et al., Nature, 354:84-88 (1991); and Eichler, Comb Chem High Throughput Screen., 8:135 (2005)), peptoids (PCT Publication No. WO 91/19735), encoded peptides (PCT Publication No. WO 93/20242), random bio-oligomers (PCT Publication No. WO 92/00091), vinylogous polypeptides (Hagihara et al., J. Amer. Chem. Soc., 114:6568 (1992)), nonpeptidal peptidomimetics with β-D-glucose scaffolding (Hirschmann et al., J. Amer. Chem. Soc., 114:9217-9218 (1992)), peptide nucleic acid libraries (*see, e.g.,* U.S. Patent No. 5,539,083), antibody libraries *(see, e.g.,* U.S. Patent Nos. 6,635,424 and 6,555,310; PCT Application No. PCT/US96/10287; and Vaughn et al., Nature Biotechnology, 14:309-314 (1996)), and peptidyl phosphonates (Campbell et al., J. Org. Chem., 59:658 (1994)).

Representative nucleic acid compound libraries include, but are not limited to, genomic DNA, cDNA, mRNA, inhibitory RNA (*e.g.,* RNAi, siRNA), and antisense RNA libraries. *See, e.g.,* Ausubel, Current Protocols in Molecular Biology, eds. 1987-2005, Wiley Interscience; and Sambrook and Russell, Molecular Cloning: A Laboratory Manual, 2000, Cold Spring Harbor Laboratory Press. Nucleic acid libraries are described in, for example, U.S. Patent Nos . 6,706,477; 6,582,914; and 6,573,098. cDNA libraries are described in, for example, U.S. Patent Nos. 6,846,655; 6,841,347; 6,828,098; 6,808,906; 6,623,965; and 6,509,175. RNA libraries, for example, ribozyme, RNA interference, or siRNA libraries, are described in, for example, Downward, Cell, 121:813 (2005) and Akashi et al., Nat. Rev. Mol. Cell Biol., 6:413 (2005). Antisense RNA libraries are described in, for example, U.S. Patent Nos. 6,586,180 and 6,518,017.

Representative small organic molecule libraries include, but are not limited to, diversomers such as hydantoins, benzodiazepines, and dipeptides (Hobbs et al., Proc. Nat. Acad. Sci. USA, 90:6909-6913 (1993)); analogous organic syntheses of small compound libraries (Chen et al., J. Amer. Chem. Soc., 116:2661 (1994)); oligocarbamates (Cho et al., Science, 261:1303 (1993)); benzodiazepines *(e.g.,* U.S. Patent No. 5,288,514; and Baum, C&EN, Jan 18, page 33 (1993)); isoprenoids (*e.g.,* U.S. Patent No. 5,569,588); thiazolidinones and metathiazanones (*e.g.,* U.S. Patent No. 5,549,974); pyrrolidines *(e.g.,* U.S. Patent Nos. 5,525,735 and 5,519,134); morpholino compounds (*e.g*., U.S. Patent No. 5,506,337); tetracyclic benzimidazoles (*e.g*., U.S. Patent No. 6,515,122); dihydrobenzpyrans (*e.g.,* U.S. Patent No. 6,790,965); amines (*e.g.,* U.S. Patent No. 6,750,344); phenyl compounds (*e.g.,* U.S. Patent No. 6,740,712); azoles (*e.g.,* U.S. Patent No. 6,683,191); pyridine carboxamides or sulfonamides (*e.g*., U.S. Patent No. 6,677,452); 2-aminobenzoxazoles (*e.g*., U.S. Patent No. 6,660,858); isoindoles, isooxyindoles, or isooxyquinolines (*e.g.,* U.S. Patent No. 6,667,406); oxazolidinones *(e.g.,* U.S. Patent No. 6,562,844); and hydroxylamines (*e.g*., U.S. Patent No. 6,541,276).

Devices for the preparation of libraries are commercially available. *See, e.g.,* 357 MPS and 390 MPS from Advanced Chem. Tech (Louisville, KY), Symphony from Rainin Instruments (Woburn, MA), 433A from Applied Biosystems (Foster City, CA), and 9050 Plus from Millipore (Bedford, MA).

### D. Undruggable Targets

In some aspects, the methods of the present disclosure relate to identifying an agent that modulates an undruggable target. It is estimated that only about 10-15% of human proteins are disease modifying, and of these proteins, as many as 85-90% are "undruggable," meaning that even though theoretical therapeutic benefits may be experimentally observed for these target proteins (*e.g., in vitro* or in a model system *in vivo* using techniques such as shRNA), targeted therapy using a drug compound (*e.g*., a small molecule or antibody) does not successfully interfere with the biological function of the protein (or of the gene encoding the protein). Typically, an undruggable target is a protein that lacks a binding site for small molecules or for which binding of small molecules does not alter biological function (*e.g*., ribosomal proteins); a protein for which, despite having a small molecule binding site, successful targeting of said site has proven intractable in practice (*e.g*., GTP/GDP proteins); or a protein for which selectivity of small molecule binding has not been obtained due to close homology of the binding site with other proteins, and for which binding of the small molecule to these other proteins obviates the therapeutic benefit that is theoretically achievable with binding to the target protein (*e.g*., protein phosphatases). A target may be undruggable to antibody-based therapeutics for a variety of reasons, such as intracellular location of the target, masking of target antigenicity (*e.g*., due to modification with carbohydrate or other masking modifications), escape by competition (*e.g*., by shedding or release of decoy molecules), or the like. By preferentially inhibiting the synthesis of such a target protein by selectively inhibiting programmed translation of a small set of proteins (*e.g*., about 1, 2, 3, 4, 5, 6, 7, 8, 9, 10, 15, or 20 proteins), it is possible to modulate (*e.g*., inhibit) the activity of the "undruggable" target protein.

In some aspects, a method of identifying an agent that modulates an undruggable target comprises:
(a) contacting a biological sample with an agent;
(b) determining a first translational profile for the contacted biological sample, wherein the translational profile comprises translational levels for a plurality of genes; and
(c) comparing the first translational profile to a second translational profile comprising translational levels for the plurality of genes in a control sample that has not been contacted with the agent;
wherein identifying one or more genes as differentially translated in the first translational profile as compared to the second translational profile identifies the agent as modulating the activity of the undruggable target. In some aspects, one or more genes of a biological pathway are differentially translated in the first translational profile as compared to the second translational profile, wherein the biological pathway is selected from a protein synthesis pathway, a cell invasion/metastasis pathway, a cell division pathway, an apoptosis pathway, a signal transduction pathway, a cellular transport pathway, a post-translational protein modification pathway, a DNA repair pathway, and DNA methylation pathway.

In some aspects, one or more genes from each of at least two, at least three, at least four, at least five, or more of the biological pathways is differentially translated in the first translational profile as compared to the second translational profile. In some aspects, two, three, four, five or more genes (*e.g.,* 1, 2, 3, 4, 5, 6, 7, 8, 9, 10, 11, 12, 13, 14, 15, 16, 17, 18, 19, 20, 30, 40, 50, 60, 70, 80, 90, 100 or more genes) from one or more of the biological pathways are differentially translated in the first translational profile as compared to the second translational profile. Non-limiting examples of protein synthesis, cell invasion/metastasis, cell division, apoptosis pathway, signal transduction, cellular transport, post-translational protein modification, DNA repair, and DNA methylation pathways are described herein.

In some aspects, the first and/or second translational profile comprises translational levels for a plurality of genes in the biological sample. In some aspects, the first and/or second translational profile comprises translational levels for at least 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, 500, 550, 600, 650, 700, 750, 800, 850, 900, 950, 1000, 1500, 2000, 2500, 3000, 3500, 4000, 4500, 5000, 5500, 6000, 6500, 7000, 7500, 8000, 8500, 9000, 9500, 10,000 genes or more in the biological sample. In some aspects, the first and/or second translational profile comprises translational levels for at least about 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50% of all genes in the biological sample or more. In some aspects, the first and/or second translational profile comprises a genome-wide measurement of gene translational levels in the biological sample.

In some aspects, there is at least a 1.5-fold or at least a two-fold difference in translational level for the one or more genes (e.g., for 1, 2, 3, 4, 5, 6, 7, 8, 9, 10, 11, 12, 13, 14, 15, 16, 17, 18, 19, 20, 30, 40, 50, 60, 70, 80, 90, 100 or more genes) in the first translational profile as compared to the second translational profile. In some aspects, there is at least a three-fold difference, at least a four-fold difference, at least a five-fold difference, at least a six-fold difference, at least a seven-fold difference, at least an eight-fold difference, at least a nine-fold difference, at least a ten-fold difference or more in the translational level for the one or more genes in the first translational profile as compared to the second translational profile. In some aspects, the translational level of the one or more genes is decreased in the first translational profile as compared to the second translational profile. In some aspects, the translational level of the one or more genes is increased in the first translational profile as compared to the second translational profile. In some aspects, the translational level of one or more genes is decreased in the first translational profile, while the translational level of another one or more genes is increased in the first translational profile, as compared to the second translational profile.

In some aspects, the agent is an RNA molecule. In some aspects, the agent is an shRNA, siRNA, or miRNA molecule.

### E. Synthesizing and Validating Agents Based on Identified Agents

In some aspects, an agent that is identified as modulating an oncogenic signaling pathway is optimized in order to improve the agent's biological and/or pharmacological properties. To optimize the agent, structurally related analogs of the agent can be chemically synthesized to systematically modify the structure of the initially-identified agent.

For chemical synthesis, solid phase synthesis can be used for compounds such as peptides, nucleic acids, organic molecules, etc., since in general solid phase synthesis is a straightforward approach with excellent scalability to commercial scale. Techniques for solid phase synthesis are described in the art. *See, e.g.,* Seneci, Solid Phase Synthesis and Combinatorial Technologies (John Wiley & Sons 2002); Barany & Merrifield, Solid-Phase Peptide Synthesis, pp. 3-284 in The Peptides: Analysis, Synthesis, Biology, Vol. 2 (E. Gross and J. Meienhofer, eds., Academic Press 1979).

The synthesized structurally related analogs can be screened to determine whether the analogs induce a similar translational profile when contacted to a biological sample as compared to the initial agent from which the analog was derived. In some aspects, a selected-for structurally related analog is one that induces an identical or substantially identical translational profile in a biological sample as the initial agent from which the structurally related analog was derived.

A structurally related analog that is determined to induce a sufficiently similar translational profile in a biological sample as the initial agent from which the structurally related analog was derived can be further screened for biological and pharmacological properties, including but not limited to oral bioavailability, half-life, metabolism, toxicity, and pharmacodynamic activity (*e.g*., duration of the therapeutic effect) according to methods known in the art. Typically, the screening of the structurally related analogs is performed *in vivo* in an appropriate animal model (*e.g.,* a mammal such as a mouse or rat). Animal models for analyzing pharmacological and pharmacokinetic properties, including animal models for various disease states, are well known in the art and are commercially available, *e.g*., from Charles River Laboratories Int'l, Inc. (Wilmington, MA).

In some aspects, an agent that is identified as having a suitable biological profile, or a structurally related analog thereof, is used for the preparation of a medicament for the treatment of a disease or condition associated with the modulation of the biological pathway *(e.g.,* a cancer associated with the modulation of the mTOR pathway).

### V. Methods of Validating a Target for Therapeutic Intervention

In another aspect, the present disclosure provides methods of validating a target for therapeutic intervention. In some aspects, the present disclosure provides a method of validating a target for therapeutic intervention when treatment mimics the translational effect of a known active compound. In some aspects, the method comprises:
(a) contacting a biological sample with an agent that modulates the target;
(b) determining a first translational profile for the contacted biological sample, wherein the first translational profile comprises translational levels for a plurality of genes; and
(c) comparing the first translational profile to a second translational profile comprising translational levels for the plurality of genes in a control sample that has not been contacted with the agent;
wherein identifying one or more genes of a biological pathway as differentially translated in the first translational profile as compared to the second translational profile validates the target for therapeutic intervention, wherein said biological pathway is selected from a protein synthesis pathway, a cell invasion/metastasis pathway, a cell division pathway, an apoptosis pathway, a signal transduction pathway, a cellular transport pathway, a post-translational protein modification pathway, a DNA repair pathway, and a DNA methylation pathway.

In some aspects, translational levels are compared for the first translational profile and the second translational profile for 1, 2, 3, 4, 5, 6, 7, 8, 9, 10, 11, 12, 13, 14, 15, 16, 17, 18, 19, 20 or more genes in one or more biological pathways selected from a protein synthesis pathway, a cell invasion/metastasis pathway, a cell division pathway, an apoptosis pathway, a signal transduction pathway, a cellular transport pathway, a post-translational protein modification pathway, a DNA repair pathway, and a DNA methylation pathway. In some aspects, one or more genes from each of at least two of the biological pathways are differentially translated in the first translational profile as compared to the second translational profile. In some aspects, one or more genes from each of at least three of the biological pathways are differentially translated in the first translational profile as compared to the second translational profile. In some aspects, the biological pathway, or one of the biological pathways, is the mTOR pathway.

In some aspects, translational levels are compared for the first and second translational profiles for one or more genes in a protein synthesis pathway. Examples of protein synthesis pathway genes include, but are not limited to, EEF2, RPS12, RPL12, RPS2, RPL13A, RPL18A, EEF1A1, RPL28, RPS28, and RPS27. In some aspects, translational levels are compared for the first and second translational profiles for one or more genes in a cell invasion/metastasis pathway. Examples of cell invasion/metastasis pathway genes include, but are not limited to, YB1, MTA1, Vimentin, and CD44. In some aspects, translational levels are compared for the first and second translational profiles for one or more genes in a cell division pathway. Examples of cell division pathway genes include, but are not limited to, CCNI. In some aspects, translational levels are compared for the first and second translational profiles for one or more genes in an apoptosis pathway. Examples of apoptosis pathway genes include, but are not limited to, ARF, FADD, TNFRSF21, BAX, DAPK, TMS-1, BCL2, RASSF1A, and TERT. In some aspects, translational levels are compared for the first and second translational profiles for one or more genes in a signal transduction pathway. Examples of signal transduction pathway genes include, but are not limited to, MAPK, MYC, RAS, and RAF. In some aspects, translational levels are compared for the first and second translational profiles for one or more genes in a cellular transport pathway. Examples of cellular transport pathway genes include, but are not limited to, SLC25A5. In some aspects, translational levels are compared for the first and second translational profiles for one or more genes in a post-translational protein modification pathway. Examples of post-translational protein modification pathway genes include, but are not limited to, LCMT1 and RABGGTB. In some aspects, translational levels are compared for the first and second translational profiles for one or more genes in a DNA repair pathway. Examples of DNA repair pathway genes include, but are not limited to, PNKP. In some aspects, translational levels are compared for the first and second translational profiles for one or more genes in a DNA methylation pathway. Examples of DNA methylation pathway genes include, but are not limited to, AHCY.

In some aspects, the one or more genes has a 5' TOP sequence, a PRTE sequence, or both a 5' TOP sequence and a PRTE sequence. In some aspects, the one or more genes is selected from the genes listed in Table 1, Table 2, and/or Table 3. In some aspects, the one or more genes is selected from the group consisting of SEQ ID NOs:1-144.

In some aspects, the target for therapeutic intervention is a part of an oncogenic signaling pathway. In some aspects, the oncogenic signaling pathway is the mammalian target of rapamycin (mTOR) pathway, the PI3K pathway, the AKT pathway, the Ras pathway, the Myc pathway, the Wnt pathway, or the BRAF pathway. In some embodiments, the oncogenic signaling pathway that is modulated is the mTOR pathway.

In some aspects, a method for validating a target for therapeutic intervention in a disease (*e.g.,* an inflammatory disease, an autoimmune disease, a fibrotic disorder, a neurodegenerative disease, a neurodevelopmental disease, a metabolic disease, a viral infection, a cardiomyopathy or a cancer), comprises:
(a) determining a first translational profile for a plurality of genes from a disease sample contacted with an agent that modulates a target;
(b) determining a second translational profile for a plurality of genes from a control disease sample not contacted with the agent; and
(c) validating the target for therapeutic intervention in the disease (*e.g.,* an inflammatory disease, an autoimmune disease, a fibrotic disorder, a neurodegenerative disease, a neurodevelopmental disease, a metabolic disease, a viral infection, a cardiomyopathy or a cancer, respectively) when one or more genes are differentially translated in the first translational profile as compared to the second translational profile and when the differential translation results in a biological benefit.

In some aspects, a method for validating a target for therapeutic intervention in a disease (*e.g.,* an inflammatory disease, an autoimmune disease, a fibrotic disorder, a neurodegenerative disease, a neurodevelopmental disease, a metabolic disease, a viral infection, a cardiomyopathy or a cancer), comprises:
(a) determining a first translational profile for a plurality of genes from a disease sample contacted with an agent that modulates a target;
(b) determining a second translational profile for a plurality of genes from a disease sample contacted with a known active compound for treating the disease; and
(c) validating the target for therapeutic intervention in a disease *(e.g.,* an inflammatory disease, an autoimmune disease, a fibrotic disorder, a neurodegenerative disease, a neurodevelopmental disease, a metabolic disease, a viral infection, a cardiomyopathy or a cancer, respectively) when the first translational profile is comparable to the second translational profile.

In certain aspects, a method for validating a target for therapeutic intervention in a disease (*e.g.,* an inflammatory disease, an autoimmune disease, a fibrotic disorder, a neurodegenerative disease, a neurodevelopmental disease, a metabolic disease, a viral infection, a cardiomyopathy or a cancer), comprises:
(a) determining three independent translational profiles, each for a plurality of genes from a disease sample, wherein (i) a first translational profile is from the sample not contacted with any compound, (ii) a second translational profile is from the sample contacted with an agent that modulates a target, and (iii) a third translational profile is from the sample contacted with a known active compound for treating the disease;
(b) identifying one or more genes as differentially translated in the first translational profile as compared to the second translational profile; and
(c) validating the target as a target for therapeutic intervention in the disease (*e.g.,* an inflammatory disease, an autoimmune disease, a fibrotic disorder, a neurodegenerative disease, a neurodevelopmental disease, a metabolic disease, a viral infection, a cardiomyopathy or a cancer, respectively) when the one or more differentially translated genes from step (b) are in the third translational profile and have a translational profile closer to the translational profile of the one or more genes in the second translational profile than to the translational profile of the one or more genes in the first translational profile.

In certain aspects, a method for validating a target for therapeutic intervention in a disease (*e.g.,* an inflammatory disease, an autoimmune disease, a fibrotic disorder, a neurodegenerative disease, a neurodevelopmental disease, a metabolic disease, a viral infection, a cardiomyopathy or a cancer), comprises:
(a) determining three independent translational profiles, each for a plurality of genes from a disease sample, wherein (i) a first translational profile is from the sample not contacted with any compound, (ii) a second translational profile is from the sample contacted with an agent that modulates a target, and (iii) a third translational profile is from the sample contacted with a known active compound for treating the disease;
(b) determining a first differential translational profile comprising one or more genes differentially translated in the first translational profile as compared to the second translational profile, and determining a second differential translational profile comprising one or more genes differentially translated in the first translational profile as compared to the third translational profile; and
(c) validating the target as a target for therapeutic intervention in the disease (*e.g*., an inflammatory disease, an autoimmune disease, a fibrotic disorder, a neurodegenerative disease, a neurodevelopmental disease, a metabolic disease, a viral infection, a cardiomyopathy or a cancer, respectively) when the first differential translational profile is comparable to the second differential translational profile.

In any of the aforementioned aspects for validating a target, the target is suspected of being associated with a disease, is indirectly associated with a disease, or is associated with a disease (*e.g.,* an inflammatory disease, an autoimmune disease, a fibrotic disorder, a neurodegenerative disease, a neurodevelopmental disease, a metabolic disease, a viral infection, a cardiomyopathy or a cancer).

Agents that can be used to validate a target for therapeutic intervention include any agent described herein (*e.g.,* in Section IV(C) above), and include but are not limited to, peptides, proteins, oligopeptides, circular peptides, peptidomimetics, antibodies, polysaccharides, lipids, fatty acids, inhibitory RNAs (*e.g*., siRNA, miRNA, or shRNA), polynucleotides, oligonucleotides, aptamers, small organic molecules, or drug compounds. In some aspects, the agent is a small organic molecule. In some aspects, the agent is a peptide or protein. In some aspects, the agent is an RNA or inhibitory RNA.

The translational profiles that are generated for validating a target for therapeutic intervention can be generated according to any of the methods described herein. In some aspects, the translational profiles are generated by ribosomal profiling. In some aspects, the translational profiles are generated by polysome microarray. In some aspects, the translational profiles are generated by immunoassay. In some aspects, the translational profiles comprise translational levels for 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, 500, 550, 600, 650, 700, 750, 800, 850, 900, 950, 1000, 1500, 2000, 2500, 3000, 3500, 4000, 4500, 5000, 5500, 6000, 6500, 7000, 7500, 8000, 8500, 9000, 9500, 10,000 genes or more in the biological sample. In some aspects, the first and/or second translational profile comprises translational levels for at least about 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50% or more of all genes in the biological sample. In some aspects, the translational profiles comprise genome-wide measurements of gene translational levels.

In some aspects, a target is validated when one or more genes of one or more biological pathways is differentially translated by at least 1.5-fold or at least two-fold *(e.g.,* at least 1.5-fold, at least two-fold, at least three-fold, at least four-fold, at least five-fold, at least six-fold, at least seven-fold, at least eight-fold, at least nine-fold, at least ten-fold or more) in the first translational profile as to the second translational profile. In some aspects, a target is validated when the translational level for one or more genes of one or more biological pathways is decreased by at least 1.5-fold or at least two-fold (*e.g.,* at least 1.5-fold, at least two-fold, at least three-fold, at least four-fold, at least five-fold, at least six-fold, at least seven-fold, at least eight-fold, at least nine-fold, at least ten-fold or more) in the first translational profile as to the second translational profile. In some aspects, a target is validated when the translational level for one or more genes of one or more biological pathways is increased by at least 1.5-fold or at least two-fold (*e.g.,* at least 1.5-fold, at least two-fold, at least three-fold, at least four-fold, at least five-fold, at least six-fold, at least seven-fold, at least eight-fold, at least nine-fold, at least ten-fold or more) in the first translational profile as to the second translational profile. In some aspects, less than 20% of the genes in the genome are differentially translated by at least 1.5-fold or at least two-fold in the first translational profile as compared to the second translational profile. In some aspects, less than 5% of the genes in the genome are differentially translated by at least 1.5-fold, at least 2-fold, at least 3-fold, or at least 4-fold in the first translational profile as compared to the second translational profile. In some aspects, less than 1% of the genes in the genome are differentially translated by at least 1.5-fold, at least 2-fold, at least 3-fold, at least 4-fold, or at least 5-fold in the first translational profile as compared to the second translational profile.

In some aspects, a target is validated when a first differential translational profile is comparable to a second differential translational profile, *e.g*., when at least of 99%, 95%, 90%, 85%, 80%, 75%, 70%, 65%, 60%, 55%, or 50% of a selected portion of differentially translated genes, a majority of differentially translated genes, or all differentially translated genes show a translational profile within 75%, 70%, 65%, 60%, 55%, 50%, 45%, 40%, 35%, 30%, or 25%, respectively, of their corresponding genes in the reference translational profile. In further aspects, a first differential translational profile comprising a selected portion of the differentially translated genes or all the differentially translated genes has a differential translational profile comparable to the differential translational profile of the same genes in a second differential translational profile when the amount of protein translated in the first and second differential translational profiles are within about 3.0 log₂, 2.5 log₂, 2.0 log₂, 1.5 log₂, 1.1 log₂, 0.5 log₂, 0.2 log₂ or closer. In still further aspects, a first differential translational profile comprising a selected portion of the differentially translated genes or all the differentially translated genes has a differential translational profile comparable to the differential translational profile of the same genes in a second differential translational profile when the amount of protein translated in the first and second differential translational profiles differs by no more than about 50%, 40%, 35%, 30%, 25%, 20%, 15%, 10%, 5%, 1% or less.

In some aspects, a target is validated when the translational profiles comprise one or more gene signatures, wherein one or more gene signatures are comparable in the first and second translational profiles. In certain aspects, the first and second translational profiles are comparable when an amount of protein translated from one or more differentially translated genes in the first and second translational profiles differs by no more than about 50%, 40%, 35%, 30%, 25%, 20%, 15%, 10%, 5%, 1% or less. In further aspects, the one or more differentially translated genes from the third translational profile have a translational profile closer to the translational profile of the one or more genes in the second translational profile when the amount of protein translated from the one or more differentially translated genes in the third and second translational profiles differs by no more than about 50%, 40%, 35%, 30%, 25%, 20%, 15%, 10%, 5%, 1% or less.

### VI. Methods of Identifying Drug Candidate Molecules or Agents

In another aspect, the present disclosure comprises a method of identifying a drug candidate molecule. In some aspects, the method comprises:
(a) contacting a biological sample with the drug candidate molecule;
(b) determining a translational profile for the contacted biological sample, wherein the translational profile comprises translational levels for a plurality of genes; and
(c) comparing the first translational profile to a second translational profile comprising translational levels for the plurality of genes in a control sample that has not been contacted with the drug candidate molecule,
wherein the drug candidate molecule is identified as suitable for use in a therapeutic intervention when one or more genes of a biological pathway is differentially translated in the first translational profile as compared to the second translational profile, wherein the biological pathway is selected from a protein synthesis pathway, a cell invasion/metastasis pathway, a cell division pathway, an apoptosis pathway, a signal transduction pathway, a cellular transport pathway, a post-translational protein modification pathway, a DNA repair pathway, and DNA methylation pathway.

In some aspects, the one or more genes (*e.g.,* 1, 2, 3, 4, 5, 6, 7, 8, 9, 10 or more genes) have a 5' TOP sequence, a PRTE sequence, or both a 5' TOP sequence and a PRTE sequence. In some aspects, the one or more genes is selected from the genes listed in Table 1, Table 2, and/or Table 3. In some aspects, the one or more genes (*e.g.,* 1, 2, 3, 4, 5, 6, 7, 8, 9, 10 or more genes) are selected from the group consisting of SEQ ID NOs: 1-144. In some aspects, one or more genes from each of at least two of the biological pathways are differentially translated in the first translational profile as compared to the second translational profile. In some aspects, one or more genes from each of at least three of the biological pathways are differentially translated in the first translational profile as compared to the second translational profile. In certain aspects, the one or more differentially translated genes comprise a plurality of genes and optionally the plurality of differentially translated genes may comprise one or more gene signatures.

In some aspects, translational levels are compared for the first and second translational profiles for one or more genes in a protein synthesis pathway. Examples of protein synthesis pathway genes include, but are not limited to, EEF2, RPS12, RPL12, RPS2, RPL13A, RPL18A, EEF1A1, RPL28, RPS28, and RPS27. In some aspects, translational levels are compared for the first and second translational profiles for one or more genes in a cell invasion/metastasis pathway. Examples of cell invasion/metastasis pathway genes include, but are not limited to, YB1, MTA1, Vimentin, and CD44. In some aspects, translational levels are compared for the first and second translational profiles for one or more genes in a cell division pathway. Examples of cell division pathway genes include, but are not limited to, CCNI. In some aspects, translational levels are compared for the first and second translational profiles for one or more genes in an apoptosis pathway. Examples of apoptosis pathway genes include, but are not limited to, ARF, FADD, TNFRSF21, BAX, DAPK, TMS-1, BCL2, RASSF1A, and TERT. In some aspects, translational levels are compared for the first and second translational profiles for one or more genes in a signal transduction pathway. Examples of signal transduction pathway genes include, but are not limited to, MAPK, MYC, RAS, and RAF. In some aspects, translational levels are compared for the first and second translational profiles for one or more genes in a cellular transport pathway. Examples of cellular transport pathway genes include, but are not limited to, SLC25A5. In some aspects, translational levels are compared for the first and second translational profiles for one or more genes in a post-translational protein modification pathway. Examples of post-translational protein modification pathway genes include, but are not limited to, LCMT1 and RABGGTB. In some aspects, translational levels are compared for the first and second translational profiles for one or more genes in a DNA repair pathway. Examples of DNA repair pathway genes include, but are not limited to, PNKP. In some aspects, translational levels are compared for the first and second translational profiles for one or more genes in a DNA methylation pathway. Examples of DNA methylation pathway genes include, but are not limited to, AHCY.

In some aspects, a method for identifying a drug candidate molecule or agent for treating a disease (*e.g.,* an inflammatory disease, an autoimmune disease, a fibrotic disorder, a neurodegenerative disease, a neurodevelopmental disease, a metabolic disease, a viral infection, a cardiomyopathy or a cancer), comprises:
(a) determining a first translational profile for a plurality of genes from a disease sample contacted with a candidate agent;
(b) determining a second translational profile for a plurality of genes from a control disease sample not contacted with the agent; and
(c) identifying the agent as a candidate therapeutic for use in treating a disease (*e.g.,* an inflammatory disease, an autoimmune disease, a fibrotic disorder, a neurodegenerative disease, a neurodevelopmental disease, a metabolic disease, a viral infection, a cardiomyopathy or a cancer, respectively) when one or more genes are differentially translated in the first translational profile as compared to the second translational profile and when the differential translation results in a biological benefit.

In certain aspects, the plurality of differentially translated genes may comprise a plurality of genes, one or more biological pathways, one or more gene signatures, or any combination thereof. A disease sample may be obtained from any subject having a disease of interest to identify drug candidate molecules or agents that affect translational profiles in such samples. In certain aspects, a biological sample is obtained from a subject who has or is suspected of having a disease, such as an inflammatory disease, an autoimmune disease, a fibrotic disorder, a neurodegenerative disease, a neurodevelopmental disease, a metabolic disease, a viral infection, a cardiomyopathy, or a cancer.

The translational profiles that are generated for identifying a drug candidate molecule or agent can be generated according to any of the methods described herein. In some aspects, the translational profiles are generated by ribosomal profiling. In some aspects, the translational profiles are generated by polysome microarray. In some aspects, the translational profiles are generated by immunoassay. In some aspects, the translational profiles comprise translational levels for at least 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, 500, 550, 600, 650, 700, 750, 800, 850, 900, 950, 1000, 1500, 2000, 2500, 3000, 3500, 4000, 4500, 5000, 5500, 6000, 6500, 7000, 7500, 8000, 8500, 9000, 9500, 10,000 genes or more in the biological sample. In some aspects, the first and/or second translational profile comprises translational levels for at least about 0.1%, 0.5% , 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50% or more of all genes in the biological sample. In some aspects, the translational profiles comprise genome-wide measurements of gene translational levels.

In some aspects, a drug candidate molecule or agent is identified as suitable for use in a therapeutic intervention when one or more genes of one or more biological pathways is differentially translated by at least 1.5-fold or at least two-fold (*e.g.,* at least 1.5-fold, at least two-fold, at least three-fold, at least four-fold, at least five-fold, at least six-fold, at least seven-fold, at least eight-fold, at least nine-fold, at least ten-fold or more) in the first translational profile as to the second translational profile. In some aspects, a drug candidate molecule is identified as suitable for use in a therapeutic intervention when the translational level for one or more genes of one or more biological pathways is decreased by at least 1.5-fold or at least two-fold (*e.g.,* at least 1.5-fold, at least two-fold, at least three-fold, at least four-fold, at least five-fold, at least six-fold, at least seven-fold, at least eight-fold, at least nine-fold, at least ten-fold or more) in the first translational profile as to the second translational profile. In some aspects, a drug candidate molecule is identified as suitable for use in a therapeutic intervention when the translational level for one or more genes of one or more biological pathways is increased by at least 1.5-fold or at least two-fold (*e.g.,* at least 1.5-fold, at least two-fold, at least three-fold, at least four-fold, at least five-fold, at least six-fold, at least seven-fold, at least eight-fold, at least nine-fold, at least ten-fold or more) in the first translational profile as to the second translational profile. In some embodiments, less than 20% of the genes in the genome are differentially translated by at least 1.5-fold or at least two-fold in the first translational profile as compared to the second translational profile. In some aspects, less than 5% of the genes in the genome are differentially translated by at least 1.5-fold, at least 2-fold, at least 3-fold, or at least 4-fold in the first translational profile as compared to the second translational profile. In some aspects, less than 1% of the genes in the genome are differentially translated by at least 1.5-fold, at least 2-fold, at least 3-fold, at least 4-fold, or at least 5-fold in the first translational profile as compared to the second translational profile.

Drug candidate molecules or agents are not limited by therapeutic category, and can include, for example, analgesics, anti-inflammatory agents, antihelminthics, anti-arrhythmic agents, anti-bacterial agents, anti-viral agents, anti-coagulants, anti-depressants, antidiabetics, anti-epileptics, anti-fungal agent, anti-gout agents, anti-hypertensive agents, anti-malarials, anti-migraine agents, anti-muscarinic agents, anti-neoplastic agents, erectile dysfunction improvement agents, immunosuppresants, anti-protozoal agents, anti-thyroid agents, anxiolytic agents, sedatives, hypnotics, neuroleptics, β-blockers, cardiac inotropic agents, corticosteroids, diuretics, anti-parkinsonian agents, gastro-intestinal agents, histamine receptor antagonists, keratolytics, lipid regulating agents, anti-anginal agents, Cox-2 inhibitors, leukotriene inhibitors, macrolides, muscle relaxants, anti-osteoporosis agents, anti-obesity agents, cognition enhancers, anti-urinary incontinence agents, nutritional oils, anti-benign prostate hypertrophy agents, essential fatty acids, non-essential fatty acids, and the like, as well as mixtures thereof.

In some aspects, the method further comprises comparing the translational profile for the contacted biological sample with a control translational profile for a second biological sample that has been contacted with a known active compound or therapeutic agent. For example, an active compound or therapeutic agent may be known as useful for treating a cancer, a fibrotic disorder, a neurodegenerative disease or disorder, a neurocognitive or neurodevelopmental disorder, an inflammatory disease or disorder, an autoimmune disease or disorder, a viral infection, or the like. In some cases, a candidate agent may mimic the action of an active compound or therapeutic agent known to have a particular function or induce a particular biological effect or phenotypic change in a cell or a subject. In certain aspects, a candidate agent is identified as a mimic of a known active compound or therapeutic agent by causing a shift in the translational profile to be comparable or similar to the translational profile induced by the known active compound or therapeutic agent. In some aspects, the known therapeutic agent is a known inhibitor of an oncogenic pathway. In some aspects, the known therapeutic agent is a known inhibitor of the mammalian target of rapamycin (mTOR) pathway, the PI3K pathway, the AKT pathway, the Ras pathway, the Myc pathway, the Wnt pathway, or the BRAF pathway. In some aspects, the known therapeutic agent is a known inhibitor of the mTOR pathway.

In some aspects, a method for identifying a drug candidate molecule or agent useful for treating a disease (*e.g.,* an inflammatory disease, an autoimmune disease, a fibrotic disorder, a neurodegenerative disease, a neurodevelopmental disease, a metabolic disease, a viral infection, a cardiomyopathy or a cancer), comprises:
(a) determining a first translational profile for a plurality of genes from a disease sample contacted with a candidate agent;
(b) determining a second translational profile for a plurality of genes from a control disease sample contacted with a known active compound; and
(c) identifying the agent as a candidate therapeutic for use in treating a disease (*e.g.,* an inflammatory disease, an autoimmune disease, a fibrotic disorder, a neurodegenerative disease, a neurodevelopmental disease, a metabolic disease, a viral infection, a cardiomyopathy or a cancer, respectively) when one or more genes are differentially translated in the first translational profile as compared to the second translational profile and when the differential translation results in a biological benefit.

In some aspects, a method for identifying a drug candidate molecule or agent useful for treating a disease (*e.g.,* an inflammatory disease, an autoimmune disease, a fibrotic disorder, a neurodegenerative disease, a neurodevelopmental disease, a metabolic disease, a viral infection, a cardiomyopathy or a cancer), comprises:
(a) determining a first translational profile for a plurality of genes from a disease sample contacted with a candidate agent;
(b) determining a second translational profile for a plurality of genes from a control disease sample, respectively, contacted with a known active compound; and
(c) identifying the agent as a candidate therapeutic for use in treating a disease (*e.g.,* an inflammatory disease, an autoimmune disease, a fibrotic disorder, a neurodegenerative disease, a neurodevelopmental disease, a metabolic disease, a viral infection, a cardiomyopathy or a cancer, respectively), when the first translational profile is comparable to the second translational profile.

In still more aspects, a method for identifying a drug candidate molecule or agent useful for treating a disease (*e.g.,* an inflammatory disease, an autoimmune disease, a fibrotic disorder, a neurodegenerative disease, a neurodevelopmental disease, a metabolic disease, a viral infection, a cardiomyopathy or a cancer), comprises:
(a) determining three independent translational profiles, each for a plurality of genes from a disease sample, wherein (i) a first translational profile is from the sample not contacted with any compound, (ii) a second translational profile is from the sample contacted with a known active compound, and (iii) a third translational profile is from the sample contacted with a candidate agent;
(b) identifying one or more genes as differentially translated in the first translational profile as compared to the second translational profile; and
(c) identifying the agent as a candidate therapeutic for use in treating a disease (*e.g.,* an inflammatory disease, an autoimmune disease, a fibrotic disorder, a neurodegenerative disease, a neurodevelopmental disease, a metabolic disease, a viral infection, a cardiomyopathy or a cancer, respectively) when the one or more differentially translated genes from step (b) are in the third translational profile and have a translational profile closer to the translational profile of the one or more genes in the second translational profile than to the translational profile of the one or more genes in the first translational profile.

In further aspects, a method for identifying a drug candidate molecule or agent useful for treating a disease (*e.g.,* a cancer, an inflammatory disease, an autoimmune disease, a fibrotic disorder, a neurodegenerative disease, a neurodevelopmental disease, a metabolic disease, a viral infection, or a cardiomyopathy), comprises:
(a) determining three independent translational profiles, each for a plurality of genes from a disease sample, wherein (i) a first translational profile is from the sample not contacted with any compound, (ii) a second translational profile is from the sample contacted with a known active compound, and (iii) a third translational profile is from the sample contacted with a candidate agent;
(b) determining a first differential translational profile comprising one or more genes differentially translated in the first translational profile as compared to the second translational profile, and determining a second differential translational profile comprising one or more genes differentially translated in the first translational profile as compared to the third translational profile; and
(c) identifying the agent as a candidate therapeutic for use in treating a disease (*e.g.,* a cancer, an inflammatory disease, an autoimmune disease, a fibrotic disorder, a neurodegenerative disease, a neurodevelopmental disease, a metabolic disease, a viral infection, or a cardiomyopathy, respectively) when the first differential translational profile is comparable to the second differential translational profile.

In some aspects, the differentially translated genes comprise one or more biological pathways, such as at least two or at least three biological pathways. In certain aspects, the one or more differentially translated genes comprise a plurality of genes and optionally the plurality of differentially translated genes may comprise one or more gene signatures. In further aspects, the one or more genes are differentially translated at least a two-fold or more. In still further aspects, each translational profile comprises at least 100 genes, at least 200 genes, at least 300 genes, at least 400 genes, at least 500 genes, or each translational profile comprises a genome-wide translational profile. For example, less than about 25%, about 20%, about 15%, about 10%, about 5%, about 4% , about 3% , about 2% or about 1% of the genes in the genome are differentially translated in a translational profile from a disease sample treated with a drug candidate molecule or agent as compared to a translational profile of an untreated disease sample.

In some aspects, the known active compound is for use in treating an inflammatory disease, autoimmune disease, fibrotic disorder, neurodegenerative disease, neurodevelopmental disease, metabolic disease, viral infection, cardiomyopathy or cancer. In some embodiments, the known active compound is a therapeutic for use in treating a cancer selected from prostate cancer, breast cancer, bladder cancer, lung cancer, renal cell carcinoma, endometrial cancer, melanoma, ovarian cancer, thyroid cancer, or brain cancer. In some aspects, the known active compound is a therapeutic for use in treating an inflammatory disease selected from ankylosing spondylitis, atherosclerosis, multiple sclerosis, systemic lupus erythematosus (SLE), psoriasis, psoriatic arthritis, rheumatoid arthritis, ulcerative colitis, inflammatory bowel disease, or Crohn's disease. In some aspects, the known active compound is a therapeutic for use in treating a fibrotic disorder selected from pulmonary fibrosis, idiopathic pulmonary fibrosis, cystic fibrosis, liver fibrosis, cardiac fibrosis, endomyocardial fibrosis, atrial fibrosis, mediastinal fibrosis, myelofibrosis, retroperitoneal fibrosis, chronic kidney disease, nephrogenic systemic fibrosis, Crohn's disease, hypertrophic scarring, keloid, scleroderma, organ transplant associated fibrosis, or ischemia associated fibrosis. In some aspects, the known active compound is a therapeutic for use in treating a neurodegenerative disease selected from Parkinson's disease, Alzheimer's disease, Amyotrophic Lateral Sclerosis, Creutzfeldt-Jakob disease, Huntington's disease, Lewy body dementia, frontotemporal dementia, corticobasal degeneration, primary progressive aphasia or progressive supranuclear palsy. In some aspects, the known active compound is a therapeutic for use in treating a neurodevelopmental disease selected from autism, autism spectrum disorders, Fragile X Syndrome, attention deficit disorder, or a pervasive development disorder. In some aspects, the known active compound is a therapeutic for use in treating a viral infection selected from adenovirus, bunyavirus, herpesvirus, papovavirus, paramyxovirus, picornavirus, rhabdovirus, orthomyxovirus, poxvirus, reovirus, retrovirus, lentivirus, or flavivirus.

In some aspects, the translational profiles comprise one or more gene signatures, wherein one or more gene signatures are comparable in the first and second translational profiles. In certain aspects, the first and second translational profiles are comparable when an amount of protein translated from one or more differentially translated genes in the first and second translational profiles differs by no more than about 25%, 20%, 15%, 10%, 5%, 1% or less. In further aspects, the one or more differentially translated genes from the third translational profile have a translational profile closer to the translational profile of the one or more genes in the second translational profile when the amount of protein translated from the one or more differentially translated genes in the third and second translational profiles differs by no more than about 25%, 20%, 15%, 10%, 5%, 1% or less.

In some aspects, the methods of identifying a drug candidate molecule as described herein are used to compare a group of drug candidate molecules and select one drug candidate molecule or a smaller subgroup of drug candidate molecules from this group. In some aspects, the methods described herein are used to compare drug candidate molecules and select one candidate molecule or a subgroup of drug candidate molecules which alter the translation of a relatively smaller number of proteins, as compared to the number of proteins for which translational is altered for the larger group of drug candidate molecules. In some aspects, the methods described herein are used to compare drug candidate molecules and select one candidate molecule or a subgroup of drug candidate molecules for which altered translation resides in a relatively smaller number of pathways, as compared to the number of pathways for which translation is altered for the larger group of drug candidate molecules. In some aspects, the methods described herein are used to compare drug candidate molecules and select one candidate molecule or a subgroup of drug candidate molecules which alter the translation of several proteins within one specific pathway, as compared to the larger group of drug candidate molecules for which a smaller number of proteins within that one specific pathway have altered translation.

### VII. Therapeutic Methods

In yet another aspect, the present disclosure provides therapeutic methods for identifying subjects for treatment and treating subjects in need thereof. The present invention relates to methods of identifying a subject as a candidate for treatment, *e.g.*, for treatment with an mTOR inhibitor. In some aspects, the present disclosure relates to methods of treating a subject, *e.g.,* a subject having a cancer, an inflammatory disease, an autoimmune disease, a fibrotic disorder, a neurodegenerative disease, a neurodevelopmental disease, a metabolic disease, or a viral infection.

### A. Identifying Subjects for Treatment

In some embodiments, the present invention relates to a method of identifying a subject as a candidate for treatment with an mTOR inhibitor. In some aspects, the method comprises:
(a) determining a first translational profile in a sample from the subject, wherein the first translational profile comprises translational levels for one or more genes having a 5' terminal oligopyrimidine tract (5' TOP) and/or a pyrimidine-rich translational element (PRTE); and
(b) comparing the first translational profile to a second translational profile comprising translational levels for the one or more genes, wherein the second translational profile is from a control sample, wherein the control sample is from a known responder to the mTOR inhibitor prior to administration of the mTOR inhibitor to the known responder;
wherein a translational level of the one or more genes in the first translational profile that is at least as high as the translational level of the one or more genes in the second translational profile identifies the subject as a candidate for treatment with the mTOR inhibitor.

In some aspects, the one or more genes (*e.g.,* the 1, 2, 3, 4, 5, 6, 7, 8, 9, 10, 11, 12, 13, 14, 15, 16, 17, 18, 19, 20 or more genes) are selected from the genes listed in any of Table 1, Table 2, or Table 3.

In some aspects, a method of identifying a subject as a candidate for treatment with an mTOR inhibitor comprises:
(a) determining a first translational profile in a sample from the subject, wherein the first translational profile comprises translational levels for one or more genes selected from the group consisting of SEQ ID NOs:1-144; and
(b) comparing the first translational profile to a second translational profile comprising translational levels for the one or more genes, wherein the second translational profile is from a control sample, wherein the control sample is from a known responder to the mTOR inhibitor prior to administration of the mTOR inhibitor to the known responder;
wherein a translational level of the one or more genes in the first translational profile that is at least as high as the translational level of the one or more genes in the second translational profile identifies the subject as a candidate for treatment with the mTOR inhibitor.

In some aspects, the one or more genes (*e.g.,* the 1, 2, 3, 4, 5, 6, 7, 8, 9, 10, 11, 12, 13, 14, 15, 16, 17, 18, 19, 20 or more genes) are cell invasion/metastasis genes. In some aspects, the one or more genes are selected from YB1, vimentin, MTA1, and CD44.

In some aspects, a method of identifying a subject as a candidate for treatment with an mTOR inhibitor comprises:
(a) determining a first translational profile in a sample from the subject, wherein the first translational profile comprises translational levels for one or more genes of a biological pathway, wherein the biological pathway is selected from a protein synthesis pathway, a cell invasion/metastasis pathway, a cell division pathway, an apoptosis pathway, a signal transduction pathway, a cellular transport pathway, a post-translational protein modification pathway, a DNA repair pathway, and a DNA methylation pathway; and
(b) comparing the first translational profile to a second translational profile comprising translational levels for the one or more genes, wherein the second translational profile is from a control sample, wherein the control sample is from a known responder to the mTOR inhibitor prior to administration of the mTOR inhibitor to the known responder;
wherein a translational level of the one or more genes in the first translational profile that is at least as high as the translational level of the one or more genes in the second translational profile identifies the subject as a candidate for treatment with the mTOR inhibitor.

The methods of the present invention relate to a method of identifying a subject as a candidate for treatment with a therapeutic agent. In some aspects, the method comprises:
(a) determining a first translational profile in a sample from the subject, wherein the translational profile comprises translational levels for one or more genes of a biological pathway, wherein the biological pathway is selected from a protein synthesis pathway, a cell invasion/metastasis pathway, a cell division pathway, an apoptosis pathway, a signal transduction pathway, a cellular transport pathway, a post-translational protein modification pathway, a DNA repair pathway, and a DNA methylation pathway; and
(b) comparing the first translational profile to a second translational profile comprising translational levels for the one or more genes, wherein the second translational profile is from a control sample, wherein the control sample is from a known responder to the therapeutic agent prior to administration of the therapeutic agent to the known responder;
wherein a translational level of the one or more genes that is at least as high as the translational level of the one or more genes in the second translational profile identifies the subject as a candidate for treatment with the therapeutic agent.

In further aspects, a method for identifying a subject as a candidate for treating a disease (*e.g.,* a cancer, an inflammatory disease, an autoimmune disease, a fibrotic disorder, a neurodegenerative disease, a neurodevelopmental disease, a metabolic disease, a viral infection, or a cardiomyopathy), comprises:
(a) determining a first translational profile for a plurality of genes in a sample from a subject having or suspected of having a disease;
(b) determining a second translational profile for a plurality of genes in a control disease sample, wherein the control sample is from a subject known to respond to the therapeutic agent and the sample has not been contacted with a therapeutic agent for a disease; and
(c) identifying the subject as a candidate for treating a disease (*e.g.,* a cancer, an inflammatory disease, an autoimmune disease, a fibrotic disorder, a neurodegenerative disease, a neurodevelopmental disease, a metabolic disease, a viral infection, or a cardiomyopathy) (*e.g*., a cancer, an inflammatory disease, an autoimmune disease, a fibrotic disorder, a neurodegenerative disease, a neurodevelopmental disease, a metabolic disease, a viral infection, or a cardiomyopathy, respectively) with a therapeutic agent when the first translational profile is comparable to the second translational profile.

In some embodiments, the translational profiles comprise one or more gene signatures, wherein one or more gene signatures are comparable in the first and second translational profiles. In certain embodiments, the first and second translational profiles are comparable when an amount of protein translated from one or more differentially translated genes in the first and second translational profiles differs by no more than about 25%, 20%, 15%, 10%, 5%, 1% or less.

In some aspects, translational levels are compared for the first translational profile and the second translational profile for 1, 2, 3, 4, 5, 6, 7, 8, 9, 10, 11, 12, 13, 14, 15, 16, 17, 18, 19, 20 or more genes in one or more biological pathways. In some embodiments, the translational level of one or more genes from each of at least two of the biological pathways is at least as high in the first translational profile as compared to the second translational profile. In some embodiments, the translational level of one or more genes from each of at least three of the biological pathways is at least as high in the first translational profile as compared to the second translational profile.

In some embodiments, the first and/or second translational profiles comprise translational levels for at least 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, 500, 550, 600, 650, 700, 750, 800, 850, 900, 950, 1000, 1500, 2000, 2500, 3000, 3500, 4000, 4500, 5000, 5500, 6000, 6500, 7000, 7500, 8000, 8500, 9000, 9500, 10,000 genes or more in the biological sample. In some embodiments, the first and/or second translational profile comprises translational levels for at least about 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50% or more of all genes in the biological sample. In some embodiments, the translational profiles comprise genome-wide measurements of gene translational levels. In some embodiments, the translational level of the one or more genes is increased by at least 1.5-fold or at least two-fold *(e.g.,* at least 1.5-fold, at least two-fold, at least three-fold, at least four-fold, at least five-fold, at least six-fold, at least seven-fold, at least eight-fold, at least nine-fold, at least ten-fold or more) in the first translational profile as to the second translational profile. In some embodiments, the translational level of the one or more genes is decreased by at least 1.5-fold or at least two-fold (*e.g.,* at least 1.5-fold, at least two-fold, at least three-fold, at least four-fold, at least five-fold, at least six-fold, at least seven-fold, at least eight-fold, at least nine-fold, at least ten-fold or more) in the first translational profile as to the second translational profile.

In some embodiments, the disease is a cancer. Non-limiting examples of cancers that can be treated according to the methods of the present disclosure include, but are not limited to, anal carcinoma, bladder carcinoma, breast carcinoma, cervix carcinoma, chronic lymphocytic leukemia, chronic myelogenous leukemia, endometrial carcinoma, hairy cell leukemia, head and neck carcinoma, lung (small cell) carcinoma, multiple myeloma, non-Hodgkin's lymphoma, follicular lymphoma, ovarian carcinoma, brain tumors, colorectal carcinoma, hepatocellular carcinoma, Kaposi's sarcoma, lung (non-small cell carcinoma), melanoma, pancreatic carcinoma, prostate carcinoma, renal cell carcinoma, and soft tissue sarcoma.

In some embodiments, the disease is an inflammatory disease (*e.g.,* an autoimmune disease, arthritis, or MS). In some aspects, the disease is a fibrotic disorder (*e.g*., pulmonary fibrosis, idiopathic pulmonary fibrosis, cystic fibrosis, liver fibrosis, cardiac fibrosis, mediastinal fibrosis, myelofibrosis, keloids, scleroderma, organ transplant associated fibrosis, or ischemia associated fibrosis). In some embodiments, the disease is a neurodegenerative disease (*e.g*., Parkinson's disease, Amyotrophic Lateral Sclerosis (ALS), Creutzfeldt-Jakob disease, Huntington's disease, Lewy body dementia, frontotemporal dementia, corticobasal degeneration, primary progressive aphasia, progressive supranuclear palsy or Alzheimer's disease). In some embodiments, the disease is a neurodevelopmental disease (*e.g*., autism, autism spectrum disorders, Fragile X Syndrome, attention deficit disorder, pervasive development disorders). In some embodiments, the disease is a metabolic disease (*e.g.,* diabetes, metabolic syndrome, or a cardiovascular disease). In some aspects, the disease is a viral infection (*e.g*., adenovirus, herpesvirus, papovavirus, poxvirus, retrovirus, lentivirus, or flavivirus). In some aspects, the disease is a cardiomyopathy.

In some aspects, a disease is associated with one or more altered biological pathways. In some aspects, wherein a cell communication pathway is altered, the disease is an immune or inflammatory disease, a fibrotic disorder, a neurodegenerative disease, a neurodevelopment disease, a cancer, a metabolic disorder, or a viral disease. In some aspects, wherein a cell communication pathway is altered, the disease is an immune or inflammatory disease *(e.g.,* an autoimmune disease, arthritis, or MS).

In some aspects, wherein a cellular process pathway is altered, the disease is an immune or inflammatory disease (*e.g.,* an autoimmune disease, arthritis, or MS), a fibrotic disorder, a neurodegenerative disease (*e.g*., Parkinson's disease, Amyotrophic Lateral Sclerosis (ALS), Creutzfeldt-Jakob disease, Huntington's disease, Lewy body dementia, frontotemporal dementia, corticobasal degeneration, primary progressive aphasia, progressive supranuclear palsy, or Alzheimer's disease), a neurodevelopmental disease (*e.g.*, autism, autism spectrum disorders, Fragile X Syndrome, attention deficit disorder, pervasive development disorders), a cancer, a metabolic disorder, or a viral disease.

In some aspects, wherein an immune system process pathway is altered, the disease is an immune or inflammatory disease, a fibrotic disorder, a neurodegenerative disease, a neurodevelopmental disease, a cancer, a metabolic disorder, or a viral disease. In some aspects, wherein an immune system process pathway is altered, the disease is an immune or inflammatory disease *(e.g.,* an autoimmune disease, arthritis, or MS).

In some aspects, wherein a response to stimulus pathway is altered, the disease is an immune or inflammatory disease, a fibrotic disorder, a neurodegenerative disease, a neurodevelopmental disease, a metabolic disorder, or a viral disease. In some aspects, wherein a response to stimulus pathway is altered, the disease is an immune or inflammatory disease *(e.g.,* an autoimmune disease, arthritis, or MS) or a viral disease.

In some aspects, wherein a transport pathway is altered, the disease is an immune or inflammatory disease, a fibrotic disorder, a neurodegenerative disease, or a metabolic disorder. In some aspects, wherein a transport pathway is altered, the disease is an immune or inflammatory disease *(e.g.,* an autoimmune disease, arthritis, or MS) or a metabolic disorder (*e.g*., diabetes, metabolic syndrome, or a cardiovascular disease).

In some aspects, wherein a metabolic process pathway is altered, the disease is a fibrotic disorder, a neurodegenerative disease, a neurodevelopmental disease, a cancer, or a metabolic disorder. In some embodiments, wherein a metabolic process pathway is altered, the disease is a metabolic disorder (*e.g.*, diabetes, metabolic syndrome, or a cardiovascular disease).

In some aspects, a metabolic process pathway is a carbohydrate metabolic process pathway, a lipid metabolic process pathway, a nucleobase, nucleoside, or nucleotide pathway, or a protein metabolic process pathway (*e*.*g*., a proteolysis pathway, a protein complex assembly pathway, a protein folding pathway, a protein modification process pathway, or a translation pathway). In some aspects, wherein a carbohydrate metabolic process pathway is altered, the disease is a fibrotic disorder, a neurodegenerative disease, or a metabolic disorder. In some aspects, wherein a lipid metabolic process pathway is altered, the disease is an immune or inflammatory disease, a fibrotic disorder, a neurodegenerative disease, or a metabolic disorder. In some aspects, wherein a nucleobase, nucleoside, or nucleotide pathway is altered, the disease is a cancer or a viral disease. In some aspects, wherein a protein metabolic process pathway is altered, the disease is an immune or inflammatory disease, a fibrotic disorder, a neurodegenerative disease, a neurodevelopmental disease, a cancer, a metabolic disorder, or a viral disease. In some aspects, wherein a proteolysis process pathway is altered, the disease is an immune or inflammatory disease, a fibrotic disorder, a neurodegenerative disease, a neurodevelopmental disease, a cancer, or a metabolic disorder. In some embodiments, wherein a protein complex assembly pathway is altered, the disease is a metabolic disorder. In some embodiments, wherein a protein folding pathway is altered, the disease is a neurodegenerative disease. In some aspects, wherein a protein modification process pathway is altered, the disease is an immune or inflammatory disease, a fibrotic disorder, a neurodevelopmental disease, a neurodegenerative disease, a cancer, a metabolic disorder, or a viral disease. In some aspects, wherein a protein translation pathway is altered, the disease is an immune or inflammatory disease, a fibrotic disorder, a neurodegenerative disease, a neurodevelopmental disease, or a cancer.

In some aspects, the method further comprises administering a therapeutic agent to the identified subject. In some aspects, the method further comprises administering an mTOR inhibitor to the identified subject.

### B. Administration of Therapeutic Agents

In some aspects, the present disclosure relates to a method of treating a subject having a cancer. In some aspects, the method comprises:
administering an mTOR inhibitor to a subject that has been selected as having a first translational profile comprising a translational level of one or more genes that is at least as high as the translational level of the one or more genes in a second translational profile from a control sample;
wherein the first and second translational profiles comprise translational levels for one or more genes having a 5' terminal oligopyrimidine tract (5' TOP) and/or a pyrimidine-rich translational element (PRTE); and wherein the control sample is from a known responder to the mTOR inhibitor prior to administration of the mTOR inhibitor to the known responder;
thereby treating the cancer in the subject.

In some aspects, the method of treating a subject having a cancer comprises:
administering an mTOR inhibitor to a subject that has been selected as having a first translational profile comprising a translational level of one or more genes that is at least as high as the translational level of the one or more genes in a second translational profile from a control sample;
wherein the first and second translational profiles comprise translational levels for one or more genes selected from the group consisting of SEQ ID NOs: 1-144; and wherein the control sample is from a known responder to the mTOR inhibitor prior to administration of the mTOR inhibitor to the known responder;
thereby treating the cancer in the subject.

In some aspects, the method of treating a subject having a cancer comprises:
administering an mTOR inhibitor to a subject that has been selected as having a first translational profile comprising a translational level of one or more genes that is at least as high as the translational level of the one or more genes in a second translational profile from a control sample;
wherein the first and second translational profiles comprise translational levels for one or more genes of a biological pathway selected from a protein synthesis pathway, a cell invasion/metastasis pathway, a cell division pathway, an apoptosis pathway, a signal transduction pathway, a cellular transport pathway, a post-translational protein modification pathway, a DNA repair pathway, and a DNA methylation pathway; and wherein the control sample is from a known responder to the mTOR inhibitor prior to administration of the mTOR inhibitor to the known responder;
thereby treating the cancer in the subject.

In some aspects, the present disclosure relates to a method of treating a subject in need thereof. In some aspects, the method comprises:
administering a therapeutic agent to a subject that has been selected as having a first translational profile comprising a translational level of one or more genes that is at least as high as the translational level of the one or more genes in a second translational profile;
wherein the first and second translational profiles comprise translational levels for one or more genes of a biological pathway selected from a protein synthesis pathway, a cell invasion/metastasis pathway, a cell division pathway, an apoptosis pathway, a signal transduction pathway, a cellular transport pathway, a post-translational protein modification pathway, a DNA repair pathway, and a DNA methylation pathway; and wherein the control sample is from a known responder to the therapeutic agent prior to administration of the therapeutic agent to the known responder;
thereby treating the subject.

In certain aspects, a method for treating a disease (*e.g.,* a cancer, an inflammatory disease, an autoimmune disease, a fibrotic disorder, a neurodegenerative disease, a neurodevelopmental disease, a metabolic disease, a viral infection, or a cardiomyopathy), comprises administering to a subject identified by:
(a) determining a first translational profile for a plurality of genes in a sample from a subject having or suspected of having a disease;
(b) determining a second translational profile for a plurality of genes in a control disease sample, wherein the control sample is from a subject known to respond to the therapeutic agent and the sample has not been contacted with a therapeutic agent for a disease; and
(c) identifying the subject as a candidate for treating a disease (*e.g.,* a cancer, an inflammatory disease, an autoimmune disease, a fibrotic disorder, a neurodegenerative disease, a neurodevelopmental disease, a metabolic disease, a viral infection, or a cardiomyopathy, respectively) with a therapeutic agent when the first translational profile is comparable to the second translational profile;
thereby treating the subject.

In further aspects, a method for treating a cancer, an inflammatory disease, an autoimmune disease, a fibrotic disorder, a neurodegenerative disease, a neurodevelopmental disease, a metabolic disease, a viral infection, or cardiomyopathy, comprises administering to a subject having a disease an agent or drug candidate molecule identified according to any one of the methods provided herein, thereby treating the subject.

In still further aspects, a method for treating a cancer, an inflammatory disease, an autoimmune disease, a fibrotic disorder, a neurodegenerative disease, a neurodevelopmental disease, a metabolic disease, a viral infection, or a cardiomyopathy, comprises administering to a subject having a disease an agent that modulates a target, wherein the target was validated according to any one of the methods provided herein, thereby treating the subject.

In yet further aspects, a method for treating a cancer, an inflammatory disease, an autoimmune disease, a fibrotic disorder, a neurodegenerative disease, a neurodevelopmental disease, a metabolic disease, a viral infection, or cardiomyopathy, by normalizing the disease translational profile, comprises administering to a subject having a disease an agent that modulates a target, wherein the target was validated according to any one of the methods provided herein, thereby treating the subject.

In any of the aforementioned aspects for treating a cancer, an inflammatory disease, an autoimmune disease, a fibrotic disorder, a neurodegenerative disease, a neurodevelopmental disease, a metabolic disease, a viral infection, or cardiomyopathy according to a validated target, the target that was validated was suspected of being associated with a disease, was indirectly associated with a disease, or was associated with a disease (*e.g.,* an inflammatory disease, an autoimmune disease, a fibrotic disorder, a neurodegenerative disease, a neurodevelopmental disease, a metabolic disease, a viral infection, a cardiomyopathy or a cancer, respectively).

In some aspects, the translational profiles comprise one or more gene signatures, wherein one or more gene signatures are comparable in the first and second translational profiles. In certain aspects, the first and second translational profiles are comparable when an amount of protein translated from one or more differentially translated genes in the first and second translational profiles differs by no more than about 50%, 45%, 40%, 35%, 30%, 25%, 20%, 15%, 10%, 5%, 1% or less. In further aspects, the one or more differentially translated genes from the third translational profile have a translational profile closer to the translational profile of the one or more genes in the second translational profile when the amount of protein translated from the one or more differentially translated genes in the third and second translational profiles differs by no more than about 50%, 45%, 40%, 35%, 30%, 25%, 20%, 15%, 10%, 5%, 1% or less.

A subject is selected for therapeutic treatment based on any of the translational profiling methods as described herein. In some aspects, the subject has a disease. In some embodiments, the disease is an inflammatory disease. In some aspects, the disease is a fibrotic disorder. In some aspects, the disease is a neurodegenerative disease. In some aspects, the disease is a neurodevelopmental disease. In some aspects, the disease is a metabolic disease. In some aspects, the disease is viral infection. In some aspects, the disease is a cardiomyopathy. In some aspects, the disease is cancer.

Non-limiting examples of cancers that can be treated according to the methods of the present disclosure include, but are not limited to, anal carcinoma, bladder carcinoma, breast carcinoma, cervix carcinoma, chronic lymphocytic leukemia, chronic myelogenous leukemia, endometrial carcinoma, hairy cell leukemia, head and neck carcinoma, lung (small cell) carcinoma, multiple myeloma, non-Hodgkin's lymphoma, follicular lymphoma, ovarian carcinoma, brain tumors, colorectal carcinoma, hepatocellular carcinoma, Kaposi's sarcoma, lung (non-small cell carcinoma), melanoma, pancreatic carcinoma, prostate carcinoma, renal cell carcinoma, and soft tissue sarcoma. In some aspects, the cancer is prostate cancer, breast cancer, bladder cancer, lung cancer, renal cell carcinoma, endometrial cancer, melanoma, ovarian cancer, thyroid cancer, or brain cancer. In some aspects, the cancer is an invasive cancer.

Non-limiting examples of inflammatory and autoimmune diseases that can be treated according to the methods of the present disclosure include, but are not limited to, arthritis, rheumatoid arthritis, juvenile rheumatoid arthritis, osteoarthritis, polychondritis, psoriatic arthritis, psoriasis, dermatitis, polymyositis/dermatomyositis, inclusion body myositis, inflammatory myositis, toxic epidermal necrolysis, systemic scleroderma and sclerosis, CREST syndrome, inflammatory bowel disease, Crohn's disease, ulcerative colitis, respiratory distress syndrome, adult respiratory distress syndrome (ARDS), chronic obstructive pulmonary disease, meningitis, encephalitis, uveitis, colitis, glomerulonephritis, allergic conditions, eczema, asthma, conditions involving infiltration of T cells and chronic inflammatory responses, atherosclerosis, autoimmune myocarditis, leukocyte adhesion deficiency, systemic lupus erythematosus (SLE), subacute cutaneous lupus erythematosus, discoid lupus, lupus myelitis, lupus cerebritis, juvenile onset diabetes, multiple sclerosis (MS), allergic encephalomyelitis, neuromyelitis optica, rheumatic fever, Sydenham's chorea, immune responses associated with acute and delayed hypersensitivity mediated by cytokines and T-lymphocytes, tuberculosis, sarcoidosis, granulomatosis including Wegener's granulomatosis and Churg-Strauss disease, agranulocytosis, vasculitis (including hypersensitivity vasculitis/angiitis, ANCA and rheumatoid vasculitis), aplastic anemia, Diamond Blackfan anemia, immune hemolytic anemia including autoimmune hemolytic anemia (AIHA), pernicious anemia, pure red cell aplasia (PRCA), Factor VIII deficiency, hemophilia A, autoimmune neutropenia, pancytopenia, leukopenia, diseases involving leukocyte diapedesis, central nervous system (CNS) inflammatory disorders, multiple organ injury syndrome, myasthenia gravis, antigen-antibody complex mediated diseases, anti-glomerular basement membrane disease, anti-phospholipid antibody syndrome, allergic neuritis, Behcet disease, Castleman's syndrome, Goodpasture's syndrome, Lambert-Eaton Myasthenic Syndrome, Reynaud's syndrome, Sjorgen's syndrome, Stevens-Johnson syndrome, solid organ transplant rejection, graft-versus-host disease (GVHD), bullous pemphigoid, pemphigus, autoimmune polyendocrinopathies, seronegative spondyloarthropathies, Reiter's disease, stiff-man syndrome, giant cell arteritis, immune complex nephritis, IgA nephropathy, IgM polyneuropathies or IgM mediated neuropathy, idiopathic thrombocytopenic purpura (ITP), thrombotic throbocytopenic purpura (TTP), Henoch-Schonlein purpura, autoimmune thrombocytopenia, autoimmune disease of the testis and ovary including autoimmune orchitis and oophoritis, primary hypothyroidism; autoimmune endocrine diseases including autoimmune thyroiditis, chronic thyroiditis (Hashimoto's Thyroiditis), subacute thyroiditis, idiopathic hypothyroidism, Addison's disease, Grave's disease, autoimmune polyglandular syndromes (or polyglandular endocrinopathy syndromes), Type I diabetes (also referred to as insulin-dependent diabetes mellitus or IDDM); autoimmune hepatitis, lymphoid interstitial pneumonitis (HIV), bronchiolitis obliterans (non-transplant), non-specific interstitial pneumonia (NSIP), Guillain-BarréSyndrome, large vessel vasculitis (including polymyalgia rheumatica and giant cell (Takayasu's) arteritis), medium vessel vasculitis (including Kawasaki's disease and polyarteritis nodosa), polyarteritis nodosa (PAN), ankylosing spondylitis, Berger's disease (IgA nephropathy), rapidly progressive glomerulonephritis, primary biliary cirrhosis, Celiac sprue (gluten enteropathy), cryoglobulinemia, cryoglobulinemia associated with hepatitis, amyotrophic lateral sclerosis (ALS), coronary artery disease, familial Mediterranean fever, microscopic polyangiitis, Cogan's syndrome, Whiskott-Aldrich syndrome and thromboangiitis obliterans.. In some aspects, the inflammatory disease is ankylosing spondylitis, multiple sclerosis, systemic lupus erythematosus (SLE), rheumatoid arthritis, atherosclerosis, inflammatory bowel disease, or Crohn's disease.

Non-limiting examples of infectious viruses include adenovirus, bunyavirus (*e.g*., Hantavirus), herpesvirus, papovavirus, paramyxovirus, picornavirus, rhabdovirus (*e.g*., Rabies), orthomyxovirus (*e.g.,* influenza), poxvirus (*e.g.,* Vaccinia), reovirus, retrovirus, lentivirus (*e.g.,* HIV), flavivirus (*e.g.,* HCV), or the like).

The term "fibrotic disorder" or "fibrotic disease" refers to a medical condition featuring progressive and/or irreversible fibrosis, wherein excessive deposition of extracellular matrix occurs in and around inflamed or damaged tissue. Excessive and persistent fibrosis can progressively remodel and destroy normal tissue, which may lead to dysfunction and failure of affected organs, and ultimately death. A fibrotic disorder may affect any tissue in the body and is generally initiated by an injury. It is to be understood that fibrosis alone triggered by normal wound healing processes that has not progressed to a pathogenic state is not considered a fibrotic disorder or disease of this disclosure. A "fibrotic lesion" or "fibrotic plaque" refers to a focal area of fibrosis. As used herein, "injury" refers to an event that damages tissue and initiates fibrosis. An injury may be caused by an external factor, such as mechanical insult (*e.g.,* cut, surgery), exposure to radiation, chemicals (*e.g.,* chemotherapy, toxins, irritants, smoke), or infectious agent (*e.g*., bacteria, virus, or parasite). An injury may be caused by, for example, chronic autoimmune inflammation, allergic response, HLA mismatching (*e.g.,* transplant recipients), or ischemia (*i.e.,* an "ischemic event" or "ischemia" refers to an injury that restricts in blood supply to a tissue, resulting in damage to or dysfunction of tissue, which may be caused by problems with blood vessels, atherosclerosis, thrombosis or embolism, and may affect a variety of tissues and organs; an ischemic event may include, for example, a myocardial infarction, stroke, organ or tissue transplant, or renal artery stenosis). In certain aspects, an injury leading to a fibrotic disorder may be of unknown etiology (*i.e.,* idiopathic).

Non-limiting examples of fibrotic disorders or fibrotic diseases include pulmonary fibrosis, idiopathic pulmonary fibrosis, cystic fibrosis, liver fibrosis (*e.g*., cirrhosis), cardiac fibrosis, endomyocardial fibrosis, atrial fibrosis, mediastinal fibrosis, myelofibrosis, retroperitoneal fibrosis, progressive massive fibrosis (*e.g*., lungs), chronic kidney disease, nephrogenic systemic fibrosis, Crohn's disease, hypertrophic scarring, keloid, scleroderma, systemic sclerosis *(e.g.,* skin, lungs), athrofibrosis *(e.g.,* knee, shoulder, other joints), Peyronie's disease, Dupuytren's contracture, adhesive capsulitis, organ transplant associated fibrosis, ischemia associated fibrosis, or the like.

A therapeutic agent for use according to any of the methods of the present disclosure can be any composition that has or may have a pharmacological activity. Agents include compounds that are known drugs, compounds for which pharmacological activity has been identified but which are undergoing further therapeutic evaluation, and compounds that are members of collections and libraries that are screened for a pharmacological activity. In some embodiments, the therapeutic agent is an anti-cancer, *e.g.,* an anti-signaling agent (*e.g.,* a cytostatic drug) such as a monoclonal antibody or a tyrosine kinase inhibitor; an antiproliferative agent; a chemotherapeutic agent (*i.e.,* a cytotoxic drug); a hormonal therapeutic agent; and/or a radiotherapeutic agent.

Generally, the therapeutic agent is administered at a therapeutically effective amount or dose. A therapeutically effective amount or dose will vary according to several factors, including the chosen route of administration, the formulation of the composition, patient response, the severity of the condition, the subject's weight, and the judgment of the prescribing physician. The dosage can be increased or decreased over time, as required by an individual patient. In certain instances, a patient initially is given a low dose, which is then increased to an efficacious dosage tolerable to the patient. Determination of an effective amount is well within the capability of those skilled in the art.

The route of administration of a therapeutic agent can be oral, intraperitoneal, transdermal, subcutaneous, by intravenous or intramuscular injection, by inhalation, topical, intralesional, infusion; liposome-mediated delivery; topical, intrathecal, gingival pocket, rectal, intrabronchial, nasal, transmucosal, intestinal, ocular or otic delivery, or any other methods known in the art.

In some aspects, a therapeutic agent is formulated as a pharmaceutical composition. In some aspects, a pharmaceutical composition incorporates particulate forms, protective coatings, protease inhibitors, or permeation enhancers for various routes of administration, including parenteral, pulmonary, nasal and oral. The pharmaceutical compositions can be administered in a variety of unit dosage forms depending upon the method/mode of administration. Suitable unit dosage forms include, but are not limited to, powders, tablets, pills, capsules, lozenges, suppositories, patches, nasal sprays, injectibles, implantable sustained-release formulations, *etc.*

In some aspects, a pharmaceutical composition comprises an acceptable carrier and/or excipients. A pharmaceutically acceptable carrier includes any solvents, dispersion media, or coatings that are physiologically compatible and that preferably does not interfere with or otherwise inhibit the activity of the therapeutic agent. Preferably, the carrier is suitable for intravenous, intramuscular, oral, intraperitoneal, transdermal, topical, or subcutaneous administration. Pharmaceutically acceptable carriers can contain one or more physiologically acceptable compound(s) that act, for example, to stabilize the composition or to increase or decrease the absorption of the active agent(s). Physiologically acceptable compounds can include, for example, carbohydrates, such as glucose, sucrose, or dextrans, antioxidants, such as ascorbic acid or glutathione, chelating agents, low molecular weight proteins, compositions that reduce the clearance or hydrolysis of the active agents, or excipients or other stabilizers and/or buffers. Other pharmaceutically acceptable carriers and their formulations are well-known and generally described in, for example, Remington: The Science and Practice of Pharmacy, 21st Edition, Philadelphia, PA. Lippincott Williams & Wilkins, 2005. Various pharmaceutically acceptable excipients are well-known in the art and can be found in, for example, Handbook of Pharmaceutical Excipients (5th ed., Ed. Rowe et al., Pharmaceutical Press, Washington, D.C.).

### C. Normalizing Translational Profiles

In another aspect, the methods of the present disclosure relate to normalizing a translational profile in a subject. In some aspects, the present disclosure provides a method of identifying an agent or therapeutic for normalizing a translational profile in a subject. In some aspects, the present disclosure provides a method of validating a target for normalizing a translational profile associated with a disease. In some aspects, the method comprises:
(a) determining a first translational profile for a first biological sample from the subject, wherein the first translational profile comprises translational levels for a plurality of genes;
(b) comparing the first translational profile to a second translational profile comprising translational levels for the plurality of genes, wherein the second translational profile is from a control sample, wherein the control sample is from a non-diseased subject;
(c) identifying one or more genes of a biological pathway as differentially translated in the first translational profile as compared to the second translational profile, wherein the biological pathway is selected from a protein synthesis pathway, a cell invasion/metastasis pathway, a cell division pathway, an apoptosis pathway, a signal transduction pathway, a cellular transport pathway, a post-translational protein modification pathway, a DNA repair pathway, and a DNA methylation pathway;
(d) contacting a second biological sample from the subject with the agent;
(e) determining a third translational profile for the second biological sample, wherein the third translational profile comprises translational levels for the one or more genes identified as differentially translated in the first translational profile as compared to the second translational profile; and
(f) comparing the translational levels for the one or more genes in the third translational profile to the translational levels for the one or more genes in the first and second translational profiles;
wherein a translational level for the one or more genes in the third translational profile that is closer to the translational level for the one or more genes in the second translational profile than to the translational level for the one or more genes in the first translational profile identifies the agent as an agent for normalizing the translational profile in the subject.

In some aspects, the present disclosure provides a method of normalizing a translational profile in a subject. In some embodiments, the method comprises:
administering to the subject an agent that has been selected as an agent that normalizes the translational profile in the subject, wherein the agent is selected by:
   (a) determining a first translational profile for a first biological sample from the subject, wherein the first translational profile comprises translational levels for a plurality of genes;
   (b) comparing the first translational profile to a second translational profile comprising translational levels for the plurality of genes, wherein the second translational profile is from a control sample, wherein the control sample is from a non-diseased subject;
   (c) identifying one or more genes of a biological pathway as differentially translated in the first translational profile as compared to the second translational profile, wherein the biological pathway is selected from a protein synthesis pathway, a cell invasion/metastasis pathway, a cell division pathway, an apoptosis pathway, a signal transduction pathway, a cellular transport pathway, a post-translational protein modification pathway, a DNA repair pathway, and a DNA methylation pathway;
   (d) contacting a second biological sample form the subject with the agent;
   (e) determining a third translational profile for the second biological sample, wherein the third translational profile comprises translational levels for the one or more genes identified as differentially translated in the first translational profile as compared to the second translational profile; and
   (f) comparing the translational levels for the one or more genes in the third translational profile to the translational levels for the one or more genes in the first and second translational profiles; wherein a translational level for the one or more genes in the third translational profile that is closer to the translational level for the one or more genes in the second translational profile than to the translational level for the one or more genes in the first translational profile identifies the agent as an agent for normalizing the translational profile in the subject;
thereby normalizing the translational profile in the subject.

In certain aspects, the present disclosure provides a method for identifying an agent or drug candidate molecule (*i.e.,* a candidate therapeutic) for normalizing a translational profile associated with a disease (*e.g.,* a cancer, an inflammatory disease, an autoimmune disease, a fibrotic disorder, a neurodegenerative disease, a neurodevelopmental disease, a metabolic disease, a viral infection, or a cardiomyopathy), comprising:
(a) determining a first translational profile for a plurality of genes from a disease sample contacted with an agent or drug candidate molecule;
(b) determining a second translational profile for a plurality of genes from (i) a control non-diseased sample or (ii) a control non-diseased sample contacted with the agent or drug candidate molecule; and
(c) identifying the agent or drug candidate molecule as useful for normalizing a translational profile associated with a disease *(e.g.,* a cancer, an inflammatory disease, an autoimmune disease, a fibrotic disorder, a neurodegenerative disease, a neurodevelopmental disease, a metabolic disease, a viral infection, or a cardiomyopathy, respectively) when the first translational profile is comparable to the second translational profile.

In certain aspects, the present disclosure provides a method for identifying an agent or drug candidate molecule (*i.e.,* a candidate therapeutic) for normalizing a translational profile associated with a disease (*e.g.,* a cancer, an inflammatory disease, an autoimmune disease, a fibrotic disorder, a neurodegenerative disease, a neurodevelopmental disease, a metabolic disease, a viral infection, or a cardiomyopathy), comprising:
(a) determining three independent translational profiles, each for a plurality of genes, wherein (i) a first translational profile is from a disease sample, (ii) a second translational profile is from (1) a control non-diseased sample or (2) a control non-diseased sample contacted with an agent or drug candidate molecule, and (iii) a third translational profile is from the disease sample contacted with the agent or drug candidate molecule;
(b) identifying one or more genes as differentially translated in the first translational profile as compared to the second translational profile; and
(c) identifying the agent as an agent or drug candidate molecule for normalizing a translational profile associated with a disease (*e.g.,* a cancer, an inflammatory disease, an autoimmune disease, a fibrotic disorder, a neurodegenerative disease, a neurodevelopmental disease, a metabolic disease, a viral infection, or a cardiomyopathy, respectively) when the one or more differentially translated genes from step (b) are in the third translational profile and have a translational profile closer to the translational profile of the one or more genes in the second translational profile than to the translational profile of the one or more genes in the first translational profile.

In certain aspects, the present disclosure provides a method for identifying an agent or drug candidate molecule (*i.e.,* a candidate therapeutic agent) for normalizing a translational profile associated with a disease, comprising:
(a) determining three independent translational profiles, each for a plurality of genes, wherein (i) a first translational profile is from a disease sample, (ii) a second translational profile is from (1) a control non-diseased sample or (2) a control non-diseased sample contacted with an agent or drug candidate molecule, and (iii) a third translational profile is from the disease sample contacted with the agent or drug candidate molecule;
(b) determining a first differential translational profile comprising one or more genes differentially translated in the first translational profile as compared to the second translational profile, and determining a second differential translational profile comprising one or more genes differentially translated in the first translational profile as compared to the third translational profile; and
(c) identifying the agent as a candidate therapeutic for normalizing a translational profile associated with a disease when the first differential translational profile is comparable to the second differential translational profile.

In certain aspects, the present disclosure provides a method for validating a target for normalizing a translational profile associated with a disease, comprising:
(a) determining a first translational profile for a plurality of genes from a disease sample contacted with an agent that modulates a target;
(b) determining a second translational profile for a plurality of genes from (i) a control non-diseased sample or (ii) a control non-diseased sample contacted with the agent that modulates a target; and
(c) validating the target as a target for normalizing a translational profile associated with a disease when the first translational profile is comparable to the second translational profile.

In certain aspects, the present disclosure provides a method for validating a target for normalizing a translational profile associated with a disease, comprising:
(a) determining three independent translational profiles, each for a plurality of genes, wherein (i) a first translational profile is from a disease sample, (ii) a second translational profile is from (1) a control non-diseased sample or (2) a control non-diseased sample contacted with an agent that modulates a target, and (iii) a third translational profile is from the disease sample contacted with the agent that modulates a target;
(b) identifying one or more genes as differentially translated in the first translational profile as compared to the second translational profile; and
(c) validating the target as a target for normalizing a translational profile associated with a disease when the one or more differentially translated genes from step (b) are in the third translational profile and have a translational profile closer to the translational profile of the one or more genes in the second translational profile than to the translational profile of the one or more genes in the first translational profile.

In certain aspects, the present disclosure provides a method for validating a target for normalizing a translational profile associated with a disease, comprising:
(a) determining three independent translational profiles, each for a plurality of genes, wherein (i) a first translational profile is from a disease sample, (ii) a second translational profile is from (1) a control non-diseased sample or (2) a control non-diseased sample contacted with an agent that modulates a target, and (iii) a third translational profile is from the disease sample contacted with the agent that modulates a target;
(b) determining a first differential translational profile comprising one or more genes differentially translated in the first translational profile as compared to the second translational profile, and determining a second differential translational profile comprising one or more genes differentially translated in the first translational profile as compared to the third translational profile; and
(c) validating the target as a target for normalizing a translational profile associated with a disease when the first differential translational profile is comparable to the second differential translational profile.

In any of the aforementioned aspects for validating a target for normalizing a translational profile associated with a disease, the target is suspected of being associated with a disease, is indirectly associated with a disease, or is associated with a disease (*e.g*., an inflammatory disease, an autoimmune disease, a fibrotic disorder, a neurodegenerative disease, a neurodevelopmental disease, a metabolic disease, a viral infection, a cardiomyopathy or a cancer, respectively).

In some aspects, one or more genes from each of at least two of the biological pathways are differentially translated in the first translational profile as compared to the second translational profile. In some aspects, one or more genes from each of at least three of the biological pathways are differentially translated in the first translational profile as compared to the second translational profile. In some aspects, there is at least a 1.5-fold or at least a two-fold difference (*e*.*g*.,at least 1.5-fold, at least two-fold, at least three-fold, at least four-fold, at least five-fold, at least six-fold, at least seven-fold, at least eight-fold, at least nine-fold, at least ten-fold difference or more) in translational level for the one or more genes in the first translational profile as compared to the second translational profile. In some aspects, the first, second, and/or third translational profiles comprise translational levels for a subset of the genome, *e.g*., for about 0.1%, 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50% of the genome or more. In some aspects, the first, second, and/or third translational profiles comprise a genome-wide measurement of gene translational levels.

The agent can be any agent as described herein. In some aspects, the agent is a peptide, protein, inhibitory RNA, or small organic molecule.

For comparing multiple translational profiles , for example, for determining to which translational profile a given experimentation translational profile is "closer" to, in some embodiments, an experimental translational profile has at least a 1.5 log₂ change or difference (*e.g*.*,* at least 1.5, at least 2.5, at least 3, at least 4, at least 5, at least 6, at least 7, at least 8, at least 9, at least 10 or more log₂ change or difference, *e*.*g*., increase or decrease) in translational rate, translational efficiency, or both for one or more genes or for a set of selected marker genes as compared to the same genes or gene markers from one or more reference translational profiles of interest. In some aspects, an experimental translational profile has at least a 2.5 log₂ change or difference in translational rate, translational efficiency, or both for one or more genes or for a set of selected marker genes as compared to the same genes or gene markers from one or more reference translational profiles of interest. In some aspects, an experimental translational profile has at least a 3 log₂ change or difference in translational rate, translational efficiency, or both for one or more genes or for a set of selected marker genes as compared to the same genes or gene markers from one or more reference translational profiles of interest.

In some aspects, an experimental profile as compared to one or more reference translational profiles of interest has at least a 1.1 log₂ change in translational rate, translational efficiency, or both for at least 0.05%, 0.1%, 0.25%, 0.5%, at least 1%, at least 2%, at least 3%, at least 4%, at least 5%, at least 6%, at least 7%, at least 8%, at least 9%, at least 10%, at least 15%, at least 20%, at least 30%, at least 40%, at least 50%, at least 60%, at least 70%, at least 80%, or at least 90% or more of a set of selected marker genes or for the entire set of selected marker genes. In some aspects, an experimental profile as compared to one or more reference translational profiles of interest has at least a 2 log₂ change in translational rate, translational efficiency, or both for at least 0.05%, 0.1%, 0.25%, 0.5%, at least 1%, at least 2%, at least 3%, at least 4%, at least 5%, at least 6%, at least 7%, at least 8%, at least 9%, at least 10%, at least 15%, at least 20%, at least 30%, at least 40%, at least 50%, at least 60%, at least 70%, at least 80%, or at least 90% or more of a set of selected marker genes or for the entire set of selected marker genes. In some aspects, an experimental profile as compared to one or more reference translational profiles of interest has at least a 2.5 log₂ change in translational rate, translational efficiency, or both for at least 0.05%, 0.1%, 0.25%, 0.5%, at least 1%, at least 2%, at least 3%, at least 4%, at least 5%, at least 6%, at least 7%, at least 8%, at least 9%, at least 10%, at least 15%, at least 20%, at least 30%, at least 40%, at least 50%, at least 60%, at least 70%, at least 80%, or at least 90% or more of a set of selected marker genes or for the entire set of selected marker genes. In some aspects, an experimental profile as compared to one or more reference translational profiles of interest has at least a 4 log₂ change in translational rate, translational efficiency, or both for at least 0.5%, at least 1%, at least 2%, at least 3%, at least 4%, at least 5%, at least 6%, at least 7%, at least 8%, at least 9%, at least 10%, at least 15%, at least20%, at least 30%, at least 40%, at least 50%, at least 60%, at least 70%, at least 80%, or at least 90% or more of a set of selected marker genes or for the entire set of selected marker genes.

As described herein, differentially translated genes between first and second translational profiles under a first condition may exhibit translational profiles "closer to" each other (*i.e*., identified through a series of pair-wise comparisons to confirm a similarity of pattern) under one or more different conditions (*e*.*g*., differentially translated genes between a normal sample and a disease sample may have a more similar translational profile when the normal sample is compared to a disease sample contacted with a candidate agent; differentially translated genes between a disease sample and a disease sample treated with a known active agent may have a more similar translational profile when the disease sample treated with a known active agent is compared to the disease sample contacted with a candidate agent). In certain aspects, a test translational profile is "closer to" a reference translational profile when at least of 99%, 95%, 90%, 85%, 80%, 75%, 70%, 65%, 60%, 55%, or 50% of a selected portion of differentially translated genes, a majority of differentially translated genes, or all differentially translated genes show a translational profile within 75%, 70%, 65%, 60%, 55%, 50%, 45%, 40%, 35%, 30%, or 25%, respectively, of their corresponding genes in the reference translational profile. In further aspects, a selected portion of differentially translated genes, a majority of differentially translated genes, or all differentially translated genes from an experimental translational profile have a translational profile "closer to" the translational profile of the same genes in a reference translational profile when the amount of protein translated in the experimental and reference translational profiles are within about 3.0 log₂, 2.5 log₂, 2.0 log₂, 1.5 log₂, 1.1 log₂, 0.5 log₂, 0.2 log₂ or closer. In still further aspects, a selected portion of differentially translated genes, a majority of differentially translated genes, or all differentially translated genes from an experimental translational profile have a translational profile "closer to" the translational profile of the same genes in a reference translational profile when the amount of protein translated in the experimental and reference translational profiles differs by no more than about 30%, 25%, 20%, 15%, 10%, 5%, 1% or less.

In some aspects, an experimental differential profile as compared to a reference differential translational profile of interest has at least a 1.0 log₂ change in translational rate, translational efficiency, or both for at least 0.05%, at least 0.1%, at least 0.25%, at least 0.5%, at least 1%, at least 2%, at least 3%, at least 4%, at least 5%, at least 6%, at least 7%, at least 8%, at least 9%, at least 10%, at least 15%, at least 20%, at least 30%, at least 40%, at least 50%, at least 60%, at least 70%, at least 80%, or at least 90% or more of a set of selected differentially translated genes or for the entire set of selected differentially translated genes. In some aspects, an experimental differential profile as compared to a reference differential translational profile of interest has at least a 2 log₂ change in translational rate, translational efficiency, or both for at least 0.05%, at least 0.1%, at least 0.25%, at least 0.5%, at least 1%, at least 5%, at least 10%, at least 15%, at least 20%, at least 30%, at least 40%, at least 50%, at least 60%, at least 70%, at least 80%, or at least 90% or more of a set of selected differentially translated genes or for the entire set of differentially translated genes. In some aspects, an experimental differential profile as compared to a reference differential translational profile of interest has at least a 3 log₂ change in translational rate, translational efficiency, or both for at least 0.05%, at least 0.1%, at least 0.25%, at least 0.5%, at least 1%, at least 5%, at least 10%, at least 15%, at least 20%, at least 30%, at least 40%, at least 50%, at least 60%, at least 70%, at least 80%, or at least 90% or more of a set of selected differentially translated genes or for the entire set of selected differentially translated genes. In some aspects, an experimental differential profile as compared to a reference differential translational profile of interest has at least a 4 log₂ change in translational levels for at least 0.05%, at least 0.1%, at least 0.25%, at least 0.5%, at least 1%, at least 5%, at least 10%, at least 15%, at least 20%, at least 30%, at least 40%, at least 50%, at least 60%, at least 70%, at least 80%, or at least 90% or more of a set of selected differentially translated genes or for the entire set of selected differentially translated genes.

As described herein, a differential translational profile between a first sample and a control may be "comparable" to a differential translational profile between a second sample and the control (*e.g*., the differential profile between a disease sample and the disease sample treated with a known active compound may be comparable to the differential profile between the disease sample and the disease sample contacted with a candidate agent; the differential profile between a disease sample and a non-diseased (normal) sample may be comparable to the differential profile between the disease sample and the disease sample contacted with a candidate agent). In certain aspects, a test differential translational profile is "comparable to" a reference differential translational profile when at least of 99%, 95%, 90%, 80%, 70%, 60%, 50%, 25%, or 10% of a selected portion of differentially translated genes, a majority of differentially translated genes, or all differentially translated genes show a translational profile within 75%, 70%, 65%, 60%, 55%, 50%, 45%, 40%, 35%, 30%, or 25%, respectively, of their corresponding genes in the reference translational profile. In further aspects, a differential translational profile comprising a selected portion of the differentially translated genes or all the differentially translated genes has a differential translational profile "comparable to" the differential translational profile of the same genes in a reference differential translational profile when the amount of protein translated in the experimental and reference differential translational profiles are within about 3.0 log₂, 2.5 log₂, 2.0 log₂, 1.5 log₂, 1.0 log₂, 0.5 log₂, 0.2 log₂ or closer. In still further aspects, a differential translational profile comprising a selected portion of the differentially translated genes or all the differentially translated genes has a differential translational profile "comparable to" the differential translational profile of the same genes in a reference differential translational profile when the amount of protein translated in the experimental and reference differential translational profiles differs by no more than about 50%, 45%, 40%, 35%, 30%, 25%, 20%, 15%, 10%, 5%, 1% or less.

In some aspects, the subject in need thereof is a subject having a pathogenic condition in which protein translation is known or suspected to be aberrant. In some aspects, the subject has a condition in which aberrant translation is known to be causative for the pathogenic condition. In certain aspects, the subject has a pathogenic condition in which altering the aberrant translation (*e*.*g*., increasing or decreasing) will prevent, ameliorate or treat the pathogenic condition. In certain aspects, the target is associated with a disease selected from an inflammatory disease, autoimmune disease, fibrotic disorder, neurodegenerative disease, neurodevelopmental disease, metabolic disease, viral infection, cardiomyopathy or cancer.

### VIII. Examples

The following examples are offered to illustrate, but not to limit the claimed invention.

### Example 1: Generation of a Comprehensive Map of Translationally Controlled mTOR Targets in Cancer Using Ribosome Profiling

Downstream of the phosphatidylinositol-3-OH kinase (PI(3)K)-AKT signalling pathway, mTOR assembles with either raptor or rictor to form two distinct complexes: mTORC1 and mTORC2. The major regulators of protein synthesis downstream of mTORC1 are 4EBP1 (also called EIF4EBP1) and p70S6K1/2. 4EBP1 negatively regulates eIF4E, a key rate-limiting initiation factor for cap-dependent translation. Phosphorylation of 4EBP1 by mTORC1 leads to its dissociation from eIF4E, allowing translation initiation complex formation at the 5' end of mRNAs. The mTOR-dependent phosphorylation of p70S6K1/2 also promotes translation initiation as well as elongation. In this example, ribosome profiling delineates the translational landscape of the cancer genome at a codon-by-codon resolution upon pharmacological inhibition of mTOR. This method provides a genome-wide characterization of translationally controlled mRNAs downstream of oncogenic mTOR signalling and delineates their functional roles in cancer development.

mTOR is deregulated in nearly 100% of advanced human prostate cancers, and genetic findings in mouse models implicate mTOR hyperactivation in prostate cancer initiation. Given the critical role for mTOR in prostate cancer, PC3 human prostate cancer cells, in which mTOR is constitutively hyperactivated, were used to delineate translationally controlled gene expression networks upon complete or partial mTOR inhibition. Ribosome profiling was optimized to assess quantitatively ribosome occupancy genome-wide in cancer cells. In brief, ribosome-protected mRNA fragments were deep-sequenced to determine the number of ribosomes engaged in translating specific mRNAs (*see* Fig. 6a and Example 6 ("Methods") below).

Treatment of PC3 cells with an mTOR ATP site inhibitor, PP242 (Feldman et al., PLoS Biol. 7:e38 (2009); Hsieh et al., Cancer Cell 17:249-261 (2010)), significantly inhibited the activity of the three primary downstream mTOR effectors 4EBP1, p70S6K1/2 and AKT. On the contrary, rapamycin, an allosteric mTOR inhibitor, only blocked p70S6K1/2 activity in these cells (Fig. 6b). Short 3-hr drug treatments, which precede alterations in de novo protein synthesis, were used to capture direct changes in mTOR-dependent gene expression by ribosome profiling and to minimize compensatory feedback mechanisms (Fig. 6c-f).

Ribosome profiling revealed 144 target mRNAs were selectively decreased at the translational level upon PP242 treatment (log₂ ≤-1.5 (false discovery rate <0.05)) as compared to rapamycin treatment, with limited changes in transcription (Figs. 1a, 7a-b, and 8-10, Table 3, Table 5, Table 6, and Table 7). The fact that at this time point rapamycin treatment did not markedly affect gene expression is consistent with incomplete, allosteric, inhibition of mTOR activity (Fig. 6b). By monitoring footprints of translating 80S ribosomes, these findings showed that the effects of PP242 were largely at the level of translation initiation and not elongation (Fig. 8). It has been proposed that mRNAs translationally regulated by mTOR may contain long 5' untranslated regions (5' UTRs) with complex RNA secondary structures. On the contrary, ribosome profiling revealed that mTOR-responsive 5' UTRs possess less complex features (Fig. 1b-d), providing a unique data set to investigate the nature of regulatory elements that render these mRNAs mTOR-sensitive. It has been previously shown that some mTOR translationally regulated mRNAs, most notably those involved in protein synthesis, possess a 5' terminal oligopyrimidine tract (5' TOP) that is regulated by distinct trans-acting factors. Of the 144 mTOR-sensitive target genes, 68% possessed a 5' TOP (see Table 1). Additionally, another 5' UTR consensus sequence, termed a pyrimidine-rich translational element (PRTE), was identified within the 5' UTRs of 63% of mTOR target mRNAs (P = 3.2 x 10⁻¹¹). This PRTE element, unlike the 5' TOP sequence, consists of an invariant uridine at position 6 flanked by pyrimidines and does not reside at position +1 of the 5' UTR (Fig. 7c and Table 2). 89% of the mTOR-responsive genes were found to possess a PRTE and/or 5' TOP, making the presence of one or both sequences a strong predictor for mTOR sensitivity (Fig. 7d and Table 3). Notably, mRNA isoforms arising from distinct transcription start sites may possess both a 5' TOP and a PRTE. Given the significant number of mRNAs that contain both the PRTE and 5' TOP, a functional interplay may exist between these regulatory elements. Additionally, these findings show that the PRTE imparts translational control specificity to 4EBP1 activity.

Surprisingly, mTOR-sensitive genes stratified into unique functional categories that may promote cancer development and progression, including cellular invasion (*P* = 0.009), cell proliferation (*P* = 0.04), metabolism (*P* = 0.0002) and regulators of protein modification (*P* = 0.01) (Fig. 1e). The largest fraction of mTOR-responsive mRNAs clustered into a node consisting of key components of the translational apparatus: 70 ribosomal proteins, 6 elongation factors, and 4 translation initiation factors (*P* = 7.5 x 10⁻⁸²) (Fig. 1e). Therefore, this class of mTOR-responsive mRNAs may represent an important regulon that sustains the elevated protein synthetic capacity of cancer cells.

The second largest node of mTOR translationally regulated genes comprised bona fide cell invasion and metastasis mRNAs and putative regulators of this process (Fig. 1e). This group included YB1 (Y-box binding protein 1; also called YBX1), vimentin, MTA1 (metastasis associated 1) and CD44 (Fig. 11a). YB1 regulates the post-transcriptional expression of a network of invasion genes. Vimentin, an intermediate filament protein, is highly upregulated during the epithelial-to-mesenchymal transition associated with cellular invasion. MTA1, a putative chromatin-remodeling protein, is overexpressed in invasive human prostate cancer and has been shown to drive cancer metastasis by promoting neoangiogenesis. CD44 is commonly overexpressed in tumor-initiating cells and is implicated in prostate cancer metastasis. Consistent with their status as mTOR-sensitive genes, YB1, vimentin, MTA1 and CD44 all possess a PRTE (Table 2). Vimentin and CD44 also possess a 5' TOP (Table 3). To test the functional role of the PRTE in mediating translational control, the PRTE was mutated within the 5' UTR of YB1, which rendered the YB1 5' UTR insensitive to inhibition by 4EBP1 (Fig. 11b). These findings highlight a novel cis-regulatory element that may modulate translational control of subsets of mRNAs upon mTOR activation. Moreover, ribosome profiling reveals unexpected transcript-specific translational control, mediated by oncogenic mTOR signaling, including a distinct set of pro-invasion and metastasis genes.

**Table 5. Mean list of translationally regulated PP242-responsive genes**

| | | **Rapamycin** | | **PP242** | |
|---|---|---|---|---|---|
| **Gene** | **Description** | **mRNA** | **TrlEff** | **mRNA** | **TrlEff** |
| EEF2 | eukaryotic translation elongation factor 2 | 0.39 | -1.12 | 0.76 | -3.60 |
| EEF1A1 | eukaryotic translation elongation factor 1 alpha 1 | 0.43 | -1.58 | 0.36 | -3.21 |
| RPL13A | ribosomal protein L13a | 0.15 | -1.25 | 0.30 | -3.10 |
| RPS12 | ribosomal protein S12 | 0.11 | -1.22 | 0.04 | -3.00 |
| RPL12 | ribosomal protein L12 | 0.07 | -0.94 | 0.12 | -2.95 |
| RPS27 | ribosomal protein S27 | 0.10 | -1.54 | 0.07 | -2.71 |
| RPS28 | ribosomal protein S28 | 0.01 | -0.80 | 0.28 | -2.67 |
| RPL18A | ribosomal protein L18a | 0.17 | -0.82 | 0.23 | -2.63 |
| RPL34 | ribosomal protein L34 | 0.11 | -1.12 | 0.04 | -2.63 |
| RPL28 | ribosomal protein L28 isoform 1 | 0.24 | -1.09 | 0.22 | -2.54 |
| RPL27A | ribosomal protein L27a | 0.06 | -0.96 | 0.07 | -2.53 |
| CRTAP | cartilage associated protein | 0.29 | -1.17 | 0.33 | -2.50 |
| RPL10 | ribosomal protein L10 | 0.09 | -0.79 | 0.25 | -2.46 |
| RPS20 | ribosomal protein S20 isoform 1 | 0.18 | -1.35 | -0.01 | -2.46 |
| RPL21 | ribosomal protein L21 | 0.14 | -1.25 | -0.04 | -2.45 |
| RPL3 | ribosomal protein L3 isoform a | 0.18 | -1.08 | 0.22 | -2.44 |
| RPL39 | ribosomal protein L39 | 0.17 | -1.65 | -0.15 | -2.41 |
| RPL37A | ribosomal protein L37a | 0.08 | -1.02 | 0.01 | -2.38 |
| VIM | vimentin | 0.36 | -0.40 | 0.67 | -2.38 |
| EEF1D | eukaryotic translation elongation factor 1 delta | 0.18 | -0.84 | 0.35 | -2.37 |
| GNB2L1 | Guanine nucleotide binding protein (G protein) | 0.19 | -0.77 | 0.27 | -2.35 |
| RPS19 | ribosomal protein S19 | 0.15 | -0.74 | 0.23 | -2.34 |
| RPL32 | ribosomal protein L32 | 0.22 | -0.97 | 0.11 | -2.33 |
| RPS15A | ribosomal protein S15a | 0.07 | -0.96 | 0.07 | -2.31 |
| RPL11 | ribosomal protein L11 | 0.09 | -1.08 | 0.14 | -2.31 |
| RPL7A | ribosomal protein L7a | 0.17 | -0.74 | 0.15 | -2.30 |
| YB1 | Y-box binding protein 1 | 0.11 | -0.59 | 0.24 | -2.30 |
| RPS9 | ribosomal protein S9 | 0.10 | -0.60 | 0.34 | -2.27 |
| EIF4B | eukaryotic translation initiation factor 4B | 0.55 | -1.21 | 0.61 | -2.27 |
| EEF1G | eukaryotic translation elongation factor 1, gamma | 0.21 | -1.15 | 0.15 | -2.26 |
| RPS2 | ribosomal protein S2 | 0.07 | -0.56 | 0.20 | -2.25 |
| RPS5 | ribosomal protein S5 | 0.14 | -0.77 | 0.23 | -2.25 |
| HSPA8 | heat shock 70kDa protein 8 isoform 1 | -0.21 | -0.46 | -0.40 | -2.25 |
| RPS3A | ribosomal protein S3a | 0.22 | -1.15 | -0.06 | -2.17 |
| RPS3 | ribosomal protein S3 | 0.22 | -0.92 | 0.24 | -2.16 |
| RPL10A | ribosomal protein L10a | 0.16 | -0.94 | 0.14 | -2.16 |
| RPS25 | ribosomal protein S25 | 0.04 | -0.89 | -0.04 | -2.13 |
| GLTSCR2 | glioma tumor suppressor candidate region gene 2 | 0.31 | -0.68 | 0.70 | -2.12 |
| HNRNPA1 | heterogeneous nuclear ribonucleoprotein A1 | 0.18 | -0.86 | 0.27 | -2.12 |
| RPLP2 | ribosomal protein P2 | 0.26 | -1.18 | 0.14 | -2.10 |
| RPL31 | ribosomal protein L31 isoform 2 | -0.02 | -0.62 | 0.05 | -2.10 |
| PABPC1 | poly(A) binding protein, cytoplasmic 1 | 0.35 | -1.44 | 0.16 | -2.09 |
| RPS21 | ribosomal protein S21 | -0.01 | -0.60 | 0.09 | -2.09 |
| RPS4X | ribosomal protein S4, X-linked X isoform | 0.18 | -1.15 | 0.12 | -2.06 |
| RPLP1 | ribosomal protein P1 isoform 1 | 0.28 | -1.09 | 0.12 | -2.06 |
| RPL7 | ribosomal protein L7 | 0.15 | -1.06 | 0.01 | -2.02 |
| RPL26 | ribosomal protein L26 | 0.15 | -1.11 | 0.02 | -2.00 |
| PABPC4 | poly A binding protein, cytoplasmic 4 isoform 1 | 0.24 | -0.80 | 0.40 | -1.98 |
| RPL36A | ribosomal protein L36a | 0.13 | -1.11 | -0.01 | -1.98 |
| EEF1A2 | eukaryotic translation elongation factor 1 alpha 2 | 0.03 | -0.03 | 0.40 | -1.94 |
| TPT1 | tumor protein, translationally-controlled 1 | 0.24 | -1.22 | 0.01 | -1.94 |
| AHCY | adenosylhomocysteinase isoform 1 | 0.20 | -0.23 | 0.38 | -1.93 |
| RPL22L1 | ribosomal protein L22-like 1 | 0.15 | -0.68 | 0.39 | -1.90 |
| GAPDH | glyceraldehyde-3-phosphate dehydrogenase | 0.17 | -0.27 | 0.28 | -1.90 |
| RPL30 | ribosomal protein L30 | 0.11 | -0.99 | 0.01 | -1.89 |
| RPS11 | ribosomal protein S11 | 0.11 | -0.59 | 0.20 | -1.88 |
| RPL29 | ribosomal protein L29 | 0.10 | -0.50 | 0.20 | -1.88 |
| RPL14 | ribosomal protein L14 | 0.07 | -0.68 | -0.02 | -1.85 |
| RPL36 | ribosomal protein L36 | 0.09 | -0.43 | 0.28 | -1.85 |
| EIF2S3 | eukaryotic translation initiation factor 2, S3 | 0.33 | -1.04 | 0.15 | -1.85 |
| RPL23 | ribosomal protein L23 | 0.09 | -0.92 | 0.07 | -1.82 |
| RPS16 | ribosomal protein S16 | 0.13 | -0.38 | 0.19 | -1.81 |
| SLC25A5 | adenine nucleotide translocator 2 | 0.21 | -0.30 | 0.15 | -1.80 |
| RPL17 | ribosomal protein L17 | 0.05 | -0.93 | 0.07 | -1.80 |
| RPL37 | ribosomal protein L37 | 0.11 | -0.68 | 0.10 | -1.79 |
| RPL8 | ribosomal protein L8 | 0.12 | -0.40 | 0.29 | -1.79 |
| NAP1L1 | nucleosome assembly protein 1-like 1 | 0.24 | -0.97 | 0.15 | -1.79 |
| RPS10 | ribosomal protein S10 | 0.16 | -0.69 | 0.19 | -1.78 |
| IPO7 | importin 7 | 0.20 | -0.83 | 0.26 | -1.75 |
| RPS8 | ribosomal protein S8 | 0.09 | -0.44 | 0.14 | -1.74 |
| RPL5 | ribosomal protein L5 | 0.17 | -1.11 | 0.06 | -1.73 |
| RPS24 | ribosomal protein S24 isoform d | 0.11 | -1.16 | -0.01 | -1.73 |
| EEF1B2 | eukaryotic translation elongation factor 1 beta 2 | 0.12 | -1.10 | -0.06 | -1.70 |
| RPL6 | ribosomal protein L6 | 0.09 | -0.68 | 0.06 | -1.68 |
| RPS23 | ribosomal protein S23 | 0.15 | -1.19 | -0.03 | -1.68 |
| RPL18 | ribosomal protein L18 | 0.08 | -0.42 | 0.18 | -1.65 |
| RPS29 | ribosomal protein S29 isoform 2 | -0.01 | -0.69 | 0.11 | -1.65 |
| RPS6 | ribosomal protein S6 | 0.14 | -1.06 | -0.02 | -1.65 |
| RPL22 | ribosomal protein L22 | 0.08 | -0.89 | 0.00 | -1.64 |
| UBA52 | ubiquitin and ribosomal protein L40 | 0.12 | -0.22 | 0.18 | -1.62 |
| RPLP0 | ribosomal protein PO | 0.15 | -0.42 | 0.12 | -1.61 |
| RPS27A | ubiquitin and ribosomal protein S27a | 0.16 | -0.89 | -0.04 | -1.61 |
| RPL9 | ribosomal protein L9 | 0.16 | -1.00 | -0.08 | -1.59 |
| TKT | transketolase isoform 1 | 0.02 | -0.11 | 0.33 | -1.58 |
| RPL13 | ribosomal protein L13 | 0.14 | -0.38 | 0.26 | -1.56 |
| EIF3H | eukatyotic translation initiation factor 3, | 0.16 | -0.79 | 0.09 | -1.54 |
| RPS13 | ribosomal protein S13 | 0.07 | -0.82 | -0.08 | -1.54 |
| RPS7 | ribosomal protein S7 | 0.11 | -0.76 | -0.04 | -1151 |
| RPS14 | ribosomal protein S14 | 0.10 | -0.60 | 0.16 | -1.50 |
| RPL4 | ribosomal protein L4 | 0.22 | -0.85 | 0.10 | -1.50 |
| FAM128B | hypothetical protein LOC80097 | 0.06 | 0.27 | 0.43 | -1.47 |
| EIF3L | eukaryotic translation initiation factor 3L | 0.28 | -0.85 | 0.21 | -1.47 |
| RABGGTB | RAB geranylgeranyltransferase, beta subunit | -0.20 | -0.84 | 0.20 | -1.46 |
| FASN | fatty acid synthase | -0.37 | 0.47 | 0.30 | -1.42 |
| RPL24 | ribosomal protein L24 | 0.11 | -0.63 | 0.00 | -1.41 |
| ACTG1 | actin, gamma 1 propeptide | 0.02 | -0.07 | 0.28 | -1.40 |
| PFDN5 | prefoldin subunit 5 isoform alpha | 0.11 | -0.51 | 0.04 | -1.38 |
| LMF2 | lipase maturation factor 2 | 0.22 | 0.39 | 0.62 | -1.36 |
| RPL19 | ribosomal protein L19 | 0.14 | -0.66 | 0.11 | -1.35 |
| PGM1 | phosphoglucomutase 1 | 0.40 | -0.55 | 0.23 | -1.35 |
| CCNI | cyclin I | 0.29 | -0.45 | 0.24 | -1.33 |
| IMPDH2 | inosine monophosphate dehydrogenase 2 | 0.11 | -0.39 | 0.21 | -1.33 |
| AP2A1 | adaptor-related protein complex 2, alpha 1 | 0.09 | -0.04 | 0.42 | -1.32 |
| AGRN | agrin precursor | 0.01 | 0.51 | 0.50 | -1.29 |
| COL6A2 | alpha 2 type VI collagen isoform 2C2 | -0.08 | 0.43 | 0.57 | -1.29 |
| CD44 | CD44 antigen isoform 1 | 0.34 | -0.46 | 0.43 | -1.29 |
| RPL41 | ribosomal protein L41 | 0.04 | -1.15 | -0.01 | -1.28 |
| ALKBH7 | spermatogenesis associated 11 precursor | 0.06 | 0.28 | 0.51 | -1.27 |
| RPL27 | ribosomal protein L27 | 0.05 | -0.33 | -0.13 | -1.23 |
| RPL15 | ribosomal protein L15 | 0.11 | -0.51 | 0.19 | -1.20 |
| RPS15 | ribosomal protein S15 | -0.01 | 0.03 | 0.21 | -1.19 |
| CLPTM1 | cleft lip and palate associated transmembrane | 0.07 | 0.26 | 0.41 | -1.13 |
| FAM83H | FAM83H | -0.17 | 0.71 | 0.33 | -1.11 |
| PGLS | 6-phosphogluconolactonase | 0.03 | 0.20 | 0.21 | -1.11 |
| MTA1 | metastasis associated 1 | 0.00 | -0.05 | 0.21 | -1.09 |
| TSC2 | tuberous sclerosis 2 isoform 1 | -0.15 | 0.34 | 0.21 | -1.09 |
| PACS1 | phosphofurin acidic cluster sorting protein 1 | 0.07 | 0.04 | 0.45 | -1.09 |
| CIRBP | cold inducible RNA binding protein | 0.14 | 0.10 | 0.54 | -1.08 |
| SLC19A1 | solute carrier family 19 member 1 | -036 | 0.23 | 0.10 | -1.07 |
| ECSIT | evolutionarily conserved signaling intermediate | -0.04 | 0.41 | 0.26 | -1.06 |
| ARD1A | alpha-N-acetyltransferase 1A | -0.04 | 0.01 | 0.03 | -1.05 |
| C21orf66 | GC-rich sequence DNA-binding factor candidate | -0.30 | -0.09 | -0.31 | -1.03 |
| ATP5G2 | ATP synthase, H+ transporting, mitochondrial F0 | 0.29 | -0.28 | 0.17 | -1.01 |
| LAMA5 | laminin alpha 5 | -0.32 | 0.87 | 0.40 | -0.94 |
| PNKP | polynucleotide kinase 3' phosphatase | -0.24 | 0.74 | 0.33 | -0.79 |
| EVPL | envoplakin | -0.08 | 0.30 | 0.38 | -0.79 |
| NCLN | nicalin | -0.05 | 0.67 | 0.29 | -0.76 |
| PTGES2 | prostaglandin E synthase 2 | -0.19 | 0.52 | 0.17 | -0.65 |
| GAMT | guanidinoacetate N-methyltransferase isoform b | n/a | n/a | n/a | n/a |
| CTSH | cathepsin H isoform b | n/a | n/a | n/a | n/a |
| TUBB3 | tubulin, beta, 4 | n/a | n/a | n/a | n/a |
| CSDA | cold shock domain protein A | n/a | n/a | n/a | n/a |
| ETHE1 | ETHE1 protein | n/a | n/a | n/a | n/a |
| LCMT1 | leucine carboxyl methyltransferase 1 isoform a | n/a | n/a | n/a | n/a |
| PC | pyruvate carboxylase | n/a | n/a | n/a | n/a |
| SECTM1 | secreted and transmembrane 0 | n/a | n/a | n/a | n/a |
| COL18A1 | alpha 1 type XVIII collagen isoform 3 | n/a | n/a | n/a | n/a |
| CHP | calcium binding protein P22 | n/a | n/a | n/a | n/a |
| BRF1 | transcription initiation factor IIIB | n/a | n/a | n/a | n/a |
| C2orf79 | hypothetical protein LOC391356 | n/a | n/a | n/a | n/a |
| SEPT8 | septin 8 isoform a | n/a | n/a | n/a | n/a |
| ABCB7 | ATP-binding cassette, sub-family B, member 7 | n/a | n/a | n/a | n/a |
| MYH14 | myosin, heavy chain 14 isoform 3 | n/a | n/a | n/a | n/a |
| SIGMAR1 | sigma non-opioid intracellular receptor 1 | n/a | n/a | n/a | n/a |
| C3orf38 | hypothetical protein LOC285237 | n/a | n/a | n/a | n/a |

**Table 6. List of rapamycin-sensitive translationally regulated genes after 3-hour treatment with rapamycin (50 nM) or PP242 (2.5 µM) in PC3 cells.**

| | | **Rapamycin** | | **PP242** | |
|---|---|---|---|---|---|
| **Gene** | **Description** | **mRNA** | **TrlEff** | **mRNA** | **TrlEff** |
| MAPK6 | mitogen-activated protein kinase 6 | 0.13 | -2.43 | 0.10 | -0.29 |
| RPL39 | ribosomal protein L39 | 0.30 | -2.11 | -0.42 | -2.53 |
| RPS20 | ribosomal protein S20 isoform 1 | 0.14 | -1.79 | -0.10 | -2.78 |
| PRKD3 | protein kinase D3 | -0.22 | -1.72 | -0.46 | 0.68 |
| UBTD2 | dendritic cell-derived ubiquitin-like protein | 0.19 | -1.64 | 0.25 | 0.27 |
| RPL28 | ribosomal protein L28 isoform 1 | 0.64 | -1.59 | 0.55 | -3.48 |
| RBPJ | recombining binding protein suppressor of | 1.09 | -1.58 | 0.17 | -0.03 |
| EEF1A1 | eukaryotic translation elongation factor 1 alpha | 0.46 | -1.57 | 0.29 | -3.53 |
| UCHL5 | ubiquitin carboxyl-terminal hydrolase L5 | -0.08 | -1.56 | -0.51 | 0.40 |
| RPS27 | ribosomal protein S27 | 0.07 | -1.55 | 0.06 | -3.35 |
| SDCCAG10 | serologically defined colon cancer antigen 10 | -0.19 | -1.50 | -0.37 | 0.23 |
| MAPKAPK2 | mitogen-activated protein kinase-activated | -0.21 | 1.50 | -0.22 | 0.92 |
| NFATC21P | nuclear factor of activated T-cells, 2IP | -0.16 | 1.54 | 0.08 | 0.35 |
| GTPBP3 | GTP binding protein 3 (mitochondrial) isoform V | -0.73 | 1.56 | 0.15 | -0.83 |
| C17orf28 | hypothetical protein LOC283987 | -0.44 | 1.66 | 0.21 | -0.20 |
| VHL | von Hippel-Lindau tumor suppressor isoform 1 | -0.23 | 1.67 | 0.43 | 0.52 |
| DDX51 | DEAD (Asp-Glu-Ala-Asp) box polypeptide 51 | -0.24 | 1.68 | 0.17 | -0.51 |
| DGCR2 | integral membrane protein DGCR2 | -0.66 | 1.69 | 0.05 | 0.02 |
| CCNA1 | cyclin A1 isoform a | -0.51 | 1.81 | -0.33 | 0.66 |
| NR2F1 | nuclear receptor subfamily 2, group F, member 1 | 0.05 | 1.94 | 0.87 | -0.09 |
| ACD | adrenocortical dysplasia homolog isoform 1 | -0.96 | 2.06 | 0.20 | -1.02 |

**Table 7. PP242 and rapamycin transcriptional targets.**

| **A. PP242 sensitive transcriptionally regulated genes upon 3-hour treatment with PP242 (2.5 µM) in PC3 cells*** | | |
|---|---|---|
| **Gene** | **Description** | **mRNA** |
| FGFBP1 | fibroblast growth factor binding protein 1 | -1.75 |
| BRIX1 | ribosome biogenesis protein BRX1 homolog | -1.51 |
| FOXA1 | forkhead box A1 | 1.45 |
| CYR61 | cysteine-rich, angiogenic inducer, 61 precursor | 1.47 |
| MT2A | metallothionein 2A | 1.47 |
| SOX4 | SRY (sex determining region Y)-box 4 | 1.51 |
| BCL6 | B-cell lymphoma 6 protein isoform 1 | 1.59 |
| KLF6 | Kruppel-like factor 6 isoform A | 1.75 |
| RND3 | ras homolog gene family, member E precursor | 1.78 |
| CTGF | connective tissue growth factor precursor | 1.80 |
| HBP1 | HMG-box transcription factor 1 | 1.88 |
| ARID5B | AT rich interactive domain 5B (MRF1-like) | 1.93 |
| PLAU | plasminogen activator, urokinase isoform 1 | 2.04 |
| GDF15 | growth differentiation factor 15 | 3.02 |

| **B. Rapamycin sensitive transcriptionally regulated genes upon 3-hour treatment with rapamycin (50 nM) in PC3 cells*** | | |
|---|---|---|
| **Gene** | **Description** | **mRNA** |
| HBP1 | HMG-box transcription factor 1 | -1.75 |

| | | |
|---|---|---|
| * log₂ fold change | | |

### Example 2: Translation of Pro-Invasion mRNAs by mTOR

To extend the use of the mTOR pharmacological tools used in ribosome profiling towards functional characterization of the newly identified mTOR-sensitive cell invasion gene signature, a new clinical-grade mTOR ATP site inhibitor was developed that was derived from the PP242 chemical scaffold. In brief, a structure-guided optimization of pyrazolopyrimidine derivatives was performed that improved oral bioavailability while retaining mTOR kinase potency and selectivity. The ATP site inhibitor of mTOR was selected for clinical studies on the basis of its high potency (1.4 nM inhibition constant (Kᵢ)), selectivity for mTOR, low molecular mass, and favorable pharmaceutical properties.

Using either PP242 or the new (or optimized) ATP site inhibitor of mTOR, a selective decrease in the expression of YB1, MTA1, vimentin, and CD44 was observed at the protein but not transcript level in PC3 cells starting at 6 hr of treatment, which preceded any decrease in *de novo* protein synthesis (Figs. If, 6c-d, 12, and 13). In contrast, rapamycin treatment did not alter their expression (Figs. 1g and 12a). Similar findings were observed using a broad panel of metastatic cell lines of distinct histological origins (Fig. 14). The four-gene invasion signature (YB1, MTA1, vimentin and CD44) was positively regulated by mTOR hyperactivation, as silencing PTEN expression increased their protein but not mRNA expression levels (Fig. 15). Next, the effects of mTOR ATP site inhibitors on prostate cancer cell migration and invasion were investigated. The ATP site inhibitor of mTOR, but not rapamycin, decreased the invasive potential of PC3 prostate cancer cells (Fig. 2a). Furthermore, the ATP site inhibitor of mTOR inhibited cancer cell migration starting at 6 hr of treatment, precisely correlating with when decreases in the expression of pro-invasion genes were evident, but preceding any changes in the cell cycle or overall global protein synthesis (Figs. 2b-c, 6c, 6e, 6f, 12b, and 16).

Among the genes comprising the pro-invasion signature, YB1 has been shown to act directly as a translation factor that controls expression of a larger set of genes involved in breast cancer cell invasion. Notably, YB1 translationally-regulated target mRNAs, including SNAIL1 (also called SNAI), LEF1 and TWIST1, decreased at the protein but not transcript level upon YB1 knockdown in PC3 cells (Figs. 17 and 18). To determine the functional role of YB1 in prostate cancer cell invasion, YB1 gene expression was silenced in PC3 cells and a 50% reduction in cell invasion was observed (Fig. 2d). Similarly, knockdown of MTA1, CD44, or vimentin also inhibited prostate cancer cell invasion (Figs. 2d and 17). These mTOR target mRNAs may be sufficient to endow primary prostate cells with invasive features, as overexpression of YB1 and/or MTA1 (Fig. 19a) in BPH-1 cells, an untransformed prostate epithelial cell line, increased the invasive capacity of these cells in an additive manner (Fig. 2e). Notably, the effects of YB1 and MTA1 on cell invasion were independent from any effect on cell proliferation in both knockdown or overexpression studies (Fig. 19b-c). Therefore, translational control of pro-invasion mRNAs by oncogenic mTOR signaling alters the ability of epithelial cells to migrate and invade, a key feature of cancer metastasis.

### Example 3: Dissecting mTOR Translational Effectors

To determine the molecular mechanism by which pro-invasion genes are regulated at the translational level and why these mRNAs are sensitive to an ATP site inhibitor of mTOR but not rapamycin, we investigated whether the downstream translational regulators mTORC1, 4EBP1, and/or p70S6K1/2 controlled the expression of these mTOR-sensitive targets. A human prostate cancer cell line was generated that stably expressed a doxycycline-inducible dominant-negative mutant of 4EBP1 (4EBP1^{M}) (Fig. 3a) (Hsieh et al., Cancer Cell 17:249-261 (2010)). This mutant binds to eIF4E, decreasing its hyperactivation without inhibiting general mTORC1 function (Fig 20a). Notably, expression of 4EBP1^{M} did not alter global protein synthesis (Fig. 20b), probably because endogenous 4EBP1 and 4EBP2 proteins retain their ability to bind to eIF4E (Fig. 24c). Upon induction of 4EBP1^{M}, YB1, vimentin, CD44 and MTA1 decreased at the protein but not mRNA level (Figs. 3b-c and 24d).

Next, we tested whether an ATP site inhibitor of mTOR decreases expression of the four invasion genes through the 4EBP-eIF4E axis. Notably, knockdown of 4EBP1 and 4EBP2 in PC3 cells or using 4EBP1 and 4EBP2 double knockout mouse embryonic fibroblasts (MEFs) (Dowling et al., Science 328:1172-1176 (2010)) reduced the ability of the ATP site inhibitor of mTOR to decrease expression of these pro-invasion mRNAs (Figs. 3d-e and 21). Furthermore, ablation of mTORC2 activity had no effect on the expression of these mRNAs or responsiveness to ATP site inhibitor of mTOR (Figs. 3f and 22a-c). Next, we determined the effect of 4EBP1^{M} on human prostate cancer cell invasion. The expression of 4EBP1^{M} resulted in a significant decrease in prostate cancer cell invasion without affecting the cell cycle, whereas DG-2 had no effect (Figs. 3g and 22d). These findings demonstrate that eIF4E hyperactivation downstream of oncogenic mTOR regulates translational control of the pro-invasion mRNAs and provides an explanation for the selective targeting of this gene signature by mTOR ATP site inhibitors.

### Example 4: Examining Cell Invasion Networks in Vivo

Both CK5⁺ and CK8⁺ prostate epithelial cells have been implicated in the initiation of prostate cancer upon loss of PTEN (Wang et al., Nature 461:495-500 (2009); Mulholland et al., Cancer Res. 69:8555-8562 (2009)). *Pten*^{*loxp*/*loxp*};*Pb-cre* (*Pten*^{*L*/*L*}) mice are an ideal model of prostate cancer because they display distinct stages of cancer development (prostatic intraepithelial neoplasia, invasive adenocarcinoma, and metastasis) (Wang et al., Cancer Cell 4:209-221 (2003)). However, the expression patterns of YB1, vimentin, CD44 and MTA1 in prostate basal (CK5⁺) and luminal (CK8⁺) epithelial cells have not been characterized.

We therefore analyzed their expression patterns in the *Pten*^{*L*/*L*} prostate cancer mouse model, where mTOR is constitutively hyperactivated. YB1 localized to the cytoplasm and nucleus of CK5⁺ and CK8⁺ prostate epithelial cells, consistent with its ability to shuttle between the two cellular compartments (Figs. 4a-b, 23a-b). MTA1 expression was exclusively nuclear in both cell types (Fig. 4c-d). CD44 expression was observed within a subset of CK5⁺ and CK8⁺ epithelial cells (Fig. 4e-f). CD44, together with other cell-surface markers, has been used to isolate a rare prostate stem-cell population (Leong et al., Nature 456:804-818 (2008)). In contrast, vimentin was not detected in either cell type (Fig. 4g). Next, the impact of mTOR hyperactivation on the expression pattern of the pro-invasion gene signature was determined. YB1, MTA1, and CD44 protein, but not transcript, levels were significantly increased in both *Pten*^{*L*/*L*} luminal and basal epithelial cells compared to wild-type (Figs. 4h and 23c-e). These studies reveal a unique, translationally controlled signature of gene expression downstream of mTOR hyperactivation in a cancer-initiating subset of prostate epithelial cells.

### Example 5: Targeting Prostate Cancer Metastasis

In a preclinical trial of RAD001 (rapalog) versus an ATP site inhibitor of mTOR in *Pten*^{*L*/*L*} mice, 4EBP1 and p70S6K1/2 phosphorylation was completely restored to wild-type levels after treatment with the ATP site inhibitor of mTOR, whereas RAD001 only decreased p70S6K1/2 phosphorylation levels (Fig. 24a-b). Next, the cellular consequences of complete versus partial mTOR inhibition during distinct stages of prostate cancer were determined. Treatment with the ATP site inhibitor of mTOR resulted in a 50% decrease in prostatic intraepithelial neoplasia (PIN) lesions in *Pten*^{*L*/*L*} mice that was associated with decreased proliferation and a tenfold increase in apoptosis (Fig. 24d-f). Notably, the unique cytotoxic properties of ATP site inhibitor of mTOR treatment in *Pten*^{*L*/*L*} mice were evidenced by a marked reduction in prostate cancer volume. In addition, and consistent with these findings, the ATP site inhibitor of mTOR induced programmed cell death in multiple cancer cell lines (Fig. 25a-b). In contrast, RAD001 treatment mainly had cytostatic effects leading to only partial regression of PIN lesions associated with a limited decrease in cell proliferation and no significant effect on apoptosis (Fig. 28c-f).

The preclinical trial was extended by examining the effects of the ATP site inhibitor of mTOR treatment on the pro-invasion gene signature and prostate cancer metastasis, which is incurable and the primary cause of patient mortality. Cell invasion is the critical first step in metastasis, required for systemic dissemination. In *Pten*^{*L*/*L*} mice after the onset of PIN, a subset of prostate glands showed characteristics of luminal epithelial cell invasion by 12 months (Figs. 5a and 25c). After 12 months of age, *Pten*^{*L*/*L*} mice developed lymph-node metastases and these cells maintained strong YB1 and MTA1 expression (Fig. 5b). These findings were extended directly to human prostate cancer patient specimens, in which it was observed that YB1 expression levels increased in a stepwise fashion from normal prostate to castration-resistant prostate cancer (CRPC), an advanced form of the disease associated with increased metastatic potential (Fig. 5c). Similar increases have been observed in MTA1 levels (Hofer et al., Cancer Res. 64:825-829 (2004)).

In human prostate cancer, high-grade primary tumors that display invasive features are more likely to develop systemic metastasis than low-grade non-invasive tumors. Remarkably, treatment with the ATP site inhibitor of mTOR completely blocked the progression of invasive prostate cancer locally in the prostate gland, and profoundly inhibited the total number and size of distant metastases (Fig. 5d-f). This was associated with a marked decrease in the expression of YB1, vimentin, CD44, and MTA1 at the protein, but not transcript, level in specific epithelial cell types within pre-invasive PIN lesions in *Pten*^{*L*/*L*} mice (Fig. 5g and Fig. 23c). Together, these findings reveal an unexpected role for oncogenic mTOR signaling in control of a pro-invasion translational program that, along with the lethal metastatic form of prostate cancer, can be efficiently targeted with clinically relevant mTOR ATP site inhibitors. These findings also demonstrate that translational profiling can be used to identify or validate targets for therapeutic intervention, such as genes that are modulated in cancer.

### Example 6: Methods

**Mice.** *Pten*^{*loxp*/*loxp*} and *Pb-cre* mice where obtained from Jackson Laboratories and Mouse Models of Human Cancers Consortium (MMHCC), respectively, and maintained in the C57BL/6 background. Mice were maintained under specific pathogen-free conditions, and experiments were performed in compliance with institutional guidelines as approved by the Institutional Animal Care and Use Committee of UCSF.

**Cell culturing and reagents.** Human cell lines were obtained from the ATCC and maintained in the appropriate medium with supplements as suggested by ATCC. Wild-type, *mSin1*^{*-*/*-*}, and *4EBP1*/*4EBP2* double knockout MEFs were cultured as previously described (Dowling et al., Science 328:1172-1176 (2010); Jacinto et al., Cell 127:125-137 (2006). SMARTvector 2.0 (Thermo Scientific) lentiviral shRNA constructs were used to knock down PTEN (SH-003023-02-10). For generation of GFP-labeled PC3 cells, SMARTvector 2.0 lentiviral empty vector control particles that contained TurboGFP (S-004000-01) were used. Control (D-001810-01), YB1 (L-010213), MTA1 (L-004127), CD44 (L-009999), vimentin (L-003551), rictor (LL-016984), 4EBP1 (L-003005), and 4EBP2 (L-018671) pooled siRNAs were purchased from Thermo Scientific. The ATP site inhibitors of mTOR INK128 and PP242 were used at 200 nM and 2.5 µM in cell-based assays unless otherwise specified. RAD001 was obtained from LC Laboratories. DG-2 was used at 20 µM in cell-based assays. Rapamycin was purchased from Calbiochem and used at 50 nM in cell-based assays. Doxycyline (Sigma) was used at 1 µg ml⁻¹ in 4EBP1^{M} induction assays. Lipofectamine 2000 (Invitrogen) was used to transfect cancer cell lines with siRNA. Amaxa Cell Line Nucleofector Kit R (Lonza) was used to electroporate BPH-1 cells with overexpression vectors. The 4EBP1^{M} has been previously described (Hsieh et al., Cancer Cell 17:249-261 (2010)).

**Plasmids.** pcDNA3-HA-YB1 was provided by V. Evdokimova. pCMV6-Myk-DDK-MTA1 was purchased from Origene. pGL3-Promoter was purchased from Promega. To clone the 5' UTR of YB1 into pGL3-Promoter, the entire 5' UTR sequence of YB1 was amplified from PC3 cDNA. PCR fragments were digested with HindIII and NcoI and ligated into the corresponding sites of pGL3- Promoter. The PRTE sequence at position +20-34 in the YB1 5' UTR (UCSC kgID uc001chs.2) was mutated using the QuikChange Site-Directed Mutagenesis Kit following the manufacturer's protocol (Stratagene).

**Ribosome profiling.** PC3 cells were treated with rapamycin (50 nM) or PP242 (2.5 µM) for 3 hr. Cells were subsequently treated with cycloheximide (100 µg ml⁻¹) and detergent lysis was performed in the dish. The lysate was treated with DNase and clarified, and a sample was taken for RNA-seq analysis. Lysates were subjected to ribosome footprinting by nuclease treatment. Ribosome-protected fragments were purified, and deep sequencing libraries were generated from these fragments, as well as from poly(A) mRNA purified from non-nuclease-treated lysates. These libraries were analyzed by sequencing on an Illumina GAII.

Each sequencing run resulted in approximately 20-25 million raw reads per sample, of which 5-12 million unique reads were used for subsequent analysis. Ribosome footprint and RNA-seq sequencing reads were aligned against a library of transcripts from the UCSC Known Genes database GRCh37/hg19. The first 25 nucleotides of each read were aligned using Bowtie and this initial alignment was then extended to encompass the full fragment-derived portion of the sequencing read while excluding the linker sequence. Read density profiles were then constructed for the canonical transcript of each gene, using only reads with 0 or 1 total mismatches between the read sequence and the reference sequence, comprised of the transcript fragment followed by the linker sequence. Footprint reads were assigned to an A site nucleotide at position +15 to +17 of the alignment, based on the total fragment length; mRNA reads were assigned to the first nucleotide of the alignment. The average read density per codon was then computed for the coding sequence of each transcript, excluding the first 15 and last 5 codons, which can display atypical ribosome accumulation.

Average read density was used as a measure of mRNA abundance (RNA-seq reads) and of protein synthesis (ribosome profiling reads). For most analyses, genes were filtered to require at least 256 reads in the relevant RNA-seq samples. Translational efficiency was computed as the ratio of ribosome footprint read density to RNA-seq read density, scaled to normalize the translational efficiency of the median gene to 1.0 after excluding regulated genes (log₂ fold-change ±1.5 after normalizing for the all-gene median). Changes in protein synthesis, mRNA abundance and translational efficiency were similarly computed as the ratio of read densities between different samples, normalized to give the median gene a ratio of 1.0. This normalization corrects for differences in the absolute number of sequencing reads obtained for different libraries. 3,977 (replicate 1), and 5,333 (replicate 2) unique mRNAs passed a preset read threshold of 256 reads for single-gene quantification for all treatment conditions.

**Western blot analysis.** Western blot analysis was performed as previously described (Hsieh et al., Cancer Cell 17:249-261 (2010)) with antibodies specific to phospho-AKT^{S471} (Cell Signaling), AKT (Cell Signaling), phospho-p70S6K^{T389} (Cell Signaling), phospho-fpS6^{S240/244} (Cell Signaling), rpS6 (Cell Signaling), phospho-4EBP1^{T37/46} (Cell Signaling), 4EBP1 (Cell Signaling), 4EBP2 (Cell Signaling), YB1 (Cell Signaling), CD44 (Cell Signaling), LEF1 (Cell Signaling), PTEN (Cell Signaling), eEF2 (Cell Signaling), GAPDH (Cell Signaling), vimentin (BD Biosciences), eIF4E (BD Biosciences), Flag (Sigma), β-actin (Sigma), MTA1 (Santa Cruz Biotechnology), Twist (Santa Cruz Biotechnology), rpL28 (Santa Cruz Biotechnology), HA (Covance) and rictor (Bethyl Laboratory).

**qPCR analysis.** RNA was isolated using the manufacturer's protocol for RNA extraction with TRIzol Reagent (Invitrogen) using the Pure Link RNA mini kit (Invitrogen). RNA was DNase-treated with Pure Link Dnase (Invitrogen). DNase-treated RNA was transcribed to cDNA with SuperScript III First-Strand Synthesis System for RT-PCR (Invitrogen), and 1 µl of cDNA was used to run a SYBR green detection qPCR assay (SYBR Green Supermix and MyiQ2, Biorad). Primers were used at 200 nM.

**5' UTR analysis.** 5' UTRs of the 144 downregulated mTOR target genes were obtained using the known gene ID from the UCSC Genome Browser (GRCh37/ hg19). Target versus non-target mRNAs were compared for 5' UTR length, %G+C content and Gibbs free energy by the Wilcoxon two-sided test. Multiple *E*ₘ (expectation maximization) for Motif Elicitation (MEME) and Find Individual Motif Occurrences (FIMO) was used to derive the PRTE and determine its enrichment in the 144 mTOR-sensitive genes compared a background list of 3,000 genes. The Database of Transcriptional Start Sites (DBTSS Release 8.0) was used to identify putative 5' TOP genes and putative transcription start sites in the 144 mTOR target genes.

**Luciferase assay.** PC3 4EBP1^{M} cells were treated with 1 µg ml⁻¹ doxycycline (Sigma) for 24 hr. Cells were transfected with various pGL3-Promoter constructs using lipofectamine 2000 (Invitrogen). After 24 hr, cells were collected. 20% of the cells were aliquoted for RNA isolation. The remaining cells were used for the luciferase assay per the manufacturer's protocol (Promega). Samples were measured for luciferase activity on a Glomax 96-well plate luminometer (Promega). Firefly luciferase activity was normalized to luciferase mRNA expression levels.

**Kinase assays**. mTOR activity was assayed using LanthaScreen Kinase kit reagents (Invitrogen) according to the manufacturer's protocol. PI(3)K α, β, γ, and δ activity were assayed using the PI(3)K HTRF assay kit (Millipore) according to the manufacturer's protocol. The concentration of ATP site inhibitor of mTOR necessary to achieve inhibition of enzyme activity by 50% (IC₅₀) was calculated using concentrations ranging from 20 µM to 0.1 nM (12-point curve). IC₅₀ values were determined using a nonlinear regression model (GraphPad Prism 5).

**Cell proliferation assay.** PC3 cells were treated with the appropriate drug for 48 hr, and proliferation was measured using Cell Titer-Glo Luminescent reagent (Promega) per the manufacturer's protocol. The concentration of ATP site inhibitor of mTOR necessary to achieve inhibition of cell growth by 50% (IC₅₀) was calculated using concentrations ranging from 20.0 µM to 0.1 nM (12-point curve).

**Mouse xenograft study.** Nude mice were inoculated subcutaneously in the right subscapular region with 5 x 10⁶ MDA-MB-361 cells. After tumors reached a size of 150-200 mm³, mice were randomly assigned into vehicle control or treatment groups. The ATP site inhibitor of mTOR was formulated in 5% polyvinylpropyline, 15% NMP, 80% water and administered by oral gavage at 0.3 mg kg⁻¹ and 1 mg kg⁻¹ daily.

**Pharmacokinetic analysis.** The area under the plasma drug concentration versus time curves, AUC₍₀₋ₜₗₐₛₜ₎ and AUC_{(0-inf)}, were calculated from concentration data using the linear trapezoidal rule. The terminal t_{1/2} in plasma was calculated from the elimination rate constant (*lz*), estimated as the slope of the log-linear terminal portion of the plasma concentration versus time curve, by linear regression analysis. The bioavailability (*F*) was calculated using *F* = AUC_{(0-tlast),po}*D*_{i.v}.) / AUC_{(0-last),iv}*D*_{p.o.}) x 100%, where *D*_{i.v.} and *D*_{p.o}. are intravenous and oral doses, respectively. Cmax was a highest drug concentration in plasma after oral administration. Tₘₐₓ was the time at which Cₘₐₓ is observed after extravascular administration of drug. Tₗₐₛₜ was the last time point a quantifiable drug concentration can be measured.

**Polysome analysis.** PC3 cells were treated for 3 hr with either DMSO or the ATP site inhibitor of mTOR (100 nM). Cells were re-suspended in PBS containing 100 µg ml⁻¹ cycloheximide (Sigma) and incubated on ice for 10 min. Cells were centrifuged at 300g for 5 min at 4°C and lysed in 10 mM Tris-HCl pH 8, 140 mM NaCl, 5 mM MgCl₂, 640 U ml⁻¹ Rnasin, 0.05% NP-40, 250 µg ml⁻¹ cycloheximide, 20 mM DTT, and protease inhibitors. Samples were incubated for 20 min on ice, then centrifuged once for 5 min at 3,300g and once for 5 min at 9,300g, isolating the supernatant after each centrifugation. Lysates were loaded onto 10-50% sucrose gradients containing 0.1 mg ml⁻¹ heparin and 2 mM DTT and centrifuged at 37,000 r.p.m. for 2.5 hr at 4°C. The sample was subsequently fractionated on a gradient fractionation system (ISCO). RNA was extracted from all fractions and run on a TBE-agarose gel to visualize 18S and 28S rRNA. Fractions 7-13 were found to correspond to the polysome fractions and were used for further qPCR analysis.

**[³⁵S] metabolic labeling.** PC3 or PC3 4EBP1^{M} cells with or without indicated treatment were incubated with 30 µCi of [³⁵S]-methionine for 1 hr after pre-incubation in methionine-free DMEM (Invitrogen). Cells were prepared using a standard protein lysate protocol, resolved on a 10% SDS polyacrylamide gel and transferred onto a PVDF membrane (Bio-Rad). The membrane was exposed to autoradiography film (Denville) for 24 hr and developed.

**Cell cycle analysis.** Appropriately treated PC3, BPH-1, or PC3-4EBP1^{M} cells were fixed in 70% ethanol overnight at -20°C. Cells were subsequently washed with PBS and treated with RNase (Roche) for 30 min. After this incubation, the cells were permeabilized and treated with 50 µg ml⁻¹ propidium iodide (Sigma) in a solution of 0.1% Tween, 0.1% sodium citrate. Cell cycle data was acquired using a BD FACS Caliber (BD Biosciences) and analyzed with FlowJo (v.9.1).

**Apoptosis analysis.** Appropriately treated LNCaP and A498 cells were labeled with Annexin V-FITC (BD Biosciences) and propidium iodide (Sigma) following the manufacturer's instructions. PI/Annexin data was acquired using a BD FACS Caliber (BD Biosciences) and analyzed with FlowJo (v.9.1).

**Matrigel invasion assay.** BioCoat Matrigel Invasion Chambers (modified Boyden Chamber Assay; BD Biosciences) were used according to the manufacturer's instructions.

**Real-time imaging of cell migration**. Real-time imaging of GFP-labeled PC3 cells was performed in poly-D-lysine-coated chamber cover glass slides (Lab-Tek). PC3 GFP cells were plated and allowed to adhere for 24 hr. Wells were wounded with a P200 pipette tip. The chamber slides were imaged with an IX81 Olympus wide-field fluorescence microscope equipped with a CO₂-and temperature-controlled chamber and time-lapse tracking system. Images from DIC and GFP channels were taken every 2 min and processed using ImageJ and analyzed for cell migration with Manual Tracking, using local maximum centering correction to maintain a centroid xy coordinate for each cell per frame over time. Tracking data was subsequently processed with the Chemotaxis and Migration tool from ibidi to create *xy* coordinate plots, velocity, and distance measurements.

**Snail1 immunocytochemistry.** Appropriately transfected or treated PC3 cells were plated on a poly-L-lysine-coated chamber slide (Lab-Tek) and cultured for 48 hr. Cells were fixed with 4% paraformaldehye (EMS), rinsed with PBS, and permeabilized with 0.1% Triton X-100. The samples were blocked in 5% goat serum and then incubated with anti-Snail1 antibody (Cell Signaling) in 5% goat serum for 2 hr at room temperature. Cells were washed with PBS and incubated with Alexa 594 anti-mouse antibody (Invitrogen) and DAPI (Invitrogen) for 2 hr at room temperature. Specimens were again washed with PBS and subsequently mounted with Aqua Poly/Mount (Polysciences). Image capture and quantification were completed as described below (see "Immunofluorescence").

**Cap-binding assay.** PC3 4EBP1^{M} cells were induced with doxycycline (1 µg ml⁻¹, Sigma) for 48 hr, then collected and lysed in buffer A (10 mM Tris-HCl pH 7.6, 150 mM KCl, 4 mM MgCl₂, 1 mM DTT, 1 mM EDTA, and protease inhibitors, supplemented with 1% NP-40). Cell lysates were incubated overnight at 4°C with 50 ml of the mRNA cap analogue m⁷GTP-sepharose (GE Healthcare) in buffer A. The beads were washed with buffer A supplemented with 0.5% NP-40. Protein complexes were dissociated using 1X sample buffer, and resolved by SDS-PAGE and western blotted with the appropriate antibodies.

**Pharmacological treatment of *Pten*^{*L*/*L*} mice and MRI imaging.** Nine- and twelve-month-old ***Pten*^{*L*/*L*}** mice were gavaged daily with either vehicle (see "Mouse xenograft study"), RAD001 (10 mg kg⁻¹), or an ATP site inhibitor of mTOR (1 mg kg⁻¹) for the indicated times. Weight measurements were taken every 3 days to monitor for toxicity. For the 28-day study, mice were imaged via MRI at day 0 and day 28 in a 14-T GE MR scanner (GE Healthcare).

**Prostate tissue processing.** Whole mouse prostates were removed from wild-type and *Pten*^{*L*/*L*} mice, microdissected, and frozen in liquid nitrogen. Frozen tissues were subsequently manually disassociated using a biopulverizer (Biospec) and additionally processed for protein and mRNA analysis as described above.

**Immunofluorescence.** Prostates and lymph nodes were dissected from mice within 2 hr of the indicated treatment and fixed in 10% formalin overnight at 4°C. Tissues were subsequently dehydrated in ethanol (Sigma) at room temperature, mounted into paraffin blocks, and sectioned at 5 µm. Specimens were de-paraffinized and rehydrated using CitriSolv (Fisher) followed by serial ethanol washes. Antigen unmasking was performed on each section using Citrate pH 6 (Vector Labs) in a pressure cooker at 125°C for 10-30 min. Sections were washed in distilled water followed by TBS washes. The sections were then incubated in 5% goat serum, 1% BSA in TBS for 1 hr at room temperature. Various primary antibodies were used, including those specific for keratin 5 (Covance), cytokeratin 8 (Abcam and Covance), YB1 (Abcam), vimentin (Abcam), MTA1 (Cell signaling), CD44 (BD Pharmingen), and the androgen receptor (Epitomics), which were diluted 1:50-1:500 in blocking solution and incubated on sections overnight at 4°C. Specimens were then washed in TBS and incubated with the appropriate Alexa 488 and 594 labeled secondary (Invitrogen) at 1:500 for 2 hr at room temperature, with the exception of YB1 which was incubated with biotinylated anti-rabbit secondary (Vector) followed by incubation with Alexa 594 labeled Streptavidin (Invitrogen). A final set of washes in TBS was completed at room temperature followed by mounting with DAPI Hardset Mounting Medium (Vector Lab). A Zeiss Spinning Disc confocal (Zeiss, CSU-X1) was used to image the sections at 40X - 100X. Individual prostate cells were quantified for mean fluorescence intensity (m.f.i.) using the Axiovision (Zeiss, Release 4.8) densitometric tool.

**Lymph node metastasis measurements.** Mouse lymph nodes were processed as described above and stained for CK8 and androgen receptor. Lymph nodes were imaged using a Zeiss AX10 microscope. Metastases were identified and areas were measured using the Axiovision (Zeiss, Release 4.8) measurement tool.

**Semi-quantitative RT-PCR.** Whole prostates were removed from wild-type and ***Pten*^{*L*/*L*}** mice, microdissected, dissociated into single-cell suspension, and stained for epithelial cell markers as previously described (Lukacs et al., Nature Protocols 5:702-713 (2010)) using fluorescence-conjugated antibodies for CD49f, Sca-1, CD31, CD45, and Ter119 (BD Biosciences). Luminal epithelial cells were sorted using a FACS Aria (BD Biosciences). Cell pellets were resuspended in 500 µl TRIzol Reagent and RNA was isolated and transcribed into cDNA as described above. Semi-quantitative PCR analysis was performed using oligonucleotides for vimentin and β-actin at 200 nM in a 25 µl reaction with 12.5 µl GoTaq (Promega) for 32 and 33 cycles, respectively, which were within the linear range (Fig. 23f).

**Immunohistochemistry.** Immunohistochemistry was performed as described above (see "Immunofluorescence") with the exception that immediately after antigen presentation and TBS washes, specimens were incubated in 3% hydrogen peroxide in TBS followed by TBS washes. The following primary antibodies were used: phospho-AKT^{S473} (Cell Signaling), phospho-rpS6^{S240/244} (Cell Signaling), phospho-4EBP1^{T37/46} (Cell Signaling), phospho-histone H3 (Upstate), and cleaved caspase (Cell Signaling). This was followed by TBS washes and incubation with the appropriate biotinylated secondary antibody (Vector Lab) for 30 min at room temperature. An ABC-HRP Kit (Vector Lab) was used to amplify the signal, followed by a brief incubation in hydrogen peroxide. The protein of interest was detected using DAB (Sigma). Specimens were counterstained with haematoxylin (Thermo Scientific), dehydrated with Citrisolv (Fisher), and mounted with Cytoseal XYL (Vector Lab).

**Haematoxylin and eosin staining.** Paraffin-embedded prostate specimens were deparaffinized and rehydrated as described above (see "Immunofluorescence"), stained with haematoxylin (Thermo Scientific), and washed with water. This was followed by a brief incubation in differentiation RTU (VWR) and two washes with water followed by two 70% ethanol washes. The samples were then stained with eosin (Thermo Scientific) and dehydrated with ethanol followed by CitriSolv (Fisher). Slides were mounted with Cytoseal XYL (Richard Allan Scientific).

**Oligonucleotides.** *YB1* 5' UTR cloning and site-directed mutagenesis oligonucleotides are as follows. *YB1* 5' UTR cloning: forward 5'-GCTACAAGCTTGGGCTTATCCCGCCT-3' (SEQ ID NO:146), reverse 5'-TCGATCCATGGGGTTGCGGTGATGGT-3' (SEQ ID NO:147); deletion (20-34): forward 5'-TGGGCTTATCCCGCCTGTCCTTCGATCGGTAGCGGGAGCG-3' (SEQ ID NO:148), reverse 5'-CGCTCCCGCTACCGATCGAAGGACAGGCGGGATAAGCCCA-3' (SEQ ID NO:149); transversion (20-34): forward 5'-TGGGCTTATCCCGCCTGTCCGCGGTAAGAGCGATCTTCGATCGGTAGCGGGAGCG -3' (SEQ ID NO:150), reverse 5'-CGCTCCCGCTACCGATCGAAGATCGCTCTTACCGCGGACAGGCGGGATAAGCCC A-3' (SEQ ID NO:151).

Human qPCR oligonucleotides are as follows. β-actin forward 5' - GCAAAGACCTGTACGCCAAC-3' (SEQ ID NO:152), reverse 5' - AGTACTTGCGCTCAGGAGGA-3' (SEQ ID NO:153); *CD44* forward 5' - CAACAACACAAATGGCTGGT-3' (SEQ ID NO:154), reverse 5' - CTGAGGTGTCTGTCTCTTTCATCT-3' (SEQ ID NO:155); vimentin forward 5' - GGCCCAGCTGTAAGTTGGTA-3' (SEQ ID NO:156), reverse 5' - GGAGCGAGAGTGGCAGAG-3' (SEQ ID NO:157); *Snail1* forward 5'-CACTATGCCGCGCTCTTTC-3' (SEQ ID NO:158), reverse 5'-GCTGGAAGGTAAACTCTGGATTAGA-3' (SEQ ID NO:159); *YB1* forward 5'-TCGCCAAAGACAGCCTAGAGA-3' (SEQ ID NO:160), reverse 5'-TCTGCGTCGGTAATTGAAGTTG-3' (SEQ ID NO:161); *MTA1* forward 5'-CAAAGTGGTGTGCTTCTACCG-3' (SEQ ID NO:162), reverse 5'-CGGCCTTATAGCAGACTGACA-3' (SEQ ID NO:163); *PLAU* forward 5'-TTGCTCACCACAACGACATT-3' (SEQ ID NO:164), reverse 5'-GGCAGGCAGATGGTCTGTAT-3' (SEQ ID NO:165); *FGFBP1* forward 5'-ACTGGATCCGTGTGCTCAG-3' (SEQ ID NO:166), reverse 5'-GAGCAGGGTGAGGCTACAGA-3' (SEQ ID NO:167); *ARID5B* forward 5'-TGGACTCAACTTCAAAGACGTTC-3' (SEQ ID NO:168), reverse 5'-ACGTTCGTTTCTTCCTCGTC-3' (SEQ ID NO:169); *CTGF* forward 5'-CTCCTGCAGGCTAGAGAAGC-3' (SEQ ID NO:170), reverse 5'-GATGCACTTTTTGCCCTTCTT-3' (SEQ ID NO:171); *RND3* forward 5'-AAAAACTGCGCTGCTCCAT-3' (SEQ ID NO:172), reverse 5'-TCAAAACTGGCCGTGTAATTC-3' (SEQ ID NO:173); *KLF6* forward 5'-AAAGCTCCCACTTGAAAGCA-3' (SEQ ID NO:174), reverse 5'-CCTTCCCATGAGCATCTGTAA-3' (SEQ ID NO:175); *BCL6* forward 5'-TTCCGCTACAAGGGCAAC-3' (SEQ ID NO:176), reverse 5'-TGCAACGATAGGGTTTCTCA-3' (SEQ ID NO:177); *FOXA1* forward 5'-AGGGCTGGATGGTTGTATTG-3' (SEQ ID NO:178), reverse 5'-ACCGGGACGGAGGAGTAG-3' (SEQ ID NO:179); *GDF15* forward 5'-CCGGATACTCACGCCAGA-3' (SEQ ID NO:180), reverse 5'-AGAGATACGCAGGTGCAGGT-3' (SEQ ID NO:181); *HBP1* forward 5'-GCTGGTGGTGTTGTCGTG-3' (SEQ ID NO:182), reverse 5'-CATGTTATGGTGCTCTGACTGC-3' (SEQ ID NO:183); *Twist1* forward 5'-CATCCTCACACCTCTGCATT-3' (SEQ ID NO:184), reverse 5'-TTCCTTTCAGTGGCTGATTG-3' (SEQ ID NO:185); *LEF1* forward 5'-CCTTGGTGAACGAGTCTGAAATC-3' (SEQ ID NO:186), reverse 5'-GAGGTTTGTGCTTGTCTGGC-3' (SEQ ID NO:187); *rpS19* forward 5'-GCTGGCCAAACATAAAGAGC-3' (SEQ ID NO:188), reverse 5'-CTGGGTCTGACACCGTTTCT-3' (SEQ ID NO:189); 5S rRNA forward 5'-GCCCGATCTCGTCTGATCT-3' (SEQ ID NO:190), reverse 5'-AGCCTACAGCACCCGGTATT-3' (SEQ ID NO:191); firefly luciferase forward 5'-AATCAAAGAGGCGAACTGTG-3' (SEQ ID NO:192), reverse 5'-TTCGTCTTCGTCCCAGTAAG-3' (SEQ ID NO:193).

Mouse qPCR oligonucleotides are as follows. β-actin forward 5'-CTAAGGCCAACCGTGAAAAG-3' (SEQ ID NO:194), reverse 5'-ACCAGAGGCATACAGGGACA-3' (SEQ ID NO:195); *Yb1* forward 5'-GGGTTACAGACCACGATTCC-3' (SEQ ID NO:196), reverse 5'-GGCGATACCGACGTTGAG-3' (SEQ ID NO:197); vimentin forward 5'-TCCAGCAGCTTCCTGTAGGT-3' (SEQ ID NO:198), reverse 5'-CCCTCACCTGTGAAGTGGAT-3' (SEQ ID NO:199); *Cd44* forward 5'-ACAGTACCTTACCCACCATG-3' (SEQ ID NO:200), reverse 5'-GGATGAATCCTCGGAATTAC-3' (SEQ ID NO:201); *Mta1* forward 5'-AGTGCGCCTAATCCGTGGTG-3' (SEQ ID NO:202), reverse 5'-CTGAGGATGAGAGCAGCTTTCG-3' (SEQ ID NO:203).

siRNA/shRNA sequences are as follows. Control (D-001810-01) 5'-UGGUUUACAUGUCGACUAA-3' (SEQ ID NO:204); vimentin (L-003551) 5'-UCACGAUGACCUUGAAUAA-3' (SEQ ID NO:205), 5'-GGAAAUGGCUCGUCACCUU-3' (SEQ ID NO:206), 5'-GAGGGAAACUAAUCUGGAU-3' (SEQ ID NO:207), 5'-UUAAGACGGUUGAAACUAG-3' (SEQ ID NO:208); *YB1* (L-010213) 5'-CUGAGUAAAUGCCGGCUUA-3' (SEQ ID NO:209), 5'-CGACGCAGACGCCCAGAAA-3' (SEQ ID NO:210), 5'-GUAAGGAACGGAUAUGGUU-3' (SEQ ID NO:211), 5' -GCGGAGGCAGCAAAUGUUA-3' (SEQ ID NO:212); *MTA1* (L-004127) 5'-UCACGGACAUUCAGCAAGA-3' (SEQ ID NO:213), 5'-GGACCAAACCGCAGUAACA-3' (SEQ ID NO:214), 5'-GCAUCUUGUUGGACAUAUU-3' (SEQ ID NO:215), 5'-CCAGCAUCAUUGAGUACUA-3' (SEQ ID NO:216); *CD44* (L-009999) 5'-GAAUAUAACCUGCCGCUUU-3' (SEQ ID NO:217), 5'-CAAGUGGACUCAACGGAGA-3' (SEQ ID NO:218), 5'-CGAAGAAGGUGUGGGCAGA-3' (SEQ ID NO:219), 5'- GAUCAACAGUGGCAAUGGA-3' (SEQ ID NO:220); *4EBP1* (L-003005) 5'-CUGAUGGAGUGUCGGAACU-3' (SEQ ID NO:221), 5'-CAUCUAUGACCGGAAAUUC-3' (SEQ ID NO:222), 5'-GCAAUAGCCCAGAAGAUAA-3' (SEQ ID NO:223), 5'-GAGAUGGACAUUUAAAGCA-3' (SEQ ID NO:224); *4EBP2* (L-018671) 5'-GCAGCUACCUCAUGACUAU-3' (SEQ ID NO:225), 5'-GGAGGAACUCGAAUCAUUU-3' (SEQ ID NO:226), 5'-GCAAUUCUCCCAUGGCUCA-3' (SEQ ID NO:227), 5'-UUGAACAACUUGAACAAUC-3' (SEQ ID NO:228); rictor (LL-016984) 5' -GACACAAGCACUUCGAUUA-3' (SEQ ID NO:229), 5' -GAAGAUUUAUUGAGUCCUA-3' (SEQ ID NO:230), 5'-GCGAGCUGAUGUAGAAUUA-3' (SEQ ID NO:231), 5'-GGGAAUACAACUCCAAAUA-3' (SEQ ID NO:232); *PTEN* SH-003023-01-10 5'-GCTAAGAGAGGTTTCCGAA-3' (SEQ ID NO:233), SH-003023-02-10 5'-AGACTGATGTGTATACGTA-3' (SEQ ID NO:234).

### Example 7: Effect of mTOR and MEK Inhibitors on Translation Efficiency

To further examine the effect of mTOR inhibitors on translational efficiency in PC3 prostate cancer cells, the ATP site inhibitor of mTOR PP242 was compared to the allosteric inhibitor of mTOR, rapamycin and to another ATP site inhibitor. Figure 26 shows a representative comparison of change in translational efficiency versus DMSO control by the allosteric mTOR inhibitor rapamycin and the ATP site inhibitor PP242 (Figure 26A) and the two ATP site inhibitors INK128 (100 nM) and PP242 (as described in Example 6) (Figure 26B). Each data point represents a single gene. Data points highlighted in red have statistically significant changes in translational efficiency versus DMSO control as described herein. Figure 26A shows that most of the genes where translational efficiency decreases due to PP242 also have decreased translational efficiencies caused by rapamycin; however, the magnitude of rapamycin decrease is substantially less than with PP242. In contrast, treatment with INK128 not only impacts the same gene set as PP242, but also has approximately the same magnitude of change on a gene by gene basis (Figure 26B). This experiment shows that two different drugs that act on a target through the same mechanism (such as PP242 and INK128) will affect translational efficiency in a similar manner - that is, the methods of this disclosure can be used to find active compounds that are pharmacological "mimics" of each other. Even when two compounds have different mechanisms of action on the same target (such as PP242 and rapamycin), effects on translational efficiency can be detected although the degree of the translational effect may be different.

The following experiment was performed to show that translational profiling can be used for a variety of agents and targets. The mTOR inhibitors alter the PI3K/AKT pathway. Here, a MEK/ERK pathway inhibitor (GSK212) was examined.

**Cell Culture.** SW620 human colon cancer cells were cultured in DMEM media supplemented with penicillin G (100 U/ml), streptomycin (100 µg/ml), and 10% FBS in a humidified atmosphere of 5% CO₂ maintained at 37°C.

**MEK and mTOR Inhibitor Treatment.** SW620 cells (ATCC, passage 12) were seeded at about 75% confluence 24 hrs prior to drug treatment. The following day, cells were treated with either DMSO (vehicle control) or MEK inhibitor GSK-11202012 (referred to herein as "GSK212") at 250 nM for 8 hrs or with either DMSO or the mTOR inhibitor PP242 at 2.5 µM for 3 hrs. About 6 x 10⁶ cells/10 cm plate and about 1 x 10⁶ cells/well of a 6-well plate were harvested for ribosome profiling and Western blot analysis, respectively, following drug treatment.

**Western Blot Analysis.** Cells were washed with PBS and lysed in 1X cell lysis buffer (Cell Signaling) for 15 min at 4°C. Lysates were sonicated briefly, clarified by centrifugation for 15 min at 14, 000 rpm, and supernatants were then collected. Protein concentration in the soluble fraction was determined by BCA protein assay (Thermo Scientific). A 4-20% Bis-Tris gradient gel (Invitrogen) was used to resolve 20 µg of protein and transferred to nitrocellulose membrane. The resulting membranes were blocked for 1 hr at room temperature with Odyssey blocking solution (LI-COR) and then incubated with primary antibodies at 4°C overnight. The following day, the blots were washed 3 times, 10 min each in TBST, and incubated with IR-conjugated goat anti-rabbit IgG secondary antibody (IRDye 800 CW at 1:20,000; LI-COR) for 1 hour at room temperature. The blots were then washed, scanned, and specific proteins were detected using the LI-COR Odyssey infrared imager. The following antibodies from Cell Signaling were used at 1:1000 dilution: anti-phospho-eIF4E(Ser209)(#9741),anti-phospho-rpS6(Ser235/236)(#4858),anti-phospho-ERK1/2(Thr202/Tyr204)(#4370),anti-phospho-p70S6K(Thr421/Ser424)(#9204),anti-phospho-p90RSK(Thr359/Ser363)(#9344), anti-phospho-4EBP(Ser65), anti-phospho-pAKT(Ser473), anti-phospho-eIF4E(Ser209), and anti-β-actin (#4970). Actin was used as a loading amount control.

### mTOR inhibitor PP242 and MEK inhibitor GSK212 are clearly distinguishable by differential effects on translational efficiencies.

GSK212 is a very potent and selective MEK inhibitor with IC₅₀ values of about 1 nM for both MEK1 and MEK2. The potency of GSK212 in 72 hour proliferation assays on SW620 cells is 20-30 nM (data not shown). In this concentration range, GSK212 has profound effects on the transcriptional program of sensitive cells like SW620. In the experiments described herein, exposure to SW620 cells was at a supra-therapeutic concentration (250 nM) for 8 hours. No evidence of inhibition of proliferation or induction of apoptosis was apparent over this time frame. Phosphorylation of ERK and p90RSK in SW620 cells was completely inhibited (Figure 27A). At this concentration, only partial inhibition of the phosphorylation of the ribosomal protein S6 (rpS6) and its canonical kinase S6K (p70RSK) was achieved. Similarly, partial inhibition of the phosphorylation of eIF4E was observed (Figure 27A). When used at concentrations relevant for MEK inhibition (*e.g*., at 25 nM to 100 nM), little or no effect on phosphorylation of S6, eIF4E and 4EBP1 was detectable (data not shown).

SW620 cells are less sensitive to inhibition by PP242 than are PC3 cells. At 2.5 µM PP242, phosphorylation of S6K, S6 and 4EBP1 was substantially inhibited in PC3 cells (Figure 27B). The inhibitor is less potent in SW620 cells, such that some phosphorylation of 4EBP was observed even at 10 µM (Figure 27B). From the dose response shown in this figure, it is nonetheless clear that significant inhibition of phosphorylation could be achieved with 2.5 µM PP242.

As is apparent in Figure 27, treatment of SW620 cells with the MEK inhibitor and with the mTOR inhibitor have distinctly different impacts on the phosphorylation state of important components of the translational machinery, most notably 4EBP1. A corresponding difference on the translation efficiencies of mRNAs that are strongly dependent on the levels of free eIF4E was confirmed by comparing the effects of 250 nM GSK212 and 2.5 µM PP242 in SW620 cells (Figure 26C). First, most genes shown to be sensitive to 2.5 µM PP242 in PC3 cells (data points in red) are also sensitive in SW620 cells. Second, with only three exceptions, treatment with the MEK inhibitor has little or no effect on the translational efficiencies of these genes. This further demonstrates the ability of translational efficiency measurements to distinguish between drugs and drug mechanisms of action.

### Characteristic transcriptional gene signature of MEK inhibitor GSK212 can be observed in translational rates as distinct from translational efficiencies.

A signature for MEK inhibition in cells sensitive to these agents as determined by microarray analysis has been described previously (Pratilas et al., Proc. Nat'l Acad. Sci U.S.A. 105: 4519, 2009). This signature was compared with signatures derived from RNA-seq and transcriptional profiling of GSK212 on SW620 cells, as provided in Table 8. There is general agreement between the published signature and the signatures observed both in transcription (RNA) and in translational rates (RPF). The strong concordance between signatures from transcription and translational rate in this setting corresponds to the MEK signature that was originally identified and is associated with robust transcriptional changes which, for the most part, are reflected in changes in translational rate.

**Table 8. Transcriptional, translational rate, and translational efficiency signatures of MEK inhibitor on SW620 cells**

| **PNAS ID / HGNC ID** | **SEQ ID NO** | **ENSEMBL ID** | **Description** | **PNAS** | **Profile** | | |
|---|---|---|---|---|---|---|---|
| | | | | **rna** | **rna** | **rpf** | **TE** |
| ALF / GTF2A1L | 235 | ENSG00000242441 | general transcription factor Iia, 1-like | 2.7 | NA | NA | NA |
| SEMA6A / SEMA6A | 236 | ENSG00000092421 | semaphorin 6A | 2.1 | 2.3 | 0.7 | -1.5 |
| HYDIN / HYDIN | 237 | ENSG00000157423 | hydrocephalus inducing | 2.1 | **mInf** | **Inf** | **Inf** |
| KIR3DL2 / KIR3DL2 | 238 | ENSG00000240403 | killer cell immunoglobulin-like receptor, three domains, long cytoplasmic tail, 2 | 1.7 | NA | NA | NA |
| BYSL / BYSL | 239 | ENSG00000112578 | bystin-like | -1.2 | -0.8 | -0.6 | 0.2 |
| ELOVL6 / ELOVL6 | 240 | ENSG00000170522 | ELOVL family member 6, elongation of long chain fatty acids-like 6 | -1.5 | -1.4 | -0.8 | 0.6 |
| SLC1A5 / SLC1A5 | 241 | ENSG00000105281 | solute carrier family 1 (neutral amino acid transporter), member 5 | -1.2 | -0.1 | -0.5 | -0.3 |
| CHSY1 / CHSY1 | 242 | ENSG00000131873 | carbohydrate (chondroitin) synthase 1 | -1.3 | 0.0 | -0.7 | -0.7 |
| IL8 / IL8 | 243 | ENSG00000169429 | interleukin 8 | -2.5 | -5.6 | -3.2 | 2.4 |
| FOS / FOS | 244 | ENSG00000170345 | v-fos FBJ murine osteosarcoma viral oncogene homolog | -3.4 | -2.9 | -2.7 | 0.2 |
| B4GALT6/ B4GALT6 | 245 | ENSG00000118276 | UDP-Gal:betaGlcNAc beta 1,4-galactosyltransferase, polypeptide 6 | -1.7 | 0.0 | -0.1 | 0.0 |
| CCND1 / CCND1 | 246 | ENSG00000110092 | cyclin D1 (PRAD1: parathyroid adenomatosis 1) | -2.2 | -1.8 | -2.0 | -0.3 |
| ETV5 / ETV5 | 247 | ENSG00000244405 | ets variant gene 5 (ets-related molecule) | -1.7 | -2.6 | -5.6 | -3.0 |
| ETV4 / ETV4 | 248 | ENSG00000175832 | ets variant gene 4 (E1A enhancer binding protein, E1AF) | -2.2 | -2.2 | -2.8 | -0.6 |
| SLC4A7 / SLC4A7 | 249 | ENSG00000033867 | solute carrier family 4, sodium bicarbonate cotransporter, member 7 | -1.6 | -0.2 | -1.1 | -0.9 |
| ETV1 / ETV1 | 250 | ENSG00000006468 | ets variant gene 1 | -2.6 | -3.5 | **mInf** | **mInf** |
| MAFF / MAFF | 251 | ENSG00000185022 | v-maf musculoaponeurotic fibrosarcoma oncogene homolog F (avian) | -2.7 | -1.7 | -1.1 | 0.6 |
| IER3 / IER3 | 252 | ENSG00000137331 | immediate early response 3 | -3.3 | -2.2 | -2.3 | -0.1 |
| LIF / LIF | 253 | ENSG00000128342 | leukemia inhibitory factor (cholinergic differentiation factor) | -3.2 | -1.7 | -0.9 | 0.9 |
| SPRY4 / SPRY4 | 254 | ENSG00000187678 | sprouty homolog 4 (Drosophila) | -2.6 | -5.2 | -7.1 | -2.0 |
| DUSP4 / DUSP4 | 255 | ENSG00000120875 | dual specificity phosphatase 4 | -2.2 | -2.0 | -2.4 | -0.4 |
| LNK / SH2B3 | 256 | ENSG00000111252 | SH2B adaptor protein 3 | -2.0 | 0.2 | -0.7 | -0.9 |
| GPR3 / GPR3 | 257 | ENSG00000181773 | G protein-coupled receptor 3 | -2.1 | **mInf** | -2.8 | **Inf** |
| TNC/ TNC | 258 | ENSG00000041982 | tenascin C (hexabrachion) | -2.5 | 1.1 | -0.5 | -1.6 |
| POLR3G / POLR3G | 259 | ENSG00000113356 | polymerase (RNA) III (DNA directed) polypeptide G (32kD) | -1.0 | -0.7 | -0.8 | -0.1 |
| WDR3 / WDR3 | 260 | ENSG00000065183 | WD repeat domain 3 | -1.1 | 0.0 | -1.0 | -1.0 |
| BXDC2 / BRIX1 | 261 | ENSG00000113460 | brix domain containing 2 | -1.2 | -1.1 | -0.8 | 0.2 |
| CD3EAP / CD3EAP | 262 | ENSG00000117877 | CD3E antigen, epsilon polypeptide associated protein | -1.4 | -0.6 | -1.1 | -0.5 |
| EGR1 / EGR1 | 263 | ENSG00000120738 | early growth response 1 | -2.1 | -1.0 | -4.1 | -3.1 |
| PHLDA2 / PHLDA2 | 264 | ENSG00000181649 | pleckstrin homology-like domain, family A, member 2 | -2.2 | -2.1 | -1.1 | 1.1 |
| ARID5A / ARID5A | 265 | ENSG00000196843 | AT rich interactive domain 5A (MRF1-like) | -1.7 | -0.5 | -0.9 | -0.4 |
| DUSP6 / DUSP6 | 266 | ENSG00000139318 | dual specificity phosphatase 6 | -2.6 | -6.3 | -9.3 | -3.1 |
| SPRY2 / SPRY2 | 267 | ENSG00000136158 | sprouty homolog 2 (Drosophila) | -4.0 | -1.7 | -1.3 | 0.4 |
| DDX21 / DDX21 | 268 | ENSG00000165732 | DEAD (Asp-Glu-Ala-Asp) (SEQ ID NO:287)box polypeptide 21 | -1.1 | -0.3 | -1.0 | -0.6 |
| GTPBP4/ GTPBP4 | 269 | ENSG00000107937 | GTP binding protein 4 | -1.1 | -0.4 | -0.7 | -0.3 |
| PPAT / PPAT | 270 | ENSG00000128059 | phosphoribosyl pyrophosphate amidotransferase | -1.1 | -0.4 | -0.7 | -0.3 |
| HSPC111 / NOP16 | 271 | ENSG00000048162 | hypothetical protein HSPC111 | -1.3 | -1.5 | -0.9 | 0.6 |
| MYC / MYC | 272 | ENSG00000136997 | v-myc myelocytomatosis viral oncogene homolog (avian) | -2.4 | -1.3 | -1.6 | -0.3 |
| MAP2K3 / MAP2K3 | 273 | ENSG00000034152 | mitogen-activated protein kinase kinase 3 | -1.5 | -1.6 | -0.6 | 1.0 |
| GNL3 / GNL3 | 274 | ENSG00000163938 | guanine nucleotide binding protein-like 3 (nucleolar) | -1.0 | -0.7 | -0.8 | -0.2 |
| RRS1 / RRS1 | 275 | ENSG00000179041 | RRS1 ribosome biogenesis regulator homolog (S. cerevisiae) | -1.8 | -0.9 | -0.8 | 0.1 |
| FOSL1 / FOSL1 | 276 | ENSG00000175592 | FOS-like antigen 1 | -4.2 | -3.6 | -4.1 | -0.5 |
| FLJ10534 / TSR1 | 277 | ENSG00000167721 | TSR1, 20S rRNA accumulation, homolog (S. cerevisiae) | -1.1 | -0.3 | -0.8 | -0.6 |
| SPRED2 / SPRED2 | 278 | ENSG00000198369 | sprouty-related, EVH1 domain containing 2 | -1.0 | -2.1 | -3.5 | -1.3 |
| HMGA2 / HMGA2 | 279 | ENSG00000149948 | high mobility group AT-hook 2 | -1.6 | -2.9 | -1.7 | 1.2 |
| PLK3 / PLK3 | 280 | ENSG00000173846 | polo-like kinase 3 (Drosophila) | -2.3 | -3.1 | -2.4 | 0.7 |
| YRDC / YRDC | 281 | ENSG00000196449 | yrdC domain containing (E. coli) | -1.2 | -0.7 | -0.6 | 0.1 |
| POLR1C / POLR1C | 282 | ENSG00000171453 | polymerase (RNA) I polypeptide C, 30kDa | -1.0 | -0.8 | -0.6 | 0.2 |
| PPAN / PPAN | 283 | ENSG00000130810 | peter pan homolog (Drosophila) | -1.2 | -0.4 | -1.1 | -0.7 |
| PYCRL / PYCRL | 284 | ENSG00000104524 | pyrroline-5-carboxylate reductase-like | -2.6 | -0.2 | 0.0 | 0.1 |
| GEMIN4 / GEMIN4 | 285 | ENSG00000179409 | gem (nuclear organelle) associated protein 4 | -1.2 | -0.1 | -0.6 | -0.5 |
| TNFRSF12A/TNFRSF12A | 286 | ENSG00000006327 | tumor necrosis factor receptor superfamily, member 12A | -1.5 | -2.2 | -2.0 | 0.2 |

| | | | | | | | |
|---|---|---|---|---|---|---|---|
| For Table 8: The transcriptional signature of V600E-BRAF tumor cells treated with MEK inhibitor PD0325901 is compared with the transcriptional, translational, and translational efficiency signatures of SW620 cells treated with the MEK inhibitor GSK212. The depicted gene set and data for V600E-BRAF tumor cells are adapted from Table S2 of V600E-BRAF is associated with disabled feedback inhibition of RAF-MEK signaling and elevated transcriptional output of the pathway (Pratilas *et al*., 2009). Any gene where the value for the GSK212 treated sample is 0 is shown as "mInf" (log₂(0/x) = -infinity) for the log₂ fold-change value. Any gene where the value for the DMSO sample is 0 is shown as "Inf" (log₂(x/0) = infinity) for the log₂ fold-change value. All values are log₂ MEKi/DMSO. Any gene where data is unavailable in the ribosomal profiling experiment is shown as "NA." | | | | | | | |

### Unique insights into MEK inhibition are nonetheless apparent in translational efficiencies.

In contrast to solely translation rate, examination of the translational efficiencies of the mRNAs that make up the MEK signature indicates a set of gene products that may have unique importance. Protein synthesis from some mRNAs, such as those from BYSL, DUSP4 and POLR3G, was almost exclusively transcriptionally mediated and accordingly had translational efficiency changes near zero. In contrast, mRNAs from genes like ETV5 and SPRY4, which are transcriptionally down-regulated, had the production of their corresponding proteins further inhibited at the translational level leading to profound control. Conversely, production of the mRNA from the IL8, PHLDA2 and MAP2K3 genes are examples where synthesis is less inhibited (despite transcriptional data) due to an offsetting increase in translational efficiency, such as a counter-regulation. In addition to genes involved in the MEK signature, there were a number of other genes in SW620 cells that had changes in translational efficiency associated with MEK inhibition (data not shown). In any case, such genes having translational efficiency or a combination of translational efficiency and transcription control are of interest as therapeutic targets or for use in examining the action of different therapeutic agents (*e.g*., such as mimic action).

### Example 8: Translational Profiling of a Fibrotic Disease Cell Model

TGFβ-mediated transformation of fibroblasts is well-established as an essential step in fibroplasia, a key component of many fibrotic disorders (Blobe et al., N. Engl. J. Med. 342:1350, 2000; Border and Noble, N. Engl. J. Med. 331:1286, 1994). As described in this Example, analysis of changes in translational efficiencies reveals disease-associated cellular changes accompanying this transformation. For example, co-administration of TGF-β with an inhibitor of a PI3K/Akt/mTOR pathway enzyme ("PAMi") reverses or prevents the changes observed in a fibrotic disorder-related pathway (*i.e*., normalizes the translational efficiencies of the genes) and inhibits increased production of fibrotic disorder biomarker proteins, type 1 procollagen and α-actin (which are both hallmarks of TGF-β-mediated fibroblast transformation to myofibroblasts). Although these biomarkers are only affected at the transcriptional level and not the translational level, they nonetheless provide a means to monitor the pathogenic state of the cell that is mediated by other fibrosis-related genes that are affected at the translational level.

**Cell Culture.** Normal human lung fibroblasts (Lonza #CC-2512) were cultured in DMEM + 10% FBS supplemented with Penicillin, Streptomycin and Glutamax (Invitrogen) at 37°C in a humidified incubator with 5% CO₂. Cell passage numbers 2 through 5 were used for all experiments.

**Fibroblast Transformation and Treatment.** On Day 0, fibroblasts were seeded and cultured under normal conditions overnight. On Day 1, media was removed, cells were washed with PBS and then incubated for 48 hrs in serum free media (DMEM supplemented with penicillin, streptomycin, and glutamax). On Day3, media was removed and cells were cultured for 24 hrs with fresh serum free media ± PAMi and ± 10ng/ml TGF-β. After this 24 hour incubation, about 6x10⁶ cells/10 cm plate and about 1x10⁶ cells/well of a 6-well plate were used for ribosomal profiling and western blot analysis, respectively.

**Ribosomal Profiling.** Cells were washed with cold PBS supplemented with cycloheximide and lysed with 1x mammalian lysis buffer for 10min on ice. Lysates were clarified by centrifugation for 10 min at 14,000 rpm and supernatants were collected. Cell lysates were processed to generate the ribosomal protected fragments and total mRNA according to the instructions included with the ARTseq Ribosome Profiling Kit. Sequencing of total RNA (RNA) and of ribosome-protected fragments of RNA (RPF) was carried out with standard Illumina rna seq methodology.

**Bioinformatics Analysis.** RNA-seq reads were processed with tools from the FASTX-Toolkit (fastq_quality_trimmer, fastx_clipper and fastx_trimmer). Unprocessed and processed reads were evaluated for a variety of quality measures using FastQC. Processed reads were mapped to the human genome using Tophat. Gene-by-gene assessment of the number of fragments strictly and uniquely mapping to the coding region of each gene was conducted using HTSeq-count, a component of the HTSeq package. Differential analyses of the transforming effect of TGF-β on fibroblasts and effect of PAMi treatment on this transformation were carried out with the software packages DESeq for transcription (RNA counts) and translational rate (RPF counts) and BABEL for translational efficiency based upon ribosomal occupancy as a function of RNA level (RNA and RPF counts). Genes with low counts in either RPF or RNA were excluded from differential analyses. Pathway and network analyses of differential data was conducted using Ingenuity Pathway Analysis (IPA).

**Western Blot Analysis.** Cells were washed with PBS and lysed in 1X cell lysis buffer (Cell Signaling) for 15 min at 4°C. Lysates were sonicated briefly and clarified by centrifugation for 15 min at 14, 000 rpm and supernatants were collected. Protein concentration in the soluble fraction was determined by BCA protein assay (Thermo Scientific). Samples of protein (20 µg) were resolved on 4-20% Bis-Tris gradient gel (Invitrogen) and transferred to nitrocellulose membrane. The resulting blots were blocked for 1 hr at room temperature with Odyssey blocking solution (LI-COR) and then incubated with primary antibodies at 4°C overnight. The following day, the blots were washed 3 times, 10 min each in TBST, and incubated with goat anti-rabbit fluorescent conjugated secondary antibody (IRDye 800 CW at 1:20,000; LI-COR) for 1 hour at room temperature. The blots were then washed and scanned, specific proteins were detected by using the LI-COR Odyssey infrared imager. The following antibodies were used at 1:1000 dilution from Sigma (α-actin #A2547) and Cell Signaling: anti-phospho-4EBP(Ser65), anti-phospho-rpS6(Ser235/236)(#4858), anti-phospho-ERK1/2(Thr202/Tyr204)(#4370), anti-phospho-p70S6K(Thr421/Ser424)(#9204), anti-phospho-pAKT(Ser473), anti-phospho-MNK(Thr197/202), anti-β-actin (#4970).

**Procollagen Type 1 Analysis.** Culture Media was collected, centrifuged to pellet cellular debris, and stored at -80°C. Procollagen Type 1 C-Peptide (PIPC) was quantified using the (PIP) EIA kit (Clontech Cat# MK101) according to manufacturer's instructions.

### PI3K/Akt/mTORi co-administration prevents transformation of fibroblasts to myofibroblasts by TGFβ.

Transformation of fibroblasts to myofibroblasts by treatment with TGF-β for 24 hours was accompanied by an approximately 7-fold increase in procollagen production, while treatment with a PAMi was able to block this increase (EC₅₀ of about 0.2 µM) (Figure 28). Expression of TGF-β induced myofibroblast differentiation marker, smooth muscle actin (α-SMA), was also analyzed by Western blot analysis (Figure 29). After 24 hours of TGF-β stimulation, increased α-SMA protein levels were detected, while the level of β-actin did not change. As with procollagen, co-incubation of the cells with a PAMi maintained the α-SMA protein at pretreatment levels. Ribosomal profiling showed that the effect of the PAMi on both procollagen and α-SMA were a consequence of preventing fibroblast transformation and transcriptional regulation (instead of a decrease in translation efficiency of mRNA to protein). Specifically, the translational efficiencies of the procollagen and α-SMA were essentially independent of TGF-β and PAMi treatment.

TGF-β-dependent activation of the PI3K/Akt/mTOR and ERK pathways were also examined by Western blot analysis (Figure 29). Western analysis also indicates that TGF-β stimulated phosphorylation of AKT, 4EBP, S6K, S6 in the mTOR pathway and modestly increased the phosphorylation of ERK. Co-incubation of cells with the PAMi abolished TGF-β-dependent increases in phosphorylation of AKT, 4EBP, S6K, and S6 at 0.625 µM, as well as decreasing the α-SMA protein to pretreatment levels.

Ribosomal profiling was used to measure changes in transcription and translation on a genome-wide basis accompanying TGF-β-dependent transformation of fibroblasts to myofibroblasts. This system is known to be driven in large part by transcriptional activation, and changes in translational rate and RNA levels on a genome-wide level were highly correlated (*see* Figure 30). In contrast, changes in translational efficiency were relatively independent of transcriptional and translational rate changes. Thus, in this case, measurements of translational efficiency provide a unique window into cellular biology of fibrotic disorders. Correspondingly, the outcome of pathway analysis based on gene identification via changes in translational efficiency upon TGF-β treatment is quite distinct from analyses based on transcription or translational rate. These three gene signatures were analyzed for pathway and network connections using Ingenuity Pathway Analysis (IPA). Some characteristics of these gene lists, including the identity of the pathway with the highest statistical association for each signature, are listed in Table 9 (while these are the most significant, it should be noted that significant association of these gene lists with other pathways were observed). Most notably, genes showing changes in RNA levels and translational rates were most strongly associated with Hepatic Fibrosis/Hepatic Stellate Cell Activation (*see* Figures 31 and 32). This action of TGF-β in fibroblasts recapitulates much of the behavior observed in liver fibrosis.

**Table 9. Properties of Fibrotic Disorder Gene Signatures from IPA Analysis**

| | **RNA (Transcriptome)** | **RPF (Translational Rate)** | **Translational Efficiency** |
|---|---|---|---|
| **p-value threshold for differential** | 0.1 | 0.05 | 0.05 |
| **# genes meeting threshold** | 194 | 211 | 238 |
| **% of total gene set** | 4.20% | 4.50% | 5.10% |
| **Most significant pathway / category from IPA analysis** | Hepatic Fibrosis / Hepatic Stellate Cell Activation | Hepatic Fibrosis / Hepatic Stellate Cell Activation | Unique Fibrosis-Associated Pathway |

In contrast, the gene signature showing changes in translational efficiency was most strongly associated with regulation of a pathway not previously observed to be associated with fibrotic disorders. All genes identified in this new pathway showed a significant increase in translational efficiency (TE) (Figure 33), which extends far beyond these few genes. For example, 118 of the 141 genes in the pathway evaluated in this study move in concert, having an increase in translational efficiency (Figure 34, panel A). The translational efficiencies of the 141 pathway-associated genes in fibroblasts before treatment with TGF-β (which induces a fibrotic-disease type condition) were low (mean value -1.70 log2 relative to population mean); the impact of TGF-β induced transformation was to increase the translational efficiency of many genes in this signature (mean value of signature upon TGF-β treatment was -1.05). Nonetheless, this was still 2-fold lower than the overall population and indicates this pathway is a bottleneck in cellular transformation. For the subset of 12 genes described previously, 11 of 12 move toward the untransformed, normal state after treatment with PAMi (Figure 33). The mean increase in translational efficiency by TGF-β in these 12 genes is 1.3 log2; the presence of PAMi decreases this value to only 0.4 log2. Similar results are seen for all genes in the pathway (Figure 34), wherein 104 of 141 genes move toward normal. The mean increase in translational efficiency by TGF-β in these 12 genes was 0.65 log2; the presence of PAMi decreases this value to only 0.09 log2. Clearly, the presence of PAMi maintains the translational efficiencies of the genes in fibrotic disorder-associated pathway at their normal state in fibroblasts. Normalization of this pathway by PAMi is due to substantially inhibiting TGF-β induced transformation of fibroblasts to fibrotic myofibroblasts.

**Conclusion.** Comparison of translational efficiencies between the normal, healthy state (fibroblasts) and pathogenic state (fibrotic myofibroblasts induced by TCFβ treatment) identified a novel pathway previously not associated with fibrosis, which is a novel insight into a key role of translational efficiency in the pathogenesis of fibrotic disease. Further, a PAMi agent that modulates this fibrotic disorder-associated pathway and prevents TGF-β-mediated fibroblast to myofibroblast transformation confirms the association of this pathway with fibrotic disease and, thus, shows that components and regulators of this pathway are new targets. The methods of the instant disclosure show that new gene signatures having altered translational profiles (*e.g*., altered translational efficiency) may be identified using such methods. Furthermore, these data show that an agent or therapeutic that normalizes a translational profile may also be identified. Finally, these data show that targets not previously validated for a particular disorder (in this case, fibrosis), can be identified and validated using the methods of this disclosure.

### Example 9: Translational Profiling of a Neurodevelopmental Disease Model

An exemplary neurodevelopmental disease or disorder is Fragile X syndrome, which is caused by a redundant trinucleotide (CGG) repeat in the 5' UTR of the fragile X mental retardation 1 gene (FMR1). This causes silencing of the FMR1 gene at the transcriptional level and results in the lack of fragile X mental retardation 1 protein (FMRP) expression. FMRP is a cytoplasmic RNA binding protein that associates with polyribosomes as part of a large ribonucleoprotein complex and acts as a negative regulator of translation. Hence, FMRP is thought to regulate the translation of specific mRNAs that are critical for correct development of neurons and synaptic function. The Fragile X syndrome is directly linked to this lack of FMRP expression or loss of FMRP function (*i.e.*, loss of translational control). Indeed, *Fmr1* knockout mice have abnormal dendritic spines, which are thought to be the basis of the disease associated mental retardation (*see*, *e.g*., Darnell et al., Cell 146: 247,2011).

**Cell Culture.** SH-SY5Y human neuroblastoma cells were cultured in F12/DMEM media (1:1 ratio) supplemented with penicillin G (100 U/ml), streptomycin (100 µg/ml), and 10% FBS. HEK293 human embryonic fibroblasts were cultured in DMEM media supplemented with penicillin G (100 U/ml), streptomycin (100 µg/ml), and 10% FBS. All cells were cultured in a humidified atmosphere of 5% CO₂ maintained at 37°C.

**siRNA Transfection.** SH-SY5Y cells (ATCC, passage 8) and HEK293 (ATCC, passage 12) were reverse transfected with 100 nM siControl (AM4611) or siFRM1 (ID# s5316) for 3 days using Lipofectamine RNAiMax (Invitrogen) according to manufacturer's protocol. All siRNAs were purchased from Invitrogen. About 3 x 10⁶ cells/10 cm plate and about 5 x 10⁵ cells/well of a 6-well plate were harvested for ribosome profiling and Western blot analysis following siRNA transfection, respectively.

**Western Blot Analysis.** Cells were washed with PBS and lysed in 1X cell lysis buffer (Cell Signaling) for 15 min at 4°C. Lysates were sonicated briefly, clarified by centrifugation for 15 min at 14, 000 rpm, and supernatants were then collected. Protein concentration in the soluble fraction was determined by BCA protein assay (Thermo Scientific). A 4-20% Bis-Tris gradient gel (Invitrogen) was used to resolve 20 µg of protein and transferred to nitrocellulose membrane. The resulting membranes were blocked for 1 hr at room temperature with Odyssey blocking solution (LI-COR) and then incubated with primary antibodies at 4°C overnight. The following day, the blots were washed 3 times, 10 min each in TBST, and incubated with IR-conjugated anti-rabbit IgG and anti-mouse IgG secondary antibody (IRDye 800 CW at 1:20,000; LI-COR) for 1 hour at room temperature. The blots were then washed, scanned, and specific proteins were detected using the LI-COR Odyssey infrared imager. The following antibodies were used at 1:1000 dilution from Cell Signaling: anti-FMRP (#4317), anti-TSC2 (#4308), and anti-β-actin (#4970).

**Ribosomal Profiling.** Cells were washed with cold PBS supplemented with cycloheximide and lysed with 1x mammalian lysis buffer for 10 min on ice. Lysates were clarified by centrifugation for 10 min at 14,000 rpm and supernatants were collected. Cell lysates were processed to generate the ribosomal protected fragments and total mRNA according to the instructions included with the ARTseq Ribosome Profiling Kit. Sequencing of total RNA (RNA) and of ribosome-protected fragments of RNA (RPF) was carried out with standard Illumina rna seq methodology.

**Bioinformatics Analysis.** RNA-seq reads were processed with tools from the FASTX-Toolkit (fastq_quality_trimmer, fastx_clipper and fastx_trimmer). Unprocessed and processed reads were evaluated for a variety of quality measures using FastQC. Processed reads were mapped to the human genome using Tophat. Gene-by-gene assessment of the number of fragments strictly and uniquely mapping to the coding region of each gene was conducted using HTSeq-count, a component of the HTSeq package. Differential analyses of the knockdown of the FMR1 gene were carried out with the software packages DESeq for transcription (RNA counts) and translational rate (RPF counts) and BABEL for translational efficiency based upon ribosomal occupancy as a function of RNA level (RNA and RPF counts). Genes with low counts in either RPF or RNA were excluded from differential analyses.

### Expression levels of FMRP and TSC2 following transient knockdown of FMR1.

SH-SY5Y cells were transfected with either siControl or siFMR1 at 100 nM for 3 days. Protein levels of FMRP and TSC2 (a known translational target of FMRP) were evaluated by western blot analysis (Figure 35), and β-actin was used as a loading control. The uppermost band observed in the western blot analysis represents the FMR1 isoform and is sensitive to the siFMR1 knockdown. An approximately 30% knockdown efficiency of FMRP was determined by integrating the band intensities, as well as quantitating by q-PCR analysis (data not shown). The protein expression levels of TSC2 increased after knocking down FMRP, a negative translational regulator.

Ribosomal profiling was used to measure changes in transcription and translation on a genome-wide basis after transfecting the cells with either siControl or siFMR1. Analysis of the sequencing results for the FMR1 gene shows that about a 30% reduction was observed, consistent with the western blot and q-PCR analyses. The FMRP specific target, TSC2, showed a corresponding ∼30% increase in the translational rate in the absence of a change in transcriptional levels. On a genome-wide evaluation, knockdown of the FMR1 gene resulted in minimal changes in the transcriptome (*see*, Figure 36) with only a log2 fold change of 2.3 and 1.6 for the top two up-regulated genes (log2 fold change of -1.6 and -1.2 for the top two down-regulated genes). Changes in the translational rate were identified for a number of genes in the absence of a change in transcriptional levels, corresponding to a change in the translational efficiency. These results indicate that FMRP is responsible for the translational regulation of this specific set of genes.

Known translation targets of FMRP have been reported to include eEF2, eEF1, all three eIF4G isoforms, TSC2 and SYNGAP1. Consistent with these reports, the sequencing data showed that for the knockdown of FMRP, the elongation factors (eEF2 and eEF1) as well as TSC2 and SYNGAP1 had an associated increase in translational rate (increased translation of these targets) by 30-50% in the absence of changes of RNA levels. In contrast, no changes in either RNA levels or translational rates were observed for the three eIF4G isoforms.

The set of genes identified via changes in translational efficiency or rate upon knockdown of the FMR1 gene is quite distinct from the corresponding set based on transcription. Of particular interest are the top 20 up- or down-regulated genes (log2 fold increase of 1.9-3.5 (p-value ≤0.001) or decrease of 1.5-2.2 (p-value ≤0.05), respectively) from changes in translational efficiency. Of these 40 genes, only 3 also had significant (p<0.05) movement in mRNA levels. As shown in Figure 37, 60 and 45% of these 20 translationally up- and 20 down-regulated genes, respectively, are associated with neurological disease or development. This enrichment for neurological association increased to 70% and 50% for the top 10 up- and down-regulated genes, respectively.

**Conclusions.** Fragile X is the most inheritable form of mental retardation. Current concepts of how FMRP regulates the translation of specific mRNAs are still being elucidated. This example shows that ribosome profiling and pathway analysis of genome-wide translational efficiencies after FMRP knockdown translationally regulates genes that are highly associated with neurological disease and development providing a novel insight into the key genes that are translationally regulated. The genes identified represent a new set of validated targets for points of intervention for the treatment of fragile X syndrome.

### Example 10: Translational Profiling of an Inflammation Cell Model

Macrophages treated with LPS have been shown to stimulate cytokine production as well as activation of both the PI3K and RAS pathways (Weintz et al., Mol. Sys. Biol. 371:1, 2010). In this example, LPS-induced macrophage activation was evaluated by monitoring TNF-α levels along with phosphorylation of components in the PI3K and RAS pathways.

**Cell Culture and TNFα Measurements.** RAW264.7 murine macrophages (ATCC) were cultured in DMEM containing 10% FBS supplemented with Penicillin, Streptomycin, Glutamax (Invitrogen) at 37°C in a humidified incubator with 5% CO2. Cells were treated with inhibitor or DMSO for 2 hrs prior to 1ng/ml LPS challenge (Sigma) for an additional 1 hr. Media was collected, centrifuged, and supernatants were used for TNF-α ELISA according to manufacturer's instructions (R&D Systems #MTA00B). Approximately 5 x 10⁶ cells/10 cm dish and 0.5 x 10⁶ cells/well of a 6-well plate were used for ribosome profiling and Western blot analysis, respectively.

**Western Blot Analysis.** Cells were washed with PBS and lysed in 1X cell lysis buffer (Cell Signaling) for 15 min at 4°C. Lysates were sonicated briefly, clarified by centrifugation for 15 min at 14, 000 rpm, and supernatants were then collected. Protein concentration in the soluble fraction was determined by BCA protein assay (Thermo Scientific). A 4-20% Bis-Tris gradient gel (Invitrogen) was used to resolve 20 µg of protein and transferred to nitrocellulose membrane. The resulting membranes were blocked for 1 hr at room temperature with Odyssey blocking solution (LI-COR) and then incubated with primary antibodies at 4°C overnight. The following day, the blots were washed 3 times, 10 min each in TBST, and incubated with IR-conjugated anti-rabbit IgG and anti-mouse IgG secondary antibody (IRDye 800 CW at 1:20,000; LI-COR) for 1 hour at room temperature. The blots were then washed, scanned, and specific proteins were detected using the LI-COR Odyssey infrared imager. The following antibodies were used at 1:1000 dilution from Cell Signaling: anti-phospho-4EBP(Ser65), anti-phospho-rpS6(Ser235/236) (#4858), anti-phospho-ERK1/2(Thr202/Tyr204) (#4370), anti-phospho-p70S6K(Thr421/Ser424) (#9204), anti-phospho-pAKT(Ser473), anti-phospho-eIF4E(Ser209), anti-phospho-RSK(Thr359/Ser363), anti-β-actin (#4970).

**Ribosomal Profiling.** Cells were washed with cold PBS supplemented with cycloheximide and lysed with 1x mammalian lysis buffer for 10 min on ice. Lysates were clarified by centrifugation for 10 min at 14,000 rpm and supernatants were collected. Cell lysates were processed to generate the ribosomal protected fragments and total mRNA according to the instructions included with the ARTseq Ribosome Profiling Kit. Sequencing of total RNA (RNA) and of ribosome-protected fragments of RNA (RPF) was carried out with standard Illumina rna seq methodology.

**Bioinformatics Analysis.** RNA-seq reads were processed with tools from the FASTX-Toolkit (fastq_quality_trimmer, fastx_clipper and fastx_trimmer). Unprocessed and processed reads were evaluated for a variety of quality measures using FastQC. Processed reads were mapped to the human genome using Tophat. Gene-by-gene assessment of the number of fragments strictly and uniquely mapping to the coding region of each gene was conducted using HTSeq-count, a component of the HTSeq package. Differential analyses of the stimulation of LPS and effect of drug treatment were carried out with the software packages DESeq for transcription (RNA counts) and translational rate (RPF counts) and BABEL for translational efficiency based upon ribosomal occupancy as a function of RNA level (RNA and RPF counts). Genes with low counts in either RPF or RNA were excluded from differential analyses. Pathway and network analyses of differential data was conducted using Ingenuity Pathway Analysis (IPA).

**Results.** These data show that after 1 hour of 1 ng/mL LPS stimulation, TNF-α levels were seen to rapidly increase (Figure 38), and increased phosphorylation was observed for RSK and ERK within the RAS pathway, as well as for AKT, S6K (modest) and S6 in the PI3K pathway (*see*, Figure 40). No changes in the phosphorylation levels of 4EBP, eIF4E or the housekeeping gene, β-actin, were discernable (Figure 40). Treatment with an inhibitor of a PI3K/Akt/mTOR pathway enzyme ("PAMi") or a MEK/ERK pathway enzyme ("MEi"), for 2 hours prior to the LPS stimulation, reduced the levels of TNF-α production (Figures 38 and 39). In particular, PAMi substantially inhibited phosphorylation of AKT, 4EBP and S6K at the lowest concentration tested and reduced phosphorylation of S6 and eIF4E, but did not alter the phosphorylation of RSK or ERK at the concentrations used (Figure 41). The MEi induced a dose dependent inhibition of the phosphorylation of ERK and S6 (with no effects on the phosphorylation of 4EBP, eIF4E, and S6K) (Figure 41), wherein preincubation with 16 nM MEi essentially prevented LPS stimulated production of TNF-α (Figure 39).

Ribosomal profiling was used to measure changes in transcription and translation on a genome-wide basis after stimulating macrophages with LPS. LPS is known to activate transcription for a number of genes. The majority of transcriptional changes were correlated with a change in translational rate as shown by the data points along the diagonal (*see*, Figure 42). Conversely, a significant number of changes in translational rate were independent of transcriptional changes (data points in red along the y-axis where x is zero). The level of TNF-α mRNA increased with LPS stimulation; however, the amount of ribosome protected fragments were in excess to the mRNA increases. These data demonstrate that TNF-α is regulated at the translational level in addition to having transcriptional changes. In addition, a number of genes were seen to be regulated at a translational level in the absence of a change in transcription levels providing a unique window of understanding the mechanism of inflammatory disease.

The gene sets for transcription, translational rate and translational efficiency were analyzed for pathway and network connections using IPA software. The output of the pathway analysis demonstrated that the transcriptome was strongly associated with inflammatory disease (p-value = 3.3E-09). The pathway analysis did not highlight pathways for the translational efficiency set of genes that were strongly supported statistically. However, the top 20 genes that were identified as translationally regulated were enriched for association with inflammatory diseases. Specifically, the top 10 translationally up- and down-regulated genes were enriched 70% and 50%, respectively, for association with inflammatory disease (p-value ≤0.05). Only 3 of these 20 translationally regulated genes were statistically significant for changes in RNA levels.

Treatment of macrophages with a PAMi or MEi followed with LPS stimulation showed that drug treatment was able to restore the translational efficiencies back to normal levels for the top 20 regulated genes. Interestingly, PAMi was more effective at renormalizing this gene subset when compared with MEi. Treatment of the cells with these drugs did not correspond with altering the translational efficiency of TNF-α. These results indicate that drug treatment modulates the level of TNF-α by regulating the translational levels of other inflammatory disease related genes.

This example shows that ribosome profiling and pathway analysis of genome-wide translational efficiencies after LPS stimulation translationally regulates genes that are highly associated with inflammatory disease providing a novel insight into the key genes that are translationally regulated.

### Example 11: Translational Profiling of Primary Cells

A subject diagnosed with prostate cancer (a Gleason 3+4 tumor) underwent a radical prostatectomy, and the isolated prostate was frozen. Samples removed from frozen pieces of the prostate were were reviewed by a pathologist and areas were deemed cancer versus normal. Translational profiles of normal prostate tissue and cancer prostate tissue were generated using ribosomal profiling as described herein. Figure 43 shows a representative comparison of the change in translational efficiency in normal versus tumor tissue, with each data point representing a single gene. Data points highlighted in red and green have statistically significant changes in translational efficiency versus the population of genes as a whole. For example, the green dots represent genes that have statistically significant lower ribosome occupancy and, therefore, a reduced translational efficiency as compared to the population of genes examined. The differential translational profile between the healthy and cancer tissues shows that there are many genes with significantly greater translational efficiency and significantly reduced translational efficiency (Figure 44).

## Claims

1. A method of identifying a subject as a candidate for treating a disease with a therapeutic agent, the method comprising:
(a) determining by ribosomal profiling a first translational profile for a plurality of genes in a sample from a subject having or suspected of having a disease selected from a cancer, an inflammatory disease, an autoimmune disease, a neurodegenerative disease, a neurodevelopmental disease, and a metabolic disease;
(b) determining by ribosomal profiling a second translational profile for a plurality of genes in a control sample, wherein the control sample is from a subject known to respond to the therapeutic agent and wherein the sample has not been contacted with the therapeutic agent; and
(c) identifying the subject as a candidate for treating the disease with the therapeutic agent when the first translational profile is comparable to the second translational profile.

2. The method of claim 1, wherein the disease is a cancer selected from prostate cancer, breast cancer, bladder cancer, lung cancer, renal cell carcinoma, endometrial cancer, melanoma, ovarian cancer, thyroid cancer, and brain cancer.

3. The method of claim 1 or 2, wherein the disease is cancer and the therapeutic agent modulates an oncogenic signaling pathway and optionally inhibits the activity of a downstream effector of the oncogenic signaling pathway.

4. The method of claim 1, wherein the disease is an inflammatory disease selected from ankylosing spondylitis, atherosclerosis, multiple sclerosis, systemic lupus erythematosus (SLE), psoriasis, psoriatic arthritis, rheumatoid arthritis, ulcerative colitis, inflammatory bowel disease, and Crohn's disease.

5. The method of claim 1, wherein the disease is a fibrotic disorder selected from pulmonary fibrosis, idiopathic pulmonary fibrosis, cystic fibrosis, liver fibrosis, cardiac fibrosis, endomyocardial fibrosis, atrial fibrosis, mediastinal fibrosis, myelofibrosis, retroperitoneal fibrosis, chronic kidney disease, nephrogenic systemic fibrosis, Crohn's disease, hypertrophic scarring, keloid, scleroderma, organ transplant associated fibrosis, and ischemia associated fibrosis.

6. The method of claim 1, wherein the disease is a neurodegenerative disease selected from Parkinson's disease, Alzheimer's disease, Amyotrophic Lateral Sclerosis, Creutzfeldt-Jakob disease, Huntington's disease, Lewy body dementia, frontotemporal dementia, corticobasal degeneration, primary progressive aphasia, and progressive supranuclear palsy.

7. The method of claim 1, wherein the disease is a neurodevelopmental disease selected from autism, autism spectrum disorders, Fragile X Syndrome, attention deficit disorder, and pervasive development disorder.

8. The method of any of claims 1-7, wherein the translational profiles comprise one or more gene signatures, and wherein the translational profiles of the one or more gene signatures are comparable in the first and second translational profiles.

9. The method of claim 8, wherein the one or more gene signatures comprise one or more genes having a 5' terminal oligopyrimidine tract (5' TOP) and/or a pyrimidine-rich translational element (PRTE).

10. The method of claim 8, wherein the one or more gene signatures comprise one or more biological pathways, optionally wherein the biological pathway is selected from a protein synthesis pathway, a cell invasion/metastasis pathway, a cellular metabolism pathway, a cell division pathway, an apoptosis pathway, a signal transduction pathway, a cellular transport pathway, a post-translational protein modification pathway, a DNA repair pathway, and a DNA methylation pathway.

11. The method of any of claims 1-10, wherein the first and second translational profiles are comparable when an amount of protein translated from one or more differentially translated genes in the first and second translational profiles differs by no more than 50%, 45%, 40%, 35%, 30%, 25%, 20%, 15%, 10%, 5%, 1% or less.

12. The method of any of claims 1-11, wherein each translational profile comprises a genome-wide translational profile.

13. The method of claim 12, wherein (a) less than 20% of the genes in the genome are differentially translated in the first translational profile as compared to the second translational profile, or (b) less than 5% of the genes in the genome are differentially translated by at least two-fold in the first translational profile as compared to the second translational profile, or (c) less than 1% of the genes in the genome are differentially translated by at least two-fold in the first translational profile as compared to the second translational profile.

14. The method of any one of claims 1-13, wherein in step (c) the plurality of genes of the first translational profile have a translational efficiency that is comparable to the translational efficiency of the plurality of genes of the second translational profile.

## Patentansprüche

1. Verfahren zur Identifizierung eines Subjekts als Kandidat für die Behandlung einer Krankheit mit einem therapeutischen Mittel, das Verfahren umfassend:
(a) Bestimmen durch ribosomales Profiling eines ersten Translationsprofils für eine Vielzahl von Genen in einer Probe von einem Subjekt, das eine Krankheit hat oder bei dem der Verdacht besteht, dass dieses eine Krankheit hat, die ausgewählt ist aus Krebs, einer Entzündungskrankheit, einer Autoimmunkrankheit, einer neurodegenerativen Krankheit, einer neurologischen Entwicklungskrankheit und einer Stoffwechselkrankheit;
(b) Bestimmen durch ribosomales Profiling eines zweiten Translationsprofils für eine Vielzahl von Genen in einer Kontrollprobe, wobei die Kontrollprobe von einem Subjekt stammt, von dem bekannt ist, dass es auf das therapeutische Mittel reagiert, und wobei die Probe nicht mit dem therapeutischen Mittel in Kontakt gebracht wurde; und
(c) Identifizieren des Subjekts als Kandidat für die Behandlung der Krankheit mit dem therapeutischen Mittel, wenn das erste Translationsprofil mit dem zweiten Translationsprofil vergleichbar ist.

2. Verfahren nach Anspruch 1, wobei die Krankheit ein Krebs ist, der ausgewählt ist aus Prostatakrebs, Brustkrebs, Blasenkrebs, Lungenkrebs, Nierenzellkarzinom, Endometriumkrebs, Melanom, Eierstockkrebs, Schilddrüsenkrebs und Hirnkrebs.

3. Verfahren nach Anspruch 1 oder 2, wobei die Krankheit Krebs ist und das therapeutische Mittel einen onkogenen Signalweg moduliert und optional die Aktivität eines nachgeschalteten Effektors des onkogenen Signalweges hemmt.

4. Verfahren nach Anspruch 1, wobei die Krankheit eine Entzündungskrankheit ist, die ausgewählt ist aus Spondylitis ankylosans, Atherosklerose, multipler Sklerose, systemischem Lupus erythematodes (SLE), Psoriasis, psoriatischer Arthritis, rheumatoider Arthritis, Colitis ulcerosa, entzündlicher Darmerkrankung und Morbus Crohn.

5. Verfahren nach Anspruch 1, wobei die Krankheit eine fibrotische Krankheit ist, die ausgewählt ist aus Lungenfibrose, idiopathischer Lungenfibrose, zystischer Fibrose, Leberfibrose, Herzfibrose, Endomyokardfibrose, Vorhoffibrose, mediastinaler Fibrose, Myelofibrose, retroperitonealer Fibrose, chronischer Nierenerkrankung, nephrogener systemischer Fibrose, Morbus Crohn, hypertrophischer Narbenbildung, Keloid, Sklerodermie, organtransplantationsassoziierter Fibrose und ischämieassoziierter Fibrose.

6. Verfahren nach Anspruch 1, wobei die Krankheit eine neurodegenerative Krankheit ist, die ausgewählt ist aus der Parkinson-Krankheit, der Alzheimer-Krankheit, der amyotrophen Lateralsklerose, der Creutzfeldt-Jakob-Krankheit, der Huntington-Krankheit, der Lewy-Körperchen-Demenz, der frontotemporalen Demenz, der kortikobasalen Degeneration, der primär-progressiven Aphasie und der progressiven supranukleären Lähmung.

7. Verfahren nach Anspruch 1, wobei die Krankheit eine neurologische Entwicklungskrankheit ist, die ausgewählt ist aus Autismus, Autismus-Spektrum-Störungen, Fragiles-X-Syndrom, Aufmerksamkeitsdefizit-Störung und pervasiver Entwicklungsstörung.

8. Verfahren nach einem der Ansprüche 1-7, wobei die Translationsprofile eine oder mehrere Gensignaturen umfassen und wobei die Translationsprofile der einen oder mehreren Gensignaturen in den ersten und zweiten Translationsprofilen vergleichbar sind.

9. Verfahren nach Anspruch 8, wobei die eine oder die mehreren Gensignaturen ein oder mehrere Gene mit einem 5'-terminalen Oligopyrimidin-Trakt (5' TOP) und/oder einem pyrimidinreichen Translationselement (PRTE) umfassen.

10. Verfahren nach Anspruch 8, wobei die eine oder die mehreren Gensignaturen einen oder mehrere biologische Wege umfassen, wobei der biologische Weg optional ausgewählt ist aus einem Proteinsyntheseweg, einem Zellinvasions-/Metastasierungsweg, einem zellulären Stoffwechselweg, einem Zellteilungsweg, einem Apoptoseweg, einem Signaltransduktionsweg, einem zellulären Transportweg, einem posttranslationalen Proteinmodifikationsweg, einem DNA-Reparaturweg und einem DNA-Methylierungsweg.

11. Verfahren nach einem der Ansprüche 1-10, wobei das erste und das zweite Translationsprofil vergleichbar sind, wenn sich eine Proteinmenge, die von einem oder mehreren unterschiedlich translatierten Genen im ersten und zweiten Translationsprofil translatiert wird, um nicht mehr als 50 %, 45 %, 40 %, 35 %, 30 %, 25 %, 20 %, 15 %, 10 %, 5 %, 1 % oder weniger unterscheidet.

12. Verfahren nach einem der Ansprüche 1-11, wobei jedes Translationsprofil ein genomweites Translationsprofil umfasst.

13. Verfahren nach Anspruch 12, wobei (a) weniger als 20 % der Gene im Genom im ersten Translationsprofil im Vergleich zum zweiten Translationsprofil differentiell translatiert werden, oder (b) weniger als 5 % der Gene im Genom im ersten Translationsprofil im Vergleich zum zweiten Translationsprofil um mindestens das Zweifache differentiell translatiert werden, oder (c) weniger als 1 % der Gene im Genom im ersten Translationsprofil im Vergleich zum zweiten Translationsprofil um mindestens das Zweifache differentiell translatiert werden.

14. Verfahren nach einem der Ansprüche 1-13, wobei im Schritt (c) die Vielzahl der Gene des ersten Translationsprofils eine Translationseffizienz aufweist, die mit der Translationseffizienz der Vielzahl der Gene des zweiten Translationsprofils vergleichbar ist.

## Revendications

1. Procédé d'identification d'un sujet comme candidat pour traiter une maladie avec un agent thérapeutique, le procédé comprenant :
(a) la détermination par profil ribosomique d'un premier profil de traduction pour une pluralité de gènes dans un échantillon provenant d'un sujet ayant ou suspecté d'avoir une maladie sélectionnée parmi un cancer, une maladie inflammatoire, une maladie auto-immune, une maladie neurodégénérative, une maladie neurodéveloppementale, et une maladie métabolique ;
(b) la détermination par profil ribosomique d'un deuxième profil de traduction pour une pluralité de gènes dans un échantillon témoin, dans lequel l'échantillon témoin provient d'un sujet connu pour répondre à l'agent thérapeutique et dans lequel l'échantillon n'a pas été mis en contact avec l'agent thérapeutique ; et
(c) l'identification du sujet comme candidat pour traiter la maladie avec l'agent thérapeutique lorsque le premier profil de traduction est comparable au deuxième profil de traduction.

2. Procédé selon la revendication 1, dans lequel la maladie est un cancer choisi parmi le cancer de la prostate, le cancer du sein, le cancer de la vessie, le cancer du poumon, le carcinome à cellules rénales, le cancer de l'endomètre, le mélanome, le cancer de l'ovaire, le cancer de la thyroïde et le cancer du cerveau.

3. Procédé selon la revendication 1 ou 2, dans lequel la maladie est le cancer et l'agent thérapeutique module une voie de signalisation oncogénique et inhibe éventuellement l'activité d'un effecteur en aval de la voie de signalisation oncogénique.

4. Procédé selon la revendication 1, dans lequel la maladie est une maladie inflammatoire choisie parmi la spondylarthrite ankylosante, l'athérosclérose, la sclérose en plaques, le lupus érythémateux disséminé (LED), le psoriasis, l'arthrite psoriasique, la polyarthrite rhumatoïde, la colite ulcéreuse, la maladie intestinale inflammatoire et la maladie de Crohn.

5. Procédé selon la revendication 1, dans lequel la maladie est un trouble fibrotique choisi parmi la fibrose pulmonaire, la fibrose pulmonaire idiopathique, la fibrose kystique, la fibrose hépatique, la fibrose cardiaque, la fibrose endomyocardique, la fibrose auriculaire, la fibrose médiastinale, la myélofibrose, la fibrose rétropéritonéale, la maladie rénale chronique, la fibrose systémique néphrogénique, la maladie de Crohn, les cicatrices hypertrophiques, les chéloïdes, la sclérodermie, la fibrose associée à une greffe d'organe et la fibrose associée à une ischémie.

6. Procédé selon la revendication 1, dans lequel la maladie est une maladie neurodégénérative choisie parmi la maladie de Parkinson, la maladie d'Alzheimer, la sclérose latérale amyotrophique, la maladie de Creutzfeldt-Jakob, la maladie de Huntington, la démence à corps de Lewy, la démence frontotemporale, la dégénérescence corticobasale, l'aphasie progressive primaire, et la paralysie supranucléaire progressive.

7. Procédé selon la revendication 1, dans lequel la maladie est une maladie neurodéveloppementale choisie parmi l'autisme, les troubles du spectre autistique, le syndrome de l'X fragile, le trouble déficitaire de l'attention et le trouble envahissant du développement.

8. Procédé selon l'une quelconque des revendications 1 à 7, dans lequel les profils de traduction comprennent une ou plusieurs signatures de gènes, et dans lequel les profils de traduction de la ou des signatures de gènes sont comparables dans les premier et deuxième profils de traduction.

9. Procédé selon la revendication 8, dans lequel la ou les signatures de gènes comprennent un ou plusieurs gènes ayant un tractus oligopyrimidine en 5' terminal (5' TOP) et / ou un élément de traduction riche en pyrimidine (PRTE).

10. Procédé selon la revendication 8, dans lequel la ou les signatures de gènes comprennent une ou plusieurs voies biologiques, éventuellement dans lesquelles la voie biologique est choisie parmi une voie de synthèse protéique, une voie d'invasion / métastase cellulaire, une voie de métabolisme cellulaire, une voie de division cellulaire, une voie d'apoptose, une voie de transduction de signal, une voie de transport cellulaire, une voie de modification protéique post-traductionnelle, une voie de réparation de l'ADN et une voie de méthylation de l'ADN.

11. Procédé selon l'une quelconque des revendications 1 à 10, dans lequel les premier et deuxième profils de traduction sont comparables lorsqu'une quantité de protéine traduite à partir d'un ou plusieurs gènes traduits différentiellement dans les premier et deuxième profils de traduction ne diffère pas de plus de 50%, 45%, 40%, 35%, 30%, 25%, 20%, 15%, 10%, 5%, 1% ou moins.

12. Procédé selon l'une quelconque des revendications 1 à 11, dans lequel chaque profil de traduction comprend un profil de traduction à l'échelle du génome.

13. Procédé selon la revendication 12, dans lequel (a) moins de 20% des gènes dans le génome sont traduits différentiellement dans le premier profil de traduction par rapport au deuxième profil de traduction, ou (b) moins de 5% des gènes dans le génome sont traduits différentiellement au moins deux fois dans le premier profil de traduction par rapport au deuxième profil de traduction, ou (c) moins de 1% des gènes dans le génome sont traduits de différentiellement au moins deux fois dans le premier profil de traduction par rapport au deuxième profil de traduction.

14. Procédé selon l'une quelconque des revendications 1 à 13, dans lequel à l'étape (c) la pluralité de gènes du premier profil de traduction a une efficacité de traduction qui est comparable à l'efficacité de traduction de la pluralité de gènes du deuxième profil de traduction.
